# EUROPEAN PATENT SPECIFICATION

(11) **EP 3 371 313 B1**
(45) Date of publication and mention of the grant of the patent: **14.10.2020**
(21) Application number: 16763143.1
(22) Date of filing: 26.08.2016
(51) Int. Cl.: C12N 15/82, C07K 14/195

(54) **METHODS AND COMPOSITIONS OF IMPROVED PLANT TRANSFORMATION**
VERFAHREN UND ZUSAMMENSETZUNGEN ZUR VERBESSERTEN PFLANZENTRANSFORMATION
PROCÉDÉS ET COMPOSITIONS DE TRANSFORMATION VÉGÉTALE AMÉLIORÉE

(30) Priority: 06.11.2015 US 201562252229 P
(43) Date of publication of application: 12.09.2018
(73) Proprietor: Pioneer Hi-Bred International, Inc., Johnston, Iowa 50131-1014 (US)
(72) Inventor: ANAND, Ajith, Johnston, Iowa 50131-0552 (US); BASS, Steven Henry, Johnston, Iowa 50131-0552 (US); CHO, Hyeon-Je, Johnston, Iowa 50131-0552 (US); KLEIN, Theodore Mitchell, Wilmington, Delaware 19810 (US); LASSNER, Michael, Portland, Oregon 97215 (US); MCBRIDE, Kevin E, Johnston, Iowa 50131-0552 (US)
(74) Representative: J A Kemp LLP
(86) International application number: PCT/US2016/049132
(87) International publication number: WO 2017/078836

(56) References cited:
- WO-A1-2014/157541
- WO-A2-2006/004914
- WO-A2-2012/016222
- L. D. HODGES ET AL: "Agrobacterium rhizogenes GALLS Protein Contains Domains for ATP Binding, Nuclear Localization, and Type IV Secretion", JOURNAL OF BACTERIOLOGY, vol. 188, no. 23, 1 December 2006 (2006-12-01), pages 8222-8230, XP55319172, US ISSN: 0021-9193, DOI: 10.1128/JB.00747-06

### SEQUENCE LISTING

<110> Pioneer Hi-Bred International, Inc.
   Anand, Ajith
   Bass, Steven
   Cho, Hyeon-Je
   Klein, Theodore
   Lassner, Michael
   McBride, Kevin
<120> METHODS AND COMPOSITIONS OF IMPROVED PLANT TRANSFORMATION
<130> 6469
<150> 62/252229
   <151> 2015-11-06
<160> 102
<170> PatentIn version 3.5
<210> 1
   <211> 534
   <212> DNA
   <213> Pseudomonas aeruginosa
<400> 1
   atgttacgca gcagcaacga tgttacgcag cagggcagtc gccctaaaac aaagttaggt 60
   ggctcaagta tgggcatcat tcgcacatgt aggctcggcc ctgaccaagt caaatccatg 120
   cgggctgctc ttgatctttt cggtcgtgag ttcggagacg tagccaccta ctcccaacat 180
   cagccggact ccgattacct cgggaacttg ctccgtagta agacattcat cgcgcttgct 240
   gccttcgacc aagaagcggt tgttggcgct ctcgcggctt acgttctgcc caagtttgag 300
   cagccgcgta gtgagatcta tatctatgat ctcgcagtct ccggcgagca ccggaggcag 360
   ggcattgcca ccgcgctcat caatctcctc aagcatgagg ccaacgcgct tggtgcttat 420
   gtgatctacg tgcaagcaga ttacggtgac gatcccgcag tggctctcta tacaaagttg 480
   ggcatacggg aagaagtgat gcactttgat atcgacccaa gtaccgccac ctaa 534
<210> 2
   <211> 1221
   <212> DNA
   <213> Escherichia coli
<400> 2
   gaccaagttt actcatatat actttagatt gatttaaaac ttcattttta atttaaaagg 60
   atctaggtga agatcctttt tgataatctc atgaccaaaa tcccttaacg tgagttttcg 120
   ttccactgag cgtcagaccc cgtagaaaag atcaaaggat cttcttgaga tccttttttt 180
   ctgcgcgtaa tctgctgctt gcaaacaaaa aaaccaccgc taccagcggt ggtttgtttg 240
   ccggatcaag agctaccaac tctttttccg aaggtaactg gcttcagcag agcgcagata 300
   ccaaatactg tccttctagt gtagccgtag ttaggccacc acttcaagaa ctctgtagca 360
   ccgcctacat acctcgctct gctaatcctg ttaccagtgg ctgctgccag tggcgataag 420
   tcgtgtctta ccgggttgga ctcaagacga tagttaccgg ataaggcgca gcggtcgggc 480
   tgaacggggg gttcgtgcac acagcccagc ttggagcgaa cgacctacac cgaactgaga 540
   tacctacagc gtgagctatg agaaagcgcc acgcttcccg aagggagaaa ggcggacagg 600
   tatccggtaa gcggcagggt cggaacagga gagcgcacga gggagcttcc agggggaaac 660
   gcctggtatc tttatagtcc tgtcgggttt cgccacctct gacttgagcg tcgatttttg 720
   tgatgctcgt caggggggcg gagcctatgg aaaaacgcca gcaacgcggc ctttttacgg 780
   ttcctggcct tttgctggcc ttttgctcac atgttctttc ctgcgttatc ccctgattct 840
   gtggataacc gtattaccgc ctttgagtga gctgataccg ctcgccgcag ccgaacgacc 900
   gagcgcagcg agtcagtgag cgaggaagcg gaagagcgcc tgatgcggta ttttctcctt 960
   acgcatctgt gcggtatttc acaccgcata tggtgcactc tcagtacaat ctgctctgat 1020
   gccgcatagt taagccagta tacactccgc tatcgctacg tgactgggtc atggctgcgc 1080
   cccgacaccc gccaacaccc gctgacgcgc cctgacgggc ttgtctgctc ccggcatccg 1140
   cttacagaca agctgtgacc gtctccggga gctgcatgtg tcagaggttt tcaccgtcat 1200
   caccgaaacg cgcgaggcag c 1221
<210> 3
   <211> 2559
   <212> .DNA
   <213> Pseudomonas aeruginosa
<400> 3
   taaaacagct tgcgtcatgc ggtcgctgcg tatatgatgc gatgagtaaa taaacaaata 60
   cgcaagggga acgcatgaag gttatcgctg tacttaacca gaaaggcggg tcaggcaaga 120
   cgaccatcgc aacccatcta gcccgcgccc tgcaactcgc cggggccgat gttctgttag 180
   tcgattccga tccccagggc agtgcccgcg attgggcggc cgtgcgggaa gatcaaccgc 240
   taaccgttgt cggcatcgac cgcccgacga ttgaccgcga cgtgaaggcc atcggccggc 300
   gcgacttcgt agtgatcgac ggagcgcccc aggcggcgga cttggctgtg tccgcgatca 360
   aggcagccga cttcgtgctg attccggtgc agccaagccc ttacgacata tgggccaccg 420
   ccgacctggt ggagctggtt aagcagcgca ttgaggtcac ggatggaagg ctacaagcgg 480
   cctttgtcgt gtcgcgggcg atcaaaggca cgcgcatcgg cggtgaggtt gccgaggcgc 540
   tggccgggta cgagctgccc attcttgagt cccgtatcac gcagcgcgtg agctacccag 600
   gcactgccgc cgccggcaca accgttcttg aatcagaacc cgagggcgac gctgcccgcg 660
   aggtccaggc gctggccgct gaaattaaat caaaactcat ttgagttaat gaggtaaaga 720
   gaaaatgagc aaaagcacaa acacgctaag tgccggccgt ccgagcgcac gcagcagcaa 780
   ggctgcaacg ttggccagcc tcgcagacac gccagccatg aagcgggtca actttcagtt 840
   gccggcggag gatcacacca agctgaagat gtacgcggta cgccaaggca agaccattac 900
   cgagctgcta tctgaataca tcgcgcagct accagagtaa atgagcaaat gaataaatga 960
   gtagatgaat tttagcggct aaaggaggcg gcatggaaaa tcaagaacaa ccaggcaccg 1020
   acgccgtgga atgccccatg tgtggaggaa cgggcggttg gccaggcgta agcggctggg 1080
   ttgcctgccg gccctgcaat ggcactggaa cccccaagcc cgaggaatcg gcgtgagcgg 1140
   tcgcaaacca tccggcccgg tacaaatcgg cgcggcgctg ggtgatgacc tggtggagaa 1200
   gttgaaggca gcgcaggccg cccagcggca acgcatcgag gcagaagcac gccccggtga 1260
   atcgtggcaa gcagccgctg atcgaatccg caaagaatcc cggcaaccgc cggcagccgg 1320
   tgcgccgtcg attaggaagc cgcccaaggg cgacgagcaa ccagattttt tcgttccgat 1380
   gctctatgac gtgggcaccc gcgatagtcg cagcatcatg gacgtggccg ttttccgtct 1440
   gtcgaagcgt gaccgacgag ctggcgaggt gatccgctac gagcttccag acgggcacgt 1500
   agaggtttcc gcagggccgg caggcatggc cagtgtgtgg gattacgacc tggtactgat 1560
   ggcggtttcc catctaaccg aatccatgaa ccgataccgg gaagggaagg gagacaagcc 1620
   cggccgcgtg ttccgtccac acgttgcgga cgtactcaag ttctgccggc gagccgatgg 1680
   cggaaagcag aaagacgacc tggtagaaac ctgcattcgg ttaaacacca cgcacgttgc 1740
   catgcagcgt acgaagaagg ccaagaacgg ccgcctggtg acggtatccg agggtgaagc 1800
   cttgattagc cgctacaaga tcgtaaagag cgaaaccggg cggccggagt acatcgagat 1860
   cgagctagct gattggatgt accgcgagat cacagaaggc aagaacccgg acgtgctgac 1920
   ggttcacccc gattactttt tgatcgatcc cggcatcggc cgttttctct accgcctggc 1980
   acgccgcgcc gcaggcaagg cagaagccag atggttgttc aagacgatct acgaacgcag 2040
   tggcagcgcc ggagagttca agaagttctg tttcaccgtg cgcaagctga tcgggtcaaa 2100
   tgacctgccg gagtacgatt tgaaggagga ggcggggcag gctggcccga tcctagtcat 2160
   gcgctaccgc aacctgatcg agggcgaagc atccgccggt tcctaatgta cggagcagat 2220
   gctagggcaa attgccctag caggggaaaa aggtcgaaaa ggtctctttc ctgtggatag 2280
   cacgtacatt gggaacccaa agccgtacat tgggaaccgg aacccgtaca ttgggaaccc 2340
   aaagccgtac attgggaacc ggtcacacat gtaagtgact gatataaaag agaaaaaagg 2400
   cgatttttcc gcctaaaact ctttaaaact tattaaaact cttaaaaccc gcctggcctg 2460
   tgcataactg tctggccagc gcacagccga agagctgcaa aaagcgccta cccttcggtc 2520
   gctgcgctcc ctacgccccg ccgcttcgcg tcggcctat 2559
<210> 4
   <211> 720
   <212> DNA
   <213> Agrobacterium tumefaciens
<400> 4
   atgcttaaga gatcggggtc gctttctctt gccttgatgg tctccttctg ttcgtcgagc      60
   cttgccacgc cactctcatc tgctgagttt gaccatgttg ctcgcaagtg tgccccatca     120
   gttgcgacat ctacgcttgc ggcgatagct aaggtggaga gtcgctttga tcctttagcg     180
   attcatgaca acacgaccgg cgaaacgctt cactggcaag atcacagcca agcaacccaa     240
   gtcgtcaggc accgtctcga tgcacggcat tcgctggatg ttggcctcat gcaaataaac     300
   tctcgaaatt tttctatgct cggtctgaca cctgacggtg cgctccaggc gtgcacatca     360
   ttatctgccg ctgcaaacat gctgaaaagt cgttatgcag gcggcgaaac gattgacgag     420
   aagcaatttg cgcttcgtcg ggcgatctcc gcttacaaca ccggtaattt catcggcggt     480
   tttgcaaacg gctacgtgcg aaaagttgaa acagctgctc aatcgctggt gcccgcgtta     540
   atcgagcctc caaaagacga tcacgaggcg ctaaaatccg aagagacgtg ggatgtttgg     600
   gggtcatatc agcgccgctc gcaggaggat ggcgctggcg gtttaatcgc tccgccaccg     660
   ccacaccagg acaacggcaa atccgcagac gacaatcaag tcttattcga cttatactaa     720
<210> 5
   <211> 365
   <212> DNA
   <213> Agrobacterium tumefaciens
<400> 5
   atgcgatgct ttgagagata ccgtttacat ctaaatcgcc tctcgctctc gaatgcgatg      60
   atgcgcgtga tatcgagctg cgccccaagc ttgtgcggtg caattgcatg gagcatttcc     120
   tcatccggac ccgccgcagc gcaatctgcg ggtggcggca ctgaccccgc cacaatggtt     180
   aacaatatat gcacgtttat ccttggtccg ttcggccagt cactcgctgt tctcggcatt     240
   gtcgctatcg ggatctcctg gatgttcggg cgggcttcgc ttgggctggt tgccggcgtc     300
   gtcggcggca ttgttatcat gtttggggcg agcttcctcg gccaaacgct cactggcggt     360
   agttg                                                                 365
<210> 6
   <211> 326
   <212> DNA
   <213> Agrobacterium tumefaciens
<400> 6
   atggctgatc gtttggaaga atcgaccctt tacctcgcag ccacacggcc cgcattgttt      60
   cttggggtgc cactgacatt ggcagggtta ttcatgatgt tcgccggctt tgtcatcgtt     120
   atcgttcaga acccgctcta cgaagtcgtt ctcgtgccgt tatggtttgc agcccggctc     180
   atcgtggagc gagactacaa tgcggcgagc gtcgtcctgc tatttttgcg gaccgcggga     240
   agaagcattg atagtgcagt ttgggggggc gctactgtta gcccaaatcc aattagggtt     300
   cccccacgag ggagaggaat ggtgtg                                          326
<210> 7
   <211> 2370
   <212> DNA
   <213> Agrobacterium tumefaciens
<400> 7
   atgctcggcg cgagtggaac gaccgaaaga tccggtgaga tctatctccc ttatattggc 60
   cacctcagcg accatatcgt ccttcttgaa gacggatcga tcatgaccat tgcgagaatt 120
   gatggcgttg cattcgagct tgaggaaact gaaatgcgca atgcgcgttg tcgtgcgttc 180
   aacacgctgt tgcgcaatat cgctgatgat catgtgtcaa tatatgctca cctcgtacgt 240
   catgccgacg tgccatcatc ggcgccgcga cacttccgta gtgttttcgc cgctagcctg 300
   aacgaagctt ttgaacagcg cgtgctctcc ggccaactcc tccgcaatga acacttcctt 360
   acgttgattg tctacccaca ggcggcttta gggaaggtaa agaggaggtt caccaagcta 420
   agcggaaaaa gggaaaacga tctcacgggc cagatcagga acatggaaga tctttggcat 480
   gttgtcgctg gctctcttaa agcgtatggc ctgcatcgtc ttggcatccg cgagaagcag 540
   ggtgtgctct tcaccgaaat tggcgaagcg ctacggttga tcatgactgg tcggttcaca 600
   ccggttccgg tcgtcagcgg ctcactcggc gcttcgattt ataccgacag agtcatttgc 660
   ggcaagcgag gactcgagat cagaacgcca aaagacagtt acgttggatc catctattcg 720
   tttcgcgaat accctgcaaa aacacggccg ggcatgctca acgcgctgct atccctcgat 780
   tttccacttg ttctcacgca gagtttttcg ttcctgactc gcccgcaagc gcacgcgaaa 840
   cttagcctca aatcgagcca gatgctgagt tccggcgata aagccgtgac tcaaatcggc 900
   aaattatccg aggctgagga cgcacttgcg agcaacgaat tcgttatggg ctcacatcat 960
   ttgagccttt gcgtctatgc agacgatctc aatagtcttg gggacagggg cgcgcgggct 1020
   cggacacgaa tggcggatgc aggtgccgtg gttgtccaag aaggtattgg tatggaagcg 1080
   gcctattggt cccaattgcc ggggaatttt aagtggcgca cacgccctgg cgcaatcact 1140
   tcacgcaatt tcgcagggtt tgtctctttc gaaaactttc cagagggcgc cagctcaggc 1200
   cactggggca acgcgattgc ccgatttcgt accaatggcg gaacgccttt cgactatatc 1260
   ccgcatgagc acgatgttgg catgacggca atattcgggc ctatcgggag gggtaagacg 1320
   acgctcatga tgtttgttct agccatgctc gaacagagca tggtcgaccg tgcaggtacg 1380
   gtcgtgttct ttgacaagga ccggggtggc gaattgctgg ttcgcgccac aggaggaaca 1440
   tatttggcac ttcacagagg cacacccagc gggttggcgc cgttgcgtgg cctagaaaac 1500
   acagcagcct cacacgattt tctgcgcgaa tggatcgtgg ctctcatcga gagtgatggt 1560
   cggggtggga tttctccgga agagaaccgc cgtctggtcc gtggtatcca tcgtcagctc 1620
   tcgtttgatc cacaaatgcg ttcaatcgcg gggttacgtg aatttttgtt gcatgggccc 1680
   gccgaaggcg caggagcgcg gctccaacgc tggtgccggg gccatgcgct tggctgggca 1740
   tttgacggcg aagttgacga agtaaagtta gatccgtcga ttaccggctt cgacatgacg 1800
   catcttctcg aatacgagga agtatgcgct cccgctgcag catatctcct gcatcggatt 1860
   ggagccatga tcgacggccg ccgttttgtg atgagctgcg atgagtttcg cgcctatttg 1920
   ttaaacccta aattttcgac tgtcgtcgac aaattcctcc tgaccgttcg aaaaaacaac 1980
   gggatgctaa tactggcaac gcagcaacca gagcatgttc tggaatcgcc gctaggagcc 2040
   agcttggttg cgcaatgtat gacgaagatt ttctatccat caccaaccgc agatcgatcg 2100
   gcttatgtcg atggactgaa atgtaccgaa aaggaatttc aggcgatccg tgaagacatg 2160
   acggtcggca gccgtaagtt tcttcttaaa cgagaaagtg gaagcgtcat ctgcgaattt 2220
   gatctgcggg atatgcgtga atatgtcgcc gtgctttcgg ggcgtgccaa cacggtgcgc 2280
   tttgcaactc gactacgcga ggcacaagaa ggcaactcat ctggctggct cagcgaattc 2340
   atggcccgtc accacgaggc agaagattga 2370
<210> 8
   <211> 663
   <212> DNA
   <213> Agrobacterium tumefaciens
<400> 8
   atgaagacga cgcaacttat tgcaacagtt ttgacctgca gctttctata tattcagccc 60
   gcgcgggcgc agtttgttgt tagcgacccg gcaacggagg ctgagacgct cgcgactgcg 120
   ctcgcgactg cggagaatct cactcagact atagcgatgg ttacgatgtt gacgtcggcc 180
   tacggcgtta ctggactact gacttcgctc aaccagaaaa atcagtatcc ttcgacgaag 240
   gacctagaca atgaaatgtt ttcgccgcga atgccaatgt cgaccacggc acgtgcgatc 300
   accagcgata cagatcgtgc agtcgtgggt agtgatgctg aagcggacct gttgcgatcg 360
   cagatcaccg gttccgcaaa cagcgctggc attgcggctg acaatctgga aacgatggac 420
   aaacgcttga cggcgaatgc tgatacgtct gctcagcttt cccgatctcg caatatcatg 480
   caggcaaccg tgaccaatgg tttgcttctc aagcagatcc atgacgcaat gattcaaaat 540
   gtacaggcga caagcctatt aacgatgact accgcgcagg ccggccttca cgaggcggaa 600
   gaggcggccg ctcaacgcaa ggagcatcaa aagaccgctg tcatctttgg tgccctcccc 660
   taa 663
<210> 9
   <211> 888
   <212> DNA
   <213> Agrobacterium tumefaciens
<400> 9
   atgaatttca cgattccggc gccgtttacg gccattcata cgatcttcga tgtagccttc      60
   acgacaggct tggactcgat gcttgagact atccaggagg cggtgagtgc gccattgatc     120
   gcctgtgtca ctctttggat tattgttcag ggtattttag tcatacgcgg cgaagtcgat     180
   acccgtagcg gtatcactcg ggtgatcacg gtcaccatcg ttgttgctct aattgttggg     240
   caggctaact accaagacta tgtggtttcc atcttcgaaa agacggtccc aaactttgtt     300
   cagcagttta gtgtaaccgg cttgcctctg cagactgttc cggcacagtt ggatacaatg     360
   ttcgccgtga cccaggccgt ttttcagaaa atcgcatccg aaatcggtcc gatgaacgac     420
   caggacatcc ttgctttcca aggggcacag tgggtccttt acggcacgct ctggtctgcc     480
   ttcggagttt acgacgccgt tggaattctc acgaaagtgc ttctcgcgat cgggcctctg     540
   atcctcgtcg gatatatttt tgatcgcacg cgggacatcg cagctaagtg gatcgggcaa     600
   cttatcacct acggtctctt gcttctcctc ttaaacctcg tggcaacgat cgtcatccta     660
   accgaagcga ctgcgctcac ccttatgctt ggtgtaatca ccttcgccgg tacgaccgcg     720
   gccaagatca ttggtcttta cgaactcgat atgttttttc tgacagggga tgcgctcatt     780
   gtcgctttgc cggcgatcgc cggcaacatt ggaggcagtt actggagcgg cgcaacccaa     840
   tctgccagca gcttgtaccg tcgcttcgct caggttgagc gaggctag                  888
<210> 10
   <211> 154
   <212> DNA
   <213> Agrobacterium tumefaciens
<400> 10
   atgaagtatt gcctgctgtg cctagttgtc gctttgagcg gctgccagac aaacgacaca      60
   ttagcgagct gcaaaggccc gatcttcccg ctgaatgtgg ggcgatggca gcctactccg     120
   tcagatcttc agctcggcaa ttcgggtgga cgct                                154
<210> 11
   <211> 710
   <212> DNA
   <213> Agrobacterium tumefaciens
<400> 11
   atgacggggc ctgaatatgc catgctagtg gcgcgcgaaa gccttgccga gcactataag 60
   gaagtagaag cctttcaaac cgcgcgagcg aaatcggcgc gacgtctctc caaactcatt 120
   gcagctgtcg cagctatcgc gattttggga aatgttgctc aagcgttcgc tatagccaca 180
   atggtgccgt tgagcaggct tgtgcccgta tatctatgga tacggccgga cggcaccgtt 240
   gacagcgagg tgtctgtctc gcgattgcct gcaactcaag aggaggccgt cgttaacgcc 300
   tcattgtggg agtacgttcg cctgcgcgag agttatgatg ccgacaccgc tcagtacgcc 360
   tacgacctgg tatcgaactt cagtgcccca acagtgcgcc aggattacca gcaattcttc 420
   aactatccca atcccagttc gcctcaagtc attcttggca aacgcggcag ggtggaggtc 480
   gagcacatcg cttcaaatga tgtaactcca agcacgcagc aaattcgcta taaaaggacc 540
   ctcgtcgttg acggcaaaat gcctgtggtg agtacgtgga ccgcgacagt tcgctacgaa 600
   aaggtgacca gcttgcccgg cagattgaga ctaaccaacc cggcaggtct ggttgtcacc 660
   tcctatcaga catcggaaga taccgtttca aacgtaggcc acagcgaacc 710
<210> 12
   <211> 878
   <212> DNA
   <213> Agrobacterium tumefaciens
<400> 12
   atgatcagaa aagcactttt cattttagca tgtttatttg ccgctgcgac tggtgcggag 60
   gctgaagaca ctccaatggc gggcaagcta gatccgcgca tgcgttattt ggcttacaat 120
   cccgatcaag tggtgcgcct ctcgacggcg gttggagcta ctttggtcgt aacattcgcc 180
   acgaacgaaa cggtgacagc ggttgccgtt tcaaatagca aagatctagc agccctaccg 240
   cggggaaatt atctattttt caaggcaagc caggtcctca cgcctcagcc agtaatcgtg 300
   ctaaccgcaa gcgactccgg gatgcgccgt tatgttttca gtataagttc caagactctg 360
   tcccacctcg ataaagagca gcccgatctc tattacagcg tccaattcgc ctaccccgcc 420
   gacgatgcgg cggctcggcg aagggaggca caacagaagg ctgttgtgga cagactacac 480
   gcggaagcac aatatcaacg gaaagctgag aatttattgg atcagcctgt cacagccctt 540
   ggtgcggcgg acagtaattg gcactacgtc gcccaaggcg atcgttcgct gttgccactc 600
   gaagtcttcg acaatggatt tacgacggta ttccactttc cgggcaatgt acgcataccc 660
   tccatctaca ccatcaatcc tgatggcaag gaagctgttg ccaactattc agttaaaggg 720
   agcgatgtcg agatttcttc ggtttcccga ggttggcgtc tgagggatgg ccacacagta 780
   ctatgtatct ggaacaccgc ttacgatccc gttggccaaa ggccgcaaac gggcacggtg 840
   aggcccgatg tgaaacgcgt cctgaagggg gcgaaggg 878
<210> 13
   <211> 1134
   <212> DNA
   <213> Agrobacterium tumefaciens
<400> 13
   atgaataacg atagtcagca agcggcacat gaggttgatg catctggatc cctggtctcc 60
   gacaaacatc gccggcgtct ttcggggtct cagaaattga tcgtcggagg tgtcgttctc 120
   gcgttatcat taagcctcat ttggctaggt gggcgccaaa agaaggtgaa tgagaacgca      180
   tcgccgtcaa ctttgatcgc aacaaacacc aagccatttc atccagctcc gattgaggtg      240
   ccgccggatc ctccagcggt tcaagaggct gttcagcctg ctgctcctct accgccgagg      300
   ggcgaaccgg agcggcatga gccacggccg gaagaaacac cgatttttgc atatagcagc      360
   ggcgatcaag gggtcagcaa acgcgccatt cagggcgaca cgggccgaag acaagaaggc      420
   aagcgtgacg acaactcctt gccgaatggc gaagtgtccg gcgagaacga tttgtcgata      480
   cgtatgaaac ccaccgagct gcagcccagc agcgccacgc tcttgccgca ccccgatttt      540
   atggtaacgc aagggacaat aattccgtgc atcttgcaaa ccgcaatcga cacaaatttg      600
   gcaggctatg taaagtgtgt cttgcctcag gatattcgtg gaacaacgaa caatatcgtg      660
   cttcttgatc gtggcaccac cgttgttggc gaaatacagc gtggcttgca acagggagat      720
   gggcgcgttt ttgtgttgtg ggatcgcgcc gagacacctg accatgcgat gatctcgtta      780
   acatcgccaa gcgcggacga actcggtcgc tcaggattgc cgggctcggt cgacagccac      840
   ttctggcagc gttttagcgg agctatgctc ttgagtgttg ttcaaggcgc cttccaggca      900
   gctagcacct acgccggcag ctcgggtggc gggatgagct tcaacagctt tcaaaataac      960
   ggtgagcaga caactgagac agcccttaag gcaaccatca acataccgcc aaccctgaag     1020
   aagaatcagg gtgacaccgt ttccattttc gtagcacggg acctcgattt ctttggtgtt     1080
   taccagctcc gcctgactgg cggcgccacg cgggggagga accgccgctc ttaa           1134
<210> 14
   <211> 1032
   <212> DNA
   <213> Agrobacterium tumefaciens
<400> 14
   atggaagtgg atccgcaact acgctttctt ctgaagccga ttttggaatg gctcgatgac      60
   ccgaagactg aagaaattgc gatcaatcga cctggagagg catttgtgcg ccaagccggc     120
   atttttacca agatgccttt gcccgtctct tatgatgatc ttgaagatat cgctatttta     180
   gcgggcgcgc tgagaaagca ggatgtcgga ccacgtaacc ccctctgcgc cactgaactt     240
   cctggtggtg aacgactaca aatctgtctg ccgccgaccg ttccctcggg caccgtcagc     300
   ttgaccattc gacggccaag ctcgcgtgtt tctggtctta aagaagtctc ctcccgttat     360
   gatgcttcga ggtggaacca gtggcagaca cgaaggaaac gccaaaatca ggatgatgaa     420
   gctatccttc agcattttga caacggggat ttggaagcgt ttctgcacgc atgcgtcgtc     480
   agccgactga cgatgttgct atgtggccct accggaagcg gcaagacaac aatgagcaag     540
   accttgatca gcgccatccc cccccaggaa aggctaatca ccatagaaga tacgctcgaa     600
   ctcgtcattc cacacgataa tcatgttaga ctactctact ccaagaacgg tgctgggctg     660
   ggtgctgtga gcgccgagca cttgctccaa gcaagtctgc gtatgcggcc ggaccggata     720
   ttgcttggcg agatgcgcga cgatgcagca tgggcttatc tgagtgaagt cgtctcggga     780
   catccgggat cgatttcaac aatacacggc gcgaatccaa tccaaggatt caagaaactg     840
   ttttcccttg tcaaaagtag cgcccaaggt gctagcttgg aagatcgcac actgattgac     900
   atgctctcta cggcgatcga tgtcatcatt ccattccgtg cctatgagga cgtttatgaa     960
   gtaggcgaga tctggctcgc ggcggacgca cgacgccggg gcgagaccat aggcgatctc    1020
   cttaatcaat ag                                                        1032
<210> 15
   <211> 804
   <212> DNA
   <213> Agrobacterium tumefaciens
<400> 15
   atgattgtac atccttcacg tgaaaatttc tcaagcgctg tgaacaaggg ttcagatttt      60
   agattgagag gtgagccgtt gaaacacgtt cttcttatcg atgacgatgt cgctatgcgg     120
   catcttatta tcgaatacct tacgatccac gccttcaaag tgaccgcggt agccgacagc     180
   acccagttca ctagagtact ctcttccgcg acggtcgatg tcgtggttgt tgatctaaat     240
   ttaggtcgtg aagatgggct tgagatcgtt cgaaatctgg cggcaaagtc tgatattcca     300
   atcataatta tcagtggcga ccgccttgag gagacggata aagttgttgc actcgagcta     360
   ggagcaagtg attttatcgc taagccgttt agtacgagag agtttcttgc acgcattcgg     420
   gttgccttgc gcgtgcgccc caacgttgtc cgctccaaag accgacggtc tttttgtttt     480
   actgactgga cacttaatct caggcaacgt cgcttgatgt ccgaagctgg cggtgaggtg     540
   aaacttacgg caggtgagtt caatcttctc ctcgcgtttt tagagaaacc ccgcgacgtt     600
   ctatcgcgcg agcaacttct cattgccagt cgagtacgcg acgaggaggt ttacgacagg     660
   agtatagatg ttctcatttt gcggctgcgc cgcaaacttg aggcggatcc gtcaagccct     720
   caactgataa aaacagcaag aggtgccggt tatttctttg acgcggacgt gcaggtttcg     780
   cacgggggga cgatggcagc ctga                                            804
<210> 16
   <211> 696
   <212> DNA
   <213> Agrobacterium tumefaciens
<400> 16
   atgcaacttt tgacgttttg ttctttcaaa gggggtgctg gcaaaaccac cgcactcatg      60
   ggcctttgcg ctgctttggc aaatgacggt aaacgagtgg ccctctttga tgccgacgaa     120
   aaccggcctc tgacgcgatg gagagaaaac gccttacaaa gcagtacctg ggatcctcgc     180
   tgtgaagtct attccgccga cgaaatgccc cttcttgaag cagcctatga aaatgccgag     240
   ctcgaaggat ttgattatgc gttggccgat acgcgtggcg gctcgagcga gctcaacaac     300
   acaatcatcg ctagctcaaa cctgcttctg atccccacca tgctaacgcc gctcgacatc     360
   gatgaggcac tatctaccta ccgctacgtc atcgagctgc tgttgagtga aaatttggca     420
   attcctacag ctgttttgcg ccaacgcgtc ccggtcggcc gattgacaac atcgcaacgc     480
   aggatgtcag agacgctaga gagccttcca gttgtaccgt ctcccatgca tgaaagagat     540
   gcatttgccg cgatgaaaga acgcggcatg ttgcatctta cattactaaa cacgggaact     600
   gatccgacga tgcgcctcat agagaggaat cttcggattg cgatggagga agtcgtggtc     660
   atttcgaaac tgatcagcaa aatcttggag gcttga                              696
<210> 17
   <211> 609
   <212> DNA
   <213> Agrobacterium tumefaciens
<400> 17
   atggcaattc gcaagcccgc attgtcggtc ggcgaagcac ggcggcttgc tggtgctcga 60
   cccgagatcc accatcccaa cccgacactt gttccccaga agctggacct ccagcacttg 120
   cctgaaaaag ccgacgagaa agaccagcaa cgtgagcctc tcgtcgccga tcacatttac 180
   agtcccgatc gacaacttaa gctaactgtg gatgccctta gtccacctcc gtccccgaaa 240
   aagctccagg tttttctttc agcgcgaccg cccgcgcctc aagtgtcgaa aacatatgac 300
   aacctcgttc ggcaatacag tccctcgaag tcgctacaaa tgattttaag gcgcgcgttg 360
   gacgatttcg aaagcatgct ggcagatgga tcatttcgcg tggccccgaa aagttatccg 420
   atcccttcaa ctacagaaaa atccgttctc gttcagacct cacgcatgtt cccggttgcg 480
   ttgctcgagg tcgctcgaag tcattttgat ccgttggggt tggagaccgc tcgagctttc 540
   ggccacaagc tggctaccgc cgcgctcgcg tcattctttg ctggagagaa gccatcgagc 600
   aattggtga 609
<210> 18
   <211> 444
   <212> DNA
   <213> Agrobacterium tumefaciens
<400> 18
   atgtcaaaac acaccagagc cacgtcgagt gagactacca tcaaccagca tcgatccctg 60
   aaagttgaag ggttcaaggt cgtgagtgcc cgtctgcgat cggccgagta tgaaaccttt 120
   tcctatcaag cgcgcctgct gggactttcg gatagtatgg caattcgcgt tgcggtgcgc 180
   cgcatcgggg gctttctcga aatagatgca gacacacgag aaaagatgga agccatactt 240
   cagtccatcg gaatactctc aagcaatgta tccatgcttc tatctgccta cgccgaagac 300
   cctcgatcgg atctggaggc tgtgcgagat gaacgtattg cttttggcga ggctttcgcc 360
   gccctcgatg gactactgcg ctccattttg tccgtatccc ggcgacggat cgacggtcgc 420
   tcgttactga aaggtgcctt gtag 444
<210> 19
   <211> 1275
   <212> DNA
   <213> Agrobacterium tumefaciens
<400> 19
   atgcccgatc gcgctcaagt aatcattcgc attgtgccag gaggtggaac caagaccctt      60
   cagcagataa tcaatcagct ggagtacctg tcccgaaagg gaaagctgga actgcagcgt     120
   tcagcccggc atctcgatat tcccgttccg ccggatcaaa tccgtgagct tgcccaaagc     180
   tgggttacgg aggccgggat ttatgacgaa agtcagtcag acgatgacag gcaacaagac     240
   ttaacaacac acattattgt aagcttcccc gcaggtaccg accaaaccgc agcttatgaa     300
   gcaagccggg aatgggcagc cgagatgttt gggtcaggat acgggggtgg ccgctataac     360
   tatctgacag cctaccacgt cgaccgcgat catccacatt tacatgtcgt ggtcaatcgt     420
   cgggaacttc tggggcaggg gtggctgaaa atatccaggc gccatcccca gctgaattat     480
   gacggcttac ggaaaaagat ggcagagatt tcacttcgtc acggcatagt cctggatgcg     540
   acttcgcgag cagaaagggg aatagcagag cgaccaatca catatgctga atatcgacgc     600
   cttgagcgga tgcaggctca aaagattcaa ttcgaagata cagattttga tgagacctcg     660
   cctgaggaag atcgtcggga cctcagtcaa tcgttcgatc catttcgatc ggacgcatct     720
   gccggcgaac cggaccgtgc aacccgacat gacaaacaac cgcttgaacc gcacgcccgt     780
   ttccaggagc ccgccggctc cagcatcaaa gccgacgcac ggatccgcgt accattggag     840
   agcgagcggg gtgcccaacc atccgcgtcc aaaatccctg taactgggca tttcgggatt     900
   gagacttcgt atgtcgctga agccagcgtg cccaaacaaa gcggcaattc cgatacttct     960
   cgcccggtga ctgacgttgc catgcacaca gtcgagcgcc agcagcgatc aaaacgacgt    1020
   catgacgagg aggcaggtcc gagcggagca aaccgtaaaa gattgaaggc cgcgcaagtt    1080
   gattccgagg caaatgtcgg tgagcccgac ggtcgcgatg acagcaacaa ggcggctgat    1140
   ccggtgtctg cttccatccg taccgagcaa ccggaagctt ctccaacgtg tccgcgtgac    1200
   cgtcacgatg gagaattggg agaacgcaaa cgtgcaagag gtaatcgtcg cgacgatggg    1260
   cgcgggggga cctag                                                     1275
<210> 20
   <211> 635
   <212> DNA
   <213> Agrobacterium tumefaciens
<400> 20
   atggcaaatg gtcagttcac gatacgctct gctcgcccgg cctccgtcgg actgacaggc       60
   gaacggcgtg gagccgcatc cgcctctagc tctgcactgt ccaatgttca aagagatgtt      120
   agggataggc tgattccaac tagctcacca agattaccaa atgcagccat attgcgtgat      180
   tcctcgggaa gagcgtcgac tggtctgcgg tacatggcgg ctactcttca ttggtctgcg      240
   atcgcgccat tatcgctaat aaacagcaac gacctggctc cggccgctta tgactttgag      300
   acgcgaaata acgcaagaaa tgtgactgcc aaagtcggca gggcagtccc tgttcccaag      360
   caaggcgggc tcggcaaaac gctcgcaccc gtacccctta gtacacgtat atcaagggtc      420
   aattccgacc gaagactgcc cgctgacgca gaagaccgcc ctgaaacgcg cgacccccag      480
   aaaggacgtg gcagtcatgg tgcgacgcca accttacatg aaaagattgg aaccgcgttt      540
   gctcgaagat tgcgaaagca tacgtactat attgtttgca gttgctgcca gacccggagc      600
   gcgttgacga tgggtgcaaa gatttcggtg aagtc                                 635
<210> 21
   <211> 1959
   <212> DNA
   <213> Agrobacterium tumefaciens
<400> 21
   atgaactcca gcaagacttc gccccagcgt atgaccctga gcatcgtatg ttcgctggca       60
   gccggttttt gtgcggccag ctgctatgta acgttccgcc ggggcttcaa cggcgaagcg      120
   atgatgacgt tcgacgtttt cgctttttgg tatgagaccc cgctttactt gggttatgcc      180
   agcaccgtct tctggcgtgg tttatctgtt gtcatcttta cctcgctgat cgttctttca      240
   agtcagctca tcatatcgct gcgcaatcag aagcatcatg ggacagctcg ttgggcagaa      300
   attggcgaaa tgcggcatgc tggttatctg cagcgttaca gtcgcatcaa ggggccgatc      360
   tttggaaaga catgtggtcc cctttggttc ggcagttatt tgaccaatgg cgaacagcca      420
   cacagtcttg tcgtcgcgcc aacgcgtgct ggcaaaggcg tcggcatcgt cattccaacg      480
   ctgttgacct tcaagggctc ggtaatcgcc cttgacgtca agggagaatt gtttgaactg      540
   acgtccagag cacgcaaagc gagcggcgac gcagttttca agttctcccc cctagatcct      600
   gagcggaaga ctcattgtta caatccggtc ctggatattg ccgcacttcc gcccgaacgc      660
   cagttcactg aaacacgccg tctagctgcg aaccttatta cggctaaggg aaagggagca      720
   gaaggcttta ttgacggcgc acgtgacctg ttcgtcgcgg gaatccttac ctgcattgag      780
   cgtggcacac caacgattgg cgcggtatat gacctatttg cgcagcctgg cgaaaagtat      840
   aagctttttg cgcaactcgc ggaggaaagc ctaaacaaag aggctcagcg tatcttcgat      900
   aatatggcgg gcaacgacac gaaaattctg acatcgtaca cctctgtgct gggcgacggt      960
   ggactgaacc tgtgggctga tccgcttatc aaagcagcga caagccggtc agacttttcc 1020
   gtttacgatc tccggaggaa gaagacctgc atttatcttt gtgtcagtcc caacgatctg 1080
   gaggtcttgg caccacttat gcgcctgatg tttcagcagc tcgtgtcaat cttgcagaga 1140
   tcgctgccag gtgaagacga gtgccatgaa gttttatttc tcctcgacga attcaaacac 1200
   ctgggcaagc ttgaggccat agagaccgcg atcacaacca tcgccggtta cagaggccgc 1260
   tttatgttta ttattcaaag tctttcggcc ttgtcgggca catacgatga cgcaggaaaa 1320
   caaaactttc tgagcaatac tggcgtacaa gtatttatgg ccacggctga tgacgaaact 1380
   ccaacctaca tctcaaaagc tatcggcgaa tatacgttta aagcgcgttc gacctcttac 1440
   agtcaagcca gaatgttcga ccacaacatc cagatttctg atcaaggtgc agcccttttg 1500
   cgccccgaac aagtgcgcct gctagacgat cagagtgaaa tcgttctcat caaagggcga 1560
   cctccactca aattacgaaa ggtgcagtat tattccgatc gtacgctgaa aggccttttc 1620
   gaacgccaga tgggctctct gcctgagccc gcacccttga tgctttccga ctatagcaac 1680
   gatcaagttc aataccactt ggctccgata gcaaatttta atgaggatgc tgcaccgcaa 1740
   aacagaactg tggccgagga ccatggaagt gttaaagtcg gtgctgatat ccctgaacgc 1800
   gtgatgggaa taaatggtga cgaggaacaa gccgatgcgg gcgagatacc gccggaatcg 1860
   gttgtgcctc cagaattgac gctcgctctg accgctcaac agcaattgtt ggaccagatt 1920
   attgcacttc agcaaagatc gaggtccgca ccggcatag 1959
<210> 22
   <211> 2511
   <212> DNA
   <213> Agrobacterium tumefaciens
<400> 22
   atgacaggaa agtcgaaagt tcacataaga ggttcggctg acgcgcttcc tgacgttcct      60
   ggcggaagta ctaccgcccc ttttttaacc gaaccttctc gggatcaggt tgatgcctcg     120
   tttgaggtcc aaaccgacta cagccagtct acttccgtgt cgtttaccta tgatggtgtt     180
   ggacttggtc ctgccgagcg tgcggcttac gagaactggt gcgaaccggg ccggcccact     240
   tggaaagatc ttataatcaa ggcacgtgtc gatccgattg acgatgtgac ctggctccga     300
   gatttagaag aggacacccc ctcaaccttc agatacgaag ggatgcctct gggcatcggg     360
   gaacgacagg cctacgaaaa ttggcaagag gacgctcagc cgacatggga agaccttgtt     420
   gtcagcgcac gcttgacgga acttggccgt ccacacggga ttaccggcga gtatacatcc     480
   ctcgcaggat cgaagaatac aagttcaatt tcattgaagc gaaagcggag caacttaatt     540
   gatgatgaga attcatccgg atcgttttca tatgacggga tgaagctcgg ggaagccgag     600
   cgttctgcat atggtgactg ggccgaggcg gagccaccca cgtggaaaga tttggtattg     660
   agggcacgcg tttcctcgat caatgactct gcttggcttt ttgattcaca aacatcttca 720
   tcatcatttg aatacaacgg tgttcccttg ggcgagccgg aacggcaggc tctcagacaa 780
   tggcaaggag acgctcagcc tacctgggaa gatctcgttg ttaacgcgcg tatggcagaa 840
   ctttgccatg ctggttggat tgaaggtcaa aaaggttgct ttgaagagcg cggggaggct 900
   ctgcccgcgt cggaacgcgg ttcgcaacgc cccattggtc aacggacaga ttcctccgat 960
   tcttttgtgt atgatggcac aaggctcgga gcacctgagc gaactgctta tgaacgctgg 1020
   agtaagaggg aacgcccgac ttgggaagat ctcatcttag atgcacacca ggccaggact 1080
   gaaagtgacg ctgttacgac ccaagcgatt ggtcagtcgt cctcaccggt tttcttatat 1140
   gaaggaaagt cgctcggaga cagggaacga aaggcttacg aaaaatggcg gcagccagcc 1200
   caaccgcgat ggcaaaatct tgtagtgaac gctcgtctgg cagaaatcga tccctcagcc 1260
   tggattgccg atgagcgcga tccgcttgat gatagcgacg cgcttggtcg cccgtcgtac 1320
   acaagcttga cggatagatc agacgtccct ttagacgatc aatcaatcta tcgtcgttcc 1380
   gacctagtaa gggagcaggt gccagaatcg tctcaaaggc aattcgcagc atgttcagaa 1440
   tctgaaacga ggcctgtgca atggtttact gcttctgggt cagatgcaaa caatacggaa 1500
   aatatcaccg ccagcgatcc cgtcgatcgc acgggtggag ttaagcggct aggctccaaa 1560
   agcgacagaa ccgttacagc ttctatccat gacgtgaatt ccagcacaag gcgactgttg 1620
   cttaacgaat ttggatcgga ggctccgcgc ccttcgccag aaaagactgt tcgcttaaga 1680
   agcgacaata ttggcaccta tgggagccgg aaaaatgaac gagcgcggct cgcgaccgaa 1740
   accggtgcgt atgagtcgga gcatattttc gggttcaagg ctgtccacga tactgcgaga 1800
   gcgacgaaag agggccggcg tctcgaaagg cccatgcccg cctaccttga ggataagggg 1860
   cttcatcgcc aacatattgg caccgggaga ggacggacca aacttgtcgg gcgcggatgg 1920
   ccggatgaca caagctatcg ctcggatcaa agggcaactc tgtcggaccc cgttgcgcgc 1980
   tcggaaggcg cgacggcctc aaatgggtat caattgaacc aattgggtta cgcgcaccaa 2040
   ctcgctagcg atggcctgca aagtgaatcg cccgatggtg ttgccttgcc aattcaagtg 2100
   gcaacaacga gctacaacta tacagtgagc cgcgatcctg tccttgttcc gccggataaa 2160
   aacgaagccc ctcaattgct gcatcttggt ccccgtggtc aaaccgaagc tgttcttgcc 2220
   cgcgaaacag cattgactgg aaaatggccg actctcgagc gtgagcagca agtgtatcgc 2280
   gagtttttgg ccttatatga cgtaaaaaaa gatcttgagg ccaaatcagt cggcgtaaga 2340
   cggaaaaaaa aagaagttat ttctgcgtta gaccgaactg cgcgcttgat aagcacgtcg 2400
   ccttcgaaag ctcgatccaa agcagagact gaaaaagcca ttgatgagct cgatgatcga 2460
   cgagtttatg atccgcgtga tcgagctcaa gacaaagcgt ttaaacgctg a 2511
<210> 23
   <211> 198
   <212> DNA
   <213> Agrobacterium tumefaciens
<400> 23
   atggccatca tcaagccgca tgtgaacaaa aataggacaa cctcgccgat agagagaccg        60
   gagtctctca tagaggaaat gagcggcagt catccgccga gtggttttac caacctggat       120
   ctcgctatga tcgagctgga ggactttgtc catcggtgcc cgctcccaga agacaatctt       180
   gctggtcaga aggagtga                                                     198
<210> 24
   <211> 1650
   <212> DNA
   <213> Agrobacterium tumefaciens
<400> 24
   atggatccgt ctagcaatga gaatgtctat gtgggtcgcg gtcacaacat cgaaaatgat 60
   gatgacactg accccaggcg ttggaagaag gcgaatatca gttccaacac catctccgat 120
   attcagatga cgaatggcga agacgtacaa tcagggagcc ctacccgaac ggaagttgta 180
   agcccacgtc tggattatgg atcggtcgac tcctcctcca gcctttattc tggcagcgag 240
   cacggaaatc aagctgagat tcaaaaagag ctgtccgtct tgttctcgaa catgtctttg 300
   ccaggcaacg atcggcgccc ggacgaatac attctcgtgc atcaaacggg acaagatgct 360
   tttactggta ttgccaaagg caacctcgac caaatgccca ccaaggcgga atttaacgcg 420
   tgctgccgtc tctacaggga cggagccggt aattactacc cgccacctct cgcattcgac 480
   aagattagcg ttccagagca actggaggaa aaatggggga tgatggaggc gaaggaacgt 540
   aacaaactgc ggtttcagta caagttggac gtatggaatc atgcgcacgc tgatatgggg 600
   atcacgggca cagagatctt ttatcaaaca gataagaaca taaagctcga ccggaattat 660
   aaactaagac ctgaagaccg atacgtacaa acagaaaaat acgggcgccg ggaaattcaa 720
   aagcgatatc aacacgaact ccaggctggt tcgctgctgc ccgatattat gatcaaaact 780
   ccccaaaatg acatccactt cgtgtacagg tttgccggcg acaattacgc caacaaacag 840
   ttcagcgagt ttgaacacac cgtcaagcgc aggtatggcg acgagactga gatcaaattg 900
   aagtcaaagt caggcattat gcatgactcg aaatatctgg aatcctggga acggggcagt 960
   gcggatattc gcttcgcgga attcgttggg gaaaatagag ctcacaatcg gcagtttcca 1020
   actgcgacag taaatatggg acagcagcca gacgggcagg gcggtttgac ccgcgaccgt 1080
   catgtgagcg ttgacttcct aatgcaaagc gcacccaatt cgccttgggc gcaagctttg 1140
   aaaaagggag aactgtggga tcgcgttcag ttgcttgctc gcgacggcaa ccgctatctg 1200
   tcgccgccca gattggaata ttctgaccct gcacatttca ccgagttgat gaaccgggtt 1260
   ggtttacccg catcgatggg tcggcaaagc catgcggcta gtatcaaatt cgaaaagttt 1320
   gacgcgcagg cagcggttat tgtcttaaat ggcccagagt tacgtgacat tcatgacttg 1380
   tctcctgaaa aactgcaaaa tttgtccacc aaagatgtca tcgtcgccga tcgcaatgag 1440
   aatggtcaga gaactggcac gtacaccagc gtcgcggaat atgagcgctt gcagttaagg 1500
   ctgccacccg atgcagcggg ggtgcttggt gaagcaactg acaaatattc acgtgatttc 1560
   gttcggccag agccggcgtc gcgtccaatc agtgacagcc gcaggatata cgaaagtcga 1620
   ccgcgtagcc aaagcgtcaa cagcttttga 1650
<210> 25
   <211> 2019
   <212> DNA
   <213> Agrobacterium tumefaciens
<400> 25
   atggtgaaca ctacaaagaa aagttttgcg aagtcgctta cggcagatat gcgccgttct      60
   gctcagcgcg ttgtcgagca aatgcgaaaa gcattgatta ccgaagaaga ggcgctcaag     120
   cggcaagcca gactggagag tcccgatagg aagcgaaagt atgctgctga tatggcgata     180
   gtcgacaaac tcgacgtagg gtttcgaggc gaaataggct ataaaattct tggaaataac     240
   cggcttcgag tagacaacca taaagaatta acgcgtgagc acggtagact tcgcaaaacc     300
   aaaacggttc tgaagcgtaa cccggtgacg caggaagtct atttgggttt atatgaaagg     360
   aagtcctggt taagtgtcag cagccatttg tatgctgcgg acggcacact ccgcatgaag     420
   cacgtgaaat acaaagacgg acgttttgag gaaaaatggg agcgcgacga aaatggcgac     480
   ctgatccgca caaggtacgc caaccgtggc aggctctttc aacctgtatc cgagaaaatg     540
   ggcgcgccgt atcggagcgg ccctgacgac cggctctatc gcgatctaac ccgtcgaaac     600
   ggtttcagac gggagacatt cgaacgggac gatcacggaa acctcgagcg tatcggcagc     660
   aaccatgtcg gcttttccaa gatttcagtg aaggcaccca atcgtcaaac ctcccagacg     720
   aagattcaaa aacttggtgg cgctttcaac aaatctttta ggtcccttct ggacaaggag     780
   ggcaatgaaa tgggccgcga tattttgagc catcgacggc tctataacaa gcggtctgct     840
   gtctacgatg aagctaccgg acaattgaag agtgccaagc ataccttcgg caagatctac     900
   aggagcgaaa ccgaatatct cagcgcgggc ctcaagaagg tttcaaaaaa gatactcggg     960
   gtgacggtct accggaaatt tgcggcgctc agcgagcgag aatccgaggc tgagagactg    1020
   cgtagttttg aatccggtgc gcatcgccag atctggcagg agcgggcagc gattcccggt    1080
   tcgcccctcc cggagactga tgacattcat ttcgcacagc agtcgcacct agccaaagcc    1140
   aaccctgatc acgtcgaagc tgacgtcacg cgtgtgacag atcaacatgt tgatgttgct    1200
   ggacaaacat catcgtctcc ccaacggaac ttggaaggat ggttagattc tcaatcacga    1260
   tacaagccag caaacatgct gttgtcaaat ccagaccttc aagcgaacgg acctcgccca     1320
   tacgaagggt tagctcatct caccctccgg cgcgataatg aatctgacgg gcacaaggag     1380
   aatgatcagc ggctgcgaca tttctcccag ccagagccgt tggtgttacc gcatcccggg     1440
   tcgccggaaa taactaaggt gtttggctcg cggggagagc cggcacaccc gagtggaaat     1500
   ctgcacacgg cggttggaga aacggcttgc gaaggaccgg tgatgtcttc atcctcggac     1560
   aatcatcagc cagctccagg acagcaagaa cttttaagtt tccttcataa tgcgccagcc     1620
   ccagtttctg tggcaataca tgatgatcaa gagcgacttg cgggggaggc gcccggcggc     1680
   tctttcagag gtagctcagg gcgaacgagt tcaatgtcgg agagtatctt cgacgaagat     1740
   gtacaagggc atttggtacg ggattattcg atcaatacta ctaacgggtt tattgacccg     1800
   caatcgttgt tcggtgaacc ggacttatcg agaggtccaa aatcggggcc agaaattcca     1860
   tcggaagatt accatttgtc agcttcggaa caggaaaatt tgctgaatca attgcttagt     1920
   gtgccactgc cggttccttc accgaagccc gaatgcgcga ggtctatgat tttcgaaggt     1980
   tcacgttcaa gagagcgttc cacctccaga gggttctaa                            2019
<210> 26
   <211> 2490
   <212> DNA
   <213> Agrobacterium tumefaciens
<400> 26
   atgaacggaa ggtattcacc gacgcggcag gattttaaga caggcgcgaa gccttggtct 60
   atattagccc ttatcgttgc cgcaatgatt ttcgcgttca tggcggttgc gtcctggcag 120
   gacaatggga ctacccaggc aatccttagc caaatacgat cgattaacgc tgacagcgcc 180
   tcactgcagc gcgatgtact ccgcgctcac acgggcaccg tggcgaacta ccgccccatt 240
   atctccaggc tgggagctct gcggaagaat ctggaagatt tgaagcaatt atttaggcaa 300
   tctcatattg taagtgagag caatgctgct caactgctac gccggctaga agtgtctcta 360
   aattcggctg acgcggcggt cgccgccttt ggtgcgcaaa atgtccgcct gcaagattcg 420
   ctggccagtt tcactcgtgc tttaagcaat cttccaggaa aggcctcagg cgatcagact 480
   ttagaaaaac caacagaatt ggctagcatg atgctccaat ttcttcggca accaagcccg 540
   gctatttcat tcgagatcag ccttgaacta gagaggctcc aaaaacaacg cggtcttgat 600
   gaagctcccg tgcgcatact ttcgcgtgaa ggtcccatta tcttatcgct tttgccacag 660
   gtgaacgatc tggtgaacat gattcagacg tctgacaccg cagaaattgc ggaaatgttg 720
   cagcgtgagt gtttggaggt ctatagcttg aaaaatgtgg aggagcggag cgcacgtatc 780
   tttcttgggt ccgcttcagt gggtctttgc ctgtacatca tcaccttagt ctataggcta 840
   cgcaaaaaaa ccgattggtt agcgcggcgt ttagattacg aagagctaat caaagagatc 900
   ggagtatgtt ttgaaggtga ggcggccact acgtcgtccg cgcaagctgc acttggtatt      960
   attcagcgct tctttgatgc cgatacgtgc gcgttagctc tagtggacca tgaccgtagg     1020
   tgggctgtcg aaacattcgg tgcgaagcac ccaaaacccg tgtgggacga cagggtgcta     1080
   cgcgaaatag tctctcgtac caaagcgaac gaacgggcga cggtattccg catcgtatcg     1140
   acgcaaaaaa tcgtacattt gcctcccgaa attccaggtc tttcgatact actggctcac     1200
   aaatccacag ataaactaat tgcggtttgt tcactgggtt accaaagcta tcgccctcga     1260
   ccttgccaag gcgaaattca gcttcttgaa ctcgccaccg cctgcctctg tcactatatc     1320
   gatgttcggc gtaagcagac cgaatgcgac gttttggcca gacgattgga gcatgcgcaa     1380
   cgccttgagg cagttggtac acttgccggc ggaatagcac atgaatttaa taacattttg     1440
   gggtcaatcc tcgggcacgc agaattggcc caaaactcgg tgtctcgaac atctgtcacc     1500
   cgaagataca ttgactatat catttcgtca ggcgacagag ccatgctcat tatcgatcag     1560
   atcttgacgc tgagccgaaa acaagagcgc gtgatcaagc catttagtgt ctcagagctt     1620
   gtgaccgaaa tcgctccctt gctacgtatg gctcttcccc caaccatcga gcttagtttc     1680
   agatttgatc aaatgcagag cgtgatcgaa ggaagcccgc ttgaacttca acaggtacta     1740
   attaacctct gcaagaatgc ttcccaagcc atgactgcaa atggtcaaat cgacatcttc     1800
   gtcggccaag cttatttacc agctaagaaa attctggcgc atggtgttat gccacctggc     1860
   gactatgttc tactatctgt cagcgacaat ggtggaggca tttcagaggc tgtgctaccc     1920
   tacatttttg aacccttctt tacgacacgt gctcgcaacg gtggaacggg tctcggcctc     1980
   gcttctgtgc atggtcatat cagcgcgttt gcaggttaca tcgacgttag ttcaactgtt     2040
   gggcatggga cgcgctttga catttatctc cctccgtctt ctaaggagcc cgtcaatccc     2100
   gacagctttt tcggccgcaa taaggcaccg cgtggaaacg gggagattgt ggcacttgtt     2160
   gagcccgatg acctcctgcg ggaggcgtat gaagacaaga tcgccgctct gggatatgag     2220
   ccggtcggtt ttcgtacctt tagtgaaatt cgcgattgga tttcaaaagg caatgaagcc     2280
   gatctggtca tggtcgacca agcgtctctt cctgaagatc aaagtcctaa ttccgtggat     2340
   ttagtgctca agaccgcctc catcatcatt ggcggaaatg atctcaaaat gcccctttca     2400
   agggagaatg cgaccaggga cctttatctg ccgaagccga tatcgtccag aactatggcg     2460
   catgcaatcc taaccaaaat caagacgtag                                      2490
<210> 27
   <211> 744
   <212> DNA
   <213> Agrobacterium tumefaciens
<400> 27
   atggcgataa aattggtatt gatactcgta ttcacactct ttcccgcggc agacgctgca 60
   tatgcgaatg accgcgccaa cggtttcatg tggtcaaacg ggggcgaaac tggagtgagg 120
   cttcctcttc gggtgttcaa tgccaagcca gccaagaaca cggtggcgat catttattcc 180
   ggagacgctg gatggcaaaa tatcgatgag gcgattggta cctatctgca gacggaaggg 240
   attcctgtca ttggcgtcag ttcacttcgg tatttctggt cggagcggtc cccaagcgaa 300
   actgctaagg atcttggtca cataatcgat gtctacacca agcatttcgg tgtgcagaat 360
   gttttacttg taggatattc tttcggcgcg gacgtcatgc cggcaagctt caataggctt 420
   acgcttgagc aaaaaaatcg ggttaagcaa atctctctct tggcattgtc acatcaagtc 480
   gactatgtcg tctcatttag gggctggctc caactcgaaa cggaaggtaa gggcggcaat 540
   cctctggatg atctcagatc cattgaccct gcaatcgtcc aatgcatgta cgggcgcgaa 600
   gaccgtaata atgcttgccc atctctccga cagaccggcg cagaggtgat aggcttcagc 660
   ggaggccatc actttgataa tgatttcaaa aaactgtcta cgcgcgtcgt ctcaggcctc 720
   gtggcacgcc taagtcatca gtaa 744
<210> 28
   <211> 7743
   <212> DNA
   <213> Artificial sequence
<220>
   <223> Synthetic construct
<400> 28
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgt tgaaggagcc actcagcaag 180
   ctggtacgat tgtaatacga ctcactatag ggcgaattga gcgctgttta aacgctcttc 240
   aactggaaga gcggttacgc tgtttaaacg ctcttcaact ggaagagcgg ttactaccgg 300
   ttcactagct agctgctaag gttaccagag ctggtcacct ttgtccacca acttattaag 360
   tatctagttg aagacacgtt cttcttcacg taagaagaca ctcagtagtc ttcggccaga 420
   atggcctctt gattcagcgg gcctagaagg ccggatcact gactagctaa tttaaatcct 480
   gaggatatcg ctatcaactt tgtatagaaa agttgggccg aattcgagct cggtacggcc 540
   agaatggccc ggaccgggtt acccggaccg aagcttgcat gcctgcagtg cagcgtgacc 600
   cggtcgtgcc cctctctaga gataatgagc attgcatgtc taagttataa aaaattacca 660
   catatttttt ttgtcacact tgtttgaagt gcagtttatc tatctttata catatattta 720
   aactttactc tacgaataat ataatctata gtactacaat aatatcagtg ttttagagaa 780
   tcatataaat gaacagttag acatggtcta aaggacaatt gagtattttg acaacaggac 840
   tctacagttt tatcttttta gtgtgcatgt gttctccttt ttttttgcaa atagcttcac 900
   ctatataata cttcatccat tttattagta catccattta gggtttaggg ttaatggttt 960
   ttatagacta atttttttag tacatctatt ttattctatt ttagcctcta aattaagaaa 1020
   actaaaactc tattttagtt tttttattta ataatttaga tataaaatag aataaaataa 1080
   agtgactaaa aattaaacaa atacccttta agaaattaaa aaaactaagg aaacattttt 1140
   cttgtttcga gtagataatg ccagcctgtt aaacgccgtc gacgagtcta acggacacca 1200
   accagcgaac cagcagcgtc gcgtcgggcc aagcgaagca gacggcacgg catctctgtc 1260
   gctgcctctg gacccctctc gagagttccg ctccaccgtt ggacttgctc cgctgtcggc 1320
   atccagaaat tgcgtggcgg agcggcagac gtgagccggc acggcaggcg gcctcctcct 1380
   cctctcacgg caccggcagc tacgggggat tcctttccca ccgctccttc gctttccctt 1440
   cctcgcccgc cgtaataaat agacaccccc tccacaccct ctttccccaa cctcgtgttg 1500
   ttcggagcgc acacacacac aaccagatct cccccaaatc cacccgtcgg cacctccgct 1560
   tcaaggtacg ccgctcgtcc tccccccccc ccctctctac cttctctaga tcggcgttcc 1620
   ggtccatgca tggttagggc ccggtagttc tacttctgtt catgtttgtg ttagatccgt 1680
   gtttgtgtta gatccgtgct gctagcgttc gtacacggat gcgacctgta cgtcagacac 1740
   gttctgattg ctaacttgcc agtgtttctc tttggggaat cctgggatgg ctctagccgt 1800
   tccgcagacg ggatcgattt catgattttt tttgtttcgt tgcatagggt ttggtttgcc 1860
   cttttccttt atttcaatat atgccgtgca cttgtttgtc gggtcatctt ttcatgcttt 1920
   tttttgtctt ggttgtgatg atgtggtctg gttgggcggt cgttctagat cggagtagaa 1980
   ttctgtttca aactacctgg tggatttatt aattttggat ctgtatgtgt gtgccataca 2040
   tattcatagt tacgaattga agatgatgga tggaaatatc gatctaggat aggtatacat 2100
   gttgatgcgg gttttactga tgcatataca gagatgcttt ttgttcgctt ggttgtgatg 2160
   atgtggtgtg gttgggcggt cgttcattcg ttctagatcg gagtagaata ctgtttcaaa 2220
   ctacctggtg tatttattaa ttttggaact gtatgtgtgt gtcatacatc ttcatagtta 2280
   cgagtttaag atggatggaa atatcgatct aggataggta tacatgttga tgtgggtttt 2340
   actgatgcat atacatgatg gcatatgcag catctattca tatgctctaa ccttgagtac 2400
   ctatctatta taataaacaa gtatgtttta taattatttt gatcttgata tacttggatg 2460
   atggcatatg cagcagctat atgtggattt ttttagccct gccttcatac gctatttatt 2520
   tgcttggtac tgtttctttt gtcgatgctc accctgttgt ttggtgttac ttctgcaggt 2580
   cgactctaga ggatccaccg gtcgccacca tggcccacag caagcacggc ctgaaggagg 2640
   agatgaccat gaagtaccac atggagggct gcgtgaacgg ccacaagttc gtgatcaccg 2700
   gcgagggcat cggctacccc ttcaagggca agcagaccat caacctgtgc gtgatcgagg 2760
   gcggccccct gcccttcagc gaggacatcc tgagcgccgg cttcaagtac ggcgaccgga 2820
   tcttcaccga gtacccccag gacatcgtgg actacttcaa gaacagctgc cccgccggct 2880
   acacctgggg ccggagcttc ctgttcgagg acggcgccgt gtgcatctgt aacgtggaca 2940
   tcaccgtgag cgtgaaggag aactgcatct accacaagag catcttcaac ggcgtgaact 3000
   tccccgccga cggccccgtg atgaagaaga tgaccaccaa ctgggaggcc agctgcgaga 3060
   agatcatgcc cgtgcctaag cagggcatcc tgaagggcga cgtgagcatg tacctgctgc 3120
   tgaaggacgg cggccggtac cggtgccagt tcgacaccgt gtacaaggcc aagagcgtgc 3180
   ccagcaagat gcccgagtgg cacttcatcc agcacaagct gctgcgggag gaccggagcg 3240
   acgccaagaa ccagaagtgg cagctgaccg agcacgccat cgccttcccc agcgccctgg 3300
   cctgaagcgg ccgcaaccta gacttgtcca tcttctggat tggccaactt aattaatgta 3360
   tgaaataaaa ggatgcacac atagtgacat gctaatcact ataatgtggg catcaaagtt 3420
   gtgtgttatg tgtaattact agttatctga ataaaagaga aagagatcat ccatatttct 3480
   tatcctaaat gaatgtcacg tgtctttata attctttgat gaaccagatg catttcatta 3540
   accaaatcca tatacatata aatattaatc atatataatt aatatcaatt gggttagcaa 3600
   aacaaatcta gtctaggtgt gttttgcgaa tgcggccgcc accgcggtgg agctcgaatt 3660
   ccggtccggg tcacccggtc cgggcctaga aggccgatct cccgggcacc cagctttctt 3720
   gtacaaagtg gccgttaacg gatcggccag aatggcccgg accgggttac ccggaccgaa 3780
   gcttgcatgc ctgcagtgca gcgtgacccg gtcgtgcccc tctctagaga taatgagcat 3840
   tgcatgtcta agttataaaa aattaccaca tatttttttt gtcacacttg tttgaagtgc 3900
   agtttatcta tctttataca tatatttaaa ctttactcta cgaataatat aatctatagt 3960
   actacaataa tatcagtgtt ttagagaatc atataaatga acagttagac atggtctaaa 4020
   ggacaattga gtattttgac aacaggactc tacagtttta tctttttagt gtgcatgtgt 4080
   tctccttttt ttttgcaaat agcttcacct atataatact tcatccattt tattagtaca 4140
   tccatttagg gtttagggtt aatggttttt atagactaat ttttttagta catctatttt 4200
   attctatttt agcctctaaa ttaagaaaac taaaactcta ttttagtttt tttatttaat 4260
   aatttagata taaaatagaa taaaataaag tgactaaaaa ttaaacaaat accctttaag 4320
   aaattaaaaa aactaaggaa acatttttct tgtttcgagt agataatgcc agcctgttaa 4380
   acgccgtcga cgagtctaac ggacaccaac cagcgaacca gcagcgtcgc gtcgggccaa 4440
   gcgaagcaga cggcacggca tctctgtcgc tgcctctgga cccctctcga gagttccgct 4500
   ccaccgttgg acttgctccg ctgtcggcat ccagaaattg cgtggcggag cggcagacgt 4560
   gagccggcac ggcaggcggc ctcctcctcc tctcacggca ccggcagcta cgggggattc 4620
   ctttcccacc gctccttcgc tttcccttcc tcgcccgccg taataaatag acaccccctc 4680
   cacaccctct ttccccaacc tcgtgttgtt cggagcgcac acacacacaa ccagatctcc 4740
   cccaaatcca cccgtcggca cctccgcttc aaggtacgcc gctcgtcctc cccccccccc 4800
   ctctctacct tctctagatc ggcgttccgg tccatgcatg gttagggccc ggtagttcta 4860
   cttctgttca tgtttgtgtt agatccgtgt ttgtgttaga tccgtgctgc tagcgttcgt 4920
   acacggatgc gacctgtacg tcagacacgt tctgattgct aacttgccag tgtttctctt 4980
   tggggaatcc tgggatggct ctagccgttc cgcagacggg atcgatttca tgattttttt 5040
   tgtttcgttg catagggttt ggtttgccct tttcctttat ttcaatatat gccgtgcact 5100
   tgtttgtcgg gtcatctttt catgcttttt tttgtcttgg ttgtgatgat gtggtctggt 5160
   tgggcggtcg ttctagatcg gagtagaatt ctgtttcaaa ctacctggtg gatttattaa 5220
   ttttggatct gtatgtgtgt gccatacata ttcatagtta cgaattgaag atgatggatg 5280
   gaaatatcga tctaggatag gtatacatgt tgatgcgggt tttactgatg catatacaga 5340
   gatgcttttt gttcgcttgg ttgtgatgat gtggtgtggt tgggcggtcg ttcattcgtt 5400
   ctagatcgga gtagaatact gtttcaaact acctggtgta tttattaatt ttggaactgt 5460
   atgtgtgtgt catacatctt catagttacg agtttaagat ggatggaaat atcgatctag 5520
   gataggtata catgttgatg tgggttttac tgatgcatat acatgatggc atatgcagca 5580
   tctattcata tgctctaacc ttgagtacct atctattata ataaacaagt atgttttata 5640
   attattttga tcttgatata cttggatgat ggcatatgca gcagctatat gtggattttt 5700
   ttagccctgc cttcatacgc tatttatttg cttggtactg tttcttttgt cgatgctcac 5760
   cctgttgttt ggtgttactt ctgcaggtcg actttaactt agcctaggat ccatgcaaaa 5820
   actcattaac tcagtgcaaa actatgcctg gggcagcaaa acggcgttga ctgaacttta 5880
   tggtatggaa aatccgtcca gccagccgat ggccgagctg tggatgggcg cacatccgaa 5940
   aagcagttca cgagtgcaga atgccgccgg agatatcgtt tcactgcgtg atgtgattga 6000
   gagtgataaa tcgactctgc tcggagaggc cgttgccaaa cgctttggcg aactgccttt 6060
   cctgttcaaa gtattatgcg cagcacagcc actctccatt caggttcatc caaacaaaca 6120
   caattctgaa atcggttttg ccaaagaaaa tgccgcaggt atcccgatgg atgccgccga 6180
   gcgtaactat aaagatccta accacaagcc ggagctggtt tttgcgctga cgcctttcct 6240
   tgcgatgaac gcgtttcgtg aattttccga gattgtctcc ctactccagc cggtcgcagg 6300
   tgcacatccg gcgattgctc actttttaca acagcctgat gccgaacgtt taagcgaact 6360
   gttcgccagc ctgttgaata tgcagggtga agaaaaatcc cgcgcgctgg cgattttaaa 6420
   atcggccctc gatagccagc agggtgaacc gtggcaaacg attcgtttaa tttctgaatt 6480
   ttacccggaa gacagcggtc tgttctcccc gctattgctg aatgtggtga aattgaaccc 6540
   tggcgaagcg atgttcctgt tcgctgaaac accgcacgct tacctgcaag gcgtggcgct 6600
   ggaagtgatg gcaaactccg ataacgtgct gcgtgcgggt ctgacgccta aatacattga 6660
   tattccggaa ctggttgcca atgtgaaatt cgaagccaaa ccggctaacc agttgttgac 6720
   ccagccggtg aaacaaggtg cagaactgga cttcccgatt ccagtggatg attttgcctt 6780
   ctcgctgcat gaccttagtg ataaagaaac caccattagc cagcagagtg ccgccatttt 6840
   gttctgcgtc gaaggcgatg caacgttgtg gaaaggttct cagcagttac agcttaaacc 6900
   gggtgaatca gcgtttattg ccgccaacga atcaccggtg actgtcaaag gccacggccg 6960
   tttagcgcgc gtttacaaca agctgtaaat cactagcgaa cgcgtaggta ccacatggtt 7020
   aacctagact tgtccatctt ctggattggc caacttaatt aatgtatgaa ataaaaggat 7080
   gcacacatag tgacatgcta atcactataa tgtgggcatc aaagttgtgt gttatgtgta 7140
   attactagtt atctgaataa aagagaaaga gatcatccat atttcttatc ctaaatgaat 7200
   gtcacgtgtc tttataattc tttgatgaac cagatgcatt tcattaacca aatccatata 7260
   catataaata ttaatcatat ataattaata tcaattgggt tagcaaaaca aatctagtct 7320
   aggtgtgttt tgcgaatgcg gccgccaccg cggtggagct cgaattccgg tccgggtcac 7380
   ccggtccggg cctagaaggc cagcttcggc cgccccgggc aactttatta tacaaagttg 7440
   atagatatct ggtctaacta actagtccta aggacccggc ggaccgatta aactgattcg 7500
   gtccgaagct taagccatgg cccgggaatc ttagcggccg cctgcagagt taacggcgcg 7560
   ccgactagct agctaaggta ccgagctcga attcattccg attaatcgtg gcctcttgct 7620
   cttcaggatg aagagctatg tttaaacgtg caagcgctac tagacaattc agtacattaa 7680
   aaacgtccgc aatgtgttat taagttgtct aagcgtcaat ttgtttacac cacaatatat 7740
   cct 7743
<210> 29
   <211> 6496
   <212> DNA
   <213> Artificial sequence
<220>
   <223> Synthetic construct
<400> 29
   gtgcagcgtg acccggtcgt gcccctctct agagataatg agcattgcat gtctaagtta      60
   taaaaaatta ccacatattt tttttgtcac acttgtttga agtgcagttt atctatcttt     120
   atacatatat ttaaacttta ctctacgaat aatataatct atagtactac aataatatca     180
   gtgttttaga gaatcatata aatgaacagt tagacatggt ctaaaggaca attgagtatt     240
   ttgacaacag gactctacag ttttatcttt ttagtgtgca tgtgttctcc tttttttttg     300
   caaatagctt cacctatata atacttcatc cattttatta gtacatccat ttagggttta     360
   gggttaatgg tttttataga ctaatttttt tagtacatct attttattct attttagcct 420
   ctaaattaag aaaactaaaa ctctatttta gtttttttat ttaataattt agatataaaa 480
   tagaataaaa taaagtgact aaaaattaaa caaataccct ttaagaaatt aaaaaaacta 540
   aggaaacatt tttcttgttt cgagtagata atgccagcct gttaaacgcc gtcgacgagt 600
   ctaacggaca ccaaccagcg aaccagcagc gtcgcgtcgg gccaagcgaa gcagacggca 660
   cggcatctct gtcgctgcct ctggacccct ctcgagagtt ccgctccacc gttggacttg 720
   ctccgctgtc ggcatccaga aattgcgtgg cggagcggca gacgtgagcc ggcacggcag 780
   gcggcctcct cctcctctca cggcaccggc agctacgggg gattcctttc ccaccgctcc 840
   ttcgctttcc cttcctcgcc cgccgtaata aatagacacc ccctccacac cctctttccc 900
   caacctcgtg ttgttcggag cgcacacaca cacaaccaga tctcccccaa atccacccgt 960
   cggcacctcc gcttcaaggt acgccgctcg tcctcccccc cccccctctc taccttctct 1020
   agatcggcgt tccggtccat gcatggttag ggcccggtag ttctacttct gttcatgttt 1080
   gtgttagatc cgtgtttgtg ttagatccgt gctgctagcg ttcgtacacg gatgcgacct 1140
   gtacgtcaga cacgttctga ttgctaactt gccagtgttt ctctttgggg aatcctggga 1200
   tggctctagc cgttccgcag acgggatcga tttcatgatt ttttttgttt cgttgcatag 1260
   ggtttggttt gcccttttcc tttatttcaa tatatgccgt gcacttgttt gtcgggtcat 1320
   cttttcatgc ttttttttgt cttggttgtg atgatgtggt ctggttgggc ggtcgttcta 1380
   gatcggagta gaattctgtt tcaaactacc tggtggattt attaattttg gatctgtatg 1440
   tgtgtgccat acatattcat agttacgaat tgaagatgat ggatggaaat atcgatctag 1500
   gataggtata catgttgatg cgggttttac tgatgcatat acagagatgc tttttgttcg 1560
   cttggttgtg atgatgtggt gtggttgggc ggtcgttcat tcgttctaga tcggagtaga 1620
   atactgtttc aaactacctg gtgtatttat taattttgga actgtatgtg tgtgtcatac 1680
   atcttcatag ttacgagttt aagatggatg gaaatatcga tctaggatag gtatacatgt 1740
   tgatgtgggt tttactgatg catatacatg atggcatatg cagcatctat tcatatgctc 1800
   taaccttgag tacctatcta ttataataaa caagtatgtt ttataattat tttgatcttg 1860
   atatacttgg atgatggcat atgcagcagc tatatgtgga tttttttagc cctgccttca 1920
   tacgctattt atttgcttgg tactgtttct tttgtcgatg ctcaccctgt tgtttggtgt 1980
   tacttctgca ggtcgacttt aacttagcct agcgaagttc ctattccgaa gttcctattc 2040
   tctagaaagt ataggaactt cagatccacc ggctagagga tccaccatgg ttgaacaaga 2100
   tggattgcac gcaggttctc cggccgcttg ggtggagagg ctattcggct atgactgggc 2160
   acaacagaca atcggctgct ctgatgccgc cgtgttccgg ctgtcagcgc aggggcgccc 2220
   ggttcttttt gtcaagaccg acctgtccgg tgccctgaat gaactgcagg acgaggcagc 2280
   gcggctatcg tggctggcca cgacgggcgt tccttgcgca gctgtgctcg acgttgtcac 2340
   tgaagcggga agggactggc tgctattggg cgaagtgccg gggcaggatc tcctgtcatc 2400
   tcaccttgct cctgccgaga aagtatccat catggctgat gcaatgcggc ggctgcatac 2460
   gcttgatccg gctacctgcc cattcgacca ccaagcgaaa catcgcatcg agcgagcacg 2520
   tactcggatg gaagccggtc ttgtcgatca ggatgatctg gacgaagagc atcaggggct 2580
   cgcgccagcc gaactgttcg ccaggctcaa ggcgcgcatg cccgacggcg atgatctcgt 2640
   cgtgacccat ggcgatgcct gcttgccgaa tatcatggtg gaaaatggcc gcttttctgg 2700
   attcatcgac tgtggccggc tgggtgtggc ggaccgctat caggacatag cgttggctac 2760
   ccgtgatatt gctgaagagc ttggcggcga atgggctgac cgcttcctcg tgctttacgg 2820
   tatcgccgct cccgattcgc agcgcatcgc cttctatcgc cttcttgacg agttcttctg 2880
   aggatccacc atggttaacc tagacttgtc catcttctgg attggccaac ttaattaatg 2940
   tatgaaataa aaggatgcac acatagtgac atgctaatca ctataatgtg ggcatcaaag 3000
   ttgtgtgtta tgtgtaatta ctagttatct gaataaaaga gaaagagatc atccatattt 3060
   cttatcctaa atgaatgtca cgtgtcttta taattctttg atgaaccaga tgcatttcat 3120
   taaccaaatc catatacata taaatattaa tcatatataa ttaatatcaa ttgggttagc 3180
   aaaacaaatc tagtctaggt gtgttttgcg aattgcggcc gggtaccgag ctcgaattcg 3240
   gcccaagttt gtacaaaaaa gcaggctccg gccagaatgg cccggaccga agcttgcatg 3300
   cctgcagtgc agcgtgaccc ggtcgtgccc ctctctagag ataatgagca ttgcatgtct 3360
   aagttataaa aaattaccac atattttttt tgtcacactt gtttgaagtg cagtttatct 3420
   atctttatac atatatttaa actttactct acgaataata taatctatag tactacaata 3480
   atatcagtgt tttagagaat catataaatg aacagttaga catggtctaa aggacaattg 3540
   agtattttga caacaggact ctacagtttt atctttttag tgtgcatgtg ttctcctttt 3600
   tttttgcaaa tagcttcacc tatataatac ttcatccatt ttattagtac atccatttag 3660
   ggtttagggt taatggtttt tatagactaa tttttttagt acatctattt tattctattt 3720
   tagcctctaa attaagaaaa ctaaaactct attttagttt ttttatttaa taatttagat 3780
   ataaaataga ataaaataaa gtgactaaaa attaaacaaa taccctttaa gaaattaaaa 3840
   aaactaagga aacatttttc ttgtttcgag tagataatgc cagcctgtta aacgccgtcg 3900
   acgagtctaa cggacaccaa ccagcgaacc agcagcgtcg cgtcgggcca agcgaagcag 3960
   acggcacggc atctctgtcg ctgcctctgg acccctctcg agagttccgc tccaccgttg 4020
   gacttgctcc gctgtcggca tccagaaatt gcgtggcgga gcggcagacg tgagccggca 4080
   cggcaggcgg cctcctcctc ctctcacggc accggcagct acgggggatt cctttcccac 4140
   cgctccttcg ctttcccttc ctcgcccgcc gtaataaata gacaccccct ccacaccctc 4200
   tttccccaac ctcgtgttgt tcggagcgca cacacacaca accagatctc ccccaaatcc 4260
   acccgtcggc acctccgctt caaggtacgc cgctcgtcct cccccccccc cctctctacc 4320
   ttctctagat cggcgttccg gtccatgcat ggttagggcc cggtagttct acttctgttc 4380
   atgtttgtgt tagatccgtg tttgtgttag atccgtgctg ctagcgttcg tacacggatg 4440
   cgacctgtac gtcagacacg ttctgattgc taacttgcca gtgtttctct ttggggaatc 4500
   ctgggatggc tctagccgtt ccgcagacgg gatcgatttc atgatttttt ttgtttcgtt 4560
   gcatagggtt tggtttgccc ttttccttta tttcaatata tgccgtgcac ttgtttgtcg 4620
   ggtcatcttt tcatgctttt ttttgtcttg gttgtgatga tgtggtctgg ttgggcggtc 4680
   gttctagatc ggagtagaat tctgtttcaa actacctggt ggatttatta attttggatc 4740
   tgtatgtgtg tgccatacat attcatagtt acgaattgaa gatgatggat ggaaatatcg 4800
   atctaggata ggtatacatg ttgatgcggg ttttactgat gcatatacag agatgctttt 4860
   tgttcgcttg gttgtgatga tgtggtgtgg ttgggcggtc gttcattcgt tctagatcgg 4920
   agtagaatac tgtttcaaac tacctggtgt atttattaat tttggaactg tatgtgtgtg 4980
   tcatacatct tcatagttac gagtttaaga tggatggaaa tatcgatcta ggataggtat 5040
   acatgttgat gtgggtttta ctgatgcata tacatgatgg catatgcagc atctattcat 5100
   atgctctaac cttgagtacc tatctattat aataaacaag tatgttttat aattattttg 5160
   atcttgatat acttggatga tggcatatgc agcagctata tgtggatttt tttagccctg 5220
   ccttcatacg ctatttattt gcttggtact gtttcttttg tcgatgctca ccctgttgtt 5280
   tggtgttact tctgcaggtc gactctagag gatccaccgg tcgccaccat ggccctgtcc 5340
   aacaagttca tcggcgacga catgaagatg acctaccaca tggacggctg cgtgaacggc 5400
   cactacttca ccgtgaaggg cgagggcagc ggcaagccct acgagggcac ccagacctcc 5460
   accttcaagg tgaccatggc caacggcggc cccctggcct tctccttcga catcctgtcc 5520
   accgtgttca tgtacggcaa ccgctgcttc accgcctacc ccaccagcat gcccgactac 5580
   ttcaagcagg ccttccccga cggcatgtcc tacgagagaa ccttcaccta cgaggacggc 5640
   ggcgtggcca ccgccagctg ggagatcagc ctgaagggca actgcttcga gcacaagtcc 5700
   accttccacg gcgtgaactt ccccgccgac ggccccgtga tggccaagaa gaccaccggc 5760
   tgggacccct ccttcgagaa gatgaccgtg tgcgacggca tcttgaaggg cgacgtgacc 5820
   gccttcctga tgctgcaggg cggcggcaac tacagatgcc agttccacac ctcctacaag 5880
   accaagaagc ccgtgaccat gccccccaac cacgtggtgg agcaccgcat cgccagaacc 5940
   gacctggaca agggcggcaa cagcgtgcag ctgaccgagc acgccgtggc ccacatcacc 6000
   tccgtggtgc ccttctgaag cggccgcaac ctagacttgt ccatcttctg gattggccaa 6060
   cttaattaat gtatgaaata aaaggatgca cacatagtga catgctaatc actataatgt 6120
   gggcatcaaa gttgtgtgtt atgtgtaatt actagttatc tgaataaaag agaaagagat 6180
   catccatatt tcttatccta aatgaatgtc acgtgtcttt ataattcttt gatgaaccag 6240
   atgcatttca ttaaccaaat ccatatacat ataaatatta atcatatata attaatatca 6300
   attgggttag caaaacaaat ctagtctagg tgtgttttgc gaatgcggcc gccaccgcgg 6360
   tggagctcga attccggtcc gggcctagaa ggccgatctc ccgggcaccc agctttcttg 6420
   tacaaagtgg ccgttaacgg atcccggtga agttcctatt ccgaagttcc tattctccag 6480
   aaagtatagg aacttc 6496
<210> 30
   <211> 1991
   <212> DNA
   <213> Artificial sequence
<220>
   <223> Synthetic construct
<400> 30
   gtgcagcgtg acccggtcgt gcccctctct agagataatg agcattgcat gtctaagtta      60
   taaaaaatta ccacatattt tttttgtcac acttgtttga agtgcagttt atctatcttt     120
   atacatatat ttaaacttta ctctacgaat aatataatct atagtactac aataatatca     180
   gtgttttaga gaatcatata aatgaacagt tagacatggt ctaaaggaca attgagtatt     240
   ttgacaacag gactctacag ttttatcttt ttagtgtgca tgtgttctcc tttttttttg     300
   caaatagctt cacctatata atacttcatc cattttatta gtacatccat ttagggttta     360
   gggttaatgg tttttataga ctaatttttt tagtacatct attttattct attttagcct     420
   ctaaattaag aaaactaaaa ctctatttta gtttttttat ttaataattt agatataaaa     480
   tagaataaaa taaagtgact aaaaattaaa caaataccct ttaagaaatt aaaaaaacta     540
   aggaaacatt tttcttgttt cgagtagata atgccagcct gttaaacgcc gtcgacgagt     600
   ctaacggaca ccaaccagcg aaccagcagc gtcgcgtcgg gccaagcgaa gcagacggca     660
   cggcatctct gtcgctgcct ctggacccct ctcgagagtt ccgctccacc gttggacttg     720
   ctccgctgtc ggcatccaga aattgcgtgg cggagcggca gacgtgagcc ggcacggcag     780
   gcggcctcct cctcctctca cggcaccggc agctacgggg gattcctttc ccaccgctcc     840
   ttcgctttcc cttcctcgcc cgccgtaata aatagacacc ccctccacac cctctttccc     900
   caacctcgtg ttgttcggag cgcacacaca cacaaccaga tctcccccaa atccacccgt     960
   cggcacctcc gcttcaaggt acgccgctcg tcctcccccc cccccctctc taccttctct    1020
   agatcggcgt tccggtccat gcatggttag ggcccggtag ttctacttct gttcatgttt   1080
   gtgttagatc cgtgtttgtg ttagatccgt gctgctagcg ttcgtacacg gatgcgacct 1140
   gtacgtcaga cacgttctga ttgctaactt gccagtgttt ctctttgggg aatcctggga 1200
   tggctctagc cgttccgcag acgggatcga tttcatgatt ttttttgttt cgttgcatag 1260
   ggtttggttt gcccttttcc tttatttcaa tatatgccgt gcacttgttt gtcgggtcat 1320
   cttttcatgc ttttttttgt cttggttgtg atgatgtggt ctggttgggc ggtcgttcta 1380
   gatcggagta gaattctgtt tcaaactacc tggtggattt attaattttg gatctgtatg 1440
   tgtgtgccat acatattcat agttacgaat tgaagatgat ggatggaaat atcgatctag 1500
   gataggtata catgttgatg cgggttttac tgatgcatat acagagatgc tttttgttcg 1560
   cttggttgtg atgatgtggt gtggttgggc ggtcgttcat tcgttctaga tcggagtaga 1620
   atactgtttc aaactacctg gtgtatttat taattttgga actgtatgtg tgtgtcatac 1680
   atcttcatag ttacgagttt aagatggatg gaaatatcga tctaggatag gtatacatgt 1740
   tgatgtgggt tttactgatg catatacatg atggcatatg cagcatctat tcatatgctc 1800
   taaccttgag tacctatcta ttataataaa caagtatgtt ttataattat tttgatcttg 1860
   atatacttgg atgatggcat atgcagcagc tatatgtgga tttttttagc cctgccttca 1920
   tacgctattt atttgcttgg tactgtttct tttgtcgatg ctcaccctgt tgtttggtgt 1980
   tacttctgca g 1991
<210> 31
   <211> 685
   <212> DNA
   <213> Unknown
<220>
   <223> Discosoma sp.
<400> 31
   cgccaccatg gcctcctccg agaacgtcat caccgagttc atgcgcttca aggtgcgcat      60
   ggagggcacc gtgaacggcc acgagttcga gatcgagggc gagggcgagg gccgccccta     120
   cgagggccac aacaccgtga agctgaaggt gacgaagggc ggccccctgc ccttcgcctg      180
   ggacatcctg tccccccagt tccagtacgg ctccaaggtg tacgtgaagc accccgccga     240
   catccccgac tacaagaagc tgtccttccc cgagggcttc aagtgggagc gcgtgatgaa     300
   cttcgaggac ggcggcgtgg cgaccgtgac ccaggactcc tccctgcagg acggctgctt     360
   catctacaag gtgaagttca tcggcgtgaa cttcccctcc gacggccccg tgatgcagaa     420
   gaagaccatg ggctgggagg cctccaccga gcgcctgtac ccccgcgacg gcgtgctgaa     480
   gggcgagacc cacaaggccc tgaagctgaa ggacggcggc cactacctgg tggagttcaa     540
   gtccatctac atggccaaga agcccgtgca gctgcccggc tactactacg tggacgccaa     600
   gctggacatc acctcccaca acgaggacta caccatcgtg gagcagtacg agcgcaccga     660
   gggccgccac cacctgttcc tgtag                                          685
<210> 32
   <211> 48
   <212> DNA
   <213> Saccharomyces cerevisiae
<400> 32
   gaagttccta ttccgaagtt cctattctct agaaagtata ggaacttc 48
<210> 33
   <211> 48
   <212> DNA
   <213> Saccharomyces cerevisiae
<400> 33
   gaagttccta ttccgaagtt cctattctcc agaaagtata ggaacttc 48
<210> 34
   <211> 18676
   <212> DNA
   <213> Artificial sequence
<220>
   <223> Synthetic construct
<400> 34
<210> 35
   <211> 28390
   <212> DNA
   <213> Artificial sequence
<220>
   <223> Synthetic construct
<400> 35
   gacccgcgag gtttctcgct tcaattgaaa tcataaagaa gcaattgaaa attttcgagt 60
   aaccgaccct cccgataatc ttcaacataa aacaacgcac ttcttccaac gggagaggcg 120
   gtgttagttg cgagctaagg agataaggta tgcttaagag atcggggtcg ctttctcttg 180
   ccttgatggt ctccttctgt tcgtcgagcc ttgccacgcc actctcatct gctgagtttg 240
   accatgttgc tcgcaagtgt gccccatcag ttgcgacatc tacgcttgcg gcgatagcta 300
   aggtggagag tcgctttgat cctttagcga ttcatgacaa cacgaccggc gaaacgcttc 360
   actggcaaga tcacagccaa gcaacccaag tcgtcaggca ccgtctcgat gcacggcatt 420
   cgctggatgt tggcctcatg caaataaact ctcgaaattt ttctatgctc ggtctgacac 480
   ctgacggtgc gctccaggcg tgcacatcat tatctgccgc tgcaaacatg ctgaaaagtc 540
   gttatgcagg cggcgaaacg attgacgaga agcaatttgc gcttcgtcgg gcgatctccg 600
   cttacaacac cggtaatttc atcggcggtt ttgcaaacgg ctacgtgcga aaagttgaaa 660
   cagctgctca atcgctggtg cccgcgttaa tcgagcctcc aaaagacgat cacgaggcgc 720
   taaaatccga agagacgtgg gatgtttggg ggtcatatca gcgccgctcg caggaggatg 780
   gcgctggcgg tttaatcgct ccgccaccgc cacaccagga caacggcaaa tccgcagacg 840
   acaatcaagt cttattcgac ttatactaag gaggtgcgca ttgatgcgat gctttgagag 900
   ataccgttta catctaaatc gcctctcgct ctcgaatgcg atgatgcgcg tgatatcgag 960
   ctgcgcccca agcttgtgcg gtgcaattgc atggagcatt tcctcatccg gacccgccgc 1020
   agcgcaatct gcgggtggcg gcactgaccc cgccacaatg gttaacaata tatgcacgtt 1080
   tatccttggt ccgttcggcc agtcactcgc tgttctcggc attgtcgcta tcgggatctc 1140
   ctggatgttc gggcgggctt cgcttgggct ggttgccggc gtcgtcggcg gcattgttat 1200
   catgtttggg gcgagcttcc tcggccaaac gctcactggc ggtagttgat ggctgatcgt 1260
   ttggaagaat cgacccttta cctcgcagcc acacggcccg cattgtttct tggggtgcca 1320
   ctgacattgg cagggttatt catgatgttc gccggctttg tcatcgttat cgttcagaac 1380
   ccgctctacg aagtcgttct cgtgccgtta tggtttgcag cccggctcat cgtggagcga 1440
   gactacaatg cggcgagcgt cgtcctgcta tttttgcgga ccgcgggaag aagcattgat 1500
   agtgcagttt gggggggcgc tactgttagc ccaaatccaa ttagggttcc cccacgaggg 1560
   agaggaatgg tgtgatgctc ggcgcgagtg gaacgaccga aagatccggt gagatctatc 1620
   tcccttatat tggccacctc agcgaccata tcgtccttct tgaagacgga tcgatcatga 1680
   ccattgcgag aattgatggc gttgcattcg agcttgagga aactgaaatg cgcaatgcgc 1740
   gttgtcgtgc gttcaacacg ctgttgcgca atatcgctga tgatcatgtg tcaatatatg 1800
   ctcacctcgt acgtcatgcc gacgtgccat catcggcgcc gcgacacttc cgtagtgttt 1860
   tcgccgctag cctgaacgaa gcttttgaac agcgcgtgct ctccggccaa ctcctccgca 1920
   atgaacactt ccttacgttg attgtctacc cacaggcggc tttagggaag gtaaagagga 1980
   ggttcaccaa gctaagcgga aaaagggaaa acgatctcac gggccagatc aggaacatgg 2040
   aagatctttg gcatgttgtc gctggctctc ttaaagcgta tggcctgcat cgtcttggca 2100
   tccgcgagaa gcagggtgtg ctcttcaccg aaattggcga agcgctacgg ttgatcatga 2160
   ctggtcggtt cacaccggtt ccggtcgtca gcggctcact cggcgcttcg atttataccg 2220
   acagagtcat ttgcggcaag cgaggactcg agatcagaac gccaaaagac agttacgttg 2280
   gatccatcta ttcgtttcgc gaataccctg caaaaacacg gccgggcatg ctcaacgcgc 2340
   tgctatccct cgattttcca cttgttctca cgcagagttt ttcgttcctg actcgcccgc 2400
   aagcgcacgc gaaacttagc ctcaaatcga gccagatgct gagttccggc gataaagccg 2460
   tgactcaaat cggcaaatta tccgaggctg aggacgcact tgcgagcaac gaattcgtta 2520
   tgggctcaca tcatttgagc ctttgcgtct atgcagacga tctcaatagt cttggggaca 2580
   ggggcgcgcg ggctcggaca cgaatggcgg atgcaggtgc cgtggttgtc caagaaggta 2640
   ttggtatgga agcggcctat tggtcccaat tgccggggaa ttttaagtgg cgcacacgcc 2700
   ctggcgcaat cacttcacgc aatttcgcag ggtttgtctc tttcgaaaac tttccagagg 2760
   gcgccagctc aggccactgg ggcaacgcga ttgcccgatt tcgtaccaat ggcggaacgc 2820
   ctttcgacta tatcccgcat gagcacgatg ttggcatgac ggcaatattc gggcctatcg 2880
   ggaggggtaa gacgacgctc atgatgtttg ttctagccat gctcgaacag agcatggtcg 2940
   accgtgcagg tacggtcgtg ttctttgaca aggaccgggg tggcgaattg ctggttcgcg 3000
   ccacaggagg aacatatttg gcacttcaca gaggcacacc cagcgggttg gcgccgttgc 3060
   gtggcctaga aaacacagca gcctcacacg attttctgcg cgaatggatc gtggctctca 3120
   tcgagagtga tggtcggggt gggatttctc cggaagagaa ccgccgtctg gtccgtggta 3180
   tccatcgtca gctctcgttt gatccacaaa tgcgttcaat cgcggggtta cgtgaatttt 3240
   tgttgcatgg gcccgccgaa ggcgcaggag cgcggctcca acgctggtgc cggggccatg 3300
   cgcttggctg ggcatttgac ggcgaagttg acgaagtaaa gttagatccg tcgattaccg 3360
   gcttcgacat gacgcatctt ctcgaatacg aggaagtatg cgctcccgct gcagcatatc 3420
   tcctgcatcg gattggagcc atgatcgacg gccgccgttt tgtgatgagc tgcgatgagt 3480
   ttcgcgccta tttgttaaac cctaaatttt cgactgtcgt cgacaaattc ctcctgaccg 3540
   ttcgaaaaaa caacgggatg ctaatactgg caacgcagca accagagcat gttctggaat 3600
   cgccgctagg agccagcttg gttgcgcaat gtatgacgaa gattttctat ccatcaccaa 3660
   ccgcagatcg atcggcttat gtcgatggac tgaaatgtac cgaaaaggaa tttcaggcga 3720
   tccgtgaaga catgacggtc ggcagccgta agtttcttct taaacgagaa agtggaagcg 3780
   tcatctgcga atttgatctg cgggatatgc gtgaatatgt cgccgtgctt tcggggcgtg 3840
   ccaacacggt gcgctttgca actcgactac gcgaggcaca agaaggcaac tcatctggct 3900
   ggctcagcga attcatggcc cgtcaccacg aggcagaaga ttgataaggt aggaaacgat 3960
   gaagacgacg caacttattg caacagtttt gacctgcagc tttctatata ttcagcccgc 4020
   gcgggcgcag tttgttgtta gcgacccggc aacggaggct gagacgctcg cgactgcgct 4080
   cgcgactgcg gagaatctca ctcagactat agcgatggtt acgatgttga cgtcggccta 4140
   cggcgttact ggactactga cttcgctcaa ccagaaaaat cagtatcctt cgacgaagga 4200
   cctagacaat gaaatgtttt cgccgcgaat gccaatgtcg accacggcac gtgcgatcac 4260
   cagcgataca gatcgtgcag tcgtgggtag tgatgctgaa gcggacctgt tgcgatcgca 4320
   gatcaccggt tccgcaaaca gcgctggcat tgcggctgac aatctggaaa cgatggacaa 4380
   acgcttgacg gcgaatgctg atacgtctgc tcagctttcc cgatctcgca atatcatgca 4440
   ggcaaccgtg accaatggtt tgcttctcaa gcagatccat gacgcaatga ttcaaaatgt 4500
   acaggcgaca agcctattaa cgatgactac cgcgcaggcc ggccttcacg aggcggaaga 4560
   ggcggccgct caacgcaagg agcatcaaaa gaccgctgtc atctttggtg ccctccccta 4620
   aggctgggcg atttgttcat ccgcccgcat cctcgccgaa tgcgagctca ttttatccaa 4680
   cattatgcga caaaccagtc aagttcaggt ccaatcgatg aatttcacga ttccggcgcc 4740
   gtttacggcc attcatacga tcttcgatgt agccttcacg acaggcttgg actcgatgct 4800
   tgagactatc caggaggcgg tgagtgcgcc attgatcgcc tgtgtcactc tttggattat 4860
   tgttcagggt attttagtca tacgcggcga agtcgatacc cgtagcggta tcactcgggt 4920
   gatcacggtc accatcgttg ttgctctaat tgttgggcag gctaactacc aagactatgt 4980
   ggtttccatc ttcgaaaaga cggtcccaaa ctttgttcag cagtttagtg taaccggctt 5040
   gcctctgcag actgttccgg cacagttgga tacaatgttc gccgtgaccc aggccgtttt 5100
   tcagaaaatc gcatccgaaa tcggtccgat gaacgaccag gacatccttg ctttccaagg 5160
   ggcacagtgg gtcctttacg gcacgctctg gtctgccttc ggagtttacg acgccgttgg 5220
   aattctcacg aaagtgcttc tcgcgatcgg gcctctgatc ctcgtcggat atatttttga 5280
   tcgcacgcgg gacatcgcag ctaagtggat cgggcaactt atcacctacg gtctcttgct 5340
   tctcctctta aacctcgtgg caacgatcgt catcctaacc gaagcgactg cgctcaccct 5400
   tatgcttggt gtaatcacct tcgccggtac gaccgcggcc aagatcattg gtctttacga 5460
   actcgatatg ttttttctga caggggatgc gctcattgtc gctttgccgg cgatcgccgg 5520
   caacattgga ggcagttact ggagcggcgc aacccaatct gccagcagct tgtaccgtcg 5580
   cttcgctcag gttgagcgag gctaggtcgc gcaaaaattc gcctcaatgg agaattctat 5640
   gaagtattgc ctgctgtgcc tagttgtcgc tttgagcggc tgccagacaa acgacacatt 5700
   agcgagctgc aaaggcccga tcttcccgct gaatgtgggg cgatggcagc ctactccgtc 5760
   agatcttcag ctcggcaatt cgggtggacg ctatgacggg gcctgaatat gccatgctag 5820
   tggcgcgcga aagccttgcc gagcactata aggaagtaga agcctttcaa accgcgcgag 5880
   cgaaatcggc gcgacgtctc tccaaactca ttgcagctgt cgcagctatc gcgattttgg 5940
   gaaatgttgc tcaagcgttc gctatagcca caatggtgcc gttgagcagg cttgtgcccg 6000
   tatatctatg gatacggccg gacggcaccg ttgacagcga ggtgtctgtc tcgcgattgc 6060
   ctgcaactca agaggaggcc gtcgttaacg cctcattgtg ggagtacgtt cgcctgcgcg 6120
   agagttatga tgccgacacc gctcagtacg cctacgacct ggtatcgaac ttcagtgccc 6180
   caacagtgcg ccaggattac cagcaattct tcaactatcc caatcccagt tcgcctcaag 6240
   tcattcttgg caaacgcggc agggtggagg tcgagcacat cgcttcaaat gatgtaactc 6300
   caagcacgca gcaaattcgc tataaaagga ccctcgtcgt tgacggcaaa atgcctgtgg 6360
   tgagtacgtg gaccgcgaca gttcgctacg aaaaggtgac cagcttgccc ggcagattga 6420
   gactaaccaa cccggcaggt ctggttgtca cctcctatca gacatcggaa gataccgttt 6480
   caaacgtagg ccacagcgaa ccatgatcag aaaagcactt ttcattttag catgtttatt 6540
   tgccgctgcg actggtgcgg aggctgaaga cactccaatg gcgggcaagc tagatccgcg 6600
   catgcgttat ttggcttaca atcccgatca agtggtgcgc ctctcgacgg cggttggagc 6660
   tactttggtc gtaacattcg ccacgaacga aacggtgaca gcggttgccg tttcaaatag 6720
   caaagatcta gcagccctac cgcggggaaa ttatctattt ttcaaggcaa gccaggtcct 6780
   cacgcctcag ccagtaatcg tgctaaccgc aagcgactcc gggatgcgcc gttatgtttt 6840
   cagtataagt tccaagactc tgtcccacct cgataaagag cagcccgatc tctattacag 6900
   cgtccaattc gcctaccccg ccgacgatgc ggcggctcgg cgaagggagg cacaacagaa 6960
   ggctgttgtg gacagactac acgcggaagc acaatatcaa cggaaagctg agaatttatt 7020
   ggatcagcct gtcacagccc ttggtgcggc ggacagtaat tggcactacg tcgcccaagg 7080
   cgatcgttcg ctgttgccac tcgaagtctt cgacaatgga tttacgacgg tattccactt 7140
   tccgggcaat gtacgcatac cctccatcta caccatcaat cctgatggca aggaagctgt 7200
   tgccaactat tcagttaaag ggagcgatgt cgagatttct tcggtttccc gaggttggcg 7260
   tctgagggat ggccacacag tactatgtat ctggaacacc gcttacgatc ccgttggcca 7320
   aaggccgcaa acgggcacgg tgaggcccga tgtgaaacgc gtcctgaagg gggcgaaggg 7380
   atgaataacg atagtcagca agcggcacat gaggttgatg catctggatc cctggtctcc 7440
   gacaaacatc gccggcgtct ttcggggtct cagaaattga tcgtcggagg tgtcgttctc 7500
   gcgttatcat taagcctcat ttggctaggt gggcgccaaa agaaggtgaa tgagaacgca 7560
   tcgccgtcaa ctttgatcgc aacaaacacc aagccatttc atccagctcc gattgaggtg 7620
   ccgccggatc ctccagcggt tcaagaggct gttcagcctg ctgctcctct accgccgagg 7680
   ggcgaaccgg agcggcatga gccacggccg gaagaaacac cgatttttgc atatagcagc 7740
   ggcgatcaag gggtcagcaa acgcgccatt cagggcgaca cgggccgaag acaagaaggc 7800
   aagcgtgacg acaactcctt gccgaatggc gaagtgtccg gcgagaacga tttgtcgata 7860
   cgtatgaaac ccaccgagct gcagcccagc agcgccacgc tcttgccgca ccccgatttt 7920
   atggtaacgc aagggacaat aattccgtgc atcttgcaaa ccgcaatcga cacaaatttg 7980
   gcaggctatg taaagtgtgt cttgcctcag gatattcgtg gaacaacgaa caatatcgtg 8040
   cttcttgatc gtggcaccac cgttgttggc gaaatacagc gtggcttgca acagggagat 8100
   gggcgcgttt ttgtgttgtg ggatcgcgcc gagacacctg accatgcgat gatctcgtta 8160
   acatcgccaa gcgcggacga actcggtcgc tcaggattgc cgggctcggt cgacagccac 8220
   ttctggcagc gttttagcgg agctatgctc ttgagtgttg ttcaaggcgc cttccaggca 8280
   gctagcacct acgccggcag ctcgggtggc gggatgagct tcaacagctt tcaaaataac 8340
   ggtgagcaga caactgagac agcccttaag gcaaccatca acataccgcc aaccctgaag 8400
   aagaatcagg gtgacaccgt ttccattttc gtagcacggg acctcgattt ctttggtgtt 8460
   taccagctcc gcctgactgg cggcgccacg cgggggagga accgccgctc ttaatgaatt 8520
   caaatttccg cttagagata ggatacattg taaatggaag tggatccgca actacgcttt 8580
   cttctgaagc cgattttgga atggctcgat gacccgaaga ctgaagaaat tgcgatcaat 8640
   cgacctggag aggcatttgt gcgccaagcc ggcattttta ccaagatgcc tttgcccgtc 8700
   tcttatgatg atcttgaaga tatcgctatt ttagcgggcg cgctgagaaa gcaggatgtc 8760
   ggaccacgta accccctctg cgccactgaa cttcctggtg gtgaacgact acaaatctgt 8820
   ctgccgccga ccgttccctc gggcaccgtc agcttgacca ttcgacggcc aagctcgcgt 8880
   gtttctggtc ttaaagaagt ctcctcccgt tatgatgctt cgaggtggaa ccagtggcag 8940
   acacgaagga aacgccaaaa tcaggatgat gaagctatcc ttcagcattt tgacaacggg 9000
   gatttggaag cgtttctgca cgcatgcgtc gtcagccgac tgacgatgtt gctatgtggc 9060
   cctaccggaa gcggcaagac aacaatgagc aagaccttga tcagcgccat ccccccccag 9120
   gaaaggctaa tcaccataga agatacgctc gaactcgtca ttccacacga taatcatgtt 9180
   agactactct actccaagaa cggtgctggg ctgggtgctg tgagcgccga gcacttgctc 9240
   caagcaagtc tgcgtatgcg gccggaccgg atattgcttg gcgagatgcg cgacgatgca 9300
   gcatgggctt atctgagtga agtcgtctcg ggacatccgg gatcgatttc aacaatacac 9360
   ggcgcgaatc caatccaagg attcaagaaa ctgttttccc ttgtcaaaag tagcgcccaa 9420
   ggtgctagct tggaagatcg cacactgatt gacatgctct ctacggcgat cgatgtcatc 9480
   attccattcc gtgcctatga ggacgtttat gaagtaggcg agatctggct cgcggcggac 9540
   gcacgacgcc ggggcgagac cataggcgat ctccttaatc aatagtagct gtaacctcga 9600
   agcgtttcac ttgtaacaac gattgagaac ttttgtcata aaattgaaat acttggttcg 9660
   cattttcgtc atccgcggtc agccgcaatt ctgacgaact gcccatttag ctggagatga 9720
   ttgtacatcc ttcacgtgaa aatttctcaa gcgctgtgaa caagggttca gattttagat 9780
   tgagaggtga gccgttgaaa cacgttcttc ttatcgatga cgatgtcgct atgcggcatc 9840
   ttattatcga ataccttacg atccacgcct tcaaagtgac cgcggtagcc gacagcaccc 9900
   agttcactag agtactctct tccgcgacgg tcgatgtcgt ggttgttgat ctaaatttag 9960
   gtcgtgaaga tgggcttgag atcgttcgaa atctggcggc aaagtctgat attccaatca 10020
   taattatcag tggcgaccgc cttgaggaga cggataaagt tgttgcactc gagctaggag 10080
   caagtgattt tatcgctaag ccgtttagta cgagagagtt tcttgcacgc attcgggttg 10140
   ccttgcgcgt gcgccccaac gttgtccgct ccaaagaccg acggtctttt tgttttactg 10200
   actggacact taatctcagg caacgtcgct tgatgtccga agctggcggt gaggtgaaac 10260
   ttacggcagg tgagttcaat cttctcctcg cgtttttaga gaaaccccgc gacgttctat 10320
   cgcgcgagca acttctcatt gccagtcgag tacgcgacga ggaggtttac gacaggagta 10380
   tagatgttct cattttgcgg ctgcgccgca aacttgaggc ggatccgtca agccctcaac 10440
   tgataaaaac agcaagaggt gccggttatt tctttgacgc ggacgtgcag gtttcgcacg 10500
   gggggacgat ggcagcctga gccaattgca tttgattaat ttaggtgact gaggacgcgg 10560
   ccagcggcct caaacctaca ctcaatattt ggtgaggggt tccgataggt ccctcttcac 10620
   caattgctcg atggcttctc tccagcaaag aatgacgcga gcgcggcggt agccagcttg 10680
   tggccgaaag ctcgagcggt ctccaacccc aacggatcaa aatgacttcg agcgacctcg 10740
   agcaacgcaa ccgggaacat gcgtgaggtc tgaacgagaa cggatttttc tgtagttgaa 10800
   gggatcggat aacttttcgg ggccacgcga aatgatccat ctgccagcat gctttcgaaa 10860
   tcgtccaacg cgcgccttaa aatcatttgt agcgacttcg agggactgta ttgccgaacg 10920
   aggttgtcat atgttttcga cacttgaggc gcgggcggtc gcgctgaaag aaaaacctgg 10980
   agctttttcg gggacggagg tggactaagg gcatccacag ttagcttaag ttgtcgatcg 11040
   ggactgtaaa tgtgatcggc gacgagaggc tcacgttgct ggtctttctc gtcggctttt 11100
   tcaggcaagt gctggaggtc cagcttctgg ggaacaagtg tcgggttggg atggtggatc 11160
   tcgggtcgag caccagcaag ccgccgtgct tcgccgaccg acaatgcggg cttgcgaatt 11220
   gccatcttca agcctccaag attttgctga tcagtttcga aatgaccacg acttcctcca 11280
   tcgcaatccg aagattcctc tctatgaggc gcatcgtcgg atcagttccc gtgtttagta 11340
   atgtaagatg caacatgccg cgttctttca tcgcggcaaa tgcatctctt tcatgcatgg 11400
   gagacggtac aactggaagg ctctctagcg tctctgacat cctgcgttgc gatgttgtca 11460
   atcggccgac cgggacgcgt tggcgcaaaa cagctgtagg aattgccaaa ttttcactca 11520
   acagcagctc gatgacgtag cggtaggtag atagtgcctc atcgatgtcg agcggcgtta 11580
   gcatggtggg gatcagaagc aggtttgagc tagcgatgat tgtgttgttg agctcgctcg 11640
   agccgccacg cgtatcggcc aacgcataat caaatccttc gagctcggca ttttcatagg 11700
   ctgcttcaag aaggggcatt tcgtcggcgg aatagacttc acagcgagga tcccaggtac 11760
   tgctttgtaa ggcgttttct ctccatcgcg tcagaggccg gttttcgtcg gcatcaaaga 11820
   gggccactcg tttaccgtca tttgccaaag cagcgcaaag gcccatgagt gcggtggttt 11880
   tgccagcacc ccctttgaaa gaacaaaacg tcaaaagttg catattctga tcccgcctat 11940
   cctgtgaaac cggagtgcat ttgtattttt gttcgtataa atgtttttgt gattatcgat 12000
   gagtaaaagc gttgttacac tatttttatt tcacattcgt tataagacaa ttgcaaatgt 12060
   agcaagtata ttcagtattg actgtaaatg tactgttgat ttcatattga gcagggctag 12120
   acttccatcc gtctacccgg gcacatttcg tgctggagta tccagacctt ccgctttctt 12180
   tggaggaagc tatgtcaaaa cacaccagag ccacgtcgag tgagactacc atcaaccagc 12240
   atcgatccct gaaagttgaa gggttcaagg tcgtgagtgc ccgtctgcga tcggccgagt 12300
   atgaaacctt ttcctatcaa gcgcgcctgc tgggactttc ggatagtatg gcaattcgcg 12360
   ttgcggtgcg ccgcatcggg ggctttctcg aaatagatgc agacacacga gaaaagatgg 12420
   aagccatact tcagtccatc ggaatactct caagcaatgt atccatgctt ctatctgcct 12480
   acgccgaaga ccctcgatcg gatctggagg ctgtgcgaga tgaacgtatt gcttttggcg 12540
   aggctttcgc cgccctcgat ggactactgc gctccatttt gtccgtatcc cggcgacgga 12600
   tcgacggtcg ctcgttactg aaaggtgcct tgtagcactt gaccacgcac ctgacgggag 12660
   aaaattggat gcccgatcgc gctcaagtaa tcattcgcat tgtgccagga ggtggaacca 12720
   agacccttca gcagataatc aatcagctgg agtacctgtc ccgaaaggga aagctggaac 12780
   tgcagcgttc agcccggcat ctcgatattc ccgttccgcc ggatcaaatc cgtgagcttg 12840
   cccaaagctg ggttacggag gccgggattt atgacgaaag tcagtcagac gatgacaggc 12900
   aacaagactt aacaacacac attattgtaa gcttccccgc aggtaccgac caaaccgcag 12960
   cttatgaagc aagccgggaa tgggcagccg agatgtttgg gtcaggatac gggggtggcc 13020
   gctataacta tctgacagcc taccacgtcg accgcgatca tccacattta catgtcgtgg 13080
   tcaatcgtcg ggaacttctg gggcaggggt ggctgaaaat atccaggcgc catccccagc 13140
   tgaattatga cggcttacgg aaaaagatgg cagagatttc acttcgtcac ggcatagtcc 13200
   tggatgcgac ttcgcgagca gaaaggggaa tagcagagcg accaatcaca tatgctgaat 13260
   atcgacgcct tgagcggatg caggctcaaa agattcaatt cgaagataca gattttgatg 13320
   agacctcgcc tgaggaagat cgtcgggacc tcagtcaatc gttcgatcca tttcgatcgg 13380
   acgcatctgc cggcgaaccg gaccgtgcaa cccgacatga caaacaaccg cttgaaccgc 13440
   acgcccgttt ccaggagccc gccggctcca gcatcaaagc cgacgcacgg atccgcgtac 13500
   cattggagag cgagcggggt gcccaaccat ccgcgtccaa aatccctgta actgggcatt 13560
   tcgggattga gacttcgtat gtcgctgaag ccagcgtgcc caaacaaagc ggcaattccg 13620
   atacttctcg cccggtgact gacgttgcca tgcacacagt cgagcgccag cagcgatcaa 13680
   aacgacgtca tgacgaggag gcaggtccga gcggagcaaa ccgtaaaaga ttgaaggccg 13740
   cgcaagttga ttccgaggca aatgtcggtg agcccgacgg tcgcgatgac agcaacaagg 13800
   cggctgatcc ggtgtctgct tccatccgta ccgagcaacc ggaagcttct ccaacgtgtc 13860
   cgcgtgaccg tcacgatgga gaattgggag aacgcaaacg tgcaagaggt aatcgtcgcg 13920
   acgatgggcg cggggggacc tagactagta gacaggaagg accgaataat ggcaaatggt 13980
   cagttcacga tacgctctgc tcgcccggcc tccgtcggac tgacaggcga acggcgtgga 14040
   gccgcatccg cctctagctc tgcactgtcc aatgttcaaa gagatgttag ggataggctg 14100
   attccaacta gctcaccaag attaccaaat gcagccatat tgcgtgattc ctcgggaaga 14160
   gcgtcgactg gtctgcggta catggcggct actcttcatt ggtctgcgat cgcgccatta 14220
   tcgctaataa acagcaacga cctggctccg gccgcttatg actttgagac gcgaaataac 14280
   gcaagaaatg tgactgccaa agtcggcagg gcagtccctg ttcccaagca aggcgggctc 14340
   ggcaaaacgc tcgcacccgt accccttagt acacgtatat caagggtcaa ttccgaccga 14400
   agactgcccg ctgacgcaga agaccgccct gaaacgcgcg acccccagaa aggacgtggc 14460
   agtcatggtg cgacgccaac cttacatgaa aagattggaa ccgcgtttgc tcgaagattg 14520
   cgaaagcata cgtactatat tgtttgcagt tgctgccaga cccggagcgc gttgacgatg 14580
   ggtgcaaaga tttcggtgaa gtcatgaact ccagcaagac ttcgccccag cgtatgaccc 14640
   tgagcatcgt atgttcgctg gcagccggtt tttgtgcggc cagctgctat gtaacgttcc 14700
   gccggggctt caacggcgaa gcgatgatga cgttcgacgt tttcgctttt tggtatgaga 14760
   ccccgcttta cttgggttat gccagcaccg tcttctggcg tggtttatct gttgtcatct 14820
   ttacctcgct gatcgttctt tcaagtcagc tcatcatatc gctgcgcaat cagaagcatc 14880
   atgggacagc tcgttgggca gaaattggcg aaatgcggca tgctggttat ctgcagcgtt 14940
   acagtcgcat caaggggccg atctttggaa agacatgtgg tcccctttgg ttcggcagtt 15000
   atttgaccaa tggcgaacag ccacacagtc ttgtcgtcgc gccaacgcgt gctggcaaag 15060
   gcgtcggcat cgtcattcca acgctgttga ccttcaaggg ctcggtaatc gcccttgacg 15120
   tcaagggaga attgtttgaa ctgacgtcca gagcacgcaa agcgagcggc gacgcagttt 15180
   tcaagttctc ccccctagat cctgagcgga agactcattg ttacaatccg gtcctggata 15240
   ttgccgcact tccgcccgaa cgccagttca ctgaaacacg ccgtctagct gcgaacctta 15300
   ttacggctaa gggaaaggga gcagaaggct ttattgacgg cgcacgtgac ctgttcgtcg 15360
   cgggaatcct tacctgcatt gagcgtggca caccaacgat tggcgcggta tatgacctat 15420
   ttgcgcagcc tggcgaaaag tataagcttt ttgcgcaact cgcggaggaa agcctaaaca 15480
   aagaggctca gcgtatcttc gataatatgg cgggcaacga cacgaaaatt ctgacatcgt 15540
   acacctctgt gctgggcgac ggtggactga acctgtgggc tgatccgctt atcaaagcag 15600
   cgacaagccg gtcagacttt tccgtttacg atctccggag gaagaagacc tgcatttatc 15660
   tttgtgtcag tcccaacgat ctggaggtct tggcaccact tatgcgcctg atgtttcagc 15720
   agctcgtgtc aatcttgcag agatcgctgc caggtgaaga cgagtgccat gaagttttat 15780
   ttctcctcga cgaattcaaa cacctgggca agcttgaggc catagagacc gcgatcacaa 15840
   ccatcgccgg ttacagaggc cgctttatgt ttattattca aagtctttcg gccttgtcgg 15900
   gcacatacga tgacgcagga aaacaaaact ttctgagcaa tactggcgta caagtattta 15960
   tggccacggc tgatgacgaa actccaacct acatctcaaa agctatcggc gaatatacgt 16020
   ttaaagcgcg ttcgacctct tacagtcaag ccagaatgtt cgaccacaac atccagattt 16080
   ctgatcaagg tgcagccctt ttgcgccccg aacaagtgcg cctgctagac gatcagagtg 16140
   aaatcgttct catcaaaggg cgacctccac tcaaattacg aaaggtgcag tattattccg 16200
   atcgtacgct gaaaggcctt ttcgaacgcc agatgggctc tctgcctgag cccgcaccct 16260
   tgatgctttc cgactatagc aacgatcaag ttcaatacca cttggctccg atagcaaatt 16320
   ttaatgagga tgctgcaccg caaaacagaa ctgtggccga ggaccatgga agtgttaaag 16380
   tcggtgctga tatccctgaa cgcgtgatgg gaataaatgg tgacgaggaa caagccgatg 16440
   cgggcgagat accgccggaa tcggttgtgc ctccagaatt gacgctcgct ctgaccgctc 16500
   aacagcaatt gttggaccag attattgcac ttcagcaaag atcgaggtcc gcaccggcat 16560
   agcctgcgaa atgatcttaa tggtgcaatt cgtttcaggc ggcatgttgc ggttcaacaa 16620
   agtgtacgcc agatcccaat tggcccttta cgaggtgtcg gtatgacagg aaagtcgaaa 16680
   gttcacataa gaggttcggc tgacgcgctt cctgacgttc ctggcggaag tactaccgcc 16740
   ccttttttaa ccgaaccttc tcgggatcag gttgatgcct cgtttgaggt ccaaaccgac 16800
   tacagccagt ctacttccgt gtcgtttacc tatgatggtg ttggacttgg tcctgccgag 16860
   cgtgcggctt acgagaactg gtgcgaaccg ggccggccca cttggaaaga tcttataatc 16920
   aaggcacgtg tcgatccgat tgacgatgtg acctggctcc gagatttaga agaggacacc 16980
   ccctcaacct tcagatacga agggatgcct ctgggcatcg gggaacgaca ggcctacgaa 17040
   aattggcaag aggacgctca gccgacatgg gaagaccttg ttgtcagcgc acgcttgacg 17100
   gaacttggcc gtccacacgg gattaccggc gagtatacat ccctcgcagg atcgaagaat 17160
   acaagttcaa tttcattgaa gcgaaagcgg agcaacttaa ttgatgatga gaattcatcc 17220
   ggatcgtttt catatgacgg gatgaagctc ggggaagccg agcgttctgc atatggtgac 17280
   tgggccgagg cggagccacc cacgtggaaa gatttggtat tgagggcacg cgtttcctcg 17340
   atcaatgact ctgcttggct ttttgattca caaacatctt catcatcatt tgaatacaac 17400
   ggtgttccct tgggcgagcc ggaacggcag gctctcagac aatggcaagg agacgctcag 17460
   cctacctggg aagatctcgt tgttaacgcg cgtatggcag aactttgcca tgctggttgg 17520
   attgaaggtc aaaaaggttg ctttgaagag cgcggggagg ctctgcccgc gtcggaacgc 17580
   ggttcgcaac gccccattgg tcaacggaca gattcctccg attcttttgt gtatgatggc 17640
   acaaggctcg gagcacctga gcgaactgct tatgaacgct ggagtaagag ggaacgcccg 17700
   acttgggaag atctcatctt agatgcacac caggccagga ctgaaagtga cgctgttacg 17760
   acccaagcga ttggtcagtc gtcctcaccg gttttcttat atgaaggaaa gtcgctcgga 17820
   gacagggaac gaaaggctta cgaaaaatgg cggcagccag cccaaccgcg atggcaaaat 17880
   cttgtagtga acgctcgtct ggcagaaatc gatccctcag cctggattgc cgatgagcgc 17940
   gatccgcttg atgatagcga cgcgcttggt cgcccgtcgt acacaagctt gacggataga 18000
   tcagacgtcc ctttagacga tcaatcaatc tatcgtcgtt ccgacctagt aagggagcag 18060
   gtgccagaat cgtctcaaag gcaattcgca gcatgttcag aatctgaaac gaggcctgtg 18120
   caatggttta ctgcttctgg gtcagatgca aacaatacgg aaaatatcac cgccagcgat 18180
   cccgtcgatc gcacgggtgg agttaagcgg ctaggctcca aaagcgacag aaccgttaca 18240
   gcttctatcc atgacgtgaa ttccagcaca aggcgactgt tgcttaacga atttggatcg 18300
   gaggctccgc gcccttcgcc agaaaagact gttcgcttaa gaagcgacaa tattggcacc 18360
   tatgggagcc ggaaaaatga acgagcgcgg ctcgcgaccg aaaccggtgc gtatgagtcg 18420
   gagcatattt tcgggttcaa ggctgtccac gatactgcga gagcgacgaa agagggccgg 18480
   cgtctcgaaa ggcccatgcc cgcctacctt gaggataagg ggcttcatcg ccaacatatt 18540
   ggcaccggga gaggacggac caaacttgtc gggcgcggat ggccggatga cacaagctat 18600
   cgctcggatc aaagggcaac tctgtcggac cccgttgcgc gctcggaagg cgcgacggcc 18660
   tcaaatgggt atcaattgaa ccaattgggt tacgcgcacc aactcgctag cgatggcctg 18720
   caaagtgaat cgcccgatgg tgttgccttg ccaattcaag tggcaacaac gagctacaac 18780
   tatacagtga gccgcgatcc tgtccttgtt ccgccggata aaaacgaagc ccctcaattg 18840
   ctgcatcttg gtccccgtgg tcaaaccgaa gctgttcttg cccgcgaaac agcattgact 18900
   ggaaaatggc cgactctcga gcgtgagcag caagtgtatc gcgagttttt ggccttatat 18960
   gacgtaaaaa aagatcttga ggccaaatca gtcggcgtaa gacggaaaaa aaaagaagtt 19020
   atttctgcgt tagaccgaac tgcgcgcttg ataagcacgt cgccttcgaa agctcgatcc 19080
   aaagcagaga ctgaaaaagc cattgatgag ctcgatgatc gacgagttta tgatccgcgt 19140
   gatcgagctc aagacaaagc gtttaaacgc tgataagtcg ccaatatagt gatcatttgc 19200
   agtattcgca tcgatcgctg gttgatattc tgccgctggt cgaccggctg ctcgtcgcca 19260
   aaaatgctca cagggatacg atggcctcgg tcaggccgcg tgcgtcctgt ctttccagtt 19320
   cctccctttc agctcgattg tggcatcatt tattgcctgc tcattgcagt tgaaacgcga 19380
   tatccgtttc aagacccggg tatggatggt actttggaga tatgagcact tgccagactt 19440
   cccggtcgga gacttaaaaa ttccgataag aggttacgga cgacggacct tataagtgga 19500
   tcgtctagtg gtggcgccga tcaaaacagt tccgcccccg gctttgctca aagtagcaaa 19560
   gcagctttat ctcgggttgc ggaggatttt ctagaaaacc gcaattttgc aggagagaac 19620
   atatggccat catcaagccg catgtgaaca aaaataggac aacctcgccg atagagagac 19680
   cggagtctct catagaggaa atgagcggca gtcatccgcc gagtggtttt accaacctgg 19740
   atctcgctat gatcgagctg gaggactttg tccatcggtg cccgctccca gaagacaatc 19800
   ttgctggtca gaaggagtga gacgatggat ccgtctagca atgagaatgt ctatgtgggt 19860
   cgcggtcaca acatcgaaaa tgatgatgac actgacccca ggcgttggaa gaaggcgaat 19920
   atcagttcca acaccatctc cgatattcag atgacgaatg gcgaagacgt acaatcaggg 19980
   agccctaccc gaacggaagt tgtaagccca cgtctggatt atggatcggt cgactcctcc 20040
   tccagccttt attctggcag cgagcacgga aatcaagctg agattcaaaa agagctgtcc 20100
   gtcttgttct cgaacatgtc tttgccaggc aacgatcggc gcccggacga atacattctc 20160
   gtgcatcaaa cgggacaaga tgcttttact ggtattgcca aaggcaacct cgaccaaatg 20220
   cccaccaagg cggaatttaa cgcgtgctgc cgtctctaca gggacggagc cggtaattac 20280
   tacccgccac ctctcgcatt cgacaagatt agcgttccag agcaactgga ggaaaaatgg 20340
   gggatgatgg aggcgaagga acgtaacaaa ctgcggtttc agtacaagtt ggacgtatgg 20400
   aatcatgcgc acgctgatat ggggatcacg ggcacagaga tcttttatca aacagataag 20460
   aacataaagc tcgaccggaa ttataaacta agacctgaag accgatacgt acaaacagaa 20520
   aaatacgggc gccgggaaat tcaaaagcga tatcaacacg aactccaggc tggttcgctg 20580
   ctgcccgata ttatgatcaa aactccccaa aatgacatcc acttcgtgta caggtttgcc 20640
   ggcgacaatt acgccaacaa acagttcagc gagtttgaac acaccgtcaa gcgcaggtat 20700
   ggcgacgaga ctgagatcaa attgaagtca aagtcaggca ttatgcatga ctcgaaatat 20760
   ctggaatcct gggaacgggg cagtgcggat attcgcttcg cggaattcgt tggggaaaat 20820
   agagctcaca atcggcagtt tccaactgcg acagtaaata tgggacagca gccagacggg 20880
   cagggcggtt tgacccgcga ccgtcatgtg agcgttgact tcctaatgca aagcgcaccc 20940
   aattcgcctt gggcgcaagc tttgaaaaag ggagaactgt gggatcgcgt tcagttgctt 21000
   gctcgcgacg gcaaccgcta tctgtcgccg cccagattgg aatattctga ccctgcacat 21060
   ttcaccgagt tgatgaaccg ggttggttta cccgcatcga tgggtcggca aagccatgcg 21120
   gctagtatca aattcgaaaa gtttgacgcg caggcagcgg ttattgtctt aaatggccca 21180
   gagttacgtg acattcatga cttgtctcct gaaaaactgc aaaatttgtc caccaaagat 21240
   gtcatcgtcg ccgatcgcaa tgagaatggt cagagaactg gcacgtacac cagcgtcgcg 21300
   gaatatgagc gcttgcagtt aaggctgcca cccgatgcag cgggggtgct tggtgaagca 21360
   actgacaaat attcacgtga tttcgttcgg ccagagccgg cgtcgcgtcc aatcagtgac 21420
   agccgcagga tatacgaaag tcgaccgcgt agccaaagcg tcaacagctt ttgacgttcc 21480
   tgctgccgcg tcaacgagga agctcgtttg acccgggttt gccaatgaaa gggctcaatc 21540
   atggtgaaca ctacaaagaa aagttttgcg aagtcgctta cggcagatat gcgccgttct 21600
   gctcagcgcg ttgtcgagca aatgcgaaaa gcattgatta ccgaagaaga ggcgctcaag 21660
   cggcaagcca gactggagag tcccgatagg aagcgaaagt atgctgctga tatggcgata 21720
   gtcgacaaac tcgacgtagg gtttcgaggc gaaataggct ataaaattct tggaaataac 21780
   cggcttcgag tagacaacca taaagaatta acgcgtgagc acggtagact tcgcaaaacc 21840
   aaaacggttc tgaagcgtaa cccggtgacg caggaagtct atttgggttt atatgaaagg 21900
   aagtcctggt taagtgtcag cagccatttg tatgctgcgg acggcacact ccgcatgaag 21960
   cacgtgaaat acaaagacgg acgttttgag gaaaaatggg agcgcgacga aaatggcgac 22020
   ctgatccgca caaggtacgc caaccgtggc aggctctttc aacctgtatc cgagaaaatg 22080
   ggcgcgccgt atcggagcgg ccctgacgac cggctctatc gcgatctaac ccgtcgaaac 22140
   ggtttcagac gggagacatt cgaacgggac gatcacggaa acctcgagcg tatcggcagc 22200
   aaccatgtcg gcttttccaa gatttcagtg aaggcaccca atcgtcaaac ctcccagacg 22260
   aagattcaaa aacttggtgg cgctttcaac aaatctttta ggtcccttct ggacaaggag 22320
   ggcaatgaaa tgggccgcga tattttgagc catcgacggc tctataacaa gcggtctgct 22380
   gtctacgatg aagctaccgg acaattgaag agtgccaagc ataccttcgg caagatctac 22440
   aggagcgaaa ccgaatatct cagcgcgggc ctcaagaagg tttcaaaaaa gatactcggg 22500
   gtgacggtct accggaaatt tgcggcgctc agcgagcgag aatccgaggc tgagagactg 22560
   cgtagttttg aatccggtgc gcatcgccag atctggcagg agcgggcagc gattcccggt 22620
   tcgcccctcc cggagactga tgacattcat ttcgcacagc agtcgcacct agccaaagcc 22680
   aaccctgatc acgtcgaagc tgacgtcacg cgtgtgacag atcaacatgt tgatgttgct 22740
   ggacaaacat catcgtctcc ccaacggaac ttggaaggat ggttagattc tcaatcacga 22800
   tacaagccag caaacatgct gttgtcaaat ccagaccttc aagcgaacgg acctcgccca 22860
   tacgaagggt tagctcatct caccctccgg cgcgataatg aatctgacgg gcacaaggag 22920
   aatgatcagc ggctgcgaca tttctcccag ccagagccgt tggtgttacc gcatcccggg 22980
   tcgccggaaa taactaaggt gtttggctcg cggggagagc cggcacaccc gagtggaaat 23040
   ctgcacacgg cggttggaga aacggcttgc gaaggaccgg tgatgtcttc atcctcggac 23100
   aatcatcagc cagctccagg acagcaagaa cttttaagtt tccttcataa tgcgccagcc 23160
   ccagtttctg tggcaataca tgatgatcaa gagcgacttg cgggggaggc gcccggcggc 23220
   tctttcagag gtagctcagg gcgaacgagt tcaatgtcgg agagtatctt cgacgaagat 23280
   gtacaagggc atttggtacg ggattattcg atcaatacta ctaacgggtt tattgacccg 23340
   caatcgttgt tcggtgaacc ggacttatcg agaggtccaa aatcggggcc agaaattcca 23400
   tcggaagatt accatttgtc agcttcggaa caggaaaatt tgctgaatca attgcttagt 23460
   gtgccactgc cggttccttc accgaagccc gaatgcgcga ggtctatgat tttcgaaggt 23520
   tcacgttcaa gagagcgttc cacctccaga gggttctaag gtggaacgcg tctaggcttg 23580
   cctatcatca ccacctcatg atggtcgcgg agaccttcga agagatatcc gacatcattg 23640
   gagaagctgg gccggaaact agtgtacctc gcgaatgcat ctagatccaa tccaatatgc 23700
   atggcatgcc gaatccatgt gggagtttat tcttgacaca gatatttatg atataataac 23760
   tgagtaagct taacataagg aggaaaaaca tatgttacgc agcagcaacg atgttacgca 23820
   gcagggcagt cgccctaaaa caaagttagg tggctcaagt atgggcatca ttcgcacatg 23880
   taggctcggc cctgaccaag tcaaatccat gcgggctgct cttgatcttt tcggtcgtga 23940
   gttcggagac gtagccacct actcccaaca tcagccggac tccgattacc tcgggaactt 24000
   gctccgtagt aagacattca tcgcgcttgc tgccttcgac caagaagcgg ttgttggcgc 24060
   tctcgcggct tacgttctgc ccaagtttga gcagccgcgt agtgagatct atatctatga 24120
   tctcgcagtc tccggcgagc accggaggca gggcattgcc accgcgctca tcaatctcct 24180
   caagcatgag gccaacgcgc ttggtgctta tgtgatctac gtgcaagcag attacggtga 24240
   cgatcccgca gtggctctct atacaaagtt gggcatacgg gaagaagtga tgcactttga 24300
   tatcgaccca agtaccgcca cctaataatg tctaacaatt cgttcaagcc gacgccgctt 24360
   cgcggcgcgg cttaactcaa gcgttagatg cactatacgt accaatccaa atcggatccc 24420
   cctgcagggc tgagataggt gcctcactga ttaagcattg gtaactgtca gaccaagttt 24480
   actcatatat actttagatt gatttaaaac ttcattttta atttaaaagg atctaggtga 24540
   agatcctttt tgataatctc atgaccaaaa tcccttaacg tgagttttcg ttccactgag 24600
   cgtcagaccc cgtagaaaag atcaaaggat cttcttgaga tccttttttt ctgcgcgtaa 24660
   tctgctgctt gcaaacaaaa aaaccaccgc taccagcggt ggtttgtttg ccggatcaag 24720
   agctaccaac tctttttccg aaggtaactg gcttcagcag agcgcagata ccaaatactg 24780
   tccttctagt gtagccgtag ttaggccacc acttcaagaa ctctgtagca ccgcctacat 24840
   acctcgctct gctaatcctg ttaccagtgg ctgctgccag tggcgataag tcgtgtctta 24900
   ccgggttgga ctcaagacga tagttaccgg ataaggcgca gcggtcgggc tgaacggggg 24960
   gttcgtgcac acagcccagc ttggagcgaa cgacctacac cgaactgaga tacctacagc 25020
   gtgagctatg agaaagcgcc acgcttcccg aagggagaaa ggcggacagg tatccggtaa 25080
   gcggcagggt cggaacagga gagcgcacga gggagcttcc agggggaaac gcctggtatc 25140
   tttatagtcc tgtcgggttt cgccacctct gacttgagcg tcgatttttg tgatgctcgt 25200
   caggggggcg gagcctatgg aaaaacgcca gcaacgcggc ctttttacgg ttcctggcct 25260
   tttgctggcc ttttgctcac atgttctttc ctgcgttatc ccctgattct gtggataacc 25320
   gtattaccgc ctttgagtga gctgataccg ctcgccgcag ccgaacgacc gagcgcagcg 25380
   agtcagtgag cgaggaagcg gaagagcgcc tgatgcggta ttttctcctt acgcatctgt 25440
   gcggtatttc acaccgcata tggtgcactc tcagtacaat ctgctctgat gccgcatagt 25500
   taagccagta tacactccgc tatcgctacg tgactgggtc atggctgcgc cccgacaccc 25560
   gccaacaccc gctgacgcgc cctgacgggc ttgtctgctc ccggcatccg cttacagaca 25620
   agctgtgacc gtctccggga gctgcatgtg tcagaggttt tcaccgtcat caccgaaacg 25680
   cgcgaggcag cagatctatc gatggtacct atctgcagac ggaagggatt cctgttaatt 25740
   aaaacagctt gcgtcatgcg gtcgctgcgt atatgatgcg atgagtaaat aaacaaatac 25800
   gcaaggggaa cgcatgaagg ttatcgctgt acttaaccag aaaggcgggt caggcaagac 25860
   gaccatcgca acccatctag cccgcgccct gcaactcgcc ggggccgatg ttctgttagt 25920
   cgattccgat ccccagggca gtgcccgcga ttgggcggcc gtgcgggaag atcaaccgct 25980
   aaccgttgtc ggcatcgacc gcccgacgat tgaccgcgac gtgaaggcca tcggccggcg 26040
   cgacttcgta gtgatcgacg gagcgcccca ggcggcggac ttggctgtgt ccgcgatcaa 26100
   ggcagccgac ttcgtgctga ttccggtgca gccaagccct tacgacatat gggccaccgc 26160
   cgacctggtg gagctggtta agcagcgcat tgaggtcacg gatggaaggc tacaagcggc 26220
   ctttgtcgtg tcgcgggcga tcaaaggcac gcgcatcggc ggtgaggttg ccgaggcgct 26280
   ggccgggtac gagctgccca ttcttgagtc ccgtatcacg cagcgcgtga gctacccagg 26340
   cactgccgcc gccggcacaa ccgttcttga atcagaaccc gagggcgacg ctgcccgcga 26400
   ggtccaggcg ctggccgctg aaattaaatc aaaactcatt tgagttaatg aggtaaagag 26460
   aaaatgagca aaagcacaaa cacgctaagt gccggccgtc cgagcgcacg cagcagcaag 26520
   gctgcaacgt tggccagcct cgcagacacg ccagccatga agcgggtcaa ctttcagttg 26580
   ccggcggagg atcacaccaa gctgaagatg tacgcggtac gccaaggcaa gaccattacc 26640
   gagctgctat ctgaatacat cgcgcagcta ccagagtaaa tgagcaaatg aataaatgag 26700
   tagatgaatt ttagcggcta aaggaggcgg catggaaaat caagaacaac caggcaccga 26760
   cgccgtggaa tgccccatgt gtggaggaac gggcggttgg ccaggcgtaa gcggctgggt 26820
   tgcctgccgg ccctgcaatg gcactggaac ccccaagccc gaggaatcgg cgtgagcggt 26880
   cgcaaaccat ccggcccggt acaaatcggc gcggcgctgg gtgatgacct ggtggagaag 26940
   ttgaaggcag cgcaggccgc ccagcggcaa cgcatcgagg cagaagcacg ccccggtgaa 27000
   tcgtggcaag cagccgctga tcgaatccgc aaagaatccc ggcaaccgcc ggcagccggt 27060
   gcgccgtcga ttaggaagcc gcccaagggc gacgagcaac cagatttttt cgttccgatg 27120
   ctctatgacg tgggcacccg cgatagtcgc agcatcatgg acgtggccgt tttccgtctg 27180
   tcgaagcgtg accgacgagc tggcgaggtg atccgctacg agcttccaga cgggcacgta 27240
   gaggtttccg cagggccggc aggcatggcc agtgtgtggg attacgacct ggtactgatg 27300
   gcggtttccc atctaaccga atccatgaac cgataccggg aagggaaggg agacaagccc 27360
   ggccgcgtgt tccgtccaca cgttgcggac gtactcaagt tctgccggcg agccgatggc 27420
   ggaaagcaga aagacgacct ggtagaaacc tgcattcggt taaacaccac gcacgttgcc 27480
   atgcagcgta cgaagaaggc caagaacggc cgcctggtga cggtatccga gggtgaagcc 27540
   ttgattagcc gctacaagat cgtaaagagc gaaaccgggc ggccggagta catcgagatc 27600
   gagctagctg attggatgta ccgcgagatc acagaaggca agaacccgga cgtgctgacg 27660
   gttcaccccg attacttttt gatcgatccc ggcatcggcc gttttctcta ccgcctggca 27720
   cgccgcgccg caggcaaggc agaagccaga tggttgttca agacgatcta cgaacgcagt 27780
   ggcagcgccg gagagttcaa gaagttctgt ttcaccgtgc gcaagctgat cgggtcaaat 27840
   gacctgccgg agtacgattt gaaggaggag gcggggcagg ctggcccgat cctagtcatg 27900
   cgctaccgca acctgatcga gggcgaagca tccgccggtt cctaatgtac ggagcagatg 27960
   ctagggcaaa ttgccctagc aggggaaaaa ggtcgaaaag gtctctttcc tgtggatagc 28020
   acgtacattg ggaacccaaa gccgtacatt gggaaccgga acccgtacat tgggaaccca 28080
   aagccgtaca ttgggaaccg gtcacacatg taagtgactg atataaaaga gaaaaaaggc 28140
   gatttttccg cctaaaactc tttaaaactt attaaaactc ttaaaacccg cctggcctgt 28200
   gcataactgt ctggccagcg cacagccgaa gagctgcaaa aagcgcctac ccttcggtcg 28260
   ctgcgctccc tacgccccgc cgcttcgcgt cggcctatgc atataattgt ggtttcaaaa 28320
   tcggctccgt cgatactatg ttatacgcca actctgaaaa caactttgaa aaagctgttt 28380
   tctggtattt 28390
<210> 36
   <211> 31939
   <212> DNA
   <213> Artificial sequence
<220>
   <223> Synthetic construct
<400> 36
   cgccaacacc cgctgacgcg ccctgacggg cttgtctgct cccggcatcc gcttacagac 60
   aagctgtgac cgtctccggg agctgcatgt gtcagaggtt ttcaccgtca tcaccgaaac 120
   gcgcgaggca gcagatctat cgatggtacc tatctgcaga cggaagggat tcctgttaat 180
   taaaacagct tgcgtcatgc ggtcgctgcg tatatgatgc gatgagtaaa taaacaaata 240
   cgcaagggga acgcatgaag gttatcgctg tacttaacca gaaaggcggg tcaggcaaga 300
   cgaccatcgc aacccatcta gcccgcgccc tgcaactcgc cggggccgat gttctgttag 360
   tcgattccga tccccagggc agtgcccgcg attgggcggc cgtgcgggaa gatcaaccgc 420
   taaccgttgt cggcatcgac cgcccgacga ttgaccgcga cgtgaaggcc atcggccggc 480
   gcgacttcgt agtgatcgac ggagcgcccc aggcggcgga cttggctgtg tccgcgatca 540
   aggcagccga cttcgtgctg attccggtgc agccaagccc ttacgacata tgggccaccg 600
   ccgacctggt ggagctggtt aagcagcgca ttgaggtcac ggatggaagg ctacaagcgg 660
   cctttgtcgt gtcgcgggcg atcaaaggca cgcgcatcgg cggtgaggtt gccgaggcgc 720
   tggccgggta cgagctgccc attcttgagt cccgtatcac gcagcgcgtg agctacccag 780
   gcactgccgc cgccggcaca accgttcttg aatcagaacc cgagggcgac gctgcccgcg 840
   aggtccaggc gctggccgct gaaattaaat caaaactcat ttgagttaat gaggtaaaga 900
   gaaaatgagc aaaagcacaa acacgctaag tgccggccgt ccgagcgcac gcagcagcaa 960
   ggctgcaacg ttggccagcc tcgcagacac gccagccatg aagcgggtca actttcagtt 1020
   gccggcggag gatcacacca agctgaagat gtacgcggta cgccaaggca agaccattac 1080
   cgagctgcta tctgaataca tcgcgcagct accagagtaa atgagcaaat gaataaatga 1140
   gtagatgaat tttagcggct aaaggaggcg gcatggaaaa tcaagaacaa ccaggcaccg 1200
   acgccgtgga atgccccatg tgtggaggaa cgggcggttg gccaggcgta agcggctggg 1260
   ttgcctgccg gccctgcaat ggcactggaa cccccaagcc cgaggaatcg gcgtgagcgg 1320
   tcgcaaacca tccggcccgg tacaaatcgg cgcggcgctg ggtgatgacc tggtggagaa 1380
   gttgaaggca gcgcaggccg cccagcggca acgcatcgag gcagaagcac gccccggtga 1440
   atcgtggcaa gcagccgctg atcgaatccg caaagaatcc cggcaaccgc cggcagccgg 1500
   tgcgccgtcg attaggaagc cgcccaaggg cgacgagcaa ccagattttt tcgttccgat 1560
   gctctatgac gtgggcaccc gcgatagtcg cagcatcatg gacgtggccg ttttccgtct 1620
   gtcgaagcgt gaccgacgag ctggcgaggt gatccgctac gagcttccag acgggcacgt 1680
   agaggtttcc gcagggccgg caggcatggc cagtgtgtgg gattacgacc tggtactgat 1740
   ggcggtttcc catctaaccg aatccatgaa ccgataccgg gaagggaagg gagacaagcc 1800
   cggccgcgtg ttccgtccac acgttgcgga cgtactcaag ttctgccggc gagccgatgg 1860
   cggaaagcag aaagacgacc tggtagaaac ctgcattcgg ttaaacacca cgcacgttgc 1920
   catgcagcgt acgaagaagg ccaagaacgg ccgcctggtg acggtatccg agggtgaagc 1980
   cttgattagc cgctacaaga tcgtaaagag cgaaaccggg cggccggagt acatcgagat 2040
   cgagctagct gattggatgt accgcgagat cacagaaggc aagaacccgg acgtgctgac 2100
   ggttcacccc gattactttt tgatcgatcc cggcatcggc cgttttctct accgcctggc 2160
   acgccgcgcc gcaggcaagg cagaagccag atggttgttc aagacgatct acgaacgcag 2220
   tggcagcgcc ggagagttca agaagttctg tttcaccgtg cgcaagctga tcgggtcaaa 2280
   tgacctgccg gagtacgatt tgaaggagga ggcggggcag gctggcccga tcctagtcat 2340
   gcgctaccgc aacctgatcg agggcgaagc atccgccggt tcctaatgta cggagcagat 2400
   gctagggcaa attgccctag caggggaaaa aggtcgaaaa ggtctctttc ctgtggatag 2460
   cacgtacatt gggaacccaa agccgtacat tgggaaccgg aacccgtaca ttgggaaccc 2520
   aaagccgtac attgggaacc ggtcacacat gtaagtgact gatataaaag agaaaaaagg 2580
   cgatttttcc gcctaaaact ctttaaaact tattaaaact cttaaaaccc gcctggcctg 2640
   tgcataactg tctggccagc gcacagccga agagctgcaa aaagcgccta cccttcggtc 2700
   gctgcgctcc ctacgccccg ccgcttcgcg tcggcctatg catataattg tggtttcaaa 2760
   atcggctccg tcgatactat gttatacgcc aactctgaaa acaactttga aaaagctgtt 2820
   ttctggtatt taaatcctag gaaatatcag ccgcttgtgt gttcaattct tctctgtttc 2880
   acttgaaaca aattgaatag atattcccgc tttcaaagcc atttacaaat cctctcgtgc 2940
   agcctaacgc cattggcgtg gaacaagcgt tggcacgagg aagtaagtgc gatgaacgga 3000
   aggtattcac cgacgcggca ggattttaag acaggcgcga agccttggtc tatattagcc 3060
   cttatcgttg ccgcaatgat tttcgcgttc atggcggttg cgtcctggca ggacaatggg 3120
   actacccagg caatccttag ccaaatacga tcgattaacg ctgacagcgc ctcactgcag 3180
   cgcgatgtac tccgcgctca cacgggcacc gtggcgaact accgccccat tatctccagg 3240
   ctgggagctc tgcggaagaa tctggaagat ttgaagcaat tatttaggca atctcatatt 3300
   gtaagtgaga gcaatgctgc tcaactgcta cgccggctag aagtgtctct aaattcggct 3360
   gacgcggcgg tcgccgcctt tggtgcgcaa aatgtccgcc tgcaagattc gctggccagt 3420
   ttcactcgtg ctttaagcaa tcttccagga aaggcctcag gcgatcagac tttagaaaaa 3480
   ccaacagaat tggctagcat gatgctccaa tttcttcggc aaccaagccc ggctatttca 3540
   ttcgagatca gccttgaact agagaggctc caaaaacaac gcggtcttga tgaagctccc 3600
   gtgcgcatac tttcgcgtga aggtcccatt atcttatcgc ttttgccaca ggtgaacgat 3660
   ctggtgaaca tgattcagac gtctgacacc gcagaaattg cggaaatgtt gcagcgtgag 3720
   tgtttggagg tctatagctt gaaaaatgtg gaggagcgga gcgcacgtat ctttcttggg 3780
   tccgcttcag tgggtctttg cctgtacatc atcaccttag tctataggct acgcaaaaaa 3840
   accgattggt tagcgcggcg tttagattac gaagagctaa tcaaagagat cggagtatgt 3900
   tttgaaggtg aggcggccac tacgtcgtcc gcgcaagctg cacttggtat tattcagcgc 3960
   ttctttgatg ccgatacgtg cgcgttagct ctagtggacc atgaccgtag gtgggctgtc 4020
   gaaacattcg gtgcgaagca cccaaaaccc gtgtgggacg acagggtgct acgcgaaata 4080
   gtctctcgta ccaaagcgaa cgaacgggcg acggtattcc gcatcgtatc gacgcaaaaa 4140
   atcgtacatt tgcctcccga aattccaggt ctttcgatac tactggctca caaatccaca 4200
   gataaactaa ttgcggtttg ttcactgggt taccaaagct atcgccctcg accttgccaa 4260
   ggcgaaattc agcttcttga actcgccacc gcctgcctct gtcactatat cgatgttcgg 4320
   cgtaagcaga ccgaatgcga cgttttggcc agacgattgg agcatgcgca acgccttgag 4380
   gcagttggta cacttgccgg cggaatagca catgaattta ataacatttt ggggtcaatc 4440
   ctcgggcacg cagaattggc ccaaaactcg gtgtctcgaa catctgtcac ccgaagatac 4500
   attgactata tcatttcgtc aggcgacaga gccatgctca ttatcgatca gatcttgacg 4560
   ctgagccgaa aacaagagcg cgtgatcaag ccatttagtg tctcagagct tgtgaccgaa 4620
   atcgctccct tgctacgtat ggctcttccc ccaaccatcg agcttagttt cagatttgat 4680
   caaatgcaga gcgtgatcga aggaagcccg cttgaacttc aacaggtact aattaacctc 4740
   tgcaagaatg cttcccaagc catgactgca aatggtcaaa tcgacatctt cgtcggccaa 4800
   gcttatttac cagctaagaa aattctggcg catggtgtta tgccacctgg cgactatgtt 4860
   ctactatctg tcagcgacaa tggtggaggc atttcagagg ctgtgctacc ctacattttt 4920
   gaacccttct ttacgacacg tgctcgcaac ggtggaacgg gtctcggcct cgcttctgtg 4980
   catggtcata tcagcgcgtt tgcaggttac atcgacgtta gttcaactgt tgggcatggg 5040
   acgcgctttg acatttatct ccctccgtct tctaaggagc ccgtcaatcc cgacagcttt 5100
   ttcggccgca ataaggcacc gcgtggaaac ggggagattg tggcacttgt tgagcccgat 5160
   gacctcctgc gggaggcgta tgaagacaag atcgccgctc tgggatatga gccggtcggt 5220
   tttcgtacct ttagtgaaat tcgcgattgg atttcaaaag gcaatgaagc cgatctggtc 5280
   atggtcgacc aagcgtctct tcctgaagat caaagtccta attccgtgga tttagtgctc 5340
   aagaccgcct ccatcatcat tggcggaaat gatctcaaaa tgcccctttc aagggagaat 5400
   gcgaccaggg acctttatct gccgaagccg atatcgtcca gaactatggc gcatgcaatc 5460
   ctaaccaaaa tcaagacgta gagttgcgac gtatcaggac tgggcaatcg cggccgccgg 5520
   gcttacgtgt cttttgtaac aaattgcgac gcagttgata tccacttgaa acattagtcc 5580
   gaaattatcg agatctccgc tgataaggta tcgaaatggc gataaaattg gtattgatac 5640
   tcgtattcac actctttccc gcggcagacg ctgcatatgc gaatgaccgc gccaacggtt 5700
   tcatgtggtc aaacgggggc gaaactggag tgaggcttcc tcttcgggtg ttcaatgcca 5760
   agccagccaa gaacacggtg gcgatcattt attccggaga cgctggatgg caaaatatcg 5820
   atgaggcgat tggtacctat ctgcagacgg aagggattcc tgtcattggc gtcagttcac 5880
   ttcggtattt ctggtcggag cggtccccaa gcgaaactgc taaggatctt ggtcacataa 5940
   tcgatgtcta caccaagcat ttcggtgtgc agaatgtttt acttgtagga tattctttcg 6000
   gcgcggacgt catgccggca agcttcaata ggcttacgct tgagcaaaaa aatcgggtta 6060
   agcaaatctc tctcttggca ttgtcacatc aagtcgacta tgtcgtctca tttaggggct 6120
   ggctccaact cgaaacggaa ggtaagggcg gcaatcctct ggatgatctc agatccattg 6180
   accctgcaat cgtccaatgc atgtacgggc gcgaagaccg taataatgct tgcccatctc 6240
   tccgacagac cggcgcagag gtgataggct tcagcggagg ccatcacttt gataatgatt 6300
   tcaaaaaact gtctacgcgc gtcgtctcag gcctcgtggc acgcctaagt catcagtaat 6360
   ctttagttcc tgcaccgcgg gacccgcgag gtttctcgct tcaattgaaa tcataaagaa 6420
   gcaattgaaa attttcgagt aaccgaccct cccgataatc ttcaacataa aacaacgcac 6480
   ttcttccaac gggagaggcg gtgttagttg cgagctaagg agataaggta tgcttaagag 6540
   atcggggtcg ctttctcttg ccttgatggt ctccttctgt tcgtcgagcc ttgccacgcc 6600
   actctcatct gctgagtttg accatgttgc tcgcaagtgt gccccatcag ttgcgacatc 6660
   tacgcttgcg gcgatagcta aggtggagag tcgctttgat cctttagcga ttcatgacaa 6720
   cacgaccggc gaaacgcttc actggcaaga tcacagccaa gcaacccaag tcgtcaggca 6780
   ccgtctcgat gcacggcatt cgctggatgt tggcctcatg caaataaact ctcgaaattt 6840
   ttctatgctc ggtctgacac ctgacggtgc gctccaggcg tgcacatcat tatctgccgc 6900
   tgcaaacatg ctgaaaagtc gttatgcagg cggcgaaacg attgacgaga agcaatttgc 6960
   gcttcgtcgg gcgatctccg cttacaacac cggtaatttc atcggcggtt ttgcaaacgg 7020
   ctacgtgcga aaagttgaaa cagctgctca atcgctggtg cccgcgttaa tcgagcctcc 7080
   aaaagacgat cacgaggcgc taaaatccga agagacgtgg gatgtttggg ggtcatatca 7140
   gcgccgctcg caggaggatg gcgctggcgg tttaatcgct ccgccaccgc cacaccagga 7200
   caacggcaaa tccgcagacg acaatcaagt cttattcgac ttatactaag gaggtgcgca 7260
   ttgatgcgat gctttgagag ataccgttta catctaaatc gcctctcgct ctcgaatgcg 7320
   atgatgcgcg tgatatcgag ctgcgcccca agcttgtgcg gtgcaattgc atggagcatt 7380
   tcctcatccg gacccgccgc agcgcaatct gcgggtggcg gcactgaccc cgccacaatg 7440
   gttaacaata tatgcacgtt tatccttggt ccgttcggcc agtcactcgc tgttctcggc 7500
   attgtcgcta tcgggatctc ctggatgttc gggcgggctt cgcttgggct ggttgccggc 7560
   gtcgtcggcg gcattgttat catgtttggg gcgagcttcc tcggccaaac gctcactggc 7620
   ggtagttgat ggctgatcgt ttggaagaat cgacccttta cctcgcagcc acacggcccg 7680
   cattgtttct tggggtgcca ctgacattgg cagggttatt catgatgttc gccggctttg 7740
   tcatcgttat cgttcagaac ccgctctacg aagtcgttct cgtgccgtta tggtttgcag 7800
   cccggctcat cgtggagcga gactacaatg cggcgagcgt cgtcctgcta tttttgcgga 7860
   ccgcgggaag aagcattgat agtgcagttt gggggggcgc tactgttagc ccaaatccaa 7920
   ttagggttcc cccacgaggg agaggaatgg tgtgatgctc ggcgcgagtg gaacgaccga 7980
   aagatccggt gagatctatc tcccttatat tggccacctc agcgaccata tcgtccttct 8040
   tgaagacgga tcgatcatga ccattgcgag aattgatggc gttgcattcg agcttgagga 8100
   aactgaaatg cgcaatgcgc gttgtcgtgc gttcaacacg ctgttgcgca atatcgctga 8160
   tgatcatgtg tcaatatatg ctcacctcgt acgtcatgcc gacgtgccat catcggcgcc 8220
   gcgacacttc cgtagtgttt tcgccgctag cctgaacgaa gcttttgaac agcgcgtgct 8280
   ctccggccaa ctcctccgca atgaacactt ccttacgttg attgtctacc cacaggcggc 8340
   tttagggaag gtaaagagga ggttcaccaa gctaagcgga aaaagggaaa acgatctcac 8400
   gggccagatc aggaacatgg aagatctttg gcatgttgtc gctggctctc ttaaagcgta 8460
   tggcctgcat cgtcttggca tccgcgagaa gcagggtgtg ctcttcaccg aaattggcga 8520
   agcgctacgg ttgatcatga ctggtcggtt cacaccggtt ccggtcgtca gcggctcact 8580
   cggcgcttcg atttataccg acagagtcat ttgcggcaag cgaggactcg agatcagaac 8640
   gccaaaagac agttacgttg gatccatcta ttcgtttcgc gaataccctg caaaaacacg 8700
   gccgggcatg ctcaacgcgc tgctatccct cgattttcca cttgttctca cgcagagttt 8760
   ttcgttcctg actcgcccgc aagcgcacgc gaaacttagc ctcaaatcga gccagatgct 8820
   gagttccggc gataaagccg tgactcaaat cggcaaatta tccgaggctg aggacgcact 8880
   tgcgagcaac gaattcgtta tgggctcaca tcatttgagc ctttgcgtct atgcagacga 8940
   tctcaatagt cttggggaca ggggcgcgcg ggctcggaca cgaatggcgg atgcaggtgc 9000
   cgtggttgtc caagaaggta ttggtatgga agcggcctat tggtcccaat tgccggggaa 9060
   ttttaagtgg cgcacacgcc ctggcgcaat cacttcacgc aatttcgcag ggtttgtctc 9120
   tttcgaaaac tttccagagg gcgccagctc aggccactgg ggcaacgcga ttgcccgatt 9180
   tcgtaccaat ggcggaacgc ctttcgacta tatcccgcat gagcacgatg ttggcatgac 9240
   ggcaatattc gggcctatcg ggaggggtaa gacgacgctc atgatgtttg ttctagccat 9300
   gctcgaacag agcatggtcg accgtgcagg tacggtcgtg ttctttgaca aggaccgggg 9360
   tggcgaattg ctggttcgcg ccacaggagg aacatatttg gcacttcaca gaggcacacc 9420
   cagcgggttg gcgccgttgc gtggcctaga aaacacagca gcctcacacg attttctgcg 9480
   cgaatggatc gtggctctca tcgagagtga tggtcggggt gggatttctc cggaagagaa 9540
   ccgccgtctg gtccgtggta tccatcgtca gctctcgttt gatccacaaa tgcgttcaat 9600
   cgcggggtta cgtgaatttt tgttgcatgg gcccgccgaa ggcgcaggag cgcggctcca 9660
   acgctggtgc cggggccatg cgcttggctg ggcatttgac ggcgaagttg acgaagtaaa 9720
   gttagatccg tcgattaccg gcttcgacat gacgcatctt ctcgaatacg aggaagtatg 9780
   cgctcccgct gcagcatatc tcctgcatcg gattggagcc atgatcgacg gccgccgttt 9840
   tgtgatgagc tgcgatgagt ttcgcgccta tttgttaaac cctaaatttt cgactgtcgt 9900
   cgacaaattc ctcctgaccg ttcgaaaaaa caacgggatg ctaatactgg caacgcagca 9960
   accagagcat gttctggaat cgccgctagg agccagcttg gttgcgcaat gtatgacgaa 10020
   gattttctat ccatcaccaa ccgcagatcg atcggcttat gtcgatggac tgaaatgtac 10080
   cgaaaaggaa tttcaggcga tccgtgaaga catgacggtc ggcagccgta agtttcttct 10140
   taaacgagaa agtggaagcg tcatctgcga atttgatctg cgggatatgc gtgaatatgt 10200
   cgccgtgctt tcggggcgtg ccaacacggt gcgctttgca actcgactac gcgaggcaca 10260
   agaaggcaac tcatctggct ggctcagcga attcatggcc cgtcaccacg aggcagaaga 10320
   ttgataaggt aggaaacgat gaagacgacg caacttattg caacagtttt gacctgcagc 10380
   tttctatata ttcagcccgc gcgggcgcag tttgttgtta gcgacccggc aacggaggct 10440
   gagacgctcg cgactgcgct cgcgactgcg gagaatctca ctcagactat agcgatggtt 10500
   acgatgttga cgtcggccta cggcgttact ggactactga cttcgctcaa ccagaaaaat 10560
   cagtatcctt cgacgaagga cctagacaat gaaatgtttt cgccgcgaat gccaatgtcg 10620
   accacggcac gtgcgatcac cagcgataca gatcgtgcag tcgtgggtag tgatgctgaa 10680
   gcggacctgt tgcgatcgca gatcaccggt tccgcaaaca gcgctggcat tgcggctgac 10740
   aatctggaaa cgatggacaa acgcttgacg gcgaatgctg atacgtctgc tcagctttcc 10800
   cgatctcgca atatcatgca ggcaaccgtg accaatggtt tgcttctcaa gcagatccat 10860
   gacgcaatga ttcaaaatgt acaggcgaca agcctattaa cgatgactac cgcgcaggcc 10920
   ggccttcacg aggcggaaga ggcggccgct caacgcaagg agcatcaaaa gaccgctgtc 10980
   atctttggtg ccctccccta aggctgggcg atttgttcat ccgcccgcat cctcgccgaa 11040
   tgcgagctca ttttatccaa cattatgcga caaaccagtc aagttcaggt ccaatcgatg 11100
   aatttcacga ttccggcgcc gtttacggcc attcatacga tcttcgatgt agccttcacg 11160
   acaggcttgg actcgatgct tgagactatc caggaggcgg tgagtgcgcc attgatcgcc 11220
   tgtgtcactc tttggattat tgttcagggt attttagtca tacgcggcga agtcgatacc 11280
   cgtagcggta tcactcgggt gatcacggtc accatcgttg ttgctctaat tgttgggcag 11340
   gctaactacc aagactatgt ggtttccatc ttcgaaaaga cggtcccaaa ctttgttcag 11400
   cagtttagtg taaccggctt gcctctgcag actgttccgg cacagttgga tacaatgttc 11460
   gccgtgaccc aggccgtttt tcagaaaatc gcatccgaaa tcggtccgat gaacgaccag 11520
   gacatccttg ctttccaagg ggcacagtgg gtcctttacg gcacgctctg gtctgccttc 11580
   ggagtttacg acgccgttgg aattctcacg aaagtgcttc tcgcgatcgg gcctctgatc 11640
   ctcgtcggat atatttttga tcgcacgcgg gacatcgcag ctaagtggat cgggcaactt 11700
   atcacctacg gtctcttgct tctcctctta aacctcgtgg caacgatcgt catcctaacc 11760
   gaagcgactg cgctcaccct tatgcttggt gtaatcacct tcgccggtac gaccgcggcc 11820
   aagatcattg gtctttacga actcgatatg ttttttctga caggggatgc gctcattgtc 11880
   gctttgccgg cgatcgccgg caacattgga ggcagttact ggagcggcgc aacccaatct 11940
   gccagcagct tgtaccgtcg cttcgctcag gttgagcgag gctaggtcgc gcaaaaattc 12000
   gcctcaatgg agaattctat gaagtattgc ctgctgtgcc tagttgtcgc tttgagcggc 12060
   tgccagacaa acgacacatt agcgagctgc aaaggcccga tcttcccgct gaatgtgggg 12120
   cgatggcagc ctactccgtc agatcttcag ctcggcaatt cgggtggacg ctatgacggg 12180
   gcctgaatat gccatgctag tggcgcgcga aagccttgcc gagcactata aggaagtaga 12240
   agcctttcaa accgcgcgag cgaaatcggc gcgacgtctc tccaaactca ttgcagctgt 12300
   cgcagctatc gcgattttgg gaaatgttgc tcaagcgttc gctatagcca caatggtgcc 12360
   gttgagcagg cttgtgcccg tatatctatg gatacggccg gacggcaccg ttgacagcga 12420
   ggtgtctgtc tcgcgattgc ctgcaactca agaggaggcc gtcgttaacg cctcattgtg 12480
   ggagtacgtt cgcctgcgcg agagttatga tgccgacacc gctcagtacg cctacgacct 12540
   ggtatcgaac ttcagtgccc caacagtgcg ccaggattac cagcaattct tcaactatcc 12600
   caatcccagt tcgcctcaag tcattcttgg caaacgcggc agggtggagg tcgagcacat 12660
   cgcttcaaat gatgtaactc caagcacgca gcaaattcgc tataaaagga ccctcgtcgt 12720
   tgacggcaaa atgcctgtgg tgagtacgtg gaccgcgaca gttcgctacg aaaaggtgac 12780
   cagcttgccc ggcagattga gactaaccaa cccggcaggt ctggttgtca cctcctatca 12840
   gacatcggaa gataccgttt caaacgtagg ccacagcgaa ccatgatcag aaaagcactt 12900
   ttcattttag catgtttatt tgccgctgcg actggtgcgg aggctgaaga cactccaatg 12960
   gcgggcaagc tagatccgcg catgcgttat ttggcttaca atcccgatca agtggtgcgc 13020
   ctctcgacgg cggttggagc tactttggtc gtaacattcg ccacgaacga aacggtgaca 13080
   gcggttgccg tttcaaatag caaagatcta gcagccctac cgcggggaaa ttatctattt 13140
   ttcaaggcaa gccaggtcct cacgcctcag ccagtaatcg tgctaaccgc aagcgactcc 13200
   gggatgcgcc gttatgtttt cagtataagt tccaagactc tgtcccacct cgataaagag 13260
   cagcccgatc tctattacag cgtccaattc gcctaccccg ccgacgatgc ggcggctcgg 13320
   cgaagggagg cacaacagaa ggctgttgtg gacagactac acgcggaagc acaatatcaa 13380
   cggaaagctg agaatttatt ggatcagcct gtcacagccc ttggtgcggc ggacagtaat 13440
   tggcactacg tcgcccaagg cgatcgttcg ctgttgccac tcgaagtctt cgacaatgga 13500
   tttacgacgg tattccactt tccgggcaat gtacgcatac cctccatcta caccatcaat 13560
   cctgatggca aggaagctgt tgccaactat tcagttaaag ggagcgatgt cgagatttct 13620
   tcggtttccc gaggttggcg tctgagggat ggccacacag tactatgtat ctggaacacc 13680
   gcttacgatc ccgttggcca aaggccgcaa acgggcacgg tgaggcccga tgtgaaacgc 13740
   gtcctgaagg gggcgaaggg atgaataacg atagtcagca agcggcacat gaggttgatg 13800
   catctggatc cctggtctcc gacaaacatc gccggcgtct ttcggggtct cagaaattga 13860
   tcgtcggagg tgtcgttctc gcgttatcat taagcctcat ttggctaggt gggcgccaaa 13920
   agaaggtgaa tgagaacgca tcgccgtcaa ctttgatcgc aacaaacacc aagccatttc 13980
   atccagctcc gattgaggtg ccgccggatc ctccagcggt tcaagaggct gttcagcctg 14040
   ctgctcctct accgccgagg ggcgaaccgg agcggcatga gccacggccg gaagaaacac 14100
   cgatttttgc atatagcagc ggcgatcaag gggtcagcaa acgcgccatt cagggcgaca 14160
   cgggccgaag acaagaaggc aagcgtgacg acaactcctt gccgaatggc gaagtgtccg 14220
   gcgagaacga tttgtcgata cgtatgaaac ccaccgagct gcagcccagc agcgccacgc 14280
   tcttgccgca ccccgatttt atggtaacgc aagggacaat aattccgtgc atcttgcaaa 14340
   ccgcaatcga cacaaatttg gcaggctatg taaagtgtgt cttgcctcag gatattcgtg 14400
   gaacaacgaa caatatcgtg cttcttgatc gtggcaccac cgttgttggc gaaatacagc 14460
   gtggcttgca acagggagat gggcgcgttt ttgtgttgtg ggatcgcgcc gagacacctg 14520
   accatgcgat gatctcgtta acatcgccaa gcgcggacga actcggtcgc tcaggattgc 14580
   cgggctcggt cgacagccac ttctggcagc gttttagcgg agctatgctc ttgagtgttg 14640
   ttcaaggcgc cttccaggca gctagcacct acgccggcag ctcgggtggc gggatgagct 14700
   tcaacagctt tcaaaataac ggtgagcaga caactgagac agcccttaag gcaaccatca 14760
   acataccgcc aaccctgaag aagaatcagg gtgacaccgt ttccattttc gtagcacggg 14820
   acctcgattt ctttggtgtt taccagctcc gcctgactgg cggcgccacg cgggggagga 14880
   accgccgctc ttaatgaatt caaatttccg cttagagata ggatacattg taaatggaag 14940
   tggatccgca actacgcttt cttctgaagc cgattttgga atggctcgat gacccgaaga 15000
   ctgaagaaat tgcgatcaat cgacctggag aggcatttgt gcgccaagcc ggcattttta 15060
   ccaagatgcc tttgcccgtc tcttatgatg atcttgaaga tatcgctatt ttagcgggcg 15120
   cgctgagaaa gcaggatgtc ggaccacgta accccctctg cgccactgaa cttcctggtg 15180
   gtgaacgact acaaatctgt ctgccgccga ccgttccctc gggcaccgtc agcttgacca 15240
   ttcgacggcc aagctcgcgt gtttctggtc ttaaagaagt ctcctcccgt tatgatgctt 15300
   cgaggtggaa ccagtggcag acacgaagga aacgccaaaa tcaggatgat gaagctatcc 15360
   ttcagcattt tgacaacggg gatttggaag cgtttctgca cgcatgcgtc gtcagccgac 15420
   tgacgatgtt gctatgtggc cctaccggaa gcggcaagac aacaatgagc aagaccttga 15480
   tcagcgccat ccccccccag gaaaggctaa tcaccataga agatacgctc gaactcgtca 15540
   ttccacacga taatcatgtt agactactct actccaagaa cggtgctggg ctgggtgctg 15600
   tgagcgccga gcacttgctc caagcaagtc tgcgtatgcg gccggaccgg atattgcttg 15660
   gcgagatgcg cgacgatgca gcatgggctt atctgagtga agtcgtctcg ggacatccgg 15720
   gatcgatttc aacaatacac ggcgcgaatc caatccaagg attcaagaaa ctgttttccc 15780
   ttgtcaaaag tagcgcccaa ggtgctagct tggaagatcg cacactgatt gacatgctct 15840
   ctacggcgat cgatgtcatc attccattcc gtgcctatga ggacgtttat gaagtaggcg 15900
   agatctggct cgcggcggac gcacgacgcc ggggcgagac cataggcgat ctccttaatc 15960
   aatagtagct gtaacctcga agcgtttcac ttgtaacaac gattgagaac ttttgtcata 16020
   aaattgaaat acttggttcg cattttcgtc atccgcggtc agccgcaatt ctgacgaact 16080
   gcccatttag ctggagatga ttgtacatcc ttcacgtgaa aatttctcaa gcgctgtgaa 16140
   caagggttca gattttagat tgagaggtga gccgttgaaa cacgttcttc ttatcgatga 16200
   cgatgtcgct atgcggcatc ttattatcga ataccttacg atccacgcct tcaaagtgac 16260
   cgcggtagcc gacagcaccc agttcactag agtactctct tccgcgacgg tcgatgtcgt 16320
   ggttgttgat ctaaatttag gtcgtgaaga tgggcttgag atcgttcgaa atctggcggc 16380
   aaagtctgat attccaatca taattatcag tggcgaccgc cttgaggaga cggataaagt 16440
   tgttgcactc gagctaggag caagtgattt tatcgctaag ccgtttagta cgagagagtt 16500
   tcttgcacgc attcgggttg ccttgcgcgt gcgccccaac gttgtccgct ccaaagaccg 16560
   acggtctttt tgttttactg actggacact taatctcagg caacgtcgct tgatgtccga 16620
   agctggcggt gaggtgaaac ttacggcagg tgagttcaat cttctcctcg cgtttttaga 16680
   gaaaccccgc gacgttctat cgcgcgagca acttctcatt gccagtcgag tacgcgacga 16740
   ggaggtttac gacaggagta tagatgttct cattttgcgg ctgcgccgca aacttgaggc 16800
   ggatccgtca agccctcaac tgataaaaac agcaagaggt gccggttatt tctttgacgc 16860
   ggacgtgcag gtttcgcacg gggggacgat ggcagcctga gccaattgca tttgattaat 16920
   ttaggtgact gaggacgcgg ccagcggcct caaacctaca ctcaatattt ggtgaggggt 16980
   tccgataggt ccctcttcac caattgctcg atggcttctc tccagcaaag aatgacgcga 17040
   gcgcggcggt agccagcttg tggccgaaag ctcgagcggt ctccaacccc aacggatcaa 17100
   aatgacttcg agcgacctcg agcaacgcaa ccgggaacat gcgtgaggtc tgaacgagaa 17160
   cggatttttc tgtagttgaa gggatcggat aacttttcgg ggccacgcga aatgatccat 17220
   ctgccagcat gctttcgaaa tcgtccaacg cgcgccttaa aatcatttgt agcgacttcg 17280
   agggactgta ttgccgaacg aggttgtcat atgttttcga cacttgaggc gcgggcggtc 17340
   gcgctgaaag aaaaacctgg agctttttcg gggacggagg tggactaagg gcatccacag 17400
   ttagcttaag ttgtcgatcg ggactgtaaa tgtgatcggc gacgagaggc tcacgttgct 17460
   ggtctttctc gtcggctttt tcaggcaagt gctggaggtc cagcttctgg ggaacaagtg 17520
   tcgggttggg atggtggatc tcgggtcgag caccagcaag ccgccgtgct tcgccgaccg 17580
   acaatgcggg cttgcgaatt gccatcttca agcctccaag attttgctga tcagtttcga 17640
   aatgaccacg acttcctcca tcgcaatccg aagattcctc tctatgaggc gcatcgtcgg 17700
   atcagttccc gtgtttagta atgtaagatg caacatgccg cgttctttca tcgcggcaaa 17760
   tgcatctctt tcatgcatgg gagacggtac aactggaagg ctctctagcg tctctgacat 17820
   cctgcgttgc gatgttgtca atcggccgac cgggacgcgt tggcgcaaaa cagctgtagg 17880
   aattgccaaa ttttcactca acagcagctc gatgacgtag cggtaggtag atagtgcctc 17940
   atcgatgtcg agcggcgtta gcatggtggg gatcagaagc aggtttgagc tagcgatgat 18000
   tgtgttgttg agctcgctcg agccgccacg cgtatcggcc aacgcataat caaatccttc 18060
   gagctcggca ttttcatagg ctgcttcaag aaggggcatt tcgtcggcgg aatagacttc 18120
   acagcgagga tcccaggtac tgctttgtaa ggcgttttct ctccatcgcg tcagaggccg 18180
   gttttcgtcg gcatcaaaga gggccactcg tttaccgtca tttgccaaag cagcgcaaag 18240
   gcccatgagt gcggtggttt tgccagcacc ccctttgaaa gaacaaaacg tcaaaagttg 18300
   catattctga tcccgcctat cctgtgaaac cggagtgcat ttgtattttt gttcgtataa 18360
   atgtttttgt gattatcgat gagtaaaagc gttgttacac tatttttatt tcacattcgt 18420
   tataagacaa ttgcaaatgt agcaagtata ttcagtattg actgtaaatg tactgttgat 18480
   ttcatattga gcagggctag acttccatcc gtctacccgg gcacatttcg tgctggagta 18540
   tccagacctt ccgctttctt tggaggaagc tatgtcaaaa cacaccagag ccacgtcgag 18600
   tgagactacc atcaaccagc atcgatccct gaaagttgaa gggttcaagg tcgtgagtgc 18660
   ccgtctgcga tcggccgagt atgaaacctt ttcctatcaa gcgcgcctgc tgggactttc 18720
   ggatagtatg gcaattcgcg ttgcggtgcg ccgcatcggg ggctttctcg aaatagatgc 18780
   agacacacga gaaaagatgg aagccatact tcagtccatc ggaatactct caagcaatgt 18840
   atccatgctt ctatctgcct acgccgaaga ccctcgatcg gatctggagg ctgtgcgaga 18900
   tgaacgtatt gcttttggcg aggctttcgc cgccctcgat ggactactgc gctccatttt 18960
   gtccgtatcc cggcgacgga tcgacggtcg ctcgttactg aaaggtgcct tgtagcactt 19020
   gaccacgcac ctgacgggag aaaattggat gcccgatcgc gctcaagtaa tcattcgcat 19080
   tgtgccagga ggtggaacca agacccttca gcagataatc aatcagctgg agtacctgtc 19140
   ccgaaaggga aagctggaac tgcagcgttc agcccggcat ctcgatattc ccgttccgcc 19200
   ggatcaaatc cgtgagcttg cccaaagctg ggttacggag gccgggattt atgacgaaag 19260
   tcagtcagac gatgacaggc aacaagactt aacaacacac attattgtaa gcttccccgc 19320
   aggtaccgac caaaccgcag cttatgaagc aagccgggaa tgggcagccg agatgtttgg 19380
   gtcaggatac gggggtggcc gctataacta tctgacagcc taccacgtcg accgcgatca 19440
   tccacattta catgtcgtgg tcaatcgtcg ggaacttctg gggcaggggt ggctgaaaat 19500
   atccaggcgc catccccagc tgaattatga cggcttacgg aaaaagatgg cagagatttc 19560
   acttcgtcac ggcatagtcc tggatgcgac ttcgcgagca gaaaggggaa tagcagagcg 19620
   accaatcaca tatgctgaat atcgacgcct tgagcggatg caggctcaaa agattcaatt 19680
   cgaagataca gattttgatg agacctcgcc tgaggaagat cgtcgggacc tcagtcaatc 19740
   gttcgatcca tttcgatcgg acgcatctgc cggcgaaccg gaccgtgcaa cccgacatga 19800
   caaacaaccg cttgaaccgc acgcccgttt ccaggagccc gccggctcca gcatcaaagc 19860
   cgacgcacgg atccgcgtac cattggagag cgagcggggt gcccaaccat ccgcgtccaa 19920
   aatccctgta actgggcatt tcgggattga gacttcgtat gtcgctgaag ccagcgtgcc 19980
   caaacaaagc ggcaattccg atacttctcg cccggtgact gacgttgcca tgcacacagt 20040
   cgagcgccag cagcgatcaa aacgacgtca tgacgaggag gcaggtccga gcggagcaaa 20100
   ccgtaaaaga ttgaaggccg cgcaagttga ttccgaggca aatgtcggtg agcccgacgg 20160
   tcgcgatgac agcaacaagg cggctgatcc ggtgtctgct tccatccgta ccgagcaacc 20220
   ggaagcttct ccaacgtgtc cgcgtgaccg tcacgatgga gaattgggag aacgcaaacg 20280
   tgcaagaggt aatcgtcgcg acgatgggcg cggggggacc tagactagta gacaggaagg 20340
   accgaataat ggcaaatggt cagttcacga tacgctctgc tcgcccggcc tccgtcggac 20400
   tgacaggcga acggcgtgga gccgcatccg cctctagctc tgcactgtcc aatgttcaaa 20460
   gagatgttag ggataggctg attccaacta gctcaccaag attaccaaat gcagccatat 20520
   tgcgtgattc ctcgggaaga gcgtcgactg gtctgcggta catggcggct actcttcatt 20580
   ggtctgcgat cgcgccatta tcgctaataa acagcaacga cctggctccg gccgcttatg 20640
   actttgagac gcgaaataac gcaagaaatg tgactgccaa agtcggcagg gcagtccctg 20700
   ttcccaagca aggcgggctc ggcaaaacgc tcgcacccgt accccttagt acacgtatat 20760
   caagggtcaa ttccgaccga agactgcccg ctgacgcaga agaccgccct gaaacgcgcg 20820
   acccccagaa aggacgtggc agtcatggtg cgacgccaac cttacatgaa aagattggaa 20880
   ccgcgtttgc tcgaagattg cgaaagcata cgtactatat tgtttgcagt tgctgccaga 20940
   cccggagcgc gttgacgatg ggtgcaaaga tttcggtgaa gtcatgaact ccagcaagac 21000
   ttcgccccag cgtatgaccc tgagcatcgt atgttcgctg gcagccggtt tttgtgcggc 21060
   cagctgctat gtaacgttcc gccggggctt caacggcgaa gcgatgatga cgttcgacgt 21120
   tttcgctttt tggtatgaga ccccgcttta cttgggttat gccagcaccg tcttctggcg 21180
   tggtttatct gttgtcatct ttacctcgct gatcgttctt tcaagtcagc tcatcatatc 21240
   gctgcgcaat cagaagcatc atgggacagc tcgttgggca gaaattggcg aaatgcggca 21300
   tgctggttat ctgcagcgtt acagtcgcat caaggggccg atctttggaa agacatgtgg 21360
   tcccctttgg ttcggcagtt atttgaccaa tggcgaacag ccacacagtc ttgtcgtcgc 21420
   gccaacgcgt gctggcaaag gcgtcggcat cgtcattcca acgctgttga ccttcaaggg 21480
   ctcggtaatc gcccttgacg tcaagggaga attgtttgaa ctgacgtcca gagcacgcaa 21540
   agcgagcggc gacgcagttt tcaagttctc ccccctagat cctgagcgga agactcattg 21600
   ttacaatccg gtcctggata ttgccgcact tccgcccgaa cgccagttca ctgaaacacg 21660
   ccgtctagct gcgaacctta ttacggctaa gggaaaggga gcagaaggct ttattgacgg 21720
   cgcacgtgac ctgttcgtcg cgggaatcct tacctgcatt gagcgtggca caccaacgat 21780
   tggcgcggta tatgacctat ttgcgcagcc tggcgaaaag tataagcttt ttgcgcaact 21840
   cgcggaggaa agcctaaaca aagaggctca gcgtatcttc gataatatgg cgggcaacga 21900
   cacgaaaatt ctgacatcgt acacctctgt gctgggcgac ggtggactga acctgtgggc 21960
   tgatccgctt atcaaagcag cgacaagccg gtcagacttt tccgtttacg atctccggag 22020
   gaagaagacc tgcatttatc tttgtgtcag tcccaacgat ctggaggtct tggcaccact 22080
   tatgcgcctg atgtttcagc agctcgtgtc aatcttgcag agatcgctgc caggtgaaga 22140
   cgagtgccat gaagttttat ttctcctcga cgaattcaaa cacctgggca agcttgaggc 22200
   catagagacc gcgatcacaa ccatcgccgg ttacagaggc cgctttatgt ttattattca 22260
   aagtctttcg gccttgtcgg gcacatacga tgacgcagga aaacaaaact ttctgagcaa 22320
   tactggcgta caagtattta tggccacggc tgatgacgaa actccaacct acatctcaaa 22380
   agctatcggc gaatatacgt ttaaagcgcg ttcgacctct tacagtcaag ccagaatgtt 22440
   cgaccacaac atccagattt ctgatcaagg tgcagccctt ttgcgccccg aacaagtgcg 22500
   cctgctagac gatcagagtg aaatcgttct catcaaaggg cgacctccac tcaaattacg 22560
   aaaggtgcag tattattccg atcgtacgct gaaaggcctt ttcgaacgcc agatgggctc 22620
   tctgcctgag cccgcaccct tgatgctttc cgactatagc aacgatcaag ttcaatacca 22680
   cttggctccg atagcaaatt ttaatgagga tgctgcaccg caaaacagaa ctgtggccga 22740
   ggaccatgga agtgttaaag tcggtgctga tatccctgaa cgcgtgatgg gaataaatgg 22800
   tgacgaggaa caagccgatg cgggcgagat accgccggaa tcggttgtgc ctccagaatt 22860
   gacgctcgct ctgaccgctc aacagcaatt gttggaccag attattgcac ttcagcaaag 22920
   atcgaggtcc gcaccggcat agcctgcgaa atgatcttaa tggtgcaatt cgtttcaggc 22980
   ggcatgttgc ggttcaacaa agtgtacgcc agatcccaat tggcccttta cgaggtgtcg 23040
   gtatgacagg aaagtcgaaa gttcacataa gaggttcggc tgacgcgctt cctgacgttc 23100
   ctggcggaag tactaccgcc ccttttttaa ccgaaccttc tcgggatcag gttgatgcct 23160
   cgtttgaggt ccaaaccgac tacagccagt ctacttccgt gtcgtttacc tatgatggtg 23220
   ttggacttgg tcctgccgag cgtgcggctt acgagaactg gtgcgaaccg ggccggccca 23280
   cttggaaaga tcttataatc aaggcacgtg tcgatccgat tgacgatgtg acctggctcc 23340
   gagatttaga agaggacacc ccctcaacct tcagatacga agggatgcct ctgggcatcg 23400
   gggaacgaca ggcctacgaa aattggcaag aggacgctca gccgacatgg gaagaccttg 23460
   ttgtcagcgc acgcttgacg gaacttggcc gtccacacgg gattaccggc gagtatacat 23520
   ccctcgcagg atcgaagaat acaagttcaa tttcattgaa gcgaaagcgg agcaacttaa 23580
   ttgatgatga gaattcatcc ggatcgtttt catatgacgg gatgaagctc ggggaagccg 23640
   agcgttctgc atatggtgac tgggccgagg cggagccacc cacgtggaaa gatttggtat 23700
   tgagggcacg cgtttcctcg atcaatgact ctgcttggct tttgattcca caaacatctt 23760
   catcatcatt tgaatacaac ggtgttccct tgggcgagcc ggaacggcag gctctcagac 23820
   aatggcaagg agacgctcag cctacctggg aagatctcgt tgttaacgcg cgtatggcag 23880
   aactttgcca tgctggttgg attgaaggtc aaaaaggttg ctttgaagag cgcggggagg 23940
   ctctgcccgc gtcggaacgc ggttcgcaac gccccattgg tcaacggaca gattcctccg 24000
   attcttttgt gtatgatggc acaaggctcg gagcacctga gcgaactgct tatgaacgct 24060
   ggagtaagag ggaacgcccg acttgggaag atctcatctt agatgcacac caggccagga 24120
   ctgaaagtga cgctgttacg acccaagcga ttggtcagtc gtcctcaccg gttttcttat 24180
   atgaaggaaa gtcgctcgga gacagggaac gaaaggctta cgaaaaatgg cggcagccag 24240
   cccaaccgcg atggcaaaat cttgtagtga acgctcgtct ggcagaaatc gatccctcag 24300
   cctggattgc cgatgagcgc gatccgcttg atgatagcga cgcgcttggt cgcccgtcgt 24360
   acacaagctt gacggataga tcagacgtcc ctttagacga tcaatcaatc tatcgtcgtt 24420
   ccgacctagt aagggagcag gtgccagaat cgtctcaaag gcaattcgca gcatgttcag 24480
   aatctgaaac gaggcctgtg caatggttta ctgcttctgg gtcagatgca aacaatacgg 24540
   aaaatatcac cgccagcgat cccgtcgatc gcacgggtgg agttaagcgg ctaggctcca 24600
   aaagcgacag aaccgttaca gcttctatcc atgacgtgaa ttccagcaca aggcgactgt 24660
   tgcttaacga atttggatcg gaggctccgc gcccttcgcc agaaaagact gttcgcttaa 24720
   gaagcgacaa tattggcacc tatgggagcc ggaaaaatga acgagcgcgg ctcgcgaccg 24780
   aaaccggtgc gtatgagtcg gagcatattt tcgggttcaa ggctgtccac gatactgcga 24840
   gagcgacgaa agagggccgg cgtctcgaaa ggcccatgcc cgcctacctt gaggataagg 24900
   ggcttcatcg ccaacatatt ggcaccggga gaggacggac caaacttgtc gggcgcggat 24960
   ggccggatga cacaagctat cgctcggatc aaagggcaac tctgtcggac cccgttgcgc 25020
   gctcggaagg cgcgacggcc tcaaatgggt atcaattgaa ccaattgggt tacgcgcacc 25080
   aactcgctag cgatggcctg caaagtgaat cgcccgatgg tgttgccttg ccaattcaag 25140
   tggcaacaac gagctacaac tatacagtga gccgcgatcc tgtccttgtt ccgccggata 25200
   aaaacgaagc ccctcaattg ctgcatcttg gtccccgtgg tcaaaccgaa gctgttcttg 25260
   cccgcgaaac agcattgact ggaaaatggc cgactctcga gcgtgagcag caagtgtatc 25320
   gcgagttttt ggccttatat gacgtaaaaa aagatcttga ggccaaatca gtcggcgtaa 25380
   gacggaaaaa aaaagaagtt atttctgcgt tagaccgaac tgcgcgcttg ataagcacgt 25440
   cgccttcgaa agctcgatcc aaagcagaga ctgaaaaagc cattgatgag ctcgatgatc 25500
   gacgagttta tgatccgcgt gatcgagctc aagacaaagc gtttaaacgc tgataagtcg 25560
   ccaatatagt gatcatttgc agtattcgca tcgatcgctg gttgatattc tgccgctggt 25620
   cgaccggctg ctcgtcgcca aaaatgctca cagggatacg atggcctcgg tcaggccgcg 25680
   tgcgtcctgt ctttccagtt cctccctttc agctcgattg tggcatcatt tattgcctgc 25740
   tcattgcagt tgaaacgcga tatccgtttc aagacccggg tatggatggt actttggaga 25800
   tatgagcact tgccagactt cccggtcgga gacttaaaaa ttccgataag aggttacgga 25860
   cgacggacct tataagtgga tcgtctagtg gtggcgccga tcaaaacagt tccgcccccg 25920
   gctttgctca aagtagcaaa gcagctttat ctcgggttgc ggaggatttt ctagaaaacc 25980
   gcaattttgc aggagagaac atatggccat catcaagccg catgtgaaca aaaataggac 26040
   aacctcgccg atagagagac cggagtctct catagaggaa atgagcggca gtcatccgcc 26100
   gagtggtttt accaacctgg atctcgctat gatcgagctg gaggactttg tccatcggtg 26160
   cccgctccca gaagacaatc ttgctggtca gaaggagtga gacgatggat ccgtctagca 26220
   atgagaatgt ctatgtgggt cgcggtcaca acatcgaaaa tgatgatgac actgacccca 26280
   ggcgttggaa gaaggcgaat atcagttcca acaccatctc cgatattcag atgacgaatg 26340
   gcgaagacgt acaatcaggg agccctaccc gaacggaagt tgtaagccca cgtctggatt 26400
   atggatcggt cgactcctcc tccagccttt attctggcag cgagcacgga aatcaagctg 26460
   agattcaaaa agagctgtcc gtcttgttct cgaacatgtc tttgccaggc aacgatcggc 26520
   gcccggacga atacattctc gtgcatcaaa cgggacaaga tgcttttact ggtattgcca 26580
   aaggcaacct cgaccaaatg cccaccaagg cggaatttaa cgcgtgctgc cgtctctaca 26640
   gggacggagc cggtaattac tacccgccac ctctcgcatt cgacaagatt agcgttccag 26700
   agcaactgga ggaaaaatgg gggatgatgg aggcgaagga acgtaacaaa ctgcggtttc 26760
   agtacaagtt ggacgtatgg aatcatgcgc acgctgatat ggggatcacg ggcacagaga 26820
   tcttttatca aacagataag aacataaagc tcgaccggaa ttataaacta agacctgaag 26880
   accgatacgt acaaacagaa aaatacgggc gccgggaaat tcaaaagcga tatcaacacg 26940
   aactccaggc tggttcgctg ctgcccgata ttatgatcaa aactccccaa aatgacatcc 27000
   acttcgtgta caggtttgcc ggcgacaatt acgccaacaa acagttcagc gagtttgaac 27060
   acaccgtcaa gcgcaggtat ggcgacgaga ctgagatcaa attgaagtca aagtcaggca 27120
   ttatgcatga ctcgaaatat ctggaatcct gggaacgggg cagtgcggat attcgcttcg 27180
   cggaattcgt tggggaaaat agagctcaca atcggcagtt tccaactgcg acagtaaata 27240
   tgggacagca gccagacggg cagggcggtt tgacccgcga ccgtcatgtg agcgttgact 27300
   tcctaatgca aagcgcaccc aattcgcctt gggcgcaagc tttgaaaaag ggagaactgt 27360
   gggatcgcgt tcagttgctt gctcgcgacg gcaaccgcta tctgtcgccg cccagattgg 27420
   aatattctga ccctgcacat ttcaccgagt tgatgaaccg ggttggttta cccgcatcga 27480
   tgggtcggca aagccatgcg gctagtatca aattcgaaaa gtttgacgcg caggcagcgg 27540
   ttattgtctt aaatggccca gagttacgtg acattcatga cttgtctcct gaaaaactgc 27600
   aaaatttgtc caccaaagat gtcatcgtcg ccgatcgcaa tgagaatggt cagagaactg 27660
   gcacgtacac cagcgtcgcg gaatatgagc gcttgcagtt aaggctgcca cccgatgcag 27720
   cgggggtgct tggtgaagca actgacaaat attcacgtga tttcgttcgg ccagagccgg 27780
   cgtcgcgtcc aatcagtgac agccgcagga tatacgaaag tcgaccgcgt agccaaagcg 27840
   tcaacagctt ttgacgttcc tgctgccgcg tcaacgagga agctcgtttg acccgggttt 27900
   gccaatgaaa gggctcaatc atggtgaaca ctacaaagaa aagttttgcg aagtcgctta 27960
   cggcagatat gcgccgttct gctcagcgcg ttgtcgagca aatgcgaaaa gcattgatta 28020
   ccgaagaaga ggcgctcaag cggcaagcca gactggagag tcccgatagg aagcgaaagt 28080
   atgctgctga tatggcgata gtcgacaaac tcgacgtagg gtttcgaggc gaaataggct 28140
   ataaaattct tggaaataac cggcttcgag tagacaacca taaagaatta acgcgtgagc 28200
   acggtagact tcgcaaaacc aaaacggttc tgaagcgtaa cccggtgacg caggaagtct 28260
   atttgggttt atatgaaagg aagtcctggt taagtgtcag cagccatttg tatgctgcgg 28320
   acggcacact ccgcatgaag cacgtgaaat acaaagacgg acgttttgag gaaaaatggg 28380
   agcgcgacga aaatggcgac ctgatccgca caaggtacgc caaccgtggc aggctctttc 28440
   aacctgtatc cgagaaaatg ggcgcgccgt atcggagcgg ccctgacgac cggctctatc 28500
   gcgatctaac ccgtcgaaac ggtttcagac gggagacatt cgaacgggac gatcacggaa 28560
   acctcgagcg tatcggcagc aaccatgtcg gcttttccaa gatttcagtg aaggcaccca 28620
   atcgtcaaac ctcccagacg aagattcaaa aacttggtgg cgctttcaac aaatctttta 28680
   ggtcccttct ggacaaggag ggcaatgaaa tgggccgcga tattttgagc catcgacggc 28740
   tctataacaa gcggtctgct gtctacgatg aagctaccgg acaattgaag agtgccaagc 28800
   ataccttcgg caagatctac aggagcgaaa ccgaatatct cagcgcgggc ctcaagaagg 28860
   tttcaaaaaa gatactcggg gtgacggtct accggaaatt tgcggcgctc agcgagcgag 28920
   aatccgaggc tgagagactg cgtagttttg aatccggtgc gcatcgccag atctggcagg 28980
   agcgggcagc gattcccggt tcgcccctcc cggagactga tgacattcat ttcgcacagc 29040
   agtcgcacct agccaaagcc aaccctgatc acgtcgaagc tgacgtcacg cgtgtgacag 29100
   atcaacatgt tgatgttgct ggacaaacat catcgtctcc ccaacggaac ttggaaggat 29160
   ggttagattc tcaatcacga tacaagccag caaacatgct gttgtcaaat ccagaccttc 29220
   aagcgaacgg acctcgccca tacgaagggt tagctcatct caccctccgg cgcgataatg 29280
   aatctgacgg gcacaaggag aatgatcagc ggctgcgaca tttctcccag ccagagccgt 29340
   tggtgttacc gcatcccggg tcgccggaaa taactaaggt gtttggctcg cggggagagc 29400
   cggcacaccc gagtggaaat ctgcacacgg cggttggaga aacggcttgc gaaggaccgg 29460
   tgatgtcttc atcctcggac aatcatcagc cagctccagg acagcaagaa cttttaagtt 29520
   tccttcataa tgcgccagcc ccagtttctg tggcaataca tgatgatcaa gagcgacttg 29580
   cgggggaggc gcccggcggc tctttcagag gtagctcagg gcgaacgagt tcaatgtcgg 29640
   agagtatctt cgacgaagat gtacaagggc atttggtacg ggattattcg atcaatacta 29700
   ctaacgggtt tattgacccg caatcgttgt tcggtgaacc ggacttatcg agaggtccaa 29760
   aatcggggcc agaaattcca tcggaagatt accatttgtc agcttcggaa caggaaaatt 29820
   tgctgaatca attgcttagt gtgccactgc cggttccttc accgaagccc gaatgcgcga 29880
   ggtctatgat tttcgaaggt tcacgttcaa gagagcgttc cacctccaga gggttctaag 29940
   gtggaacgcg tctaggcttg cctatcatca ccacctcatg atggtcgcgg agaccttcga 30000
   agagatatcc gacatcattg gagaagctgg gccggaaact agtgtacctc gcgaatgcat 30060
   ctagatccaa tccaatatgc atggcatgcc gaatccatgt gggagtttat tcttgacaca 30120
   gatatttatg atataataac tgagtaagct taacataagg aggaaaaaca tatgttacgc 30180
   agcagcaacg atgttacgca gcagggcagt cgccctaaaa caaagttagg tggctcaagt 30240
   atgggcatca ttcgcacatg taggctcggc cctgaccaag tcaaatccat gcgggctgct 30300
   cttgatcttt tcggtcgtga gttcggagac gtagccacct actcccaaca tcagccggac 30360
   tccgattacc tcgggaactt gctccgtagt aagacattca tcgcgcttgc tgccttcgac 30420
   caagaagcgg ttgttggcgc tctcgcggct tacgttctgc ccaagtttga gcagccgcgt 30480
   agtgagatct atatctatga tctcgcagtc tccggcgagc accggaggca gggcattgcc 30540
   accgcgctca tcaatctcct caagcatgag gccaacgcgc ttggtgctta tgtgatctac 30600
   gtgcaagcag attacggtga cgatcccgca gtggctctct atacaaagtt gggcatacgg 30660
   gaagaagtga tgcactttga tatcgaccca agtaccgcca cctaataatg tctaacaatt 30720
   cgttcaagcc gacgccgctt cgcggcgcgg cttaactcaa gcgttagatg cactatacgt 30780
   accaatccaa atcggatccc cctgcagggc tgagataggt gcctcactga ttaagcattg 30840
   gtaactgtca gaccaagttt actcatatat actttagatt gatttaaaac ttcattttta 30900
   atttaaaagg atctaggtga agatcctttt tgataatctc atgaccaaaa tcccttaacg 30960
   tgagttttcg ttccactgag cgtcagaccc cgtagaaaag atcaaaggat cttcttgaga 31020
   tccttttttt ctgcgcgtaa tctgctgctt gcaaacaaaa aaaccaccgc taccagcggt 31080
   ggtttgtttg ccggatcaag agctaccaac tctttttccg aaggtaactg gcttcagcag 31140
   agcgcagata ccaaatactg tccttctagt gtagccgtag ttaggccacc acttcaagaa 31200
   ctctgtagca ccgcctacat acctcgctct gctaatcctg ttaccagtgg ctgctgccag 31260
   tggcgataag tcgtgtctta ccgggttgga ctcaagacga tagttaccgg ataaggcgca 31320
   gcggtcgggc tgaacggggg gttcgtgcac acagcccagc ttggagcgaa cgacctacac 31380
   cgaactgaga tacctacagc gtgagctatg agaaagcgcc acgcttcccg aagggagaaa 31440
   ggcggacagg tatccggtaa gcggcagggt cggaacagga gagcgcacga gggagcttcc 31500
   agggggaaac gcctggtatc tttatagtcc tgtcgggttt cgccacctct gacttgagcg 31560
   tcgatttttg tgatgctcgt caggggggcg gagcctatgg aaaaacgcca gcaacgcggc 31620
   ctttttacgg ttcctggcct tttgctggcc ttttgctcac atgttctttc ctgcgttatc 31680
   ccctgattct gtggataacc gtattaccgc ctttgagtga gctgataccg ctcgccgcag 31740
   ccgaacgacc gagcgcagcg agtcagtgag cgaggaagcg gaagagcgcc tgatgcggta 31800
   ttttctcctt acgcatctgt gcggtatttc acaccgcata tggtgcactc tcagtacaat 31860
   ctgctctgat gccgcatagt taagccagta tacactccgc tatcgctacg tgactgggtc 31920
   atggctgcgc cccgacacc 31939
<210> 37
   <211> 3671
   <212> DNA
   <213> Escherichia coli
<400> 37
   tcagcctgcc gccttgggcc gggtgatgtc gtacttgccc gccgcgaact cggttaccgt 60
   ccagcccagc gcgaccagct ccggcaacgc ctcgcgcacc cgctggcggc gcttgcgcat 120
   ggtcgaacca ctggcctctg acggccagac atagccgcac aaggtatcta tggaagcctt 180
   gccggttttg ccggggtcga tccagccaca cagccgctgg tgcagcaggc gggcggtttc 240
   gctgtccagc gcccgcacct cgtccatgct gatgcgcaca tgctggccgc cacccatgac 300
   ggcctgcgcg atcaaggggt tcagggccac gtacaggcgc ccgtccgcct cgtcgctggc 360
   gtactccgac agcagccgaa acccctgccg cttgcggcca ttctgggcga tgatggatac 420
   cttccaaagg cgctcgatgc agtcctgtat gtgcttgagc gccccaccac tatcgacctc 480
   tgccccgatt tcctttgcca gcgcccgata gctacctttg accaccatgg catcagcggt 540
   gacggcctcc cacttgggtt ccaggaacag ccggagctgc cgtccgcctt cggtcttggg 600
   ttccgggcca agcactaggc cattaggccc agccatggcc accagccctt gcaggatgcg 660
   cagatcatca gcgcccagcg gctccgggcc gctgaactcg atccgcttgc cgtcgccgta 720
   gtcatacgtc acgtccagct tgctgcgctt gcgctcgccc cgcttgaggg cacggaacag 780
   gccgggggcc agacagtgcg ccgggtcgtg ccggacgtgg ctgaggctgt gcttgttctt 840
   aggcttcacc acggggcacc cccttgctct tgcgctgcct ctccagcacg gcgggcttga 900
   gcaccccgcc gtcatgccgc ctgaaccacc gatcagcgaa cggtgcgcca tagttggcct 960
   tgctcacacc gaagcggacg aagaaccggc gctggtcgtc gtccacaccc cattcctcgg 1020
   cctcggcgct ggtcatgctc gacaggtagg actgccagcg gatgttatcg accagtaccg 1080
   agctgccccg gctggcctgc tgctggtcgc ctgcgcccat catggccgcg cccttgctgg 1140
   catggtgcag gaacacgata gagcacccgg tatcggcggc gatggcctcc atgcgaccga 1200
   tgacctgggc catggggccg ctggcgtttt cttcctcgat gtggaaccgg cgcagcgtgt 1260
   ccagcaccat caggcggcgg ccctcggcgg cgcgcttgag gccgtcgaac cactccgggg 1320
   ccatgatgtt gggcaggctg ccgatcagcg gctggatcag caggccgtca gccacggctt 1380
   gccgttcctc ggcgctgagg tgcgccccaa gggcgtgcag gcggtgatga atggcggtgg 1440
   gcgggtcttc ggcgggcagg tagatcaccg ggccggtggg cagttcgccc acctccagca 1500
   gatccggccc gcctgcaatc tgtgcggcca gttgcagggc cagcatggat ttaccggcac 1560
   caccgggcga caccagcgcc ccgaccgtac cggccaccat gttgggcaaa acgtagtcca 1620
   gcggtggcgg cgctgctgcg aacgcctcca gaatattgat aggcttatgg gtagccattg 1680
   attgcctcct ttgcaggcag ttggtggtta ggcgctggcg gggtcactac ccccgccctg 1740
   cgccgctctg agttcttcca ggcactcgcg cagcgcctcg tattcgtcgt cggtcagcca 1800
   gaacttgcgc tgacgcatcc ctttggcctt catgcgctcg gcatatcgcg cttggcgtac 1860
   agcgtcaggg ctggccagca ggtcgccggt ctgcttgtcc ttttggtctt tcatatcagt 1920
   caccgagaaa cttgccgggg ccgaaaggct tgtcttcgcg gaacaaggac aaggtgcagc 1980
   cgtcaaggtt aaggctggcc atatcagcga ctgaaaagcg gccagcctcg gccttgtttg 2040
   acgtataacc aaagccaccg ggcaaccaat agcccttgtc acttttgatc aggtagaccg 2100
   accctgaagc gcttttttcg tattccataa aacccccttc tgtgcgtgag tactcatagt 2160
   ataacaggcg tgagtaccaa cgcaagcact acatgctgaa atctggcccg cccctgtcca 2220
   tgcctcgctg gcggggtgcc ggtgcccgtg ccagctcggc ccgcgcaagc tggacgctgg 2280
   gcagacccat gaccttgctg acggtgcgct cgatgtaatc cgcttcgtgg ccgggcttgc 2340
   gctctgccag cgctgggctg gcctcggcca tggccttgcc gatttcctcg gcactgcggc 2400
   cccggctggc cagcttctgc gcggcgataa agtcgcactt gctgaggtca tcaccgaagc 2460
   gcttgaccag cccggccatc tcgctgcggt actcgtccag cgccgtgcgc cggtggcggc 2520
   taagctgccg ctcgggcagt tcgaggctgg ccagcctgcg ggccttctcc tgctgccgct 2580
   gggcctgctc gatctgctgg ccagcctgct gcaccagcgc cgggccagcg gtggcggtct 2640
   tgcccttgga ttcacgcagc agcacccacg gctgataacc ggcgcgggtg gtgtgcttgt 2700
   ccttgcggtt ggtgaagccc gccaagcggc catagtggcg gctgtcggcg ctggccgggt 2760
   cggcgtcgta ctcgctggcc agcgtccggg caatctgccc ccgaagttca ccgcctgcgg 2820
   cgtcggccac cttgacccat gcctgatagt tcttcgggct ggtttccact accagggcag 2880
   gctcccggcc ctcggctttc atgtcatcca ggtcaaactc gctgaggtcg tccaccagca 2940
   ccagaccatg ccgctcctgc tcggcgggcc tgatatacac gtcattgccc tgggcattca 3000
   tccgcttgag ccatggcgtg ttctggagca cttcggcggc tgaccattcc cggttcatca 3060
   tctggccggt ggtggcgtcc ctgacgccga tatcgaagcg ctcacagccc atggccttga 3120
   gctgtcggcc tatggcctgc aaagtcctgt cgttcttcat cgggccacca agcgattccc 3180
   acacattata cgagccggaa gcataaagtg tagctagatc cgaaggatga gccgggctga 3240
   atgatcgacc gagacaggcc ctgcggggct gcacacgcgc ccccaccctt cgggtagggg 3300
   gaaaggccgc taaagcggct aaaagcgctc cagcgtattt ctgcggggtt tggtgtgggg 3360
   tttagcgggc tttgcccgcc tttccccctg ccgcgcagcg gtggggcggt gtgtagccta 3420
   gcgcagcgaa tagaccagct atccggcctc tggccgggca tattgggcaa gggcagcagc 3480
   gccccacaag ggcgctgata accgcgccta gtggattatt cttagataat catggatgga 3540
   tttttccaac accccgccag cccccgcccc tgctgggttt gcaggtttgg gggcgtgaca 3600
   gttattgcag gggttcgtga cagttattgc aggggggcgt gacagttatt gcaggggttc 3660
   gtgacagtta g 3671
<210> 38
   <211> 15028
   <212> DNA
   <213> Escherichia coli
<400> 38
   ctagcgtttg caatgcacca ggtcatcatt gacccaggcg tgttccacca ggccgctgcc        60
   tcgcaactct tcgcaggctt cgccgacctg ctcgcgccac ttcttcacgc gggtggaatc       120
   cgatccgcac atgaggcgga aggtttccag cttgagcggg tacggctccc ggtgcgagct       180
   gaaatagtcg aacatccgtc gggccgtcgg cgacagcttg cggtacttct cccatatgaa       240
   tttcgtgtag tggtcgccag caaacagcac gacgatttcc tcgtcgatca ggacctggca 300
   acgggacgtt ttcttgccac ggtccaggac gcggaagcgg tgcagcagcg acaccgattc 360
   caggtgccca acgcggtcgg acgtgaagcc catcgccgtc gcctgtaggc gcgacaggca 420
   ttcctcggcc ttcgtgtaat accggccatt gatcgaccag cccaggtcct ggcaaagctc 480
   gtagaacgtg aaggtgatcg gctcgccgat aggggtgcgc ttcgcgtact ccaacacctg 540
   ctgccacacc agttcgtcat cgtcggcccg cagctcgacg ccggtgtagg tgatcttcac 600
   gtccttgttg acgtggaaaa tgaccttgtt ttgcagcgcc tcgcgcggga ttttcttgtt 660
   gcgcgtggtg aacagggcag agcgggccgt gtcgtttggc atcgctcgca tcgtgtccgg 720
   ccacggcgca atatcgaaca aggaaagctg catttccttg atctgctgct tcgtgtgttt 780
   cagcaacgcg gcctgcttgg cctcgctgac ctgttttgcc aggtcctcgc cggcggtttt 840
   tcgcttcttg gtcgtcatag ttcctcgcgt gtcgatggtc atcgacttcg ccaaacctgc 900
   cgcctcctgt tcgagacgac gcgaacgctc cacggcggcc gatggcgcgg gcagggcagg 960
   gggagccagt tgcacgctgt cgcgctcgat cttggccgta gcttgctgga ccatcgagcc 1020
   gacggactgg aaggtttcgc ggggcgcacg catgacggtg cggcttgcga tggtttcggc 1080
   atcctcggcg gaaaaccccg cgtcgatcag ttcttgcctg tatgccttcc ggtcaaacgt 1140
   ccgattcatt caccctcctt gcgggattgc cccgactcac gccggggcaa tgtgccctta 1200
   ttcctgattt gacccgcctg gtgccttggt gtccagataa tccaccttat cggcaatgaa 1260
   gtcggtcccg tagaccgtct ggccgtcctt ctcgtacttg gtattccgaa tcttgccctg 1320
   cacgaatacc agcgacccct tgcccaaata cttgccgtgg gcctcggcct gagagccaaa 1380
   acacttgatg cggaagaagt cggtgcgctc ctgcttgtcg ccggcatcgt tgcgccactc 1440
   ttcattaacc gctatatcga aaattgcttg cggcttgtta gaattgccat gacgtacctc 1500
   ggtgtcacgg gtaagattac cgataaactg gaactgatta tggctcatat cgaaagtctc 1560
   cttgagaaag gagactctag tttagctaaa cattggttcc gctgtcaaga actttagcgg 1620
   ctaaaatttt gcgggccgcg accaaaggtg cgaggggcgg cttccgctgt gtacaaccag 1680
   atatttttca ccaacatcct tcgtctgctc gatgagcggg gcatgacgaa acatgagctg 1740
   tcggagaggg caggggtttc aatttcgttt ttatcagact taaccaacgg taaggccaac 1800
   ccctcgttga aggtgatgga ggccattgcc gacgccctgg aaactcccct acctcttctc 1860
   ctggagtcca ccgaccttga ccgcgaggca ctcgcggaga ttgcgggtca tcctttcaag 1920
   agcagcgtgc cgcccggata cgaacgcatc agtgtggttt tgccgtcaca taaggcgttt 1980
   atcgtaaaga aatggggcga cgacacccga aaaaagctgc gtggaaggct ctgacgccaa 2040
   gggttagggc ttgcacttcc ttctttagcc gctaaaacgg ccccttctct gcgggccgtc 2100
   ggctcgcgca tcatatcgac atcctcaacg gaagccgtgc cgcgaatggc atcgggcggg 2160
   tgcgctttga cagttgtttt ctatcagaac ccctacgtcg tgcggttcga ttagctgttt 2220
   gtcttgcagg ctaaacactt tcggtatatc gtttgcctgt gcgataatgt tgctaatgat 2280
   ttgttgcgta ggggttactg aaaagtgagc gggaaagaag agtttcagac catcaaggag 2340
   cgggccaagc gcaagctgga acgcgacatg ggtgcggacc tgttggccgc gctcaacgac 2400
   ccgaaaaccg ttgaagtcat gctcaacgcg gacggcaagg tgtggcacga acgccttggc 2460
   gagccgatgc ggtacatctg cgacatgcgg cccagccagt cgcaggcgat tatagaaacg 2520
   gtggccggat tccacggcaa agaggtcacg cggcattcgc ccatcctgga aggcgagttc 2580
   cccttggatg gcagccgctt tgccggccaa ttgccgccgg tcgtggccgc gccaaccttt 2640
   gcgatccgca agcgcgcggt cgccatcttc acgctggaac agtacgtcga ggcgggcatc 2700
   atgacccgcg agcaatacga ggtcattaaa agcgccgtcg cggcgcatcg aaacatcctc 2760
   gtcattggcg gtactggctc gggcaagacc acgctcgtca acgcgatcat caatgaaatg 2820
   gtcgccttca acccgtctga gcgcgtcgtc atcatcgagg acaccggcga aatccagtgc 2880
   gccgcagaga acgccgtcca ataccacacc agcatcgacg tctcgatgac gctgctgctc 2940
   aagacaacgc tgcgtatgcg ccccgaccgc atcctggtcg gtgaggtacg tggccccgaa 3000
   gcccttgatc tgttgatggc ctggaacacc gggcatgaag gaggtgccgc caccctgcac 3060
   gcaaacaacc ccaaagcggg cctgagccgg ctcgccatgc ttatcagcat gcacccggat 3120
   tcaccgaaac ccattgagcc gctgattggc gaggcggttc atgtggtcgt ccatatcgcc 3180
   aggaccccta gcggccgtcg agtgcaagaa attctcgaag ttcttggtta cgagaacggc 3240
   cagtacatca ccaaaaccct gtaaggagta tttccaatga caacggctgt tccgttccgt 3300
   ctgaccatga atcgcggcat tttgttctac cttgccgtgt tcttcgttct cgctctcgcg 3360
   ttatccgcgc atccggcgat ggcctcggaa ggcaccggcg gcagcttgcc atatgagagc 3420
   tggctgacga acctgcgcaa ctccgtaacc ggcccggtgg ccttcgcgct gtccatcatc 3480
   ggcatcgtcg tcgccggcgg cgtgctgatc ttcggcggcg aactcaacgc cttcttccga 3540
   accctgatct tcctggttct ggtgatggcg ctgctggtcg gcgcgcagaa cgtgatgagc 3600
   accttcttcg gtcgtggtgc cgaaatcgcg gccctcggca acggggcgct gcaccaggtg 3660
   caagtcgcgg cggcggatgc cgtgcgtgcg gtagcggctg gacggctcgc ctaatcatgg 3720
   ctctgcgcac gatccccatc cgtcgcgcag gcaaccgaga aaacctgttc atgggtggtg 3780
   atcgtgaact ggtgatgttc tcgggcctga tggcgtttgc gctgattttc agcgcccaag 3840
   agctgcgggc caccgtggtc ggtctgatcc tgtggttcgg ggcgctctat gcgttccgaa 3900
   tcatggcgaa ggccgatccg aagatgcggt tcgtgtacct gcgtcaccgc cggtacaagc 3960
   cgtattaccc ggcccgctcg accccgttcc gcgagaacac caatagccaa gggaagcaat 4020
   accgatgatc caagcaattg cgattgcaat cgcgggcctc ggcgcgcttc tgttgttcat 4080
   cctctttgcc cgcatccgcg cggtcgatgc cgaactgaaa ctgaaaaagc atcgttccaa 4140
   ggacgccggc ctggccgatc tgctcaacta cgccgctgtc gtcgatgacg gcgtaatcgt 4200
   gggcaagaac ggcagcttta tggctgcctg gctgtacaag ggcgatgaca acgcaagcag 4260
   caccgaccag cagcgcgaag tagtgtccgc ccgcatcaac caggccctcg cgggcctggg 4320
   aagtgggtgg atgatccatg tggacgccgt gcggcgtcct gctccgaact acgcggagcg 4380
   gggcctgtcg gcgttccctg accgtctgac ggcagcgatt gaagaagagc gctcggtctt 4440
   gccttgctcg tcggtgatgt acttcaccag ctccgcgaag tcgctcttct tgatggagcg 4500
   catggggacg tgcttggcaa tcacgcgcac cccccggccg ttttagcggc taaaaaagtc 4560
   atggctctgc cctcgggcgg accacgccca tcatgacctt gccaagctcg tcctgcttct 4620
   cttcgatctt cgccagcagg gcgaggatcg tggcatcacc gaaccgcgcc gtgcgcgggt 4680
   cgtcggtgag ccagagtttc agcaggccgc ccaggcggcc caggtcgcca ttgatgcggg 4740
   ccagctcgcg gacgtgctca tagtccacga cgcccgtgat tttgtagccc tggccgacgg 4800
   ccagcaggta ggccgacagg ctcatgccgg ccgccgccgc cttttcctca atcgctcttc 4860
   gttcgtctgg aaggcagtac accttgatag gtgggctgcc cttcctggtt ggcttggttt 4920
   catcagccat ccgcttgccc tcatctgtta cgccggcggt agccggccag cctcgcagag 4980
   caggattccc gttgagcacc gccaggtgcg aataagggac agtgaagaag gaacacccgc 5040
   tcgcgggtgg gcctacttca cctatcctgc ccggctgacg ccgttggata caccaaggaa 5100
   agtctacacg aaccctttgg caaaatcctg tatatcgtgc gaaaaaggat ggatataccg 5160
   aaaaaatcgc tataatgacc ccgaagcagg gttatgcagc ggaaaagcgc tgcttccctg 5220
   ctgttttgtg gaatatctac cgactggaaa caggcaaatg caggaaatta ctgaactgag 5280
   gggacaggcg agagacgatg ccaaagagct acaccgacga gctggccgag tgggttgaat 5340
   cccgcgcggc caagaagcgc cggcgtgatg aggctgcggt tgcgttcctg gcggtgaggg 5400
   cggatgtcga ggcggcgtta gcgtccggct atgcgctcgt caccatttgg gagcacatgc 5460
   gggaaacggg gaaggtcaag ttctcctacg agacgttccg ctcgcacgcc aggcggcaca 5520
   tcaaggccaa gcccgccgat gtgcccgcac cgcaggccaa ggctgcggaa cccgcgccgg 5580
   cacccaagac gccggagcca cggcggccga agcagggggg caaggctgaa aagccggccc 5640
   ccgctgcggc cccgaccggc ttcaccttca acccaacacc ggacaaaaag gatctactgt 5700
   aatggcgaaa attcacatgg ttttgcaggg caagggcggg gtcggcaagt cggccatcgc 5760
   cgcgatcatt gcgcagtaca agatggacaa ggggcagaca cccttgtgca tcgacaccga 5820
   cccggtgaac gcgacgttcg agggctacaa ggccctgaac gtccgccggc tgaacatcat 5880
   ggccggcgac gaaattaact cgcgcaactt cgacaccctg gtcgagctga ttgcgccgac 5940
   caaggatgac gtggtgatcg acaacggtgc cagctcgttc gtgcctctgt cgcattacct 6000
   catcagcaac caggtgccgg ctctgctgca agaaatgggg catgagctgg tcatccatac 6060
   cgtcgtcacc ggcggccagg ctctcctgga cacggtgagc ggcttcgccc agctcgccag 6120
   ccagttcccg gccgaagcgc ttttcgtggt ctggctgaac ccgtattggg ggcctatcga 6180
   gcatgagggc aagagctttg agcagatgaa ggcgtacacg gccaacaagg cccgcgtgtc 6240
   gtccatcatc cagattccgg ccctcaagga agaaacctac ggccgcgatt tcagcgacat 6300
   gctgcaagag cggctgacgt tcgaccaggc gctggccgat gaatcgctca cgatcatgac 6360
   gcggcaacgc ctcaagatcg tgcggcgcgg cctgtttgaa cagctcgacg cggcggccgt 6420
   gctatgagcg accagattga agagctgatc cgggagattg cggccaagca cggcatcgcc 6480
   gtcggccgcg acgacccggt gctgatcctg cataccatca acgcccggct catggccgac 6540
   agtgcggcca agcaagagga aatccttgcc gcgttcaagg aagagctgga agggatcgcc 6600
   catcgttggg gcgaggacgc caaggccaaa gcggagcgga tgctgaacgc ggccctggcg 6660
   gccagcaagg acgcaatggc gaaggtaatg aaggacagcg ccgcgcaggc ggccgaagcg 6720
   atccgcaggg aaatcgacga cggccttggc cgccagctcg cggccaaggt cgcggacgcg 6780
   cggcgcgtgg cgatgatgaa catgatcgcc ggcggcatgg tgttgttcgc ggccgccctg 6840
   gtggtgtggg cctcgttatg aatcgcagag gcgcagatga aaaagcccgg cgttgccggg 6900
   ctttgttttt gcgttagctg ggcttgtttg acaggcccaa gctctgactg cgcccgcgct 6960
   cgcgctcctg ggcctgtttc ttctcctgct cctgcttgcg catcagggcc tggtgccgtc 7020
   gggctgcttc acgcatcgaa tcccagtcgc cggccagctc gggatgctcc gcgcgcatct 7080
   tgcgcgtcgc cagttcctcg atcttgggcg cgtgaatgcc catgccttcc ttgatttcgc 7140
   gcaccatgtc cagccgcgtg tgcagggtct gcaagcgggc ttgctgttgg gcctgctgct 7200
   gctgccaggc ggcctttgta cgcggcaggg acagcaagcc gggggcattg gactgtagct 7260
   gctgcaaacg cgcctgctga cggtctacga gctgttctag gcggtcctcg atgcgctcca 7320
   cctggtcatg ctttgcctgc acgtagagcg caagggtctg ctggtaggtc tgctcgatgg 7380
   gcgcggattc taagagggcc tgctgttccg tctcggcctc ctgggccgcc tgtagcaaat 7440
   cctcgccgct gttgccgctg gactgcttta ctgccgggga ctgctgttgc cctgctcgcg 7500
   ccgtcgtcgc agttcggctt gcccccactc gattgactgc ttcatttcga gccgcagcga 7560
   tgcgatctcg gattgcgtca acggacgggg cagcgcggag gtgtccggct tctccttggg 7620
   tgagtcggtc gatgccatag ccaaaggttt ccttccaaaa tgcgtccatt gctggaccgt 7680
   gtttctcatt gatgcccgca agcatcttcg gcttgaccgc caggtcaagc gcgccttcat 7740
   gggcggtcat gacggacgcc gccatgacct tgccgccgtt gttctcgatg tagccgcgta 7800
   atgaggcaat ggtgccgccc atcgtcagcg tgtcatcgac aacgatgtac ttctggccgg 7860
   ggatcacctc cccctcgaaa gtcgggttga acgccaggcg atgatctgaa ccggctccgg 7920
   ttcgggcgac cttctcccgc tgcacaatgt ccgtttcgac ctcaaggcca aggcggtcgg 7980
   ccagaacgac cgccatcatg gccggaatct tgttgttccc cgccgcctcg acggcgagga 8040
   ctggaacgat gcggggcttg tcgtcgccga tcagcgtctt gagctgggca acagtgtcgt 8100
   ccgaaatcag gcgctcgacc aaattaagcg ccgcttccgc gtcgccctgc ttcgcagcct 8160
   ggtattcagg ctcgttggtc aaagaaccaa ggtcgccgtt gcgaaccacc ttcgggaagt 8220
   ctccccacgg tgcgcgctcg gctctgctgt agctgctcaa gacgcctccc tttttagccg 8280
   ctaaaactct aacgagtgcg cccgcgactc aacttgacgc tttcggcact tacctgtgcc 8340
   ttgccacttg cgtcataggt gatgcttttc gcactcccga tttcaggtac tttatcgaaa 8400
   tctgaccggg cgtgcattac aaagttcttc cccacctgtt ggtaaatgct gccgctatct 8460
   gcgtggacga tgctgccgtc gtggcgctgc gacttatcgg ccttttgggc catatagatg 8520
   ttgtaaatgc caggtttcag ggccccggct ttatctacct tctggttcgt ccatgcgcct 8580
   tggttctcgg tctggacaat tctttgccca ttcatgacca ggaggcggtg tttcattggg 8640
   tgactcctga cggttgcctc tggtgttaaa cgtgtcctgg tcgcttgccg gctaaaaaaa 8700
   agccgacctc ggcagttcga ggccggcttt ccctagagcc gggcgcgtca aggttgttcc 8760
   atctatttta gtgaactgcg ttcgatttat cagttacttt cctcccgctt tgtgtttcct 8820
   cccactcgtt tccgcgtcta gccgacccct caacatagcg gcctcttctt gggctgcctt 8880
   tgcctcttgc cgcgcttcgt cacgctcggc ttgcaccgtc gtaaagcgct cggcctgcct 8940
   ggccgcctct tgcgccgcca acttcctttg ctcctggtgg gcctcggcgt cggcctgcgc 9000
   cttcgctttc accgctgcca actccgtgcg caaactctcc gcttcgcgcc tggtggcgtc 9060
   gcgctcgccg cgaagcgcct gcatttcctg gttggccgcg tccagggtct tgcggctctc 9120
   ttctttgaat gcgcgggcgt cctggtgagc gtagtccagc tcggcgcgca gctcctgcgc 9180
   tcgacgctcc acctcgtcgg cccgctgcgt cgccagcgcg gcccgctgct cggctcctgc 9240
   cagggcggtg cgtgcttcgg ccagggcttg ccgctggcgt gcggccagct cggccgcctc 9300
   ggcggcctgc tgctctagca atgtaacgcg cgcctgggct tcttccagct cgcgggcctg 9360
   cgcctcgaag gcgtcggcca gctccccgcg cacggcttcc aactcgttgc gctcacgatc 9420
   ccagccggct tgcgctgcct gcaacgattc attggcaagg gcctgggcgg cttgccagag 9480
   ggcggccacg gcctggttgc cggcctgctg caccgcgtcc ggcacctgga ctgccagcgg 9540
   ggcggcctgc gccgtgcgct ggcgtcgcca ttcgcgcatg ccggcgctgg cgtcgttcat 9600
   gttgacgcgg gcggccttac gcactgcatc cacggtcggg aagttctccc ggtcgccttg 9660
   ctcgaacagc tcgtccgcag ccgcaaaaat gcggtcgcgc gtctctttgt tcagttccat 9720
   gttggctccg gtaattggta agaataataa tactcttacc taccttatca gcgcaagagt 9780
   ttagctgaac agttctcgac ttaacggcag gttttttagc ggctgaaggg caggcaaaaa 9840
   aagccccgca cggtcggcgg gggcaaaggg tcagcgggaa ggggattagc gggcgtcggg 9900
   cttcttcatg cgtcggggcc gcgcttcttg ggatggagca cgacgaagcg cgcacgcgca 9960
   tcgtcctcgg ccctatcggc ccgcgtcgcg gtcaggaact tgtcgcgcgc taggtcctcc 10020
   ctggtgggca ccaggggcat gaactcggcc tgctcgatgt aggtccactc catgaccgca 10080
   tcgcagtcga ggccgcgttc cttcaccgtc tcttgcaggt cgcggtacgc ccgctcgttg 10140
   agcggctggt aacgggccaa ttggtcgtaa atggctgtcg gccatgagcg gcctttcctg 10200
   ttgagccagc agccgacgac gaagccggca atgcaggccc ctggcacaac caggccgacg 10260
   ccgggggcag gggatggcag cagctcgcca accaggaacc ccgccgcgat gatgccgatg 10320
   ccggtcaacc agcccttgaa actatccggc cccgaaacac ccctgcgcat tgcctggatg 10380
   ctgcgccgga tagcttgcaa catcaggagc cgtttctttt gttcgtcagt catggtccgc 10440
   cctcaccagt tgttcgtatc ggtgtcggac gaactgaaat cgcaagagct gccggtatcg 10500
   gtccagccgc tgtccgtgtc gctgctgccg aagcacggcg aggggtccgc gaacgccgca 10560
   gacggcgtat ccggccgcag cgcatcgccc agcatggccc cggtcagcga gccgccggcc 10620
   aggtagccca gcatggtgct gttggtcgcc ccggccacca gggccgacgt gacgaaatcg 10680
   ccgtcattcc ctctggattg ttcgctgctc ggcggggcag tgcgccgcgc cggcggcgtc 10740
   gtggatggct cgggttggct ggcctgcgac ggccggcgaa aggtgcgcag cagctcgtta 10800
   tcgaccggct gcggcgtcgg ggccgccgcc ttgcgctgcg gtcggtgttc cttcttcggc 10860
   tcgcgcagct tgaacagcat gatcgcggaa accagcagca acgccgcgcc tacgcctccc 10920
   gcgatgtaga acagcatcgg attcattctt cggtcctcct tgtagcggaa ccgttgtctg 10980
   tgcggcgcgg gtggcccgcg ccgctgtctt tggggatcag ccctcgatga gcgcgaccag 11040
   tttcacgtcg gcaaggttcg cctcgaactc ctggccgtcg tcctcgtact tcaaccaggc 11100
   atagccttcc gccggcggcc gacggttgag gataaggcgg gcagggcgct cgtcgtgctc 11160
   gacctggacg atggcctttt tcagcttgtc cgggtccggc tccttcgcgc ccttttcctt 11220
   ggcgtcctta ccgtcctggt cgccgtcctc gccgtcctgg ccgtcgccgg cctccgcgtc 11280
   acgctcggca tcagtctggc cgttgaaggc atcgacggtg ttgggatcgc ggcccttctc 11340
   gtccaggaac tcgcgcagca gcttgaccgt gccgcgcgtg atttcctggg tgtcgtcgtc 11400
   aagccacgcc tcgacttcct ccgggcgctt cttgaaggcc gtcaccagct cgttcaccac 11460
   ggtcacgtcg cgcacgcggc cggtgttgaa cgcatcggcg atcttctccg gcaggtccag 11520
   cagcgtgacg tgctgggtga tgaacgccgg cgacttgccg atttccttgg cgatatcgcc 11580
   tttcttcttg cccttcgcca gctcgcggcc aatgaagtcg gcaatttcgc gcggggtcag 11640
   ctcgttgcgt tgcaggttct cgataacctg gtcggcttcg ttgtagtcgt tgtcgatgaa 11700
   cgccgggatg gacttcttgc cggcccactt cgagccacgg tagcggcggg cgccgtgatt 11760
   gatgatatag cggcccggct gctcctggtt ctcgcgcacc gaaatgggtg acttcacccc 11820
   gcgctctttg atcgtggcac cgatttccgc gatgctctcc ggggaaaagc cggggttgtc 11880
   ggccgtccgc ggctgatgcg gatcttcgtc gatcaggtcc aggtccagct cgatagggcc 11940
   ggaaccgccc tgagacgccg caggagcgtc caggaggctc gacaggtcgc cgatgctatc 12000
   caaccccagg ccggacggct gcgccgcgcc tgcggcttcc tgagcggccg cagcggtgtt 12060
   tttcttggtg gtcttggctt gagccgcagt cattgggaaa tctccatctt cgtgaacacg 12120
   taatcagcca gggcgcgaac ctctttcgat gccttgcgcg cggccgtttt cttgatcttc 12180
   cagaccggca caccggatgc gagggcatcg gcgatgctgc tgcgcaggcc aacggtggcc 12240
   ggaatcatca tcttggggta cgcggccagc agctcggctt ggtggcgcgc gtggcgcgga 12300
   ttccgcgcat cgaccttgct gggcaccatg ccaaggaatt gcagcttggc gttcttctgg 12360
   cgcacgttcg caatggtcgt gaccatcttc ttgatgccct ggatgctgta cgcctcaagc 12420
   tcgatggggg acagcacata gtcggccgcg aagagggcgg ccgccaggcc gacgccaagg 12480
   gtcggggccg tgtcgatcag gcacacgtcg aagccttggt tcgccagggc cttgatgttc 12540
   gccccgaaca gctcgcgggc gtcgtccagc gacagccgtt cggcgttcgc cagtaccggg 12600
   ttggactcga tgagggcgag gcgcgcggcc tggccgtcgc cggctgcggg tgcggtttcg 12660
   gtccagccgc cggcagggac agcgccgaac agcttgcttg catgcaggcc ggtagcaaag 12720
   tccttgagcg tgtaggacgc attgccctgg gggtccaggt cgatcacggc aacccgcaag 12780
   ccgcgctcga aaaagtcgaa ggcaagatgc acaagggtcg aagtcttgcc gacgccgcct 12840
   ttctggttgg ccgtgaccaa agttttcatc gtttggtttc ctgttttttc ttggcgtccg 12900
   cttcccactt ccggacgatg tacgcctgat gttccggcag aaccgccgtt acccgcgcgt 12960
   acccctcggg caagttcttg tcctcgaacg cggcccacac gcgatgcacc gcttgcgaca 13020
   ctgcgcccct ggtcagtccc agcgacgttg cgaacgtcgc ctgtggcttc ccatcgacta 13080
   agacgccccg cgctatctcg atggtctgct gccccacttc cagcccctgg atcgcctcct 13140
   ggaactggct ttcggtaagc cgtttcttca tggataacac ccataatttg ctccgcgcct 13200
   tggttgaaca tagcggtgac agccgccagc acatgagaga agtttagcta aacatttctc 13260
   gcacgtcaac acctttagcc gctaaaactc gtccttggcg taacaaaaca aaagcccgga 13320
   aaccgggctt tcgtctcttg ccgcttatgg ctctgcaccc ggctccatca ccaacaggtc 13380
   gcgcacgcgc ttcactcggt tgcggatcga cactgccagc ccaacaaagc cggttgccgc 13440
   cgccgccagg atcgcgccga tgatgccggc cacaccggcc atcgcccacc aggtcgccgc 13500
   cttccggttc cattcctgct ggtactgctt cgcaatgctg gacctcggct caccataggc 13560
   tgaccgctcg atggcgtatg ccgcttctcc ccttggcgta aaacccagcg ccgcaggcgg 13620
   cattgccatg ctgcccgccg ctttcccgac cacgacgcgc gcaccaggct tgcggtccag 13680
   accttcggcc acggcgagct gcgcaaggac ataatcagcc gccgacttgg ctccacgcgc 13740
   ctcgatcagc tcttgcactc gcgcgaaatc cttggcctcc acggccgcca tgaatcgcgc 13800
   acgcggcgaa ggctccgcag ggccggcgtc gtgatcgccg ccgagaatgc ccttcaccaa 13860
   gttcgacgac acgaaaatca tgctgacggc tatcaccatc atgcagacgg atcgcacgaa 13920
   cccgctgaat tgaacacgag cacggcaccc gcgaccacta tgccaagaat gcccaaggta 13980
   aaaattgccg gccccgccat gaagtccgtg aatgccccga cggccgaagt gaagggcagg 14040
   ccgccaccca ggccgccgcc ctcactgccc ggcacctggt cgctgaatgt cgatgccagc 14100
   acctgcggca cgtcaatgct tccgggcgtc gcgctcgggc tgatcgccca tcccgttact 14160
   gccccgatcc cggcaatggc aaggactgcc agcgctgcca tttttggggt gaggccgttc 14220
   gcggccgagg ggcgcagccc ctggggggat gggaggcccg cgttagcggg ccgggagggt 14280
   tcgagaaggg ggggcacccc ccttcggcgt gcgcggtcac gcgcacaggg cgcagccctg 14340
   gttaaaaaca aggtttataa atattggttt aaaagcaggt taaaagacag gttagcggtg 14400
   gccgaaaaac gggcggaaac ccttgcaaat gctggatttt ctgcctgtgg acagcccctc 14460
   aaatgtcaat aggtgcgccc ctcatctgtc agcactctgc ccctcaagtg tcaaggatcg 14520
   cgcccctcat ctgtcagtag tcgcgcccct caagtgtcaa taccgcaggg cacttatccc 14580
   caggcttgtc cacatcatct gtgggaaact cgcgtaaaat caggcgtttt cgccgatttg 14640
   cgaggctggc cagctccacg tcgccggccg aaatcgagcc tgcccctcat ctgtcaacgc 14700
   cgcgccgggt gagtcggccc ctcaagtgtc aacgtccgcc cctcatctgt cagtgagggc 14760
   caagttttcc gcgaggtatc cacaacgccg gcggccgcgg tgtctcgcac acggcttcga 14820
   cggcgtttct ggcgcgtttg cagggccata gacggccgcc agcccagcgg cgagggcaac 14880
   cagcccggtg agcgtcggaa aggcgctgga agccccgtag cgacgcggag aggggcgaga 14940
   caagccaagg gcgcaggctc gatgcgcagc acgacatagc cggttctcgc aaggacgaga 15000
   atttccctgc ggtgcccctc aagtgtca 15028
<210> 39
   <211> 1011
   <212> DNA
   <213> Escherichia coli
<400> 39
   atgcgctcac gcaactggtc cagaaccttg accgaacgca gcggtggtaa cggcgcagtg 60
   gcggttttca tggcttgtta tgactgtttt tttggggtac agtctatgcc tcgggcatcc 120
   aagcagcaag cgcgttacgc cgtgggtcga tgtttgatgt tatggagcag caacgatgtt 180
   acgcagcagg gcagtcgccc taaaacaaag ttaaacatca tgagggaagc ggtgatcgcc 240
   gaagtatcga ctcaactatc agaggtagtt ggcgtcatcg agcgccatct cgaaccgacg 300
   ttgctggccg tacatttgta cggctccgca gtggatggcg gcctgaagcc acacagtgat 360
   attgatttgc tggttacggt gacggtaagg cttgatgaaa caacgcggcg agctttgatc 420
   aacgaccttt tggaaacttc ggcttcccct ggagagagcg agattctccg cgctgtagaa 480
   gtcaccattg ttgtgcacga cgacatcatt ccgtggcgtt atccagctaa gcgcgaactg 540
   caatttggag aatggcagcg caatgacatt cttgcaggta tcttcgagcc agccacgatc 600
   gacattgatc tggctatctt gctgacaaaa gcaagagaac atagcgttgc cttggtaggt 660
   ccagcggcgg aggaactctt tgatccggtt cctgaacagg atctatttga ggcgctaaat 720
   gaaaccttaa cgctatggaa ctcgccgccc gactgggctg gcgatgagcg aaatgtagtg 780
   cttacgttgt cccgcatttg gtacagcgca gtaaccggca aaatcgcgcc gaaggatgtc 840
   gctgccgact gggcaatgga gcgcctgccg gcccagtatc agcccgtcat acttgaagct 900
   agacaggctt atcttggaca agaagaagat cgcttggcct cgcgcgcaga tcagttggaa 960
   gaatttgtcc actacgtgaa aggcgagatc accaaggtag tcggcaaata a 1011
<210> 40
   <211> 816
   <212> DNA
   <213> Escherichia coli
<400> 40
   atgagccata ttcaacggga aacgtcttgc tcgaggccgc gattaaattc caacatggat 60
   gctgatttat atgggtataa atgggctcgc gataatgtcg ggcaatcagg tgcgacaatc 120
   tatcgattgt atgggaagcc cgatgcgcca gagttgtttc tgaaacatgg caaaggtagc 180
   gttgccaatg atgttacaga tgagatggtc agactaaact ggctgacgga atttatgcct 240
   cttccgacca tcaagcattt tatccgtact cctgatgatg catggttact caccactgcg 300
   atccccggga aaacagcatt ccaggtatta gaagaatatc ctgattcagg tgaaaatatt 360
   gttgatgcgc tggcagtgtt cctgcgccgg ttgcattcga ttcctgtttg taattgtcct 420
   tttaacagcg atcgcgtatt tcgtctcgct caggcgcaat cacgaatgaa taacggtttg 480
   gttgatgcga gtgattttga tgacgagcgt aatggctggc ctgttgaaca agtctggaaa 540
   gaaatgcata agcttttgcc attctcaccg gattcagtcg tcactcatgg tgatttctca 600
   cttgataacc ttatttttga cgaggggaaa ttaataggtt gtattgatgt tggacgagtc 660
   ggaatcgcag accgatacca ggatcttgcc atcctatgga actgcctcgg tgagttttct 720
   ccttcattac agaaacggct ttttcaaaaa tatggtattg ataatcctga tatgaataaa 780
   ttgcagtttc atttgatgct cgatgagttt ttctaa 816
<210> 41
   <211> 795
   <212> DNA
   <213> Escherichia coli
<400> 41
   atggttgaac aagatggatt gcacgcaggt tctccggccg cttgggtgga gaggctattc 60
   ggctatgact gggcacaaca gacaatcggc tgctctgatg ccgccgtgtt ccggctgtca 120
   gcgcaggggc gcccggttct ttttgtcaag accgacctgt ccggtgccct gaatgaactg 180
   caggacgagg cagcgcggct atcgtggctg gccacgacgg gcgttccttg cgcagctgtg 240
   ctcgacgttg tcactgaagc gggaagggac tggctgctat tgggcgaagt gccggggcag 300
   gatctcctgt catctcacct tgctcctgcc gagaaagtat ccatcatggc tgatgcaatg 360
   cggcggctgc atacgcttga tccggctacc tgcccattcg accaccaagc gaaacatcgc 420
   atcgagcgag cacgtactcg gatggaagcc ggtcttgtcg atcaggatga tctggacgaa 480
   gagcatcagg ggctcgcgcc agccgaactg ttcgccaggc tcaaggcgcg catgcccgac 540
   ggcgatgatc tcgtcgtgac ccatggcgat gcctgcttgc cgaatatcat ggtggaaaat 600
   ggccgctttt ctggattcat cgactgtggc cggctgggtg tggcggaccg ctatcaggac 660
   atagcgttgg ctacccgtga tattgctgaa gagcttggcg gcgaatgggc tgaccgcttc 720
   ctcgtgcttt acggtatcgc cgctcccgat tcgcagcgca tcgccttcta tcgccttctt 780
   gacgagttct tctga 795
<210> 42
   <211> 492
   <212> DNA
   <213> Agrobacterium tumefaciens
<400> 42
   atgcactcaa ccagattgcg aacaaacgaa gtcgcggtca cgctacccga agaaggcgac 60
   gcatcgcttt tctttgttgg aaagatccgg accccttgga aaagtcgagc tgacactcct 120
   cggcagggca gtgaatccgg gccaccatgc acacttgaaa tttcagaacc gtgggccgtg 180
   gcgctaaagg gggtcgaggc gtattcgcaa cttgaggttc tatattggtt gcacgagtca 240
   ccacgcgata tcgtcctact aagtcccgct gacgatggag agattcacgg tgctttctcc 300
   ctacgagcac ctgtgaggcc caatccgatc ggaacatcga ttgtgaaagt ggagagggta 360
   tctgggaact cgattgttgt acgtggtctt gattgcctgg atggaacacc gctgttggat 420
   ataaaaccgg accgctcgct atcaaagccg ctagccccgg tgaggaaaag gttctcgatt 480
   cctgccgtct ga 492
<210> 43
   <211> 1269
   <212> DNA
   <213> Agrobacterium tumefaciens
<400> 43
   atgattgcga acagctcttc cgatgtctct atggccgacc agaagtttct aaatgtcgcg 60
   aagtcaaatg aaatcgatcc cgacgccgtt cctataagca gacttgattc tgaaggtcac 120
   agtatttttg cagaatggcg accgaagcgc ccgtttcttc ggagagaaga tggcgtcttt 180
   ctcgttctcc gcgcagacca tatctttctg ctgggcaccg atccacgcac ccggcaaata 240
   gaaactgagc tcatgctgaa tcgaggcgtc aaagctggtg ccgtttttga ctttataggt 300
   cacagcatgt tgttttcgaa cggtgaaacg catgggaagc ggcgctcagg tctttcaaag 360
   gcgttctcat tccgcatggt tgaagcgtta cgcccggaga ttgcgaagat aacggagcgt 420
   ttgtgggacg aactacaaaa agttgacgat ttcaatttta ctgaaatgta cgcgtcgcaa 480
   ttgcctgcgc tgacgatcgc aagtgtcctt ggcttgccgt ctgaggacac gccgtttttc 540
   acacgacttg tttataaggt ttcccgctgc ttgagcccgt cgtggcgaga tgaggaattc 600
   gaggagattg aagcttccgc tatcgagctt caggattacg ttcggggcgt gatcgcggat 660
   agtggccgtc ggatgaggga tgattttctc tcgcgctact tgaaggcggt acgggaagcc 720
   ggaacgcttt cgcccattga ggagatcatg caactcatgc ttatcatact cgctgggagc 780
   gatacaacgc gcactgcaat ggttatggtg acggctcttg tgctccaaaa tcccgcgctc 840
   tggtcttctt taactggcaa tcaatcctat gtcgcagccg ccgtggagga agggctccga 900
   ttcgagccgc cagttggctc ttttccgcgg ttggccctcg aggatatcga tctggatgga 960
   tacgtgttgc caaagggaag cctcctcgcg ctcagtgtca tgtctggcct gcgagacgaa 1020
   aaacactacg agaaccctca tctttttgat gttgggcgtc aacaaatgcg ttggcacctc 1080
   gggtttggcg cgggagttca tcgttgcctc ggcgagacgt tggctcggat tgaattgcaa 1140
   gaaggacttg gaacactttt gcgccgcgcg ccgaatctta cggtggtagg tgactggcca 1200
   cggatgatgg gtcacggagg catccggcgc gccaccggca tgacggtcaa attgagcgtc 1260
   gaccggtga 1269
<210> 44
   <211> 1224
   <212> DNA
   <213> Agrobacterium tumefaciens
<400> 44
   gtggaagaga ggcgcgtatc aatatcaagc atcacttgga gatttccaat gccatgtgcg 60
   ccagtggacg acgtgacgac catcgatgac ctaacactcg atccctatcc gatttatcgc 120
   agaatgcgcg tgcaaaaccc ggtggtacac gtcgcctcgg tcagacggac gtttctaacg 180
   aaggcctgcg atactaagat ggtgaaggat gatccttcac gctttagttc tgatgatccc 240
   aacacgccga tgaagccggc gcttcaggct catactttga tgcgtaagga tggagttgaa 300
   cacgctcggg aacgaatggc catgactaga gcattcgcac ccaaagcgat cgcggagcat 360
   tgggtgccga tctatcgtga catcgtgaac gagtatctag atcgccttcc gcgcggagac 420
   accgtggatc tttttgcaga gatctgtggt ccggttgctg ctcgcatact ggcacatatt 480
   ctaggaattt gtgaggcatc agatgtcgag atgatccgct ggtcgcaacg gcttattgac 540
   ggagccggca acttcggatg gcgacccgag ctctttgagc gatcggacga ggccaatacg 600
   gagatgaact gcctcttcaa cgacttggtc gaaaaacacc gatccgcgcc gaacccctcg 660
   gcttttgcga tcatgttgaa tgcacctgat cctatacccc taagccagat ctatgccaac 720
   atcaagatcg ccatcggcgg aggggtcaac gagcctcgtg acgctctcgg cacgatcctc 780
   tatggattgc tgaccaatcc ggagcaactc gaggaggtca agagacagca atgctggggg 840
   caggccttcg aggaagggct gcgctgggtt gctccgattc aagcgagttc gcgtctcgtg 900
   cgggaggaaa cggaaattcg tggctttatc gttccgaagg gcgacattgt gatgactatt 960
   caggcctccg ccaatcgtga tgaagatgtc tttgaggatg gagaaagctt caatgtcttt 1020
   cgcccgaaga atgcgcatca gtcctttggc tccggcccgc atcactgccc aggcgcgcaa 1080
   atctcgcggc aaaccgtcgg cgcgatcatg ctgcccatac tgttcgatcg atttccggat 1140
   atgatattgc ccaccctgaa attagtgcag tggcgcggct tcggcttccg cgggcccatt 1200
   aatctacctg tgacactaag atga 1224
<210> 45
   <211> 438
   <212> DNA
   <213> Agrobacterium tumefaciens
<400> 45
   atgaaacgta tcagtacaat ccttgtcggc gtatttttgg ctgcgccggt ttatgcagcc 60
   gacaatattc atacattagg gacgttgtct gaaattgaac tagcgctcac tgccggaaag 120
   ccggtgaacg tcacggttga tttaagtttg tgtgctcccg gggtggccga tactccggcg 180
   acgaaaacgc aaggaggcat gcgcatagat gcgtatcgaa taacaactga cggcaccctt 240
   gcgtttgcag atcagcactt caccattgac cgtgacggta aacccataac tcaatttatt 300
   cgttatcaga ttcggtcaaa cggtgacgcc gatttcacga tggttacatt caacatgcca 360
   acatatgagc gaaaaggtac cagcttggcc tacaagtgcg cgatcgacca cgggttgagt 420
   tttcgtactc cacagtga 438
<210> 46
   <211> 1134
   <212> DNA
   <213> Agrobacterium tumefaciens
<400> 46
   atggatcatc gaaatcgccc gctggacgag agacagcagc actgggttaa cagcgtttgg 60
   gagaggctct gtcgaagcgt cgcattcgcc caacatccag attccgcccg cctgccaatc 120
   gcttcatatc aaagtgacat cgcaacaagg ctgagagaac acagtgatct cagttcgttc 180
   gaaacacgtc ccgaatatat gcgagagttt tcgaacaagt tttcatggaa tttaaagcgc 240
   gacatttctt ttgtggttaa tcgcaagagt aagaacggtg aattccgaga agatgcgttt 300
   gaagagctgt tttgcgaaag gataacgtct gtccatcatt ctcttcagga ggcgatcacg 360
   gcacggtttg aatccgccgt cggaaaatcg aatgacgaag gcgatgtacg ttctcgcgat 420
   gtttcgacat tttacgctcg cactgctggg gagagttcta caaccaagcg attgaacatc 480
   agcacaaacc cacgaggcgt ggtgccgagt gctgacgtcg tcaatatctc tgaacagcag 540
   gcacagtctg tcaagtatcg gcgtcctaag tttctcgttg aggtctcaaa gcacgtctat 600
   agaccaatcg aacagtataa tcggatggtc ttcgatcgag tcagatttcc aaacatcact 660
   cgtgaccggc gtaagttgaa tgtattgatc cgcacggaag acggcactgg ctacgaaaca 720
   cctagctcgt taaaaacgcg cctgggatcg gagcagcata atctcttcac caagacaatg 780
   aacgagggtc agccacccca tagtctcgcg tccacgaaga ctggtaaact gcctatagtt 840
   ttggttgacg cgggaaatgg tgcaaccttt atcgccggcg agaaattccc tttgttggac 900
   aacggacgcc ggataccaac gactatttct gccgatagaa ttttagtcag aaatgaaaat 960
   gggactttca gcgagctttg tcagcgtttg gctggaaaca ccgaactatt gcttccacca 1020
   gaagcgatag ttcaactggg actgcagcgt caacaagctg tcaatcgagc tcgtgccaga 1080
   gaagcatcgg agttggagca acgaactcgc gaacattcct ctggaagagt ttga 1134
<210> 47
   <211> 588
   <212> DNA
   <213> Agrobacterium tumefaciens
<400> 47
   atgagagatg atccaggagt tgctcgagcg ctttccctct cgttacaaga taagcacgac 60
   attttcgaga cccagatcat tccggcaatc ggtgcgcggg ggccgtctgt tgcaaagcca 120
   actttcaccg ttgtatgcgg ccagcagggt gccggaaaga gcaccttagt tcgccaaatc 180
   aagagtagaa cgggcggtga aagcacccag cgaataatag ctgatgatct taatgcatac 240
   attcctggta acaatgcagc ccttctgcag ggctcgcatg cgttagagcg agcgaattcg 300
   acggcagtga cagaatggta tcatcaatta tttgatcgca gtatcactaa tagatataat 360
   attatactgg agtcttgcta tccgccaaat cattacgcgt cgctcttaga taaggcgcgc 420
   tccaatggat acagaacaga actaaatatt atcgcgacgg acaggataac gagctttaca 480
   gctattcacg atcgctttga aagggcactg gcgaactcgt ttattgcctc aaccgtattg 540
   ccagatgtcg aaactcacga tcattattat tcatttggcc acgtgtag 588
<210> 48
   <211> 633
   <212> DNA
   <213> Agrobacterium tumefaciens
<400> 48
   atggctaaaa atgatgctta taagctaatt atatttgatt ttgatggtac attggccgac 60
   agcggcgcct ggatgatacg cgctctgagg gaaatgtccg aacgtcaccg attcgtatcg 120
   cccagcgaca ctcagattga atatctgcgt ggcctttcag tttgccaggt tctgaagtgg 180
   atgagagtac cggtgtggcg cattcctctg atcgtgagag acttgcgtaa actggctcgc 240
   gaagccacgt tcgatatgtt ccctaggaca gaaaaggtct tgtttctcct ccaagatcaa 300
   ggggtcgagc ttgcaatctt aagttcgaac agcgttgaaa atattcagcg cgtgctcggg 360
   ccattggaga gatactttgc ttatatagag ggcagctcgc caatatttgg gaagggcaaa 420
   aggatcaata agatacttcg tcggtttgaa aggtcccgga atgaagtgct tcttgtcgga 480
   gatgaggtac gtgatattga ggcagcgatg agtcaaaacg ttgcctccgc gggagttacg 540
   tggggttatg ctaagagaga ggctttggcg tgcagcaatc ccacgcacat cctagaagat 600
   attgaggaac taatagactt gaaacctctc taa 633
<210> 49
   <211> 213
   <212> DNA
   <213> Agrobacterium tumefaciens
<400> 49
   atgaaaacgt cgaccaaaac aatgcttgtc tacgccgcga taattttcac tccaggattt 60
   atatattttc cactatataa aattttgctc actctttata gtgaagattc aggagataca 120
   tatttttgtc agttctatac ggagttctca tttttttgga taatgggatt actggttttt 180
   gccttgtcaa cgatgcttaa aaagaagcct tag 213
<210> 50
   <211> 2101
   <212> DNA
   <213> Salmonella tymphimurium
<400> 50
   gacagtaaga cgggtaagcc tgttgatgat accgctgcct tactgggtgc attagccagt 60
   ctgaatgacc tgtcacggga taatccgaag tggtcagact ggaaaatcag agggcaggaa 120
   ctgctgaaca gcaaaaagtc agatagcacc acatagcaga cccgccataa aacgccctga 180
   gaagcccgtg acgggctttt cttgtattat gggtagtttc cttgcatgaa tccataaaag 240
   gcgcctgtag tgccatttac ccccattcac tgccagagcc gtgagcgcag cgaactgaat 300
   gtcacgaaaa agacagcgac tcaggtgcct gatggtcgga gacaaaagga atattcagcg 360
   atttgcccga gcttgcgagg gtgctactta agcctttagg gttttaaggt ctgttttgta 420
   gaggagcaaa cagcgtttgc gacatccttt tgtaatactg cggaactgac taaagtagtg 480
   agttatacac agggctggga tctattcttt ttatcttttt ttattctttc tttattctat 540
   aaattataac cacttgaata taaacaaaaa aaacacacaa aggtctagcg gaatttacag 600
   agggtctagc agaatttaca agttttccag caaaggtcta gcagaattta cagataccca 660
   caactcaaag gaaaaggact agtaattatc attgactagc ccatctcaat tggtatagtg 720
   attaaaatca cctagaccaa ttgagatgta tgtctgaatt agttgttttc aaagcaaatg 780
   aactagcgat tagtcgctat gacttaacgg agcatgaaac caagctaatt ttatgctgtg 840
   tggcactact caaccccacg attgaaaacc ctacaaggaa agaacggacg gtatcgttca 900
   cttataacca atacgctcag atgatgaaca tcagtaggga aaatgcttat ggtgtattag 960
   ctaaagcaac cagagagctg atgacgagaa ctgtggaaat caggaatcct ttggttaaag 1020
   gctttgagat tttccagtgg acaaactatg ccaagttctc aagcgaaaaa ttagaattag 1080
   tttttagtga agagatattg ccttatcttt tccagttaaa aaaattcata aaatataatc 1140
   tggaacatgt taagtctttt gaaaacaaat actctatgag gatttatgag tggttattaa 1200
   aagaactaac acaaaagaaa actcacaagg caaatataga gattagcctt gatgaattta 1260
   agttcatgtt aatgcttgaa aataactacc atgagtttaa aaggcttaac caatgggttt 1320
   tgaaaccaat aagtaaagat ttaaacactt acagcaatat gaaattggtg gttgataagc 1380
   gaggccgccc gactgatacg ttgattttcc aagttgaact agatagacaa atggatctcg 1440
   taaccgaact tgagaacaac cagataaaaa tgaatggtga caaaatacca acaaccatta 1500
   catcagattc ctacctacat aacggactaa gaaaaacact acacgatgct ttaactgcaa 1560
   aaattcagct caccagtttt gaggcaaaat ttttgagtga catgcaaagt aagtatgatc 1620
   tcaatggttc gttctcatgg ctcacgcaaa aacaacgaac cacactagag aacatactgg 1680
   ctaaatacgg aaggatctga ggttcttatg gctcttgtat ctatcagtga agcatcaaga 1740
   ctaacaaaca aaagtagaac aactgttcac cgttacatat caaagggaaa actgtccata 1800
   tgcacagatg aaaacggtgt aaaaaagata gatacatcag agcttttacg agtttttggt 1860
   gcattcaaag ctgttcacca tgaacagatc gacaatgtaa cagatgaaca gcatgtaaca 1920
   cctaatagaa caggtgaaac cagtaaaaca aagcaactag aacatgaaat tgaacacctg 1980
   agacaacttg ttacagctca acagtcacac atagacagcc tgaaacaggc gatgctgctt 2040
   atcgaatcaa agctgccgac aacacgggag ccagtgacgc ctcccgtggg gaaaaaatca 2100
   t 2101
<210> 51
   <211> 1554
   <212> DNA
   <213> Escherichia coli
<400> 51
   atggaataga ctggatggag gcggataaag ttgcaggacc acttctgcgc tcggcccttc      60
   cggctggctg gtttattgct gataaatctg gagccggtga gcgtgggtct cgcggtatca     120
   ttgcagcact ggggccagat ggtaagccct cccgtatcgt agttatctac acgacgggga     180
   gtcaggcaac tatggatgaa cgaaatagac agatcgctga gataggtgcc tcactgatta     240
   agcattggta actgtcagac caagtttact catatatact ttagattgat ttaaaacttc     300
   atttttaatt taaaaggatc taggtgaaga tcctttttga taatctcatg accaaaatcc     360
   cttaacgtga gttttcgttc cactgagcgt cagacccctt aataagatga tcttcttgag     420
   atcgttttgg tctgcgcgta atctcttgct ctgaaaacga aaaaaccgcc ttgcagggcg     480
   gtttttcgaa ggttctctga gctaccaact ctttgaaccg aggtaactgg cttggaggag     540
   cgcagtcacc aaaacttgtc ctttcagttt agccttaacc ggcgcatgac ttcaagacta     600
   actcctctaa atcaattacc agtggctgct gccagtggtg cttttgcatg tctttccggg     660
   ttggactcaa gacgatagtt accggataag gcgcagcggt cggactgaac ggggggttcg     720
   tgcatacagt ccagcttgga gcgaactgcc tacccggaac tgagtgtcag gcgtggaatg     780
   agacaaacgc ggccataaca gcggaatgac accggtaaac cgaaaggcag gaacaggaga     840
   gcgcacgagg gagccgccag gggaaacgcc tggtatcttt atagtcctgt cgggtttcgc     900
   caccactgat ttgagcgtca gatttcgtga tgcttgtcag gggggcggag cctatggaaa     960
   aacggctttg ccgcggccct ctcacttccc tgttaagtat cttcctggca tcttccagga    1020
   aatctccgcc ccgttcgtaa gccatttccg ctcgccgcag tcgaacgacc gagcgtagcg    1080
   agtcagtgag cgaggaagcg gaatatatcc tgtatcacat attctgctga cgcaccggtg    1140
   cagccttttt tctcctgcca catgaagcac ttcactgaca ccctcatcag tgccaacata    1200
   gtaagccagt atacactccg ctagcgctga ggtctgcctc gtgaagaagg tgttgctgac    1260
   tcataccagg cctgaatcgc cccatcatcc agccagaaag tgagggagcc acggttgatg    1320
   agagctttgt tgtaggtgga ccagttggtg attttgaact tttgctttgc cacggaacgg    1380
   tctgcgttgt cgggaagatg cgtgatctga tccttcaact cagcaaaagt tcgatttatt    1440
   caacaaagcc acgttgtgtc tcaaaatctc tgatgttaca ttgcacaaga taaaaatata    1500
   tcatcatgaa caataaaact gtctgcttac ataaacagta atacaagggg tgtt          1554
<210> 52
   <211> 1576
   <212> DNA
   <213> Escherichia coli
<400> 52
   gatcgctagt ttgttttgac tccatccatt agggcttcta aaacgccttc taaggccatg 60
   tcagccgtta agtgttcctg tgtcactgaa aattgctttg agaggctcta agggcttctc 120
   agtgcgttac atccctggct tgttgtccac aaccgttaaa ccttaaaagc tttaaaagcc 180
   ttatatattc ttttttttct tataaaactt aaaaccttag aggctattta agttgctgat 240
   ttatattaat tttattgttc aaacatgaga gcttagtacg tgaaacatga gagcttagta 300
   cgttagccat gagagcttag tacgttagcc atgagggttt agttcgttaa acatgagagc 360
   ttagtacgtt aaacatgaga gcttagtacg tgaaacatga gagcttagta cgtactatca 420
   acaggttgaa ctgctgatct tcagatctag cttaaaacag gtggcttttt aatcatcttt 480
   gccaagcatg gcgcgggttt ggggtaatat agcgactcat aaaagcgtta aacatgagtg 540
   gatagtacgt tgctaaaaca tgagataaaa attgactctc atgttattgg cgttaagata 600
   tacagaatga tgaggttttt ttatgagact caaggtcatg atggacgtga acaaaaaaac 660
   gaaaattcgc caccgaaacg agctaaatca caccctggct caacttcctt tgcccgcaaa 720
   gcgagtgatg tatatggcgc ttgctcccat tgatagcaaa gaacctcttg aacgagggcg 780
   agttttcaaa attagggctg aagaccttgc agcgctcgcc aaaatcaccc catcgcttgc 840
   ttatcgacaa ttaaaagagg gtggtaaatt acttggtgcc agcaaaattt cgctaagagg 900
   ggatgatatc attgctttag ctaaagagct taacctgccc tttactgcta aaaactcccc 960
   tgaagagtta gatcttaaca ttattgagtg gatagcttat tcaaatgatg aaggatactt 1020
   gtctttaaaa ttcaccagaa ccatagaacc atatatctct agccttattg ggaaaaaaaa 1080
   taaattcaca acgcaattgt taacggcaag cttacgctta agtagccagt attcatcttc 1140
   tctttatcaa cttatcagga agcattactc taattttaag aagaaaaatt attttattat 1200
   ttccgttgat gagttaaagg aagagttaac agcttatact tttgataaag atggaaatat 1260
   tgagtacaaa taccctgact ttcctatttt taaaagggat gtgttaaata aagccattgc 1320
   tgaaattaaa aagaaaacag aaatatcgtt tgttggcttc actgttcatg aaaaagaagg 1380
   aagaaaaatt agtaagctga agttcgaatt tgtcgttgat gaagatgaat tttctggcga 1440
   taaagatgat gaagcttttt ttatgaattt atctgaagct gatgcagctt ttctcaaggt 1500
   atttgatgaa accgtacctc ccaaaaaagc taaggggtga tatatggcta aaatttacga 1560
   tttccctcaa ggagcc 1576
<210> 53
   <211> 2400
   <212> DNA
   <213> Escherichia coli
<400> 53
   cacctaaacg aaggccgggc cactcacccg gccttttttg tacgctcata gggcagaaca 60
   aaccaacgtt ttatctatac cgctacaggg tatttaattc ctatttaatc tgcgctagaa 120
   tgaggcatgt ttaaccgaat ctgacgtttt ccctgcaaat gccaaaatac tatgcctatc 180
   tccgggtttc gcgtgacggc caagacccgg aaaaccaaaa atacggtttg ctcgaatacg 240
   cgaacgccaa aggcttcgcg ccgctacaga tcgaggaaga aattgccagc agagcaaagg 300
   actggcgcaa gcgcaagctc ggagcaatca tcgaaaaggc cgagcgtggc gacgtgctac 360
   tgacgccgga gattacgcgc attgccggtt ccgccctcgc cgccttggaa attctcaaag 420
   cggcgagcga gcgcggccta atcgtccatg tgaccaaaca gaagatcatc atggacggca 480
   gcctacaaag cgacatcatg gcaaccgtgc ttggcttggc tgcacagatc gagcggcatt 540
   tcattcaggc acgtaccacc gaggcgctac aagtcgccag agagcgcggc aagacgctcg 600
   ggcgacccaa gggcagcaaa tcgagcgcct tgaagctgga cagccgtatt gatgaagtac 660
   aggcatacgt gaaccttggc ttgccgcaaa gtcgcgcagc cgagttgtta ggcgtcagcc 720
   ctcacacctt gcgcctgttc atcaaacgcc ggaacatcaa acccacaaac actagaccaa 780
   ccatcaccat gccggggagg gaacaacatg cctaagaaca acaaagcccc cggccatcgt 840
   atcaacgaga tcatcaagac gagcctcgcg ctcgaaatgg aggatgcccg cgaagctggc 900
   ttagtcggct acatggcccg ttgccttgtg caagcgacca tgccccacac cgaccccaag 960
   accagctact ttgagcgcac caatggcatc gtcaccttgt cgatcatggg caagccgagc 1020
   atcggcctgc cctacggttc tatgccgcgc accttgcttg cttggatatg caccgaggcc 1080
   gtgcgaacga aagaccccgt gttgaacctt ggccggtcgc aatcggaatt tctacaaagg 1140
   ctcggaatgc acaccgatgg ccgttacacg gccacccttc gcaatcaggc gcaacgcctg 1200
   ttttcatcca tgatttcgct tgccggcgag caaggcaatg acttcggcat tgagaacgtc 1260
   gtcattgcca agcgcgcttt tctattctgg aatcccaagc ggccagaaga tcgggcgcta 1320
   tgggatagca ccctcaccct cacaggcgat ttcttcgagg aagtcacccg ctcaccggtt 1380
   cctatccgaa tcgactacct gcatgccttg cggcagtctc cgcttgcgat ggacatttac 1440
   acgtggctga cctatcgcgt gttcctgttg cgggccaagg gccgcccctt cgtgcaaatc 1500
   ccttgggtcg ccctgcaagc gcaattcggc tcatcctatg gcagccgcgc acgcaactcg 1560
   cccgaactgg acgataaggc ccgagagcgg gcagagcggg cagcactcgc cagcttcaaa 1620
   tacaacttca aaaagcgcct acgcgaagtg ttgattgtct atcccgaggc aagcgactgc 1680
   atcgaagatg acggcgaatg cctgcgcatc aaatccacac gcctgcatgt cacccgcgca 1740
   cccggcaagg gcgctcgcat cggcccccct ccgacttgac caggccaacg ctacgcttgg 1800
   cttggtcaag ccttcccatc caacagcccg ccgtcgagcg ggctttttta tccccggaag 1860
   cctgtggata gagggtagtt atccacgtga aaccgctaat gccccgcaaa gccttgattc 1920
   acggggcttt ccggcccgct ccaaaaacta tccacgtgaa atcgctaatc agggtacgtg 1980
   aaatcgctaa tcggagtacg tgaaatcgct aataaggtca cgtgaaatcg ctaatcaaaa 2040
   aggcacgtga gaacgctaat agccctttca gatcaacagc ttgcaaacac ccctcgctcc 2100
   ggcaagtagt tacagcaagt agtatgttca attagctttt caattatgaa tatatatatc 2160
   aattattggt cgcccttggc ttgtggacaa tgcgctacgc gcaccggctc cgcccgtgga 2220
   caaccgcaag cggttgccca ccgtcgagcg ccagcgcctt tgcccacaac ccggcggccg 2280
   caacagatcg ttttataaat tttttttttt gaaaaagaaa aagcccgaaa ggcggcaacc 2340
   tctcgggctt ctggatttcc gatcaacgca ggagtcgttc ggaaagtagc tgttccagaa 2400
<210> 54
   <211> 599
   <212> DNA
   <213> Artificial sequence
<220>
   <223> Synthetic construct
<400> 54
   cggcgttgtg gatacctcgc ggaaaacttg gccctcactg acagatgagg ggcggacgtt 60
   gacacttgag gggccgactc acccggcgcg gcgttgacag atgaggggca ggctcgattt 120
   cggccggcga cgtggagctg gccagcctcg caaatcggcg aaaacgcctg attttacgcg 180
   agtttcccac agatgatgtg gacaagcctg gggataagtg ccctgcggta ttgacacttg 240
   aggggcgcga ctactgacag atgaggggcg cgatccttga cacttgaggg gcagagtgct 300
   gacagatgag gggcgcacct attgacattt gaggggctgt ccacaggcag aaaatccagc 360
   atttgcaagg gtttccgccc gtttttcggc caccgctaac ctgtctttta acctgctttt 420
   aaaccaatat ttataaacct tgtttttaac cagggctgcg ccctgtgcgc gtgaccgcgc 480
   acgccgaagg ggggtgcccc cccttctcga accctcccgg cccgctaacg cgggcctccc 540
   atccccccag gggctgcgcc cctcggccgc gaacggcctc accccaaaaa tggcagcgc 599
<210> 55
   <211> 801
   <212> DNA
   <213> Escherichia coli
<400> 55
   tgcgcgtccc ccttggtcaa attgggtata cccatttggg cctagtctag ccggcatggc       60
   gcattacagc aatacgcaat ttaaatgcgc ctagcgcatt ttcccgacct taatgcgcct      120
   cgcgctgtag cctcacgccc acatatgtgc taatgtggtt acgtgtattt tatggaggtt      180
   atccaatgag ccgcctgaca atcgacatga cggaccagca gcaccagagc ctgaaagccc      240
   tggccgcctt gcagggcaag accattaagc aatacgccct cgaacgtctg ttccccggtg      300
   acgctgatgc cgatcaggca tggcaggaac tgaaaaccat gctggggaac cgcatcaacg      360
   atgggcttgc cggcaaggtg tccaccaaga gcgtcggcga aattcttgat gaagaactca      420
   gcggggatcg cgcttgacgg cctacatcct cacggctgag gccgaagccg atctacgcgg      480
   catcatccgc tacacgcgcc gggagtgggg cgcggcgcag gtgcgccgct atatcgctaa      540
   gctggaacag ggcatagcca ggcttgccgc cggcgaaggc ccgtttaagg acatgagcga      600
   actctttccc gcgctgcgga tggcccgctg cgaacaccac tacgtttttt gcctgccgcg      660
   tgcgggcgaa cccgcgttgg tcgtggcgat cctgcatgag cgcatggacc tcatgacgcg      720
   acttgccgac aggctcaagg gctgatttca gccgctaaaa atcgcgccac tcacaacgtc      780
   ctgatggcgt actaagctgg g                                                801
<210> 56
   <211> 3208
   <212> DNA
   <213> Artificial sequence
<220>
   <223> Synthetic construct
<400> 56
   cccagcttag tacgccatca ggacgttgtg agtggcgcga tttttagcgg ctgaaatcag 60
   cccttgagcc tgtcggcaag tcgcgtcatg aggtccatgc gctcatgcag gatcgccacg 120
   accaacgcgg gttcgcccgc acgcggcagg caaaaaacgt agtggtgttc gcagcgggcc 180
   atccgcagcg cgggaaagag ttcgctcatg tccttaaacg ggccttcgcc ggcggcaagc 240
   ctggctatgc cctgttccag cttagcgata tagcggcgca cctgcgccgc gccccactcc 300
   cggcgcgtgt agcggatgat gccgcgtaga tcggcttcgg cctcagccgt gaggatgtag 360
   gccgtcaagc gcgatccccg ctgagttctt catcaagaat ttcgccgacg ctcttggtgg 420
   acaccttgcc ggcaagccca tcgttgatgc ggttccccag catggttttc agttcctgcc 480
   atgcctgatc ggcatcagcg tcaccgggga acagacgttc gagggcgtat tgcttaatgg 540
   tcttgccctg caaggcggcc agggctttca ggctctggtg ctgctggtcc gtcatgtcga 600
   ttgtcaggcg gctcattgga taacctccat aaaatacacg taaccacatt agcacatatg 660
   tgggcgtgag gctacagcgc gaggcgcatt aaggtcggga aaatgcgcta ggcgcattta 720
   aattgcgtat tgctgtaatg cgccatgccg gctagactag gcccaaatgg gtatacccaa 780
   tttgaccaag ggggacgcgc aatctaggtt ctggaacagc tactttccga acgactcctg 840
   cgttgatcgg aaatccagaa gcccgagagg ttgccgcctt tcgggctttt tctttttcaa 900
   aaaaaaaaat ttataaaacg atctgttgcg gccgccgggt tgtgggcaaa ggcgctggcg 960
   ctcgacggtg ggcaaccgct tgcggttgtc cacgggcgga gccggtgcgc gtagcgcatt 1020
   gtccacaagc caagggcgac caataattga tatatatatt cataattgaa aagctaattg 1080
   aacatactac ttgctgtaac tacttgccgg agcgaggggt gtttgcaagc tgttgatctg 1140
   aaagggctat tagcgttctc acgtgccttt ttgattagcg atttcacgtg accttattag 1200
   cgatttcacg tactccgatt agcgatttca cgtaccctga ttagcgattt cacgtggata 1260
   gtttttggag cgggccggaa agccccgtga atcaaggctt tgcggggcat tagcggtttc 1320
   acgtggataa ctaccctcta tccacaggct tccggggata aaaaagcccg ctcgacggcg 1380
   ggctgttgga tgggaaggct tgaccaagcc aagcgtagcg ttggcctggt caagtcggag 1440
   gggggccgat gcgagcgccc ttgccgggtg cgcgggtgac atgcaggcgt gtggatttga 1500
   tgcgcaggca ttcgccgtca tcttcgatgc agtcgcttgc ctcgggatag acaatcaaca 1560
   cttcgcgtag gcgctttttg aagttgtatt tgaagctggc gagtgctgcc cgctctgccc 1620
   gctctcgggc cttatcgtcc agttcgggcg agttgcgtgc gcggctgcca taggatgagc 1680
   cgaattgcgc ttgcagggcg acccaaggga tttgcacgaa ggggcggccc ttggcccgca 1740
   acaggaacac gcgataggtc agccacgtgt aaatgtccat cgcaagcgga gactgccgca 1800
   aggcatgcag gtagtcgatt cggataggaa ccggtgagcg ggtgacttcc tcgaagaaat 1860
   cgcctgtgag ggtgagggtg ctatcccata gcgcccgatc ttctggccgc ttgggattcc 1920
   agaatagaaa agcgcgcttg gcaatgacga cgttctcaat gccgaagtca ttgccttgct 1980
   cgccggcaag cgaaatcatg gatgaaaaca ggcgttgcgc ctgattgcga agggtggccg 2040
   tgtaacggcc atcggtgtgc attccgagcc tttgtagaaa ttccgattgc gaccggccaa 2100
   ggttcaacac ggggtctttc gttcgcacgg cctcggtgca tatccaagca agcaaggtgc 2160
   gcggcataga accgtagggc aggccgatgc tcggcttgcc catgatcgac aaggtgacga 2220
   tgccattggt gcgctcaaag tagctggtct tggggtcggt gtggggcatg gtcgcttgca 2280
   caaggcaacg ggccatgtag ccgactaagc cagcttcgcg ggcatcctcc atttcgagcg 2340
   cgaggctcgt cttgatgatc tcgttgatac gatggccggg ggctttgttg ttcttaggca 2400
   tgttgttccc tccccggcat ggtgatggtt ggtctagtgt ttgtgggttt gatgttccgg 2460
   cgtttgatga acaggcgcaa ggtgtgaggg ctgacgccta acaactcggc tgcgcgactt 2520
   tgcggcaagc caaggttcac gtatgcctgt acttcatcaa tacggctgtc cagcttcaag 2580
   gcgctcgatt tgctgccctt gggtcgcccg agcgtcttgc cgcgctctct ggcgacttgt 2640
   agcgcctcgg tggtacgtgc ctgaatgaaa tgccgctcga tctgtgcagc caagccaagc 2700
   acggttgcca tgatgtcgct ttgtaggctg ccgtccatga tgatcttctg tttggtcaca 2760
   tggacgatta ggccgcgctc gctcgccgct ttgagaattt ccaaggcggc gagggcggaa 2820
   ccggcaatgc gcgtaatctc cggcgtcagt agcacgtcgc cacgctcggc cttttcgatg 2880
   attgctccga gcttgcgctt gcgccagtcc tttgctctgc tggcaatttc ttcctcgatc 2940
   tgtagcggcg cgaagccttt ggcgttcgcg tattcgagca aaccgtattt ttggttttcc 3000
   gggtcttggc cgtcacgcga aacccggaga taggcatagt attttggcat ttgcagggaa 3060
   aacgtcagat tcggttaaac atgcctcatt ctagcgcaga ttaaatagga attaaatacc 3120
   ctgtagcggt atagataaaa cgttggtttg ttctgcccta tgagcgtaca aaaaaggccg 3180
   ggtgagtggc ccggccttcg tttaggtg 3208
<210> 57
   <211> 3754
   <212> DNA
   <213> Agrobacterium rhizogenes
<400> 57
   atgcgttatc ttgagacggc aataacgcgt ctgacgctaa taaaccgtct gaaacctgag      60
   gcccaagtga acgtgatcga ccggcacatt aatcgagcat caacttccgc acacatcacc     120
   cagcgcgcgg aagcgttgtc tgcgagactc agggcggttg gtgagcgcgc gtttccccct     180
   accgcacaaa agtcgcttcg atctttcact tccggcgagg tcgccgaaat tgtcggcgtg     240
   tcagacggct atcttcgcca gctttcgctc gatggactcg gcccttcgcc agatttggga     300
   agcgccggtc gccgctctta cactttggag caaatcaatc aactccgtga gtatcttgcc     360
   ggagctcgac ctaaggaagc aagtagattt tggccacgtc ggcgcagtgg cgaaaaactt     420
   caagtgatca cggtcgcaaa tttcaaaggc ggctccgcga agacaacaac tgctgtttat     480
   ctcgcacaag gacttgcgtt gcagggctac cgtgtcttgg cggttgatct tgatcctcag     540
   gcttccctct ccgcaatgtt cggttatcag ccagagttcg acgttgccga aaacacgacc     600
   ctttatggtg cgatccgcta cgacgaccag agggtgacga tgaaggacgt tatccggcgc     660
   acctacttta ccgggataag cattgtcccg ggtaatctcg aactgatgga gtttgaacat     720
   cagacccctc gattcatgct gcagaatcga gggcgtccgg aggatctatt tttcagacgt     780
   gtcgcaggcg caatcaatca ggtcgaacag gacttcgacg tcgttgtcgt cgattgcccc     840
   ccacaactgg gctttttgac catgggcgcc cttaacgcag cgagtggcat gattgtgact     900
   gtccatcccc aaatggtcga tgtcgcatcc atgagccagt tcctcttgat gacgtccgat     960
   ctcgtgtcgg taattgaaga agccggcgga cggctcgact acgactttct aaggtttctt    1020
   attacgaggc atgatccgcg cgacgtcccc gagcaggaaa tcgtggcgct gcttcgtgac    1080
   gtcttcggaa ccgacgtcat ggcggcatcc gcatggaagt cgacggcaat cgccaatgcc    1140
   ggtcttacca agcagtcgct ctatgaactc tcgcgcggcg cggtaggacg gacgacgtat    1200
   gatcgagcaa tggaatccat caatgccgtg aatctggaaa ttatcgacca gataaacaag 1260
   gtctggggtc gatgatggcg gctcaatcgc tgataaatca aaaccttagt gtgttgtcag 1320
   ctgactacac ggtcttacta gaactgttgt ttggataggg tcgaaacgat gagcaaacgc 1380
   acacaatcca ttcgctcaat gtttgccggg ggcaacgatc agaccgcttc taatgacgca 1440
   cagcagcctg tgcagcgagt tgcctcgggt gcggtccggt cgttgaaaga cacgttctca 1500
   gaggtggaaa gacattacga agagctcaag cagcaagttg ctgacggagc tgttccagtc 1560
   gaaatagacc cagaattggt cgacccctcg cccttcgcgg accggtttgc ggaccaagac 1620
   atggctgtct tggaggtcct taagacttcg atcaaagatc atggacaaga aattccgatc 1680
   cttgttcgac cgcatccagc tgagatcgga cgatatcagg ttgcctacgg ccaccgcaga 1740
   ctacgtgcga ctgccgaact cggcttgaaa gtcaaagcct atgtccgcga actggatgac 1800
   gatcgtcttg tcgtcgcgca gggcatcgag aattccgctc gtgaagacct gactttcatc 1860
   gaacgggcgg cgtttgcact caaactggac gagggtggtt ttcaacggtc gctaatacaa 1920
   atggctttgt ctgttgaccg acacgaagct caaaagctgg taacagttgc tcgggcagtt 1980
   ccgcagtggt tgattgattc cattggccgt gcgccgaaga tcggtcgacc gaggtggctg 2040
   gagctcgcag aacttttgaa agtcccgggc gcagagaaga aggcccgcaa agccacccag 2100
   gacaaatcct tctcacataa ggattctgac aatcgtttcc tggtcgtcct aagagcgtcc 2160
   aagatagatg atgtgacggc gtcgtctgta gcccccaccc gcttggtcgc aaaatctgct 2220
   gacgggcttc aaatcgcgac gctcgccatc ggcggcaagc agtgcaaaat cgaaatgaac 2280
   cgagatagag acgagacttt tgcaaagttt gttatggagc agcttcctgc gctctacgcg 2340
   aacttcagaa aggaaaatcc gtcatctgaa ggataaataa ggataggaac agcaaaagaa 2400
   aggcccccaa acgtcgccgt cgtggaagcc tctctcaatg gtctaagcag cctgagaatc 2460
   gcatttccat gaatcgcagt caagagtctt tggcaccgtt tttggtgagc agatttcttt 2520
   tgcctattga agggtgaaag acatgcagag tggaaatgtg acgacgccgt ttgggcggcg 2580
   gccgatgacg cttgccttag tggcggcaca gttcaaatcc gccgacatca ggcagggtaa 2640
   atcggccgac aagtggaaga tctatcgcga tgcttgtgac gcgcgatcat tgttgggttt 2700
   gcgcgatcgg gcacttgcag tgctcaacgc gcttctatca ttctaccctg aggctgaact 2760
   caaggaggaa gcgaatctta tcgtgtttcc gtccaatgcc caactcgcgg cacgggcaaa 2820
   cggtatcgcc ggcacgacgc tacgcgagaa cttggccctc ttggtcgatg caggtctcgt 2880
   ccaacggaat gatagcccga acgggaaacg ctatgcgcgc aagagctcag atgggtccat 2940
   cgagacagcc tttggcttca gtctagcgcc gcttctggcc cgatcagaag aacttgcggt 3000
   tatggcccag caggtcgcag agggtaatcg caagctcaaa atcgtcaaag agcgcatcac 3060
   aattgcccga cgggatgtcc ggaaattgat ctctgcggca atggaagatc acgttcccgg 3120
   ggattgggcc aaggtcgagg agctttatgt tgaagccatg gcacggctgc ggcgggcaaa 3180
   aggggcggac gaattgatcg gaattctcga cgaagtcgag atgctgaagg aagaagtagt 3240
   caatctattg gaacggcacg tcaatcttga aaaatccgac gccaatgatg acggattccg 3300
   tcagcacata cagaattcaa ataccgaatc cagcaatgaa cttgaacctt gctctgaaaa 3360
   cgagcagggc gcgaagcaga gtaaaaacgt tgaactaaga gccgaatcga taaaatcatt 3420
   cccgcttggt atggtccttc gggcatgccc ggagatttcc atgtatggtc cgggcggagc 3480
   aatcggcagc tggcgggacc ttatgatggc cgccgtggtc gtgcgatcaa tgctgggggt 3540
   aagcccgtcg gcctaccagg acgcctgcga agtaataggg cctgaaaacg cggccgcaat 3600
   gatggctgca atcctggagc gggcggggca catcacatcg gccggtggat atcttcgcga 3660
   tttgacgtcg agggcaaaac gcggcgagtt ttctctcgga ccaatgttga tggcgttgct 3720
   gcgggccaat ggcggcgaac aaaggcgggc ttaa 3754
<210> 58
   <211> 3992
   <212> DNA
   <213> Agrobacterium tumefaciens
<400> 58
   gtgagcaaag ccgctgccat atcccgaaat gatcgcccgt cggtagatgt taccattggt 60
   gagcatgctg agcagctcag ctctcagctt caagcgatga gcgaggcttt gtttcctccg 120
   acgtcgcaca agagcttgcg caaattcacc tcgggtgaag ccgcacgctt gatgaaaata 180
   tctgactcaa ctcttcgaaa gatgacactg gctggcgaag ggccgcaacc tgaactcgcc 240
   agcaacggac ggcgctttta caccctcggt cagataaacg aaatccggca gatgcttgcc 300
   ggctcgactc gaggacgtga aagcattgat tttgtgcctc atcgccgagg ttctgagcat 360
   ttgcaagtcg ttgctgtaac caacttcaaa ggtggctctg ggaagacgac gacgtccgct 420
   catcttgcac agtatctggc gttgcaaggt tacagggttc tcgcagtcga tctcgatccg 480
   caggctagtc tttcagcact cctcggcgtt ctgccagaaa ctgatgtcgg tgcaaacgaa 540
   acgctctatg cggctattcg gtacgacgac acacgtcgtc cgttgcgaga tgtgatccga 600
   ccgacgtatt ttgatggtct tcaccttgtt cctggaaatc tcgagcttat ggagttcgag 660
   cataccaccc cgaaagcatt gactgacaaa ggtacgcgcg acggattgtt cttcactcgc 720
   gtggcccaag cctttgatga ggtcgccgac gattacgatg tcgtggtcat cgactgccct 780
   cctcagcttg ggtttttgac tctcagcggg ttgtgtgctg caacatcaat ggtaatcacc 840
   gtacatcctc agatgctgga tatcgcttcc atgagccagt ttctcctcat gacacgcgac 900
   cttctgggtg tcgtgaaaga ggcggggggc aatctccagt acgatttcat acgctatctc 960
   ttgacgcgct atgagcccca ggacgcgccg cagacgaaag tgacggcact gctgcgcaac 1020
   atgttcgagg atcacgtcct tacaaatcct atggtcaagt cggcagcggt atctgatgcc 1080
   ggtttaacca agcagacgct ctatgagata gggcgagaga accttacgcg atcgacatac 1140
   gaccgggcga tggaatcttt agatgcggtg aattcggaga tcgaggcttt gatcaagatg 1200
   gcgtgggggc gggtctaatg aaaggctttg cgttcctcac agatctgttg ggagctccca 1260
   acagacaggt gttgattcgc cccctggaca tggggcactg gagaagccgg ggtaatttga 1320
   gacgacgacg cacgcccatc gctaattggc cagggtgcag ttgtcttgtc ttgttgggag 1380
   ctcccaacca agcgcatttg caatcaaaaa tgcgacgcca cgacgccaaa cccaagaggc 1440
   tgatatcatg agccgcaaag acgcaatcga tactttgttc ctcaagaagc aacctgcgac 1500
   cgatagagca gcagtcgaca agtcgaccgc tcgtgttcgt accggagcga tttcggccat 1560
   gggttcgtct ttgcaagaga tggctgaggg cgcaaaggct gcagctcggc tgcaggatca 1620
   actggctaca ggcgaagccg tcgtgtccct ggatccatcc atgatcgacg ggtcgccgat 1680
   cgcggatcgg ctgccctcag acgtggatcc gaaattcgag cagcttgagg cgagcatttc 1740
   gcaggagggg cagcaggtgc cggttcttgt cagaccgcac cctgaggctg ccggtcgata 1800
   tcagatcgta tatggaaggc ggcggctgcg cgcggcagta aatctgcgga gagaggtttc 1860
   tgccattgtt cgaaatctca cggactgtga actggtcgtg gcccagggcc gcgaaaatct 1920
   taaccgtgct gacctctcgt tcattgagaa ggctctcttc gccctgcgcc tcgaagatgc 1980
   gggttttgat agagccacca tcattgccgc gctatccact gacaaggccg acctcagccg 2040
   ctacataact gtagcaaggg gcataccgct gaacctcgcc acacaaatcg gcccagcgtc 2100
   gaaagcgggt cgatcgcgtt gggtcgcact tgccgagggg cttgggaagc ctaaggcaac 2160
   ggacgcaatc gaagcgatgc ttgggtcaga gcagttcaag caatctgata gcgatacccg 2220
   ctttaacctc attttcaacg ccgtttcaag gccacctgcg aagactccaa aaaaggtaag 2280
   ggcctggagc acgccaaagg ggaaaaaggc agcgacgatc cgacaagaaa ctggacgaac 2340
   ggcgctggtt ttcgacgaga gactggtgcc aacttttggc gaatatgtcg ctgaccagtt 2400
   ggacagtctg tacgcccagt tcattgaaac caacggagga ggcaagctcg accaatagtc 2460
   agggtttcat ccaatttaaa gctccgctcg actgagatgg actggctctc accgcaaaag 2520
   aaaaaggccc ccgaaacggc gttccggaag accttctctg tagtctcgca gctaagagaa 2580
   tcgcatttcc aggaatcgta gtcaagggtc ccgtaaggga aagcgtcatt tcgacgggcg 2640
   gatttcaatt gcctaacaaa aggtaaaagg aaatgcagac gcatatctca acgacgtcct 2700
   ttgggcggcg gccgatgaca ctcggccata ttgcaagcca gatggcagca aaagcggtcg 2760
   catcagacac tgtcgcccac aaatggcagg tcttccagca catccgtgaa tcccggggac 2820
   tgatcggagc cacggaccgc tcactctcga tcctgaacgc gctgttgacg ttttacccgg 2880
   agaccgcctt gactggtggt gccgaactgg tcgtatggcc ttctaacgaa cagctgatgg 2940
   ctcgcgccaa cggcatgccc gccacgacac tgcgccggca tcttgccata ctggttgatt 3000
   gcgggctcat cattcgccgc gacagcccca atggcaagcg gttcgcccgc aagggaaggg 3060
   gaggggagat tgagcaggcc tatgggttcg atctgtcgcc gatcgtcgcg cgggccgagg 3120
   agttccgaga tctggcccag acagtgcaag ctgaaaaaaa ggccttccgg gtggccaagg 3180
   agcgcttgac tcttcttcgt cgtgacattg tcaaaatgat cgaaactggc gtcgaagaga 3240
   gcgttcctgg aaactgggga agagttaccc agacctatca ggggatcatc ggccgcctgc 3300
   cacgctcggc acctcggcag cttgtcgaga gtattgggca agagcttcag gaactttgca 3360
   tcgagatccg tgacgtattg gaatctttca caaaaacgat gaatctggac gccaatgagt 3420
   cccatatcgg tcgccacaaa cagaattcaa atccagactc taaatttgaa tctgaatacg 3480
   gctctggaaa aaaagatgaa gcgggcggca gcgttgcgga aaccgacaat gtacggagct 3540
   tgccgaaacg cgagctgcct ttgggaatcg tgctggatgc ctgccccgaa atgcgggaat 3600
   tggcccaggg aggtccaatt cgacattggc gcgacttgct ggcggcggct gagcttgccc 3660
   ggccgatgct ggggattagt ccgagcgcct ggcgggaggc ccgcgaaacc atgggcgagc 3720
   aacacgcggc gatcacgctg gcttcgatct atcagcgggc cggtcagatc aataacgctg 3780
   ggggctatct gcgcagcctg accgaccggg ccaaggatgg gaagttttcg acctggccga 3840
   tggtcatggc gttgctccgg gcgaagctgg acgagcagaa gaatgcagtc ggcgctggaa 3900
   agccgcgaac tgctgaggag gtcgaggatg acagccgcct ccacgtatcg gaatcgctgc 3960
   tcaaaaacct gcgaaagccg agatcttggt ga 3992
<210> 59
   <211> 865
   <212> DNA
   <213> artificial sequence
<220>
   <223> PR1b plasmid, Ruegeria sp.
<400> 59
   gcgccggctg gtaatcgatg atgatgcaat ccagcgtttc gttcagcgcc tgctcgagac 60
   ggacaggatc gacctttcca tctgcaacaa gttcgttggg agccgttgcc ggcggatacg 120
   cctctttcca gcgatcaatc gcatcccgca gatagcgata gaaactcttc cccgcaggcg 180
   cttcgcgaac caagcgggcg atctgaattt cgccctcaga agtctctccg tgcgccggca 240
   ccaatcgaag ccccggccaa gaagtcttca aaaagaagct atccagcgtc tccgcagagt 300
   gatccgtgta cggcgcctcc tcagactgga agaggcctgc gaagtccacc atcgatggcg 360
   tctcttcagt aggcatctga gacaaaccct caccgcctac aaagtaaagt gtgatcgtgc 420
   tttgcggatc cgcatccatc acaccaacgc gcattccgta gtgcaagctc aggtactgag 480
   cgaaatgtgc agcgctaagg ctcttggctg tgccgccctt ttggctggca aatgaaatga 540
   ccggaagcgg atcgccgggc ttgcgccacg ctagatagtc aaaagggcgc tttgcagacg 600
   ccgccatcaa agcgcgcata tgcatcagtt cgtgaagatt gaagacacgc tcgcgtccga 660
   cgtactcacc ctttggaaag tcgtcgttgc cattcgcaaa cttagtcaag tattggatgc 720
   tgacgttcaa aaagcgagcg cactgtcgca tcgaccatgt acgcttgata cgatggcgca 780
   acgtcaactc tttgttgacg gcgaccattg tgcgctccag acctcgtgac aggtcggtca 840
   aaaactgttc tagacttccg ctcat 865
<210> 60
   <211> 3120
   <212> DNA
   <213> Sinorhizobium fredii
<400> 60
   atgaacgcta attcgccgcc catcgctccc gcacagccga tgcatttcga ggatctcatc      60
   ctcgaacagg gcgatctgat ttcaaagaaa ttgcatcttt taagcatgca gcagttccca     120
   ccgaatgcca agaaactgct tcgccagttc tcgctctctg aagtggcgca gttcctcggc     180
   gtctctcaaa gtacgctgaa gaaacttcat cttgagggaa aaggccccct cccccagaca    240
   tcgtcgtcgg gtcgccgttc ttatagtgct gagcagatgg cggaactgcg tcagtatctc     300
   gatcagcatg gacgatcgga agccaggaac tacgtgccgc accgtcggtc gggcgaaaag     360
   ctccaggtca ttgccgttgt caatttcaaa ggcggttcgg gaaagacgac gacggccgcc     420
   catctcgcgc agtacatggc gttgaccggg caccgtgtct tggcggtgga cctcgacccg     480
   caggcgtcac tttcgtcgct tcacggtttt cagcccgaac tcgacatgtc gccgtcgctc     540
   tatgaggcgc tcagatatga cgatcaacgc cgatcgatca gtgagatcat ccagcctacc     600
   aatttcccgg gcctcgatat cgtgcctgcg aacctcgaac ttcaggagta cgagtacgac     660
   accccgcttg cgatgtcgaa caagagctcg aacgatggca agaccttctt cacgcggatt     720
   tcgcgcgcgc tgtcggaagt caacgaccgc tatgacgttg tcgtcatcga ctgccctccc     780
   cagctcggct atctcacgat caccgcgctg actgcggcta cgagcgtcct gatcacgatc     840
   catccacaga tgctcgacgt catgtcgatg ggccagttcc ttttgatgct gggcgggatc     900
   ctgaagccga tccgggatgt gggtgctgcg gtcaatctcg aatggtatcg ctatctgatc     960
   acccggtacg agccaacgga tgggccgcag gcgcagatgg tgggcttcat gcagaccttg    1020
   ttccaccagt ttgtgctgaa gaaccagatg ctgaaatcga cagcggtttc agacgccgga    1080
   atcaccaagc agacccttta cgaagtcgac aagagtcaga tgacgcggtc cacctatgag    1140
   cgcgcgatgg actccttgaa cgcagtcaac gccgagatcg tcgaactcgt ccatgcctct    1200
   tgggggagga aggttgtcag ttgagaagtg taaacttccc cgggacggcc cggtagatct    1260
   caggcaaacg gagcaggcac atggcgagaa agaacctact ggcgggactg gtagacacag 1320
   cggaaatccc gcacgcagac gttgcgcccg cctacccgat gcgcggggct tcgaagagca 1380
   tggtgcgctc gctcgacgag ttgtcgcgcc aggccgagaa atttctcgaa ggagaaacgg 1440
   tcgtcgaact cgaccctgag acactcgatg gttcgttcgt ctctgatcgc atgggcgata 1500
   gttccgagca gttcgaggaa ttgaagcagg cgattgccga gcgcgggcag gatacgccca 1560
   tccttgtgcg cccccacccg tcggcagccg atcgttacca gatcgtcttt ggacaccgcc 1620
   gagcccgcgt tgcccgcgag ctcggccgca aggttaaggc ggtcgtcaag gcgctggatg 1680
   accgcacgca cgttatcgcc cagggacagg aaaactcggc gcgcgccaac ctctccttca 1740
   ttgaacgggc gaacttcgcc tcgcacctcg agaagcttgg ctatgaccgg acaatcatag 1800
   gatcggcgct tgctgccaat gcggccgcta tctcgaaaat gatcgccgta atagatcgta 1860
   ttcctgaaga gacaatcgca agaatcgggc cctgcccggc ggtcggccga gaacgctggg 1920
   tcgagttgtc attgcttgtg ggtaaaaccg ccaacgaagc gaaggtgaag gcaatcgtct 1980
   ctgatccttc cttcaatgaa ctgagcactg acgatcgctt caattccctg ttttctggtc 2040
   tgaacagcgc ggcgaagcct gtccggaaga cgactccaaa gatcctggag aactggcaac 2100
   cggcggacaa gaccgtctcc gctaagtatt cgaactccgc caaggcgttt gccttgtcta 2160
   tgaaatcaag gaatgccggc ccctttgggc ggtatattgc agacaacctg gaccggctct 2220
   atgccgagtt cctggagcag ggtaatcgga aggaagactg acgcaaaaga aaaaggcccc 2280
   cagacggtaa ccatcgtgga agcctctctc atcgtttagc agcctgagaa tcgcatttcc 2340
   acgaatcgca gtcaagagtc tttggcaccg gaacgggtga gcggacgtct tttgcctgag 2400
   gataggtgaa agaagatgca agatggaagt gtgacgacgc ccttcgggcg gcggtcgatg 2460
   acgcttggca tgttggcaag ccaatatatg tcgcgtgaac tcgaacctga aacatcggct 2520
   gacaagtgga agctgtttcg agcgctctgc gaagctaagc cgaagctcgg catcagcgaa 2580
   cgtgcgctct ctgttatgaa cgcacttttg agcttttatc ccgagacgac cttgtctgag 2640
   gaaaacggcc tcatcgtgtt cccctctaac atgcagttgt cgcttcgtgc gcatggcatg 2700
   gctgaagcga cgttgcgtcg ccacatcgca gcgctcgtcg acgctggcct tctggcacgc 2760
   cgggatagcc caaatggcaa acgctacgcc cgcaaggacg gagacggttc gatcgatgag 2820
   gcctatgggt tttcgttggc tccgttgctg tcacgcgcac gcgagatcga gcaaatagct 2880
   gcctatgtca aaattgagcg actgcaattg cgtagacttc gcgagcgcct gacgatctgc 2940
   cggcgcgata tcggtaagct gattgaagtc gccattgagg agggagtgga cggtaattgg 3000
   gacggaattc accagcacta ccgcagccta gtagcgacca ttcctcgagt ggcaacggcc 3060
   gcgaccgtcg cccccatcct cgaagagatg gagatgttgc gcgaggaaat ctccaacctt 3120
<210> 61
   <211> 32
   <212> DNA
   <213> Artificial sequence
<220>
   <223> Synthetic construct
<400> 61
   cgttcctcga ggcctcgagg cctcgaggaa cg 32
<210> 62
   <211> 146
   <212> DNA
   <213> Artificial sequence
<220>
   <223> Synthetic construct
<400> 62
   gagtccacgc tagatgagag ctttgttgta ggtggaccag ttggtgattt tgaacttttg 60
   ctttgccacg gaacggtctg cgttgtcggg aagatgcgtg atctgatcct tcaactcagc 120
   aaaagttcga tttattcaac aaagcc 146
<210> 63
   <211> 6968
   <212> DNA
   <213> Artificial sequence
<220>
   <223> Synthetic construct
<400> 63
   gtgtacaacc agatattttt caccaacatc cttcgtctgc tcgatgagcg gggcatgacg      60
   aaacatgagc tgtcggagag ggcaggggtt tcaatttcgt ttttatcaga cttaaccaac     120
   ggtaaggcca acccctcgtt gaaggtgatg gaggccattg ccgacgccct ggaaactccc     180
   ctacctcttc tcctggagtc caccgacctt gaccgcgagg cactcgcgga gattgcgggt     240
   catcctttca agagcagcgt gccgcccgga tacgaacgca tcagtgtggt tttgccgtca     300
   cataaggcgt ttatcgtaaa gaaatggggc gacgacaccc gaaaaaagct gcgtggaagg     360
   ctctgacgcc aagggttagg gcttgcactt ccttctttag ccgctaaaac ggccccttct     420
   ctgcgggccg tcggctcgcg catcatatcg acatcctcaa cggaagccgt gccgcgaatg     480
   gcatcgggcg ggtgcgcttt gacagttgtt ttctatcaga acccctacgt cgtgcggttc     540
   gattagctgt ttgtcttgca ggctaaacac tttcggtata tcgtttgcct gtgcgataat     600
   gttgctaatg atttgttgcg taggggttac tgaaaagtga gcgggaaaga agagtttcag     660
   accatcaagg agcgggccaa gcgcaagctg gaacgcgaca tgggtgcgga cctgttggcc     720
   gcgctcaacg acccgaaaac cgttgaagtc atgctcaacg cggacggcaa ggtgtggcac     780
   gaacgccttg gcgagccgat gcggtacatc tgcgacatgc ggcccagcca gtcgcaggcg 840
   attatagaaa cggtggccgg attccacggc aaagaggtca cgcggcattc gcccatcctg 900
   gaaggcgagt tccccttgga tggcagccgc tttgccggcc aattgccgcc ggtcgtggcc 960
   gcgccaacct ttgcgatccg caagcgcgcg gtcgccatct tcacgctgga acagtacgtc 1020
   gaggcgggca tcatgacccg cgagcaatac gaggtcatta aaagcgccgt cgcggcgcat 1080
   cgaaacatcc tcgtcattgg cggtactggc tcgggcaaga ccacgctcgt caacgcgatc 1140
   atcaatgaaa tggtcgcctt caacccgtct gagcgcgtcg tcatcatcga ggacaccggc 1200
   gaaatccagt gcgccgcaga gaacgccgtc caataccaca ccagcatcga cgtctcgatg 1260
   acgctgctgc tcaagacaac gctgcgtatg cgccccgacc gcatcctggt cggtgaggta 1320
   cgtggccccg aagcccttga tctgttgatg gcctggaaca ccgggcatga aggaggtgcc 1380
   gccaccctgc acgcaaacaa ccccaaagcg ggcctgagcc ggctcgccat gcttatcagc 1440
   atgcacccgg attcaccgaa acccattgag ccgctgattg gcgaggcggt tcatgtggtc 1500
   gtccatatcg ccaggacccc tagcggccgt cgagtgcaag aaattctcga agttcttggt 1560
   tacgagaacg gccagtacat caccaaaacc ctgtaaggag tatttccaat gacaacggct 1620
   gttccgttcc gtctgaccat gaatcgcggc attttgttct accttgccgt gttcttcgtt 1680
   ctcgctctcg cgttatccgc gcatccggcg atggcctcgg aaggcaccgg cggcagcttg 1740
   ccatatgaga gctggctgac gaacctgcgc aactccgtaa ccggcccggt ggccttcgcg 1800
   ctgtccatca tcggcatcgt cgtcgccggc ggcgtgctga tcttcggcgg cgaactcaac 1860
   gccttcttcc gaaccctgat cttcctggtt ctggtgatgg cgctgctggt cggcgcgcag 1920
   aacgtgatga gcaccttctt cggtcgtggt gccgaaatcg cggccctcgg caacggggcg 1980
   ctgcaccagg tgcaagtcgc ggcggcggat gccgtgcgtg cggtagcggc tggacggctc 2040
   gcctaatcat ggctctgcgc acgatcccca tccgtcgcgc aggcaaccga gaaaacctgt 2100
   tcatgggtgg tgatcgtgaa ctggtgatgt tctcgggcct gatggcgttt gcgctgattt 2160
   tcagcgccca agagctgcgg gccaccgtgg tcggtctgat cctgtggttc ggggcgctct 2220
   atgcgttccg aatcatggcg aaggccgatc cgaagatgcg gttcgtgtac ctgcgtcacc 2280
   gccggtacaa gccgtattac ccggcccgct cgaccccgtt ccgcgagaac accaatagcc 2340
   aagggaagca ataccgatga tccaagcaat tgcgattgca atcgcgggcc tcggcgcgct 2400
   tctgttgttc atcctctttg cccgcatccg cgcggtcgat gccgaactga aactgaaaaa 2460
   gcatcgttcc aaggacgccg gcctggccga tctgctcaac tacgccgctg tcgtcgatga 2520
   cggcgtaatc gtgggcaaga acggcagctt tatggctgcc tggctgtaca agggcgatga 2580
   caacgcaagc agcaccgacc agcagcgcga agtagtgtcc gcccgcatca accaggccct 2640
   cgcgggcctg ggaagtgggt ggatgatcca tgtggacgcc gtgcggcgtc ctgctccgaa 2700
   ctacgcggag cggggcctgt cggcgttccc tgaccgtctg acggcagcga ttgaagaaga 2760
   gcgctcggtc ttgccttgct cgtcggtgat gtacttcacc agctccgcga agtcgctctt 2820
   cttgatggag cgcatgggga cgtgcttggc aatcacgcgc accccccggc cgttttagcg 2880
   gctaaaaaag tcatggctct gccctcgggc ggaccacgcc catcatgacc ttgccaagct 2940
   cgtcctgctt ctcttcgatc ttcgccagca gggcgaggat cgtggcatca ccgaaccgcg 3000
   ccgtgcgcgg gtcgtcggtg agccagagtt tcagcaggcc gcccaggcgg cccaggtcgc 3060
   cattgatgcg ggccagctcg cggacgtgct catagtccac gacgcccgtg attttgtagc 3120
   cctggccgac ggccagcagg taggccgaca ggctcatgcc ggccgccgcc gccttttcct 3180
   caatcgctct tcgttcgtct ggaaggcagt acaccttgat aggtgggctg cccttcctgg 3240
   ttggcttggt ttcatcagcc atccgcttgc cctcatctgt tacgccggcg gtagccggcc 3300
   agcctcgcag agcaggattc ccgttgagca ccgccaggtg cgaataaggg acagtgaaga 3360
   aggaacaccc gctcgcgggt gggcctactt cacctatcct gcccggctga cgccgttgga 3420
   tacaccaagg aaagtctaca cgaacccttt ggcaaaatcc tgtatatcgt gcgaaaaagg 3480
   atggatatac cgaaaaaatc gctataatga ccccgaagca gggttatgca gcggaaaagc 3540
   gctgcttccc tgctgttttg tggaatatct accgactgga aacaggcaaa tgcaggaaat 3600
   tactgaactg aggggacagg cgagagacga tgccaaagag ctacaccgac gagctggccg 3660
   agtgggttga atcccgcgcg gccaagaagc gccggcgtga tgaggctgcg gttgcgttcc 3720
   tggcggtgag ggcggatgtc gaggcggcgt tagcgtccgg ctatgcgctc gtcaccattt 3780
   gggagcacat gcgggaaacg gggaaggtca agttctccta cgagacgttc cgctcgcacg 3840
   ccaggcggca catcaaggcc aagcccgccg atgtgcccgc accgcaggcc aaggctgcgg 3900
   aacccgcgcc ggcacccaag acgccggagc cacggcggcc gaagcagggg ggcaaggctg 3960
   aaaagccggc ccccgctgcg gccccgaccg gcttcacctt caacccaaca ccggacaaaa 4020
   aggatctact gtaatggcga aaattcacat ggttttgcag ggcaagggcg gggtcggcaa      4080
   gtcggccatc gccgcgatca ttgcgcagta caagatggac aaggggcaga cacccttgtg      4140
   catcgacacc gacccggtga acgcgacgtt cgagggctac aaggccctga acgtccgccg 4200
   gctgaacatc atggccggcg acgaaattaa ctcgcgcaac ttcgacaccc tggtcgagct 4260
   gattgcgccg accaaggatg acgtggtgat cgacaacggt gccagctcgt tcgtgcctct 4320
   gtcgcattac ctcatcagca accaggtgcc ggctctgctg caagaaatgg ggcatgagct 4380
   ggtcatccat accgtcgtca ccggcggcca ggctctcctg gacacggtga gcggcttcgc 4440
   ccagctcgcc agccagttcc cggccgaagc gcttttcgtg gtctggctga acccgtattg 4500
   ggggcctatc gagcatgagg gcaagagctt tgagcagatg aaggcgtaca cggccaacaa 4560
   ggcccgcgtg tcgtccatca tccagattcc ggccctcaag gaagaaacct acggccgcga 4620
   tttcagcgac atgctgcaag agcggctgac gttcgaccag gcgctggccg atgaatcgct 4680
   cacgatcatg acgcggcaac gcctcaagat cgtgcggcgc ggcctgtttg aacagctcga 4740
   cgcggcggcc gtgctatgag cgaccagatt gaagagctga tccgggagat tgcggccaag 4800
   cacggcatcg ccgtcggccg cgacgacccg gtgctgatcc tgcataccat caacgcccgg 4860
   ctcatggccg acagtgcggc caagcaagag gaaatccttg ccgcgttcaa ggaagagctg 4920
   gaagggatcg cccatcgttg gggcgaggac gccaaggcca aagcggagcg gatgctgaac 4980
   gcggccctgg cggccagcaa ggacgcaatg gcgaaggtaa tgaaggacag cgccgcgcag 5040
   gcggccgaag cgatccgcag ggaaatcgac gacggccttg gccgccagct cgcggccaag 5100
   gtcgcggacg cgcggcgcgt ggcgatgatg aacatgatcg ccggcggcat ggtgttgttc 5160
   gcggccgccc tggtggtgtg ggcctcgtta tgaatcgcag aggcgcagat gaaaaagccc 5220
   ggcgttgccg ggctttgttt ttgcgttagc tgggcttgtt tgacaggccc aagctctgac 5280
   tgcgcccgcg ctcgcgctcc tgggcctgtt tcttctcctg ctcctgcttg cgcatcaggg 5340
   cctggtgccg tcgggctgct tcacgcatcg aatcccagtc gccggccagc tcgggatgct 5400
   ccgcgcgcat cttgcgcgtc gccagttcct cgatcttggg cgcgtgaatg cccatgcctt 5460
   ccttgatttc gcgcaccatg tccagccgcg tgtgcagggt ctgcaagcgg gcttgctgtt 5520
   gggcctgctg ctgctgccag gcggcctttg tacgcggcag ggacagcaag ccgggggcat 5580
   tggactgtag ctgctgcaaa cgcgcctgct gacggtctac gagctgttct aggcggtcct 5640
   cgatgcgctc cacctggtca tgctttgcct gcacgtagag cgcaagggtc tgctggtagg 5700
   tctgctcgat gggcgcggat tctaagaggg cctgctgttc cgtctcggcc tcctgggccg 5760
   cctgtagcaa atcctcgccg ctgttgccgc tggactgctt tactgccggg gactgctgtt 5820
   gccctgctcg cgccgtcgtc gcagttcggc ttgcccccac tcgattgact gcttcatttc 5880
   gagccgcagc gatgcgatct cggattgcgt caacggacgg ggcagcgcgg aggtgtccgg 5940
   cttctccttg ggtgagtcgg tcgatgccat agccaaaggt ttccttccaa aatgcgtcca 6000
   ttgctggacc gtgtttctca ttgatgcccg caagcatctt cggcttgacc gccaggtcaa 6060
   gcgcgccttc atgggcggtc atgacggacg ccgccatgac cttgccgccg ttgttctcga 6120
   tgtagccgcg taatgaggca atggtgccgc ccatcgtcag cgtgtcatcg acaacgatgt 6180
   acttctggcc ggggatcacc tccccctcga aagtcgggtt gaacgccagg cgatgatctg 6240
   aaccggctcc ggttcgggcg accttctccc gctgcacaat gtccgtttcg acctcaaggc 6300
   caaggcggtc ggccagaacg accgccatca tggccggaat cttgttgttc cccgccgcct 6360
   cgacggcgag gactggaacg atgcggggct tgtcgtcgcc gatcagcgtc ttgagctggg 6420
   caacagtgtc gtccgaaatc aggcgctcga ccaaattaag cgccgcttcc gcgtcgccct 6480
   gcttcgcagc ctggtattca ggctcgttgg tcaaagaacc aaggtcgccg ttgcgaacca 6540
   ccttcgggaa gtctccccac ggtgcgcgct cggctctgct gtagctgctc aagacgcctc 6600
   cctttttagc cgctaaaact ctaacgagtg cgcccgcgac tcaacttgac gctttcggca 6660
   cttacctgtg ccttgccact tgcgtcatag gtgatgcttt tcgcactccc gatttcaggt 6720
   actttatcga aatctgaccg ggcgtgcatt acaaagttct tccccacctg ttggtaaatg 6780
   ctgccgctat ctgcgtggac gatgctgccg tcgtggcgct gcgacttatc ggccttttgg 6840
   gccatataga tgttgtaaat gccaggtttc agggccccgg ctttatctac cttctggttc 6900
   gtccatgcgc cttggttctc ggtctggaca attctttgcc cattcatgac caggaggcgg 6960
   tgtttcat 6968
<210> 64
   <211> 1660
   <212> DNA
   <213> Escherichia coli
<400> 64
   gtggcgcaac gatgccggcg acaagcagga gcgcaccgac ttcttccgca tcaagtgttt 60
   tggctctcag gccgaggccc acggcaagta tttgggcaag gggtcgctgg tattcgtgca 120
   gggcaagatt cggaatacca agtacgagaa ggacggccag acggtctacg ggaccgactt 180
   cattgccgat aaggtggatt atctggacac caaggcacca ggcgggtcaa atcaggaata 240
   agggcacatt gccccggcgt gagtcggggc aatcccgcaa ggagggtgaa tgaatcggac 300
   gtttgaccgg aaggcataca ggcaagaact gatcgacgcg gggttttccg ccgaggatgc 360
   cgaaaccatc gcaagccgca ccgtcatgcg tgcgccccgc gaaaccttcc agtccgtcgg 420
   ctcgatggtc cagcaagcta cggccaagat cgagcgcgac agcgtgcaac tggctccccc 480
   tgccctgccc gcgccatcgg ccgccgtgga gcgttcgcgt cgtctcgaac aggaggcggc 540
   aggtttggcg aagtcgatga ccatcgacac gcgaggaact atgacgacca agaagcgaaa 600
   aaccgccggc gaggacctgg caaaacaggt cagcgaggcc aagcaggccg cgttgctgaa 660
   acacacgaag cagcagatca aggaaatgca gctttccttg ttcgatattg cgccgtggcc 720
   ggacacgatg cgagcgatgc caaacgacac ggcccgctct gccctgttca ccacgcgcaa 780
   caagaaaatc ccgcgcgagg cgctgcaaaa caaggtcatt ttccacgtca acaaggacgt 840
   gaagatcacc tacaccggcg tcgagctgcg ggccgacgat gacgaactgg tgtggcagca 900
   ggtgttggag tacgcgaagc gcacccctat cggcgagccg atcaccttca cgttctacga 960
   gctttgccag gacctgggct ggtcgatcaa tggccggtat tacacgaagg ccgaggaatg 1020
   cctgtcgcgc ctacaggcga cggcgatggg cttcacgtcc gaccgcgttg ggcacctgga 1080
   atcggtgtcg ctgctgcacc gcttccgcgt cctggaccgt ggcaagaaaa cgtcccgttg 1140
   ccaggtcctg atcgacgagg aaatcgtcgt gctgtttgct ggcgaccact acacgaaatt 1200
   catatgggag aagtaccgca agctgtcgcc gacggcccga cggatgttcg actatttcag 1260
   ctcgcaccgg gagccgtacc cgctcaagct ggaaaccttc cgcctcatgt gcggatcgga 1320
   ttccacccgc gtgaagaagt ggcgcgagca ggtcggcgaa gcctgcgaag agttgcgagg 1380
   cagcggcctg gtggaacacg cctgggtcaa tgatgacctg gtgcattgca aacgctaggg 1440
   ccttgtgggg tcagttccgg ctgggggttc agcagccagc gctttactgg catttcagga 1500
   acaagcgggc actgctcgac gcacttgctt cgctcagtat cgctcgggac gcacggcgcg 1560
   ctctacgaac tgccgataaa cagaggatta aaattgacaa ttgtgattaa ggctcagatt 1620
   cgacggcttg gagcggccga cgtgcaggat ttccgcgaga 1660
<210> 65
   <211> 868
   <212> DNA
   <213> Escherichia coli
<400> 65
   agaacacgag cacggcaccc gcgaccacta tgccaagaat gcccaaggta aaaattgccg 60
   gccccgccat gaagtccgtg aatgccccga cggccgaagt gaagggcagg ccgccaccca 120
   ggccgccgcc ctcactgccc ggcacctggt cgctgaatgt cgatgccagc acctgcggca 180
   cgtcaatgct tccgggcgtc gcgctcgggc tgatcgccca tcccgttact gccccgatcc 240
   cggcaatggc aaggactgcc agcgctgcca tttttggggt gaggccgttc gcggccgagg 300
   ggcgcagccc ctggggggat gggaggcccg cgttagcggg ccgggagggt tcgagaaggg 360
   ggggcacccc ccttcggcgt gcgcggtcac gcgcacaggg cgcagccctg gttaaaaaca 420
   aggtttataa atattggttt aaaagcaggt taaaagacag gttagcggtg gccgaaaaac 480
   gggcggaaac ccttgcaaat gctggatttt ctgcctgtgg acagcccctc aaatgtcaat 540
   aggtgcgccc ctcatctgtc agcactctgc ccctcaagtg tcaaggatcg cgcccctcat 600
   ctgtcagtag tcgcgcccct caagtgtcaa taccgcaggg cacttatccc caggcttgtc 660
   cacatcatct gtgggaaact cgcgtaaaat caggcgtttt cgccgatttg cgaggctggc 720
   cagctccacg tcgccggccg aaatcgagcc tgcccctcat ctgtcaacgc cgcgccgggt 780
   gagtcggccc ctcaagtgtc aacgtccgcc cctcatctgt cagtgagggc caagttttcc 840
   gcgaggtatc cacaacgccg gcggccgc 868
<210> 66
   <211> 3129
   <212> DNA
   <213> Artificial sequence
<220>
   <223> Synthetic construct
<400> 66
   cgctcaccgg gctggttgcc ctcgccgctg ggctggcggc cgtctatggc cctgcaaacg 60
   cgccagaaac gccgtcgaag ccgtgtgcga gacaccgcgg ccgccggcgt tgtggatacc 120
   tcgcggaaaa cttggccctc actgacagat gaggggcgga cgttgacact tgaggggccg 180
   actcacccgg cgcggcgttg acagatgagg ggcaggctcg atttcggccg gcgacgtgga 240
   gctggccagc ctcgcaaatc ggcgaaaacg cctgatttta cgcgagtttc ccacagatga 300
   tgtggacaag cctggggata agtgccctgc ggtattgaca cttgaggggc gcgactactg 360
   acagatgagg ggcgcgatcc ttgacacttg aggggcagag tgctgacaga tgaggggcgc 420
   acctattgac atttgagggg ctgtccacag gcagaaaatc cagcatttgc aagggtttcc 480
   gcccgttttt cggccaccgc taacctgtct tttaacctgc ttttaaacca atatttataa 540
   accttgtttt taaccagggc tgcgccctgt gcgcgtgacc gcgcacgccg aaggggggtg 600
   cccccccttc tcgaaccctc ccggcccgct ctcgagttgg cagcatcacc cataattgtg 660
   gtttcaaaat cggctccgtc gatactatgt tatacgccaa ctttgaaaac aactttgaaa 720
   aagctgtttt ctggtattta aggttttaga atgcaaggaa cagtgaattg gagttcgtct 780
   tgttataatt agcttcttgg ggtatcttta aatactgtag aaaagaggaa ggaaataata 840
   aatggctaaa atgagaatat caccggaatt gaaaaaactg atcgaaaaat accgctgcgt 900
   aaaagatacg gaaggaatgt ctcctgctaa ggtatataag ctggtgggag aaaatgaaaa 960
   cctatattta aaaatgacgg acagccggta taaagggacc acctatgatg tggaacggga 1020
   aaaggacatg atgctatggc tggaaggaaa gctgcctgtt ccaaaggtcc tgcactttga 1080
   acggcatgat ggctggagca atctgctcat gagtgaggcc gatggcgtcc tttgctcgga 1140
   agagtatgaa gatgaacaaa gccctgaaaa gattatcgag ctgtatgcgg agtgcatcag 1200
   gctctttcac tccatcgaca tatcggattg tccctatacg aatagcttag acagccgctt 1260
   agccgaattg gattacttac tgaataacga tctggccgat gtggattgcg aaaactggga 1320
   agaagacact ccatttaaag atccgcgcga gctgtatgat tttttaaaga cggaaaagcc 1380
   cgaagaggaa cttgtctttt cccacggcga cctgggagac agcaacatct ttgtgaaaga 1440
   tggcaaagta agtggcttta ttgatcttgg gagaagcggc agggcggaca agtggtatga 1500
   cattgccttc tgcgtccggt cgatcaggga ggatatcggg gaagaacagt atgtcgagct 1560
   attttttgac ttactgggga tcaagcctga ttgggagaaa ataaaatatt atattttact 1620
   ggatgaattg ttttagtacc tagatgtggc gcaacgatgc cggcgacaag caggagcgca 1680
   ccgacttctt ccgcatcaag tgttttggct ctcaggccga ggcccacggc aagtatttgg 1740
   gcaaggggtc gctggtattc gtgcagggca agattcggaa taccaagtac gagaaggacg 1800
   gccagacggt ctacgggacc gacttcattg ccgataaggt ggattatctg gacaccaagg 1860
   caccaggcgg gtcaaatcag gaataagggc acattgcccc ggcgtgagtc ggggcaatcc 1920
   cgcaaggagg gtgaatgaat cggacgtttg accggaaggc atacaggcaa gaactgatcg 1980
   acgcggggtt ttccgccgag gatgccgaaa ccatcgcaag ccgcaccgtc atgcgtgcgc 2040
   cccgcgaaac cttccagtcc gtcggctcga tggtccagca agctacggcc aagatcgagc 2100
   gcgacagcgt gcaactggct ccccctgccc tgcccgcgcc atcggccgcc gtggagcgtt 2160
   cgcgtcgtct cgaacaggag gcggcaggtt tggcgaagtc gatgaccatc gacacgcgag 2220
   gaactatgac gaccaagaag cgaaaaaccg ccggcgagga cctggcaaaa caggtcagcg 2280
   aggccaagca ggccgcgttg ctgaaacaca cgaagcagca gatcaaggaa atgcagcttt 2340
   ccttgttcga tattgcgccg tggccggaca cgatgcgagc gatgccaaac gacacggccc 2400
   gctctgccct gttcaccacg cgcaacaaga aaatcccgcg cgaggcgctg caaaacaagg 2460
   tcattttcca cgtcaacaag gacgtgaaga tcacctacac cggcgtcgag ctgcgggccg 2520
   acgatgacga actggtgtgg cagcaggtgt tggagtacgc gaagcgcacc cctatcggcg 2580
   agccgatcac cttcacgttc tacgagcttt gccaggacct gggctggtcg atcaatggcc 2640
   ggtattacac gaaggccgag gaatgcctgt cgcgcctaca ggcgacggcg atgggcttca 2700
   cgtccgaccg cgttgggcac ctggaatcgg tgtcgctgct gcaccgcttc cgcgtcctgg 2760
   accgtggcaa gaaaacgtcc cgttgccagg tcctgatcga cgaggaaatc gtcgtgctgt 2820
   ttgctggcga ccactacacg aaattcatat gggagaagta ccgcaagctg tcgccgacgg 2880
   cccgacggat gttcgactat ttcagctcgc accgggagcc gtacccgctc aagctggaaa 2940
   ccttccgcct catgtgcgga tcggattcca cccgcgtgaa gaagtggcgc gagcaggtcg 3000
   gcgaagcctg cgaagagttg cgaggcagcg gcctggtgga acacgcctgg gtcaatgatg 3060
   acctggtgca ttgcaaacgc tagggccttg tggggtcagt tccggctggg ggttcagcca 3120
   gcgctttac 3129
<210> 67
   <211> 1026
   <212> DNA
   <213> Artificial sequence
<220>
   <223> Synthetic construct
<400> 67
   atgaaaaagc ctgaactcac cgcgacgtct gtcgagaagt ttctgatcga aaagttcgac      60
   agcgtctccg acctgatgca gctctcggag ggcgaagaat ctcgtgcttt cagcttcgat     120
   gtaggagggc gtggatatgt cctgcgggta aatagctgcg ccgatggttt ctacaaagat     180
   cgttatgttt atcggcactt tgcatcggcc gcgctcccga ttccggaagt gcttgacatt     240
   ggggaattca gcgagagcct gacctattgc atctcccgcc gtgcacaggg tgtcacgttg     300
   caagacctgc ctgaaaccga actgcccgct gttctgcagc cggtcgcgga ggccatggat     360
   gcgatcgctg cggccgatct tagccagacg agcgggttcg gcccattcgg accgcaagga     420
   atcggtcaat acactacatg gcgtgatttc atatgcgcga ttgctgatcc ccatgtgtat     480
   cactggcaaa ctgtgatgga cgacaccgtc agtgcgtccg tcgcgcaggc tctcgatgag     540
   ctgatgcttt gggccgagga ctgccccgaa gtccggcacc tcgtgcacgc ggatttcggc     600
   tccaacaatg tcctgacgga caatggccgc ataacagcgg tcattgactg gagcgaggcg     660
   atgttcgggg attcccaata cgaggtcgcc aacatcttct tctggaggcc gtggttggct     720
   tgtatggagc agcagacgcg ctacttcgag cggaggcatc cggagcttgc aggatcgccg     780
   cggctccggg cgtatatgct ccgcattggt cttgaccaac tctatcagag cttggttgac     840
   ggcaatttcg atgatgcagc ttgggcgcag ggtcgatgcg acgcaatcgt ccgatccgga     900
   gccgggactg tcgggcgtac acaaatcgcc cgcagaagcg cggccgtctg gaccgatggc     960
   tgtgtagaag tactcgccga tagtggaaac cgacgcccca gcactcgtcc gagggcaaag    1020
   gaatag                                                               1026
<210> 68
   <211> 4148
   <212> DNA
   <213> Artificial sequence
<220>
   <223> Synthetic construct
<400> 68
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgt tgaaggagcc actcagcaag 180
   ctggtacgat tgtaatacga ctcactatag ggcgaattga gcgctgttta aacgctcttc 240
   aactggaaga gcggttacca gagctggtca cctttgtcca ccaagatgga actgcggcct 300
   cgagatccac tagagcttgc atgcctgcag tgcagcgtga cccggtcgtg cccctctcta 360
   gagataatga gcattgcatg tctaagttat aaaaaattac cacatatttt ttttgtcaca 420
   cttgtttgaa gtgcagttta tctatcttta tacatatatt taaactttac tctacgaata 480
   atataatcta tagtactaca ataatatcag tgttttagag aatcatataa atgaacagtt 540
   agacatggtc taaaggacaa ttgagtattt tgacaacagg actctacagt tttatctttt 600
   tagtgtgcat gtgttctcct ttttttttgc aaatagcttc acctatataa tacttcatcc 660
   attttattag tacatccatt tagggtttag ggttaatggt ttttatagac taattttttt 720
   agtacatcta ttttattcta ttttagcctc taaattaaga aaactaaaac tctattttag 780
   tttttttatt taataattta gatataaaat agaataaaat aaagtgacta aaaattaaac 840
   aaataccctt taagaaatta aaaaaactaa ggaaacattt ttcttgtttc gagtagataa 900
   tgccagcctg ttaaacgccg tcgacgagtc taacggacac caaccagcga accagcagcg 960
   tcgcgtcggg ccaagcgaag cagacggcac ggcatctctg tcgctgcctc tggacccctc 1020
   tcgagagttc cgctccaccg ttggacttgc tccgctgtcg gcatccagaa attgcgtggc 1080
   ggagcggcag acgtgagccg gcacggcagg cggcctcctc ctcctctcac ggcaccggca 1140
   gctacggggg attcctttcc caccgctcct tcgctttccc ttcctcgccc gccgtaataa 1200
   atagacaccc cctccacacc ctctttcccc aacctcgtgt tgttcggagc gcacacacac 1260
   acaaccagat ctcccccaaa tccacccgtc ggcacctccg cttcaaggta cgccgctcgt 1320
   cctccccccc ccccctctct accttctcta gatcggcgtt ccggtccatg catggttagg 1380
   gcccggtagt tctacttctg ttcatgtttg tgttagatcc gtgtttgtgt tagatccgtg 1440
   ctgctagcgt tcgtacacgg atgcgacctg tacgtcagac acgttctgat tgctaacttg 1500
   ccagtgtttc tctttgggga atcctgggat ggctctagcc gttccgcaga cgggatcgat 1560
   ttcatgattt tttttgtttc gttgcatagg gtttggtttg cccttttcct ttatttcaat 1620
   atatgccgtg cacttgtttg tcgggtcatc ttttcatgct tttttttgtc ttggttgtga 1680
   tgatgtggtc tggttgggcg gtcgttctag atcggagtag aattctgttt caaactacct 1740
   ggtggattta ttaattttgg atctgtatgt gtgtgccata catattcata gttacgaatt 1800
   gaagatgatg gatggaaata tcgatctagg ataggtatac atgttgatgc gggttttact 1860
   gatgcatata cagagatgct ttttgttcgc ttggttgtga tgatgtggtg tggttgggcg 1920
   gtcgttcatt cgttctagat cggagtagaa tactgtttca aactacctgg tgtatttatt 1980
   aattttggaa ctgtatgtgt gtgtcataca tcttcatagt tacgagttta agatggatgg 2040
   aaatatcgat ctaggatagg tatacatgtt gatgtgggtt ttactgatgc atatacatga 2100
   tggcatatgc agcatctatt catatgctct aaccttgagt acctatctat tataataaac 2160
   aagtatgttt tataattatt ttgatcttga tatacttgga tgatggcata tgcagcagct 2220
   atatgtggat ttttttagcc ctgccttcat acgctattta tttgcttggt actgtttctt 2280
   ttgtcgatgc tcaccctgtt gtttggtgtt acttctgcag gtcgacttta acttagccta 2340
   ggatccacac gacaccatgt cccccgagcg ccgccccgtc gagatccgcc cggccaccgc 2400
   cgccgacatg gccgccgtgt gcgacatcgt gaaccactac atcgagacct ccaccgtgaa 2460
   cttccgcacc gagccgcaga ccccgcagga gtggatcgac gacctggagc gcctccagga 2520
   ccgctacccg tggctcgtgg ccgaggtgga gggcgtggtg gccggcatcg cctacgccgg 2580
   cccgtggaag gcccgcaacg cctacgactg gaccgtggag tccaccgtgt acgtgtccca 2640
   ccgccaccag cgcctcggcc tcggctccac cctctacacc cacctcctca agagcatgga 2700
   ggcccagggc ttcaagtccg tggtggccgt gatcggcctc ccgaacgacc cgtccgtgcg 2760
   cctccacgag gccctcggct acaccgcccg cggcaccctc cgcgccgccg gctacaagca 2820
   cggcggctgg cacgacgtcg gcttctggca gcgcgacttc gagctgccgg ccccgccgcg 2880
   cccggtgcgc ccggtgacgc agatctccgg tggaggcggc agcggtggcg gaggctccgg 2940
   aggcggtggc tccatggcct cctccgagga cgtcatcaag gagttcatgc gcttcaaggt 3000
   gcgcatggag ggctccgtga acggccacga gttcgagatc gagggcgagg gcgagggccg 3060
   cccctacgag ggcacccaga ccgccaagct gaaggtgacc aagggcggcc ccctgccctt 3120
   cgcctgggac atcctgtccc cccagttcca gtacggctcc aaggtgtacg tgaagcaccc 3180
   cgccgacatc cccgactaca agaagctgtc cttccccgag ggcttcaagt gggagcgcgt 3240
   gatgaacttc gaggacggcg gcgtggtgac cgtgacccag gactcctccc tgcaggacgg 3300
   ctccttcatc tacaaggtga agttcatcgg cgtgaacttc ccctccgacg gccccgtaat 3360
   gcagaagaag actatgggct gggaggcctc caccgagcgc ctgtaccccc gcgacggcgt 3420
   gctgaagggc gagatccaca aggccctgaa gctgaaggac ggcggccact acctggtgga 3480
   gttcaagtcc atctacatgg ccaagaagcc cgtgcagctg cccggctact actacgtgga 3540
   ctccaagctg gacatcacct cccacaacga ggactacacc atcgtggagc agtacgagcg 3600
   cgccgagggc cgccaccacc tgttcctgta gtcaggatct gagtcgaaac ctagacttgt 3660
   ccatcttctg gattggccaa cttaattaat gtatgaaata aaaggatgca cacatagtga 3720
   catgctaatc actataatgt gggcatcaaa gttgtgtgtt atgtgtaatt actagttatc 3780
   tgaataaaag agaaagagat catccatatt tcttatccta aatgaatgtc acgtgtcttt 3840
   ataattcttt gatgaaccag atgcatttca ttaaccaaat ccatatacat ataaatatta 3900
   atcatatata attaatatca attgggttag caaaacaaat ctagtctagg tgtgttttgc 3960
   gaatgcggcc gccaccgcgg tggagctcga attcattccg attaatcgtg gcctcttgct 4020
   cttcaggatg aagagctatg tttaaacgtg caagcgctac tagacaattc agtacattaa 4080
   aaacgtccgc aatgtgttat taagttgtct aagcgtcaat ttgtttacac cacaatatat 4140
   cctgccac 4148
<210> 69
   <211> 18762
   <212> DNA
   <213> Artificial sequence
<220>
   <223> Synthetic construct
<400> 69
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgt tgaaggagcc actcagcaag 180
   ctggtacgat tgtaatacga ctcactatag ggcgaattga gcgctgttta aacgctcttc 240
   aactggaaga gcggttacgc tgtttaaacg ctcttcaact ggaagagcgg ttactaccgg 300
   ttcactagct agctgctaag gttaccagag ctggtcacct ttgtccacca acttattaag 360
   tatctagttg aagacacgtt cttcttcacg taagaagaca ctcagtagtc ttcggccaga 420
   atggcctctt gattcagcgg gcctagaagg ccggatcact gactagctaa tttaaatcct 480
   gaggatctgg tcttcctaag gacccgggat atcggaccga ttaaacttta attcggtccg 540
   ataacttcgt atagcataca ttatacgaag ttatatgcat acgcgtctta agtcgatcgc 600
   tatcaacttt gtatagaaaa gttgggccga gctcggtacg gccagaatgg cccggaccgg 660
   gttaccgaat tcaggcgacc catcgctgct ttgtctacat catgttcttc atcatcctcc 720
   ccaggcgacg cgtgctgctg ttcttattca gactaccgtt cgagtgactg catggcgtac 780
   atctttctgc atcgactttg tacggctaca tcgaacatat acacgagatg tctcgtgtga 840
   atagagtcac taatgcctta agcatcggtt actccgtagg gtacattctg ttcttcttat 900
   ttgtgcatat ttttattgtt gtttactgat tatacgagta gttatacata catgcacata 960
   catatcatca catatatcac aatatttttc taaattaaat taaaactaaa aatgactaaa 1020
   tttctaacac caacgacatt gtaatgtttt ctccaacaac tttacctatt ctacattgtt 1080
   ctatttcgaa tttcactcta taaacaacat agtctacaat ggaaaacagt gctttgtacg 1140
   actatatacg cgatgtgtgg ctacaacata agacaatata gtcgtttgaa gattgaacct 1200
   atatatcggt acggttaatc cgtctatgta cgtgggcatg acgaacaccc gtgataacga 1260
   aggattaacg tgcacaatca taaatccaaa gtaggagcgg tgcatgatga gaatcgctct 1320
   cagtactcga cataatgaac cttacgaggt acaacaggca ggcaggcagg gaccaggggc 1380
   cgcctttatt tcaggctcgc tggccccacg ggcgtgctgc gtgcacgaag ggcactaccc 1440
   caacctctca ccgaaaaccg cgctggatcg gcaaatcaaa cgaggtggtg ccccgtgccc 1500
   actctccacg tccacggcac catccctctg cagccgctca ccagccatgc cgtgtcgcgg 1560
   aacggcacaa ccacccccaa cccactcacg aaaccccgtc ccggccgtgc ccgtgtcggt 1620
   ccgcgctcgg caacgaggcg gcccgcgctg ctgagtcccc tggacacccg acaccctgtc 1680
   ggccctttgt ttattcatcc cgaaatctca tctgccccca cggccgactg cgctgcgccg 1740
   cccggatata tatacccatc gttatcgatc gactggggac tctatcagtg atagagtcta 1800
   gaggatcgct caggaaggcc gctgagatag aggcatggcg gccaatgcgg gcggcggtgg 1860
   agcgggagga ggcagcggca gcggcagcgt ggctgcgccg gcggtgtgcc gccccagcgg 1920
   ctcgcggtgg acgccgacgc cggagcagat caggatgctg aaggagctct actacggctg 1980
   cggcatccgg tcgcccagct cggagcagat ccagcgcatc accgccatgc tgcggcagca 2040
   cggcaagatc gagggcaaga acgtcttcta ctggttccag aaccacaagg cccgcgagcg 2100
   ccagaagcgc cgcctcacca gcctcgacgt caacgtgccc gccgccggcg cggccgacgc 2160
   caccaccagc caactcggcg tcctctcgct gtcgtcgccg ccgccttcag gcgcggcgcc 2220
   tccctcgccc accctcggct tctacgccgc cggcaatggc ggcggatcgg ctgtgctgct 2280
   ggacacgagt tccgactggg gcagcagcgg cgctgccatg gccaccgaga catgcttcct 2340
   gcaggactac atgggcgtga cggacacggg cagctcgtcg cagtggccac gcttctcgtc 2400
   gtcggacacg ataatggcgg cggccgcggc gcgggcggcg acgacgcggg cgcccgagac 2460
   gctccctctc ttcccgacct gcggcgacga cggcggcagc ggtagcagca gctacttgcc 2520
   gttctggggt gccgcgtcca caactgccgg cgccacttct tccgttgcga tccagcagca 2580
   acaccagctg caggagcagt acagctttta cagcaacagc aacagcaccc agctggccgg 2640
   caccggcaac caagacgtat cggcaacagc agcagcagcc gccgccctgg agctgagcct 2700
   cagctcatgg tgctcccctt accctgctgc agggagtatg tgagagcaac gcgagctgcc 2760
   actgctcttc actggtaccg ttaacagatc attcgacaaa gcagcattag tccgttgatc 2820
   ggtggaagac cactcgtcag tgttgagttg aatgtttgat caataaaata cggcaatgct 2880
   gtaagggttg ttttttatgc cattgataat acactgtact gttcagttgt tgaactctat 2940
   ttcttagcca tgccaagtgc ttttcttatt ttgaataaca ttacagcaaa aagttgaaag 3000
   acaaaaaaaa aaacccccga acagagtgct ttgggtccca agcttcttta gactgtgttc 3060
   ggcgttcccc ctaaatttct ccccctatat ctcactcact tgtcacatca gcgttctctt 3120
   tccccctata tctccacgct ctacagcagt tccacctata tcaaacctct ataccccacc 3180
   acaacaatat tatatacttt catcttcaac taactcatgt accttccaat ttttttctac 3240
   taataattat ttacgtgcac agaaacttag caaggagaga gagagcgggg tgacccacct 3300
   tgctagttgg atattacctc ttctcttcaa agtatccttg aacgctcacc ggttatcaaa 3360
   tctctacact atagctctgt agtcttgcta gatagttagt tctttagctc tcggtgacca 3420
   agcttggcgc gatcaagctt atcgataccg tcgacctcga agcttggtca cccggtccgg 3480
   gcctagaagg ccagcttcaa gtttgtacaa aaaagcaggc tccggccaga atggcccgga 3540
   ccgggttacc gaattcttac cctagctccc tgcggctgtt acgcggtccc ccatcaatct 3600
   tctgttcttg cggttgtagc ctgtgtaaca gtgctagagt atgtatgata aataggtttt 3660
   aagtctgctt acatgacatt ttttattgtg gaagagacat ataaaaatta gagagagtgg 3720
   ttctcatgca acggcggacg gcccggtgct aaaagagctt caagacaaaa taatgaaaca 3780
   ggaagagagt agatttatct aagagccaac tttattatat gaatgtgttt attgttggct 3840
   ttagatgata tggtaaggag ttagagctaa taatagatag gctctattat tattattatt 3900
   aattaaactc gctctaagga ggaaagtggg aggaagggac gaggacgaag actactggaa 3960
   gcatcgtgca tggatgatgg atgtggtgtc tcttaatgta ggtggccgga ggatgtacgt 4020
   gttaattgcg cgataagcac tcagatccaa ccgcaaacta cctccacact gacacactga 4080
   tagagagaaa gagagacctc cgacgactgc cgccgcagat gagccacgta cgtatacgac 4140
   gtctgccggc cggctcaggc tgccgccatc accctgctcg aaagtcgcgt taggcggcgc 4200
   cagctacata ggagtatcta gtctagccag ttagtatact actactgcgc tgatgatgaa 4260
   ttaactctgc atagatactg tacttgcctc cctccaacac ccaaccacct cctgctcggc 4320
   tcttaataac ttggacacgg atcgatgcca tccaaggaag aacacgacga cgacgacgga 4380
   acatccacca tgcaagcttg catccatacg ccgatacgcg tgcatccatc catccaccat 4440
   tatttccatt ttccaccgat cacacgtaca caggcctatt taaggagcga catcccactg 4500
   caactctcct caccactcat caccagctag ctctagcaaa gcacttgcca tctaccgacc 4560
   gccgcattcc aaacagcccg acgagctagc agagcggcag gcacctccct cctcaaggaa 4620
   cccatggcca ctgtgaacaa ctggctcgct ttctccctct ccccgcagga gctgccgccc 4680
   tcccagacga cggactccac actcatctcg gccgccaccg ccgaccatgt ctccggcgat 4740
   gtctgcttca acatccccca agattggagc atgaggggat cagagctttc ggcgctcgtc 4800
   gcggagccga agctggagga cttcctcggc ggcatctcct tctccgagca gcatcacaag 4860
   gccaactgca acatgatacc cagcactagc agcacagttt gctacgcgag ctcaggtgct 4920
   agcaccggct accatcacca gctgtaccac cagcccacca gctcagcgct ccacttcgcg 4980
   gactccgtaa tggtggcttc ctcggccggt gtccacgacg gcggtgccat gctcagcgcg 5040
   gccgccgcta acggtgtcgc tggcgctgcc agtgccaacg gcggcggcat cgggctgtcc 5100
   atgattaaga actggctgcg gagccaaccg gcgcccatgc agccgagggt ggcggcggct 5160
   gagggcgcgc aggggctctc tttgtccatg aacatggcgg ggacgaccca aggcgctgct 5220
   ggcatgccac ttctcgctgg agagcgcgca cgggcgcccg agagtgtatc gacgtcagca 5280
   cagggtggag ccgtcgtcgt cacggcgccg aaggaggata gcggtggcag cggtgttgcc 5340
   ggcgctctag tagccgtgag cacggacacg ggtggcagcg gcggcgcgtc ggctgacaac 5400
   acggcaagga agacggtgga cacgttcggg cagcgcacgt cgatttaccg tggcgtgaca 5460
   aggcatagat ggactgggag atatgaggca catctttggg ataacagttg cagaagggaa 5520
   gggcaaactc gtaagggtcg tcaagtctat ttaggtggct atgataaaga ggagaaagct 5580
   gctagggctt atgatcttgc tgctctgaag tactggggtg ccacaacaac aacaaatttt 5640
   ccagtgagta actacgaaaa ggagctcgag gacatgaagc acatgacaag gcaggagttt 5700
   gtagcgtctc tgagaaggaa gagcagtggt ttctccagag gtgcatccat ttacagggga 5760
   gtgactaggc atcaccaaca tggaagatgg caagcacgga ttggacgagt tgcagggaac 5820
   aaggatcttt acttgggcac cttcagcacc caggaggagg cagcggaggc gtacgacatc 5880
   gcggcgatca agttccgcgg cctcaacgcc gtcaccaact tcgacatgag ccgctacgac 5940
   gtgaagagca tcctggacag cagcgccctc cccatcggca gcgccgccaa gcgcctcaag 6000
   gaggccgagg ccgcagcgtc cgcgcagcac caccacgccg gcgtggtgag ctacgacgtc 6060
   ggccgcatcg cctcgcagct cggcgacggc ggagccctgg cggcggcgta cggcgcgcac 6120
   taccacggcg ccgcctggcc gaccatcgcg ttccagccgg gcgccgccag cacaggcctg 6180
   taccacccgt acgcgcagca gccaatgcgc ggcggcgggt ggtgcaagca ggagcaggac 6240
   cacgcggtga tcgcggccgc gcacagcctg caggacctcc accacctgaa cctgggcgcg 6300
   gccggcgcgc acgacttttt ctcggcaggg cagcaggccg ccgccgctgc gatgcacggc 6360
   ctgggtagca tcgacagtgc gtcgctcgag cacagcaccg gctccaactc cgtcgtctac 6420
   aacggcgggg tcggcgacag caacggcgcc agcgccgtcg gcggcagtgg cggtggctac 6480
   atgatgccga tgagcgctgc cggagcaacc actacatcgg caatggtgag ccacgagcag 6540
   gtgcatgcac gggcctacga cgaagccaag caggctgctc agatggggta cgagagctac 6600
   ctggtgaacg cggagaacaa tggtggcgga aggatgtctg catgggggac tgtcgtgtct 6660
   gcagccgcgg cggcagcagc aagcagcaac gacaacatgg ccgccgacgt cgggcatggc 6720
   ggcgcgcagc tcttcagtgt ctggaacgac acttaagcgt acctagtggt acctgacatc 6780
   ttatagtctg caacctctcg tgtctgaatt cctatcttta tcaagtgtta ttgcttccac 6840
   gactatagga cagctttcgt cgaaagcttt tgctcatgtg atctcgaagg attcatctag 6900
   tctgattttt cgtgacttgt atcggtttta ttggattcat ccaacatata tcaataaaaa 6960
   atgagttgtg tttcctttct tcctagttca gttaaaatta tttccctcct gcgcttgtgc 7020
   tgtaattgtc tgtgtacctg ttgtttgtga ctgtgttagt tcccttggat atgatttcgt 7080
   atttgatatg tacatggaga tagcttagct tcattattgg agtatgaagt tagtatgaca 7140
   tagtcactct cctggaaaat tgacactgca aaccatattt ttattctgaa ccacaaatcc 7200
   tagtcagtcc gctggcatat gccgtccgtt tgctgaatcc agaacgtggg tttggagatg 7260
   tacggctgag atgcctctat gcgaagggga tttcgtggtg aaacgagatg ggagtagagc 7320
   aacgcccgtg gaagatgctt caaacttcca cacttttgag caacgatcgg cagtagtaag 7380
   gtagacgatt tcaagatcaa agcatatgaa gataaacaac atcaacaaca aaatttgttg 7440
   gggttctata gagagaaaca gagctacata catacactgt tttgtatcta ccatctgaga 7500
   tgatgaaaag atgaaaaact aaagaatgcc ccggcgccaa cgccaggaca cgccgcgcgc 7560
   gcgtcacccg agccatctct tgacccagcc ggcgctgtat atttacacac gttgcagcat 7620
   cgatcaccac ctgttcgatc gcgtcgccgt caccgggatc tgagtcgaaa cctagacttg 7680
   tccatcttct ggattggcca acttaattaa tgtatgaaat aaaaggatgc acacatagtg 7740
   acatgctaat cactataatg tgggcatcaa agttgtgtgt tatgtgtaat tactagttat 7800
   ctgaataaaa gagaaagaga tcatccatat ttcttatcct aaatgaatgt cacgtgtctt 7860
   tataattctt tgatgaacca gatgcatttc attaaccaaa tccatataca tataaatatt 7920
   aatcatatat aattaatatc aattgggtta gcaaaacaaa tctagtctag gtgtgttttg 7980
   cgaatgcgac cttcttatgt gcttctagtc tccaaatgtg gttgatagtt attttgctct 8040
   aagatcaaca gtaatgaagt ataaatcatc gttgtggtgt gctactcggt taattgagca 8100
   ttaacacaca caaacatgac gaggatggta taatctccaa aaatgtgtac tttgttaggt 8160
   gggaccctat agccttgatt aatgtgctat gttaggcatg cctggaaacg tgtgacgcat 8220
   atgttttgtg aacctgttga tattatatgt gcttttatat taccatattt tattaaaata 8280
   ctaatattta ttactagtaa gatataacat tctatctagc ttaaaaacta accataaata 8340
   ttccataata actagattta ccaaactaat atactaaata tacataataa atacaaaatt 8400
   aacaagacaa taatcaatat ttatgagctt aatatattta gacattatgg ttggtcgacg 8460
   ataatcatgc taacttttcg taattgcttg attgaaatat gcttagaata atgcctcttt 8520
   gttctacatg gcaaataggg accattatgg tgtaacaccc tgggaaccac aaacaccccg 8580
   aaatgctact aaactacaca actaaccttc atatataaaa tttcgacagc atctcctttg 8640
   aaaatttgca tagacgtgga agcaacagag tataaacaga tatcatgata agaaaacata 8700
   ctagacatta ataatctgct agaaatggga agaatcctaa cttgacgact gcgtaactga 8760
   ctagagtcac acttagctga ccctagtcac ttacaactga cttcgtgtcc tagatcgatg 8820
   ggccctggcc gaagcttggt cacccggtcc gggcctagaa ggccagcttc aagtttgtac 8880
   aaaaaagcag gctccggcca gaatggcccg gaccgggtta ccgaattcga gctcggtacc 8940
   ctgggatccg attgactatc tcattcctca aaccaaacac ctcaaatata tctgctatcg 9000
   ggattggcat tcctgtatcc ctacgcccgt gtaccccctg tttagagaac ctccaaaggt 9060
   ataagatggc gaagattatt gttgtcttgt ctttcatcat atatcgagtc tttccctagg 9120
   atattattat tggcaatgag cattacacgg ttaatcgatt gagagaacat gcatctcacc 9180
   ttcagcaaat aattacgata atccatattt tacgcttcgt aacttctcat gagtttcgat 9240
   atacaaattt gttttctgga caccctacca ttcatcctct tcggagaaga gaggaagtgt 9300
   cctcaattta aatatgttgt catgctgtag ttcttcacaa aatctcaaca ggtaccaagc 9360
   acattgtttc cacaaattat attttagtca caataaatct atattattat taatatacta 9420
   aaactatact gacgctcaga tgcttttact agttcttgct agtatgtgat gtaggtctac 9480
   gtggaccaga aaatagtgag acacggaaga caaaagaagt aaaagaggcc cggactacgg 9540
   cccacatgag attcggcccc gccacctccg gcaaccagcg gccgatccaa cggcagtgcg 9600
   cgcacacaca caacctcgta tatatcgccg cgcggaagcg gcgcgaccga ggaagccttg 9660
   tcctcgacac cccctacaca ggtgtcgcgc tgcccccgac acgagtcccg catgcgtccc 9720
   acgcggccgc gccagatccc gcctccgcgc gttgccacgc cctctataaa cacccagctc 9780
   tccctcgccc tcatctacct cactcgtagt cgtagctcaa gcatcagcgg cagcggcagc 9840
   ggcaggagct ctgggcagcg tgcgcacgtg gggtacctag ctcgctctgc tagcctacct 9900
   taagatatcg gatccatgtc caacctgctc acggttcacc agaaccttcc ggctcttcca 9960
   gtggacgcga cgtccgatga agtcaggaag aacctcatgg acatgttccg cgacaggcaa 10020
   gcgttcagcg agcacacctg gaagatgctg ctctccgtct gccgctcctg ggctgcatgg 10080
   tgcaagctga acaacaggaa gtggttcccc gctgagcccg aggacgtgag ggattacctt 10140
   ctgtacctgc aagcgcgagg tttgtttctg cttctacctt tgatatatat ataataatta 10200
   tcattaatta gtagtaatat aatatttcaa atattttttt caaaataaaa gaatgtagta 10260
   tatagcaatt gcttttctgt agtttataag tgtgtatatt ttaatttata acttttctaa 10320
   tatatgacca aaacatggtg atgcctaggt ctggcagtga agaccatcca gcaacacctt 10380
   ggacaactga acatgcttca caggcgctcc ggcctcccgc gccccagcga ctcgaacgcc 10440
   gtgagcctcg tcatgcgccg catcaggaag gaaaacgtcg atgccggcga aagggcaaag 10500
   caggccctcg cgttcgagag gaccgatttc gaccaggtcc gcagcctgat ggagaacagc 10560
   gacaggtgcc aggacattag gaacctggcg ttcctcggaa ttgcatacaa cacgctcctc 10620
   aggatcgcgg aaattgcccg cattcgcgtg aaggacatta gccgcaccga cggcggcagg 10680
   atgcttatcc acattggcag gaccaagacg ctcgtttcca ccgcaggcgt cgaaaaggcc 10740
   ctcagcctcg gagtgaccaa gctcgtcgaa cgctggatct ccgtgtccgg cgtcgcggac 10800
   gacccaaaca actacctctt ctgccgcgtc cgcaagaacg gggtggctgc ccctagcgcc 10860
   accagccaac tcagcacgag ggccttggaa ggtattttcg aggccaccca ccgcctgatc 10920
   tacggcgcga aggatgacag cggtcaacgc tacctcgcat ggtccgggca ctccgcccgc 10980
   gttggagctg ctagggacat ggcccgcgcc ggtgtttcca tccccgaaat catgcaggcg 11040
   ggtggatgga cgaacgtgaa cattgtcatg aactacattc gcaaccttga cagcgagacg 11100
   ggcgcaatgg ttcgcctcct ggaagatggt gactgaggta cccaacctag acttgtccat 11160
   cttctggatt ggccaactta attaatgtat gaaataaaag gatgcacaca tagtgacatg 11220
   ctaatcacta taatgtgggc atcaaagttg tgtgttatgt gtaattacta gttatctgaa 11280
   taaaagagaa agagatcatc catatttctt atcctaaatg aatgtcacgt gtctttataa 11340
   ttctttgatg aaccagatgc atttcattaa ccaaatccat atacatataa atattaatca 11400
   tatataatta atatcaattg ggttagcaaa acaaatctag tctaggtgtg ttttgcgatc 11460
   cgatatcgat gggccctggc cgaagcttgg tcacccggtc cgggcctaga aggccgatct 11520
   cccgggcacc cagctttctt gtacaaagtg gccgttaacg gatcggccag aatggcccgg 11580
   accgggttac cgaattcctg cccttaaggc caattgttca agattcattc aacaattgaa 11640
   acatctccca tgattaaatc agtataaggt tgctatggtc ttgcttgaca aagttttttt 11700
   ttgagggaat ttcaactaaa tttttgagtg aaactatcaa atactgattt taaaaaattt 11760
   ttataaaagg aagcgcagag ataaaaggcc atctatgcta caaaagtacc caaaaatgta 11820
   atcctaaagt atgaattgca tttttttttt gtttggacga aaggaaagga gtattaccac 11880
   aagaatgata tcatcttcat atttagatct tttttgggta aagcttgaga ttctctaaat 11940
   atagagaaat cagaagaaaa aaaaaccgtg ttttggtggt tttgatttct agcctccaca 12000
   ataactttga cggcgtcgac aagtctaacg gacaccaagc agcgaaccac cagcgccgag 12060
   ccaagcgaag cagacggccg agacgttgac accttcggcg cggcatctct cgagagttcc 12120
   gctccggcgc tccacctcca ccgctggcgg tttcttattc cgttccgttc cgcctcctgc 12180
   tctgctcctc tccacaccac acggcacgaa accgttacgg caccggcagc acccagcacg 12240
   ggagagggga ttcctttccc accgttcctt ccctttccgc cccgccgcta taaatagcca 12300
   gccccatccc cagctttttt ccccaatctc atctcctctc tcctgttgtt cggagcacac 12360
   gcacaatccg atcgatcccc aaatcccctt cgtctctcct cgcgagcctc gtggatccca 12420
   gcttcaaggt acggcgatcg atcatccccc ctccttctct ctaccttctt ttctctagac 12480
   tacatcggat ggcgatccat ggttagggcc tgctagtttc ccttcctgtt ttgtcgatgg 12540
   ctgcgaggca caatagatct gatggcgtta tgacggctaa cttgtcatgt tgttgcgatt 12600
   tatagtccct ttaggagatc agtttaattt ctcggatggt tcgagatcgg tggtccatgg 12660
   ttagtaccct aagatccgcg ctgttagggt tcgtagatgg aggcgacctg ttctgattgt 12720
   taacttgtca gtacctggga aatcctggga tggttctagc tcgtccgcag atgagatcga 12780
   tttcatgatc ctctgtatct tgtttcgttg cctaggttcc gtctaatcta tccgtggtat 12840
   gatgtagatg ttttgatcgt gctaactacg tcttgtaaag ttaattgtca ggtcataatt 12900
   tttagcatgc cttttttttt gtttggtttt gtctaattgg gctgtcgttc tagatcagag 12960
   tagaagactg ttccaaacta cctgctggat ttattgaact tggatctgta tgtgtgtcac 13020
   atatcttcat aaattcatga ttaagatgga ttgaaatatc ttttatcttt ttggtatgga 13080
   tagttctata tgttggtgtg gctttgttag atgtatacat gcttagatac atgaagcaac 13140
   gtgctgctac tgtttagtaa ttgctgttca tttgtctaat aaacagataa ggatatgtat 13200
   ttatgttgct gttggttttg ctggtacttt gttggataca aatgcttcaa tacagaaaac 13260
   agcatgctgc tacgatttac catttatcta atcttatcat atgtctaatc taataaacaa 13320
   acatgctttt aaattatctt catatgcttg gatgatggca tacacagcgg ctatgtgtgg 13380
   ttttttaaat acccagcatc atgggcatgc atgacactgc tttaatatgc tttttatttg 13440
   cttgagactg tttcttttgt ttatactgac cctttagttc ggtgactctt ctgcaggtcg 13500
   actttaactt agcctaggat ccatggccca gtccaagcac ggcctgacca aggagatgac 13560
   catgaagtac cgcatggagg gctgcgtgga cggccacaag ttcgtgatca ccggcgaggg 13620
   catcggctac cccttcaagg gcaagcaggc catcaacctg tgcgtggtgg agggcggccc 13680
   cttgcccttc gccgaggaca tcttgtccgc cgccttcatg tacggcaacc gcgtgttcac 13740
   cgagtacccc caggacatcg tcgactactt caagaactcc tgccccgccg gctacacctg 13800
   ggaccgctcc ttcctgttcg aggacggcgc cgtgtgcatc tgcaacgccg acatcaccgt 13860
   gagcgtggag gagaactgca tgtaccacga gtccaagttc tacggcgtga acttccccgc 13920
   cgacggcccc gtgatgaaga agatgaccga caactgggag ccctcctgcg agaagatcat 13980
   ccccgtgccc aagcagggca tcttgaaggg cgacgtgagc atgtacctgc tgctgaagga 14040
   cggtggccgc ttgcgctgcc agttcgacac cgtgtacaag gccaagtccg tgccccgcaa 14100
   gatgcccgac tggcacttca tccagcacaa gctgacccgc gaggaccgca gcgacgccaa 14160
   gaaccagaag tggcacctga ccgagcacgc catcgcctcc ggctccgcct tgccctccgg 14220
   actcagatct cgatagggta ccaatggcca gttaacagat ccagctgctg ctgttctagg 14280
   gttcacaagt ctgcctattt gtcttcccca atggagctat ggttgtctgg tctggtcctt 14340
   ggtcgtgtcc cgtttcattg tgtactattt acctgtaatg tgtatcctta agtctggttt 14400
   gatggtgtct gaaacgtttt gctgtggtag agcagcatgg aagaactata atgaataagt 14460
   gatccctaat cattgtgtcc aaattttgct tctgctatac ccttttgtgc tgtttcttat 14520
   gttttgctta aaaatttgat ctgacaaaca aatttgtcta aattatgctc ttgttctgac 14580
   tgtgtactgt acacatttgt attgctaccg gtttggcaca tagttttctc tttgattgca 14640
   gatcaaaccc ttctgattgc agattgcttg gctatggctg atgtgctcac tgttcttctt 14700
   tagcttgtat attgctgatg gtggcttggc tacggctgaa aaagttgctg ctgcgccgac 14760
   ttggcaactg ttgctccagt aatgctttgt cctctcttcc tagccgccga cttggcactt 14820
   ggcagctgtt gctcctgtaa tgcgttgtta tctcttatct cttgagatgt actatgattt 14880
   tgaggctgtt tgacttgtgc cagattgcca gtgctgatgc tatattatct caggttttta 14940
   tgctagtata tgttttactg tgtgctgcct gtgtcgagtt acgtaatcta ttgaaccgct 15000
   atgcgtgttg atgccctgtt gttgcttgcc agattgatgc ccctgttgct tgcagcttgc 15060
   aagctgtagt gaaatcctaa aattcgaaac caaatctatt ctgaactttt gaagatctgt 15120
   gatgtccgga agaaaagatt caaacagata aacgggactc tccagaatct ataaaataaa 15180
   tgatcgacaa acgctttgct ttggtaggct tcctagttag ttagcggacc gaagcttcgg 15240
   tccgggccta gaaggccagc ttcggccgcc ccgggcaact ttattataca aagttgatag 15300
   atcgaataac ttcgtatagc atacattata cgaagttata cctggtggcg ccgctagggg 15360
   ctgcaggaat tcgatatcaa gcttatcgat accgtcgacc tcgagggggg gcccggtacc 15420
   ctgggatctg cccttacttt aacgcctcta accaacaccc ctttatcttt ataaggaaca 15480
   ataaacagaa tttgccccac tgttctaaat cacctaataa tatccccagc taaaaacaat 15540
   aaaggtttcc tagaattaag acaagcatga ctgttcctcc aggagggttt ggaacattgt 15600
   tgcagtcttg cagatacggg cgaagggtga gaaacagagc ggagggctgg aggtgacctc 15660
   ggtagtcgac gccggagttg agcttgacaa cgacggggcg gcccctgatg gacttgagga 15720
   agtccgatgg cgtcttcacc gtcccgccgg cgcccgaggc gggcctgtcg ctgccgccgc 15780
   cgccgctgct catcttgcgc gctgtgcccc cggcggtgtc cctgtgttgc ggatcgcggg 15840
   tgggccaggt ggatgcgagg gcgacccgtt tggactccgg ccggagccgc cggatccctg 15900
   gtcggtgtca gtgccgttta ctctgggccc cacgtgtcag taccgtctgt agatgacaac 15960
   aacccgtcgt ccacagtcat gtccaaaata tcctttcttc ttttttttcg attcggatat 16020
   ctatcttcct tttttttttc caaaaatctt cttgacgcac cagcgcgcac gtttgtggta 16080
   aacgccgaca cgtcggtccc acgtcgatag accccaccca ccagtgagta gcgtgtacgt 16140
   attcgggggt gacggacgtg tcgccgtcgt cttgctagtc ccattcccat ctgagccaca 16200
   catctctgaa caaaaaaaag gagggaggcc tccacgcaca tccccctccg tgccacccgc 16260
   cccaaaccct cgcgccgcct ccgagacagc cgccgcaacc atggccaccg ccgccgccgc 16320
   gtctaccgcg ctcactggcg ccactaccgc tgcgcccaag gcgaggcgcc gggcgcacct 16380
   cctggccacc cgccgcgccc tcgccgcgcc catcaggtgc tcagcggcgt cacccgccat 16440
   gccgatggct cccccggcca ccccgctccg gccgtggggc cccaccgagc cccgcaaggg 16500
   tgctgacatc ctcgtcgagt ccctcgagcg ctgcggcgtc cgcgacgtct tcgcctaccc 16560
   cggcggcgcg tccatggaga tccaccaggc actcacccgc tcccccgtca tcgccaacca 16620
   cctcttccgc cacgagcaag gggaggcctt tgccgcctcc ggctacgcgc gctcctcggg 16680
   ccgcgtcggc gtctgcatcg ccacctccgg ccccggcgcc accaacctag tctccgcgct 16740
   cgccgacgcg ctgctcgatt ccgtccccat ggtcgccatc acgggacagg tggcgcgacg 16800
   catgattggc accgacgcct tccaggagac gcccatcgtc gaggtcaccc gctccatcac 16860
   caagcacaac tacctggtcc tcgacgtcga cgacatcccc cgcgtcgtgc aggaggcttt 16920
   cttcctcgcc tcctctggtc gaccagggcc ggtgcttgtc gacatcccca aggacatcca 16980
   gcagcagatg gcggtgcctg tctgggacaa gcccatgagt ctgcctgggt acattgcgcg 17040
   ccttcccaag ccccctgcga ctgagttgct tgagcaggtg ctgcgtcttg ttggtgaatc 17100
   gcggcgccct gttctttatg tgggcggtgg ctgcgcagca tctggtgagg agttgcgacg 17160
   ctttgtggag ctgactggaa tcccggtcac aactactctt atgggcctcg gcaacttccc 17220
   cagcgacgac ccactgtctc tgcgcatgct aggtatgcat gggacggtgt atgcaaatta 17280
   tgcagtggat aaggccgatc tgttgcttgc acttggtgtg cggtttgatg atcgcgtgac 17340
   agggaagatt gaggcttttg caagcagggc taagattgtg cacgttgata ttgatccggc 17400
   tgagattggc aagaacaagc agccacatgt gtccatctgt gcagatgtta agcttgcttt 17460
   gcagggcatg aatgctcttc ttgaaggaag cacatcaaag aagagctttg actttggctc 17520
   atggaacgat gagttggatc agcagaagag ggaattcccc cttgggtata aaacatctaa 17580
   tgaggagatc cagccacaat atgctattca ggttcttgat gagctgacga aaggcgaggc 17640
   catcatcggc acaggtgttg ggcagcacca gatgtgggcg gcacagtact acacttacaa 17700
   gcggccaagg cagtggttgt cttcagctgg tcttggggct atgggatttg gtttgccggc 17760
   tgctgctggt gcttctgtgg caaacccagg tgtcactgtt gttgacatcg atggagatgg 17820
   tagctttctc atgaacgttc aggagctagc tatgatccga attgagaacc tcccagtgaa 17880
   ggtctttgtg ctaaacaacc agcacctggg gatggtggtg cagttggagg acaggttcta 17940
   taaggccaac agagcgcaca catacttggg aaacccagag aatgaaagtg agatatatcc 18000
   agatttcgtg acgatcgcca aagggttcaa cattccagcg gtccgtgtga caaagaagaa 18060
   cgaagtccgc gcagcgataa agaagatgct cgagactcca gggccgtacc tcttggatat 18120
   aatcgtccca caccaggagc atgtgttgcc tatgatccct agtggtgggg ctttcaagga 18180
   tatgatcctg gatggtgatg gcaggactgt gtactgacta gctagtcagt taacctagac 18240
   ttgtccatct tctggattgg ccaacttaat taatgtatga aataaaagga tgcacacata 18300
   gtgacatgct aatcactata atgtgggcat caaagttgtg tgttatgtgt aattactagt 18360
   tatctgaata aaagagaaag agatcatcca tatttcttat cctaaatgaa tgtcacgtgt 18420
   ctttataatt ctttgatgaa ccagatgcat ttcattaacc aaatccatat acatataaat 18480
   attaatcata tataattaat atcaattggg ttagcaaaac aaatctagtc taggtgtgtt 18540
   ttgcgaatgc ggccgcctgc agagttaacg gcgcgccgac tagctagcta aggtaccgag 18600
   ctcgaattca ttccgattaa tcgtggcctc ttgctcttca ggatgaagag ctatgtttaa 18660
   acgtgcaagc gctactagac aattcagtac attaaaaacg tccgcaatgt gttattaagt 18720
   tgtctaagcg tcaatttgtt tacaccacaa tatatcctgc ca 18762
<210> 70
   <211> 18716
   <212> DNA
   <213> Artificial sequence
<220>
   <223> Synthetic construct
<400> 70
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgt tgaaggagcc actcagcaag 180
   ctggtacgat tgtaatacga ctcactatag ggcgaattga gcgctgttta aacgctcttc 240
   aactggaaga gcggttacgc tgtttaaacg ctcttcaact ggaagagcgg ttactaccgg 300
   ttcactagct agctgctaag gttaccagag ctggtcacct ttgtccacca acttattaag 360
   tatctagttg aagacacgtt cttcttcacg taagaagaca ctcagtagtc ttcggccaga 420
   atggcctctt gattcagcgg gcctagaagg ccggatcact gactagctaa tttaaatcct 480
   gaggatctgg tcttcctaag gacccgggat atcggaccga ttaaacttta attcggtccg 540
   ataacttcgt atagcataca ttatacgaag ttatatgcat acgcgtctta agtcgatcgc 600
   tatcaacttt gtatagaaaa gttgggccga gctcggtacg gccagaatgg cccggaccgg 660
   gttaccgaat tcaggcgacc catcgctgct ttgtctacat catgttcttc atcatcctcc 720
   ccaggcgacg cgtgctgctg ttcttattca gactaccgtt cgagtgactg catggcgtac 780
   atctttctgc atcgactttg tacggctaca tcgaacatat acacgagatg tctcgtgtga 840
   atagagtcac taatgcctta agcatcggtt actccgtagg gtacattctg ttcttcttat 900
   ttgtgcatat ttttattgtt gtttactgat tatacgagta gttatacata catgcacata 960
   catatcatca catatatcac aatatttttc taaattaaat taaaactaaa aatgactaaa 1020
   tttctaacac caacgacatt gtaatgtttt ctccaacaac tttacctatt ctacattgtt 1080
   ctatttcgaa tttcactcta taaacaacat agtctacaat ggaaaacagt gctttgtacg 1140
   actatatacg cgatgtgtgg ctacaacata agacaatata gtcgtttgaa gattgaacct 1200
   atatatcggt acggttaatc cgtctatgta cgtgggcatg acgaacaccc gtgataacga 1260
   aggattaacg tgcacaatca taaatccaaa gtaggagcgg tgcatgatga gaatcgctct 1320
   cagtactcga cataatgaac cttacgaggt acaacaggca ggcaggcagg gaccaggggc 1380
   cgcctttatt tcaggctcgc tggccccacg ggcgtgctgc gtgcacgaag ggcactaccc 1440
   caacctctca ccgaaaaccg cgctggatcg gcaaatcaaa cgaggtggtg ccccgtgccc 1500
   actctccacg tccacggcac catccctctg cagccgctca ccagccatgc cgtgtcgcgg 1560
   aacggcacaa ccacccccaa cccactcacg aaaccccgtc ccggccgtgc ccgtgtcggt 1620
   ccgcgctcgg caacgaggcg gcccgcgctg ctgagtcccc tggacacccg acaccctgtc 1680
   ggccctttgt ttattcatcc cgaaatctca tctgccccca cggccgactg cgctgcgccg 1740
   cccggatata tatacccatc gttatcgatc gactggggac tctatcagtg atagagtcta 1800
   gaggatcgct caggaaggcc gctgagatag aggcatggcg gccaatgcgg gcggcggtgg 1860
   agcgggagga ggcagcggca gcggcagcgt ggctgcgccg gcggtgtgcc gccccagcgg 1920
   ctcgcggtgg acgccgacgc cggagcagat caggatgctg aaggagctct actacggctg 1980
   cggcatccgg tcgcccagct cggagcagat ccagcgcatc accgccatgc tgcggcagca 2040
   cggcaagatc gagggcaaga acgtcttcta ctggttccag aaccacaagg cccgcgagcg 2100
   ccagaagcgc cgcctcacca gcctcgacgt caacgtgccc gccgccggcg cggccgacgc 2160
   caccaccagc caactcggcg tcctctcgct gtcgtcgccg ccgccttcag gcgcggcgcc 2220
   tccctcgccc accctcggct tctacgccgc cggcaatggc ggcggatcgg ctgtgctgct 2280
   ggacacgagt tccgactggg gcagcagcgg cgctgccatg gccaccgaga catgcttcct 2340
   gcaggactac atgggcgtga cggacacggg cagctcgtcg cagtggccac gcttctcgtc 2400
   gtcggacacg ataatggcgg cggccgcggc gcgggcggcg acgacgcggg cgcccgagac 2460
   gctccctctc ttcccgacct gcggcgacga cggcggcagc ggtagcagca gctacttgcc 2520
   gttctggggt gccgcgtcca caactgccgg cgccacttct tccgttgcga tccagcagca 2580
   acaccagctg caggagcagt acagctttta cagcaacagc aacagcaccc agctggccgg 2640
   caccggcaac caagacgtat cggcaacagc agcagcagcc gccgccctgg agctgagcct 2700
   cagctcatgg tgctcccctt accctgctgc agggagtatg tgagagcaac gcgagctgcc 2760
   actgctcttc actggtaccg ttaacagatc attcgacaaa gcagcattag tccgttgatc 2820
   ggtggaagac cactcgtcag tgttgagttg aatgtttgat caataaaata cggcaatgct 2880
   gtaagggttg ttttttatgc cattgataat acactgtact gttcagttgt tgaactctat 2940
   ttcttagcca tgccaagtgc ttttcttatt ttgaataaca ttacagcaaa aagttgaaag 3000
   acaaaaaaaa aaacccccga acagagtgct ttgggtccca agcttcttta gactgtgttc 3060
   ggcgttcccc ctaaatttct ccccctatat ctcactcact tgtcacatca gcgttctctt 3120
   tccccctata tctccacgct ctacagcagt tccacctata tcaaacctct ataccccacc 3180
   acaacaatat tatatacttt catcttcaac taactcatgt accttccaat ttttttctac 3240
   taataattat ttacgtgcac agaaacttag caaggagaga gagagcgggg tgacccacct 3300
   tgctagttgg atattacctc ttctcttcaa agtatccttg aacgctcacc ggttatcaaa 3360
   tctctacact atagctctgt agtcttgcta gatagttagt tctttagctc tcggtgacca 3420
   agcttggcgc gatcaagctt atcgataccg tcgacctcga agcttggtca cccggtccgg 3480
   gcctagaagg ccagcttcaa gtttgtacaa aaaagcaggc tccggccaga atggcccgga 3540
   ccgggttacc gaattcttac cctagctccc tgcggctgtt acgcggtccc ccatcaatct 3600
   tctgttcttg cggttgtagc ctgtgtaaca gtgctagagt atgtatgata aataggtttt 3660
   aagtctgctt acatgacatt ttttattgtg gaagagacat ataaaaatta gagagagtgg 3720
   ttctcatgca acggcggacg gcccggtgct aaaagagctt caagacaaaa taatgaaaca 3780
   ggaagagagt agatttatct aagagccaac tttattatat gaatgtgttt attgttggct 3840
   ttagatgata tggtaaggag ttagagctaa taatagatag gctctattat tattattatt 3900
   aattaaactc gctctaagga ggaaagtggg aggaagggac gaggacgaag actactggaa 3960
   gcatcgtgca tggatgatgg atgtggtgtc tcttaatgta ggtggccgga ggatgtacgt 4020
   gttaattgcg cgataagcac tcagatccaa ccgcaaacta cctccacact gacacactga 4080
   tagagagaaa gagagacctc cgacgactgc cgccgcagat gagccacgta cgtatacgac 4140
   gtctgccggc cggctcaggc tgccgccatc accctgctcg aaagtcgcgt taggcggcgc 4200
   cagctacata ggagtatcta gtctagccag ttagtatact actactgcgc tgatgatgaa 4260
   ttaactctgc atagatactg tacttgcctc cctccaacac ccaaccacct cctgctcggc 4320
   tcttaataac ttggacacgg atcgatgcca tccaaggaag aacacgacga cgacgacgga 4380
   acatccacca tgcaagcttg catccatacg ccgatacgcg tgcatccatc catccaccat 4440
   tatttccatt ttccaccgat cacacgtaca caggcctatt taaggagcga catcccactg 4500
   caactctcct caccactcat caccagctag ctctagcaaa gcacttgcca tctaccgacc 4560
   gccgcattcc aaacagcccg acgagctagc agagcggcag gcacctccct cctcaaggaa 4620
   cccatggcca ctgtgaacaa ctggctcgct ttctccctct ccccgcagga gctgccgccc 4680
   tcccagacga cggactccac actcatctcg gccgccaccg ccgaccatgt ctccggcgat 4740
   gtctgcttca acatccccca agattggagc atgaggggat cagagctttc ggcgctcgtc 4800
   gcggagccga agctggagga cttcctcggc ggcatctcct tctccgagca gcatcacaag 4860
   gccaactgca acatgatacc cagcactagc agcacagttt gctacgcgag ctcaggtgct 4920
   agcaccggct accatcacca gctgtaccac cagcccacca gctcagcgct ccacttcgcg 4980
   gactccgtaa tggtggcttc ctcggccggt gtccacgacg gcggtgccat gctcagcgcg 5040
   gccgccgcta acggtgtcgc tggcgctgcc agtgccaacg gcggcggcat cgggctgtcc 5100
   atgattaaga actggctgcg gagccaaccg gcgcccatgc agccgagggt ggcggcggct 5160
   gagggcgcgc aggggctctc tttgtccatg aacatggcgg ggacgaccca aggcgctgct 5220
   ggcatgccac ttctcgctgg agagcgcgca cgggcgcccg agagtgtatc gacgtcagca 5280
   cagggtggag ccgtcgtcgt cacggcgccg aaggaggata gcggtggcag cggtgttgcc 5340
   ggcgctctag tagccgtgag cacggacacg ggtggcagcg gcggcgcgtc ggctgacaac 5400
   acggcaagga agacggtgga cacgttcggg cagcgcacgt cgatttaccg tggcgtgaca 5460
   aggcatagat ggactgggag atatgaggca catctttggg ataacagttg cagaagggaa 5520
   gggcaaactc gtaagggtcg tcaagtctat ttaggtggct atgataaaga ggagaaagct 5580
   gctagggctt atgatcttgc tgctctgaag tactggggtg ccacaacaac aacaaatttt 5640
   ccagtgagta actacgaaaa ggagctcgag gacatgaagc acatgacaag gcaggagttt 5700
   gtagcgtctc tgagaaggaa gagcagtggt ttctccagag gtgcatccat ttacagggga 5760
   gtgactaggc atcaccaaca tggaagatgg caagcacgga ttggacgagt tgcagggaac 5820
   aaggatcttt acttgggcac cttcagcacc caggaggagg cagcggaggc gtacgacatc 5880
   gcggcgatca agttccgcgg cctcaacgcc gtcaccaact tcgacatgag ccgctacgac 5940
   gtgaagagca tcctggacag cagcgccctc cccatcggca gcgccgccaa gcgcctcaag 6000
   gaggccgagg ccgcagcgtc cgcgcagcac caccacgccg gcgtggtgag ctacgacgtc 6060
   ggccgcatcg cctcgcagct cggcgacggc ggagccctgg cggcggcgta cggcgcgcac 6120
   taccacggcg ccgcctggcc gaccatcgcg ttccagccgg gcgccgccag cacaggcctg 6180
   taccacccgt acgcgcagca gccaatgcgc ggcggcgggt ggtgcaagca ggagcaggac 6240
   cacgcggtga tcgcggccgc gcacagcctg caggacctcc accacctgaa cctgggcgcg 6300
   gccggcgcgc acgacttttt ctcggcaggg cagcaggccg ccgccgctgc gatgcacggc 6360
   ctgggtagca tcgacagtgc gtcgctcgag cacagcaccg gctccaactc cgtcgtctac 6420
   aacggcgggg tcggcgacag caacggcgcc agcgccgtcg gcggcagtgg cggtggctac 6480
   atgatgccga tgagcgctgc cggagcaacc actacatcgg caatggtgag ccacgagcag 6540
   gtgcatgcac gggcctacga cgaagccaag caggctgctc agatggggta cgagagctac 6600
   ctggtgaacg cggagaacaa tggtggcgga aggatgtctg catgggggac tgtcgtgtct 6660
   gcagccgcgg cggcagcagc aagcagcaac gacaacatgg ccgccgacgt cgggcatggc 6720
   ggcgcgcagc tcttcagtgt ctggaacgac acttaagcgt acctagtggt acctgacatc 6780
   ttatagtctg caacctctcg tgtctgaatt cctatcttta tcaagtgtta ttgcttccac 6840
   gactatagga cagctttcgt cgaaagcttt tgctcatgtg atctcgaagg attcatctag 6900
   tctgattttt cgtgacttgt atcggtttta ttggattcat ccaacatata tcaataaaaa 6960
   atgagttgtg tttcctttct tcctagttca gttaaaatta tttccctcct gcgcttgtgc 7020
   tgtaattgtc tgtgtacctg ttgtttgtga ctgtgttagt tcccttggat atgatttcgt 7080
   atttgatatg tacatggaga tagcttagct tcattattgg agtatgaagt tagtatgaca 7140
   tagtcactct cctggaaaat tgacactgca aaccatattt ttattctgaa ccacaaatcc 7200
   tagtcagtcc gctggcatat gccgtccgtt tgctgaatcc agaacgtggg tttggagatg 7260
   tacggctgag atgcctctat gcgaagggga tttcgtggtg aaacgagatg ggagtagagc 7320
   aacgcccgtg gaagatgctt caaacttcca cacttttgag caacgatcgg cagtagtaag 7380
   gtagacgatt tcaagatcaa agcatatgaa gataaacaac atcaacaaca aaatttgttg 7440
   gggttctata gagagaaaca gagctacata catacactgt tttgtatcta ccatctgaga 7500
   tgatgaaaag atgaaaaact aaagaatgcc ccggcgccaa cgccaggaca cgccgcgcgc 7560
   gcgtcacccg agccatctct tgacccagcc ggcgctgtat atttacacac gttgcagcat 7620
   cgatcaccac ctgttcgatc gcgtcgccgt caccgggatc tgagtcgaaa cctagacttg 7680
   tccatcttct ggattggcca acttaattaa tgtatgaaat aaaaggatgc acacatagtg 7740
   acatgctaat cactataatg tgggcatcaa agttgtgtgt tatgtgtaat tactagttat 7800
   ctgaataaaa gagaaagaga tcatccatat ttcttatcct aaatgaatgt cacgtgtctt 7860
   tataattctt tgatgaacca gatgcatttc attaaccaaa tccatataca tataaatatt 7920
   aatcatatat aattaatatc aattgggtta gcaaaacaaa tctagtctag gtgtgttttg 7980
   cgaatgcgac cttcttatgt gcttctagtc tccaaatgtg gttgatagtt attttgctct 8040
   aagatcaaca gtaatgaagt ataaatcatc gttgtggtgt gctactcggt taattgagca 8100
   ttaacacaca caaacatgac gaggatggta taatctccaa aaatgtgtac tttgttaggt 8160
   gggaccctat agccttgatt aatgtgctat gttaggcatg cctggaaacg tgtgacgcat 8220
   atgttttgtg aacctgttga tattatatgt gcttttatat taccatattt tattaaaata 8280
   ctaatattta ttactagtaa gatataacat tctatctagc ttaaaaacta accataaata 8340
   ttccataata actagattta ccaaactaat atactaaata tacataataa atacaaaatt 8400
   aacaagacaa taatcaatat ttatgagctt aatatattta gacattatgg ttggtcgacg 8460
   ataatcatgc taacttttcg taattgcttg attgaaatat gcttagaata atgcctcttt 8520
   gttctacatg gcaaataggg accattatgg tgtaacaccc tgggaaccac aaacaccccg 8580
   aaatgctact aaactacaca actaaccttc atatataaaa tttcgacagc atctcctttg 8640
   aaaatttgca tagacgtgga agcaacagag tataaacaga tatcatgata agaaaacata 8700
   ctagacatta ataatctgct agaaatggga agaatcctaa cttgacgact gcgtaactga 8760
   ctagagtcac acttagctga ccctagtcac ttacaactga cttcgtgtcc tagatcgatg 8820
   ggccctggcc gaagcttggt cacccggtcc gggcctagaa ggccagcttc aagtttgtac 8880
   aaaaaagcag gctccggcca gaatggcccg gaccgaagct ggccgctcta gaactagtgg 8940
   atctcgatgt gtagtctacg agaagggtta accgtctctt cgtgagaata accgtggcct 9000
   aaaaataagc cgatgaggat aaataaaatg tggtggtaca gtacttcaag aggtttactc 9060
   atcaagagga tgcttttccg atgagctcta gtagtacatc ggacctcaca tacctccatt 9120
   gtggtgaaat attttgtgct catttagtga tgggtaaatt ttgtttatgt cactctaggt 9180
   tttgacattt cagttttgcc actcttaggt tttgacaaat aatttccatt ccgcggcaaa 9240
   agcaaaacaa ttttatttta cttttaccac tcttagcttt cacaatgtat cacaaatgcc 9300
   actctagaaa ttctgtttat gccacagaat gtgaaaaaaa acactcactt atttgaagcc 9360
   aaggtgttca tggcatggaa atgtgacata aagtaacgtt cgtgtataag aaaaaattgt 9420
   actcctcgta acaagagacg gaaacatcat gagacaatcg cgtttggaag gctttgcatc 9480
   acctttggat gatgcgcatg aatggagtcg tctgcttgct agccttcgcc taccgcccac 9540
   tgagtccggg cggcaactac catcggcgaa cgacccagct gacctctacc gaccggactt 9600
   gaatgcgcta ccttcgtcag cgacgatggc cgcgtacgct ggcgacgtgc ccccgcatgc 9660
   atggcggcac atggcgagct cagaccgtgc gtggctggct acaaatacgt accccgtgag 9720
   tgccctagct agaaacttac acctgcaact gcgagagcga gcgtgtgagt gtagccgagt 9780
   agatcccccg ggctgcaggt cgactctaga ggatccgaag gagatagaac cgatccacca 9840
   tgtccaacct gctcacggtt caccagaacc ttccggctct tccagtggac gcgacgtccg 9900
   atgaagtcag gaagaacctc atggacatgt tccgcgacag gcaagcgttc agcgagcaca 9960
   cctggaagat gctgctctcc gtctgccgct cctgggctgc atggtgcaag ctgaacaaca 10020
   ggaagtggtt ccccgctgag cccgaggacg tgagggatta ccttctgtac ctgcaagcgc 10080
   gaggtttgtt tctgcttcta cctttgatat atatataata attatcatta attagtagta 10140
   atataatatt tcaaatattt ttttcaaaat aaaagaatgt agtatatagc aattgctttt 10200
   ctgtagttta taagtgtgta tattttaatt tataactttt ctaatatatg accaaaacat 10260
   ggtgatgcct aggtctggca gtgaagacca tccagcaaca ccttggacaa ctgaacatgc 10320
   ttcacaggcg ctccggcctc ccgcgcccca gcgactcgaa cgccgtgagc ctcgtcatgc 10380
   gccgcatcag gaaggaaaac gtcgatgccg gcgaaagggc aaagcaggcc ctcgcgttcg 10440
   agaggaccga tttcgaccag gtccgcagcc tgatggagaa cagcgacagg tgccaggaca 10500
   ttaggaacct ggcgttcctc ggaattgcat acaacacgct cctcaggatc gcggaaattg 10560
   cccgcattcg cgtgaaggac attagccgca ccgacggcgg caggatgctt atccacattg 10620
   gcaggaccaa gacgctcgtt tccaccgcag gcgtcgaaaa ggccctcagc ctcggagtga 10680
   ccaagctcgt cgaacgctgg atctccgtgt ccggcgtcgc ggacgaccca aacaactacc 10740
   tcttctgccg cgtccgcaag aacggggtgg ctgcccctag cgccaccagc caactcagca 10800
   cgagggcctt ggaaggtatt ttcgaggcca cccaccgcct gatctacggc gcgaaggatg 10860
   acagcggtca acgctacctc gcatggtccg ggcactccgc ccgcgttgga gctgctaggg 10920
   acatggcccg cgccggtgtt tccatccccg aaatcatgca ggcgggtgga tggacgaacg 10980
   tgaacattgt catgaactac attcgcaacc ttgacagcga gacgggcgca atggttcgcc 11040
   tcctggaaga tggtgactga gctagaccca gctttcttgt acaaagtggc cgttaacgga 11100
   tccagacttg tccatcttct ggattggcca acttaattaa tgtatgaaat aaaaggatgc 11160
   acacatagtg acatgctaat cactataatg tgggcatcaa agttgtgtgt tatgtgtaat 11220
   tactagttat ctgaataaaa gagaaagaga tcatccatat ttcttatcct aaatgaatgt 11280
   cacgtgtctt tataattctt tgatgaacca gatgcatttc attaaccaaa tccatataca 11340
   tataaatatt aatcatatat aattaatatc aattgggtta gcaaaacaaa tctagtctag 11400
   gtgtgttttg cgaattgcgg caagcttcgg ccgccccagc ttggtcaccc ggtccgggcc 11460
   tagaaggccg atctcccggg cacccagctt tcttgtacaa agtggccgtt aacggatcgg 11520
   ccagaatggc ccggaccggg ttaccgaatt cctgccctta aggccaattg ttcaagattc 11580
   attcaacaat tgaaacatct cccatgatta aatcagtata aggttgctat ggtcttgctt 11640
   gacaaagttt ttttttgagg gaatttcaac taaatttttg agtgaaacta tcaaatactg 11700
   attttaaaaa atttttataa aaggaagcgc agagataaaa ggccatctat gctacaaaag 11760
   tacccaaaaa tgtaatccta aagtatgaat tgcatttttt ttttgtttgg acgaaaggaa 11820
   aggagtatta ccacaagaat gatatcatct tcatatttag atcttttttg ggtaaagctt 11880
   gagattctct aaatatagag aaatcagaag aaaaaaaaac cgtgttttgg tggttttgat 11940
   ttctagcctc cacaataact ttgacggcgt cgacaagtct aacggacacc aagcagcgaa 12000
   ccaccagcgc cgagccaagc gaagcagacg gccgagacgt tgacaccttc ggcgcggcat 12060
   ctctcgagag ttccgctccg gcgctccacc tccaccgctg gcggtttctt attccgttcc 12120
   gttccgcctc ctgctctgct cctctccaca ccacacggca cgaaaccgtt acggcaccgg 12180
   cagcacccag cacgggagag gggattcctt tcccaccgtt ccttcccttt ccgccccgcc 12240
   gctataaata gccagcccca tccccagctt ttttccccaa tctcatctcc tctctcctgt 12300
   tgttcggagc acacgcacaa tccgatcgat ccccaaatcc ccttcgtctc tcctcgcgag 12360
   cctcgtggat cccagcttca aggtacggcg atcgatcatc ccccctcctt ctctctacct 12420
   tcttttctct agactacatc ggatggcgat ccatggttag ggcctgctag tttcccttcc 12480
   tgttttgtcg atggctgcga ggcacaatag atctgatggc gttatgacgg ctaacttgtc 12540
   atgttgttgc gatttatagt ccctttagga gatcagttta atttctcgga tggttcgaga 12600
   tcggtggtcc atggttagta ccctaagatc cgcgctgtta gggttcgtag atggaggcga 12660
   cctgttctga ttgttaactt gtcagtacct gggaaatcct gggatggttc tagctcgtcc 12720
   gcagatgaga tcgatttcat gatcctctgt atcttgtttc gttgcctagg ttccgtctaa 12780
   tctatccgtg gtatgatgta gatgttttga tcgtgctaac tacgtcttgt aaagttaatt 12840
   gtcaggtcat aatttttagc atgccttttt ttttgtttgg ttttgtctaa ttgggctgtc 12900
   gttctagatc agagtagaag actgttccaa actacctgct ggatttattg aacttggatc 12960
   tgtatgtgtg tcacatatct tcataaattc atgattaaga tggattgaaa tatcttttat 13020
   ctttttggta tggatagttc tatatgttgg tgtggctttg ttagatgtat acatgcttag 13080
   atacatgaag caacgtgctg ctactgttta gtaattgctg ttcatttgtc taataaacag 13140
   ataaggatat gtatttatgt tgctgttggt tttgctggta ctttgttgga tacaaatgct 13200
   tcaatacaga aaacagcatg ctgctacgat ttaccattta tctaatctta tcatatgtct 13260
   aatctaataa acaaacatgc ttttaaatta tcttcatatg cttggatgat ggcatacaca 13320
   gcggctatgt gtggtttttt aaatacccag catcatgggc atgcatgaca ctgctttaat 13380
   atgcttttta tttgcttgag actgtttctt ttgtttatac tgacccttta gttcggtgac 13440
   tcttctgcag gtcgacttta acttagccta ggatccatgg cccagtccaa gcacggcctg 13500
   accaaggaga tgaccatgaa gtaccgcatg gagggctgcg tggacggcca caagttcgtg 13560
   atcaccggcg agggcatcgg ctaccccttc aagggcaagc aggccatcaa cctgtgcgtg 13620
   gtggagggcg gccccttgcc cttcgccgag gacatcttgt ccgccgcctt catgtacggc 13680
   aaccgcgtgt tcaccgagta cccccaggac atcgtcgact acttcaagaa ctcctgcccc 13740
   gccggctaca cctgggaccg ctccttcctg ttcgaggacg gcgccgtgtg catctgcaac 13800
   gccgacatca ccgtgagcgt ggaggagaac tgcatgtacc acgagtccaa gttctacggc 13860
   gtgaacttcc ccgccgacgg ccccgtgatg aagaagatga ccgacaactg ggagccctcc 13920
   tgcgagaaga tcatccccgt gcccaagcag ggcatcttga agggcgacgt gagcatgtac 13980
   ctgctgctga aggacggtgg ccgcttgcgc tgccagttcg acaccgtgta caaggccaag 14040
   tccgtgcccc gcaagatgcc cgactggcac ttcatccagc acaagctgac ccgcgaggac 14100
   cgcagcgacg ccaagaacca gaagtggcac ctgaccgagc acgccatcgc ctccggctcc 14160
   gccttgccct ccggactcag atctcgatag ggtaccaatg gccagttaac agatccagct 14220
   gctgctgttc tagggttcac aagtctgcct atttgtcttc cccaatggag ctatggttgt 14280
   ctggtctggt ccttggtcgt gtcccgtttc attgtgtact atttacctgt aatgtgtatc 14340
   cttaagtctg gtttgatggt gtctgaaacg ttttgctgtg gtagagcagc atggaagaac 14400
   tataatgaat aagtgatccc taatcattgt gtccaaattt tgcttctgct ataccctttt 14460
   gtgctgtttc ttatgttttg cttaaaaatt tgatctgaca aacaaatttg tctaaattat 14520
   gctcttgttc tgactgtgta ctgtacacat ttgtattgct accggtttgg cacatagttt 14580
   tctctttgat tgcagatcaa acccttctga ttgcagattg cttggctatg gctgatgtgc 14640
   tcactgttct tctttagctt gtatattgct gatggtggct tggctacggc tgaaaaagtt 14700
   gctgctgcgc cgacttggca actgttgctc cagtaatgct ttgtcctctc ttcctagccg 14760
   ccgacttggc acttggcagc tgttgctcct gtaatgcgtt gttatctctt atctcttgag 14820
   atgtactatg attttgaggc tgtttgactt gtgccagatt gccagtgctg atgctatatt 14880
   atctcaggtt tttatgctag tatatgtttt actgtgtgct gcctgtgtcg agttacgtaa 14940
   tctattgaac cgctatgcgt gttgatgccc tgttgttgct tgccagattg atgcccctgt 15000
   tgcttgcagc ttgcaagctg tagtgaaatc ctaaaattcg aaaccaaatc tattctgaac 15060
   ttttgaagat ctgtgatgtc cggaagaaaa gattcaaaca gataaacggg actctccaga 15120
   atctataaaa taaatgatcg acaaacgctt tgctttggta ggcttcctag ttagttagcg 15180
   gaccgaagct tcggtccggg cctagaaggc cagcttcggc cgccccgggc aactttatta 15240
   tacaaagttg atagatcgaa taacttcgta tagcatacat tatacgaagt tatacctggt 15300
   ggcgccgcta ggggctgcag gaattcgata tcaagcttat cgataccgtc gacctcgagg 15360
   gggggcccgg taccctggga tctgccctta ctttaacgcc tctaaccaac acccctttat 15420
   ctttataagg aacaataaac agaatttgcc ccactgttct aaatcaccta ataatatccc 15480
   cagctaaaaa caataaaggt ttcctagaat taagacaagc atgactgttc ctccaggagg 15540
   gtttggaaca ttgttgcagt cttgcagata cgggcgaagg gtgagaaaca gagcggaggg 15600
   ctggaggtga cctcggtagt cgacgccgga gttgagcttg acaacgacgg ggcggcccct 15660
   gatggacttg aggaagtccg atggcgtctt caccgtcccg ccggcgcccg aggcgggcct 15720
   gtcgctgccg ccgccgccgc tgctcatctt gcgcgctgtg cccccggcgg tgtccctgtg 15780
   ttgcggatcg cgggtgggcc aggtggatgc gagggcgacc cgtttggact ccggccggag 15840
   ccgccggatc cctggtcggt gtcagtgccg tttactctgg gccccacgtg tcagtaccgt 15900
   ctgtagatga caacaacccg tcgtccacag tcatgtccaa aatatccttt cttctttttt 15960
   ttcgattcgg atatctatct tccttttttt tttccaaaaa tcttcttgac gcaccagcgc 16020
   gcacgtttgt ggtaaacgcc gacacgtcgg tcccacgtcg atagacccca cccaccagtg 16080
   agtagcgtgt acgtattcgg gggtgacgga cgtgtcgccg tcgtcttgct agtcccattc 16140
   ccatctgagc cacacatctc tgaacaaaaa aaaggaggga ggcctccacg cacatccccc 16200
   tccgtgccac ccgccccaaa ccctcgcgcc gcctccgaga cagccgccgc aaccatggcc 16260
   accgccgccg ccgcgtctac cgcgctcact ggcgccacta ccgctgcgcc caaggcgagg 16320
   cgccgggcgc acctcctggc cacccgccgc gccctcgccg cgcccatcag gtgctcagcg 16380
   gcgtcacccg ccatgccgat ggctcccccg gccaccccgc tccggccgtg gggccccacc 16440
   gagccccgca agggtgctga catcctcgtc gagtccctcg agcgctgcgg cgtccgcgac 16500
   gtcttcgcct accccggcgg cgcgtccatg gagatccacc aggcactcac ccgctccccc 16560
   gtcatcgcca accacctctt ccgccacgag caaggggagg cctttgccgc ctccggctac 16620
   gcgcgctcct cgggccgcgt cggcgtctgc atcgccacct ccggccccgg cgccaccaac 16680
   ctagtctccg cgctcgccga cgcgctgctc gattccgtcc ccatggtcgc catcacggga 16740
   caggtggcgc gacgcatgat tggcaccgac gccttccagg agacgcccat cgtcgaggtc 16800
   acccgctcca tcaccaagca caactacctg gtcctcgacg tcgacgacat cccccgcgtc 16860
   gtgcaggagg ctttcttcct cgcctcctct ggtcgaccag ggccggtgct tgtcgacatc 16920
   cccaaggaca tccagcagca gatggcggtg cctgtctggg acaagcccat gagtctgcct     16980
   gggtacattg cgcgccttcc caagccccct gcgactgagt tgcttgagca ggtgctgcgt     17040
   cttgttggtg aatcgcggcg ccctgttctt tatgtgggcg gtggctgcgc agcatctggt 17100
   gaggagttgc gacgctttgt ggagctgact ggaatcccgg tcacaactac tcttatgggc 17160
   ctcggcaact tccccagcga cgacccactg tctctgcgca tgctaggtat gcatgggacg 17220
   gtgtatgcaa attatgcagt ggataaggcc gatctgttgc ttgcacttgg tgtgcggttt 17280
   gatgatcgcg tgacagggaa gattgaggct tttgcaagca gggctaagat tgtgcacgtt 17340
   gatattgatc cggctgagat tggcaagaac aagcagccac atgtgtccat ctgtgcagat 17400
   gttaagcttg ctttgcaggg catgaatgct cttcttgaag gaagcacatc aaagaagagc     17460
   tttgactttg gctcatggaa cgatgagttg gatcagcaga agagggaatt cccccttggg     17520
   tataaaacat ctaatgagga gatccagcca caatatgcta ttcaggttct tgatgagctg 17580
   acgaaaggcg aggccatcat cggcacaggt gttgggcagc accagatgtg ggcggcacag 17640
   tactacactt acaagcggcc aaggcagtgg ttgtcttcag ctggtcttgg ggctatggga 17700
   tttggtttgc cggctgctgc tggtgcttct gtggcaaacc caggtgtcac tgttgttgac 17760
   atcgatggag atggtagctt tctcatgaac gttcaggagc tagctatgat ccgaattgag 17820
   aacctcccag tgaaggtctt tgtgctaaac aaccagcacc tggggatggt ggtgcagttg 17880
   gaggacaggt tctataaggc caacagagcg cacacatact tgggaaaccc agagaatgaa 17940
   agtgagatat atccagattt cgtgacgatc gccaaagggt tcaacattcc agcggtccgt 18000
   gtgacaaaga agaacgaagt ccgcgcagcg ataaagaaga tgctcgagac tccagggccg 18060
   tacctcttgg atataatcgt cccacaccag gagcatgtgt tgcctatgat ccctagtggt 18120
   ggggctttca aggatatgat cctggatggt gatggcagga ctgtgtactg actagctagt 18180
   cagttaacct agacttgtcc atcttctgga ttggccaact taattaatgt atgaaataaa 18240
   aggatgcaca catagtgaca tgctaatcac tataatgtgg gcatcaaagt tgtgtgttat 18300
   gtgtaattac tagttatctg aataaaagag aaagagatca tccatatttc ttatcctaaa 18360
   tgaatgtcac gtgtctttat aattctttga tgaaccagat gcatttcatt aaccaaatcc 18420
   atatacatat aaatattaat catatataat taatatcaat tgggttagca aaacaaatct 18480
   agtctaggtg tgttttgcga atgcggccgc ctgcagagtt aacggcgcgc cgactagcta 18540
   gctaaggtac cgagctcgaa ttcattccga ttaatcgtgg cctcttgctc ttcaggatga 18600
   agagctatgt ttaaacgtgc aagcgctact agacaattca gtacattaaa aacgtccgca 18660
   atgtgttatt aagttgtcta agcgtcaatt tgtttacacc acaatatatc ctgcca 18716
<210> 71
   <211> 13825
   <212> DNA
   <213> Artificial sequence
<220>
   <223> Synthetic construct
<400> 71
   gtttacccgc caatatatcc tgtcaaacac tgatagttta aactgaaggc gggaaacgac      60
   aatctgatca tgagcggaga attaagggag tcacgttatg acccccgccg atgacgcggg     120
   acaagccgtt ttacgtttgg aactgacaga accgcaacgt tgaaggagcc actcagcaag     180
   ctggtacgat tgtaatacga ctcactatag ggcgaattga gcgctgttta aacgctcttc     240
   aactggaaga gcggttacgc tgtttaaacg ctcttcaact ggaagagcgg ttactaccgg     300
   ttcactagct agctgctaat cgagctagtt accctatgag gtgacatgaa gcgctcacgg     360
   ttactatgac ggttagcttc acgactgttg gtggcagtag cgtacgactt agctatagtt     420
   ccggacttac ccttaagcga tttaaatcct gaggatatcg ctatcaactt tgtatagaaa     480
   agttgggccg agctcggtac ggccagaatg gcccggaccg ggttaccgaa ttcaggcgac     540
   ccatcgctgc tttgtctaca tcatgttctt catcatcctc cccaggcgac gcgtgctgct     600
   gttcttattc agactaccgt tcgagtgact gcatggcgta catctttctg catcgacttt     660
   gtacggctac atcgaacata tacacgagat gtctcgtgtg aatagagtca ctaatgcctt     720
   aagcatcggt tactccgtag ggtacattct gttcttctta tttgtgcata tttttattgt     780
   tgtttactga ttatacgagt agttatacat acatgcacat acatatcatc acatatatca     840
   caatattttt ctaaattaaa ttaaaactaa aaatgactaa atttctaaca ccaacgacat     900
   tgtaatgttt tctccaacaa ctttacctat tctacattgt tctatttcga atttcactct     960
   ataaacaaca tagtctacaa tggaaaacag tgctttgtac gactatatac gcgatgtgtg    1020
   gctacaacat aagacaatat agtcgtttga agattgaacc tatatatcgg tacggttaat    1080
   ccgtctatgt acgtgggcat gacgaacacc cgtgataacg aaggattaac gtgcacaatc    1140
   ataaatccaa agtaggagcg gtgcatgatg agaatcgctc tcagtactcg acataatgaa    1200
   ccttacgagg tacaacaggc aggcaggcag ggaccagggg ccgcctttat ttcaggctcg    1260
   ctggccccac gggcgtgctg cgtgcacgaa gggcactacc ccaacctctc accgaaaacc    1320
   gcgctggatc ggcaaatcaa acgaggtggt gccccgtgcc cactctccac gtccacggca    1380
   ccatccctct gcagccgctc accagccatg ccgtgtcgcg gaacggcaca accaccccca    1440
   acccactcac gaaaccccgt cccggccgtg cccgtgtcgg tccgcgctcg gcaacgaggc    1500
   ggcccgcgct gctgagtccc ctggacaccc gacaccctgt cggccctttg tttattcatc    1560
   ccgaaatctc atctgccccc acggccgact gcgctgcgcc gcccggatat atatacccat 1620
   cgttatcgat cgactgggga ctctatcagt gatagagtct agaggatcgc tcaggaaggc 1680
   cgctgagata gaggcatggc ggccaatgcg ggcggcggtg gagcgggagg aggcagcggc 1740
   agcggcagcg tggctgcgcc ggcggtgtgc cgccccagcg gctcgcggtg gacgccgacg 1800
   ccggagcaga tcaggatgct gaaggagctc tactacggct gcggcatccg gtcgcccagc 1860
   tcggagcaga tccagcgcat caccgccatg ctgcggcagc acggcaagat cgagggcaag 1920
   aacgtcttct actggttcca gaaccacaag gcccgcgagc gccagaagcg ccgcctcacc 1980
   agcctcgacg tcaacgtgcc cgccgccggc gcggccgacg ccaccaccag ccaactcggc 2040
   gtcctctcgc tgtcgtcgcc gccgccttca ggcgcggcgc ctccctcgcc caccctcggc 2100
   ttctacgccg ccggcaatgg cggcggatcg gctgtgctgc tggacacgag ttccgactgg 2160
   ggcagcagcg gcgctgccat ggccaccgag acatgcttcc tgcaggacta catgggcgtg 2220
   acggacacgg gcagctcgtc gcagtggcca cgcttctcgt cgtcggacac gataatggcg 2280
   gcggccgcgg cgcgggcggc gacgacgcgg gcgcccgaga cgctccctct cttcccgacc 2340
   tgcggcgacg acggcggcag cggtagcagc agctacttgc cgttctgggg tgccgcgtcc 2400
   acaactgccg gcgccacttc ttccgttgcg atccagcagc aacaccagct gcaggagcag 2460
   tacagctttt acagcaacag caacagcacc cagctggccg gcaccggcaa ccaagacgta 2520
   tcggcaacag cagcagcagc cgccgccctg gagctgagcc tcagctcatg gtgctcccct 2580
   taccctgctg cagggagtat gtgagagcaa cgcgagctgc cactgctctt cactggtacc 2640
   gttaacagat cattcgacaa agcagcatta gtccgttgat cggtggaaga ccactcgtca 2700
   gtgttgagtt gaatgtttga tcaataaaat acggcaatgc tgtaagggtt gttttttatg 2760
   ccattgataa tacactgtac tgttcagttg ttgaactcta tttcttagcc atgccaagtg 2820
   cttttcttat tttgaataac attacagcaa aaagttgaaa gacaaaaaaa aaaacccccg 2880
   aacagagtgc tttgggtccc aagcttcttt agactgtgtt cggcgttccc cctaaatttc 2940
   tccccctata tctcactcac ttgtcacatc agcgttctct ttccccctat atctccacgc 3000
   tctacagcag ttccacctat atcaaacctc tataccccac cacaacaata ttatatactt 3060
   tcatcttcaa ctaactcatg taccttccaa tttttttcta ctaataatta tttacgtgca 3120
   cagaaactta gcaaggagag agagagcggg gtgacccacc ttgctagttg gatattacct 3180
   cttctcttca aagtatcctt gaacgctcac cggttatcaa atctctacac tatagctctg 3240
   tagtcttgct agatagttag ttctttagct ctcggtgacc aagcttggcg cgatcaagct 3300
   tatcgatacc gtcgacctcg aagcttggtc acccggtccg ggcctagaag gccagcttca 3360
   agtttgtaca aaaaagcagg ctccggccag aatggcccgg accgggttac cgaattctta 3420
   ccctagctcc ctgcggctgt tacgcggtcc cccatcaatc ttctgttctt gcggttgtag 3480
   cctgtgtaac agtgctagag tatgtatgat aaataggttt taagtctgct tacatgacat 3540
   tttttattgt ggaagagaca tataaaaatt agagagagtg gttctcatgc aacggcggac 3600
   ggcccggtgc taaaagagct tcaagacaaa ataatgaaac aggaagagag tagatttatc 3660
   taagagccaa ctttattata tgaatgtgtt tattgttggc tttagatgat atggtaagga 3720
   gttagagcta ataatagata ggctctatta ttattattat taattaaact cgctctaagg 3780
   aggaaagtgg gaggaaggga cgaggacgaa gactactgga agcatcgtgc atggatgatg 3840
   gatgtggtgt ctcttaatgt aggtggccgg aggatgtacg tgttaattgc gcgataagca 3900
   ctcagatcca accgcaaact acctccacac tgacacactg atagagagaa agagagacct 3960
   ccgacgactg ccgccgcaga tgagccacgt acgtatacga cgtctgccgg ccggctcagg 4020
   ctgccgccat caccctgctc gaaagtcgcg ttaggcggcg ccagctacat aggagtatct 4080
   agtctagcca gttagtatac tactactgcg ctgatgatga attaactctg catagatact 4140
   gtacttgcct ccctccaaca cccaaccacc tcctgctcgg ctcttaataa cttggacacg 4200
   gatcgatgcc atccaaggaa gaacacgacg acgacgacgg aacatccacc atgcaagctt 4260
   gcatccatac gccgatacgc gtgcatccat ccatccacca ttatttccat tttccaccga 4320
   tcacacgtac acaggcctat ttaaggagcg acatcccact gcaactctcc tcaccactca 4380
   tcaccagcta gctctagcaa agcacttgcc atctaccgac cgccgcattc caaacagccc 4440
   gacgagctag cagagcggca ggcacctccc tcctcaagga acccatggcc actgtgaaca 4500
   actggctcgc tttctccctc tccccgcagg agctgccgcc ctcccagacg acggactcca 4560
   cactcatctc ggccgccacc gccgaccatg tctccggcga tgtctgcttc aacatccccc 4620
   aagattggag catgagggga tcagagcttt cggcgctcgt cgcggagccg aagctggagg 4680
   acttcctcgg cggcatctcc ttctccgagc agcatcacaa ggccaactgc aacatgatac 4740
   ccagcactag cagcacagtt tgctacgcga gctcaggtgc tagcaccggc taccatcacc 4800
   agctgtacca ccagcccacc agctcagcgc tccacttcgc ggactccgta atggtggctt 4860
   cctcggccgg tgtccacgac ggcggtgcca tgctcagcgc ggccgccgct aacggtgtcg 4920
   ctggcgctgc cagtgccaac ggcggcggca tcgggctgtc catgattaag aactggctgc 4980
   ggagccaacc ggcgcccatg cagccgaggg tggcggcggc tgagggcgcg caggggctct 5040
   ctttgtccat gaacatggcg gggacgaccc aaggcgctgc tggcatgcca cttctcgctg 5100
   gagagcgcgc acgggcgccc gagagtgtat cgacgtcagc acagggtgga gccgtcgtcg 5160
   tcacggcgcc gaaggaggat agcggtggca gcggtgttgc cggcgctcta gtagccgtga 5220
   gcacggacac gggtggcagc ggcggcgcgt cggctgacaa cacggcaagg aagacggtgg 5280
   acacgttcgg gcagcgcacg tcgatttacc gtggcgtgac aaggcataga tggactggga 5340
   gatatgaggc acatctttgg gataacagtt gcagaaggga agggcaaact cgtaagggtc 5400
   gtcaagtcta tttaggtggc tatgataaag aggagaaagc tgctagggct tatgatcttg 5460
   ctgctctgaa gtactggggt gccacaacaa caacaaattt tccagtgagt aactacgaaa 5520
   aggagctcga ggacatgaag cacatgacaa ggcaggagtt tgtagcgtct ctgagaagga 5580
   agagcagtgg tttctccaga ggtgcatcca tttacagggg agtgactagg catcaccaac 5640
   atggaagatg gcaagcacgg attggacgag ttgcagggaa caaggatctt tacttgggca 5700
   ccttcagcac ccaggaggag gcagcggagg cgtacgacat cgcggcgatc aagttccgcg 5760
   gcctcaacgc cgtcaccaac ttcgacatga gccgctacga cgtgaagagc atcctggaca 5820
   gcagcgccct ccccatcggc agcgccgcca agcgcctcaa ggaggccgag gccgcagcgt 5880
   ccgcgcagca ccaccacgcc ggcgtggtga gctacgacgt cggccgcatc gcctcgcagc 5940
   tcggcgacgg cggagccctg gcggcggcgt acggcgcgca ctaccacggc gccgcctggc 6000
   cgaccatcgc gttccagccg ggcgccgcca gcacaggcct gtaccacccg tacgcgcagc 6060
   agccaatgcg cggcggcggg tggtgcaagc aggagcagga ccacgcggtg atcgcggccg 6120
   cgcacagcct gcaggacctc caccacctga acctgggcgc ggccggcgcg cacgactttt 6180
   tctcggcagg gcagcaggcc gccgccgctg cgatgcacgg cctgggtagc atcgacagtg 6240
   cgtcgctcga gcacagcacc ggctccaact ccgtcgtcta caacggcggg gtcggcgaca 6300
   gcaacggcgc cagcgccgtc ggcggcagtg gcggtggcta catgatgccg atgagcgctg 6360
   ccggagcaac cactacatcg gcaatggtga gccacgagca ggtgcatgca cgggcctacg 6420
   acgaagccaa gcaggctgct cagatggggt acgagagcta cctggtgaac gcggagaaca 6480
   atggtggcgg aaggatgtct gcatggggga ctgtcgtgtc tgcagccgcg gcggcagcag 6540
   caagcagcaa cgacaacatg gccgccgacg tcgggcatgg cggcgcgcag ctcttcagtg 6600
   tctggaacga cacttaagcg tacctagtgg tacctgacat cttatagtct gcaacctctc 6660
   gtgtctgaat tcctatcttt atcaagtgtt attgcttcca cgactatagg acagctttcg 6720
   tcgaaagctt ttgctcatgt gatctcgaag gattcatcta gtctgatttt tcgtgacttg 6780
   tatcggtttt attggattca tccaacatat atcaataaaa aatgagttgt gtttcctttc 6840
   ttcctagttc agttaaaatt atttccctcc tgcgcttgtg ctgtaattgt ctgtgtacct 6900
   gttgtttgtg actgtgttag ttcccttgga tatgatttcg tatttgatat gtacatggag 6960
   atagcttagc ttcattattg gagtatgaag ttagtatgac atagtcactc tcctggaaaa 7020
   ttgacactgc aaaccatatt tttattctga accacaaatc ctagtcagtc cgctggcata 7080
   tgccgtccgt ttgctgaatc cagaacgtgg gtttggagat gtacggctga gatgcctcta 7140
   tgcgaagggg atttcgtggt gaaacgagat gggagtagag caacgcccgt ggaagatgct 7200
   tcaaacttcc acacttttga gcaacgatcg gcagtagtaa ggtagacgat ttcaagatca 7260
   aagcatatga agataaacaa catcaacaac aaaatttgtt ggggttctat agagagaaac 7320
   agagctacat acatacactg ttttgtatct accatctgag atgatgaaaa gatgaaaaac 7380
   taaagaatgc cccggcgcca acgccaggac acgccgcgcg cgcgtcaccc gagccatctc 7440
   ttgacccagc cggcgctgta tatttacaca cgttgcagca tcgatcacca cctgttcgat 7500
   cgcgtcgccg tcaccggtac cgagctcgaa ttccggtccg ggcctagaag gccgatctcc 7560
   cgggcaccca gctttcttgt acaaagtggc cgttaacgga tcggccagaa tggcccggac 7620
   cgggttaccg aattcgagct cggtaccctg ggatctgccc ttactttaac gcctctaacc 7680
   aacacccctt tatctttata aggaacaata aacagaattt gccccactgt tctaaatcac 7740
   ctaataatat ccccagctaa aaacaataaa ggtttcctag aattaagaca agcatgactg 7800
   ttcctccagg agggtttgga acattgttgc agtcttgcag atacgggcga agggtgagaa 7860
   acagagcgga gggctggagg tgacctcggt agtcgacgcc ggagttgagc ttgacaacga 7920
   cggggcggcc cctgatggac ttgaggaagt ccgatggcgt cttcaccgtc ccgccggcgc 7980
   ccgaggcggg cctgtcgctg ccgccgccgc cgctgctcat cttgcgcgct gtgcccccgg 8040
   cggtgtccct gtgttgcgga tcgcgggtgg gccaggtgga tgcgagggcg acccgtttgg 8100
   actccggccg gagccgccgg atccctggtc ggtgtcagtg ccgtttactc tgggccccac 8160
   gtgtcagtac cgtctgtaga tgacaacaac ccgtcgtcca cagtcatgtc caaaatatcc 8220
   tttcttcttt tttttcgatt cggatatcta tcttcctttt ttttttccaa aaatcttctt 8280
   gacgcaccag cgcgcacgtt tgtggtaaac gccgacacgt cggtcccacg tcgatagacc 8340
   ccacccacca gtgagtagcg tgtacgtatt cgggggtgac ggacgtgtcg ccgtcgtctt 8400
   gctagtccca ttcccatctg agccacacat ctctgaacaa aaaaaaggag ggaggcctcc 8460
   acgcacatcc ccctccgtgc cacccgcccc aaaccctcgc gccgcctccg agacagccgc 8520
   cgcaaccatg gccaccgccg ccgccgcgtc taccgcgctc actggcgcca ctaccgctgc 8580
   gcccaaggcg aggcgccggg cgcacctcct ggccacccgc cgcgccctcg ccgcgcccat 8640
   caggtgctca gcggcgtcac ccgccatgcc gatggctccc ccggccaccc cgctccggcc 8700
   gtggggcccc accgagcccc gcaagggtgc tgacatcctc gtcgagtccc tcgagcgctg 8760
   cggcgtccgc gacgtcttcg cctaccccgg cggcgcgtcc atggagatcc accaggcact 8820
   cacccgctcc cccgtcatcg ccaaccacct cttccgccac gagcaagggg aggcctttgc 8880
   cgcctccggc tacgcgcgct cctcgggccg cgtcggcgtc tgcatcgcca cctccggccc 8940
   cggcgccacc aacctagtct ccgcgctcgc cgacgcgctg ctcgattccg tccccatggt 9000
   cgccatcacg ggacaggtgg cgcgacgcat gattggcacc gacgccttcc aggagacgcc 9060
   catcgtcgag gtcacccgct ccatcaccaa gcacaactac ctggtcctcg acgtcgacga 9120
   catcccccgc gtcgtgcagg aggctttctt cctcgcctcc tctggtcgac cagggccggt 9180
   gcttgtcgac atccccaagg acatccagca gcagatggcg gtgcctgtct gggacaagcc 9240
   catgagtctg cctgggtaca ttgcgcgcct tcccaagccc cctgcgactg agttgcttga 9300
   gcaggtgctg cgtcttgttg gtgaatcgcg gcgccctgtt ctttatgtgg gcggtggctg 9360
   cgcagcatct ggtgaggagt tgcgacgctt tgtggagctg actggaatcc cggtcacaac 9420
   tactcttatg ggcctcggca acttccccag cgacgaccca ctgtctctgc gcatgctagg 9480
   tatgcatggg acggtgtatg caaattatgc agtggataag gccgatctgt tgcttgcact 9540
   tggtgtgcgg tttgatgatc gcgtgacagg gaagattgag gcttttgcaa gcagggctaa 9600
   gattgtgcac gttgatattg atccggctga gattggcaag aacaagcagc cacatgtgtc 9660
   catctgtgca gatgttaagc ttgctttgca gggcatgaat gctcttcttg aaggaagcac 9720
   atcaaagaag agctttgact ttggctcatg gaacgatgag ttggatcagc agaagaggga 9780
   attccccctt gggtataaaa catctaatga ggagatccag ccacaatatg ctattcaggt 9840
   tcttgatgag ctgacgaaag gcgaggccat catcggcaca ggtgttgggc agcaccagat 9900
   gtgggcggca cagtactaca cttacaagcg gccaaggcag tggttgtctt cagctggtct 9960
   tggggctatg ggatttggtt tgccggctgc tgctggtgct tctgtggcaa acccaggtgt 10020
   cactgttgtt gacatcgatg gagatggtag ctttctcatg aacgttcagg agctagctat 10080
   gatccgaatt gagaacctcc cagtgaaggt ctttgtgcta aacaaccagc acctggggat 10140
   ggtggtgcag ttggaggaca ggttctataa ggccaacaga gcgcacacat acttgggaaa 10200
   cccagagaat gaaagtgaga tatatccaga tttcgtgacg atcgccaaag ggttcaacat 10260
   tccagcggtc cgtgtgacaa agaagaacga agtccgcgca gcgataaaga agatgctcga 10320
   gactccaggg ccgtacctct tggatataat cgtcccacac caggagcatg tgttgcctat 10380
   gatccctagt ggtggggctt tcaaggatat gatcctggat ggtgatggca ggactgtgta 10440
   ctgactagct agtcagttaa cagatctgcc agatcctcgg tgtacaaata acccgtctta 10500
   tcctatgaga cgggccggcg tcagtgtgtt ctggaggaat ttttatgtca gagccttttt 10560
   ctttgtgtgc ttgatgtaga tgccaaggga agcttattgg ctgttgaagc ttgatgcaaa 10620
   ataaattatg gaactctgtt ttttgtttat ctaataataa ctagcaaata tgcttccatt 10680
   gcattgaaac taacagcctt ttgtgtttcc aagttttatt ttgtgacaat gtcatctatt 10740
   tcaattagtt gtggaatcgg aaacttgcag gactaacttg gaaactccaa tccctcagca 10800
   tcctggactt tttcctggtg taatccatgt agatattatt ttaatcatca ttttagttct 10860
   ggaggttttt ccatctccgg ttttgctccc ctttcttcaa aaaaaaaaaa aaaatgccgt 10920
   aggcgccgca acgcccacct gttgttcaaa ctcatgggca cgagtggctc gaagatttta 10980
   tacaacaatt gctgtagttt caccgttgct ggtgaagaag cattttttta aaaaaatata 11040
   gtggtattca ttttaattag tttagttgtg cagcgagcaa aatttggaca tgctctgctc 11100
   ggaatctgat cgacctagac acagattagc agcagtagct ttgtcatctg ttccaagagt 11160
   tgcgatctga tagaagaaaa aaaaaccttc ccttcaatgt aaaaccgaaa ctaacaagaa 11220
   agaagcacag tgccgtttag gcaagtatgg agtacgtatt aagcatgtag aaggccatgc 11280
   atgaaccact aacaagaaag aagcgcagtg ccattcaggc aagcataagc atgtagattg 11340
   gcatgcatga acaactaaca gtagatcgtc tctggtctga ttagaagttt tttgggaagc 11400
   caagaaatca tgtacaactg gttccatctc aaattccgtg gcaaaaagag gcctaagcaa 11460
   cataaagctt cggtccgggc ctagaaggcc attgggtcat cggatcccgg gcaactttat 11520
   tatacaaagt tgatagatat ctggtctaac taactagtcc taaggacccg gcggaccgaa 11580
   gctggccgct ctagaactag tggatctcga tgtgtagtct acgagaaggg ttaaccgtct 11640
   cttcgtgaga ataaccgtgg cctaaaaata agccgatgag gataaataaa atgtggtggt 11700
   acagtacttc aagaggttta ctcatcaaga ggatgctttt ccgatgagct ctagtagtac 11760
   atcggacctc acatacctcc attgtggtga aatattttgt gctcatttag tgatgggtaa 11820
   attttgttta tgtcactcta ggttttgaca tttcagtttt gccactctta ggttttgaca 11880
   aataatttcc attccgcggc aaaagcaaaa caattttatt ttacttttac cactcttagc 11940
   tttcacaatg tatcacaaat gccactctag aaattctgtt tatgccacag aatgtgaaaa 12000
   aaaacactca cttatttgaa gccaaggtgt tcatggcatg gaaatgtgac ataaagtaac 12060
   gttcgtgtat aagaaaaaat tgtactcctc gtaacaagag acggaaacat catgagacaa 12120
   tcgcgtttgg aaggctttgc atcacctttg gatgatgcgc atgaatggag tcgtctgctt 12180
   gctagccttc gcctaccgcc cactgagtcc gggcggcaac taccatcggc gaacgaccca 12240
   gctgacctct accgaccgga cttgaatgcg ctaccttcgt cagcgacgat ggccgcgtac 12300
   gctggcgacg tgcccccgca tgcatggcgg cacatggcga gctcagaccg tgcgtggctg 12360
   gctacaaata cgtaccccgt gagtgcccta gctagaaact tacacctgca actgcgagag 12420
   cgagcgtgtg agtgtagccg agtagatccc ccgggctgca ggtcgactct agaggatcca 12480
   ccggtcgcca ccatggccca cagcaagcac ggcctgaagg aggagatgac catgaagtac 12540
   cacatggagg gctgcgtgaa cggccacaag ttcgtgatca ccggcgaggg catcggctac 12600
   cccttcaagg gcaagcagac catcaacctg tgcgtgatcg agggcggccc cctgcccttc 12660
   agcgaggaca tcctgagcgc cggcttcaag tacggcgacc ggatcttcac cgagtacccc 12720
   caggacatcg tggactactt caagaacagc tgccccgccg gctacacctg gggccggagc 12780
   ttcctgttcg aggacggcgc cgtgtgcatc tgtaacgtgg acatcaccgt gagcgtgaag 12840
   gagaactgca tctaccacaa gagcatcttc aacggcgtga acttccccgc cgacggcccc 12900
   gtgatgaaga agatgaccac caactgggag gccagctgcg agaagatcat gcccgtgcct 12960
   aagcagggca tcctgaaggg cgacgtgagc atgtacctgc tgctgaagga cggcggccgg 13020
   taccggtgcc agttcgacac cgtgtacaag gccaagagcg tgcccagcaa gatgcccgag 13080
   tggcacttca tccagcacaa gctgctgcgg gaggaccgga gcgacgccaa gaaccagaag 13140
   tggcagctga ccgagcacgc catcgccttc cccagcgccc tggcctgaag cggcccatgg 13200
   atattcgaac gcgtaggtac cacatggtta acctagactt gtccatcttc tggattggcc 13260
   aacttaatta atgtatgaaa taaaaggatg cacacatagt gacatgctaa tcactataat 13320
   gtgggcatca aagttgtgtg ttatgtgtaa ttactagtta tctgaataaa agagaaagag 13380
   atcatccata tttcttatcc taaatgaatg tcacgtgtct ttataattct ttgatgaacc 13440
   agatgcattt cattaaccaa atccatatac atataaatat taatcatata taattaatat 13500
   caattgggtt agcaaaacaa atctagtcta ggtgtgtttt gcgaatgcgg ccgccaccgc 13560
   ggtggagctc gaattccggt ccgaagctta agccatggcc cgggaatctt agcggccgcc 13620
   tgcagagtta acggcgcgcc gactagctag ctaaggtacc gagctcgaat tcattccgat 13680
   taatcgtggc ctcttgctct tcaggatgaa gagctatgtt taaacgtgca agcgctacta 13740
   gacaattcag tacattaaaa acgtccgcaa tgtgttatta agttgtctaa gcgtcaattt 13800
   gtttacacca caatatatcc tgcca 13825
<210> 72
   <211> 20570
   <212> DNA
   <213> Artificial sequence
<220>
   <223> Synthetic construct
<400> 72
   gtttacccgc caatatatcc tgtcaaacac tgatagttta aactgaaggc gggaaacgac        60
   aatctgatca tgagcggaga attaagggag tcacgttatg acccccgccg atgacgcggg      120
   acaagccgtt ttacgtttgg aactgacaga accgcaacgt tgaaggagcc actcagcaag       180
   ctggtacgat tgtaatacga ctcactatag ggcgaattga gcgctgttta aacgctcttc       240
   aactggaaga gcggttacgc tgtttaaacg ctcttcaact ggaagagcgg ttactaccgg       300
   ttcactagct agctgctaat cgagctagtt accctatgag gtgacatgaa gcgctcacgg       360
   ttactatgac ggttagcttc acgactgttg gtggcagtag cgtacgactt agctatagtt       420
   ccggacttac ccttaagata acttcgtata gcatacatta tacgaagtta tgggcccacg       480
   cgtgaagagc ggttaccaga gctggtcacc tttgtccacc aagatggaac tgtcttcggc       540
   cagaatggcc tccggaattt atcgctatca actttgtata gaaaagttgg gccgaattcg       600
   agctcggtac ggccagaatg gcccggaccg ggttaccgaa ttcgagctcg gtaccctggg       660
   atccggtgcg ggcctcttcg ctattacgcc agctggcgaa agggggatgt gctgcaaggc 720
   gattaagttg ggtaacgcca gggttttccc agtcacgacg ttgtaaaacg acggccagtg 780
   ccaagctcag atcagcttgc atgcctgcag tgcagcgtga cccggtcgtg cccctctcta 840
   gagataatga gcattgcatg tctaagttat aaaaaattac cacatatttt ttttgtcaca 900
   cttgtttgaa gtgcagttta tctatcttta tacatatatt taaactttac tctacgaata 960
   atataatcta tagtactaca ataatatcag tgttttagag aatcatataa atgaacagtt 1020
   agacatggtc taaaggacaa ttgagtattt tgacaacagg actctacagt tttatctttt 1080
   tagtgtgcat gtgttctcct ttttttttgc aaatagcttc acctatataa tacttcatcc 1140
   attttattag tacatccatt tagggtttag ggttaatggt ttttatagac taattttttt 1200
   agtacatcta ttttattcta ttttagcctc taaattaaga aaactaaaac tctattttag 1260
   tttttttatt taataattta gatataaaat agaataaaat aaagtgacta aaaattaaac 1320
   aaataccctt taagaaatta aaaaaactaa ggaaacattt ttcttgtttc gagtagataa 1380
   tgccagcctg ttaaacgccg tcgacgagtc taacggacac caaccagcga accagcagcg 1440
   tcgcgtcggg ccaagcgaag cagacggcac ggcatctctg tcgctgcctc tggacccctc 1500
   tcgagagttc cgctccaccg ttggacttgc tccgctgtcg gcatccagaa attgcgtggc 1560
   ggagcggcag acgtgagccg gcacggcagg cggcctcctc ctcctctcac ggcaccggca 1620
   gctacggggg attcctttcc caccgctcct tcgctttccc ttcctcgccc gccgtaataa 1680
   atagacaccc cctccacacc ctctttcccc aacctcgtgt tgttcggagc gcacacacac 1740
   acaaccagat ctcccccaaa tccacccgtc ggcacctccg cttcaaggta cgccgctcgt 1800
   cctccccccc ccccctctct accttctcta gatcggcgtt ccggtccatg catggttagg 1860
   gcccggtagt tctacttctg ttcatgtttg tgttagatcc gtgtttgtgt tagatccgtg 1920
   ctgctagcgt tcgtacacgg atgcgacctg tacgtcagac acgttctgat tgctaacttg 1980
   ccagtgtttc tctttgggga atcctgggat ggctctagcc gttccgcaga cgggatcgat 2040
   ttcatgattt tttttgtttc gttgcatagg gtttggtttg cccttttcct ttatttcaat 2100
   atatgccgtg cacttgtttg tcgggtcatc ttttcatgct tttttttgtc ttggttgtga 2160
   tgatgtggtc tggttgggcg gtcgttctag atcggagtag aattctgttt caaactacct 2220
   ggtggattta ttaattttgg atctgtatgt gtgtgccata catattcata gttacgaatt 2280
   gaagatgatg gatggaaata tcgatctagg ataggtatac atgttgatgc gggttttact 2340
   gatgcatata cagagatgct ttttgttcgc ttggttgtga tgatgtggtg tggttgggcg 2400
   gtcgttcatt cgttctagat cggagtagaa tactgtttca aactacctgg tgtatttatt 2460
   aattttggaa ctgtatgtgt gtgtcataca tcttcatagt tacgagttta agatggatgg 2520
   aaatatcgat ctaggatagg tatacatgtt gatgtgggtt ttactgatgc atatacatga 2580
   tggcatatgc agcatctatt catatgctct aaccttgagt acctatctat tataataaac 2640
   aagtatgttt tataattatt ttgatcttga tatacttgga tgatggcata tgcagcagct 2700
   atatgtggat ttttttagcc ctgccttcat acgctattta tttgcttggt actgtttctt 2760
   ttgtcgatgc tcaccctgtt gtttggtgtt acttctgcag gtcgactcta gaggatccac 2820
   cggtcgccac catggcaccg aagaagaagc gcaaggtgat ggacaagaag tacagcatcg 2880
   gcctcgacat cggcaccaac tcggtgggct gggccgtcat cacggacgaa tataaggtcc 2940
   cgtcgaagaa gttcaaggtc ctcggcaata cagaccgcca cagcatcaag aaaaacttga 3000
   tcggcgccct cctgttcgat agcggcgaga ccgcggaggc gaccaggctc aagaggaccg 3060
   ccaggagacg gtacactagg cgcaagaaca ggatctgcta cctgcaggag atcttcagca 3120
   acgagatggc gaaggtggac gactccttct tccaccgcct ggaggaatca ttcctggtgg 3180
   aggaggacaa gaagcatgag cggcacccaa tcttcggcaa catcgtcgac gaggtaagtt 3240
   tctgcttcta cctttgatat atatataata attatcatta attagtagta atataatatt 3300
   tcaaatattt ttttcaaaat aaaagaatgt agtatatagc aattgctttt ctgtagttta 3360
   taagtgtgta tattttaatt tataactttt ctaatatatg accaaaacat ggtgatgtgc 3420
   aggtggccta ccacgagaag tacccgacaa tctaccacct ccggaagaaa ctggtggaca 3480
   gcacagacaa ggcggacctc cggctcatct accttgccct cgcgcatatg atcaagttcc 3540
   gcggccactt cctcatcgag ggcgacctga acccggacaa ctccgacgtg gacaagctgt 3600
   tcatccagct cgtgcagacg tacaatcaac tgttcgagga gaaccccata aacgctagcg 3660
   gcgtggacgc caaggccatc ctctcggcca ggctctcgaa atcaagaagg ctggagaacc 3720
   ttatcgcgca gttgccaggc gaaaagaaga acggcctctt cggcaacctt attgcgctca 3780
   gcctcggcct gacgccgaac ttcaaatcaa acttcgacct cgcggaggac gccaagctcc 3840
   agctctcaaa ggacacctac gacgacgacc tcgacaacct cctggcccag ataggagacc 3900
   agtacgcgga cctcttcctc gccgccaaga acctctccga cgctatcctg ctcagcgaca 3960
   tccttcgggt caacaccgaa attaccaagg caccgctgtc cgccagcatg attaaacgct 4020
   acgacgagca ccatcaggac ctcacgctgc tcaaggcact cgtccgccag cagctccccg 4080
   agaagtacaa ggagatcttc ttcgaccaat caaaaaacgg ctacgcggga tatatcgacg 4140
   gcggtgccag ccaggaagag ttctacaagt tcatcaaacc aatcctggag aagatggacg 4200
   gcaccgagga gttgctggtc aagctcaaca gggaggacct cctcaggaag cagaggacct 4260
   tcgacaacgg ctccatcccg catcagatcc acctgggcga actgcatgcc atcctgcggc 4320
   gccaggagga cttctacccg ttcctgaagg ataaccggga gaagatcgag aagatcttga 4380
   cgttccgcat cccatactac gtgggcccgc tggctcgcgg caactcccgg ttcgcctgga 4440
   tgacccggaa gtcggaggag accatcacac cctggaactt tgaggaggtg gtcgataagg 4500
   gcgctagcgc tcagagcttc atcgagcgca tgaccaactt cgataaaaac ctgcccaatg 4560
   aaaaagtcct ccccaagcac tcgctgctct acgagtactt caccgtgtac aacgagctca 4620
   ccaaggtcaa atacgtcacc gagggcatgc ggaagccggc gttcctgagc ggcgagcaga 4680
   agaaggcgat agtggacctc ctcttcaaga ccaacaggaa ggtgaccgtg aagcaattaa 4740
   aagaggacta cttcaagaaa atagagtgct tcgactccgt ggagatctcg ggcgtggagg 4800
   atcggttcaa cgcctcactc ggcacgtatc acgacctcct caagatcatt aaagacaagg 4860
   acttcctcga caacgaggag aacgaggaca tcctcgagga catcgtcctc accctgaccc 4920
   tgttcgagga ccgcgaaatg atcgaggaga ggctgaagac ctacgcgcac ctgttcgacg 4980
   acaaggtcat gaaacagctc aagaggcgcc gctacactgg ttggggaagg ctgtcccgca 5040
   agctcattaa tggcatcagg gacaagcaga gcggcaagac catcctggac ttcctcaagt 5100
   ccgacgggtt cgccaaccgc aacttcatgc agctcattca cgacgactcg ctcacgttca 5160
   aggaagacat ccagaaggca caggtgagcg ggcagggtga ctccctccac gaacacatcg 5220
   ccaacctggc cggctcgccg gccattaaaa agggcatcct gcagacggtc aaggtcgtcg 5280
   acgagctcgt gaaggtgatg ggccggcaca agcccgaaaa tatcgtcata gagatggcca 5340
   gggagaacca gaccacccaa aaagggcaga agaactcgcg cgagcggatg aaacggatcg 5400
   aggagggcat taaagagctc gggtcccaga tcctgaagga gcaccccgtg gaaaataccc 5460
   agctccagaa tgaaaagctc tacctctact acctgcagaa cggccgcgac atgtacgtgg 5520
   accaggagct ggacattaat cggctatcgg actacgacgt cgaccacatc gtgccgcagt 5580
   cgttcctcaa ggacgatagc atcgacaaca aggtgctcac ccggtcggat aaaaatcggg 5640
   gcaagagcga caacgtgccc agcgaggagg tcgtgaagaa gatgaaaaac tactggcgcc 5700
   agctcctcaa cgcgaaactg atcacccagc gcaagttcga caacctgacg aaggcggaac 5760
   gcggtggctt gagcgaactc gataaggcgg gcttcataaa aaggcagctg gtcgagacgc 5820
   gccagatcac gaagcatgtc gcccagatcc tggacagccg catgaatact aagtacgatg 5880
   aaaacgacaa gctgatccgg gaggtgaagg tgatcacgct gaagtccaag ctcgtgtcgg 5940
   acttccgcaa ggacttccag ttctacaagg tccgcgagat caacaactac caccacgccc 6000
   acgacgccta cctgaatgcg gtggtcggga ccgccctgat caagaagtac ccgaagctgg 6060
   agtcggagtt cgtgtacggc gactacaagg tctacgacgt gcgcaaaatg atcgccaagt 6120
   ccgagcagga gatcggcaag gccacggcaa aatacttctt ctactcgaac atcatgaact 6180
   tcttcaagac cgagatcacc ctcgcgaacg gcgagatccg caagcgcccg ctcatcgaaa 6240
   ccaacggcga gacgggcgag atcgtctggg ataagggccg ggatttcgcg acggtccgca 6300
   aggtgctctc catgccgcaa gtcaatatcg tgaaaaagac ggaggtccag acgggcgggt 6360
   tcagcaagga gtccatcctc ccgaagcgca actccgacaa gctcatcgcg aggaagaagg 6420
   attgggaccc gaaaaaatat ggcggcttcg acagcccgac cgtcgcatac agcgtcctcg 6480
   tcgtggcgaa ggtggagaag ggcaagtcaa agaagctcaa gtccgtgaag gagctgctcg 6540
   ggatcacgat tatggagcgg tcctccttcg agaagaaccc gatcgacttc ctagaggcca 6600
   agggatataa ggaggtcaag aaggacctga ttattaaact gccgaagtac tcgctcttcg 6660
   agctggaaaa cggccgcaag aggatgctcg cctccgcagg cgagttgcag aagggcaacg 6720
   agctcgccct cccgagcaaa tacgtcaatt tcctgtacct cgctagccac tatgaaaagc 6780
   tcaagggcag cccggaggac aacgagcaga agcagctctt cgtggagcag cacaagcatt 6840
   acctggacga gatcatcgag cagatcagcg agttctcgaa gcgggtgatc ctcgccgacg 6900
   cgaacctgga caaggtgctg tcggcatata acaagcaccg cgacaaacca atacgcgagc 6960
   aggccgaaaa tatcatccac ctcttcaccc tcaccaacct cggcgctccg gcagccttca 7020
   agtacttcga caccacgatt gaccggaagc ggtacacgag cacgaaggag gtgctcgatg 7080
   cgacgctgat ccaccagagc atcacagggc tctatgaaac acgcatcgac ctgagccagc 7140
   tgggcggaga caagagacca cgggaccgcc acgatggcga gctgggaggc cgcaagcggg 7200
   caaggtaggt accgttaacc tagacttgtc catcttctgg attggccaac ttaattaatg 7260
   tatgaaataa aaggatgcac acatagtgac atgctaatca ctataatgtg ggcatcaaag 7320
   ttgtgtgtta tgtgtaatta ctagttatct gaataaaaga gaaagagatc atccatattt 7380
   cttatcctaa atgaatgtca cgtgtcttta taattctttg atgaaccaga tgcatttcat 7440
   taaccaaatc catatacata taaatattaa tcatatataa ttaatatcaa ttgggttagc 7500
   aaaacaaatc tagtctaggt gtgttttgcg aattcgtaat catgtcatag ctgtttcctg 7560
   tgtgaaattg ttatccgctc acaattccac acaacatacg agccggaagc ataaagtgta 7620
   aagcctgggg tgcctaatga gtgagctaac tcacattaat tgcgttgcgc tcactgcccg 7680
   ctttccagtc gggaaacctg tcgtgccagc tgcattaatg aatcggccaa cgcgcgggga 7740
   gaggcggttt gcgtattggg cgctcttccg ctgatccgat atcgatgggc cctggccgaa 7800
   gcttggtcac ccggtccggg cctagaaggc cagcttcaag tttgtacaaa aaagcaggct 7860
   ccggccagaa tggcccggac cgggttaccg aattcgagct cggtaccctg ggatccgata 7920
   tcgatgggcc ctggccgaag ctttgagagt acaatgatga acctagatta atcaatgcca 7980
   aagtctgaaa aatgcaccct cagtctatga tccagaaaat caagattgct tgaggccctg 8040
   ttcggttgtt ccggattaga gccccggatt aattcctagc cggattactt ctctaattta 8100
   tatagatttt gatgagctgg aatgaatcct ggcttattcc ggtacaaccg aacaggccct 8160
   gaaggatacc agtaatcgct gagctaaatt ggcatgctgt cagagtgtca gtattgcagc 8220
   aaggtagtga gataaccggc atcatggtgc cagtttgatg gcaccattag ggttagagat 8280
   ggtggccatg ggcgcatgtc ctggccaact ttgtatgata tatggcaggg tgaataggaa 8340
   agtaaaattg tattgtaaaa agggatttct tctgtttgtt agcgcatgta caaggaatgc 8400
   aagttttgag cgagggggca tcaaagatct ggctgtgttt ccagctgttt ttgttagccc 8460
   catcgaatcc ttgacataat gatcccgctt aaataagcaa cctcgcttgt atagttcctt 8520
   gtgctctaac acacgatgat gataagtcgt aaaatagtgg tgtccaaaga atttccaggc 8580
   ccagttgtaa aagctaaaat gctattcgaa tttctactag cagtaagtcg tgtttagaaa 8640
   ttattttttt atataccttt tttccttcta tgtacagtag gacacagtgt cagcgccgcg 8700
   ttgacggaga atatttgcaa aaaagtaaaa gagaaagtca tagcggcgta tgtgccaaaa 8760
   acttcgtcac agagagggcc ataagaaaca tggcccacgg cccaatacga agcaccgcga 8820
   cgaagcccaa acagcagtcc gtaggtggag caaagcgctg ggtaatacgc aaacgttttg 8880
   tcccaccttg actaatcaca agagtggagc gtaccttata aaccgagccg caagcaccga 8940
   attgcatcat atctatccgt agccgtttta gagctagaaa tagcaagtta aaataaggct 9000
   agtccgttat caacttgaaa aagtggcacc gagtcggtgc tttttttttg cggccgcgaa 9060
   ttcctgcagg gccctcttgt cggaccagtt gcccaccacg ttggtgaggg gcacccagct 9120
   ttcttgtaca aagtggccgt taacggatcg gccagaatgg cccggaccgg gttaccgaat 9180
   tcgagctcgg taccctggga tccgatatcg atgggccctg gccgaagctt tgagagtaca 9240
   atgatgaacc tagattaatc aatgccaaag tctgaaaaat gcaccctcag tctatgatcc 9300
   agaaaatcaa gattgcttga ggccctgttc ggttgttccg gattagagcc ccggattaat 9360
   tcctagccgg attacttctc taatttatat agattttgat gagctggaat gaatcctggc 9420
   ttattccggt acaaccgaac aggccctgaa ggataccagt aatcgctgag ctaaattggc 9480
   atgctgtcag agtgtcagta ttgcagcaag gtagtgagat aaccggcatc atggtgccag 9540
   tttgatggca ccattagggt tagagatggt ggccatgggc gcatgtcctg gccaactttg 9600
   tatgatatat ggcagggtga ataggaaagt aaaattgtat tgtaaaaagg gatttcttct 9660
   gtttgttagc gcatgtacaa ggaatgcaag ttttgagcga gggggcatca aagatctggc 9720
   tgtgtttcca gctgtttttg ttagccccat cgaatccttg acataatgat cccgcttaaa 9780
   taagcaacct cgcttgtata gttccttgtg ctctaacaca cgatgatgat aagtcgtaaa 9840
   atagtggtgt ccaaagaatt tccaggccca gttgtaaaag ctaaaatgct attcgaattt 9900
   ctactagcag taagtcgtgt ttagaaatta tttttttata tacctttttt ccttctatgt 9960
   acagtaggac acagtgtcag cgccgcgttg acggagaata tttgcaaaaa agtaaaagag 10020
   aaagtcatag cggcgtatgt gccaaaaact tcgtcacaga gagggccata agaaacatgg 10080
   cccacggccc aatacgaagc accgcgacga agcccaaaca gcagtccgta ggtggagcaa 10140
   agcgctgggt aatacgcaaa cgttttgtcc caccttgact aatcacaaga gtggagcgta 10200
   ccttataaac cgagccgcaa gcaccgaatt gttacttctc taagcacggc gttttagagc 10260
   tagaaatagc aagttaaaat aaggctagtc cgttatcaac ttgaaaaagt ggcaccgagt 10320
   cggtgctttt tttttgcggc cgcgaattcc tgcagggccc tcttgtcgga ccagttgccc 10380
   accacgttgg tgaggggcaa ctttattata caaagttgat agataatacg cgtctatagt 10440
   attttaaaat tgcattaaca aacatgtcct aattggtact cctgagatac tataccctcc 10500
   tgttttaaaa tagttggcat tatcgaatta tcattttact ttttaatgtt ttctcttctt 10560
   ttaatatatt ttatgaattt taatgtattt taaaatgtta tgcagttcgc tctggacttt 10620
   tctgctgcgc ctacacttgg gtgtactggg cctaaattca gcctgaccga ccgcctgcat 10680
   tgaataatgg atgagcaccg gtaaaatccg cgtacccaac tttcgagaag aaccgagacg 10740
   tggcgggccg ggccaccgac gcacggcacc agcgactgca cacgtcccgc cggcgtacgt 10800
   gtacgtgctg ttccctcact ggccgcccaa tccactcatg catgcccacg tacacccctg 10860
   ccgtggcgcg cccagatcct aatcctttcg ccgttctgca cttctgctgc ctataaatgg 10920
   cggcatcgac cgtcacctgc ttcaccaccg gcgagccaca tcgagaacac gatcgagcac 10980
   acaagcacga agactcgttt aggagaaacc acaaaccacc aagccgtgca agcaccaggc 11040
   ttgggcaccc gctccgggct tagaaggcca gcttcaagtt tgtacaaaaa agcaggcttc 11100
   gaaggagata gaaccgatcc accatgtcca acctgctcac ggttcaccag aaccttccgg 11160
   ctcttccagt ggacgcgacg tccgatgaag tcaggaagaa cctcatggac atgttccgcg 11220
   acaggcaagc gttcagcgag cacacctgga agatgctgct ctccgtctgc cgctcctggg 11280
   ctgcatggtg caagctgaac aacaggaagt ggttccccgc tgagcccgag gacgtgaggg 11340
   attaccttct gtacctgcaa gcgcgaggtt tgtttctgct tctacctttg atatatatat 11400
   aataattatc attaattagt agtaatataa tatttcaaat atttttttca aaataaaaga 11460
   atgtagtata tagcaattgc ttttctgtag tttataagtg tgtatatttt aatttataac 11520
   ttttctaata tatgaccaaa acatggtgat gcctaggtct ggcagtgaag accatccagc 11580
   aacaccttgg acaactgaac atgcttcaca ggcgctccgg cctcccgcgc cccagcgact 11640
   cgaacgccgt gagcctcgtc atgcgccgca tcaggaagga aaacgtcgat gccggcgaaa 11700
   gggcaaagca ggccctcgcg ttcgagagga ccgatttcga ccaggtccgc agcctgatgg 11760
   agaacagcga caggtgccag gacattagga acctggcgtt cctcggaatt gcatacaaca 11820
   cgctcctcag gatcgcggaa attgcccgca ttcgcgtgaa ggacattagc cgcaccgacg 11880
   gcggcaggat gcttatccac attggcagga ccaagacgct cgtttccacc gcaggcgtcg 11940
   aaaaggccct cagcctcgga gtgaccaagc tcgtcgaacg ctggatctcc gtgtccggcg 12000
   tcgcggacga cccaaacaac tacctcttct gccgcgtccg caagaacggg gtggctgccc 12060
   ctagcgccac cagccaactc agcacgaggg ccttggaagg tattttcgag gccacccacc 12120
   gcctgatcta cggcgcgaag gatgacagcg gtcaacgcta cctcgcatgg tccgggcact 12180
   ccgcccgcgt tggagctgct agggacatgg cccgcgccgg tgtttccatc cccgaaatca 12240
   tgcaggcggg tggatggacg aacgtgaaca ttgtcatgaa ctacattcgc aaccttgaca 12300
   gcgagacggg cgcaatggtt cgcctcctgg aagatggtga ctgagctaga cccagctttc 12360
   ttgtacaaag tggccgttaa cggatccaga cttgtccatc ttctggattg gccaacttaa 12420
   ttaatgtatg aaataaaagg atgcacacat agtgacatgc taatcactat aatgtgggca 12480
   tcaaagttgt gtgttatgtg taattactag ttatctgaat aaaagagaaa gagatcatcc 12540
   atatttctta tcctaaatga atgtcacgtg tctttataat tctttgatga accagatgca 12600
   tttcattaac caaatccata tacatataaa tattaatcat atataattaa tatcaattgg 12660
   gttagcaaaa caaatctagt ctaggtgtgt tttgcgaatg cggccgccac cgcggcctaa 12720
   ggggtcaccg gcctagaagg ccatttaaat cagatatctg gttcctaagg acccggcgga 12780
   ccgattaaac tgattcggtc cgacgcgtta cctaataact tcgtatagca tacattatac 12840
   gaagttatta gctaactaac tagtaagctt aagccatggg tcgacggatc cgttaactca 12900
   cttaagatgt actcgacaat ggtgccctca taccggcatg tgtttcctag aaataatcaa 12960
   tatattgatt gagatttatc tcgatatatt tctgaactat gttcatcata taaataactg 13020
   aaaacatcaa atcgtaattt taaactcatg cttggtcaat acatagataa tacaatatta 13080
   cttcatcatc ccaatgatgt cctagcacaa cctattgaat gttaatgttt ggttgtgtgg 13140
   gggtgtgttt ataacataga tgtgattatt tgtgcttttt gttgagtata tacatatatg 13200
   gtatgttgat ttgatatagt gatggacaca tgctttggcc ttggatattc aaatcacttg 13260
   tacttgcacg aagcaaaaca taatataagt ttagaagtaa acttgtaact gtgtccaaac 13320
   atgctcacac aaagtcatat cgcattatat ttttttggta aatattcaac acatgtattt 13380
   tttacaagaa cccaaatttt acagacaaat gcagcattgt agacatgtag aattctttga 13440
   agcatgtgaa cttaacaaca ctaatgtcat taaatcaact ggaccctatg agtaacaatt 13500
   tcgatattgc aaacaccaaa ttatggaact tatttgctga aaaaattatg atcaatgtga 13560
   agtttaaatt attataccat aaatatatca aagatttttt ttgaggaagg taaaaattgc 13620
   atggaatggg ctgcccaacg tgatagctca cttttatgct aggtagcatt accaaagatg 13680
   ggaatgttct gatgaacacc aaacccactc aaataatatt tatatttggg ttgtttagtt 13740
   gtaaaagtga agacccaaga ttaaagtacc aattgtcgat gccatacact agaagtgaag 13800
   aatgttgtta gcgttaggag tagatgtgtc aatggggacc gatactcgtt atcgatgggc 13860
   cgcacgtaag cttgcatgcc tgcagtgcag cgtgacccgg tcgtgcccct ctctagagat 13920
   aatgagcatt gcatgtctaa gttataaaaa attaccacat attttttttg tcacacttgt 13980
   ttgaagtgca gtttatctat ctttatacat atatttaaac tttactctac gaataatata 14040
   atctatagta ctacaataat atcagtgttt tagagaatca tataaatgaa cagttagaca 14100
   tggtctaaag gacaattgag tattttgaca acaggactct acagttttat ctttttagtg 14160
   tgcatgtgtt ctcctttttt tttgcaaata gcttcaccta tataatactt catccatttt 14220
   attagtacat ccatttaggg tttagggtta atggttttta tagactaatt tttttagtac 14280
   atctatttta ttctatttta gcctctaaat taagaaaact aaaactctat tttagttttt 14340
   ttatttaata atttagatat aaaatagaat aaaataaagt gactaaaaat taaacaaata 14400
   ccctttaaga aattaaaaaa actaaggaaa catttttctt gtttcgagta gataatgcca 14460
   gcctgttaaa cgccgtcgac gagtctaacg gacaccaacc agcgaaccag cagcgtcgcg 14520
   tcgggccaag cgaagcagac ggcacggcat ctctgtcgct gcctctggac ccctctcgag 14580
   agttccgctc caccgttgga cttgctccgc tgtcggcatc cagaaattgc gtggcggagc 14640
   ggcagacgtg agccggcacg gcaggcggcc tcctcctcct ctcacggcac cggcagctac 14700
   gggggattcc tttcccaccg ctccttcgct ttcccttcct cgcccgccgt aataaataga 14760
   caccccctcc acaccctctt tccccaacct cgtgttgttc ggagcgcaca cacacacaac 14820
   cagatctccc ccaaatccac ccgtcggcac ctccgcttca aggtacgccg ctcgtcctcc 14880
   cccccccccc tctctacctt ctctagatcg gcgttccggt ccatgcatgg ttagggcccg 14940
   gtagttctac ttctgttcat gtttgtgtta gatccgtgtt tgtgttagat ccgtgctgct 15000
   agcgttcgta cacggatgcg acctgtacgt cagacacgtt ctgattgcta acttgccagt 15060
   gtttctcttt ggggaatcct gggatggctc tagccgttcc gcagacggga tcgatttcat 15120
   gatttttttt gtttcgttgc atagggtttg gtttgccctt ttcctttatt tcaatatatg 15180
   ccgtgcactt gtttgtcggg tcatcttttc atgctttttt ttgtcttggt tgtgatgatg 15240
   tggtctggtt gggcggtcgt tctagatcgg agtagaattc tgtttcaaac tacctggtgg 15300
   atttattaat tttggatctg tatgtgtgtg ccatacatat tcatagttac gaattgaaga 15360
   tgatggatgg aaatatcgat ctaggatagg tatacatgtt gatgcgggtt ttactgatgc 15420
   atatacagag atgctttttg ttcgcttggt tgtgatgatg tggtgtggtt gggcggtcgt 15480
   tcattcgttc tagatcggag tagaatactg tttcaaacta cctggtgtat ttattaattt 15540
   tggaactgta tgtgtgtgtc atacatcttc atagttacga gtttaagatg gatggaaata 15600
   tcgatctagg ataggtatac atgttgatgt gggttttact gatgcatata catgatggca 15660
   tatgcagcat ctattcatat gctctaacct tgagtaccta tctattataa taaacaagta 15720
   tgttttataa ttattttgat cttgatatac ttggatgatg gcatatgcag cagctatatg 15780
   tggatttttt tagccctgcc ttcatacgct atttatttgc ttggtactgt ttcttttgtc 15840
   gatgctcacc ctgttgtttg gtgttacttc tgcaggtcga ctctagagga tccaccatgg 15900
   ttgaacaaga tggattgcac gcaggttctc cggccgcttg ggtggagagg ctattcggct 15960
   atgactgggc acaacagaca atcggctgct ctgatgccgc cgtgttccgg ctgtcagcgc 16020
   aggggcgccc ggttcttttt gtcaagaccg acctgtccgg tgccctgaat gaactgcagg 16080
   acgaggcagc gcggctatcg tggctggcca cgacgggcgt tccttgcgca gctgtgctcg 16140
   acgttgtcac tgaagcggga agggactggc tgctattggg cgaagtgccg gggcaggatc 16200
   tcctgtcatc tcaccttgct cctgccgaga aagtatccat catggctgat gcaatgcggc 16260
   ggctgcatac gcttgatccg gctacctgcc cattcgacca ccaagcgaaa catcgcatcg 16320
   agcgagcacg tactcggatg gaagccggtc ttgtcgatca ggatgatctg gacgaagagc 16380
   atcaggggct cgcgccagcc gaactgttcg ccaggctcaa ggcgcgcatg cccgacggcg 16440
   atgatctcgt cgtgacccat ggcgatgcct gcttgccgaa tatcatggtg gaaaatggcc 16500
   gcttttctgg attcatcgac tgtggccggc tgggtgtggc ggaccgctat caggacatag 16560
   cgttggctac ccgtgatatt gctgaagagc ttggcggcga atgggctgac cgcttcctcg 16620
   tgctttacgg tatcgccgct cccgattcgc agcgcatcgc cttctatcgc cttcttgacg 16680
   agttcttctg aggatccacc atggttaacc tagacttgtc catcttctgg attggccaac 16740
   ttaattaatg tatgaaataa aaggatgcac acatagtgac atgctaatca ctataatgtg 16800
   ggcatcaaag ttgtgtgtta tgtgtaatta ctagttatct gaataaaaga gaaagagatc 16860
   atccatattt cttatcctaa atgaatgtca cgtgtcttta taattctttg atgaaccaga 16920
   tgcatttcat taaccaaatc catatacata taaatattaa tcatatataa ttaatatcaa 16980
   ttgggttagc aaaacaaatc tagtctaggt gtgttttgcg aatgcgacct tcttatgtgc 17040
   ttctagtctc caaatgtggt tgatagttat tttgctctaa gatcaacagt aatgaagtat 17100
   aaatcatcgt tgtggtgtgc tactcggtta attgagcatt aacacacaca aacatgacga 17160
   ggatggtata atctccaaaa atgtgtactt tgttaggtgg gaccctatag ccttgattaa 17220
   tgtgctatgt taggcatgcc tggaaacgtg tgacgcatat gttttgtgaa cctgttgata 17280
   ttatatgtgc ttttatatta ccatatttta ttaaaatact aatatttatt actagtaaga 17340
   tataacattc tatctagctt aaaaactaac cataaatatt ccataataac tagatttacc 17400
   aaactaatat actaaatata cataataaat acaaaattaa caagacaata atcaatattt 17460
   atgagcttaa tatatttaga cattatggtt ggtcgacgat aatcatgcta acttttcgta 17520
   attgcttgat tgaaatatgc ttagaataat gcctctttgt tctacatggc aaatagggac 17580
   cattatggtg taacaccctg ggaaccacaa acaccccgaa atgctactaa actacacaac 17640
   taaccttcat atataaaatt tcgacagcat ctcctttgaa aatttgcata gacgtggaag 17700
   caacagagta taaacagata tcatgataag aaaacatact agacattaat aatctgctag 17760
   aaatgggaag aatcctaact tgacgactgc gtaactgact agagtcacac ttagctgacc 17820
   ctagtcactt acaactgact tcgtgtccta gggcgatccg atatcgatgg gccctggccg 17880
   aagcttggtc acccggtccg ggcctagaag gccagcttcg gccgccccaa ttcccatgga 17940
   gtcaaagatt caaatagagg acctaacaga actcgccgta aagactggcg aacagttcat 18000
   acagagtctc ttacgactca atgacaagaa gaaaatcttc gtcaacatgg tggagcacga 18060
   cacgcttgtc tactccaaaa atatcaaaga tacagtctca gaagaccaaa gggcaattga 18120
   gacttttcaa caaagggtaa tatccggaaa cctcctcgga ttccattgcc cagctatctg 18180
   tcactttatt gtgaagatag tggaaaagga aggtggctcc tacaaatgcc atcattgcga 18240
   taaaggaaag gccatcgttg aagatgcctc tgccgacagt ggtcccaaag atggaccccc 18300
   acccacgagg agcatcgtgg aaaaagaaga cgttccaacc acgtcttcaa agcaagtgga 18360
   ttgatgtgat atctccactg acgtaaggga tgacgcacaa tcccactaag ctgaccgaag 18420
   ctggccgctc tagaactagt ggatctcgat gtgtagtcta cgagaagggt taaccgtctc 18480
   ttcgtgagaa taaccgtggc ctaaaaataa gccgatgagg ataaataaaa tgtggtggta 18540
   cagtacttca agaggtttac tcatcaagag gatgcttttc cgatgagctc tagtagtaca 18600
   tcggacctca catacctcca ttgtggtgaa atattttgtg ctcatttagt gatgggtaaa 18660
   ttttgtttat gtcactctag gttttgacat ttcagttttg ccactcttag gttttgacaa 18720
   ataatttcca ttccgcggca aaagcaaaac aattttattt tacttttacc actcttagct 18780
   ttcacaatgt atcacaaatg ccactctaga aattctgttt atgccacaga atgtgaaaaa 18840
   aaacactcac ttatttgaag ccaaggtgtt catggcatgg aaatgtgaca taaagtaacg 18900
   ttcgtgtata agaaaaaatt gtactcctcg taacaagaga cggaaacatc atgagacaat 18960
   cgcgtttgga aggctttgca tcacctttgg atgatgcgca tgaatggagt cgtctgcttg 19020
   ctagccttcg cctaccgccc actgagtccg ggcggcaact accatcggcg aacgacccag 19080
   ctgacctcta ccgaccggac ttgaatgcgc taccttcgtc agcgacgatg gccgcgtacg 19140
   ctggcgacgt gcccccgcat gcatggcggc acatggcgag ctcagaccgt gcgtggctgg 19200
   ctacaaatac gtaccccgtg agtgccctag ctagaaactt acacctgcaa ctgcgagagc 19260
   gagcgtgtga gtgtagccga gtagatcccc cgggctgcag gtcgactcta gaggatccac 19320
   cggtcgccac catggccctg tccaacaagt tcatcggcga cgacatgaag atgacctacc 19380
   acatggacgg ctgcgtgaac ggccactact tcaccgtgaa gggcgagggc agcggcaagc 19440
   cctacgaggg cacccagacc tccaccttca aggtgaccat ggccaacggc ggccccctgg 19500
   ccttctcctt cgacatcctg tccaccgtgt tcatgtacgg caaccgctgc ttcaccgcct 19560
   accccaccag catgcccgac tacttcaagc aggccttccc cgacggcatg tcctacgaga 19620
   gaaccttcac ctacgaggac ggcggcgtgg ccaccgccag ctgggagatc agcctgaagg 19680
   gcaactgctt cgagcacaag tccaccttcc acggcgtgaa cttccccgcc gacggccccg 19740
   tgatggccaa gaagaccacc ggctgggacc cctccttcga gaagatgacc gtgtgcgacg 19800
   gcatcttgaa gggcgacgtg accgccttcc tgatgctgca gggcggcggc aactacagat 19860
   gccagttcca cacctcctac aagaccaaga agcccgtgac catgcccccc aaccacgtgg 19920
   tggagcaccg catcgccaga accgacctgg acaagggcgg caacagcgtg cagctgaccg 19980
   agcacgccgt ggcccacatc acctccgtgg tgcccttctg aagcggccca tggatattcg 20040
   aacgcgtcgc tgaaatcacc agtctctctc tacaaatcta tctctctcta taataatgtg 20100
   tgagtagttc ccagataagg gaattagggt tcttataggg tttcgctcat gtgttgagca 20160
   tataagaaac ccttagtatg tatttgtatt tgtaaaatac ttctatcaat aaaatttcta 20220
   attcctaaaa ccaaaatcca gtggcgagct cgagccaggg tgcttcctga gctgattccg 20280
   atgacttcgt aggttcctag ctcaagccgc tcgtgtccaa gcgtcactta cgattagcta 20340
   atgattacgg catctaggac cgactagcta actaactagt acgccgacta gctagctaag 20400
   gtaccgagct cgaattcatt ccgattaatc gtggcctctt gctcttcagg atgaagagct 20460
   atgtttaaac gtgcaagcgc tactagacaa ttcagtacat taaaaacgtc cgcaatgtgt 20520
   tattaagttg tctaagcgtc aatttgttta caccacaata tatcctgcca 20570
<210> 73
   <211> 20572
   <212> DNA
   <213> Artificial sequence
<220>
   <223> Synthetic construct
<400> 73
<210> 74
   <211> 26187
   <212> DNA
   <213> Artificial sequence
<220>
   <223> Synthetic construct
<400> 74
   gtttacccgc caatatatcc tgtcaaacac tgatagttta aactgaaggc gggaaacgac 60
   aatctgatca tgagcctagt tagttagggg cagctaacgt cccctagcta actagttgct 120
   attaaccttt agcctagttt agtgcggtcg cgctagtgaa ctagtctaat gggtgcctag 180
   cttagattag atccccgttc ggcttagact aggttagtcg gatagggcta gtctagtggc 240
   ttagcttgga actgttaagc taggctgtta gtgcgggact tctaggctaa cctattgacc 300
   cctaacgcct agaacctaag aaacgctaag gacacctagc taactttggc gacgtccaga 360
   ggtccgattc cggctaacta actagcttaa gcttgatatc gaattcctgc agcccatccc 420
   tcagccgcct ttcactatct tttttgcccg agtcattgtc atgtgaacct tggcatgtat 480
   aatcggtgaa ttgcgtcgat tttcctctta taggtgggcc aatgaatccg tgtgatcgcg 540
   tctgattggc tagagatatg tttcttcctt gttggatgta ttttcataca taatcatatg 600
   catacaaata tttcattaca ctttatagaa atggtcagta ataaacccta tcactatgtc 660
   tggtgtttca ttttatttgc ttttaaacga aaattgactt cctgattcaa tatttaagga 720
   tcgtcaacgg tgtgcagtta ctaaattctg gtttgtagga actatagtaa actattcaag 780
   tcttcactta ttgtgcactc acctctcgcc acatcaccac agatgttatt cacgtcttaa 840
   atttgaacta cacatcatat tgacacaata ttttttttaa ataagcgatt aaaacctagc 900
   ctctatgtca acaatggtgt acataaccag cgaagtttag ggagtaaaaa acatcgcctt 960
   acacaaagtt cgctttaaaa aataaagagt aaattttact ttggaccacc cttcaaccaa 1020
   tgtttcactt tagaacgagt aattttatta ttgtcacttt ggaccaccct caaatctttt 1080
   ttccatctac atccaattta tcatgtcaaa gaaatggtct acatacagct aaggagattt 1140
   atcgacgaat agtagctagc atactcgagg tcattcatat gcttgagaag agagtcggga 1200
   tagtccaaaa taaaacaaag gtaagattac ctggtcaaaa gtgaaaacat cagttaaaag 1260
   gtggtataaa gtaaaatatc ggtaataaaa ggtggcccaa agtgaaattt actcttttct 1320
   actattataa aaattgagga tgtttttgtc ggtactttga tacgtcattt ttgtatgaat 1380
   tggtttttaa gtttattcgc ttttggaaat gcatatctgt atttgagtcg ggttttaagt 1440
   tcgtttgctt ttgtaaatac agagggattt gtataagaaa tatctttaaa aaaacccata 1500
   tgctaatttg acataatttt tgaaaaaaat atatattcag gcgaattctc acaatgaaca 1560
   ataataagat taaaatagct ttcccccgtt gcagcgcatg ggtatttttt ctagtaaaaa 1620
   taaaagataa acttagactc aaaacattta caaaaacaac ccctaaagtt cctaaagccc 1680
   aaagtgctat ccacgatcca tagcaagccc agcccaaccc aacccaaccc aacccacccc 1740
   agtccagcca actggacaat agtctccaca cccccccact atcaccgtga gttgtccgca 1800
   cgcaccgcac gtctcgcagc caaaaaaaaa aaaagaaaga aaaaaaagaa aaagaaaaaa 1860
   cagcaggtgg gtccgggtcg tgggggccgg aaacgcgagg aggatcgcga gccagcgacg 1920
   aggccggccc tccctccgct tccaaagaaa cgccccccat cgccactata tacatacccc 1980
   cccctctcct cccatccccc caaccctacc accaccacca ccaccacctc cacctcctcc 2040
   cccctcgctg ccggacgacg agctcctccc ccctccccct ccgccgccgc cgcgccggta 2100
   accaccccgc ccctctcctc tttctttctc cgtttttttt ttccgtcacg gtctcgatct 2160
   ttggccttgg tagtttgggt gggcgagagg cggcttcgtg cgcgcccaga tcggtgcgcg 2220
   ggaggggcgg gatctcgcgg ctggggctct cgccggcgtg gatcaggccc ggatctcgcg 2280
   gggaatgggg ctctcggatg tagatctgcg atccgccgtt gttgggggag atgatggggg 2340
   gtttaaaatt tccgccatgc taaacaagat caggaagagg ggaaaagggc actatggttt 2400
   atatttttat atatttctgc tgcttcgtca ggcttagatg tgctagatct ttctttcttc 2460
   tttttgtggg tagaatttga atccctcagc attgttcatc ggtagttttt cttttcatga 2520
   tttgtgacaa atgcagcctc gtgcggagct tttttgtagg tagaaggatc catggcggcc 2580
   aatgcgggcg gcggtggagc gggaggaggc agcggcagcg gcagcgtggc tgcgccggcg 2640
   gtgtgccgcc ccagcggctc gcggtggacg ccgacgccgg agcagatcag gatgctgaag 2700
   gagctctact acggctgcgg catccggtcg cccagctcgg agcagatcca gcgcatcacc 2760
   gccatgctgc ggcagcacgg caagatcgag ggcaagaacg tcttctactg gttccagaac 2820
   cacaaggccc gcgagcgcca gaagcgccgc ctcaccagcc tcgacgtcaa cgtgcccgcc 2880
   gccggcgcgg ccgacgccac caccagccaa ctcggcgtcc tctcgctgtc gtcgccgccg 2940
   ccttcaggcg cggcgcctcc ctcgcccacc ctcggcttct acgccgccgg caatggcggc 3000
   ggatcggctg tgctgctgga cacgagttcc gactggggca gcagcggcgc tgctatggcc 3060
   accgagacat gcttcctgca ggactacatg ggcgtgacgg acacgggcag ctcgtcgcag 3120
   tggccacgct tctcgtcgtc ggacacgata atggcggcgg ccgcggcgcg ggcggcgacg 3180
   acgcgggcgc ccgagacgct ccctctcttc ccgacctgcg gcgacgacgg cggcagcggt 3240
   agcagcagct acttgccgtt ctggggtgcc gcgtccacaa ctgccggcgc cacttcttcc 3300
   gttgcgatcc aacagcaaca ccagctgcag gagcagtaca gcttttacag caacagcaac 3360
   agcacccagc tggccggcac cggcaaccaa gacgtatcgg caacagcagc agcagccgcc 3420
   gccctggagc tgagcctcag ctcatggtgc tccccttacc ctgctgcagg gagtatgtga 3480
   acctagactt gtccatcttc tggattggcc aacttaatta atgtatgaaa taaaaggatg 3540
   cacacatagt gacatgctaa tcactataat gtgggcatca aagttgtgtg ttatgtgtaa 3600
   ttactagtta tctgaataaa agagaaagag atcatccata tttcttatcc taaatgaatg 3660
   tcacgtgtct ttataattct ttgatgaacc agatgcattt cattaaccaa atccatatac 3720
   atataaatat taatcatata taattaatat caattgggtt agcaaaacaa atctagtcta 3780
   ggtgtgtttt gcggcgatcg cggtaccatt taaattgcgc ccgccacggc cgtggaggtc 3840
   gtattccggt cagcttgcat ccctgcagtg cagcgtgacc cggtcgtgcc cctctctaga 3900
   gataatgagc attgcatgtc taagttataa aaaattacca catatttttt ttgtcacact 3960
   tgtttgaagt gcagtttatc tatctttata catatattta aactttactc tacgaataat 4020
   ataatctata gtactacaat aatatcagtg ttttagagaa tcatataaat gaacagttag 4080
   acatggtcta aaggacaatt gagtattttg acaacaggac tctacagttt tatcttttta 4140
   gtgtgcatgt gttctccttt ttttttgcaa atagcttcac ctatataata cttcatccat 4200
   tttattagta catccattta gggtttaggg ttaatggttt ttatagacta atttttttag 4260
   tacatctatt ttattctatt ttagcctcta aattaagaaa actaaaactc tattttagtt 4320
   tttttattta ataatttaga tataaaatag aataaaataa agtgactaaa aattaaacaa 4380
   atacccttta agaaattaaa aaaactaagg aaacattttt cttgtttcga gtagataatg 4440
   ccagcctgtt aaacgccgtc gacgagtcta acggacacca accagcgaac cagcagcgtc 4500
   gcgtcgggcc aagcgaagca gacggcacgg catctctgtc gctgcctctg gacccctctc 4560
   gagagttccg ctccaccgtt ggacttgctc cgctgtcggc atccagaaat tgcgtggcgg 4620
   agcggcagac gtgagccggc acggcaggcg gcctcctcct cctctcacgg caccggcagc 4680
   tacgggggat tcctttccca ccgctccttc gctttccctt cctcgcccgc cgtaataaat 4740
   agacaccccc tccacaccct ctttccccaa cctcgtgttg ttcggagcgc acacacacac 4800
   aaccagatct cccccaaatc cacccgtcgg cacctccgct tcaaggtacg ccgctcgtcc 4860
   tccccccccc ccctctctac cttctctaga tcggcgttcc ggtccatgca tggttagggc 4920
   ccggtagttc tacttctgtt catgtttgtg ttagatccgt gtttgtgtta gatccgtgct 4980
   gctagcgttc gtacacggat gcgacctgta cgtcagacac gttctgattg ctaacttgcc 5040
   agtgtttctc tttggggaat cctgggatgg ctctagccgt tccgcagacg ggatcgattt 5100
   catgattttt tttgtttcgt tgcatagggt ttggtttgcc cttttccttt atttcaatat 5160
   atgccgtgca cttgtttgtc gggtcatctt ttcatgcttt tttttgtctt ggttgtgatg 5220
   atgtggtctg gttgggcggt cgttctagat cggagtagaa ttctgtttca aactacctgg 5280
   tggatttatt aattttggat ctgtatgtgt gtgccataca tattcatagt tacgaattga 5340
   agatgatgga tggaaatatc gatctaggat aggtatacat gttgatgcgg gttttactga 5400
   tgcatataca gagatgcttt ttgttcgctt ggttgtgatg atgtggtgtg gttgggcggt 5460
   cgttcattcg ttctagatcg gagtagaata ctgtttcaaa ctacctggtg tatttattaa 5520
   ttttggaact gtatgtgtgt gtcatacatc ttcatagtta cgagtttaag atggatggaa 5580
   atatcgatct aggataggta tacatgttga tgtgggtttt actgatgcat atacatgatg 5640
   gcatatgcag catctattca tatgctctaa ccttgagtac ctatctatta taataaacaa 5700
   gtatgtttta taattatttt gatcttgata tacttggatg atggcatatg cagcagctat 5760
   atgtggattt ttttagccct gccttcatac gctatttatt tgcttggtac tgtttctttt 5820
   gtcgatgctc accctgttgt ttggtgttac ttctgcaggt cgactctaga ggatccatgg 5880
   ccactgtgaa caactggctc gctttctccc tctccccgca ggagctgccg ccctcccaga 5940
   cgacggactc cacactcatc tcggccgcca ccgccgacca tgtctccggc gatgtctgct 6000
   tcaacatccc ccaagattgg agcatgaggg gatcagagct ttcggcgctc gtcgcggagc 6060
   cgaagctgga ggacttcctc ggcggcatct ccttctccga gcagcatcac aaggccaact 6120
   gcaacatgat acccagcact agcagcacag tttgctacgc gagctcaggt gctagcaccg 6180
   gctaccatca ccagctgtac caccagccca ccagctcagc gctccacttc gcggactccg 6240
   taatggtggc ttcctcggcc ggtgtccacg acggcggtgc catgctcagc gcggccgccg 6300
   ctaacggtgt cgctggcgct gccagtgcca acggcggcgg catcgggctg tccatgatta 6360
   agaactggct gcggagccaa ccggcgccca tgcagccgag ggtggcggcg gctgagggcg 6420
   cgcaggggct ctctttgtcc atgaacatgg cggggacgac ccaaggcgct gctggcatgc 6480
   cacttctcgc tggagagcgc gcacgggcgc ccgagagtgt atcgacgtca gcacagggtg 6540
   gagccgtcgt cgtcacggcg ccgaaggagg atagcggtgg cagcggtgtt gccggcgctc 6600
   tagtagccgt gagcacggac acgggtggca gcggcggcgc gtcggctgac aacacggcaa 6660
   ggaagacggt ggacacgttc gggcagcgca cgtcgattta ccgtggcgtg acaaggcata 6720
   gatggactgg gagatatgag gcacatcttt gggataacag ttgcagaagg gaagggcaaa 6780
   ctcgtaaggg tcgtcaagtc tatttaggtg gctatgataa agaggagaaa gctgctaggg 6840
   cttatgatct tgctgctctg aagtactggg gtgccacaac aacaacaaat tttccagtga 6900
   gtaactacga aaaggagctc gaggacatga agcacatgac aaggcaggag tttgtagcgt 6960
   ctctgagaag gaagagcagt ggtttctcca gaggtgcatc catttacagg ggagtgacta 7020
   ggcatcacca acatggaaga tggcaagcac ggattggacg agttgcaggg aacaaggatc 7080
   tttacttggg caccttcagc acccaggagg aggcagcgga ggcgtacgac atcgcggcga 7140
   tcaagttccg cggcctcaac gccgtcacca acttcgacat gagccgctac gacgtgaaga 7200
   gcatcctgga cagcagcgcc ctccccatcg gcagcgccgc caagcgcctc aaggaggccg 7260
   aggccgcagc gtccgcgcag caccaccacg ccggcgtggt gagctacgac gtcggccgca 7320
   tcgcctcgca gctcggcgac ggcggagccc tggcggcggc gtacggcgcg cactaccacg 7380
   gcgccgcctg gccgaccatc gcgttccagc cgggcgccgc cagcacaggc ctgtaccacc 7440
   cgtacgcgca gcagccaatg cgcggcggcg ggtggtgcaa gcaggagcag gaccacgcgg 7500
   tgatcgcggc cgcgcacagc ctgcaggacc tccaccacct gaacctgggc gcggccggcg 7560
   cgcacgactt tttctcggca gggcagcagg ccgccgccgc tgcgatgcac ggcctgggta 7620
   gcatcgacag tgcgtcgctc gagcacagca ccggctccaa ctccgtcgtc tacaacggcg 7680
   gggtcggcga cagcaacggc gccagcgccg tcggcggcag tggcggtggc tacatgatgc 7740
   cgatgagcgc tgccggagca accactacat cggcaatggt gagccacgag caggtgcatg 7800
   cacgggccta cgacgaagcc aagcaggctg ctcagatggg gtacgagagc tacctggtga 7860
   acgcggagaa caatggtggc ggaaggatgt ctgcatgggg gactgtcgtg tctgcagccg 7920
   cggcggcagc agcaagcagc aacgacaaca tggccgccga cgtcgggcat ggcggcgcgc 7980
   agctcttcag tgtctggaac gacacttaag gcgtacgtgc cggcctggct ctccgaaagg 8040
   gcgtattcca gcacactggc ggccgttact agacccaacc tagacttgtc catcttctgg 8100
   attggccaac ttaattaatg tatgaaataa aaggatgcac acatagtgac atgctaatca 8160
   ctataatgtg ggcatcaaag ttgtgtgtta tgtgtaatta ctagttatct gaataaaaga 8220
   gaaagagatc atccatattt cttatcctaa atgaatgtca cgtgtcttta taattctttg 8280
   atgaaccaga tgcatttcat taaccaaatc catatacata taaatattaa tcatatataa 8340
   ttaatatcaa ttgggttagc aaaacaaatc tagtctaggt gtgttttgcg aatgcggcct 8400
   ccggattctt atgtgcttct agtctccaaa tgtggttgat agttattttg ctctaagatc 8460
   aacagtaatg aagtataaat catcgttgtg gtgtgctact cggttaattg agcattaaca 8520
   cacacaaaca tgacgaggat ggtataatct ccaaaaatgt gtactttgtt aggtgggacc 8580
   ctatagcctt gattaatgtg ctatgttagg catgcctgga aacgtgtgac gcatatgttt 8640
   tgtgaacctg ttgatattat atgtgctttt atattaccat attttattaa aatactaata 8700
   tttattacta gtaagatata acattctatc tagcttaaaa actaaccata aatattccat 8760
   aataactaga tttaccaaac taatatacta aatatacata ataaatacaa aattaacaag 8820
   acaataatca atatttatga gcttaatata tttagacatt atggttggtc gacgataatc 8880
   atgctaactt ttcgtaattg cttgattgaa atatgcttag aataatgcct ctttgttcta 8940
   catggcaaat agggaccatt atggtgtaac accctgggaa ccacaaacac cccgaaatgc 9000
   tactaaacta cacaactaac cttcatatat aaaatttcga cagcatctcc tttgaaaatt 9060
   tgcatagacg tggaagcaac agagtataaa cagatatcat gataagaaaa catactagac 9120
   attaataatc tgctagaaat gggaagaatc acgcgtaagc ttgcatgcct gcagtgcagc 9180
   gtgacccggt cgtgcccctc tctagagata atgagcattg catgtctaag ttataaaaaa 9240
   ttaccacata ttttttttgt cacacttgtt tgaagtgcag tttatctatc tttatacata 9300
   tatttaaact ttactctacg aataatataa tctatagtac tacaataata tcagtgtttt 9360
   agagaatcat ataaatgaac agttagacat ggtctaaagg acaattgagt attttgacaa 9420
   caggactcta cagttttatc tttttagtgt gcatgtgttc tccttttttt ttgcaaatag 9480
   cttcacctat ataatacttc atccatttta ttagtacatc catttagggt ttagggttaa 9540
   tggtttttat agactaattt ttttagtaca tctattttat tctattttag cctctaaatt 9600
   aagaaaacta aaactctatt ttagtttttt tatttaataa tttagatata aaatagaata 9660
   aaataaagtg actaaaaatt aaacaaatac cctttaagaa attaaaaaaa ctaaggaaac 9720
   atttttcttg tttcgagtag ataatgccag cctgttaaac gccgtcgacg agtctaacgg 9780
   acaccaacca gcgaaccagc agcgtcgcgt cgggccaagc gaagcagacg gcacggcatc 9840
   tctgtcgctg cctctggacc cctctcgaga gttccgctcc accgttggac ttgctccgct 9900
   gtcggcatcc agaaattgcg tggcggagcg gcagacgtga gccggcacgg caggcggcct 9960
   cctcctcctc tcacggcacc ggcagctacg ggggattcct ttcccaccgc tccttcgctt 10020
   tcccttcctc gcccgccgta ataaatagac accccctcca caccctcttt ccccaacctc 10080
   gtgttgttcg gagcgcacac acacacaacc agatctcccc caaatccacc cgtcggcacc 10140
   tccgcttcaa ggtacgccgc tcgtcctccc ccccccccct ctctaccttc tctagatcgg 10200
   cgttccggtc catgcatggt tagggcccgg tagttctact tctgttcatg tttgtgttag 10260
   atccgtgttt gtgttagatc cgtgctgcta gcgttcgtac acggatgcga cctgtacgtc 10320
   agacacgttc tgattgctaa cttgccagtg tttctctttg gggaatcctg ggatggctct 10380
   agccgttccg cagacgggat cgatttcatg attttttttg tttcgttgca tagggtttgg 10440
   tttgcccttt tcctttattt caatatatgc cgtgcacttg tttgtcgggt catcttttca 10500
   tgcttttttt tgtcttggtt gtgatgatgt ggtctggttg ggcggtcgtt ctagatcgga 10560
   gtagaattct gtttcaaact acctggtgga tttattaatt ttggatctgt atgtgtgtgc 10620
   catacatatt catagttacg aattgaagat gatggatgga aatatcgatc taggataggt 10680
   atacatgttg atgcgggttt tactgatgca tatacagaga tgctttttgt tcgcttggtt 10740
   gtgatgatgt ggtgtggttg ggcggtcgtt cattcgttct agatcggagt agaatactgt 10800
   ttcaaactac ctggtgtatt tattaatttt ggaactgtat gtgtgtgtca tacatcttca 10860
   tagttacgag tttaagatgg atggaaatat cgatctagga taggtataca tgttgatgtg 10920
   ggttttactg atgcatatac atgatggcat atgcagcatc tattcatatg ctctaacctt 10980
   gagtacctat ctattataat aaacaagtat gttttataat tattttgatc ttgatatact 11040
   tggatgatgg catatgcagc agctatatgt ggattttttt agccctgcct tcatacgcta 11100
   tttatttgct tggtactgtt tcttttgtcg atgctcaccc tgttgtttgg tgttacttct 11160
   gcaggtcgac tttaacttag cctaggatcc aacaatgccc cagttcgaca tcctctgcaa 11220
   gacccccccc aaggtgctcg tgaggcagtt cgtggagagg ttcgagaggc cctccggcga 11280
   gaagatcgcc ctctgcgccg ccgagctcac ctacctctgc tggatgatca cccacaacgg 11340
   caccgccatt aagagggcca ccttcatgtc atacaacacc atcatctcca actccctctc 11400
   cttcgacatc gtgaacaagt ccctccagtt caaatacaag acccagaagg ccaccatcct 11460
   cgaggcctcc ctcaagaagc tcatccccgc ctgggagttc accatcatcc cctactacgg 11520
   ccagaagcac cagtccgaca tcaccgacat cgtgtcatcc ctccagcttc agttcgagtc 11580
   ctccgaggag gctgacaagg gcaactccca ctccaagaag atgctgaagg ccctcctctc 11640
   cgagggcgag tccatctggg agatcaccga gaagatcctc aactccttcg agtacacctc 11700
   caggttcact aagaccaaga ccctctacca gttcctcttc ctcgccacct tcatcaactg 11760
   cggcaggttc tcagacatca agaacgtgga ccccaagtcc ttcaagctcg tgcagaacaa 11820
   gtacctaggt ttgtttctgc ttctaccttt gatatatata taataattat cattaattag 11880
   tagtaatata atatttcaaa tatttttttc aaaataaaag aatgtagtat atagcaattg 11940
   cttttctgta gtttataagt gtgtatattt taatttataa cttttctaat atatgaccaa 12000
   aacatggtga tgcctaggtg tcatcatcca gtgcctcgtg accgagacca agacctccgt 12060
   gtccaggcac atctacttct tctccgctcg cggcaggatc gaccccctcg tgtacctcga 12120
   cgagttcctc aggaactcag agcccgtgct caagagggtg aacaggaccg gcaactcctc 12180
   ctccaacaag caggagtacc agctcctcaa ggacaacctc gtgaggtcct acaacaaggc 12240
   cctcaagaag aacgccccct actccatctt cgccatcaag aacggcccca agtcccacat 12300
   cggtaggcac ctcatgacct ccttcctctc aatgaagggc ctcaccgagc tcaccaacgt 12360
   ggtgggcaac tggtccgaca agagggcctc cgccgtggcc aggaccacct acacccacca 12420
   gatcaccgcc atccccgacc actacttcgc cctcgtgtca aggtactacg cctacgaccc 12480
   catctccaag gagatgatcg ccctcaagga cgagactaac cccatcgagg agtggcagca 12540
   catcgagcag ctcaagggct ccgccgaggg ctccatcagg taccccgcct ggaacggcat 12600
   catctcccag gaggtgctcg actacctctc ctcctacatc aacaggagga tctgagttaa 12660
   cctagacttg tccatcttct ggattggcca acttaattaa tgtatgaaat aaaaggatgc 12720
   acacatagtg acatgctaat cactataatg tgggcatcaa agttgtgtgt tatgtgtaat 12780
   tactagttat ctgaataaaa gagaaagaga tcatccatat ttcttatcct aaatgaatgt 12840
   cacgtgtctt tataattctt tgatgaacca gatgcatttc attaaccaaa tccatataca 12900
   tataaatatt aatcatatat aattaatatc aattgggtta gcaaaacaaa tctagtctag 12960
   gtgtgttttg cggtcacacc ggttaaaacc aaaatccagt ggcgagctct cgagtcgatc 13020
   gctatcaact ttgtatagaa aagttgggcc gaattcgagc tcggtacggc cagaatggcc 13080
   cggaccgggt taccgaattc gagctcggta ccctgggatc agcttcgctg aaatcaccag 13140
   tctctctcta caaatctatc tctctctata ataatgtgtg agtagttccc agataaggga 13200
   attagggttc ttatagggtt tcgctcatgt gttgagcata taagaaaccc ttagtatgta 13260
   tttgtatttg taaaatactt ctatcaataa aatttctaat tcctaaaacc aaaatccagt 13320
   ggcgagctgc tagcgaagtt cctattccga agttcctatt ctctagaaag tataggaact 13380
   tcagatccac cgggatcccc gatcatgcaa aaactcatta actcagtgca aaactatgcc 13440
   tggggcagca aaacggcgtt gactgaactt tatggtatgg aaaatccgtc cagccagccg 13500
   atggccgagc tgtggatggg cgcacatccg aaaagcagtt cacgagtgca gaatgccgcc 13560
   ggagatatcg tttcactgcg tgatgtgatt gagagtgata aatcgactct gctcggagag 13620
   gccgttgcca aacgctttgg cgaactgcct ttcctgttca aagtattatg cgcagcacag 13680
   ccactctcca ttcaggttca tccaaacaaa cacaattctg aaatcggttt tgccaaagaa 13740
   aatgccgcag gtatcccgat ggatgccgcc gagcgtaact ataaagatcc taaccacaag 13800
   ccggagctgg tttttgcgct gacgcctttc cttgcgatga acgcgtttcg tgaattttcc 13860
   gagattgtct ccctactcca gccggtcgca ggtgcacatc cggcgattgc tcacttttta 13920
   caacagcctg atgccgaacg tttaagcgaa ctgttcgcca gcctgttgaa tatgcagggt 13980
   gaagaaaaat cccgcgcgct ggcgatttta aaatcggccc tcgatagcca gcagggtgaa 14040
   ccgtggcaaa cgattcgttt aatttctgaa ttttacccgg aagacagcgg tctgttctcc 14100
   ccgctattgc tgaatgtggt gaaattgaac cctggcgaag cgatgttcct gttcgctgaa 14160
   acaccgcacg cttacctgca aggcgtggcg ctggaagtga tggcaaactc cgataacgtg 14220
   ctgcgtgcgg gtctgacgcc taaatacatt gatattccgg aactggttgc caatgtgaaa 14280
   ttcgaagcca aaccggctaa ccagttgttg acccagccgg tgaaacaagg tgcagaactg 14340
   gacttcccga ttccagtgga tgattttgcc ttctcgctgc atgaccttag tgataaagaa 14400
   accaccatta gccagcagag tgccgccatt ttgttctgcg tcgaaggcga tgcaacgttg 14460
   tggaaaggtt ctcagcagtt acagcttaaa ccgggtgaat cagcgtttat tgccgccaac 14520
   gaatcaccgg tgactgtcaa aggccacggc cgtttagcgc gtgtttacaa caagctgtaa 14580
   gagcttactg aaaaaattaa catctcttgc taagctgggg gtggaaccta gacttgtcca 14640
   tcttctggat tggccaactt aattaatgta tgaaataaaa ggatgcacac atagtgacat 14700
   gctaatcact ataatgtggg catcaaagtt gtgtgttatg tgtaattact agttatctga 14760
   ataaaagaga aagagatcat ccatatttct tatcctaaat gaatgtcacg tgtctttata 14820
   attctttgat gaaccagatg catttcatta accaaatcca tatacatata aatattaatc 14880
   atatataatt aatatcaatt gggttagcaa aacaaatcta gtctaggtgt gttttgcgaa 14940
   tgcgaccttc ttatgtgctt ctagtctcca aatgtggttg atagttattt tgctctaaga 15000
   tcaacagtaa tgaagtataa atcatcgttg tggtgtgcta ctcggttaat tgagcattaa 15060
   cacacacaaa catgacgagg atggtataat ctccaaaaat gtgtactttg ttaggtggga 15120
   ccctatagcc ttgattaatg tgctatgtta ggcatgcctg gaaacgtgtg acgcatatgt 15180
   tttgtgaacc tgttgatatt atatgtgctt ttatattacc atattttatt aaaatactaa 15240
   tatttattac tagtaagata taacattcta tctagcttaa aaactaacca taaatattcc 15300
   ataataacta gatttaccaa actaatatac taaatataca taataaatac aaaattaaca 15360
   agacaataat caatatttat gagcttaata tatttagaca ttatggttgg tcgacgataa 15420
   tcatgctaac ttttcgtaat tgcttgattg aaatatgctt agaataatgc ctctttgttc 15480
   tacatggcaa atagggacca ttatggtgta acaccctggg aaccacaaac accccgaaat 15540
   gctactaaac tacacaacta accttcatat ataaaatttc gacagcatct cctttgaaaa 15600
   tttgcataga cgtggaagca acagagtata aacagatatc atgataagaa aacatactag 15660
   acattaataa tctgctagaa atgggaagaa tcctaacttg acgactgcgt aactgactag 15720
   agtcacactt agctgaccct agtcacttac aactgacttc gtgtcctagg cttaggctac 15780
   tgctagtccg cggtgtatcc gtgatcgagt tggcgccaga cggaatctgt tctccatcgc 15840
   tgacatcctc gagtagatca cattcaagct tgatatcgaa ttcctgcagc ccatccctca 15900
   gccgcctttc actatctttt ttgcccgagt cattgtcatg tgaaccttgg catgtataat 15960
   cggtgaattg cgtcgatttt cctcttatag gtgggccaat gaatccgtgt gatcgcgtct 16020
   gattggctag agatatgttt cttccttgtt ggatgtattt tcatacataa tcatatgcat 16080
   acaaatattt cattacactt tatagaaatg gtcagtaata aaccctatca ctatgtctgg 16140
   tgtttcattt tatttgcttt taaacgaaaa ttgacttcct gattcaatat ttaaggatcg 16200
   tcaacggtgt gcagttacta aattctggtt tgtaggaact atagtaaact attcaagtct 16260
   tcacttattg tgcactcacc tctcgccaca tcaccacaga tgttattcac gtcttaaatt 16320
   tgaactacac atcatattga cacaatattt tttttaaata agcgattaaa acctagcctc 16380
   tatgtcaaca atggtgtaca taaccagcga agtttaggga gtaaaaaaca tcgccttaca 16440
   caaagttcgc tttaaaaaat aaagagtaaa ttttactttg gaccaccctt caaccaatgt 16500
   ttcactttag aacgagtaat tttattattg tcactttgga ccaccctcaa atcttttttc 16560
   catctacatc caatttatca tgtcaaagaa atggtctaca tacagctaag gagatttatc 16620
   gacgaatagt agctagcata ctcgaggtca ttcatatgct tgagaagaga gtcgggatag 16680
   tccaaaataa aacaaaggta agattacctg gtcaaaagtg aaaacatcag ttaaaaggtg 16740
   gtataaagta aaatatcggt aataaaaggt ggcccaaagt gaaatttact cttttctact 16800
   attataaaaa ttgaggatgt ttttgtcggt actttgatac gtcatttttg tatgaattgg 16860
   tttttaagtt tattcgcttt tggaaatgca tatctgtatt tgagtcgggt tttaagttcg 16920
   tttgcttttg taaatacaga gggatttgta taagaaatat ctttaaaaaa acccatatgc 16980
   taatttgaca taatttttga gaaaaatata tattcaggcg aattctcaca atgaacaata 17040
   ataagattaa aatagctttc ccccgttgca gcgcatgggt attttttcta gtaaaaataa 17100
   aagataaact tagactcaaa acatttacaa aaacaacccc taaagttcct aaagcccaaa 17160
   gtgctatcca cgatccatag caagcccagc ccaacccaac ccaacccaac ccaccccagt 17220
   ccagccaact ggacaatagt ctccacaccc ccccactatc accgtgagtt gtccgcacgc 17280
   accgcacgtc tcgcagccaa aaaaaaaaaa agaaagaaaa aaaagaaaaa gaaaaaacag 17340
   caggtgggtc cgggtcgtgg gggccggaaa cgcgaggagg atcgcgagcc agcgacgagg 17400
   ccggccctcc ctccgcttcc aaagaaacgc cccccatcgc cactatatac ataccccccc 17460
   ctctcctccc atccccccaa ccctaccacc accaccacca ccacctccac ctcctccccc 17520
   ctcgctgccg gacgacgagc tcctcccccc tccccctccg ccgccgccgc gccggtaacc 17580
   accccgcccc tctcctcttt ctttctccgt tttttttttc cgtcacggtc tcgatctttg 17640
   gccttggtag tttgggtggg cgagaggcgg cttcgtgcgc gcccagatcg gtgcgcggga 17700
   ggggcgggat ctcgcggctg gggctctcgc cggcgtggat caggcccgga tctcgcgggg 17760
   aatggggctc tcggatgtag atctgcgatc cgccgttgtt gggggagatg atggggggtt 17820
   taaaatttcc gccatgctaa acaagatcag gaagagggga aaagggcact atggtttata 17880
   tttttatata tttctgctgc ttcgtcaggc ttagatgtgc tagatctttc tttcttcttt 17940
   ttgtgggtag aatttgaatc cctcagcatt gttcatcggt agtttttctt ttcatgattt 18000
   gtgacaaatg cagcctcgtg cggagctttt ttgtaggtag aaggatccac acgacaccat 18060
   gtcccccgag cgccgccccg tcgagatccg cccggccacc gccgccgaca tggccgccgt 18120
   gtgcgacatc gtgaaccact acatcgagac ctccaccgtg aacttccgca ccgagccgca 18180
   gaccccgcag gagtggatcg acgacctgga gcgcctccag gaccgctacc cgtggctcgt 18240
   ggccgaggtg gagggcgtgg tggccggcat cgcctacgcc ggcccgtgga aggcccgcaa 18300
   cgcctacgac tggaccgtgg agtccaccgt gtacgtgtcc caccgccacc agcgcctcgg 18360
   cctcggctcc accctctaca cccacctcct caagagcatg gaggcccagg gcttcaagtc 18420
   cgtggtggcc gtgatcggcc tcccgaacga cccgtccgtg cgcctccacg aggccctcgg 18480
   ctacaccgcc cgcggcaccc tgcgcgccgc cggctacaag cacggcggct ggcacgacgt 18540
   cggcttctgg cagcgcgact tcgagctgcc ggccccgccg cgcccggtgc gcccggtgac 18600
   gcagatctga gtcgacctgc aggcatgccg ctgaaatcac cagtctctct ctacaaatct 18660
   atctctctct ataataatgt gtgagtagtt cccagataag ggaattaggg ttcttatagg 18720
   gtttcgctca tgtgttgagc atataagaaa cccttagtat gtatttgtat ttgtaaaata 18780
   cttctatcaa taaaatttct aattcctaaa accaaaatcc agtggcgagc taatgcggcc 18840
   cgaataactt cgtatagcat acattatacg aagttatacc tggtggcgcc gctaggggct 18900
   gcaggaattc ctgcagcccg ggggatccac tagttctaga gcggccgacc tcgacagatc 18960
   taagcttact agtgccgtgg gtcgtttaag ctgccgctgt acctgtgtcg tctggtgcct 19020
   tctggtgtac ctgggaggtt gtcgtctatc aagtatctgt ggttggtgtc atgagtcagt 19080
   gagtcccaat actgttcgtg tcctgtgtgc attataccca aaactgttat gggcaaatca 19140
   tgaataagct tgatgttcga acttaaaagt ctctgctcaa tatggtatta tggttgtttt 19200
   tgttcgtctc ctaatatttg cctgggatca aattttattg gctggtgttc atttgacctc 19260
   catgttcttg ctaggctcca ttttttactc tacagccata atatgtttga ttgtttggtt 19320
   tgttctttgt tgtacacctg gttctgtcga gcttagtttt cgacactggc ttacagctta 19380
   acatgttgct attttattgg gttctgattg ctattttatt gggttctgat tgctagtttt 19440
   tgctgaatcc aaaaaccatg ttatttattt aagcgatcca ggttattatt atgatggtgg 19500
   ctaagttttt ttttttccaa gggtaaattt tctggattct ccagtgtttc tgtggccgaa 19560
   ttcactagtg attcagatct gatatcgatg ggcccactaa ctatctatac tgtaataatg 19620
   ttgtatagcc gccggatagc tagctagttt agtcattcag cggcgatggg taataataaa 19680
   gtgtcatcca tccatcacca tgggtggcaa cgtgagcaat gacctgattg aacaaattga 19740
   aatgaaaaga agaaatatgt tatatgtcaa cgagatttcc tcataatgcc actgacgacg 19800
   tgtgtccaag aaatgtatca gtgatacgta tattcacaat ttttttatga cttatactca 19860
   caatttgttt ttttactact tatactcaca atttgttgtg ggtaccataa caatttcgat 19920
   cgaatatata tcagaaagtt gacgaaagta agctcactca aaaagttaaa tgggctgcgg 19980
   aagctgcgtc aggcccaagt tttggctatt ctatccggta tccacgattt tgatggctga 20040
   gggacatatg ttcgcttaag cttggtcacc cggtccgggc ctagaaggcc agcttcaagt 20100
   ttgtacaaaa aagcaggctc cggccagaat ggcccggacc gggtgaccgg cgcgccaagc 20160
   ttggtgacgg cgacgcgatc gaacaggtgg tgatcgatgc tgcaacgtgt gtaaatatac 20220
   agcgccggct gggtcaagag atggctcggg tgacgcgcgc gcggcgtgtc ctggcgttgg 20280
   cgccggggca ttctttagtt tttcatcttt tcatcatctc agatggtaga tacaaaacag 20340
   tgtatgtatg tagctctgtt tctctctata gaaccccaac aaattttgtt gttgatgttg 20400
   tttatcttca tatgctttga tcttgaaatc gtctacctta ctactgccga tcgttgctca 20460
   aaagtgtgga agtttgaagc atcttccacg ggcgttgctc tactcccatc tcgtttcacc 20520
   acgaaatccc cttcgcatag aggcatctca gccgtacatc tccaaaccca cgttctggat 20580
   tcagcaaacg gacggcatat gccagcggac tgactaggat ttgtggttca gaataaaaat 20640
   atggtttgca gtgtcaattt tccaggagag tgactatgtc atactaactt catactccaa 20700
   taatgaagct aagctatctc catgtacata tcaaatacga aatcatatcc aagggaacta 20760
   acacagtcac aaacaacagg tacacagaca attacagcac aagcgcagga gggaaataat 20820
   tttaactgaa ctaggaagaa aggaaacaca actcattttt tattgatata tgttggatga 20880
   atccaataaa accgatacaa gtcacgaaaa atcagactag atgaatcctt cgagatcaca 20940
   tgagcaaaac ctttcgacga aagctgtcct atagtcgtgg aagcaataac acttgataaa 21000
   gataggaatt cagacacgag aggttgcaga ctataagatg tcaggtacct acgcgttcga 21060
   atcaatgaga tgatgcagta gaggtagagc gaagagggaa ggctgagatc gacgcatcac 21120
   tgccatgtcc ctgatgatga tgaggtagca atggagatct acagagcggg caattgaagt 21180
   tgagatgttg aagccagcct tccaaacact gcttatggaa gacgtgtctg caatctagct 21240
   tcctcacttc ttctccggtc ttgagtttag acagacacac gatgcagtca gaggctgcgt 21300
   tgtcggagta gcggtacgag aagaggcgat ttaggttgag ctggtcggcg aggacgctga 21360
   ggtttgaagt aacaacaaca acgggggcag aagaagggaa gaggagaaga gaccggacgt 21420
   gtctgaagaa agttgcgagg agagccagta gcatcagtgg gatcgaatct gatgacacgt 21480
   cggagagctg accttgtagt cccatggatc ctaggctaag ttaaagtcga cctgcagaag 21540
   taacaccaaa caacagggtg agcatcgaca aaagaaacag taccaagcaa ataaatagcg 21600
   tatgaaggca gggctaaaaa aatccacata tagctgctgc atatgccatc atccaagtat 21660
   atcaagatca aaataattat aaaacatact tgtttattat aatagatagg tactcaaggt 21720
   tagagcatat gaatagatgc tgcatatgcc atcatgtata tgcatcagta aaacccacat 21780
   caacatgtat acctatccta gatcgatatt tccatccatc ttaaactcgt aactatgaag 21840
   atgtatgaca cacacataca gttccaaaat taataaatac accaggtagt ttgaaacagt 21900
   attctactcc gatctagaac gaatgaacga ccgcccaacc acaccacatc atcacaacca 21960
   agcgaacaaa aagcatctct gtatatgcat cagtaaaacc cgcatcaaca tgtataccta 22020
   tcctagatcg atatttccat ccatcatctt caattcgtaa ctatgaatat gtatggcaca 22080
   cacatacaga tccaaaatta ataaatccac caggtagttt gaaacagaat tctactccga 22140
   tctagaacga ccgcccaacc agaccacatc atcacaacca agacaaaaaa aagcatgaaa 22200
   agatgacccg acaaacaagt gcacggcata tattgaaata aaggaaaagg gcaaaccaaa 22260
   ccctatgcaa cgaaacaaaa aaaatcatga aatcgatccc gtctgcggaa cggctagagc 22320
   catcccagga ttccccaaag agaaacactg gcaagttagc aatcagaacg tgtctgacgt 22380
   acaggtcgca tccgtgtacg aacgctagca gcacggatct aacacaaaca cggatctaac 22440
   acaaacatga acagaagtag aactaccggg ccctaaccat gcatggaccg gaacgccgat 22500
   ctagagaagg tagagagggg ggggggggag gacgagcggc gtaccttgaa gcggaggtgc 22560
   cgacgggtgg atttggggga gatcctcgct ttcctggagg aggcgtggag cgcacgcggt 22620
   tggaggcgaa ggcggatgaa aaacgagagg ggctgacggg ctgtcttctg tctggcttgc 22680
   gcccctccga cgggttggtt ggctttatag gcgtgtggtg catataatta ggtgcgcgaa 22740
   ccgccgttcc gatggaaaaa actggctatg gaggctctcg attcgtgccg gcctatcgag 22800
   acggagcttt ttttattatt tattttcatc tcgcatgtat gttgtttgtt cgctgatgga 22860
   atttctgttt gttcgcgctg aaaacattgg acagggttcg tttatgctgt gcccagtgag 22920
   gaaccataaa aggaacgctg tgcatatgga gtcacgtact tagtaaaaaa tccttcgctc 22980
   tggattaaac taggttgatt atactgttgc acgtagtatc tatagagaca aaaatattat 23040
   aaaaatagta gaaaccgata caaattaaag ttaagtaaac atgatgttta tctgatactc 23100
   taagtcaagc aagtcatttc tagtgttttc ttctgtgcta ctagcagagc aagagattgg 23160
   gacccgaggc gggggccatg cagacatagg gggatgctcc ttaggagaga gataaaaata 23220
   aataatagaa tatatgatta ttggtataga gtacagattt aaggtaaata ttgcttcaaa 23280
   agattgtgga atagagtaga gaatctcgtt gacaaaaatg aaatattact tttagaataa 23340
   aaatttagag ttgcataagt atagatatca ttagtttgga ttgagggcta gagctattcc 23400
   agctatttag gctctgttta taatcctaca gccaataatt accagaagct tcggtccggg 23460
   cctagaaggc cgatctcccg ggcacccagc tttcttgtac aaagtggccg ttaacggatc 23520
   ggccagaatg gcccggaccg ggttaccgaa ttgctaacta actaggagct ccctttaatc 23580
   tggcgcttga tctgcatccg cggcttgcaa agataaatgg cacatttagt gtgttatttt 23640
   gcaatacctt tcatagtaga tatccttaaa tgcagtttta ggcatgtttg ggtaattaaa 23700
   taacattttt aggaggagtt ttagatttac ctttctttcg tgatgactga tgacagacgt 23760
   ggggaattca aatgcaactc tagcgaaagt tcatatattt ttcataaata gctgaggctg 23820
   gggtaattat tttttgtaga aaaatagaat aggtggaatg gtttggggaa ggcgtaggcg 23880
   ctcgtggacg acgccccata aaagacaaga ggcggaattg ccatgaattc gaggtagcta 23940
   agtaaggcgc atatatatgc caaaaattct actgtcactt tccaatttca atgcgctgcc 24000
   aaacaagccg ccgacccttg gatactgact tgaattcagc ccaattctgt agatccaaac 24060
   agggccggcg tcagtgcctc aggtgagaga gcagcagacg atgcaaagag ccaaaagtgg 24120
   aagcagacgc agccgaagcc gaagcccaag cccaaaactg ttttgtcttt gcccagaacc 24180
   gcgacgagcc taaactgccg cttcctccta tctacaagtc cctggcacat cacgcatagt 24240
   ccaaccatcg cgcgcaggcg ataaggcgcg ccacggggac gcgacatgtg gtggcggacg 24300
   cgatcaggat agggccaggc tggccgggcg cggccacggg agaacggtgg ccactcgtcc 24360
   cacatccgct tcgtcctgtc ctgtactgcg tcctgccccc aacgagagcc ggagccggcc 24420
   atcccgtcgc acactctccc cctctatata tgccgtcggt gtgggggagc ctactacagg 24480
   acgacccaag caagcaagca agcagcgagt acatacatac taggcagcca ggcagccatg 24540
   atgctgcact gcacatttgc tatatctgag gctcctgcgc gcgccttggc ccttggccag 24600
   gtgtctgtca tgcgggcgat gccgcaggaa gaagaagccg cggtggctac gacgaccatg 24660
   gccgggggca aggtggcggc gctgctggcc acggcggccg cgctgctgct gctgctcccg 24720
   ctggcgctgc cgccgctgcc gccgccgccc acgcagctgt tgttcgtccc cgtggtcttg 24780
   ctgctcctcg tggcgtccct cgcgttctgc cccgccgcga cctcctcgcc gtcgccgatg 24840
   catgccgccg accacgggtc gttcgggacc actggatcac cgcacctatg ttgaggtacc 24900
   cggggatcct ctagtgccgt gggtcgttta agctgccgct gtacctgtgt cgtctggtgc 24960
   cttctggtgt acctgggagg ttgtcgtcta tcaagtatct gtggttggtg tcatgagtca 25020
   gtgagtccca atactgttcg tgtcctgtgt gcattatacc caaaactgtt atgggcaaat 25080
   catgaataag cttgatgttc gaacttaaaa gtctctgctc aatatggtat tatggttgtt 25140
   tttgttcgtc tcctaatatt tgcctgggat caaattttat tggctggtgt tcatttgacc 25200
   tccatgttct tgctaggctc cattttttac tctacagcca taatatgttt gattgtttgg 25260
   tttgttcttt gttgtacacc tggttctgtc gagcttagtt ttcgacactg gcttacagct 25320
   taacatgttg ctattttatt gggttctgat tgctatttta ttgggttctg attgctagtt 25380
   tttgctgaat ccaaaaacca tgttatttat ttaagcgatc caggttatta ttatgatggt 25440
   ggctaagttt ttttttttcc aagggtaaat tttctggatt ctccagtgtt tctgtggccg 25500
   aattcaatca ctagagtcga cctgcaggca tgcccgcggt ggagctcgaa ttccggtccg 25560
   ggcctagaag gccagcttcg gccgccccgg gcaactttat tatacaaagt tgatagatcg 25620
   agcgactaat taactagctg tcccacggcc taactagcac ttaatcccct agcctaacct 25680
   aagagcgcta atctaggcta gtggtcactt agggctttaa ggctagcgta tacgaagttc 25740
   ctattccgaa gttcctattc tccagaaagt ataggaactt ctgtacacct gagcctaact 25800
   aactaggacg tcccgaggtc cgattccggg ctaattaaca cctagctcgc cactcgacta 25860
   attagggact aagcctagcg cttagccgtt aaaacctagc acaccctaag cacccttagt 25920
   taggttcccc tcttaattaa gccctagtga gcccctaagt taaggggacg ctaagagccc 25980
   cctaacctag tattcggcta gaggcgaact aggctaaaca cctaagcgca cctctttaag 26040
   ctagatcgct agggggctag gctagagctt cgctagatta gtctaagggc agctaactaa 26100
   ctaggtttaa actacattaa aaacgtccgc aatgtgttat taagttgtct aagcgtcaat 26160
   ttgtttacac cacaatatat cctgcca 26187
<210> 75
   <211> 9982
   <212> DNA
   <213> Artificial sequence
<220>
   <223> Synthetic construct
<400> 75
<210> 76
   <211> 10955
   <212> DNA
   <213> Artificial sequence
<220>
   <223> Synthetic construct
<400> 76
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgt tgaaggagcc actcagcaag 180
   ctggtacgat tgtaatacga ctcactatag ggcgaattga gcgctgttta aacgctcttc 240
   aactggaaga gcggttacca gagctggtca cctttgtcca ccaagatgga actgcggccg 300
   taagtgacta gggtcacgtg accctagtca cttatcgagc tagttaccct atgaggtgac 360
   atgaagcgct cacggttact atgacggtta gcttcacgac tgttggtggc agtagcgtac 420
   gacttagcta tagttccgga cttaccgggc ccaccggtgg taccgagctc gtttaaacgc 480
   tcttcaactg gaagagcggt taccagagct ggtcaccttt gtccaccaag atggaactgg 540
   cgcgcctcat taattaagtc agcggccgct ctagttgaag acacgttcat gtcttcatcg 600
   taagaagaca ctcagtagtc ttcggccaga atggccatct ggattcagca ggcctagaag 660
   gccatttaaa tcctgaggat ctggtcttcc taaggacccg ggatatcgga ccgattaaac 720
   tttaattcgg tccgacctgg tggcgccgct agcgtatacg aagttcctat tccgaagttc 780
   ctattctcca gaaagtatag gaacttctgt acaataactt cgtatagcat acattatacg 840
   aagttatatg catacgcgtc ttaagtcgat cgctatcaac tttgtataga aaagttgggc 900
   cgaattcgag ctcggtacgg ccagaatggc ccggaccggg ttaccgaatt cgagctcggt 960
   accctgggat ccgatatcga tgggccctgg ccgaagcttg gtcacccggt ccgggcctag 1020
   aaggccagct tcaagtttgt acaaaaaagc aggctccggc cagaatggcc cggaccggaa 1080
   ttcgagctcg gatccactag taacggccgc cagtgtgctg gaattcgccc ttgacggccc 1140
   gggctggtat ttcaaaacta tagtatttta aaattgcatt aacaaacatg tcctaattgg 1200
   tactcctgag atactatacc ctcctgtttt aaaatagttg gcattatcga attatcattt 1260
   tactttttaa tgttttctct tcttttaata tattttatga attttaatgt attttaaaat 1320
   gttatgcagt tcgctctgga cttttctgct gcgcctacac ttgggtgtac tgggcctaaa 1380
   ttcagcctga ccgaccgcct gcattgaata atggatgagc accggtaaaa tccgcgtacc 1440
   caactttcga gaagaaccga gacgtggcgg gccgggccac cgacgcacgg caccagcgac 1500
   tgcacacgtc ccgccggcgt acgtgtacgt gctgttccct cactggccgc ccaatccact 1560
   catgcatgcc cacgtacacc cctgccgtgg cgcgcccaga tcctaatcct ttcgccgttc 1620
   tgcacttctg ctgcctataa atggcggcat cgaccgtcac ctgcttcacc accggcgagc 1680
   cacatcgaga acacgatcga gcacacaagc acgaagactc gtttaggaga aaccacaaac 1740
   caccaagccg tgcaagcacc aagcttggtc acccggtccg ggcctagaag gccagcttca 1800
   agtttgtaca aaaaagcagg cttcgaagga gatagaaccg atccaccatg tccaacctgc 1860
   tcacggttca ccagaacctt ccggctcttc cagtggacgc gacgtccgat gaagtcagga 1920
   agaacctcat ggacatgttc cgcgacaggc aagcgttcag cgagcacacc tggaagatgc 1980
   tgctctccgt ctgccgctcc tgggctgcat ggtgcaagct gaacaacagg aagtggttcc 2040
   ccgctgagcc cgaggacgtg agggattacc ttctgtacct gcaagcgcga ggtttgtttc 2100
   tgcttctacc tttgatatat atataataat tatcattaat tagtagtaat ataatatttc 2160
   aaatattttt ttcaaaataa aagaatgtag tatatagcaa ttgcttttct gtagtttata 2220
   agtgtgtata ttttaattta taacttttct aatatatgac caaaacatgg tgatgcctag 2280
   gtctggcagt gaagaccatc cagcaacacc ttggacaact gaacatgctt cacaggcgct 2340
   ccggcctccc gcgccccagc gactcgaacg ccgtgagcct cgtcatgcgc cgcatcagga 2400
   aggaaaacgt cgatgccggc gaaagggcaa agcaggccct cgcgttcgag aggaccgatt 2460
   tcgaccaggt ccgcagcctg atggagaaca gcgacaggtg ccaggacatt aggaacctgg 2520
   cgttcctcgg aattgcatac aacacgctcc tcaggatcgc ggaaattgcc cgcattcgcg 2580
   tgaaggacat tagccgcacc gacggcggca ggatgcttat ccacattggc aggaccaaga 2640
   cgctcgtttc caccgcaggc gtcgaaaagg ccctcagcct cggagtgacc aagctcgtcg 2700
   aacgctggat ctccgtgtcc ggcgtcgcgg acgacccaaa caactacctc ttctgccgcg 2760
   tccgcaagaa cggggtggct gcccctagcg ccaccagcca actcagcacg agggccttgg 2820
   aaggtatttt cgaggccacc caccgcctga tctacggcgc gaaggatgac agcggtcaac 2880
   gctacctcgc atggtccggg cactccgccc gcgttggagc tgctagggac atggcccgcg 2940
   ccggtgtttc catccccgaa atcatgcagg cgggtggatg gacgaacgtg aacattgtca 3000
   tgaactacat tcgcaacctt gacagcgaga cgggcgcaat ggttcgcctc ctggaagatg 3060
   gtgactgagc tagacccagc tttcttgtac aaagtggccg ttaacggatc cagacttgtc 3120
   catcttctgg attggccaac ttaattaatg tatgaaataa aaggatgcac acatagtgac 3180
   atgctaatca ctataatgtg ggcatcaaag ttgtgtgtta tgtgtaatta ctagttatct 3240
   gaataaaaga gaaagagatc atccatattt cttatcctaa atgaatgtca cgtgtcttta 3300
   taattctttg atgaaccaga tgcatttcat taaccaaatc catatacata taaatattaa 3360
   tcatatataa ttaatatcaa ttgggttagc aaaacaaatc tagtctaggt gtgttttgcg 3420
   aattgcggca agcttgcggc cgccccagct tggtcacccg gtccgggcct agaaggccga 3480
   tctcccgggc acccagcttt cttgtacaaa gtggccgtta acggatcggc cagaatggcc 3540
   cggaccgggt taccgaattc gagctcggta ccctgggatc gaccgaagct gaccgaagct 3600
   tgcggccgca cactgatagt ttaaactgaa ggcgggaaac gacaatctga tcatgagcgg 3660
   agaattaagg gagtcacgtt atgacccccg ccgatgacgc gggacaagcc gttttacgtt 3720
   tggaactgac agaaccgcaa cgattgaagg agccactcag ccgcgggttt ctggagttta 3780
   atgagctaag cacatacgtc agaaaccatt attgcgcgtt caaaagtcgc ctaaggtcac 3840
   tatcagctag caaatatttc ttgtcaaaaa tgctccactg acgttccata aattcccctc 3900
   ggtatccaat tagagtctca tattcactct cccgggggat ctcgactcta gaggatcgct 3960
   caggaaggcc gctgagatag aggcatggcg gccaatgcgg gcggcggtgg agcgggagga 4020
   ggcagcggca gcggcagcgt ggctgcgccg gcggtgtgcc gccccagcgg ctcgcggtgg 4080
   acgccgacgc cggagcagat caggatgctg aaggagctct actacggctg cggcatccgg 4140
   tcgcccagct cggagcagat ccagcgcatc accgccatgc tgcggcagca cggcaagatc 4200
   gagggcaaga acgtcttcta ctggttccag aaccacaagg cccgcgagcg ccagaagcgc 4260
   cgcctcacca gcctcgacgt caacgtgccc gccgccggcg cggccgacgc caccaccagc 4320
   caactcggcg tcctctcgct gtcgtcgccg ccgccttcag gcgcggcgcc tccctcgccc 4380
   accctcggct tctacgccgc cggcaatggc ggcggatcgg ctgtgctgct ggacacgagt 4440
   tccgactggg gcagcagcgg cgctgccatg gccaccgaga catgcttcct gcaggactac 4500
   atgggcgtga cggacacggg cagctcgtcg cagtggccac gcttctcgtc gtcggacacg 4560
   ataatggcgg cggccgcggc gcgggcggcg acgacgcggg cgcccgagac gctccctctc 4620
   ttcccgacct gcggcgacga cggcggcagc ggtagcagca gctacttgcc gttctggggt 4680
   gccgcgtcca caactgccgg cgccacttct tccgttgcga tccagcagca acaccagctg 4740
   caggagcagt acagctttta cagcaacagc aacagcaccc agctggccgg caccggcaac 4800
   caagacgtat cggcaacagc agcagcagcc gccgccctgg agctgagcct cagctcatgg 4860
   tgctcccctt accctgctgc agggagtatg tgagagcaac gcgagctgcc actgctcttc 4920
   actgatgtct ctggaatgga aggaggagga agtgagcata gcgttggtgc gttgctgtca 4980
   agggcgaatt gtaccacatg gttaacctag acttgtccat cttctggatt ggccaactta 5040
   attaatgtat gaaataaaag gatgcacaca tagtgacatg ctaatcacta taatgtgggc 5100
   atcaaagttg tgtgttatgt gtaattacta gttatctgaa taaaagagaa agagatcatc 5160
   catatttctt atcctaaatg aatgtcacgt gtctttataa ttctttgatg aaccagatgc 5220
   atttcattaa ccaaatccat atacatataa atattaatca tatataatta atatcaattg 5280
   ggttagcaaa acaaatctag tctaggtgtg ttttgcgaat gcggccgcga ctctagatca 5340
   taatcagcca taccacattc gaatgtgagt tgatccccgg cggtgtcccc cactgaagaa 5400
   actatgtgct gtagtatagc cgctggctag ctagctagtt gagtcattta gcggcgatga 5460
   ttgagtaata atgtgtcacg catcaccatg catgggtggc agtctcagtg tgagcaatga 5520
   cctgaatgaa caattgaaat gaaaagaaaa aagtattgtt ccaaattaaa cgttttaacc 5580
   ttttaatagg tttatacaat aattgatata tgttttctgt atatgtctaa tttgttatca 5640
   tccatttaga tatagacgaa aaaaaatcta agaactaaaa caaatgctaa tttgaaatga 5700
   agggagtata tattgggata atgtcgatga gatccctcgt aatatcaccg acatcacacg 5760
   tgtccagtta atgtatcagt gatacgtgta ttcacatttg ttgcgcgtag gcgtacccaa 5820
   caattttgat cgactatcag aaagtcaacg gaagcgagtc gacctcgagg gggggcccgg 5880
   taccaagata tcaaccgcgg aaagatctaa gcatgcaagg gcccaagtcg acctgcagaa 5940
   gcttcggtcc gggcctagaa ggccgatctc ccgggcaccc agctttcttg tacaaagtgg 6000
   ccgttaacgg atcggccaga atggcccgga ccgggttacc gaattcgagc tcggtaccct 6060
   gggatcgacc gaagctgacc gaagcttgca tgcctgcagt gcagcgtgac ccggtcgtgc 6120
   ccctctctag agataatgag cattgcatgt ctaagttata aaaaattacc acatattttt 6180
   tttgtcacac ttgtttgaag tgcagtttat ctatctttat acatatattt aaactttact 6240
   ctacgaataa tataatctat agtactacaa taatatcagt gttttagaga atcatataaa 6300
   tgaacagtta gacatggtct aaaggacaat tgagtatttt gacaacagga ctctacagtt 6360
   ttatcttttt agtgtgcatg tgttctcctt tttttttgca aatagcttca cctatataat 6420
   acttcatcca ttttattagt acatccattt agggtttagg gttaatggtt tttatagact 6480
   aattttttta gtacatctat tttattctat tttagcctct aaattaagaa aactaaaact 6540
   ctattttagt ttttttattt aataatttag atataaaata gaataaaata aagtgactaa 6600
   aaattaaaca aatacccttt aagaaattaa aaaaactaag gaaacatttt tcttgtttcg 6660
   agtagataat gccagcctgt taaacgccgt cgacgagtct aacggacacc aaccagcgaa 6720
   ccagcagcgt cgcgtcgggc caagcgaagc agacggcacg gcatctctgt cgctgcctct 6780
   ggacccctct cgagagttcc gctccaccgt tggacttgct ccgctgtcgg catccagaaa 6840
   ttgcgtggcg gagcggcaga cgtgagccgg cacggcaggc ggcctcctcc tcctctcacg 6900
   gcaccggcag ctacggggga ttcctttccc accgctcctt cgctttccct tcctcgcccg 6960
   ccgtaataaa tagacacccc ctccacaccc tctttcccca acctcgtgtt gttcggagcg 7020
   cacacacaca caaccagatc tcccccaaat ccacccgtcg gcacctccgc ttcaaggtac 7080
   gccgctcgtc ctcccccccc cccctctcta ccttctctag atcggcgttc cggtccatgc 7140
   atggttaggg cccggtagtt ctacttctgt tcatgtttgt gttagatccg tgtttgtgtt 7200
   agatccgtgc tgctagcgtt cgtacacgga tgcgacctgt acgtcagaca cgttctgatt 7260
   gctaacttgc cagtgtttct ctttggggaa tcctgggatg gctctagccg ttccgcagac 7320
   gggatcgatt tcatgatttt ttttgtttcg ttgcataggg tttggtttgc ccttttcctt 7380
   tatttcaata tatgccgtgc acttgtttgt cgggtcatct tttcatgctt ttttttgtct 7440
   tggttgtgat gatgtggtct ggttgggcgg tcgttctaga tcggagtaga attctgtttc 7500
   aaactacctg gtggatttat taattttgga tctgtatgtg tgtgccatac atattcatag 7560
   ttacgaattg aagatgatgg atggaaatat cgatctagga taggtataca tgttgatgcg 7620
   ggttttactg atgcatatac agagatgctt tttgttcgct tggttgtgat gatgtggtgt 7680
   ggttgggcgg tcgttcattc gttctagatc ggagtagaat actgtttcaa actacctggt 7740
   gtatttatta attttggaac tgtatgtgtg tgtcatacat cttcatagtt acgagtttaa 7800
   gatggatgga aatatcgatc taggataggt atacatgttg atgtgggttt tactgatgca 7860
   tatacatgat ggcatatgca gcatctattc atatgctcta accttgagta cctatctatt 7920
   ataataaaca agtatgtttt ataattattt tgatcttgat atacttggat gatggcatat 7980
   gcagcagcta tatgtggatt tttttagccc tgccttcata cgctatttat ttgcttggta 8040
   ctgtttcttt tgtcgatgct caccctgttg tttggtgtta cttctgcagg tcgactctag 8100
   aggatccatg gccactgtga acaactggct cgctttctcc ctctccccgc aggagctgcc 8160
   gccctcccag acgacggact ccacactcat ctcggccgcc accgccgacc atgtctccgg 8220
   cgatgtctgc ttcaacatcc cccaagattg gagcatgagg ggatcagagc tttcggcgct 8280
   cgtcgcggag ccgaagctgg aggacttcct cggcggcatc tccttctccg agcagcatca 8340
   caaggccaac tgcaacatga tacccagcac tagcagcaca gtttgctacg cgagctcagg 8400
   tgctagcacc ggctaccatc accagctgta ccaccagccc accagctcag cgctccactt 8460
   cgcggactcc gtaatggtgg cctcctcggc cggtgtccac gacggcggtg ccatgctcag 8520
   cgcggccgcc gctaacggtg tcgctggcgc tgccagtgcc aacggcggcg gcatcgggct 8580
   gtccatgatt aagaactggc tgcggagcca accggcgccc atgcagccga gggtggcggc 8640
   ggctgagggc gcgcaggggc tctctttgtc catgaacatg gcggggacga cccaaggcgc 8700
   tgctggcatg ccacttctcg ctggagagcg cgcacgggcg cccgagagtg tatcgacgtc 8760
   agcacagggt ggagccgtcg tcgtcacggc gccgaaggag gatagcggtg gcagcggtgt 8820
   tgccggcgct ctagtagccg tgagcacgga cacgggtggc agcggcggcg cgtcggctga 8880
   caacacggca aggaagacgg tggacacgtt cgggcagcgc acgtcgattt accgtggcgt 8940
   gacaaggcat agatggactg ggagatatga ggcacatctt tgggataaca gttgcagaag 9000
   ggaagggcaa actcgtaagg gtcgtcaagt ctatttaggt ggctatgata aagaggagaa 9060
   agctgctagg gcttatgatc ttgctgctct gaagtactgg ggtgccacaa caacaacaaa 9120
   ttttccagtg agtaactacg aaaaggagct cgaggacatg aagcacatga caaggcagga 9180
   gtttgtagcg tctctgagaa ggaagagcag tggtttctcc agaggtgcat ccatttacag 9240
   gggagtgact aggcatcacc aacatggaag atggcaagca cggattggac gagttgcagg 9300
   gaacaaggat ctttacttgg gcaccttcag cacccaggag gaggcagcgg aggcgtacga 9360
   catcgcggcg atcaagttcc gcggcctcaa cgccgtcacc aacttcgaca tgagccgcta 9420
   cgacgtgaag agcatcctgg acagcagcgc cctccccatc ggcagcgccg ccaagcgcct 9480
   caaggaggcc gaggccgcag cgtccgcgca gcaccaccac gccggcgtgg tgagctacga 9540
   cgtcggccgc atcgcctcgc agctcggcga cggcggagcc ctggcggcgg cgtacggcgc 9600
   gcactaccac ggcgccgcct ggccgaccat cgcgttccag ccgggcgccg ccagcacagg 9660
   cctgtaccac ccgtacgcgc agcagccaat gcgcggcggc gggtggtgca agcaggagca 9720
   ggaccacgcg gtgatcgcgg ccgcgcacag cctgcaggac ctccaccacc tgaacctggg 9780
   cgcggccggc gcgcacgact ttttctcggc agggcagcag gccgccgccg ctgcgatgca 9840
   cggcctgggt agcatcgaca gtgcgtcgct cgagcacagc accggctcca actccgtcgt 9900
   ctacaacggc ggggtcggcg acagcaacgg cgccagcgcc gtcggcggca gtggcggtgg 9960
   ctacatgatg ccgatgagcg ctgccggagc aaccactaca tcggcaatgg tgagccacga 10020
   gcaggtgcat gcacgggcct acgacgaagc caagcaggct gctcagatgg ggtacgagag 10080
   ctacctggtg aacgcggaga acaatggtgg cggaaggatg tctgcatggg ggactgtcgt 10140
   gtctgcagcc gcggcggcag cagcaagcag caacgacaac atggccgccg acgtcggcca 10200
   tggcggcgcg cagctcttca gtgtctggaa cgacacttaa gcgtacgtgc cggcctggct 10260
   ctccgaaagg gcgaattcca gcacactggc ggccgttact agacccaacc tagacttgtc 10320
   catcttctgg attggccaac ttaattaatg tatgaaataa aaggatgcac acatagtgac 10380
   atgctaatca ctataatgtg ggcatcaaag ttgtgtgtta tgtgtaatta ctagttatct 10440
   gaataaaaga gaaagagatc atccatattt cttatcctaa atgaatgtca cgtgtcttta 10500
   taattctttg atgaaccaga tgcatttcat taaccaaatc catatacata taaatattaa 10560
   tcatatataa ttaatatcaa ttgggttagc aaaacaaatc tagtctaggt gtgttttgcg 10620
   aattagcttg gtcacccggt ccgggcctag aaggccagct tcggccgccc cgggcaactt 10680
   tattatacaa agttgataga tcgaataact tcgtatagca tacattatac gaagttatcc 10740
   tgagctgatt ccgatgactt cgtaggttcc tagctcaagc cgctcgtgtc caagcgtcac 10800
   ttacgattag ctaatgatta cggcatctag gaccgactag ctaactaact agtacaaacg 10860
   tgcaagcgct actagacaat tcagtacatt aaaaacgtcc gcaatgtgtt attaagttgt 10920
   ctaagcgtca atttgtttac accacaatat atcct 10955
<210> 77
   <211> 965
   <212> DNA
   <213> Streptomyces spectabilis
<400> 77
   atgcagaatc ttcctgaaaa gttcgatgaa acacgcctat gggaagcttt gaggggattt 60
   ggtgtgtccc cgtcgcgtgt cacctacgcc cccgtcggat tcggggacta ccactggacg 120
   gtgaccgacg aggacggccg gccgtggttc gccaccgtct ccgacctgga gcacaaggag 180
   cactgcgggc agggggcaca ggccgcgctg aaggggctcc gacaggccat ggacacggcg 240
   ctggccctgc gcgaccgtga cggactgcgg ttcgtcgtgg caccggtggc cgccaccgac 300
   ggcggcggtc cagtactgcc cctggacgcc cggtacgcgc tcacggtgtt cccccatgtc 360
   ccgggccgca caggggagtt cgggcagcgc ctgacggagg ccgagcggga ccggctgctc 420
   gccctgctcg cggagctgca cggccggacg cccccggaga ccacaccgcc cgccgacatg 480
   gagcccccgg gccttcccgg cgtgcgcgcg gccctggccg agtcggaagg gccctggtcg 540
   ggcggcccgt tcgccgagcc cgcgcggctg ctgctgcgcg agcacgaggc gacgctccac 600
   gcgcgcctcg ccgagttcga ggccctcgtc gcgcgcgtga agggccgggg cgcgccgctc 660
   gtcgtcacgc acggcgagcc gcatccgggc aacctcatcc tgggtgagga gggctatctc 720
   ctggtggact gggacacggt gggcctcgcc ccggccgagc gcgacctctc cctgatctcg 780
   gacgacccgg cggacctcgc ccgctacgcc gagctgaccg gccgcacccc ggacccggac 840
   gccctcgcgc tctaccggtg gagtggttcc gcgccgagca ccagcgcacg caggacaccg 900
   agtccgcgtg gaagggcttc acggacacgc tggggcaact ggcggcgggc ggatccgcag 960
   cctag 965
<210> 78
   <211> 993
   <212> DNA
   <213> Legionella pneumophila
<400> 78
   atgctaaaac aaccaattca agctcaacaa cttatcgaac ttttgaaagt gcattatgga      60
   attgatattc atacagcaca attcatccag ggtggtgctg atacgaatgc atttgcatat     120
   caagcagatt cagaatccaa gtcttatttc ataaagctaa aatacggcta tcatgatgaa     180
   attaatttat cgataatccg tcttttacat gattctggaa taaaagaaat tatttttcct     240
   atccatacac ttgaagcaaa attattccag caactaaagc attttaaaat aattgcgtat     300
   ccatttattc atgcgcccaa tggtttcacc caaaatttaa caggaaaaca gtggaaacag     360
   cttggaaaag tattaagaca aattcatgaa acatcagttc ccatctcgat tcaacaacaa     420
   ttaagaaaag aaatatactc ccctaaatgg cgtgaaatag tcagatcctt ttataatcaa     480
   attgaatttg ataattcaga tgataagctc acggctgcct ttaaatcttt ttttaaccaa     540
   aatagtgctg caattcatcg attagttgat acttcagaaa aactatctaa aaaaattcaa     600
   cctgatttag ataaatacgt actatgtcat tctgatatac atgcgggcaa tgtgttagtc     660
   ggtaatgaag agtcgattta cattattgat tgggatgagc ctatgttagc tccaaaagaa     720
   cgtgatttga tgttcatagg tggtggcgtt ggtaatgtat ggaataaacc ccatgaaatc     780
   caatattttt atgaaggtta tggtgaaata aatgtcgata aaacaatttt gtcttattac     840
   aggcatgaac gaattgtcga agatatcgca gtatacgggc aagacttgct ttcacgtaat     900
   caaaacaatc agtccagact tgaaagtttt aaatatttta aagaaatgtt tgatccaaac     960
   aacgttgttg aaatagcttt tgctacagag cag                                  993
<210> 79
   <211> 2556
   <212> DNA
   <213> Agrobacterium rhizogenes
<400> 79
   atgatttggg atcatttatc accttcccga agggttctgc ttgatatcaa atcgtggtct       60
   gtattggtac tggtaattgc ctcgatatct tttgcggttc tggccatagg atcttggcaa      120
   gacaacgaaa gcaacgaagc gatcctgaca gagctgcaat cgatcgacgt ggattgcgcg      180
   atgctgcagc ggaatgtgtt gcgtgctcac gccggccttc ttcggaatta ccatcctctt      240
   atcgtccctc tggggcgact gcgcacgagc gtcgcagatt tacagcagct ttttaggcgg      300
   gcgggcctcg acggggccgg tgaattttct gaattgctgg cgcgggtgaa gagctcagta      360
   gatgcgaccg acgcagccgt tgcagcgttc ggtacgcaaa atgtaatgtt ccaggattcg      420
   ttggcgacct ttaaccagtc gatagcttcg cttccgaggt ctttggatac gagggatttg      480
   aactcgctag aagtcgctga gttgggctat ctgatgctcc agttttcgtc tcagccaaat      540
   ttcgagtttg cacgggaaat caaccaacgt ctcgaccggc ttcaggtttc ggcgaacggg      600
   gataaggcag gcgtcaagga gatcgttcgc agtgggagga ttatcctgac tctactgccg      660
   cgattgaaag acaccgttgg agtgatccaa acttctgaaa ccatcgacaa cacgaagaag      720
   ttgcagcgag cataccttga agcttatagc ttggccagcg cgggtgaaca gcgggtacgg       780
   gtcttcctcg gcgcagtttc ggttttcttt tgcttttgca taatttccct agtccatagg      840
   ttacatctgc gaaccaaggt gttgacgaga cgactggact tcgaagaagt gatcaaaaag      900
   attgggcttt gcttcgaaga tgcttcagaa gcaaaaccgt cgttggaatc ctcaacgaat      960
   gccgcgttgg gaattatcca attattcttt gaagctcatc aatgcgcgct gggactggtc     1020
   aacatcaatg agaatgacgt tggcgtcacc ttttttggaa gcactcctgc gccagagtgg 1080
   aacgaacaac gggtgcgcga aattgtttcc acggtagggg ccgaggacgg cgggcggatt 1140
   ttccgagcct atcctcaacg aaaagcgagc tgcttcggtg aggactctcc atttctgtgg 1200
   gtgctgcttg cgttcaaggt atccgatcga atagtcgctg tctttggcct gggatttgat 1260
   cgagagcatc tacagacgcc gaccgcacgt gaaatgcagc tgatggaact agcggctgca 1320
   tgtgttagcc actatgcggt cattcggcgc aagcagtccc agcgagatat tctggaacgt 1380
   cgtttgaaac atgcggaacg gcttgaagca gttggtacgc ttgccggcgg cattgcgcat 1440
   gaatttaaca acattttggg tgcagttctg ggttacgcgg aaatggcaca caacgttctg 1500
   cgccgtcgta ctcatgctcg agactacatc gatcacatca tttctgaagg caatcgagcg 1560
   cggttgattg tcaaccagat ccttgccctt agtcgaaagc gggatcgaac aacaaaacca 1620
   ttcgatcttt cggagttagt gaccgtgatc gcgccgtcgt tacgtgttgc gttgcctccg 1680
   ggcgtcgagc tcgatttcaa atatcagagc gctccaatgg ttatcgaagg aaactcactt 1740
   gaggtcgagc aaatactgat gaatttgtgc aaaaactctg ctgaagcatg ccgagagtta 1800
   gggcgtgtgg aggtcagcgt taggaggtct gtcgtacgga aacacaaggt tcttacaaac 1860
   ggtactattc ccaccggcga gtatatcctt ctttcagttg aggataacgg cgcaggcata 1920
   acagaggcgg tattatcgca cattttcgag ccttttttta ctacccgcgc ccgcagcggt 1980
   ggcacgggcc tgggattatc cacggttcac ggtcacgtta gcgcgatggc tggttacatc 2040
   gacgtggtat caactgtcgg cagaggtacg cgcttcgacg tctacttgcc accttcgtcg 2100
   aagcagccgg taaattcaga gagttttttt gaccctggaa gaatcccgct tgggaacggc 2160
   gagattgtcg cggtggtcga acccgactta gccacgctgg aaatgtacga ggaaaagatc 2220
   gcggcgctgg gctatgagcc agtcggcttt aacacgttgg atggtttaat cgactgggtg 2280
   ttggaaggaa aagaacctga tcttgttcta attgatcaac cgtctttgct ggatggagac 2340
   ggtgcagggt ccttaatctc aaagctggca aaagcgccca tcatcattat tggcgaaaac 2400
   cagaaaaatg ttcccctttc tgcggatcgc gagggatctg cccgtttttt ggaaaagccg 2460
   atttcagcaa agactctcgc ctatgtcgtt cgtgcgaata tcagaactga acgaatggtc 2520
   ggctcgaata gcagcggaat cgtgattgcg tcataa 2556
<210> 80
   <211> 720
   <212> DNA
   <213> Agrobacterium rhizogenes
<400> 80
   atgcggaaga cggcaggatc tttcgggatc atttttctga tggtccagcc aagcaaagct 60
   gcgccgctct cgttcgctga atttaaccaa ctcgctcgcg aatgcgcacc ttcagtcgct 120
   ccatcgacgc ttggagcgat tgcgaaggtt gaaagtcagt ttgatccgct cgtgctgcat 180
   gacaatacca ccggcgaaac tttacatggc aagaatccgg cggatgctac gcaaagcgta 240
   aacaatcgcg tcgcagcagg gcattcagtc gatgtaggcc taatgcaagt gaactcgaaa 300
   aatttcgcga acttgggctt gactgccggc aatgcaatca acccttgcgt gtccctgtcg 360
   gcggcggcgg acctgctcgt acggcattat agtggcggcg acacggtaga gtcagaacaa 420
   cttgccatcc ggcgagcaat ttcggcttac aataccggca acccgacacg cggcttcgcg 480
   aacggctatg tgcggagggt cgaagtggcg gctcagctac tcgtcccccc gctggctcag 540
   tcggggaaaa aaggcgaccg tgatgagcgg agtccagaag aaccctggaa cgtctggggt 600
   tcatacgacc gctcccactc agcagtcggt tcgtccgcgc cgccagaggc gcagcagccg 660
   aatgagcgca aatcccccga acaagatcaa gttttcgaac cgaatgatgg agatgcacca 720
<210> 81
   <211> 360
   <212> DNA
   <213> Agrobacterium rhizogenes
<400> 81
   atggcatggc ttgaaggata ccgcgcaccg tcgaaattcc gtggcctctg gcacagagct 60
   gtgcgcctgc tggctccgca cgtgccgagc gtcaccggcg cgatcggctg gagcttgttt 120
   ttctgcgagc cagctgccgc gcaggccgct gggggcactg accccgccac catggtcaac 180
   aatatctgca cgtttattct cggtccgttt ggtcaatcgc tcgccgttct tggtatcgta 240
   gccatcgggg tatcgtggat gttcggccga gcttcccttg gtctggtagc aggtgttgtt 300
   ggcgggatcg ttatcatgtt tggagccagc tttctcggcc aaacactgac agggggcggg 360
<210> 82
   <211> 324
   <212> DNA
   <213> Agrobacterium rhizogenes
<400> 82
   atgaatgccg atcttgaaga agccacactc tacttggcgg caacgagacc tgcattgttc 60
   tttggggtac cgctgacgct tgctggagtt ttcatgatgt tggcggggtt tctcatcgtt 120
   gttgtgcaga accccctcta tgaaattgtt ctcttgccgc tgtggcttgg agcgcggctc 180
   atcgtagagc gggactataa cgcggcaagc gtggtcttgc tgtttctcca gacggcaggg 240
   agaagcgttg acggccatgc gtggggcggc gcgagcgtca gtcccaaccc catcagagtg 300
   gcgtcgcgag gaagaggaat gacg 324
<210> 83
   <211> 2370
   <212> DNA
   <213> Agrobacterium rhizogenes
<400> 83
   atgttcggag caagcggacg gaccgagaga tctggggaaa tctacctgcc ttacgtaggg 60
   cacctcagcg accatgtcgt tcttctagag gacggttcca tcttgaccat ggcgcatatc 120
   agtggccttc ccttcgaact ggaggaagta gaggtccgaa atgcgcgctg ccgtgccttt 180
   aacacgctct ttcgcaatat tgccgatgat aacgtctcag tatatgccca tctcgtccgt 240
   cacaacgatg tgccagtacc gccggtacgc cattttcgca gcaccttcgc gtcaagccta 300
   agcgaaacgt tcgagcggcg tgttctgtct ggtaaactgt ttcgaaacga ccacttcctc 360
   acgctcattg tctctcctcg caccgctctc ggtaaagtgg gaagcagatt caccaggcgc 420
   tatgggaaga acccaggcga tcttgcgcat cagatccgac atctggagga tctttggaac 480
   gttgtcgctg gcgcgcttga tgggtatgga cttcgtcgac ttggcatccg agaaaaaaat 540
   caggtgcttt tcacggaaat tggtgaagcc cttcgcctga taatgacttg tcgtttttta 600
   ccggtgccgg tcgttagcgg atccctcggc gcctcagttt ataccgaccg ggtcatctgc 660
   ggcaagcgtg ccctcgagat tagagcgccg aaagatcgtt acgttggatc tatcttttca 720
   ttccgcgagt acccggcaaa gacgcggccc ggaatgctta acacactcct gtcatcaagt 780
   tttccgctcg ttttgagcca gagtttctct ttcctcacac gtgcacaggc tcacgccaag 840
   ctcagcctca aatcgaacca gatgactagc tccggtgaca aggcggtcac ccagatcggt 900
   gaactcgccc aagcagaaga ttcattggcc agtagcgaat tcgtcatggg ttcgcatcat       960
   ctcagccttt gtgtctatgg cgatgatctc gatacccttg ccgactatgg tgcgcgagcc      1020
   cgcacgagct tgtcggacgc gggcgcggtt atcgtgcagg aaagcattgg aatggaggct 1080
   gcatactggt ctcagcttcc aggaaaccac agatggcgta cccgtcccgg agcgatcact 1140
   tcacgcaact ttgccggctt ggtttcattc gagaacttcc ctatcggaag gaagtcgggc 1200
   cattggggag gtgcggttgc tcgtttccgt acaaacggcg ggacgccctt cgattatatt 1260
   ccgcacgaaa acgacgtggg catgaccgcg atcttcgggc cgattggcag gggaaaaacg 1320
   acacttatga ccttcatcct ggcgatgctt gagcagagcg tggttgaccg cagcgggacg 1380
   atcgtttttt tcgataagga tcgtggcggt gaactgcttg tgcgcgccac cgggggcacg 1440
   tacctcacgt tacgacgagg tgtcccaagc ggccttgcac ctttgcgcgg cctagaggat 1500
   acggcagcgg cgcgggactt tctccgcgag tggatcgtgg cgctgattga aagcgacggc 1560
   agggggggaa tttcgccgga ggaaaatcgc cgcttagagc gcggtatcca acggcaactt 1620
   tcgtttgagc ctaacatgag atcccttgcg ggcttaaggg agttcttatt gcacgggccc 1680
   tccgaaggtg cgggagctag attgcagcgg tggtgccgag gcaacgctct tggctgggcg 1740
   tttgacggcg aatcggacga agtgaagtta gatccttcga ttacgggctt cgatatgacc 1800
   catctcctcg aatatgagga ggtatgcgcg ccggccgcag catacctttt gcatcgaata 1860
   ggagcgatgg tagatggccg acgcttcgtc atgagctgcg atgagtttcg ttcctacctg 1920
   cttaatcaga aattcgcagc agtcgtcgac aagttcctgc tgaccgtccg gaagaacaac 1980
   ggaatgttag ttcttgcgac gcagcaaccc gagcacgtac ttgattcacc attgggcgca 2040
   agcctcgtag cacaatgtat gaccaaaatc ttctacccat cacccactgc agaccgctct 2100
   gcttacatag acgggttgaa gtgcaccgag cgagaatttc aggctattcg cgaggagatg 2160
   gcgattggca gccgcaaatt tctgctgaag cgcgagagcg gaagtgttgt gtgcgaattc 2220
   gatctgcgcg acatgccgga atacgtcgcc gtactatccg ggcgtgccaa tacggtgcgt 2280
   tttgcccagc agttgcgcga gacgcatgga gaggaaccct ctgcctggct ggaaaaattc 2340
   atgacgcgct accatgaggc acaggattga 2370
<210> 84
   <211> 669
   <212> DNA
   <213> Agrobacterium rhizogenes
<400> 84
   atgaatatta cgaagctcgt aataaacgca cttttcatat gccttgttct gtcggggact 60
   gccaaggcgc agtttgttgt cagcgaccct gcaacggagg ccgaaacgct gacgacagcg 120
   atcaatactg cggcgaacct cgagcaattg atcacgatgg tgacaatgtt gacctcgccc 180
   tttggcgtca ccggcatgtt atcagcgatc gaccagaaaa accaatatcc ctctgccggc 240
   caactcgaca aggaaatgtt ttcgccgcag atgcctgctt cgacaactgc gcgtgcaatt 300
   accttagatg ccgatcgcgc agtcgtgggc gacgacgctg aaggaaatct tttgcgacag 360
   cagattgcag gcgccgcaaa tgctgccggt gtcgcggctg acaatctgga tgcaatggac 420
   aagcgccttg cggcaaattc tgagacatcg ggccagctct cccgctcacg caatatcatg 480
   caggccaccg tcaccaacgg tttgcttctc aaacagatcc atgacgcaat tattcaaaac 540
   attcaggcga ccagcctgtt gacgatgacc accgcacagg cggggctgca cgaggcggag 600
   gaggcggcga cccaacgaaa ggaacatcag gcaactgcgc ttatatttgg cgccgctcaa 660
   ctacactga 669
<210> 85
   <211> 888
   <212> DNA
   <213> Agrobacterium rhizogenes
<400> 85
   atgaatttta gcattcctgc gccttttacg gcgatacata cgatattcga cctcgccttc        60
   accgtcgggc tggacacgtt gcttggggac attcaacggg cagtcagcgc tcctctcgtg       120
   gcctgcgtga cgctatggat cattgtccag ggcatcctcg taatgcgtgg ggaaatggac       180
   gcacggggcg gaatcacccg ggtaatcatg gtatcggtag ttgttgctct tatcgttgag       240
   caggcggaat atcatgatta cgttgtctcg gttttcgagg atacgattcc gaatttcatt       300
   caacagttcg gtatcagtgg gcttcctttg cagacgattc ctgctcagct cgacacgatg       360
   ttttcactca cgcaagtagc ctttcagaag attgcttctg aaattgggcc gatgaacgat       420
   caagacatcc tcgcctttca aggcgcacaa tggattttct atggaacgct ttggactgcg       480
   tttggaatct acgatgccgt cggaatcctc accaaggtgc tattggcgat cggtccattg       540
   atgctcgttg gctatctctt cgatcgcact agagacatgg ccgcgaaatg gatcggccag       600
   ctcgtcactt atggcatact tctgcttctt ctaaacatag tggcaactat cgtggttctg       660
   acggaagcga cggcactcgt gctaatgctc ggggttatca cttctgctgg cacaacggcg       720
   gccaagataa taggcctcta tgaactcgac atgttctttc tgaccggcga cgccttgatc       780
   gtcgctcttc cggcaatcgc cgggaatatt ggagggagct attggagcgg tgcgacgcaa       840
   acggccggta gcttaaatcg ccatttcgcc cggacaatcc gccgttag                    888
<210> 86
   <211> 153
   <212> DNA
   <213> Agrobacterium rhizogenes
<400> 86
   atgaagtact ttctattgtt tttgatcatc ggcttggcgt cttgtcagac aagcgatcag        60
   ttggcgactt gtaaggggcc tatttttccg ctgaacgtcg ggcgatggca gcctgctcag       120
   tcggaccttc agcctaccaa cgcaggagag gct                                    153
<210> 87
   <211> 708
   <212> DNA
   <213> Agrobacterium rhizogenes
<400> 87
   atgaacggct ctgaatacgc gctgttagta gagcgggaag cattggcgga ccactataag 60
   gaagtagaag catttcagtc tgcacgtgct agatcagctc ggcgaatctc tagagccttg 120
   gctgctttgg ctgtcattgc agtcgcagga aacgtggcgc aggccttcgc tatcgccgtc 180
   atgcttccgc tgaacaaact tgttccagta tatctgtggg tgcgcccaga cggtacagtt 240
   gacagtgagg tatctatttc gcggttgccg gcgacgcagg agcaagcggt cgtgaatgcg 300
   tctctatggg agtatgttcg tctgcgggag agctactcgg cggatacagc ccaatacgcc 360
   tatgatctgg tctcgagctt cagtgcccca acggtgcgtc aagattatca gcagttcttc 420
   aattacccca gtcctagctc cccccaaacc atcattggta agcgcggaaa gctggaagcc 480
   gagcacatcg gctcaaacga acttatgact ggcgtccagc agatccgcta caaacgcact 540
   ctcatcatgg aagggcaagc tccaatagta accacctgga ccgcaacggt acattatgaa 600
   acggtaacta acttgcccgg ccgattgagg ctgacaaatc caggcggctt aatcgttacc 660
   tcctatcaaa cttcggaaga taccgtttcg aacacgacgc ggagccag 708
<210> 88
   <211> 876
   <212> DNA
   <213> Agrobacterium rhizogenes
<400> 88
   atgatcaaga atttgtttct gggcctggtt tgtatcctct ttgtaaccag cggcgcgaac       60
   gcggaagaca cgccagcggc aggcaaactg gatccacgta tgcgctatct cgcctacaat      120
   cccgatgagg ttgtgcacct ttcaaccgct gttggagcca cattggtcgt aacatttggg      180
   tccaatgaag cggtgacagc cgtcgccgtt tccaacagca aagatctcgc tgcgctccca      240
   cgcggcaatt atctgttttt caaggccagc aaagtgctgc agccgcagcc tgtgatcgtc      300
   cttaccgcaa gcgacgcagg aatgcggcgc tatgttttca gcctcgcgac cagaacgatg      360
   tctcggctcg ataaagagca gcctgacctc tactacagcg tacagttcac ttatcctgcc      420
   gacgttgccg ctgctcgccg aaaagaagcg gagcagagag atcttgcgga tcggatgcgg      480
   gcgcaagcac aatatcagcg tcgagccgag gacttgctcg agcgtccgcc agcaggtggc      540
   agcacagatg cgaaaaattg gagctatgtc gcgcaaggag accgctcgct attaccgctc      600
   gaggttttcg acaacgggta ttccacgaca tttcgctttc ccggaaacgt gcgtgtacct      660
   tcaatctacg tgattaaccc agatggcaag gaagccacgg ccaactattc agttaaggga      720
   gattacgttg aagtagcatc agtttccagg gaatggcgtc tgcgggacgg ccatacagta      780
   ctttgcatct ggaataaggc atatgacgcc gtcggacgga agccgggcac tggcacagtc      840
   agacccgatg tagtgcgcgt gctgaaggag acgagg                                876
<210> 89
   <211> 1131
   <212> DNA
   <213> Agrobacterium rhizogenes
<400> 89
   atggaggacg tgaatgccca atccagagaa ggtatcgacg cgcccggatc cctcgtcacc       60
   gatcctcatg gccggcgcct ctcggggtcg caaaagctcc ttgttgcggg tttagtcctg      120
   gtactgtcgt tgagcctcat ttggctcggg gcgcgttcaa agaaaaggac cgagccgtct      180
   ccaccaaaca caatgatcga tgccaacacg aagccctttc ggccggcccc gattgatatt      240
   cccgccaaac cggcaatgac accgccagct gcggaagcaa cggttttgcc gtcggatcaa      300
   catcagcgag aacacaacga actgaggccc gaagaaacac cgatctttgc ctacagcgga      360
   ggtgatcaga atggtgtcaa atccgcccca cacgctgata ttcagaacgg tggccaagac      420
   aatagaaacg ccaactccct agccgcgtcg gaggattctg ctgagaacga tctctctgtg 480
   cgcttgaaac cgacagtgtt gcagcctagc ctcgctatac ttttgccgca cccagatttc 540
   actgttacgc aaggaacgat cattccctgc atactgcaaa ccgccattga tacaaatctg 600
   gcagggtatg tgaaatgtgt acttcctcaa gacatacgcg gagcaaccgg aaatgttgtg 660
   cttctggatc gcggaacgac agttgtcggc gaaatccagc gtggactgca gcagggagac 720
   gcccgcgttt tcgttctctg gaatcgcgcg gagacgccct cacacgccgt cgtctctctt 780
   tcctccccgg gggccgacga actcggccgt tccggactgc cgggtacggt cgacaatcat 840
   ttctggaaac gctttagcgg ggccatgctc ctgagtgtcg tccagggcgc agtccaggcg 900
   gcaagtagct acgcagggaa ttcgaccggc gggaccagct tcaatagttt ccaaaacaat 960
   ggcgaacaag cggccgatac ggccctaagg gcggccataa acattccccc aaccttgaaa 1020
   aaaaatcagg gtgacacggt ctcgatcttc gtcgcgcggg atttggattt ctcaggaatc 1080
   taccagcttc atatgactgg tggatcggtg aaacgtcggc atctgcgcta a 1131
<210> 90
   <211> 1035
   <212> DNA
   <213> Agrobacterium rhizogenes
<400> 90
   atggaagtaa atccgcaatt gcgcgccctt cttaacccag tcttgcaatg gctcgatgac 60
   ccgaggactg aagaagttgc cataaacgga ccgggagaag cctttgtacg ccaaagtggc 120
   gtcttcacga ggttcgccgc accgttctct tatgatgatc tcgaagacat cgccattcta 180
   gcaggggcat tgcgaaaaca agatgtcggc ccccgcaacc ctctttgcgc taccgagctc 240
   ccaggtggcg agcggatgca aatctgcttg ccgccaacgg taccctctgg caccgtgagc 300
   ttgacaattc gacgaccgag caatcgtgtc tctgaattgg gggaagtctc ggctcgctac 360
   gatgttcgtc gatggaatca gtggcaaatc cggacgcagc gacgagatca actagacgaa 420
   gcgattctgc gcgactacga caatggcgat ctggaatcat ttttacgtgc atgtgttatc 480
   ggtaagcgga cgatgttgct ttgtgggccg accggaagcg gcaagaccac gatgagcaag 540
   accttgatca gtgctattcc gcgggaagaa aggctgataa ccattgaaga tacgctcgaa 600
   ctcgtcattc cacatgagaa ccacgtcaga ctgctctact ccaagagtgg ggcagggcta 660
   ggtgcagtga ctgcggaaca gctgctccag gcaagccttc ggatgcggcc tgaccgaata 720
   ttgctcggtg agatgcgcga cgacgcggcg tgggcgtacc tgagtgaggt ggtctcaggc 780
   caccccggat cgatttcaac catacatggg gcgaatcccg tccaaggctt caaaaagctg 840
   ttctcacttg tgaagagcag tgtgcagggg gcatgcttgg aagatcgcac actgattgac 900
   atgttggcaa cggcaatcga tgtcattgtt cccttccgtg cttacggcga tgtttacgaa 960
   gtgggcgaag tatggctcgc cgccgatgcc cgccgacgcg gtgagacgat aggggatctt 1020
   ctaaaccagc aatag 1035
<210> 91
   <211> 690
   <212> DNA
   <213> Agrobacterium rhizogenes
<400> 91
   atgcgccatc ttattaccga gtatttaacg attcatgcct taagggtcac cgcggtatca 60
   gatagccagc aattcaaccg ggtacttgca tcagagacgg tcgatgtcgt cgtcgtggat 120
   ctcaatttag ggcgtgaaga cggacttgag atcgttcgca gtctcgccac aaaatccgat 180
   gttccaatta taatcatcag cggtgatcgc cttgaggagg cggataaagt ggtggcgctc 240
   gagctgggag caacggattt tatcgcgaaa cctttcggaa cgcgcgagtt cctagcacgc 300
   atccgtgtcg cattacgcgt gcgaccgaat gtcatgcgaa ccaaagaccg gcgttcattt 360
   tgtttcgccg gctggacact tagtctcaga cagcggcgat tggtatccgc acaaaatggc 420
   gaggtgaagc ttacggcagg ggaattcaat ctgctcgtcg ctttcctgga aaagccacgc 480
   gacgtgcttt cccgagaaca gctcctcatc gcgagccgag tgcgtgagga ggaagtatat 540
   gacagaagca ttgatgttct cattttgagg ctccgccgaa agcttgagca ggatgcagcg 600
   agcccaaact tgatcaaaac tgccaggggt gccggctatt tcttcgacgc tgatgtggat 660
   gtttcctatg gaggtctgat ggcggcctga 690
<210> 92
   <211> 696
   <212> DNA
   <213> Agrobacterium rhizogenes
<400> 92
   atgaaacttc tgacattttg ctcatttaaa ggaggcgccg gcaaaaccac ggcgctcatg 60
   ggcctttgcg ctgcgtttgc aagagacggc aaaagagtgg ctctcttcga tgcggacgag 120
   aaccggccgt taacgcgatg gaaagaaaac gcgatgcgta gcaacacctg ggatacatcc 180
   tgcgaggtgt acgctgcaga agaaatgtcg cttcttgaag cggcttatga ggacgccgag 240
   cttcaggaat ttgattatgc gctggccgat acgcatggtg gttcgagcga gctcaataac 300
   acaatcatcg ctagctcaaa cctgcttctg atcccgacaa tgttaacgcc gctggatatc 360
   gatgaggcac tttcgacata tcgttatgtt gtcgagctgc tgctgagcga gaacttagtg 420
   attccgaccg ccgtgttacg ccagcgagtc ccggttggcc gattaaccac gtcacaacgc 480
   gcgatgtcag acatgctggt gaatctgcct gtcgtacaat ctccaatgca tgaacgagac 540
   gcgttcgctg caatgaaaga gcgtggcatg ttgcatctca cattgctgaa cacgacaaat 600
   gatccgacga tgcgcctcct tgagcggaac ctcagaattg cgatggagga actcgtaaca 660
   atttcagaat tgattggcga taccttgggg aattga 696
<210> 93
   <211> 606
   <212> DNA
   <213> Agrobacterium rhizogenes
<400> 93
   atggggatcc gcaaacctgc tttgtctgtc ggtgaagcaa gacggcttgc tgccgcgcga 60
   ccgatcgttc acccaatccc agctctttcc tcgcaaaact tggcgccttc gcaattacct 120
   gaaagagccg gaaaggaaaa acgaccagcg cctcctatcg ccgccaaacg tcgtgacaac 180
   ttcgatcggc aatcaatgct aacggcggac gccttgagtt cgactgcgcc gccggaaaaa 240
   gtccaggtct ttctttcggc acgccccccc gctcctgagg tatcgaagat atatgacaac 300
   ttgattctgc agtacagcgc taccaaggcg ctgcagatga tcttgcgccg tgcgctcgcc 360
   gattttgaga acatgttggc ggacggatca tttagcacgg ggcctcaaag ctacccgatc 420
   tcaaaagtag tcgaaaaacc tatcgttgtc ctgacttctc gcatgttccc ggtatcgctg 480
   ttagaagtcg cacgcaatca ctttgatcca ttgagattgg agaccgccag ggcttttggc 540
   cacaaactgg ctacggccgc acttgcatct ttctttgccg aagagaagat aagaaaagaa 600
   cgctaa 606
<210> 94
   <211> 447
   <212> DNA
   <213> Agrobacterium rhizogenes
<400> 94
   atgtcgcaaa gcgaaaaatc cacttcaagt gacattgtcg accggcgtga aggcccgaag 60
   attgaaggtt tcaaggtcgt tagcactcgt ttgcgatcgt cggaatatga gagcttctct 120
   cgccaagccc gtctgctggg gctctccgac agcatggcca taagagttgc ggttcgccgc 180
   attgccggct ttctcgaaat cgatgcagag actcgtcaga tgatggaagc cattcttcat 240
   tcaataggag cactctcaaa caacattgcc gcgctgctgt gtgcctatgc tgaaaatccg 300
   acaacggatt tgggcgctct gcaagctgaa cgaaacgctt tcggtaaatc gttcgctgat 360
   ctcgacggtt tgctccgttc cattttgtcc gtatcacggc gacggatcga cggttgctcc 420
   atgctggcgg acgccttgca gcattaa 447
<210> 95
   <211> 1176
   <212> DNA
   <213> Agrobacterium rhizogenes
<400> 95
   atgcccgatc ggtctcaagt catcatccgc attgtgccgg gaggtgggac gaagaccctc      60
   caacagatta tcaaccagtt ggagtatcta tctcggaagg gcaagttgga gctgcagcgt     120
   tcggcccgac atctcgatac tttcgtacca ccggatgaaa tccgcgaact cgccctaagc     180
   tgggttcaag aaaccgggac ttatcacgaa agtcggccag acgaggaaag gcaacaggag     240
   ttgacgaccc acattattgt aagcttccct gccggtacaa gtcaggtagc agcttatgca     300
   gcgagccggg agtgggcagc cgagatgttt gggtcaggcg cagggggagg ccgatacaac    360
   tatcttacag cctttcacat cgatcgcgat cacccacatc tgcatgtggt cgttaatcgt     420
   cgcgaacttt tggggcacgg ctggctgaaa atatcccggc gccaccccca actgaattat    480
   gacgccttgc gcattacaat ggccgagatt tcacttcgtc atggcgtcgt cctcgaggcg     540
   acgagccgag ctgagcgcgg catctcggag cggccgatga cttttgccca gtatcgacgc     600
   ctggagcggc agcaggccaa tcaaattcgt ttcgaggata ttgatttcga agaattctcg     660
   cctgggggaa acgacggaga accggatcaa tcttttaatt cttcgtacgg ttcgctgcct     720
   caaaacgtgt cagaaaactt gcgacgaaac ggcggtctgc aatcggtgtc tgaagtacgt     780
   tcccgggcac tgatcggatc gaacggcaac ggtgtcacgc ccgatcgtgt atcatctgga     840
   aacgattccc gttctcaatc cggcagcgat aaagcctttg tggacgatgg caacttgagg     900
   aacggccctc tcaccatcgc cggagacggc caggatcttg aaggccgttc tggcatgcat     960
   cgtttggcaa ccgaacccgt cacgcacaca acaagcgaag atgatgttcg gcaacggcct    1020
   catcgaaaac ggcctcgcgg tgatgaggaa gagcagagcg gcgcaaaact gaccagggta    1080
   gacggaattc ggacgggagt gacaatcagc gccgtaccgg ttgcccagga cgatccgatt    1140
   acatcgccga tccagccccc tggatcaaat ccgttg                              1176
<210> 96
   <211> 3024
   <212> DNA
   <213> Agrobacterium rhizogenes
<400> 96
   atgacccggg gcagaccaac atcgctacgg acgcattgcc tccgacagcc gatcgccggc 60
   agcagggaga gccaaattct aagcgtcctc gtgatgatga cgccgagccg agcattcgta 120
   agcggtcaag ggacgggcgc agccaagggg atgagggaaa cagaaggtag aaaaggatcg 180
   atcatggcag atgaagaatt tcgtcgagac ttcagccgct ccgctacgtt gaactcgaat 240
   aacgaaggcg ctacaggcgc accaatcccg gatacatcgg tgtcgactgc cttccgacaa 300
   aaccgcagag aacacttaac gaccagttcc ttacggccat taattactgg tggggccggc 360
   gaccttccag gcacttcccc cgaaaatcaa cggccgacaa cccatgcccc gaatacagca 420
   gaaatactcc cggtgagatc agccgaagct ccggctggcc aatcagcggt ctttccgagg 480
   agcaaggggt attttcgtac ggccatgcaa tacctgcggg agattgaaat gcagtccact 540
   gccagggcgg accacgaggc gacgtcgtcg tcacgttttg gcaaggggga aagaaagcag 600
   ctgcgcgaac acgagcaaac cggtgatcaa agtgaatcaa tggcgaagcg gagtaccagc 660
   tcgggaatgc aggtgggaac tattgccgcg tctttggcta tgaaagatag ctcgaccgcg 720
   acgccggctt cgcaagggcc gcaccggtct gggatggagc tggataatat cgaagggact 780
   ggagagagcc gtacgcaatc tcagcacgaa attgcgacac aacctattgc cacaagcgct 840
   tttggttcag gtcatgatgt gatcattcct ggagagggcc agcctgaaga aacattcgac 900
   ggcgcaccat attggatggt agaaagcggg gatagcgaga ccaatagcct tccgtcgatc 960
   caacaaggct tatcttggcc agggttagat gaccttgatt tgctcgccga cagcagttca 1020
   ccgctcagta ctcaggttgt tcctcctgac gtagccgcta cgaatttaag cactgaggca 1080
   gggtccgcca gcgtcgaaag cacccaaatc ctcgccgaca gtgcctattc cgacgacgcg 1140
   accgaaaccg gtggccaaac agcttcagga tcgatccatg aaggttcagt tcgttcgctt 1200
   ggctcaccgg aggaacgctt cgacgccttt cccgagttga ccgacgagga cttggcgcag 1260
   attgacgcat tctcgcgagc ccgctctcct tcgaggcgag atgctgcgca cgctattcat 1320
   gcggtcagct tggatgatgc cttctcacgc aaggatcttg ccgcgggcca cgacggccgg 1380
   cttacgaatc ctgcgttccg tcaggaggct tctctgcagg aatcgagccc atcagaaacg 1440
   aagtccgacg acttcccgga attaaccgac gaggacctgg cacggatcga cgcactctcc 1500
   gaagcccgct caacttcgag gctggatgtt gcacgcacta ttcatgcggt caggttggag 1560
   gcctctcggc aggacccgag cccatcagaa atgaagtccg acgacttccc cgaattgacc 1620
   aaagaggact tagcccggat cgacgcactc tctggacccc gcccgactac ggggcgggaa 1680
   ggtgcgaaca ctattcagcc agtcaggttg gacgatgctt tctcagccga agattatacc 1740
   gtgggccgcg gcggccggca tacgaatcct gcgttccatg aggaagcttc tcggcaggac 1800
   ccgagcccat cagagatgaa gttcgatggc ttgccggagt tgaccgacga ggatctggta 1860
   aggattgacg caatctctga accgcgctcg aagagatctc gctccgagca ggttgtcggg 1920
   ggaaaatctc ataggctcaa actgaacgag gctatcgagg aggcgggctc gcccactacc 1980
   gtttcgggaa caagcgccgc cagggcggca cctatcacgc tcgaaagcgg agtacctagg 2040
   ttgccgcagg ctacccatac ggaacttcct ggctcggacg ttgcgggtcc actgcggatc 2100
   gggaaggcgt cgtgtgccga tatacggcga gatgtttcgg ataaagggaa acgaaaagtc 2160
   tcgtcagatt atcagattca gcaaggtcct ggcgcgtcct atgcttacca gccgcgacgc 2220
   atgccgacgg aagcgatttt cgatgctgaa cggcttggtg acgaagtgct catcgggaat 2280
   gagccagttc cgcggtggcc ttacccctgg gagcacgttg ttcgttttga aacaggcaag 2340
   cagctcttcg aaagattgca gaagtggccg actgcggagg agttggaaaa cagtgtgatc 2400
   ctcgtcaaag cggatagcgc gcaacgatcc tatatgcccc gaaatcagct gctcttgcat 2460
   ctcggatccg aaaagtatta cgaacgaatg aaagcgctcg gtctatccga taataccttc 2520
   agcggattga agtccgatag caaatatttt tatgatacac ctgctggacc acgggtaagt 2580
   gaacatgatt atgagttccc gcggcaggca ggcgtgcata atccagaggt gatggttcag 2640
   catcatagcg gaaccttctt cgcgttttgg ccaaaacttc tcaggctgga aaagcttgcg 2700
   acagtaacga atacgtatgg cgcggaaaat gtttggctca aatcgccagc caatgcttac 2760
   atgacgccgg aaacctataa ttccaaggtt gcgaacggcg ccgaaaacct tttcatcgag 2820
   ttcctacgtc ccgctcctgt ccacgaccac ctgcgggaac ctggaacttc aaacgcaaaa 2880
   actgttgacg gacgaaacga agatcgcgcg caacaggccg accggttagc agatcagtcg 2940
   atgggccgga acggggacgg ccgtgacgcg cagggtggta agagaacctt ggaaccgcgc 3000
   gtgcgcgaaa ggtatgggct gtag 3024
<210> 97
   <211> 1983
   <212> DNA
   <213> Agrobacterium rhizogenes
<400> 97
   atgaattcag gcaagtacac gccaataggc ctggctgcga gcatagcatg ttcattggcc      60
   gtggggtttt gtgcggccag tctctatgtc acatttcgcc atggctttac gggcgaaacg     120
   atgatgactt tcaacgtttt tgcgttcctg tacgagacac cgccttattt gggatacgca     180
   agtcctacgt tctatcgcgg actagccatc atcgttgcga cgtcaacgct cgtgttgcta     240
   tgccaactac tgttatcgat gcgcgagcgc gaacatcacg gcactgctcg ctgggccggc     300
   tccggcgaga tgcggcacgc caggtacctc cggcgctaca gtcacgtcac gggtcccatc     360
   ttcggcaaga catgtggacc acgttggttt ggcagctacc taagtaacgg agaacaacct     420
   cacagccttg ttgtcgcgcc aactcgcgct ggtaagggtg tcggcgtcgt tatcccgacg     480
   ttgctaacct tcaagggctc agtaattgcc ttggatgtga aaggcgagct atttgaactc     540
   acatcgaggg cgcgtaaatc aagtggcgac gcggttttca agttctcgcc cttagatcca     600
   gagcggcgaa cgcattgtta caatcccgtg ctggatattg ccgcgttgcc gcccgaacgg     660
   cgctttacag aaacgcgccg cctcgctgcg aatctcatca cagccaaagg caagggagcc     720
   gaaggtttca tcgacggggc acgggatctt ttcgtagcag gcatattgag ttgcattgag     780
   cgcggcacac caacgatcgg tgccgtttac gatctgtttg ctcagccggg tgaaaagtac     840
   aaactatttg cgcaactcgc ggaggaaaca caaaacaaag aggctcagcg tatcttcgac     900
   aatatggcgg ggaatgacac caaaatcctg acatcctaca catcggtgct cggcgacggc     960
   ggactcaatc tttgggccga tccactcgtt aaagcggcta caagcacctc ggatttttcc    1020
   gtctacgatc tgcggcggaa gaggacttgt atttatcttt gtgtcagccc gaacgatctc 1080
   gaagtcatag cgcccttgat gcgtcttctc ttccagcagg ttgtatcaat cctgcagcga 1140
   tcgctgccac ttggagatga acggcacgaa gttctgtttc tcctcgatga gttcaagcac 1200
   ttgggcaagc tggaagctgt ggagacggca attacgacga tcgccggcta caagggccgc 1260
   ttcatgttta ttattcagag tctttcggct ttgacaggaa cttatgatga agctggtaag 1320
   caaaattttc tcagcaatac gggtgtgcag gtattcatgg cgactgctga tgacgagacg 1380
   cccaactaca tttccaaagc tatcggcgac tacacattcc aggctcgctc aacgtcgtac 1440
   agccaagcgc gcatgtttga tcataacatc caaatctccg accaaggagc accgcttttg 1500
   cgcgccgaac aagtccgcct actcgacgac gattatgaaa tagtccttat caaaggccaa 1560
   cctcccctca aactgagaaa agtgcgatat tattcggatt tcatattgaa gcgaattttc 1620
   gaaagccaac acggttccct tcccgagccc gcatctttga tgttgccggg agacacaaac 1680
   ctcgttgaag gcaagctcga ccagggaaca gccgatacaa cttcgcaaaa accggtgcag 1740
   attgaagaga acgaccatgg ggaagtcgtt tccccccaaa acagaactgt ggctgatgga 1800
   gtaatgccaa tagaagttcg cccccgttca aacgaagttg atgacgagcg ggaataccaa 1860
   ggcgcggacg ccgcaacctc tgagaagctt ccggaaattg ctccggctct tttggcgcag 1920
   cgcgaactcc tcgaccagat catttcgctc cagctacgag gtagaacagc ctccggcact 1980
   tga 1983
<210> 98
   <211> 2508
   <212> DNA
   <213> Agrobacterium rhizogenes
<400> 98
   atgaaaccgt cgggaagctc aaaaactgga tacgccgggt cggctagatc ctcgccccag 60
   gttcgcgcag gggttacgcc tgtccttcat cccaaagagc cttggaaccg ttttgcttgc 120
   tccccatctg acggccaaat ggatcaccgg gaaaatttat ctgcgcaatt cgcttacgat 180
   ggaatgagac tgggcgcagc cgagcggtcc gcgtacgaga catgggatag agcggaccgg 240
   ccgagttgga aagacctgat actgagcgct cgcctgaacg cgattgacag tttcgcttgg 300
   aatgtcgatg tcggagaaag tacctcttca acttttgttt atgatggtgt tcctctggcg 360
   gaaggggaac ggcatgccta cgaggaatgg gtggagccgg gacagcccag ttggcaacaa 420
   ctcgttgtga acgcgcgtat tgaagagcta aacacctctg ctgcaattcc gaatgagtgt 480
   agtccccttc aagaggaatt ccgatcggag gcgcctaagc gtaagcggac aagctccatt 540
   ggtcaaaaga acggtttccc tgaaccattc gaatttgatg ggatgagact cggctcgcct 600
   gagcgcgaag catatgagaa ttggagtaaa ccgcaaccgc catcctggaa agacctgata 660
   gttgacgccc gtcttgacgc aatcgacacc tccacctggc tcaacgaatc aaatgacacc 720
   tcggtcttcg agtacgaagg cgtcccgctt ggggaggggg aacgcctagc atatgaaaaa 780
   tggctcgagc ccgcgcaacc aggatgggaa gacctggttg tggacgcacg cgttgcggaa 840
   ttgcaccagt ctgctccgac gtggtgcgaa caccaaccag gcaacgacgt ttcttccaac 900
   gagtccgggc gcatctcggg cgtcccaatt aacgccgagc aggtgtcgcg tgcgttcttc 960
   gtgtacgatg gagtggcgct cggagcagcc gagcgcgctg cgcatgatcg ttggagcagg 1020
   ccggaccggc ccacctggga agatcttatc atagtcgcgc gccaagctgc tatcgaaggc 1080
   ggtgccgttt cgaatgggat gatcgggaag acatcttcct cagtcttttt atacgaggga 1140
   atgttgcttg gggatgcgga gcgtcaggcg tacggacggt ggaggcagct agcccagccg 1200
   cggtggcaaa atctggtggt gaacgcgcgc ctggcagagc tcgacccggc ggcctggatt 1260
   cccgatgaac atgatccgtc tgaggatggc ggggcgactg gtcttctgtc gcaagcaagc 1320
   acgaccaata agtcccgcct cagtttaggt gatcaaccgg aagcgcgctc gcctagtctc 1380
   gcacgtgagc cagcacaaca gccgactcac gtgcaaaacc tgacgtgcgc acaattggaa 1440
   gcaagacgtg ctctatattt cgggccctct gggagggatg cagaccaaac cgccagcatc 1500
   gccgacagta atcgcctcga cgaggtaagc aaagttaaac ggctgggtgc caagagccgt 1560
   cgaggcgcta aagcaacggc ctatgacgta atttcaagtg cggaaagact gtcgtctcac 1620
   gagggttgtt tgacggttca gtccactcag ccagaaaaag ccgcttgctc gagaagcgac 1680
   aatatcggca cttatggaag tcgaaaaaac gaacgagctc ggcttgcgac cgagaccggg 1740
   aaatacgagt cggagcatat tttcgggttc aaggttgtcc acgataccct gcgctcgacc 1800
   aaagagggcc ggcggctgga aaggcctatg cccgcctatc tcgaatgcaa ggaacttcat 1860
   cgacaacatg ttggtacggg aagggggcgg acccgactgg tggggcgcgg ctggccagat 1920
   gacgcaagtt accgctcgga tcaaagggca actctatcgg accctgttgc gtccacggaa 1980
   ggtgcaacgg cgtcaaacgg gtatcaatta aaccagctag gctacgcaca ccagctcgct 2040
   aacgacgggc tgcagagcga aacgccagat ggggttatca tgccgcttca ggtcgcgact 2100
   actagctaca actatacggt aagccgcgat ccggtacttt ctccgcccag taaagagcaa 2160
   gctccggaat tgttgcacct tgggccacgt ggccagacag aagctgtact cgctcgcgaa 2220
   acagcattga caggaagatg gccaacacgc gagcgtgagc agcaagtcta tcgagagttt 2280
   ttagcccttt atgacgtcaa gaaggacctg gaggccaaga cacttgggtt gcggaagaaa 2340
   aaagctgcgc ttgtttccgc gttgaaccgg actgccgcct caataggcac ttcacccttg 2400
   aaagcccaat cgtcgagcgc agaagttgaa aaagcaactc acgagtttga tgaacgacgg 2460
   gtttatgatc cgcgcgatcg cggtcgggac aaagcattac aacggtga 2508
<210> 99
   <211> 930
   <212> DNA
   <213> Agrobacterium rhizogenes
<400> 99
   atgcaggaac gcgagctttc ggtcctcggt ggtgataaga ccgaaagtgc agccacctta        60
   ccgaatgaca ttttggtgga agttgccaaa catctgccga ccgacgatcc agtcgaaacg       120
   gcagccaacc tcacaagctt taaacttgcg agcccgtcgg ttcgggcatc ggtcgatcag       180
   agcgatgtcg gaacgttcca ccgaagcgta aatcgactgg gtgcctcaag caaggcattg       240
   tatgacctgg ctgtaccccg gaacggcttt gccgagtatc cgaactatcc ggaaccggac       300
   cctgctggcg agtttgcgct ggcgagccag cgcatcagga caattggtcc aactttgaag       360
   ttccaatcgc ccgccaggaa aacagcgatc gtcaatcaca ttctcaatat gtcggaaggc       420
   ggtgagcagg cggaggcgat tatgtcgatg acgtcccatt tgagtgacct cgagagggcc       480
   gacaagagac gccttatcga cagagcaatt gaatacttca aggtagacgg gcctatcaac       540
   tacgacagtc gccattatgc agcctacgcg attgcagcgg cgcacaacca gttggaactc       600
   gaacacaagt cacaaatctt cgatgcaatg gcgaagagac cccagcttgc gagactctac       660
   gccgacgaac gcatccatgc gcaggagcat ttggggcttg cgtcgacatc cgaaggtccg       720
   cacgagcgga gcaataaaca actggccacg gacattcttc agatcgaaca aaaaatccgc       780
   actgagctcc ttcccgaaac tctcagtgct cacgaccaga tggtaaaagc ggaaggaatc       840
   gctggatcga ttgagaaggc ttaccgcgat gctcgcgcga acttaacccg ggcaccgcga       900
   gggaggtcaa attctgatct cagccggtag                                        930
<210> 100
   <211> 1356
   <212> DNA
   <213> Agrobacterium rhizogenes
<400> 100
   atggtcagta taaataaaaa agccccgcag aaatcttcta cagcgaccac gcgcaattct      60
   gatgaacgcc tggttgatca gttagggcag gcgtaccttg tggaacgcga ggcgcaggaa     120
   aggcgcgcgc gacttgaagc gccgccggcg ttgaaaaaat atgcctcgaa actgcaactc     180
   ctcaataagc tcgatgcaga tttccgtggt gtgatcgccc acaagccgtt tcgcagcgag     240
   caactgcggg tcgatagcaa tggagaattg acccatagtc ggggtctcat aaaaaagaag     300
   aagaaggttt tcattcgaga tcaaacaact ggctcccttc ggcttattca ctacgagagg     360
   tctgactgga atagtgtccg tcgctatgac tacgaccaga acggcgtgtt gagcgagaaa     420
   catatcaaaa gcaaagccgg tgccttcgaa gaaaaatggg agcgggacga aaacggtgaa     480
   ctgatgcgca cccgatacat cgatcggcgc agacttactg gacgggcgtt gcatcccatt     540
   tccgaggaaa tcggacgtcc ttatgagagt ggcctggaga agaggcttta tcgcgtttta     600
   acgcgtcggg agggctccca acaaaagact ttcgaacgcg atgacaaggg taatctggag     660
   ctcattgcaa gcaaacggat gggctattcg atgtcttcac aaaaggcacc ggatcgacaa     720
   acatctagaa caagtatccg caagcttggc ggagctttca gtgaatcata tagatcccgg     780
   ctggataagg acggccacga gttaggtcgg gatgtctccg cacatcgaag cctcctcaac     840
   aagcgttcag ccgtctacga tgacgctaca ggggagctaa agagcagcaa gcacacgttt     900
   gggaagatat acaaagcgga agcgacatac ctgaacgccg aaatcaaaga ggtctccaaa     960
   aaaatcctcg gagttacggt cggtaaagaa ttgaagacat taagtggacg cgaactcgag    1020
   gctcaagtat tacgtgccgc ggaacgtgcc ctccataaac aggcctggca gcacccttca    1080
   gctaacccct cgcggtcgca gcgggaaaat accaacagtc atcttggaaa cgaacttgat    1140
   gggccgctcg aatcggatat gaccgtcgtc catcaagccg gatccgggtt cgtcggcgac    1200
   gatcgccacg ttcatgatgg cggtggatcc aggttgaatg aattgcacga atcgcgatcg    1260
   tcgaatgata gggaaaagct tagttcgccg agagccgaaa tttgcgaccg ctcgatgaat    1320
   cgggatcgca ccggctcagg tactttgtcg cgatga                              1356
<210> 101
   <211> 5310
   <212> DNA
   <213> Agrobacterium rhizogenes
<400> 101
   atgccaaccg acgacattgt aatgtccgat cccggaatgg ctgctgttga cacgtctgtc      60
   cctacgcgct tccagacaga tcttcgccag ttcagtaacc tgctggacga gagcaatatt     120
   gtggaatggg tcgtaaacca cgccgcaaat cgcaatgcgt gcttcggaca agaccaactt     180
   aagatttcgg cggctcgggc actcacgcac tacaaacggc cggtacaaga aatcgacctg     240
   ctattccaac aggtcgtcgc aagcgacaaa ctgacccggc ttgattctcc agatggccaa     300
   cctctctaca cctctacaga gctgagagct gcggaacaga atactgtacg tcatgtcacc     360
   aatctttccg cagaagaaag cttcggcgtt ccgggaacca ttgttgatat ggtcgccgat     420
   agaggcattt tcacaccgga gctcagagga gcattacatt atctgtctag gcgaaatcga     480
   gcttccacta tggtgggcgt agcgggttcc gcaaaaacaa gtgtacttgg agccctgaac     540
   gaagcggtgg acaggttcaa cgaggaagcg ctcccgaccg agaaaataaa gttgattgcc     600
   ttcgtaccca ccaaccgggc ggcccaggag cttcgtgaga aaggtcttcg cgacgtcgcg     660
   acgactttca aggcgaaaga ccgcaccatc ggaaaaaata ccatcgtcgt gatcgacgaa     720
   atgtcgatgg ccaagacgca ggagatcgcg caactaatcg agacagttgc aaatgccact     780
   tcctatcacc caaccgaaag gccgaaactg atatgtgtgg gagacgatcg gcaactgccc     840
   cctgttggac ctggtgatct gcttccgatt attatggagc aagccggctg ctatgaactc 900
   gtccagccgc ttcgacagct cgacgcgcgg tcgcgaatag aaacagaaaa gctcggacgt 960
   gacatacgac gcgatgccaa gagcgccgtc gggcactatc tcacagcgct cgaacaaatg 1020
   ggcctcgtcc atttcgtata tgctcctcct agcggctcaa acgtcagggc gtcagaccag 1080
   atcactgacc gcatgctgga ggagatgaat agagtttgcg accgatatcc ggtgtatgat 1140
   cgtcttgtga tggcacactc caacaggagc gttaatcgcg ccaatatcgc tcttcatcag 1200
   aggtttgttg acaaatccgg attgcaagac cagcaaatga gtgtgtggtc aagcgtaata 1260
   gatagtggaa gttcggaatc tggaatagat gagggttcgt cgagacgcgt tcagcaaagg 1320
   attaatctcg tcttgggaga caggattata ttcagcgaaa accgtctgca agcggacatc 1380
   cgcaatggta cttttgcaac tgtagtcgga cttaatagca cagaagcttc ccgagctgag 1440
   aatggcacat ctgctggggt tcgcattact gcaaggttag acggatctgg acgaaccgtc 1500
   acttggaccg atagcgagtt taagggtttt acctacggct atgctgctac aatttataag 1560
   agtcaggggg ccacagttga tcacgccatt ctcctctgtg atggtgctct ttcagataag 1620
   ctgacctatg tcggcctgac gaggcatcgc agtcgtctcg acgtcattgc ttctcccctc 1680
   gtggcaaaca atgttgaggc tctcagtgca aagctcactc agcaaagcgc gccaaataat 1740
   tccattcaat tcccagcgct cgaacctgct gccttccaag agcgctcgtc ggtttcgaac 1800
   gcgcacttgc taaggccgct cgaccagatg gaaatctctc caacgacgcc gctttccttc 1860
   caaggcagac aagaaagctc cttcggggag aaccagctgc acgaaccatc cacatcggat 1920
   gtggaaatgc cggaaagcgc ggacctttac ccgagcgcca ttgacttcga agttttccag 1980
   tcttctgtca tcacgactga agagcaaatg caaccggaat tctgcgggtc ggaatcgacg 2040
   gctaacacct cagcccacga aagccaccaa gtcacgatcg caacggtaac aaacctgggg 2100
   atagcgatcg agaaacggaa acgagcggcg gcaaaggaag aaatcgattc tcgcaaaaaa 2160
   atggcccgtc actcagttag ttctgccgag gaagttgaag atatcacttc cctgttcgaa 2220
   cgattgaaac tggcaacctc ggtcaccaac acagttcaac cgacgtatgc ggacgtcgat 2280
   catatcatcg gtactgatgg ccgagtgggt ccaataccgc accgggaaga tgatccggga 2340
   tcaaattggg aattgtcgtc aacgaacgcc ccctcaaacg tctcagagaa acgatcgacc 2400
   aaagactcag gagaaaaaaa tatggcttcg aatgaccaga aaatgcaagg cgctgactct 2460
   gcaggcagcg agcgtcattt ttcgcagact tccattgtgg aagccctgca ggctggcgat 2520
   cacaagaagc tcgtggaaac gctcgccgca accggctccc ttgtcgagaa ggcggacgcc 2580
   gggtcggcgc tgaaagcaat ggcggatgct tacgaaaggg atatcaaggc agatcccgaa 2640
   aaggtccgca tgctctatgc caatcggcgc gttgatgtag atgcgctggc caaggagatc 2700
   gaaaaacagg gccttgcatc cggtcgcctg agtggcgacc ctgtcagctt caaggcgttc 2760
   aacaacaagg atatccgttt cagggtcggc gacaggctca gcgtcaccag cagctacagg 2820
   aatctcattg ccggtgaagc cggccggatc gaaaagatcg aaggcaaatc cgttaccttc 2880
   aaacccgatg gcagcagtca gagcaagacc ttcgaggcca gcaccggcga gccgggtgac 2940
   aggggcgtca agggctacaa aatcgaaggc ccgatgacgg tcatcggctc cgcctatgaa 3000
   cttacgcgac atgatgacgg caaacgtgtt gaccaaatct atctcttgaa cgacgctgcc 3060
   catggcatca acgccaatag cgctttgctg agcaaggccg acgagcggac gcagcttttc 3120
   acttcgctca aggacaccgc cgacagggac atcttggcca ggcaactggg aaagccgggc 3180
   agcgagctct accaggacaa attcccagcc ttgccggact acacagccgc catcgatcgg 3240
   attgcaaaac gccaggaagt tgtcgcggca cgtggcgagg agaaaacttc cgccccgata 3300
   gacctggcgc cccaaacgcc gcccgacgct gccgatgcgg cagtcaataa ccagaaaaga 3360
   atcgtcgagg ccttgcaggc aggcgaccac aagaagcttg tggaaacgct agccgcaacc 3420
   ggctcccttg ttgagaacac aaatctcgta tcaacgttga aagcgatagc gcagacttat 3480
   gaaagagaca ttagtgcgga ccccgagaaa gtccgcgctc tctacgccaa caaacgtgat 3540
   catgtagatt tgcttgccga agagatcgaa aagcagggcc ttgcatccgg tcgcctgagt 3600
   ggcgaccctg tcagcttcaa ggcgtttaac aacaaggata tccgtttcag ggtcggtgac 3660
   aggctcagcg tcactagcag ctataggaat ctcattgccg gtgaagccgg ccggatcgaa 3720
   aagatcgaag gcaaatccgt taccttcaaa cccgatggca gcagtcagag caagaccttc 3780
   gaggccagca ccggcgagcc gggtgacagg ggtgtcaagg gctataagat tgaggggccc 3840
   acgacaatca tcggctccgc ctacgaaatc agaaatcacg acgatggcag gcgcgtcgat 3900
   caggtttatg tcttgcacag tgccattttc ggctcaaatg ccgcgagcgc cctgttgagc 3960
   aaggccgacg aacgaaccca ggttttcacc gtgcaaagcg acacggctga cagacacatc 4020
   ttggccagcc aactcagcaa gcctggcagc gagctctatc aggacaaatt cccagtctcg 4080
   ccggactacg cagccgcgat cgaccggatc gcgaaacgcg aggaacttag ggccggacgt 4140
   ggcgaggaaa gtcctactgt tcaaatggag ccggtgaacc aaacgcccta cgaggcggga 4200
   cattggacgc ctgacagtca aaaatctgtg gtggaagccc tgcaggctgg cgatcacaag 4260
   aagctcgtgg aaacgctcgc cgcaaccggc tcccttgtcg agaaagcgga cgccgggtcg 4320
   gcgctgaaag caatggcgga tgcttacgaa agggatatca aggcagatcc cgaaaaggtc 4380
   cgcatgctct atgccaatcg gcgcgttgat gtagatgcgc tggccaagga gatcgaaaaa 4440
   cagggccttg catccggtcg cctgagtggc gaccctgtca gcttcaaggc gttcaacaac 4500
   aaggatatcc gtttcagggt cggcgacagg ctcagcgtca ccagcagcta caggaatctc 4560
   attgccggtg aagccggccg gatcgaaaag atcgaaggca aatccgttac cttcaaaccc 4620
   gatggcagca gtcagagcaa gaccttcgag gccagcaccg gcgagccggg tgacaggggc 4680
   gtcaagggct acaaaatcga aggcccgatg acggtcatcg gctccgccta tgaacttacg 4740
   cgacatgatg acggcaaacg tgttgaccaa atctatctct tgaacgacgc tgcccatggc 4800
   atcaacgcca atagcgcttt gctgagcaag gccgacgagc ggacgcagct tttcacttcg 4860
   ctcaaggaca ccgccgacag ggacatcttg gccaggcaac tgggaaagcc gggcagcgag 4920
   ctctaccagg acaaattccc agccttgccg gactacacag ccgccatcga tcggattgca 4980
   aaacgccagg aagttgtcgc ggcacgtggc gaggtcaagc cgattgagcg caccgaagcg 5040
   cccgaaatac cccaggttca agctactatc gttggcgaga cctcgatcat tcggttgccc 5100
   gatcggataa aaaccaatcg cgagaagccc caagaacttg atgtagtctc gaaagaaggg 5160
   cacgaaacta caggtagtct gaggcagagt gtccaagaga gtaatgcagc accaaaacag 5220
   acttcgccga aggcggcaaa tgacgtggat cggctgacac gggattttga cgagcgcatc 5280
   cgcgtccgcg gggatggacg tggactctaa 5310
<210> 102
   <211> 1522
   <212> DNA
   <213> Bordetella bronchiseptica
<400> 102
   ctaccggcgc ggcagcgtta cccgtgtcgg cggctccaac ggctcgccat cgtccagaaa      60
   acacggctca tcgggcatcg gcaggcgctg ctgcccgcgc cgttcccatt cctccgtttc     120
   ggtcaaggct ggcaggtctg gttccatgcc cggaatgccg ggctggctgg gcggctcctc     180
   gccggggccg gtcggtagtt gctgctcgcc cggatacagg gtcgggatgc ggcgcaggtc     240
   gccatgcccc aacagcgatt cgtcctggtc gtcgtgatca accaccacgg cggcactgaa     300
   caccgacagg cgcaactggt cgcggggctg gccccacgcc acgcggtcat tgaccacgta     360
   ggccgacacg gtgccggggc cgttgagctt cacgacggag atccagcgct cggccaccaa     420
   gtccttgact gcgtattgga ccgtccgcaa agaacgtccg atgagcttgg aaagtgtctt     480
   ctggctgacc accacggcgt tctggtggcc catctgcgcc acgaggtgat gcagcagcat     540
   tgccgccgtg ggtttcctcg caataagccc ggcccacgcc tcatgcgctt tgcgttccgt     600
   ttgcacccag tgaccgggct tgttcttggc ttgaatgccg atttctctgg actgcgtggc     660
   catgcttatc tccatgcggt aggggtgccg cacggttgcg gcaccatgcg caatcagctg     720
   caacttttcg gcagcgcgac aacaattatg cgttgcgtaa aagtggcagt caattacaga     780
   ttttctttaa cctacgcaat gagctattgc ggggggtgcc gcaatgagct gttgcgtacc      840
   cccctttttt aagttgttga tttttaagtc tttcgcattt cgccctatat ctagttcttt      900
   ggtgcccaaa gaagggcacc cctgcggggt tcccccacgc cttcggcgcg gctccccctc      960
   cggcaaaaag tggcccctcc ggggcttgtt gatcgactgc gcggccttcg gccttgccca    1020
   aggtggcgct gcccccttgg aacccccgca ctcgccgccg tgaggctcgg ggggcaggcg     1080
   ggcgggcttc gcccttcgac tgcccccact cgcataggct tgggtcgttc caggcgcgtc    1140
   aaggccaagc cgctgcgcgg tcgctgcgcg agccttgacc cgccttccac ttggtgtcca    1200
   accggcaagc gaagcgcgca ggccgcaggc cggaggcttt tccccagaga aaattaaaaa    1260
   aattgatggg gcaaggccgc aggccgcgca gttggagccg gtgggtatgt ggtcgaaggc    1320
   tgggtagccg gtgggcaatc cctgtggtca agctcgtggg caggcgcagc ctgtccatca    1380
   gcttgtccag cagggttgtc cacgggccga gcgaagcgag ccagccggtg gccgctcgcg    1440
   gccatcgtcc acatatccac gggctggcaa gggagcgcag cgaccgcgca gggcgaagcc    1500
   cggagagcaa gcccgtaggg gg                                              1522

## Description

### FIELD OF THE DISCLOSURE

The present disclosure relates generally to the field of plant molecular biology, including genetic manipulation of plants. More specifically, the present disclosure pertains to methods and compositions for plant transformation comprising vectors that can be used to generate co-integrate vectors or as a ternary helper vector.

### BACKGROUND

Agrobacterium, a natural plant pathogen, has been widely used for the transformation of dicotyledonous plants and more recently for transformation of monocotyledonous plants. The advantage of the Agrobacterium-mediated gene transfer system is that it offers the potential to regenerate transgenic cells at relatively high frequencies without a significant reduction in plant regeneration rates. Moreover, the process of DNA transfer to the plant genome is well characterized relative to other DNA delivery methods. DNA transferred via Agrobacterium is less likely to undergo any major rearrangements than is DNA transferred via direct delivery, and it integrates into the plant genome often in single or low copy numbers.

The most commonly used Agrobacterium-mediated gene transfer system is a binary transformation vector system where the Agrobacterium has been engineered to include a disarmed, or nononcogenic, Ti helper plasmid, which encodes the vir functions necessary for DNA transfer, and a much smaller separate plasmid called the binary vector plasmid, which carries the transferred DNA, or the T-DNA region. The T-DNA is defined by sequences at each end, called T-DNA borders, which play an important role in the production of T-DNA and in the transfer process.

Binary vectors are vectors in which the virulence genes are placed on a different plasmid than the one carrying the T-DNA region (Bevan, 1984, Nucl. Acids. Res. 12: 8711-8721). The development of T-DNA binary vectors has made the transformation of plant cells easier as they do not require recombination. The finding that some of the virulence genes exhibited gene dosage effects (Jin et al., J. Bacteriol. (1987) 169:4417-4425) led to the development of a superbinary vector, which carried additional virulence genes (Komari, T., et al., Plant Cell Rep. (1990), 9:303-306). These early superbinary vectors carried a large "vir" fragment (∼14.8 kbp) from the hypervirulenece Ti plasmid, pTiBo542, which had been introduced into a standard binary vector (*ibid*). The superbinary vectors resulted in vastly improved plant transformation. For example, Hiei, Y., et al. (Plant J. (1994) 6:271-282) described efficient transformation of rice by Agrobacterium, and subsequently there were reports of using this system for maize, barley and wheat (Ishida, Y., et al., Nat. Biotech. (1996) 14:745-750; Tingay, S., et al., Plant J. (1997) 11:1369-1376; and Cheng, M., et al., Plant Physiol. (1997) 115:971-980; see also U.S. Pat. No. 5,591,616 to Hiei et al). Examples of prior superbinary vectors include pTOK162 (Japanese Patent Appl. (Kokai) No. 4-222527, EP-A-504,869, EP-A-604,662, and U.S. Pat. No. 5,591,616) and pTOK233 (see Komari, T., ibid*;* and Ishida, Y., et al., ibid).

However, the design of prior superbinary vectors has several drawbacks. For example, the large vir fragment and vector backbone carry significant non-essential DNA. In addition, the use of the tetA tetracycline resistance gene both retards bacterial growth and is also a poor selectable marker for *Agrbacterium* strain C58 since this strain already has partial resistance to the antibiotic. In addition, a large origin of replication / partitioning region (derived from RK2, with a size of about ∼15kbp) resulted in a fairly large vector which was not easily amenable to further manipulation and is associated with varying degrees of instability.

The limitation with regard to further manipulation was further exacerbated by the fact that reconstitution of the super binary T-DNA vector required homologous recombination between the "super-binary" vector and the T-DNA vector in a recipient *Agrobacterium* strain such as LBA4404 or C58, which is receptive to homologous recombination. The homologous recombination process is relatively inefficient and the resulting cointegrated vector often contained unexpected deletions. Moreover, screening of many candidate clones was required to identify suitable *Agrobacterium* isolates for plant transformation. In strains such as C58 and EHA101, spontaneous mutants resistant to tetracycline are known to occur at high frequency (Luo, Z.Q. and Farrand, S.K., (1999) J. Bacteriol. 181:618-626), which further hindered the identification of true recombinant clones.

Despite advances in plant molecular biology, particularly plant transformation and vectors useful in same, there remains a need in the art of transformation to produce transgenic plants efficiently. In particular, there is a need for improved superbinary vectors that have vir genes of optimal size with minimal non-essential sequences, an optimal mix of vir genes for improved virulence, a smaller origin of replication, and improved selectable markers for *Agrobacterium* selection. Ideally, such improved vectors could be easily used to generate co-integrate vectors or be used as a ternary helper vector for plant transformation. These needs and others are addressed by the present disclosure.

### BRIEF SUMMARY

The present disclosure comprises methods and compositions for vectors comprising vir genes.

The invention provides a vector comprising:
(a) an origin of replication for propagation and stable maintenance in *Escherichia coli;*
(b) an origin of replication for propagation and stable maintenance in *Agrobacterium* spp.;
(c) a selectable marker gene; and
(d) *Agrobacterium* spp. virulence genes:
   (i) *virB1-B11* having SEQ ID NOS: 4-14, respectively, or *r-virB1-B11* having SEQ ID NOS: 80-90, respectively,
   (ii) *virCl-C2* having SEQ ID NOS: 16-17, respectively, or *r-virC1-C2* having SEQ ID NOS: 92-93, respectively,
   (iii) *virD1-D2* having SEQ ID NOS: 18-19, respectively, or *r-virD1-D2* having SEQ ID NOS: 94-95, respectively, and
   (iv) *virG* having SEQ ID NO: 15 or *r-virG* having SEQ ID NO: 91,
   wherein the vector comprising *r-virB1-B11* having SEQ ID NOS: 80-90, respectively, *r-virC1-C2* having SEQ ID NOS: 92-93, respectively, *r-virD1-D2* having SEQ ID NOS: 94-95, respectively, and *r-virG* having SEQ ID NO: 91 further comprises *r-galls* having SEQ ID NO: 101.

The invention further provides a vector comprising:
(a) an origin of replication for propagation in *Escherichia coli* having SEQ ID NO: 2;
(b) an origin of replication for propagation in *Agrobacterium* spp. having SEQ ID NO: 3;
(c) a selectable marker gene having SEQ ID NO: 1; and:
   (I)(d) *Agrobacterium* spp. virulence genes:
      (i) *virB1-B11* having SEQ ID NOS: 4-14, respectively, or *r-virB1-B11* having SEQ ID NOS: 80-90, respectively,
      (ii) *virCl-C2* having SEQ ID NOS: 16-17, respectively, or *r-virC1-C2* having SEQ ID NOS: 92-93, respectively,
      (iii) *virD1-D2* having SEQ ID NOS: 18-19, respectively, or *r-virD1-D2* having SEQ ID NOS: 94-95, respectively, and
      (iv) *virG* having SEQ ID NO: 15 or *r-virG* having SEQ ID NO: 91,
         wherein the vector comprising *r-virB1-B11* having SEQ ID NOS: 80-90, respectively, *r-virC1-C2* having SEQ ID NOS: 92-93, respectively, *r-virD1-D2* having SEQ ID NOS: 94-95, respectively, and *r-virG* having SEQ ID NO: 91 further comprises *r-galls* having SEQ ID NO: 101;
   (II)(d) *Agrobacterium* spp. virulence genes:
      (i) *virB1-B11* having SEQ ID NOS: 4-14, respectively, or *r-virB1-B11* having SEQ ID NOS: 80-90, respectively,
      (ii) *virCl-C2* having SEQ ID NOS: 16-17, respectively, or *r-virC1-C2* having SEQ ID NOS: 92-93, respectively,
      (iii) *virD1-D5* having SEQ ID NOS: 18-22, respectively, or *r-virD1-D5* having SEQ ID NOS: 94-98, respectively,
      (iv) *virE1-E3* having SEQ ID NOS: 23-25, respectively, or *r-virE3* having SEQ ID NO: 100, and
      (v) *virG* having SEQ ID NO: 15 or *r-virG* having SEQ ID NO: 91,
         wherein the vector comprising *r-virB1-B11* having SEQ ID NOS: 80-90, respectively, *r-virC1-C2* having SEQ ID NOS: 92-93, respectively, *r-virD1-D5* having SEQ ID NOS: 94-98, respectively, *r-virE3* having SEQ ID NO: 100, and *r-virG* having SEQ ID NO: 91 further comprises *r-galls* having SEQ ID NO: 101; or
   (III)(d) *Agrobacterium* spp. virulence genes:
      (i) *virA* having SEQ ID NO: 26 or *r-virA* having SEQ ID NO: 79,
      (ii) *virB1-B11* having SEQ ID NOS: 4-14, respectively, or *r-virB1-B11* having SEQ ID NOS: 80-90, respectively,
      (iii) *virCl-C2* having SEQ ID NOS: 16-17, respectively, or *r-virC1-C2* having SEQ ID NOS: 92-93, respectively,
      (iv) *virD1-D5* having SEQ ID NOS: 18-22, respectively, or *r-virD1-D5* having SEQ ID NOS: 94-98, respectively,
      (v) *virE1-E3* having SEQ ID NOS: 23-25, respectively, or *r-virE3* having SEQ ID NO: 100,
      (vi) *virG* having SEQ ID NO: 15 or *r-virG* having SEQ ID NO: 91, and
      (vii) *virJ* having SEQ ID NO: 27,
         wherein the vector comprising *r-virA* having SEQ ID NO: 79, *r-*vir*B1-B11* having SEQ ID NOS: 80-90, respectively, *r-virC1-C2* having SEQ ID NOS: 92-93, respectively, *r-virD1-D5* having SEQ ID NOS: 94-98, respectively, *r-virE3* having SEQ ID NO: 100, *r-virG* having SEQ ID NO: 91, and *virJ* having SEQ ID NO: 27 further comprises *r-galls* having SEQ ID NO: 101.

Yet further provided by the invention is a method for transformation of a plant comprising the steps of:
(a) contacting a tissue from the plant with an *Agrobacterium* strain or an *Ochrobactrum* strain comprising a first vector as described above, and a second vector comprising T-DNA borders and a polynucleotide sequence of interest for transfer to the plant;
(b) co-cultivating the tissue with the *Agrobacterium* strain or the *Ochrobactrum* strain; and
(c) regenerating a transformed plant from the tissue that expresses the polynucleotide sequence of interest.

The invention further provides a kit comprising:
(a) a vector as described above; and
(b) instructions for use in transformation of a plant using *Agrobacterium* or *Ochrobactrum.*

Further disclosed herein is a vector comprising: (a) an origin of replication for propagation and stable maintenance in *Escherichia coli;* (b) an origin of replication for propagation and stable maintenance in *Agrobacterium* spp.; (c) a selectable marker gene; and (d) *Agrobacterium* spp. virulence genes *virB1-B11; virC1-C2; virD1-D2;* and *virG* genes. In an aspect, the vector further comprises *Agrobacterium* spp. virulence genes *virA, virD3, virD4, virD5, virE1, virE2, virE3, virH, virH1, virH2, virK, virL, virM, virP,* or *virQ,* or combinations thereof. In an aspect, the vector comprises *Agrobacterium* sp. virulence genes *virB1-B11* (SEQ ID NOS: 4-14, respectively), *virCl-*C2 (SEQ ID NOS: 16-17, respectively); *virD1-D2* (SEQ ID NOS: 18-19, respectively), and *virG* (SEQ ID NO: 15) genes. In another aspect, the vector comprises *Agrobacterium* sp. virulence genes *virA* (SEQ ID NO: 26), *virB1-B11* (SEQ ID NOS: 4-14, respectively), *virCl-C2* (SEQ ID NOS: 16-17, respectively); *virD1-D5* (SEQ ID NOS: 18-22, respectively), *virE1-E3* (SEQ ID NOS: 23-25), *virG* (SEQ ID NO: 15), and *virJ* (SEQ ID NO: 27) genes.

In an aspect, the present disclosure further provides methods for transformation of a plant comprising the steps of: (a) contacting a tissue from the plant with an *Agrobacterium* strain comprising a first vector comprising: (i) an origin of replication for propagation and stable maintenance in *Escherichia coli;* (ii) an origin of replication for propagation and stable maintenance in *Agrobacterium* spp.; (iii) a selectable marker gene; and (iv) *Agrobacterium* spp.virulence genes *virB1-B11; virC1-C2; virD1-D2*; and *virG* genes, and a second vector comprising T-DNA borders and a polynucleotide sequence of interest for transfer to the plant; (b) co-cultivatiing the tissue with the *Agrobacterium;* and (c) regenerating a transformed plant from the tissue that expresses the polynucleotide sequence of interest.

In an aspect, the present disclosure further provides kits comprising: (a) a vector comprising: (i) an origin of replication for propagation and stable maintenance in *Escherichia coli;* (ii) an origin of replication for propagation and stable maintenance in *Agrobacterium* spp.; (iii) a selectable marker gene; and (iv) *Agrobacterium* spp.virulence genes *virB1-B11; virC1-C2; virD1-D2;* and *virG* genes; and (b) instructions for use in transformation of a plant using *Agrobacterium.*

The present disclosure comprises methods and compositions for vectors comprising vir genes. In various aspects, the present disclosure provides a vector comprising: (a) an origin of replication for propagation and stable maintenance in *Escherichia coli;* (b) an origin of replication for propagation and stable maintenance in *Agrobacterium* spp.; (c) a selectable marker gene; and (d) Rhizobiaceae virulence genes *virB1-B11 or r-*vir*B1-B11, virC1-C2* or *r-virC1-C2, virD1-D2* or *r-virD1-D2,* and *virG* or *r-virG,* or variants and derivatives thereof, wherein the vector comprising the virulence genes *r-*vir*B1-B11, r-virC1-C2, r-virD1-D2,* and *r-virG* further comprises a *r-galls* virulence gene, or variants and derivatives thereof. In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are vir*B1*-virB11 virulence genes having SEQ ID NOS: 4-14, respectively, or *r-*vir*B1-B11* virulence genes having SEQ ID NOS: 80-90, respectively, or variants and derivatives thereof. In an aspect, the Rhizobiaceae virulence genes are *virC1-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virEl, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a r-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof In an aspect, the Rhizobiaceae virulence gene is a *virL* virulence gene having SEQ ID NO: 46, or variants and derivatives thereof. In an aspect, the Rhizobiaceae virulence gene is a *virM* virulence gene having SEQ ID NO: 47, or variants and derivatives thereof. In an aspect, the Rhizobiaceae virulence gene is a *virP* virulence gene having SEQ ID NO: 48, or variants and derivatives thereof In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA,* r*-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof. In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is derived from the pBBR1 origin of replication. In an aspect, the origin of replication for propagation and stable maintenance in *Agrobacterium* spp. is an origin of replication having any one of SEQ ID NOS: 3, 37, 38, 53, 57, 58, 59, 60 or 102, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *nptlgene,* a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *nptl* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variant and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, a *pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof In an aspect, the vector has SEQ ID NO: 36, or variants and fragments thereof.

In another aspect, the disclosure further provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1, or variants and fragments thereof; and (d) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virCl-C2* virulence genes having SEQ ID NOS: 16-17, respectively or *r-virC1-C2* virulence genes having SEQ ID NOS: 92-93, respectively, *virD1-D2* virulence genes having SEQ ID NOS: 18-19, respectively or *r-virD1-D2* virulence genes having SEQ ID NOS: 94-95, respectively, and a *virG* virulence gene having SEQ ID NO: 15 or a *r-virG* virulence gene having SEQ ID NO: 91, or variants and derivatives thereof, wherein the vector comprising the virulence genes *r-*vir*B1-B11, r-virC1-C2, r-virD1-D2,* and *r-virG* further comprises a *r-galls* virulence gene having SEQ ID NO: 101, or variants and derivatives thereof.

In another aspect, the disclosure further provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1, or variants and fragments thereof; and (d) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virCl-C2* virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a *r-virE3* virulence gene having SEQ ID NO: 100, and a *virG* virulence gene having SEQ ID NO: 15 or a *r-virG* virulence gene having SEQ ID NO: 91, or variants and derivatives thereof, wherein the vector comprising the virulence genes *r-*vir*B1-B11, r-virC1-C2, r-virD1-D5, r-virE3,* and *r-virG* further comprises a *r-galls* virulence gene having SEQ ID NO: 101, or variants and derivatives thereof

In another aspect, the disclosure further provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1; and (d) virulence genes comprising *Agrobacterium* spp. virulence genes a *virA* virulence gene having SEQ ID NO: 26 or a *r-virA* virulence gene having SEQ ID NO: 79, *virB1-B11* virulence genes having SEQ ID NOS: 4-14, respectively or *r-virB1-B11* virulence genes having SEQ ID NOS: 80-90, respectively, *virCl-*C2 virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a r-virE3 virulence gene having SEQ ID NOS: 100, and a *virG* virulence gene having SEQ ID NO: 15 or a *r-virG* virulence gene having SEQ ID NO: 91, or variants and derivatives thereof, wherein the vector comprising the virulence genes *r-virA, r-*vir*B1-B11, r-virCl-C2, r-virD1-D5, r-*vir*E3, and r-virG* further comprises a *r-galls* virulence gene having SEQ ID NO: 101, or variants and derivatives thereof.

In an aspect, the present disclosure further provides a method for transformation of a plant comprising the steps of: (a) contacting a tissue from the plant with an *Agrobacterium* strain or an *Ochrobactrum* strain comprising a first vector comprising: (i) an origin of replication for propagation and stable maintenance in *Escherichia coli;* (ii) an origin of replication for propagation and stable maintenance in *Agrobacterium* spp.; (iii) a selectable marker gene; and (iv) Rhizobiaceae virulence genes *virB1-B11 or r-*vir*B1-B11, virC1-C2* or *r-virC1-C2, virD1-D2* or *r-virD1-D2,* and *virG* or *r-virG,* or variants and derivatives thereof, wherein the vector comprising the virulence genes *r-*vir*B1-B11, r-virC1-C2, r-virD1-D2,* and *r-virG* further comprises a *r-galls* virulence gene, or variants and derivatives thereof, and a second vector comprising T-DNA borders and a polynucleotide sequence of interest for transfer to the plant; (b) co-cultivating the tissue with the *Agrobacterium* strain or the *Ochrobactrum* strain; and (c) regenerating a transformed plant from the tissue that expresses the polynucleotide sequence of interest. In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virC1-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virE1, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a virM virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof. In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, a *pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a method for transformation of a plant comprising the steps of: (a) contacting a tissue from the plant with an *Agrobacterium* strain or an *Ochrobactrum* strain comprising a first vector comprising: (i) an origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1, or variants and fragments thereof; and (iv) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virC1-C2* virulence genes having SEQ ID NOS: 16-17, respectively or *r-virC1-C2* virulence genes having SEQ ID NOS: 92-93, respectively, *virD1-D2* virulence genes having SEQ ID NOS: 18-19, respectively or *r-virD1-D2* virulence genes having SEQ ID NOS: 94-95, respectively, and a *virG* virulence gene having SEQ ID NO: 15 or a *r-virG* virulence gene having SEQ ID NO: 91, or variants and derivatives thereof, wherein the vector comprising the virulence genes *r-*vir*B1-B11, r-virC1-C2, r-virD1-D2,* and *r-virG* further comprises a *r-galls* virulence gene having SEQ ID NO: 101, or variants and derivatives thereof, and a second vector comprising T-DNA borders and a polynucleotide sequence of interest for transfer to the plant; (b) co-cultivating the tissue with the *Agrobacterium* strain or the *Ochrobactrum* strain; and (c) regenerating a transformed plant from the tissue that expresses the polynucleotide sequence of interest. In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are vir*B1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virEl, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a virM virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, a *pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a method for transformation of a plant comprising the steps of: (a) contacting a tissue from the plant with an *Agrobacterium* strain or an *Ochrobactrum* strain comprising a first vector comprising: (i) n origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1, or variants and fragments thereof; and (iv) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virC1-C2* virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a *r-virE3* virulence gene having SEQ ID NO: 100, and a *virG* virulence gene having SEQ ID NO: 15 or a *r-virG* virulence gene having SEQ ID NO: 91, or variants and derivatives thereof, wherein the vector comprising the virulence genes *r-*vir*B1-B11, r-virC1-C2, r-virD1-D5, r-virE3,* and *r-virG* further comprises a *r-galls* virulence gene having SEQ ID NO: 101, or variants and derivatives thereof, and a second vector comprising T-DNA borders and a polynucleotide sequence of interest for transfer to the plant; (b) co-cultivating the tissue with the *Agrobacterium* strain or the *Ochrobactrum* strain; and (c) regenerating a transformed plant from the tissue that expresses the polynucleotide sequence of interest. In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virEl, virE2, virE3, virH, virH1, virH2, virK, virL, virM, virP, virQ, r-virA* , *r-virD3, r-virD4, r-virD5,* or *r-virE3, r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a virM virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, a *pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a method for transformation of a plant comprising the steps of: (a) contacting a tissue from the plant with an *Agrobacterium* strain or an *Ochrobactrum* strain comprising a first vector comprising: (i) an origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1; and (iv) virulence genes comprising *Agrobacterium* spp. virulence genes a *virA* virulence gene having SEQ ID NO: 26 or a *r-virA* virulence gene having SEQ ID NO: 79, *virB1-B11* virulence genes having SEQ ID NOS: 4-14, respectively or *r-virB1-B11* virulence genes having SEQ ID NOS: 80-90, respectively, *virC1-C2* virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a r-virE3 virulence gene having SEQ ID NOS: 100, and a *virG* virulence gene having SEQ ID NO: 15 or a *r-virG* virulence gene having SEQ ID NO: 91, or variants and derivatives thereof, wherein the vector comprising the virulence genes *r-virA, r-*vir*B1-B11, r-virC1-C2, r-virD1-D5, r-virE3, and r-virG* further comprises a *r-galls* virulence gene having SEQ ID NO: 101, or variants and derivatives thereof, and a second vector comprising T-DNA borders and a polynucleotide sequence of interest for transfer to the plant; (b) co-cultivating the tissue with the *Agrobacterium* strain or the *Ochrobactrum* strain; and (c) regenerating a transformed plant from the tissue that expresses the polynucleotide sequence of interest. In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are vir*B1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virE1, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a virM virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColE1 origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, a *pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a kit comprising: (a) a vector comprising: (i) an origin of replication for propagation and stable maintenance in *Escherichia coli;* (ii) an origin of replication for propagation and stable maintenance in *Agrobacterium* spp.; (iii) a selectable marker gene; and (iv) Rhizobiaceae virulence genes *virB1-B11 or r-*vir*B1-B11, virCl-C2* or *r-virC1-C2, virD1-D2* or *r-virD1-D2,* and *virG* or *r-virG,* or variants and derivatives thereof, wherein the vector comprising the virulence genes *r-*vir*B1-B11, r-virC1-C2, r-virD1-D2,* and *r-virG* further comprises a *r-galls* virulence gene, or variants and derivatives thereof; and (b) instructions for use in transformation of a plant using *Agrobacterium* or *Ochrobactrum.* In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virE1, virE2, virE3, virH, virH1, virH2, virK, virL, virM, virP, virQ, r-virA* , *r-virD3, r-virD4, r-virD5, r-virE3,* or *r-virF* or variants and derivatives thereof In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a virMvirulence gene having SEQ ID NO: 47, or variants and derivatives thereof In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof. In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL (strA)* gene, *a pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a kit comprising: (a) a vector comprising: (i) an origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1, or variants and fragments thereof; and (iv) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virCl-C2* virulence genes having SEQ ID NOS: 16-17, respectively or *r-virC1-C2* virulence genes having SEQ ID NOS: 92-93, respectively, *virD1-D2* virulence genes having SEQ ID NOS: 18-19, respectively or *r-virD1-D2* virulence genes having SEQ ID NOS: 94-95, respectively, and a *virG* virulence gene having SEQ ID NO: 15 or a *r-virG* virulence gene having SEQ ID NO: 91, or variants and derivatives thereof, wherein the vector comprising the virulence genes *r-virB1-B11, r-virC1-C2, r-virD1-D2,* and *r-virG* further comprises a r-*galls* virulence gene having SEQ ID NO: 101, or variants and derivatives thereof; and (b) instructions for use in transformation of a plant using *Agrobacterium* or *Ochrobactrum.* In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virE1, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a *virM* virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL* (*strA*) gene, *a pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a kit comprising: (a) a vector comprising: (i) an origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1, or variants and fragments thereof; and (iv) virulence genes comprising *Agrobacterium* spp. virulence genes *virB1-B11* virulence genes having SEQ ID NOS: 4-14, respectively or *r-virB1-B11* virulence genes having SEQ ID NOS: 80-90, respectively, *virCl-C2* virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a *r-virE3* virulence gene having SEQ ID NO: 100, and a *virG* virulence gene having SEQ ID NO: 15 or a *r-virG* virulence gene having SEQ ID NO: 91, or variants and derivatives thereof, wherein the vector comprising the virulence genes *r-virB1-B11, r-virC1-C2, r-virD1-D5, r-virE3,* and *r-virG* further comprises a *r-galls* virulence gene having SEQ ID NO: 101, or variants and derivatives thereof; and (b) instructions for use in transformation of a plant using *Agrobacterium* or *Ochrobactrum.* In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virEl, virE2, virE3, virH, virH1, virH2, virK, virL, virM, virP, virQ, r-virA* , *r-virD3, r-virD4, r-virD5, r-virE3,* or *r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a *virM* virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* or *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variants and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL* (*strA*) gene, *a pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

In an aspect, the present disclosure further provides a kit comprising: (a) a vector comprising: (i) an origin of replication for propagation in *Escherichia coli* having SEQ ID NO: 2, or variants and fragments thereof; (ii) an origin of replication for propagation in *Agrobacterium* spp. having SEQ ID NO: 3, or variants and fragments thereof; (iii) a selectable marker gene having SEQ ID NO: 1; and (iv) virulence genes comprising *Agrobacterium* spp. virulence genes a *virA* virulence gene having SEQ ID NO: 26 or a *r-virA* virulence gene having SEQ ID NO: 79, *virB1-B11* virulence genes having SEQ ID NOS: 4-14, respectively or *r-virB1-B11* virulence genes having SEQ ID NOS: 80-90, respectively, *virC1-C2* virulence genes having SEQ ID NOS: 16-17, respectively or *r-virC1-C2* virulence genes having SEQ ID NOS: 92-93, respectively, *virD1-D5* virulence genes having SEQ ID NOS: 18-22, respectively or *r-virD1-D5* virulence genes having SEQ ID NOS: 94-98, respectively, *virE1-E3* virulence genes having SEQ ID NOS: 23-25, respectively or a *r*-virE3 virulence gene having SEQ ID NOS: 100, and a *virG* virulence gene having SEQ ID NO: 15 or a *r-virG* virulence gene having SEQ ID NO: 91, or variants and derivatives thereof, wherein the vector comprising the virulence genes *r-virA, r-virB1-B11, r-virC1-C2, r-virD1-D5, r*-vir*E3*, and *r-virG* further comprises a *r-galls* virulence gene having SEQ ID NO: 101, or variants and derivatives thereof; and (b) instructions for use in transformation of a plant using *Agrobacterium* or *Ochrobactrum.* In an aspect, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. virulence genes. In an aspect, the Rhizobiaceae virulence genes are *Agrobacterium* spp. virulence genes. In an aspect, the *Agrobacterium* spp. virulence genes are *Agrobacterium albertimagni, Agrobacterium larrymoorei, Agrobacterium radiobacter, Agrobacterium rhizogenes, Agrobacterium rubi, Agrobacterium tumefaciens,* or *Agrobacterium vitis* virulence genes. In an aspect, the *Agrobacterium* spp. virulence genes are *Agrobacterium rhizogenes* or *Agrobacterium tumefaciens* virulence genes. In an aspect, the *Agrobacterium* spp. virulence genes are *Agrobacterium rhizogenes* virulence genes. In an aspect, the *Agrobacterium* spp. virulence genes are *Agrobacterium tumefaciens* virulence genes. In an aspect, the Rhizobiaceae virulence genes are *virB1*-virB11 virulence genes having SEQ ID NOS: 4-14, respectively, or *r-virB1-B11* virulence genes having SEQ ID NOS: 80-90, respectively, or variants and derivatives thereof. In an aspect, the Rhizobiaceae virulence genes are *virCl-C2* virulence genes having SEQ ID NOS: 16-17, respectively, or *r-virC1-C2* virulence genes having SEQ ID NOS: 92-93, respectively, or variants and derivatives thereof. In an aspect, the Rhizobiaceae virulence genes are *virD1-D2* virulence genes having SEQ ID NOS: 18-19, respectively, or *r- virD1-D2* virulence genes having SEQ ID NOS: 94-95, respectively, or variants and derivatives thereof. In an aspect, the Rhizobiaceae virulence gene is a *virG* virulence gene having SEQ ID NO: 15, or a *r-virG* virulence gene having SEQ ID NO: 91, or variants and derivatives thereof. In an aspect, the Rhizobiaceae virulence gene is a *r-galls* virulence gene having SEQ ID NO: 101, or variants and derivatives thereof. In an aspect, the vector further comprises one or more of Rhizobiaceae virulence genes *virA, virD3, virD4, virD5, virEl, virE2, virE3, virH, virH1, virH2, virK, virL, virM, virP, virQ, r-virA* , *r-virD3, r-virD4, r-virD5,* or *r-virE3, r-virF* or variants and derivatives thereof. In an aspect, the Rhizobiaceae virulence gene is a *virA* virulence gene having SEQ ID NO: 26 or a *r-virA* virulence gene having SEQ ID NO: 79, or variants and derivatives thereof. In an aspect, the Rhizobiaceae virulence genes are *virD3-D5* virulence genes having SEQ ID NOS: 20-22, respectively, or *r-virD3-D5* virulence genes having SEQ ID NO: 96-98, respectively, or variants and derivatives thereof. In an aspect, the Rhizobiaceae virulence genes are *virE1-E3* virulence genes having SEQ ID NOS: 23-25, respectively, or a *r*-virE3 virulence gene having SEQ ID NO: 100, or variants and derivatives thereof. In an aspect, the Rhizobiaceae virulence genes are *virH-H1* virulence genes having, SEQ ID NOS: 42-43, respectively, or variants and derivatives thereof. In an aspect, the Rhizobiaceae virulence gene is a *virK* virulence gene having SEQ ID NO: 45, or variants and derivatives thereof. In an aspect, the Rhizobiaceae virulence gene is a *virL* virulence gene having SEQ ID NO: 46, or variants and derivatives thereof. In an aspect, the Rhizobiaceae virulence gene is a *virM* virulence gene having SEQ ID NO: 47, or variants and derivatives thereof. In an aspect, the Rhizobiaceae virulence gene is a *virP* virulence gene having SEQ ID NO: 48, or variants and derivatives thereof. In an aspect, the Rhizobiaceae virulence gene is a *virQ* virulence gene having SEQ ID NO: 49, or variants and derivatives thereof. In an aspect, the vector further comprises the Rhizobiaceae virulence genes *virD3-D5* and *virE1-E3* or *r-virD3-D5* and *r-vir E3,* or variants and derivatives thereof. In an aspect, the vector further comprises the Rhizobiaceae virulence genes *virA, virD3-D5,* and *virE1-E3,* or *r-virA, r-virD3-D5,* and *r-virE3,* or variants and derivatives thereof. In an aspect, the origin of replication for propagation and stable maintenance in *Escherichia coli* is derived from a Col E1, a pSC101, a p15A, or a R6K origin of replication, or functional variants and derivatives thereof. In an aspect, the origin of replication for propagation and stable maintenance in *Escherichia coli* is derived from a Col E1 origin of replication. In an aspect, the origin of replication derived from the ColEl origin of replication has SEQ ID NO: 2, or variants and fragments thereof. In an aspect, the origin of replication for propagation and stable maintenance in *Escherichia coli* is derived from a pSC101 origin of replication. In an aspect, the origin of replication derived from the pSC101 origin of replication has SEQ ID NO: 50, or variants and fragments thereof. In an aspect, the origin of replication for propagation and stable maintenance in *Escherichia coli* is derived from a p15A origin of replication. In an aspect, the origin of replication derived from the p15A origin of replication has SEQ ID NO: 51, or variants and fragments thereof In an aspect, the origin of replication for propagation and stable maintenance in *Escherichia coli* is derived from a R6K origin of replication. In an aspect, the origin of replication derived from the R6K origin of replication has SEQ ID NO: 52, or variants and fragments thereof. In an aspect, the origin of replication for propagation and stable maintenance in *Agrobacterium* spp. is a high copy number origin of replication. In an aspect, the origin of replication for propagation and stable maintenance in *Agrobacterium* spp. is an intermediate copy number origin of replication. In an aspect, the origin of replication for propagation and stable maintenance in *Agrobacterium* spp. is a low copy number origin of replication. In an aspect, the origin of replication for propagation and stable maintenance in *Agrobacterium* spp. is derived from a pRi, a pVS1, a pRSF1010, a pRK2, a pSa, or a pBBR1 origin of replication. In an aspect, the origin of replication for propagation and stable maintenance in *Agrobacterium* spp. is a variant of the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In an aspect, the origin of replication for propagation and stable maintenance in *Agrobacterium* spp. is an origin of replication having any one of SEQ ID NOS: 3, 37, 38, 53, 57, 58, 59, or 60, or variants and fragments thereof. In an aspect, the origin of replication for propagation and stable maintenance in *Agrobacterium* spp. is a repABC compatible origin of replication. In an aspect, the repABC compatible origin of replication has any one of SEQ ID NOS: 57, 58, 59, or 60, or variants and fragments thereof. In an aspect, the origin of replication for propagation and stable maintenance in *Escherichia coli* and the origin of replication for propagation and stable maintenance in *Agrobacterium* spp. are the same origin of replication. In an aspect, the origin of replication is derived from a pRK2 origin of replication, from a pSa origin of replication, or a pRSF1010 origin of replication. In an aspect, the origin of replication is derived from the pRK2 origin of replication. In an aspect, the pRK2 origin of replication has SEQ ID NO: 38, or variants and fragments thereof. In an aspect, the pRK2 origin of replication is a mini or micro pRK2 origin of replication. In an aspect, the pRK2 origin of replication is a micro pRK2 origin of replication. In an aspect, the micro pRK2 origin of replication has SEQ ID NO: 54, or variants and fragments thereof. In an aspect, the pRK2 origin of replication is a mini pRK2 origin of replication. In an aspect, the mini pRK2 has SEQ ID NO: 66, or variants and fragments thereof. In an aspect, the pRK2 origin of replication comprises the *trfA* and *OriV* sequences. In an aspect, the pRK2 origin of replication comprises SEQ ID NOS: 64 and 65, or variants and fragments thereof. In an aspect, the origin of replication is derived from the pSa origin of replication. In an aspect, the pSa origin of replication has SEQ ID NO: 53, or variants and fragments thereof. In an aspect, the origin of replication is derived from the pRSF1010 origin of replication. In an aspect, the pRSF1010 origin of replication has SEQ ID NO: 37, or variants and fragments thereof. In an aspect, the vector further comprises a sequence derived from the par DE operon. In an aspect, the par DE operon has SEQ ID NO: 55, or variants and fragments thereof. In an aspect, the selectable marker gene provides resistance to gentamicin, neomycin/kanamycin, hygromycin, or spectinomycin. In an aspect, the selectable marker gene is an *aacC1* gene, a *npt1* gene, a *npt2* gene, a *hpt* gene, an aadA gene, a *SpcN* gene, *or an aph* gene. In an aspect, the selectable marker gene is *aacC1.* In an aspect, the *aacC1* selectable marker gene has SEQ ID NO: 1, or variants and fragments thereof. In an aspect, the selectable marker gene is *aadA.* In an aspect, the *aadA* selectable marker gene has SEQ ID NO: 39, or variants and fragments thereof. In an aspect, the selectable marker gene is *npt1.* In an aspect, the *npt1* selectable marker gene has SEQ ID NO: 40, or variants and fragments thereof. In an aspect, the selectable marker gene is *npt2.* In an aspect, the *npt2* selectable marker gene has SEQ ID NO: 41, or variants and fragments thereof. In an aspect, the selectable marker gene is *hpt.* In an aspect, the *hpt* selectable marker gene has SEQ ID NO: 67, or variants and fragments thereof. In an aspect, the selectable marker gene is *SpcN.* In an aspect, the *SpcN* selectable marker gene has SEQ ID NO: 77, or variants and fragments thereof. In an aspect, the selectable marker gene is *aph.* In an aspect, the *aph* selectable marker gene has SEQ ID NO: 78, or variant and fragments thereof. In an aspect, the selectable marker gene does not provide resistance to tetracycline. In an aspect, the selectable marker gene is not a *tetAR* gene. In an aspect, the selectable marker gene is a counter-selectable marker gene. In an aspect, the counter-selectable marker gene is a *sacB* gene, a *rpsL* (*strA*) gene, *a pheS* gene, *adhfr* (*folA*) gene, a *lacY* gene, a *Gata-1* gene, a *ccdB* gene, or a *thyA-* gene. In an aspect, the vector does not comprise SEQ ID NO: 61, or variants or fragments thereof. In an aspect, the vector does not comprise SEQ ID NO: 62, or variants or fragments thereof. In an aspect, the vector does not comprise a *tra* operon sequence or a *trb* operon sequence, or variants or fragments thereof. In an aspect, the vector does not comprise SEQ ID NO: 63, or variants or fragments thereof. In an aspect, the vector has SEQ ID NO: 34, or variants and fragments thereof. In an aspect, the vector has SEQ ID NO: 35, or variants and fragments thereof. In an aspect, the vector has SEQ ID NO: 36, or variants and fragments thereof.

### BRIEF DESCRIPTION OF THE DRAWINGS

FIG. 1 shows a plasmid map of vector pSB1.
FIG. 2 shows a plasmid map of vector pPHP70298, which has the sequence of SEQ ID NO: 34.
FIG. 3 shows a plasmid map of vector of pPHP71539, which has the sequence of SEQ ID NO: 35.
FIG. 4 shows a plasmid map of vector of pPHP79761, which has the sequence of SEQ ID NO: 36, with location of unique restriction enzyme sites. These restriction sites were included between functional elements to facilitate their exchange with other functionally equivalent elements.
FIG. 5 shows a diagram showing the relationship of the indicated genes on a 37,171 bp fragment from the *Agrobacterium tumefaciens* Ti plasmid pTiBo542, NCBI Reference Sequence: NC_010929.1
FIG. 6 shows a diagram showing the relationship of the indicated genes 2,600 bp fragment comprising the pVS1 origin of replication.
FIG. 7A shows transient RFP (Ds-RED) expression in wheat cultivar HC0456D 15 days post infection; strain AGL1. FIG. 7B shows transient RFP expression after 15 days post infenction; strain LBA4404Thy-71539. Arrows indicate the areas with strong Ds-RED expression. Scale bar equals 2mm.

### DETAILED DESCRIPTION

The disclosures herein will be described more fully hereinafter with reference to the accompanying drawings.

As used in the specification and in the claims, the term "comprising" can include the aspect of "consisting of." Unless defined otherwise, all technical and scientific terms used herein have the same meaning as commonly understood by one of ordinary skill in the art to which the disclosed compositions and methods belong. In this specification and in the claims which follow, reference will be made to a number of terms which shall be defined herein.

### I. METHODS AND COMPOSITIONS FOR IMPROVED VECTORS COMPRISING VIR GENES

The present disclosure comprises methods and compositions for the vectors comprising vir genes.

As used herein, "pPHP" refers to plasmid PHP, which is than followed by numerical digits. For example, pPHP70298 refers to plasmid PHP70298. For example, pVIR7 refers to plasmid VIR7.

Table 23 provides a list of sequence identification numbers (SEQ ID NO:) provided in this disclosure.

In an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) Rhizobiaceae virulence genes *virB1-B11, virC1-C2, virD1-D2,* and *virG* or Rhizobiaceae virulence genes *r-virB1-B11, r-virC1-C2, r- virD1-D2, r-virG,* and *r-galls,* or variants and derivatives thereof. In an aspect, the Rhizobiaceae virulence genes *virB1*-virB11 have SEQ ID NOS: 4-14, respectively, or the Rhizobiaceae virulence genes *r-virB1-B11* have SEQ ID NOS: 80-90, respectively, or variants and derivatives thereof; the Rhizobiaceae virulence genes *virC1-C2* have SEQ ID NOS: 16-17, respectively, or the Rhizobiaceae virulence genes *r-virC1-C2* have SEQ ID NOS: 92-93, respectively, or variants and derivatives thereof; the Rhizobiaceae virulence genes *virD1-D2* have SEQ ID NOS: 18-19, or the Rhizobiaceae virulence genes *r-virD1-D2* have SEQ ID NOS: 94-95, respectively, or variants and derivatives thereof; the Rhizobiaceae virulence gene *virG* has SEQ ID NO: 15, or the Rhizobiaceae virulence gene *r- virG* has SEQ ID NO: 91, respectively, or variants and derivatives thereof; and the Rhizobiaceae virulence gene *r-galls* has SEQ ID NO: 101, or variants and derivatives thereof

In aspects, the Rhizobiaceae virulence genes are *Agrobacterium* spp., *Rhizobium* spp., *Sinorhizobium* spp., *Mesorhizobium* spp., *Phyllobacterium* spp., *Ochrobactrum* spp., or *Bradyrhizobium* spp. genes. In an aspect, the Rhizobiaceae virulence genes are *Rhizobium* spp. genes. In an aspect, the Rhizobiaceae virulence genes are *Sinorhizobium* spp. genes. In an aspect, the Rhizobiaceae virulence genes are *Mesorhizobium* spp. genes. In an aspect, the Rhizobiaceae virulence genes are *Phyllobacterium* spp. genes. In an aspect, the Rhizobiaceae virulence genes are *Ochrobactrum* spp. genes. In an aspect, the Rhizobiaceae virulence genes are *Bradyrhizobium* spp. genes.

In an aspect, the Rhizobiaceae virulence genes are Agrobacterium spp. genes. In an aspect, the Agrobacterium spp. genes are Agrobacterium albertimagni, Agrobacterium larrymoorei, Agrobacterium radiobacter, Agrobacterium rhizogenes, Agrobacterium rubi, Agrobacterium tumefaciens, or Agrobacterium vitis genes. In an aspect, the Agrobacterium spp. genes are Agrobacterium rhizogenes or Agrobacterium tumefaciens. In an aspect, the Agrobacterium spp. genes are Agrobacterium rhizogenes. In an aspect, the Agrobacterium spp. genes are Agrobacterium tumefaciens.

A number of wild-type and disarmed (non-pathogenic) strains of *Agrobacterium tumefaciens* and *Agrobacterium rhizogenes* harboring Ti or Ri plasmids can be used for gene transfer into plants. Phytohormone synthesis genes located in the T-DNA of wild type *Agrobacteria* harboring a Ti or Ri plasmid are expressed in plant cells following transformation, and cause tumor formation or a hairy root phenotype depending on the *Agrobacterium* strain or species. The T-DNA of *Agrobacteria* can be engineered to replace many of its virulence and pathogenicity determinants (by disarming) with one or more sequences of interest and retain the ability to transfer the modified T-DNA into a plant cell and be integrated into a genome. Strains containing such disarmed Ti plasmids are widely used for plant transformation.

In some aspects, a construct comprises a Ti plasmid (*Agrobacterium tumefaciens*) or a Ri plasmid (*Agrobacterium rhizogenes*). In some aspects, the construct comprises one or more virulence genes. The virulence genes can be from a Ti plasmid and are represented herein as SEQ ID NOS: 4-27 and SEQ ID NOS: 42-49. The virulence genes can be from a Ri plasmid and are represented herein as SEQ ID NOS: 79-101. The Ri plasmid virulence genes disclosed herein are represented using a "r" before the vir gene name. For example, *r-virA* (SEQ ID NO: 79), *r-virB1* (SEQ ID NO: 80), *r-virB2* (SEQ ID NO: 81), *r-virB3* (SEQ ID NO: 82), *r-virB4* (SEQ ID NO: 83), *r-virB5* (SEQ ID NO: 84), *r-virB6* (SEQ ID NO: 85), *r-virB7* (SEQ ID NO: 86), *r-virB8* (SEQ ID NO: 87), *r-virB9* (SEQ ID NO: 88), *r-virB10* (SEQ ID NO: 89), *r-virB11* (SEQ ID NO: 90), *r-virG* (SEQ ID NO: 91), *r-virC1* (SEQ ID NO: 92), *r-virC2* (SEQ ID NO: 93), *r-virD1* (SEQ ID NO: 94), *r-virD2* (SEQ ID NO: 95), *r-virD3* (SEQ ID NO: 96), *r-virD4* (SEQ ID NO: 97), *r-virD5* (SEQ ID NO: 98), *r-virF* (SEQ ID NO: 99), *r-virE3* (SEQ ID NO: 100), and *r-galls* (SEQ ID NO: 101). See Table 23 herein. Different combinations of the virulence genes may be used herein. The *r-galls* gene (SEQ ID NO: 101) is necessary for virulence with the Ri plasmid vir genes described herein.

The Vir region on the Ti/Ri plasmid is a collection of genes whose aggregate function is to excise the T-DNA region of the plasmid and promote its transfer and integration into the plant genome. The *vir* system is induced by signals produced by plants in response to wounding. Phenolic compounds such as acetosyringone, syringealdehyde, or acetovanillone activate the *virA* gene, which encodes a receptor that is a constitutively expressed trans-membrane protein. The activated *virA* gene acts as a kinase, phosphorylating the *virG* gene. In its phosphorylated form, *virG* acts as a transcriptional activator for the remaining *vir* gene operons. The *virB* operon encodes proteins which produce a pore/pilus-like structure. *VirC* binds to the overdrive sequence. *VirD1* and *virD2* have endonuclease activity, and make single-stranded cuts within the left and right borders, and *virD4* is a coupling protein. *VirE* binds to the single stranded T-DNA, protecting it during the transport phase of the process. Once in the plant cell, the complementary strand of the T-DNA is synthesized.

These and other *vir* genes, function in trans, so none of these genes need to be included in the cloning vectors. For example, modified *Agrobacterium* strains can provide all the necessary Vir functions on plasmids where the T-DNA region has been deleted, allowing the cell to provide the vir functions for T-DNA transfer. In one example, there are C58-derived strains in which a portion of pBR322 was used to replace the T-DNA region, and providing resistance to ampicillin.

Provided are constructs which include one or more sequence of interest for expression and/or insertion in a cell genome. The constructs may be contained within a vector such as binary, ternary or T-DNA vectors. A construct refers to a polynucleotide molecule comprised of various types of nucleotide sequences having different functions and/or activities. Various types of sequences include linkers, adapters, regulatory regions, introns, restriction sites, enhancers, insulators, screenable markers, selectable markers, promoters, expression cassettes, coding polynucleotides, silencing polynucleotides, termination sequences, origins of replication, recombination sites, excision cassettes, recombinases, cell proliferation factors, promoter traps, other sites that aid in vector construction or analysis, or any combination thereof. In some examples a construct comprises one or more expression cassettes, wherein a polynucleotide is operably linked to a regulatory sequence. Operably linked is a functional linkage between two or more elements. For example, an operable linkage between a coding polynucleotide and a regulatory sequence *(e.g.,* a promoter) is a functional link that allows for expression of the coding polynucleotide. Operably linked elements may be contiguous or non-contiguous. When used to refer to the joining of two protein coding regions, by operably linked is intended that the coding regions are in the same reading frame. A coding polynucleotide includes any polynucleotide that either encodes a polypeptide, or that encodes a silencing polynucleotide that reduces the expression of target genes. Non-limiting examples of a silencing polynucleotide include a small interfering RNA, micro RNA, antisense RNA, and a hairpin structure. The construct may also contain a number of genetic components to facilitate transformation of the plant cell or tissue and to regulate expression of any structural nucleic acid sequence. In some examples, the genetic components are oriented so as to express a mRNA, optionally the mRNA is translated into a protein. The expression of a plant structural coding sequence (a gene, cDNA, synthetic DNA, or other DNA) that exists in double-stranded form involves transcription of messenger RNA (mRNA) from one strand of the DNA by RNA polymerase enzyme and subsequent processing of the mRNA primary transcript inside the nucleus. This processing involves a 3' non-translated region that polyadenylates the 3' ends of the mRNA.

In an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) *Agrobacterium* virulence genes *virB1-B11*; *virC1-C2*; *virD1-D2*; and *virG,* or the *Agrobacterium* virulence genes *r-virB1-B11, r-virC1-C2, r-virD1-D2, r-virG,* and *r-galls,* or variants and derivatives thereof. In an aspect, the *Agrobacterium* virulence genes *virB1-B11* have SEQ ID NOS: 4-14, respectively, or variants and derivatives thereof; the *Agrobacterium* virulence genes *virCl-C2* have SEQ ID NOS: 16-17, respectively, or variants and derivatives thereof; the *Agrobacterium* spp.virulence genes *virD1-D2* have SEQ ID NOS: 18-19, respectively, or variants and derivatives thereof; and the *Agrobacterium* virulence gene *virG* has SEQ ID NO: 15, or variants and derivatives thereof; or the *Agrobacterium* virulence genes *r-virB1-B11* have SEQ ID NOS: 80-90, respectively, or variants and derivatives thereof, *r-virC1-C2* have SEQ ID NOS: 92-93, respectively, or variants and derivatives thereof, *r-virD1-D2* have SEQ ID NOS: 95-96, respectively, or variants and derivatives thereof, *r-virG* has SEQ ID NO: 91, or variants and derivatives thereof, and *r-galls* has SEQ ID NO: 101, or variants and derivatives thereof.

In an aspect, the vector further comprises one or more Rhizobiaceae virulence genes *virA, virD3, virD4, virD5, virEl, virE2, virE3, virH, virH1, virH2, vir J, virK, virL, virM, virP,* or *virQ,* or variants and derivatives thereof, or one or more Rhizobiaceae virulence genes *r-virA* , *r-virD3, r-virD4, r-virD5, r-virE3,* or *r-virF,* or variants and derivatives thereof, wherein the vector comprising the virulence genes *r-virA* , *r-virD3, r-virD4, r-virD5, r-virE3,* and *r-virF* further comprises a *r-galls* virulence gene, or variants and derivatives thereof.. Thus, in an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) Rhizobiaceae virulence genes *virB1-B11; virC1-C2; virD1-D2,* and *virG,* or variants and derivatives thereof, or the Rhizobiaceae virulence genes *r-virB1-B11, r-virC1-C2, r-virD1-D2,* and *r-virG,* or variants and derivatives thereof and *r-galls,* or variants and derivatives thereof; and optionally one or more Rhizobiaceae virulence genes *virA, virD3, virD4, virD5, virE1, virE2, virE3, virH, virH1, virH2, vir J, virK, virL, virM, virP,* or *virQ,* or variants and derivatives thereof or one or more Rhizobiaceae virulence genes *r-virA* , *r-virD3, r-virD4, r-virD5, r-virE3,* or *r-virF,* or variants and derivatives thereof and *r-galls,* or variants and derivatives thereof In an aspect, the Rhizobiaceae virulence gene *virA* has SEQ ID NO: 26 or the Rhizobiaceae virulence gene *r-virA* has SEQ ID NO: 79, or variants and derivatives thereof. In an aspect, the Rhizobiaceae virulence genes *virD3-D5* have SEQ ID NOS: 20-22, respectively, or the Rhizobiaceae virulence genes *r-virD3-D5* have SEQ ID NO: 94-96, respectively, or variants and derivatives thereof. In an aspect, the Rhizobiaceae virulence genes *virE1-E3* have SEQ ID NOS: 23-25, respectively, or the Rhizobiaceae virulence gene *r*-virE3 has SEQ ID NO: 100, or variants and derivatives thereof. In an aspect, the Rhizobiaceae virulence genes *virH-H1* have SEQ ID NOS: 42-43 respectively, or variants and derivatives thereof. In an aspect, the Rhizobiaceae virulence gene *virJ* has SEQ ID NO: 27, or variants and derivatives thereof. In an aspect, the Rhizobiaceae virulence gene *virK* has SEQ ID NO: 45, or variants and derivatives thereof. In an aspect, the Rhizobiaceae virulence gene *virL* has SEQ ID NO: 46, or variants and derivatives thereof. In an aspect, the Rhizobiaceae virulence gene *virM* has SEQ ID NO: 47, or variants and derivatives thereof. In an aspect, the Rhizobiaceae virulence gene *virP* has SEQ ID NO: 48, or variants and derivatives thereof. In an aspect, the Rhizobiaceae virulence gene *virQ* has SEQ ID NO: 49, or variants and derivatives thereof.

In an aspect, the vector further comprises one or more *Agrobacterium* virulence genes *virA, virD3, virD4, virD5, virE1, virE2, virE3, virH, virH1, virH2, vir J, virK, virL, virM, virP,* or *virQ,* or variants and derivatives thereof, or one or more *Agrobacterium* virulence genes *r-virA* , *r-virD3, r-virD4, r-virD5, r-virE3,* or *r-virF,* or variants and derivatives thereof, and *r-galls,* or variants and derivatives thereof. Thus, in an aspect, the present disclosure provides a vector comprising: (a) an origin of replication for propagation and stable maintenance in *Escherichia coli;* (b) an origin of replication for propagation and stable maintenance in *Agrobacterium* spp.; (c) a selectable marker gene; and (d) *Agrobacterium* virulence genes *virB1-B11*; *virC1-C2; virD1-D2;* and *virG,* or variants and derivatives thereof, or the *Agrobacterium* virulence genes *r-virB1-B11, r-virC1-C2, r-virD1-D2, r-virG,* and *r-galls,* or variants and derivatives thereof; and optionally one or more *Agrobacterium* virulence genes *virA, virD3, virD4, virD5, virE1, virE2, virE3, virH, virH1, virH2, vir J, virK, virL, virM, virP,* or *virQ,* or variants and derivatives thereof, or one or more *Agrobacterium* virulence genes *r-virA* , *r-virD3, r-virD4, r-virD5, r-virE3,* or *r-virF* or variants and derivatives thereof, and *r-galls,* or variants and derivatives thereof. In an aspect, the Rhizobiaceae virulence gene *virA* has SEQ ID NO: 26 or the Rhizobiaceae virulence gene *r-virA* has SEQ ID NO: 79, or variants and derivatives thereof. In an aspect, the Rhizobiaceae virulence genes *virD3-D5* have SEQ ID NOS: 20-22, respectively, or the Rhizobiaceae virulence genes *r-virD3-D5* have SEQ ID NO: 94-96, respectively, or variants and derivatives thereof. In an aspect, the Rhizobiaceae virulence genes *virE1-E3* have SEQ ID NOS: 23-25, respectively, or the Rhizobiaceae virulence gene *r*-virE3 has SEQ ID NO: 100, or variants and derivatives thereof. In an aspect, the Rhizobiaceae virulence genes *virH-H2* have SEQ ID NOS: 42-43, respectively, or variants and derivatives thereof. In an aspect, the Rhizobiaceae virulence gene *virJ* has SEQ ID NO: 27, or variants and derivatives thereof. In an aspect, the Rhizobiaceae virulence gene *virK* has SEQ ID NO: 45, or variants and derivatives thereof. In an aspect, the Rhizobiaceae virulence gene *virL* has SEQ ID NO: 46, or variants and derivatives thereof. In an aspect, the Rhizobiaceae virulence gene *virM* has SEQ ID NO: 47, or variants and derivatives thereof. In an aspect, the Rhizobiaceae virulence gene *virP* has SEQ ID NO: 48, or variants and derivatives thereof. In an aspect, the Rhizobiaceae virulence gene *virQ* has SEQ ID NO: 49, or variants and derivatives thereof. In an aspect, the Rhizobiaceae virulence gene *r-virF* has SEQ ID NO: 99, or variants and derivatives thereof. In an aspect, the Rhizobiaceae virulence gene *r-galls* has SEQ ID NO: 101, or variants and derivatives thereof.

In an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) *Agrobacterium* virulence genes *virB1-B11*; *virC1-C2*; *virD1-D5; virE1-E3*; and *virG,* or variants and derivatives thereof, or the *Agrobacterium* virulence genes *r-virB1-B11* having SEQ ID NOS: 80-90, respectively, *r-virC1-C2* having SEQ ID NOS: 92-93, respectively, *r-virD1-D5* having SEQ ID NOS: 94-98, respectively, *r-vir-E3* having SEQ ID NOS: 100, *r-virG* having SEQ ID NO: 91, and *r-galls* having SEQ ID NO: 101, or variants and derivatives thereof.

In an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) *Agrobacterium* virulence genes *virA; virB1-B11; virC1-C2; virD1-D5; virE1-E3;* and *virG,* or variants and derivatives thereof, or the *Agrobacterium* virulence genes *r-virB1-B11; r-virC1-C2; r-virD1-D2; r-virG;* and *r-galls,* or variants and derivatives thereof.

In an aspect, the present disclosure provides a vector comprising: (a) an origin of replication for propagation and stable maintenance in *Escherichia coli*; (b) an origin of replication for propagation and stable maintenance in *Agrobacterium* spp.; (c) a selectable marker gene; and (d) *Agrobacterium* virulence genes *virA*; *virB1-B11*; *virC1-C2*; *virD1-D5; virE1-E3; virG;* and *virJ,* or variants and derivatives thereof, or the *Agrobacterium* virulence genes *r-virA* having SEQ ID NO: *79, r-virB1-B11* having SEQ ID NOS: 80-90, respectively, *r-virC1-C2* having SEQ ID NOS: 92-93, respectively, *r-virD1-D5* having SEQ ID NOS: 94-98, respectively, *r-virE3* having SEQ ID NO: 100, *r-virG* having SEQ ID NO: 91, and *r-galls* having SEQ ID NO: 101, or variants and derivatives thereof.

The present disclosure provides a vector comprising an origin of replication for propagation and stable maintenance in *Escherichia coli* derived from a Col E1, pSC101, p15A, or R6K origin of replication, or functional variants and derivatives thereof. In an aspect, any origin(s) of replication functional in *Agrobacterium* can be used in constructing the disclosed vectors. For example, different origins of replication can be selected in order to achieve different frequencies and qualities (single T-DNA copy, no backbone) of transformation events. In an aspect, the origin(s) of replication is an origin that is functional in *Agrobacterium, E. coli,* or both. In an aspect, the origin(s) of replication is selected from the group consisting of pVS1, pSa, RK2, pRi, incPa, incW, Co 1E1, pRSF1010, pBBR1 or functional variants and derivatives thereof.

In an aspect, the vector comprises an origin of replication for propagation and stable maintenance in *Escherichia coli* derived from a Col E1 origin of replication. In a further aspect, the vector comprises an origin of replication for propagation and stable maintenance in *Escherichia coli* derived from a Col E1 origin of replication, having SEQ ID NO: 2, or variants and fragments thereof.

In an aspect, the vector comprises an origin of replication for propagation and stable maintenance in *Escherichia coli* derived from a pSC101 origin of replication. In a further aspect, the vector comprises an origin of replication for propagation and stable maintenance in *Escherichia coli* derived from a pSC101 origin of replication, having SEQ ID NO: 50, or variants and fragments thereof.

In an aspect, the vector comprises an origin of replication for propagation and stable maintenance in *Escherichia coli* derived from a p15A origin of replication. In a further aspect, the vector comprises an origin of replication for propagation and stable maintenance in *Escherichia coli* derived from a p15A origin of replication, having SEQ ID NO: 51, or variants and fragments thereof.

In an aspect, the vector comprises an origin of replication for propagation and stable maintenance in *Escherichia coli* derived from a R6K origin of replication. In a further aspect, the vector comprises an origin of replication for propagation and stable maintenance in *Escherichia coli* derived from a R6K origin of replication, having SEQ ID NO: 52, or variants and fragments thereof.

In various aspects, the origin of replication for propagation and stable maintenance in *Agrobacterium* spp. can be a low copy number origin of replication, an intermediate copy number origin of replication, or a high copy number origin of replication. It is understood that a low copy number of origin of replication provides for about 1-2 copies of the vector per cell (*e.g.,* see Li et al., Plant Cell Report (2015) 34:745-54; and Cho and Winans Proc. Natl. Acad. Sci. USA (2005) 102:14843-848). Further, it is understood that an intermediate copy number of origin of replication provides for about 7-12 copies of the vector per cell (*e.g.,* see Oltmanns, et al., Plant Physiol. (2010) 152:1158-1166). Exemplary, but non-limiting examples, of intermediate copy number origins of replication include those derived from pRK2 and copy down variants of pVS1. It is to be appreciated that a high copy number of origin of replication provides for about 15-20 and greater copies of the vector per cell (*e.g.,* see Oltmanns, et al., Plant Physiol. (2010) 152:1158-1166; and Li et al., Plant Cell Report (2015) 34:745-54). Exemplary, but non-limiting examples, of intermediate copy number origins of replication include those derived from repABC and copy up variants of pVS1.

In an aspect, origin of replication for propagation and stable maintenance in *Agrobacterium* spp. is derived from a pRi, pVS1, pRSF1010, pRK2, pSa, or pBBR1 origin of replication. In an aspect, the origin of replication for propagation and stable maintenance in *Agrobacterium* spp. is derived from the pRK2 origin of replication. In an aspect, the origin of replication for propagation and stable maintenance in *Agrobacterium* spp. is derived from the pRSF1010 origin of replication. In an aspect, the origin of replication for propagation and stable maintenance in *Agrobacterium* spp. is derived from the pVS1 origin of replication. In an aspect, the origin of replication for propagation and stable maintenance in *Agrobacterium* spp. is derived from the pSa origin of replication. In various aspects, the origin of replication for propagation and stable maintenance in *Agrobacterium* spp. has SEQ ID NO: 3, 37, 38, 57, 58, 59, or 60, or variants and fragments thereof.

In an aspect, the origin of replication for propagation and stable maintenance in *Agrobacterium* spp. is a repABC compatible origin of replication. The repABC compatible origin of replication can have SEQ ID NOS: 57, 58, 59, or 60, or variants and fragments thereof.

In some aspects, the origin of replication for propagation and stable maintenance in *Escherichia coli* and the origin of replication for propagation and stable maintenance in *Agrobacterium* spp. are the same origin of replication. For example, the origin of replication for propagation and stable maintenance in *Escherichia coli* and the origin of replication for propagation and stable maintenance in *Agrobacterium* spp. can be derived from a pRK2 origin of replication, from a pSa origin of replication, or a pRSF1010 origin of replication. In an aspect, the origin of replication for propagation and stable maintenance in *Escherichia coli* and the origin of replication for propagation and stable maintenance in *Agrobacterium* spp. can be derived from the pRK2 origin of replication. In a further aspect, the origin of replication for propagation and stable maintenance in *Escherichia coli* and the origin of replication for propagation and stable maintenance in *Agrobacterium* spp. can be derived from the pRK2 origin of replication has SEQ ID NO: 38, or variants and fragments thereof. In an aspect, the origin of replication is derived from the pSa origin of replication. In an aspect, the origin of replication for propagation and stable maintenance in *Escherichia coli* and the origin of replication for propagation and stable maintenance in *Agrobacterium* spp. can be derived from the pSa origin of replication (SEQ ID NO: 53), or variants and fragments thereof. In a further aspect, the origin of replication for propagation and stable maintenance in *Escherichia coli* and the origin of replication for propagation and stable maintenance in *Agrobacterium* spp. can be derived from the origin of replication pRSF1010 origin of replication. In a further aspect, the pRSF1010 origin of replication has SEQ ID NO: 37, or variants and fragments thereof.

Variants of the pRK2 origin of replication include a mini or micro pRK2 origin of replication. In an aspect, the origin of replication for propagation and stable maintenance in *Escherichia coli* and the origin of replication for propagation and stable maintenance in *Agrobacterium* spp. is a micro pRK2 origin of replication. In a further aspect, the origin of replication for propagation and stable maintenance in *Escherichia coli* and the origin of replication for propagation and stable maintenance in *Agrobacterium* spp. is a micro pRK2 origin of replication and has SEQ ID NO: 54, or variants and fragments thereof.

In aspects, the disclosed vector further comprises a sequence derived from the par DE operon. In a further aspect, the disclosed vector comprising a pRK2 origin of replication, in particular a micro or mini pRK2 origin of replication, can further comprise a sequence derived from the par DE operon. The par DE operon sequence can have SEQ ID NO: 55, or variants and fragments thereof.

In an aspect, the selectable marker provides resistance to gentamicin, neomycin/kanamycin, hygromycin, or spectinomycin. In a further aspect, the selectable marker is *aacC1, npt1, npt2, hpt,* aadA, *SpcN, or aph.* In an aspect, the selectable marker has SEQ ID NO: 1, 39, 40, 41, 67, 77 or 78, or variants and fragments thereof, corresponding, respectively, to *aacC1, aadA, npt1, npt2, hpt, SpcN,* and *aph.* In an aspect, the selectable marker is *aacC1.* In a further aspect, the selectable marker is *aacC1,* and has SEQ ID NO: 1, or variants and fragments thereof. In an aspect, the selectable marker is *aadA.* In a further aspect, the selectable marker is *aadA,* and has SEQ ID NO: 39, or variants and fragments thereof. In an aspect, the selectable marker is *npt1.* In a further aspect, the selectable marker is *npt1,* and has SEQ ID NO: 40, or variants and fragments thereof. In an aspect, the selectable marker is *npt2.* In a further aspect, the selectable marker is *npt2,* and has SEQ ID NO: 41. In an aspect, the selectable marker is *hpt.* In a further aspect, the selectable marker is *hpt,* and has SEQ ID NO: 67, or variants and fragments thereof. In a further aspect, the selectable marker is *SpcN,* and has SEQ ID NO: 77, or variants and fragments thereof. In a further aspect, the selectable marker is *aph,* and has SEQ ID NO: 78, or variants and fragments thereof. In various aspects, the selectable marker is not a tetracycline selectable marker. In an aspect, the selectable marker is not *tetAR.*

In an aspect, the selectable marker is a counter-selectable marker or negative selectable marker. As can be appreciated, it is understood that the disclosed vector can comprise particular combinations of virulence (*vir*) genes, origins of replication, and selectable markers. Table 1 below provides exemplary, but not limiting, combinations comprising virulence genes, selectable marker(s), and origins of replication. In an aspect, the counter-selectable marker is *sacB, rpsL* (*strA*), *pheS, dhfr* (*folA*)*, lacY, Gata-1, ccdB,* or *thyA-.*

**Table 1.**

| Combination No. | Virulence Genes | Origin of Replication | Selectable marker |
|---|---|---|---|
| 1 | virB1-B11; virC1-C2; virD1-D2; and virG | pVS1 | aacC1 |
| 2 | virB1-B11; virC1-C2; virD1-D5; virE1-E3; and virG | pVS1 | aacC1 |
| 3 | virA; virB1-B11; virC1-C2; virD1-D5; virE1-E3; virG; and virJ | pVS1 | aacC1 |
| 4 | virB1-B11; virC1-C2; virD1-D5; virE1-E3; and virG | pRi repABC | aacC1 |
| 5 | virA; virB1-B11; virC1-C2; virD1-D5; virE1-E3; virG; and virJ | pRi repABC | aacC1 |
| 6 | virA; virB1-B11; virC1-C2; virD1-D5; virE1-E3; virG; and virJ | pRK2 | aacC1 |
| 7 | virA; virB1-B11; virC1-C2; virD1-D5; virE1-E3; virG; and virJ | pSa | aacC1 |
| 8 | virA; virB1-B11; virC1-C2; virD1-D5; virE1-E3; virG; and virJ | pSa and par DE | aacC1 |
| 9 | virA; virB1-B11; virC1-C2; virD1-D5; virE1-E3; virG; and virJ | pBBR1 | aacC1 |
| 10 | virA; virB1-B11; virC1-C2; virD1-D5; virE1-E3; virG; and virJ | RFS1010 | aacC1 |

In an aspect, the present disclosure provides for a vector that does not comprise SEQ ID NO: 61, or variants or fragments thereof.

In an aspect, the present disclosure provides for a vector that does not comprise SEQ ID NO: 62, or variants or fragments thereof.

In an aspect, the present disclosure provides for a vector that does not comprise a *tra* or *trb* operon sequence, or variants or fragments thereof. In a further aspect, the present disclosure provides for a vector that does not comprise a *tra* or *trb* operon sequence, wherein the *tra* or *trb* operon sequence has SEQ ID NO: 63, or variants or fragments thereof.

In an aspect, the present disclosure provides a vector having SEQ ID NOS: 34, 35, or 36, or variants and fragments thereof.

In an aspect, the present disclosure provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1, or variants and fragments thereof; and (d) virulence genes comprising *Agrobacterium* virulence genes *virB1-B11* having SEQ ID NOS: 4-14, respectively, or variants and derivatives thereof; *virCl-C2* having SEQ ID NOS: 16-17, respectively, or variants and derivatives thereof; *virD1-D2* having SEQ ID NOS: 18-19, respectively, or variants and derivatives thereof; and *virG* having SEQ ID NO: 15, or variants and derivatives thereof, or the *Agrobacterium* virulence genes *r-virB1-B11* having SEQ ID NOS: 80-90, respectively, or variants and derivatives thereof, *r-virC1-C2* having SEQ ID NOS: 92-93, respectively, or variants and derivatives thereof, *r-virD1-D2* having SEQ ID NOS: 95-96, respectively, or variants and derivatives thereof, *r-virG* having SEQ ID NO: 91, or variants and derivatives thereof, and *r-galls* having SEQ ID NO: 101, or variants and derivatives thereof.

In an aspect, the present disclosure provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1, or variants and fragments thereof; and (d) virulence genes comprising *Agrobacterium* virulence genes *virB1-B11* having SEQ ID NOS: 4-14, respectively, or variants and derivatives thereof; *virCl-C2* having SEQ ID NOS: 16-17, respectively, or variants and derivatives thereof; *virD1-D5* having SEQ ID NOS: 18-22, respectively, or variants and derivatives thereof; *virE1-E3* having SEQ ID NOS: 23-25, or variants and derivatives thereof; and *virG* having SEQ ID NO: 15, or variants and derivatives thereof, or or the *Agrobacterium* virulence genes *r-virB1-B11* having SEQ ID NOS: 80-90, respectively, or variants and derivatives thereof; *r-virC1-C2* having SEQ ID NOS: 92-93, respectively, or variants and derivatives thereof; *r-virD1-D5* having SEQ ID NOS: 94-98, respectively, or variants and derivatives thereof; *r-vir-E3* having SEQ ID NO: 100, or variants and derivatives thereof; *r-virG* having SEQ ID NO: 91, or variants and derivatives thereof; and *r-galls* having SEQ ID NO: 101, or variants and derivatives thereof.

In an aspect, the present disclosure provides a vector comprising: (a) an origin of replication for propagation in *Escherichia coli* having SEQ ID NO: 2, or variants and fragments thereof; (b) an origin of replication for propagation in *Agrobacterium* spp. having SEQ ID NO: 3, or variants and fragments thereof; (c) a selectable marker gene having SEQ ID NO: 1, or variants and fragments thereof; and (d) virulence genes comprising *Agrobacterium virA* having SEQ ID NO: 26, or variants and derivatoves thereof; *virB1-B11* having SEQ ID NOS: 4-14, respectively, or variants and derivatives thereof; *virC1-C2* having SEQ ID NOS: 16-17, respectively, or variants and derivatives thereof; *virD1-D5* having SEQ ID NOS: 18-22, respectively, or variants and derivatives thereof; *virE1-E3* having SEQ ID NOS: 23-25, or variants and derivatives thereof; *virG* havingSEQ ID NO: 15, or variants and derivatives thereof; and *virJ* having SEQ ID NO: 27, or variants and derivatives thereof, or the *Agrobacterium* virulence genes *r-virA* having SEQ ID NO: 79, or variants and derivatives thereof; *r-virB1-B11* having SEQ ID NOS: 80-90, respectively, or variants and derivatives thereof; *r-virC1-C2* having SEQ ID NOS: 92-93, respectively, or variants and derivatives thereof; *r-virD1-D5* having SEQ ID NOS: 94-98, respectively, or variants and derivatives thereof; *r-*vir*E3* having SEQ ID NOS: 100, or variants and derivatives thereof; *r-virG* having SEQ ID NO: 91, or variants and derivatives thereof; and *r-galls* having SEQ ID NO: 101, or variants and derivatives thereof.

In an aspect, the present disclosure further provides methods for transformation of a plant comprising the steps of: (a) contacting a tissue from the plant with an *Agrobacterium* strain comprising a first vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) Rhizobiaceae virulence genes *virB1-B11, virC1-C2, virD1-D2,* and *virG* genes, or Rhizobiaceae virulence genes *r-virB1-B11, r-virC1-C, r-virD1-D2, r-virG,* and *r-galls,* or variants and derivatives thereof, and a second vector comprising T-DNA borders and a polynucleotide sequence of interest for transfer to the plant; (b) co-cultivatiing the tissue with the *Agrobacterium;* and (c) regenerating a transformed plant from the tissue that expresses the polynucleotide sequence of interest.

In an aspect, the present disclosure further provides methods for transformation of a plant comprising the steps of: (a) contacting a tissue from the plant with an *Agrobacterium* strain comprising a first vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) *Agrobacterium* virulence genes *virB1-B11, virC1-C2, virD1-D2,* and *virG* genes, or *Agrobacterium* virulence genes *r-virB1-B11, r- virC1-C, r-virD1-D2, r-virG,* and *r-galls,* or variants and derivatives thereof, and a second vector comprising T-DNA borders and a polynucleotide sequence of interest for transfer to the plant; (b) co-cultivatiing the tissue with the *Agrobacterium;* and (c) regenerating a transformed plant from the tissue that expresses the polynucleotide sequence of interest.

In an aspect, the present disclosure further provides kits comprising: (a) a vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) Rhizobiaceae virulence genes *virB1-B11; virC1-C2; virD1-D2;* and *virG* genes, or variants and derivatives thereof; and (b) instructions for use in transformation of a plant using *Agrobacterium.*

In an aspect, the present disclosure further provides kits comprising: (a) a vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) *Agrobacterium* virulence genes *virB1-B11*; *virC1-C2*; *virD1-D2*; and *virG* genes, or variants and derivatives thereof, or *Agrobacterium* virulence genes *r-*vir*B1-B11, r- virC1-C2, r- virD1-D2, r-virG,* and *r-galls*; and (b) instructions for use in transformation of a plant using *Agrobacterium.*

"Plant" includes reference to whole plants, plant organs, plant tissues, seeds and plant cells and progeny of same. Progeny, variants, and mutants of the regenerated plants are also included within the scope of the present disclosure, provided that these parts comprise the introduced polynucleotides or were transformed using the vectors of the present disclosure. Plant cells include, without limitation, cells from seeds, suspension cultures, embryos, meristematic regions, callus tissue, leaves, roots, shoots, gametophytes, sporophytes, pollen and microspores.

As used herein, "regeneration" refers to the process of growing a plant from a plant cell or cells (e.g., plant protoplast, callus, or explant).

As used herein, the term "protoplast" refers to an isolated plant cell without cell walls which has the potency for regeneration into cell culture or a whole plant.

Plant parts include differentiated and undifferentiated tissues including, but not limited to the following: roots, stems, shoots, leaves, pollen, seeds, tumor tissue and various forms of cells and culture (e.g., single cells, protoplasts, embryos and callus tissue). The plant tissue may be in a plant or in a plant organ, tissue or cell culture.

The present disclosure also includes plants obtained by any of the disclosed methods or compositions herein.

The present disclosure also includes seeds from a plant obtained by any of the disclosed methods or compositions herein.

### VARIANTS AND FRAGMENTS

By "fragment" is intended a portion of a polynucleotide or a portion of the amino acid sequence and hence protein encoded thereby. Fragments of a polynucleotide may encode protein fragments that retain the biological activity of the native protein. Thus, fragments of a nucleotide sequence may range from at least about 10 nucleotides, about 15 nucleotides, about 16 nucleotides, about 17 nucleotides, about 18 nucleotides, about 19 nucleotides, about 20 nucleotides, about 22 nucleotides, about 50 nucleotides, about 75 nucleotides, about 100 nucleotides, about 200 nucleotides, about 300 nucleotides, about 400 nucleotides, about 500 nucleotides, about 600 nucleotides, and up to the full-length polynucleotide employed.

By "derivative" is intended a polynucleotide or a portion of a polynucleotide that possesses activity that is substantially similar to the biological activity of the reference polynucleotide. A derivative of a virulence gene polynucleotide will be functional and will retain the virulence gene activity.

"Variant" is intended to mean a substantially similar sequence. For polynucleotides, a variant comprises a deletion and/or addition and/or substitution of one or more nucleotides at one or more internal sites within the native polynucleotide and/or a substitution of one or more nucleotides at one or more sites in the native polynucleotide. A variant of a virulence gene polynucleotide will retain the virulence gene activity. As used herein, a "native" polynucleotide or polypeptide comprises a naturally occurring nucleotide sequence or amino acid sequence, respectively. For polynucleotides, conservative variants include those sequences that, because of the degeneracy of the genetic code, encode the amino acid sequence of a polypeptide encoded by a virulence gene. Variant polynucleotides also include synthetically derived polynucleotide, such as those generated, for example, by using site-directed mutagenesis, but continue to retain the desired activity. Generally, variants of a particular disclosed polynucleotide *(i.e.,* a virulence gene) will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide as determined by sequence alignment programs and parameters described elsewhere herein.

Variants of a particular disclosed polynucleotide (i.e., the reference polynucleotide) can also be evaluated by comparison of the percent sequence identity between the polypeptide encoded by a variant polynucleotide and the polypeptide encoded by the reference polynucleotide. Percent sequence identity between any two polypeptides can be calculated using sequence alignment programs and parameters described elsewhere herein. Where any given pair of disclosed polynucleotides employed is evaluated by comparison of the percent sequence identity shared by the two polypeptides they encode, the percent sequence identity between the two encoded polypeptides is at least about 40%, 45%, 50%, 55%, 60%, 65%, 70%, 75%, 80%, 85%, 90%, 91%, 92%, 93%, 94%, 95%, 96%, 97%, 98%, 99% or more sequence identity.

The following terms are used to describe the sequence relationships between two or more polynucleotides or polypeptides: (a) "reference sequence," (b) "comparison window." (c) "sequence identity," and, (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two polynucleotides. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.
   Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.
(c) As used herein, "sequence identity" or "identity" in the context of two polynucleotides or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

A method is further provided for identifying a virulence gene variant set forth in SEQ ID NOS.: 4-27 and 42-49, or variants and derivatives thereof. Such methods comprise obtaining a candidate derivative of any one of SEQ ID NOS.: 4-27 and 42-49, or variants and derivatives thereof, which is of sufficient length to retain the subject virulence gene activity; replacing the related virulence gene in a control vector to produce a candidate variant vector and determining if the candidate virulence polynucleotide derivative has the activity of the related virulence gene and thereby provide the desired transformation function of the vector as described herein. Methods of identifying such candidate variants based on the desired transformation efffect, in light of the teachings provided herein, are known. In various aspects, it is to be understood that the term "... SEQ ID NOS.: 4-27 and 42-49, or variants or derivatives thereof..." is intended to mean that the disclosed sequences comprise SEQ ID NOS.: 4-27 and 42-49, and/or derivatives of SEQ ID NOS.: 4-27 and 42-49, the variants of SEQ ID NOS.: 4-27 and 42-49, and/or the derivatives of SEQ ID NOS.: 4-27 and 42-49, individually (or) or inclusive of some or all listed sequences.

### II. TRANSFORMATION OF PLANTS

In aspects, the methods of the present disclosure involve introducing a polynucleotide into a plant cell. "Introducing" is intended to mean presenting to the plant cell the polynucleotide in such a manner that the sequence gains access to the interior of a plant cell. The methods of the present disclosure involve introducing a polynucleotide into a plant cell using methods such as *Agrobacterium*-mediated transformation (U.S. Patent No. 5,563,055 and U.S. Patent No. 5,981,840).

In aspects, the vectors of the present disclosure can be used to improve the efficiency and speed of introducing a polynucleotide into a plant cell.

In aspects, the vectors of the present disclosure are useful for transforming one or more cells of an explant. The explant, including mature and immature somatic plant tissue, can be used as a source or explant material in the present disclousre as long as it is capable of producing embryogenic material or somatic embryos. Suitable somatic plant tissue includes tissue from staminate (i.e., male flowers), pistolate (i.e., female flowers), perfect flowers, corm discs, flowering stems, and bracts. Immature flowers and corm discs are the preferred somatic plant tissue sources. In an aspect, the plant-derived explant used for transformation includes immature embryos, 1-5 mm zygotic embryos, and 3.5-5 mm embryos.

The explant used in the disclosed methods can be derived from a monocot, including, but not limited to, barley, maize, millet, oats, rice, rye, *Setaria* spp., sorghum, sugarcane, switchgrass, triticale, turfgrass, or wheat. Alternatively, the explant used in the disclosed methods can be derived from a dicot, including, but not limited to, kale, cauliflower, broccoli, mustard plant, cabbage, pea, clover, alfalfa, broad bean, tomato, cassava, soybean, canola, alfalfa, sunflower, safflower, tobacco, *Arabidopsis,* or cotton.

In a further aspect, the explant used in the disclosed methods can be derived from a plant that is a member of the family Poaceae. Non-limiting examples of suitable plants from which an explant of the disclosed can be derived include grain crops, including, but not limited to, barley, maize (corn), oats, rice, rye, sorghum, wheat, millet, triticale; leaf and stem crops, including, but not limited to, bamboo, marram grass, meadow-grass, reeds, ryegrass, sugarcane; lawn grasses, ornamental grasses, and other grasses such as switchgrass and turfgrass.

In a further aspect, the explant used in the disclosed methods can be derived from any plant, including higher plants, e.g., classes of Angiospermae and Gymnospermae. Plants of the subclasses of the Dicotylodenae and the Monocotyledonae are suitable. Suitable species may come from the family Acanthaceae, Alliaceae, Alstroemeriaceae, Amaryllidaceae, Apocynaceae, Arecaceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, and Vitaceae.

Suitable species from which the explant used in the disclosed methods can be derived include members of the genus Abelmoschus, Abies, Acer, Agrostis, Allium, Alstroemeria, Ananas, Andrographis, Andropogon, Artemisia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, Cucumis, Cucurbita, Cynodon, Datura, Daucus, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Juglans, Lactuca, Lavendula, Linum, Lolium, Lupinus, Lycopersicon, Lycopodium, Manihot, Medicago, Mentha, Miscanthus, Moringa, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Rosmarinus, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

In a further aspect, the explant used in the disclosed methods can be derived from a plant that is important or interesting for agriculture, horticulture, biomass for the production of liquid fuel molecules and other chemicals, and/or forestry. Non-limiting examples include, for instance, *Panicum virgatum* (switchgrass), *Sorghum bicolor* (sorghum, sudangrass), *Miscanthus giganteus* (miscanthus), *Saccharum* spp. (energycane), *Populus balsamifera* (poplar), *Zea mays* (corn), *Glycine max* (soybean), *Brassica napus* (canola), *Triticum aestivum* (wheat), *Gossypium hirsutum* (cotton), *Oryza sativa* (rice), *Helianthus annuus* (sunflower), *Medicago sativa* (alfalfa), *Beta vulgaris* (sugarbeet), *Pennisetum glaucum* (pearl millet), *Panicum* spp., *Sorghum* spp., *Miscanthus* spp., *Saccharum* spp., *Erianthus* spp., *Populus* spp., *Andropogon gerardii* (big bluestem), *Pennisetum purpureum* (elephant grass), *Phalaris arundinacea* (reed canarygrass), *Cynodon dactylon* (bermudagrass), *Festuca arundinacea* (tall fescue), *Spartina pectinata* (prairie cord-grass), *Arundo donax* (giant reed), *Secale cereale* (rye), *Salix* spp. (willow), *Eucalyptus* spp. (eucalyptus), *Triticosecale* spp. (triticum-wheat X rye), Bamboo, *Carthamus tinctorius* (safflower), *Jatropha curcas* (jatropha), *Ricinus communis* (castor), *Elaeis guineensis* (palm), *Linum usitatissimum* (flax), *Brassica juncea, Manihot esculenta* (cassava), *Lycopersicon esculentum* (tomato), *Lactuca sativa* (lettuce), *Musa paradisiaca* (banana), *Solanum tuberosum* (potato), *Brassica oleracea* (broccoli, cauliflower, brusselsprouts), *Camellia sinensis* (tea), *Fragaria ananassa* (strawberry), *Theobroma cacao* (cocoa), *Coffea arabica* (coffee), *Vitis vinifera* (grape), *Ananas comosus* (pineapple), *Capsicum annum* (hot & sweet pepper), *Allium cepa* (onion), *Cucumis melo* (melon), *Cucumis sativus* (cucumber), *Cucurbita maxima* (squash), *Cucurbita moschata* (squash), *Spinacea oleracea* (spinach), *Citrullus lanatus* (watermelon), *Abelmoschus esculentus* (okra), *Solanum melongena* (eggplant), *Papaver somniferum* (opium poppy), *Papaver orientale, Taxus baccata, Taxus brevifolia, Artemisia annua, Cannabis sativa, Camptotheca acuminate, Catharanthus roseus, Vinca rosea, Cinchona officinalis, Colchicum autumnale, Veratrum californica., Digitalis lanata, Digitalis purpurea, Dioscorea* spp., *Andrographispaniculata, Atropa belladonna, Datura stomonium, Berberis* spp., *Cephalotaxus* spp., *Ephedra sinica, Ephedra* spp., *Erythroxylum coca, Galanthus wornorii, Scopolia* spp., *Lycopodium serratum* (=*Huperzia serrata*), *Lycopodium* spp., *Rauwolfia serpentina, Rauwolfia* spp., *Sanguinaria canadensis, Hyoscyamus* spp., *Calendula officinalis, Chrysanthemum parthenium, Coleus forskohlii, Tanacetum parthenium, Parthenium argentatum* (guayule), *Hevea* spp. (rubber), *Mentha spicata* (mint), *Mentha piperita* (mint), *Bixa orellana, Alstroemeria* spp., *Rosa* spp. (rose), *Dianthus caryophyllus* (carnation), *Petunia* spp. (petunia), *Poinsettia pulcherrima* (poinsettia), *Nicotiana tabacum* (tobacco), *Lupinus albus* (lupin), *Uniola paniculata* (oats), bentgrass (*Agrostis* spp.), *Populus tremuloides* (aspen), *Pinus* spp. (pine), *Abies* spp. (fir), *Acer* spp. (maple), *Hordeum vulgare* (barley), *Poa pratensis* (bluegrass), *Lolium* spp. (ryegrass), *Phleum pratense* (timothy), and conifers. Of interest are plants grown for energy production, so called energy crops, such as cellulose-based energy crops like *Panicum virgatum* (switchgrass), *Sorghum bicolor* (sorghum, sudangrass), *Miscanthus giganteus* (miscanthus), *Saccharum* spp. (energycane), *Populus balsamifera* (poplar), *Andropogon gerardii* (big bluestem), *Pennisetum purpureum* (elephant grass), *Phalaris arundinacea* (reed canarygrass), *Cynodon dactylon* (bermudagrass), *Festuca arundinacea* (tall fescue), *Spartina pectinala* (prairie cord-grass), *Medicago sativa* (alfalfa), *Arundo donax* (giant reed), *Secale cereale* (rye), *Salix* spp. (willow), *Eucalyptus* spp. (eucalyptus), *Triticosecale* spp. (triticum - wheat X rye), and Bamboo; and starch-based energy crops like *Zea mays* (corn) and *Manihot esculenta* (cassava); and sucrose-based energy crops like *Saccharum* spp. (sugarcane) and *Beta vulgaris* (sugarbeet); and biodiesel-producing energy crops like *Glycine max* (soybean), *Brassica napus* (canola), *Helianthus annuus* (sunflower), *Carthamus tinctorius* (safflower), *Jatropha curcas* (jatropha), *Ricinus communis* (castor), *Elaeis guineensis* (palm), *Linum usitatissimum* (flax), and *Brassica juncea.*

As used herein, a "biomass renewable energy source plant" or "biomass for the production of liquid fuel molecules and other chemicals" means a plant having or producing material (either raw or processed) that comprises stored solar energy that can be converted to electrical energy, liquid fuels, and other useful chemicals. In general terms, such plants comprise dedicated energy crops as well as agricultural and woody plants. Examples of biomass renewable energy source plants include: *Panicum virgatum* (switchgrass), *Sorghum bicolor* (sorghum, sudangrass), *Miscanthus giganteus* (miscanthus), *Saccharum* spp. (energycane), *Populus balsamifera* (poplar), *Andropogon gerardii* (big bluestem), *Pennisetum purpureum* (elephant grass), *Phalaris arundinacea* (reed canarygrass), *Cynodon dactylon* (bermudagrass), *Festuca arundinacea* (tall fescue), *Spartina pectinata* (prairie cord-grass), *Medicago sativa* (alfalfa), *Arundo donax* (giant reed), *Secale cereale* (rye), *Salix* spp. (willow), *Eucalyptus* spp. (eucalyptus), *Triticosecale* spp. (triticum - wheat X rye), Bamboo, *Zea mays* (corn), *Manihot esculenta* (cassava), *Saccharum* spp. (sugarcane), *Beta vulgaris* (sugarbeet), *Glycine max* (soybean), *Brassica napus* (canola), *Helianthus annuus* (sunflower), *Carthamus tinctorius* (safflower), *Jatropha curcas* (j atropha), *Ricinus communis* (castor), *Elaeis guineensis* (palm), *Linum usitatissimum* (flax), and *Brassica juncea.*
The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the compositions and methods described herein provide transformed seeds (also referred to as "transgenic seed") comprising a polynucleotide that has been introduced into a plant using a vector of the present disclosure, stably incorporated into their genome.

Thus, the methods and compositions of the present disclosure may be used for transformation of any plant species and development of transgenic plants of any species, including, but not limited to, monocots and dicots. Examples of plant species of interest include, but are not limited to, corn (*Zea mays*), *Brassica* spp. (e.g., *B. napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*))*,* sunflower (*Helianthus annuus*), safflower (*Carthamus tinctorius*), wheat (*Triticum aestivum*), soybean (*Glycine max*), tobacco (*Nicotiana tabacum*)*,* potato (*Solanum tuberosum*), peanuts (*Arachis hypogaea*), cotton (*Gossypium barbadense, Gossypium hirsutum*), sweet potato (*Ipomoea batatus*), cassaya (*Manihot esculenta*), coffee (*Coffea* spp.), coconut (*Cocos nucifera*)*,* pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*), guava (*Psidium guajava*), mango (*Mangifera indica*), olive (*Olea europaea*), papaya (*Carica papaya*), cashew (*Anacardium occidentale*), *macadamia* (*Macadamia integrifolia*), almond (*Prunus amygdalus*)*,* sugar beets (*Beta vulgaris*), sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (C. *sativus*), cantaloupe (C. *cantalupensis*), and musk melon (C. *melo*). Ornamentals include azalea (*Rhododendron* spp.), hydrangea (*Macrophylla hydrangea*)*,* hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum.
Conifers that may be employed in practicing the present disclosure include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Eastern or Canadian hemlock (*Tsuga canadensis*); Western hemlock (*Tsuga heterophylla*); Mountain hemlock (*Tsuga mertensiana*); Tamarack or Larch (*Larix occidentalis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*)*.* Eucalyptus species may be employed in practicing the present disclosure, including *E. grandis* (and its hybrids, as *"urograndis"*), *E. globulus, E. camaldulensis, E. tereticornis, E. viminalis, E. nitens, E. saligna and E. urophylla.* Optimally, plants of the present disclosure are crop plants (for example, corn, alfalfa, sunflower, *Brassica,* soybean, cotton, safflower, peanut, sorghum, wheat, millet, tobacco, etc.), more optimally corn and soybean plants, yet more optimally corn plants.

Plants of particular interest include grain plants that provide seeds of interest, oil-seed plants, and leguminous plants. Seeds of interest include, but are not limited to, grain seeds, such as corn, wheat, barley, rice, sorghum, and rye. Oil-seed plants include, but are not limited to, cotton, soybean, safflower, sunflower, *Brassica,* maize, alfalfa, palm, and coconut, Leguminous plants include, but are not limited to, beans and peas. Beans include guar, locust bean, fenugreek, soybean, garden beans, cowpea, mungbean, lima bean, fava bean, lentils, chickpea.

### III. METHODS TO IMPROVE PLANT TRAITS AND CHARACTERISTICS

The present disclosure provides novel compositions and methods for producing transformed plants with increased efficiency. The disclosed methods and compositions can further comprise polynucleotides that provide for improved traits and characteristics. Thus, the present disclosure further provides methods for transformation of a plant, the method comprising the steps of: (a) contacting a tissue from the plant with an *Agrobacterium* strain comprising a first vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) Rhizobiaceae virulence genes *virB1-B11 or r-*vir*B1-B11, virC1-C2* or *r-virC1-C2, virD1-D2* or *r-virD1-D2,* and *virG* or *r-virG,* or variants and derivatives thereof, wherein the vector comprising the virulence genes *r-virB1-B11, r-virC1-C2, r-virD1-D2,* and *r-virG* further comprises a *r-galls* virulence gene, or variants and derivatives thereof, and a second vector comprising T-DNA borders and a polynucleotide sequence of interest for transfer to the plant; (b) co-cultivatiing the tissue with the *Agrobacterium;* and (c) regenerating a transformed plant from the tissue that expresses the polynucleotide sequence of interest; wherein the polynucleotide sequence provides for an improved trait or characteristic.

As used herein, "trait" refers to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g. by measuring uptake of carbon dioxide, or by the observation of the expression level of a gene or genes, e.g., by employing Northern analysis, RT-PCR, microarray gene expression assays, or reporter gene expression systems, or by agricultural observations such as stress tolerance, yield, or pathogen tolerance. An "enhanced trait" as used in describing the aspects of the present disclosure includes, for example, improved or enhanced water use efficiency or drought tolerance, osmotic stress tolerance, high salinity stress tolerance, heat stress tolerance, enhanced cold tolerance, including cold germination tolerance, increased yield, enhanced nitrogen use efficiency, early plant growth and development, late plant growth and development, enhanced seed protein, and enhanced seed oil production.

Any polynucleotide of interest can be used in the methods of the present disclosure. Various changes in phenotype are of interest including, but not limited to, modifying the fatty acid composition in a plant, altering the amino acid content, starch content, or carbohydrate content of a plant, altering a plant's pathogen defense mechanism, affecting kernel size and sucrose loading. The gene of interest may also be involved in regulating the influx of nutrients, and in regulating expression of phytate genes particularly to lower phytate levels in the seed. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

Genes of interest are reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for transformation will change accordingly. General categories of genes of interest include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, sterility, grain characteristics, and commercial products. Genes of interest include, generally, those involved in oil, starch, carbohydrate, or nutrient metabolism as well as those affecting kernel size and sucrose loading.

Polynucleotides introduced into a target tissue by the disclosed methods and compositions can be operably linked to a suitable promoter. A target tissue may include, but is not limited to, a somatic embryo, mature seeds, meristems, leaf explant, seeds, suspension cultures, embryos, meristematic regions, callus tissue, leaves, roots, shoots, gametophytes, sporophytes, pollen, microspores and other plant explants. "Promoter" means a region of DNA that is upstream from the start of transcription and is involved in recognition and binding of RNA polymerase and other proteins to initiate transcription. A "plant promoter" is a promoter capable of initiating transcription in plant cells whether or not its origin is a plant cell. Exemplary plant promoters include, but are not limited to, those that are obtained from plants, plant viruses, and bacteria which comprise genes expressed in plant cells such as *Agrobacterium* or *Rhizobium.* Examples of promoters under developmental control include promoters that preferentially initiate transcription in certain tissues, such as leaves, roots, or seeds. Such promoters are referred to as "tissue preferred". Promoters which initiate transcription only in certain tissues are referred to as "tissue specific". A "cell type" specific promoter primarily drives expression in certain cell types in one or more organs, for example, vascular cells in roots or leaves. An "inducible" or "repressible" promoter can be a promoter which is under either environmental or exogenous control. Examples of environmental conditions that may effect transcription by inducible promoters include anaerobic conditions, or certain chemicals, or the presence of light. Alternatively, exogenous control of an inducible or repressible promoter can be affected by providing a suitable chemical or other agent that via interaction with target polypeptides result in induction or repression of the promoter. Tissue specific, tissue preferred, cell type specific, and inducible promoters constitute the class of "non-constitutive" promoters. A "constitutive" promoter is a promoter which is active under most conditions. As used herein, "antisense orientation" includes reference to a polynucleotide sequence that is operably linked to a promoter in an orientation where the antisense strand is transcribed. The antisense strand is sufficiently complementary to an endogenous transcription product such that translation of the endogenous transcription product is often inhibited. "Operably linked" refers to the association of two or more nucleic acid fragments on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Pat. Nos. 5,703,049, 5,885,801, 5,885,802, and 5,990,389. Another example is lysine and/or sulfur rich seed protein encoded by the soybean 2S albumin described in U.S. Pat. No. 5,850,016, and the chymotrypsin inhibitor from barley, described in Williamson et al. (1987) Eur. J. Biochem. 165:99-106.

Derivatives of the coding sequences can be made by site-directed mutagenesis to increase the level of preselected amino acids in the encoded polypeptide. For example, methionine-rich plant proteins such as from sunflower seed (Lilley et al. (1989) Proceedings of the World Congress on Vegetable Protein Utilization in Human Foods and Animal Feedstuffs, ed. Applewhite (American Oil Chemists Society, Champaign, Ill.), pp. 497-502); corn (Pedersen et al. (1986) J. Biol. Chem. 261:6279; Kirihara et al. (1988) Gene 71:359); and rice (Musumura et al. (1989) Plant Mol. Biol. 12:123) could be used. Other agronomically important genes encode latex, Floury 2, growth factors, seed storage factors, and transcription factors.

Insect resistance genes may confer resistance to pests such as rootworm, cutworm, and European Corn Borer, which cause significant crop damage resulting int have great yield drag. Such genes include, for example, *Bacillus thuringiensis* toxic protein genes (U.S. Pat. Nos. 5,366,892; 5,747,450; 5,736,514; 5,723,756; 5,593,881; and Geiser et al. (1986) Gene 48:109).

Genes encoding disease resistance traits include detoxification genes, such as against fumonosin (U.S. Pat. No. 5,792,931); and avirulence (avr) and disease resistance (R) genes (Jones et al. (1994) Science 266:789; Martin et al. (1993) Science 262:1432; and Mindrinos et al. (1994) Cell 78:1089).

Herbicide resistance traits may include genes coding for resistance to herbicides that act to inhibit the action of acetolactate synthase (ALS), in particular the sulfonylurea-type herbicides (e.g., the acetolactate synthase (ALS) gene containing mutations leading to such resistance, in particular the S4 and/or Hra mutations), genes coding for resistance to herbicides that act to inhibit action of glutamine synthase, such as phosphinothricin or basta (e.g., the bar gene), glyphosate (e.g., the EPSPS gene and the GAT gene; see, for example, U.S. Publication No. 20040082770 and WO 03/092360) or other such genes known in the art. The bar gene encodes resistance to the herbicide basta, the nptII gene encodes resistance to the antibiotics kanamycin and geneticin, and the ALS-gene mutants encode resistance to the herbicide chlorsulfuron.

Sterility genes can also be encoded in an expression cassette and provide an alternative to physical detasseling. Examples of genes used in such ways include male tissue-preferred genes and genes with male sterility phenotypes such as QM, described in U.S. Pat. No. 5,583,210. Other genes include kinases and those encoding compounds toxic to either male or female gametophytic development.

The quality of grain is reflected in traits such as levels and types of oils, saturated and unsaturated, quality and quantity of essential amino acids, and levels of cellulose. In corn, modified hordothionin proteins are described in U.S. Pat. Nos. 5,703,049, 5,885,801, 5,885,802, and 5,990,389.

Commercial traits can also be encoded on a gene or genes for improved trait composition for example, starch for ethanol production, or enhanced expression of proteins. Another important commercial use of transformed plants is the production of polymers and bioplastics such as described in U.S. Pat. No. 5,602,321. Genes such as β-Ketothiolase, PHBase (polyhydroxyburyrate synthase), and acetoacetyl-CoA reductase (see Schubert et al. (1988) J. Bacteriol. 170:5837-5847) facilitate expression of polyhyroxyalkanoates (PHAs).

Exogenous products include plant enzymes and products as well as those from other sources including prokaryotes and other eukaryotes. Such products include enzymes, cofactors, and hormones. The level of proteins, particularly modified proteins having improved amino acid distribution to improve the nutrient value of the plant, can be increased. This is achieved by the expression of such proteins having enhanced amino acid content.

In an aspect, further agronomic traits of interest that can be introduced into a target tissue with increased efficiency and speed are such traits as increased yield or other traits that provide increased plant value, including, for example, improved seed quality. Of particular interest are traits that provide improved or enhanced water use efficiency or drought tolerance, osmotic stress tolerance, high salinity stress tolerance, heat stress tolerance, enhanced cold tolerance, including cold germination tolerance, increased yield, enhanced nitrogen use efficiency, early plant growth and development, late plant growth and development, enhanced seed protein, and enhanced seed oil production.

Many agronomic traits can affect "yield", including without limitation, plant height, pod number, pod position on the plant, number of internodes, incidence of pod shatter, grain size, efficiencyof nodulation and nitrogen fixation, efficiency of nutrient assimilation, resistance to biotic and abiotic stress, carbon assimilation, plant architecture, resistance to lodging, percent seed germination, seedling vigor, and juvenile traits. Other traits that can affect yield include, efficiency of germination (including germination in stressed conditions), growth rate (including growth rate in stressed conditions), ear number, seed number per ear, seed size, composition of seed (starch, oil, protein) and characteristics of seed fill. Also of interest is the generation of transgenic plants that demonstrate desirable phenotypic properties that may or may not confer an increase in overall plant yield. Such properties include enhanced plant morphology, plant physiology or improved components of the mature seed harvested from the transgenic plant.

"Increased yield" of a transgenic plant of the present disclosure may be evidenced and measured in a number of ways, including test weight, seed number per plant, seed weight, seed number per unit area (i.e. seeds, or weight of seeds, per acre), bushels per acre, tons per acre, and kilo per hectare. For example, maize yield may be measured as production of shelled corn kernels per unit of production area, e.g. in bushels per acre or metric tons per hectare, often reported on a moisture adjusted basis, *e.g.,* at 15.5% moisture. Increased yield may result from improved utilization of key biochemical compounds, such as nitrogen, phosphorous and carbohydrate, or from improved tolerance to environmental stresses, such as cold, heat, drought, salt, and attack by pests or pathogens. Trait-enhancing recombinant DNA may also be used to provide transgenic plants having improved growth and development, and ultimately increased yield, as the result of modified expression of plant growth regulators or modification of cell cycle or photosynthesis pathways.

Many agronomic traits can affect "yield", including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Other traits that can affect yield include, but are not limited to, efficiency of germination (including germination in stressed conditions), growth rate (including growth rate in stressed conditions), ear number, seed number per ear, seed size, composition of seed (starch, oil, protein) and characteristics of seed fill. Also of interest is the generation of transgenic plants that demonstrate desirable phenotypic properties that may or may not confer an increase in overall plant yield. Such properties include, but are not limited to, enhanced plant morphology, plant physiology and improved components of the mature seed harvested from the transgenic plant.

### IV. METHODS TO SUPPRESS GENES

In an aspect, the disclosed methods and compositions can be used to introduce into a plant cell polynucleotides useful for gene suppression of a target gene in a plant derived from the plant cell. Reduction of the activity of specific genes (also known as gene silencing or gene suppression) is desirable for several aspects of genetic engineering in plants. Many techniques for gene silencing are well known to one of skill in the art, including but not limited to antisense technology (see, e.g., Sheehy et al. (1988) Proc. Natl. Acad. Sci. USA 85:8805-8809; and U.S. Pat. Nos. 5,107,065; 5,453,566; and 5,759,829); cosuppression (e.g., Taylor (1997) Plant Cell 9:1245; Jorgensen (1990) Trends Biotech. 8(12):340-344; Flavell (1994) Proc. Natl. Acad. Sci. USA 91:3490-3496; Finnegan et al. (1994) Bio/Technology 12: 883-888; and Neuhuber et al. (1994) Mol. Gen. Genet. 244:230-241); RNA interference (Napoli et al. (1990) Plant Cell 2:279-289; U.S. Pat. No. 5,034,323; Sharp (1999) Genes Dev. 13:139-141; Zamore et al. (2000) Cell 101:25-33; Javier (2003) Nature 425:257-263; and, Montgomery et al. (1998) Proc. Natl. Acad. Sci. USA 95:15502-15507), virus-induced gene silencing (Burton, et al. (2000) Plant Cell 12:691-705; and Baulcombe (1999) Curr. Op. Plant Bio. 2:109-113); target-RNA-specific ribozymes (Haseloff et al. (1988) Nature 334: 585-591); hairpin structures (Smith et al. (2000) Nature 407:319-320; WO 99/53050; WO 02/00904; and WO 98/53083); ribozymes (Steinecke et al. (1992) EMBO J. 11:1525; U.S. Pat. No. 4,987,071; and, Perriman et al. (1993) Antisense Res. Dev. 3:253); oligonucleotide mediated targeted modification (e.g., WO 03/076574 and WO 99/25853); Zn-finger targeted molecules (e.g., WO 01/52620; WO 03/048345; and WO 00/42219); artificial micro RNAs (US8106180; Schwab et al. (2006) Plant Cell 18:1121-1133); and other methods or combinations of the above methods known to those of skill in the art.

### V. METHODS TO INTRODUCE GENOME EDITING TECHNOLOGIES INTO PLANTS

In an aspect, the disclosed methods and compositions can be used to introduce into a plant cell polynucleotides useful to target a specific site for modification in the genome of a plant derived from the plant cell. Site specific modifications that can be introduced with the disclosed methods and compositions include those produced using any method for introducing site specific modification, including, but not limited to, through the use of gene repair oligonucleotides (e.g. US Publication 2013/0019349), or through the use of double-stranded break technologies such as TALENs, meganucleases, zinc finger nucleases, and CRISPR-Cas. For example, the disclosed methods and compositions can be used to introduce a CRISPR-Cas system into plant cells, for the purpose of genome modification of a target sequence in the genome of a plant cell or plant derived from the plant cell, for selecting plants, for deleting a base or a sequence, for gene editing, and for inserting a polynucleotide of interest into the genome of a plant derived from a plant cell. Thus, the disclosed methods and compositions can be used together with a CRISPR-Cas system to provide for an effective system for modifying or altering target sites and nucleotides of interest within the genome of a plant, plant cell or seed.

In an aspect, the present disclosure comprises methods and compositions for transformation, wherein the method comprises the steps of: (a) contacting a plant cell with an *Agrobacterium* strain comprising a first vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) *Agrobacterium* virulence genes *virB1-B11*; *virC1-C2*; *virD1-D2*; and *virG* genes; a second vector capable of expressing a guide nucleotide; and a third construct capable of expressing a Cas endonuclease, wherein the guide nucleotide and Cas endonuclease are capable of forming a complex that enables the Cas endonuclease to introduce a double strand break at the target site; (b) co-cultivatiing the tissue with the *Agrobacterium;* and (c) regenerating a transformed plant from the tissue that expresses the polynucleotide sequence of interest.

In an aspect, the Cas endonuclease gene is a plant optimized Cas9 endonuclease, wherein the plant optimized Cas9 endonuclease is capable of binding to and creating a double strand break in a genomic target sequence of the plant genome.

The Cas endonuclease is guided by the guide nucleotide to recognize and optionally introduce a double strand break at a specific target site into the genome of a plant cell. The CRISPR-Cas system provides for an effective system for modifying target sites within the genome of a plant, plant cell or seed. Further provided are methods and compositions employing a guide polynucleotide/Cas endonuclease system to provide an effective system for modifying target sites within the genome of a cell and for editing a nucleotide sequence in the genome of a cell. Once a genomic target site is identified, a variety of methods can be employed to further modify the target sites such that they contain a variety of polynucleotides of interest. The disclosed compositions and methods can be used to introduce a CRISPR-Cas system for editing a nucleotide sequence in the genome of a cell. The nucleotide sequence to be edited (the nucleotide sequence of interest) can be located within or outside a target site that is recognized by a Cas endonuclease.

CRISPR loci (Clustered Regularly Interspaced Short Palindromic Repeats) (also known as SPIDRs-SPacer Interspersed Direct Repeats) constitute a family of recently described DNA loci. CRISPR loci consist of short and highly conserved DNA repeats (typically 24 to 40 bp, repeated from 1 to 140 times-also referred to as CRISPR-repeats) which are partially palindromic. The repeated sequences (usually specific to a species) are interspaced by variable sequences of constant length (typically 20 to 58 by depending on the CRISPR locus (WO2007/025097 published March 1, 2007).

CRISPR loci were first recognized in *E. coli* (Ishino et al. (1987) J. Bacterial. 169:5429-5433; Nakata et al. (1989) J. Bacterial. 171 :3553-3556). Similar interspersed short sequence repeats have been identified in *Haloferax mediterranei, Streptococcus pyogenes, Anabaena,* and *Mycobacterium tuberculosis* (Groenen et al. (1993) Mol. Microbiol. 10:1057-1065; Hoe et al. (1999) Emerg. Infect. Dis. 5:254- 263; Masepohl et al. (1996) Biochim. Biophys. Acta 1307:26-30; Mojica et al. (1995) Mol. Microbiol. 17:85-93). The CRISPR loci differ from other SSRs by the structure of the repeats, which have been termed short regularly spaced repeats (SRSRs) (Janssen et al. (2002) OMICS J. Integ. Biol. 6:23-33; Mojica et al. (2000) Mol. Microbiol. 36:244-246). The repeats are short elements that occur in clusters, that are always regularly spaced by variable sequences of constant length (Mojica et al. (2000) Mol. Microbiol. 36:244-246).

Cas gene includes a gene that is generally coupled, associated or close to or in the vicinity of flanking CRISPR loci. The terms "Cas gene" and "CRISPR-associated (Cas) gene" are used interchangeably herein. A comprehensive review of the Cas protein family is presented in Haft et al. (2005) Computational Biology, PLoS Comput Biol 1 (6): e60. doi:10.1371 / journal.pcbi.0010060.

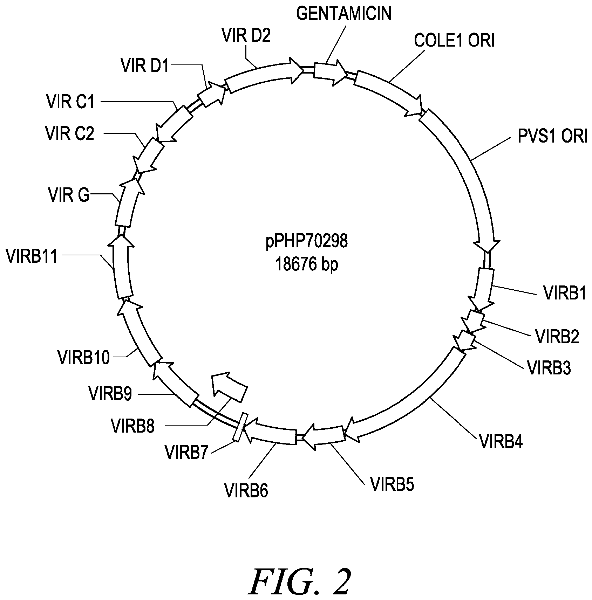

In addition to the four initially described gene families, an additional 41 CRISPR-associated (Cas) gene families have been described in WO/2015/026883. This reference shows that CRISPR systems belong to different classes, with different repeat patterns, sets of genes, and species ranges. The number of Cas genes at a given CRISPR locus can vary between species. Cas endonuclease relates to a Cas protein encoded by a Cas gene, wherein the Cas protein is capable of introducing a double strand break into a DNA target sequence. The Cas endonuclease is guided by the guide polynucleotide to recognize and optionally introduce a double strand break at a specific target site into the genome of a cell. As used herein, the term "guide polynucleotide/Cas endonuclease system" includes a complex of a Cas endonuclease and a guide polynucleotide that is capable of introducing a double strand break into a DNA target sequence. The Cas endonuclease unwinds the DNA duplex in close proximity of the genomic target site and cleaves both DNA strands upon recognition of a target sequence by a guide nucleotide, but only if the correct protospacer-adjacent motif (PAM) is approximately oriented at the 3' end of the target sequence (see Figure 2A and Figure 2B of WO/2015/026883, published February 26, 2015).

In an aspect, the Cas endonuclease gene is a Cas9 endonuclease , such as, but not limited to, Cas9 genes listed and disclosed in WO2007/025097, published March 1 , 2007. In another aspect, the Cas endonuclease gene is plant, maize or soybean optimized Cas9 endonuclease, such as, but not limited to those disclosed WO/2015/026883. In another aspect, the Cas endonuclease gene is operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region.

In an aspect, the Cas endonuclease gene is a Cas9 endonuclease gene, or any functional fragment or variant thereof, disclosed in WO/2015/026883.
The terms "functional fragment," "fragment that is functionally equivalent," and "functionally equivalent fragment" are used interchangeably herein. These terms refer to a portion or subsequence of the Cas endonuclease sequence of the present disclosure in which the ability to create a double-strand break is retained.

The terms "functional variant," "variant that is functionally equivalent" and "functionally equivalent variant" are used interchangeably herein. These terms refer to a variant of the Cas endonuclease of the present disclosure in which the ability to create a double-strand break is retained. Fragments and variants and derivatives can be obtained via methods such as site-directed mutagenesis and synthetic construction.

In an aspect, the Cas endonuclease gene is a plant codon optimized *Streptococcus pyogenes* Cas9 gene that can recognize any genomic sequence of the form N(12-30)NGG, which can be targeted.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more. (patent application PCT/US 12/30061 filed on March 22, 2012). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. Meganucleases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521 -7; and Sadowski (1993) FASEB 7:760-7. In some examples the recombinase is from the Integrase or Resolvase families. TAL effector nucleases are a new class of sequence-specific nucleases that can be used to make double-strand breaks at specific target sequences in the genome of a plant or other organism. (Miller, et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprises two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a nonspecific endonuclease domain, for example nuclease domain from a Type Ms endonuclease such as Fokl. Additional functionalities can be fused to the zinc- finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognizes a sequence of 9 contiguous nucleotides for binding upon dimerization, while two sets of zinc finger triplets are used to bind an 18 nucleotide recognition sequence.

Bacteria and Archaea have evolved adaptive immune defenses termed clustered regularly interspaced short palindromic repeats (CRISPR)/CRISPR- associated (Cas) systems that use short RNA to direct degradation of foreign nucleic acids (WO2007/025097published March 1, 2007). The type II CRISPR/Cas system from bacteria employs a crRNA and tracrRNA to guide the Cas endonuclease to its DNA target. The crRNA (CRISPR RNA) contains the region complementary to one strand of the double strand DNA target and base pairs with the tracrRNA (trans-activating CRISPR RNA) forming a RNA duplex that directs the Cas endonuclease to cleave the DNA target.

As used herein, the term "guide nucleotide" relates to a synthetic fusion of two RNA molecules, a crRNA (CRISPR RNA) comprising a variable targeting domain, and a tracrRNA. In an aspect, the guide nucleotide comprises a variable targeting domain of 12 to 30 nucleotide sequences and a RNA fragment that can interact with a Cas endonuclease.
As used herein, the term "guide polynucleotide" relates to a polynucleotide sequence that can form a complex with a Cas endonuclease and enables the Cas endonuclease to recognize and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide nucleotide".

The guide polynucleotide can be a double molecule (also referred to as duplex guide polynucleotide) comprising a first nucleotide sequence domain (referred to as Variable Targeting domain or VT domain) that is complementary to a nucleotide sequence in a target DNA and a second nucleotide sequence domain (referred to as Cas endonuclease recognition domain or CER domain) that interacts with a Cas endonuclease polypeptide. The CER domain of the double molecule guide polynucleotide comprises two separate molecules that are hybridized along a region of complementarity. The two separate molecules can be RNA, DNA, and/or RNA-DNA- combination sequences. In an aspect, the first molecule of the duplex guide polynucleotide comprising a VT domain linked to a CER domain is referred to as "crDNA" (when comprised of a contiguous stretch of DNA nucleotides) or "crRNA" (when comprised of a contiguous stretch of RNA nucleotides), or "crDNA-RNA" (when comprised of a combination of DNA and RNA nucleotides). The crNucleotide can comprise a fragment of the cRNA naturally occurring in Bacteria and Archaea. In an aspect, the size of the fragment of the cRNA naturally occurring in Bacteria and Archaea that is present in a crNucleotide disclosed herein can range from, but is not limited to, 2, 3, 4, 5, 6, 7, 8, 9,10, 11, 12, 13, 14, 15, 16, 17, 18, 19, 20 or more nucleotides.

In an aspect, the second molecule of the duplex guide polynucleotide comprising a CER domain is referred to as "tracrRNA" (when comprised of a contiguous stretch of RNA nucleotides) or "tracrDNA" (when comprised of a contiguous stretch of DNA nucleotides) or "tracrDNA-RNA" (when comprised of a combination of DNA and RNA nucleotides). In an aspect, the RNA that guides the RNA Cas9 endonuclease complex is a duplexed RNA comprising a duplex crRNA-tracrRNA.

The guide polynucleotide can also be a single molecule comprising a first nucleotide sequence domain (referred to as Variable Targeting domain or VT domain) that is complementary to a nucleotide sequence in a target DNA and a second nucleotide domain (referred to as Cas endonuclease recognition domain or CER domain) that interacts with a Cas endonuclease polypeptide. By "domain" it is meant a contiguous stretch of nucleotides that can be RNA, DNA, and/or RNA-DNA- combination sequence. The VT domain and / or the CER domain of a single guide polynucleotide can comprise a RNA sequence, a DNA sequence, or a RNA-DNA-combination sequence. In an aspect the single guide polynucleotide comprises a crNucleotide (comprising a VT domain linked to a CER domain) linked to a tracrNucleotide (comprising a CER domain), wherein the linkage is a nucleotide sequence comprising a RNA sequence, a DNA sequence, or a RNA-DNA combination sequence. The single guide polynucleotide being comprised of sequences from the crNucleotide and tracrNucleotide may be referred to as "single guide nucleotide" (when comprised of a contiguous stretch of RNA nucleotides) or "single guide DNA" (when comprised of a contiguous stretch of DNA nucleotides) or "single guide nucleotide-DNA" (when comprised of a combination of RNA and DNA nucleotides). In an aspect of the disclosure, the single guide nucleotide comprises a cRNA or cRNA fragment and a tracrRNA or tracrRNA fragment of the type II CRISPR/Cas system that can form a complex with a type II Cas endonuclease, wherein the guide nucleotide Cas endonuclease complex can direct the Cas endonuclease to a plant genomic target site, enabling the Cas endonuclease to introduce a double strand break into the genomic target site. One aspect of using a single guide polynucleotide versus a duplex guide polynucleotide is that only one expression cassette needs to be made to express the single guide polynucleotide.

The term "variable targeting domain" or "VT domain" is used interchangeably herein and includes a nucleotide sequence that is complementary to one strand (nucleotide sequence) of a double strand DNA target site. The % complementation between the first nucleotide sequence domain (VT domain) and the target sequence can be at least 50%, 51 %, 52%, 53%, 54%, 55%, 56%, 57%, 58%, 59%, 60%, 61 %, 62%, 63%, 63%, 65%, 66%, 67%, 68%, 69%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, 99% or 100%. The variable target domain can be at least 12, 13, 14, 15, 16, 17, 18, 19, 20, 21, 22, 23, 24, 25, 26, 27, 28, 29 or 30 nucleotides in length. In an aspect, the variable targeting domain comprises a contiguous stretch of 12 to 30 nucleotides. The variable targeting domain can be comprised of a DNA sequence, a RNA sequence, a modified DNA sequence, a modified RNA sequence, or any combination thereof.

The term "Cas endonuclease recognition domain" or "CER domain" of a guide polynucleotide is used interchangeably herein and includes a nucleotide sequence (such as a second nucleotide sequence domain of a guide polynucleotide), that interacts with a Cas endonuclease polypeptide. The CER domain can be comprised of a DNA sequence, a RNA sequence, a modified DNA sequence, a modified RNA sequence (see for example modifications described herein), or any combination thereof.

The nucleotide sequence linking the crNucleotide and the tracrNucleotide of a single guide polynucleotide can comprise a RNA sequence, a DNA sequence, or a RNA-DNA combination sequence. In an aspect, the nucleotide sequence linking the crNucleotide and the tracrNucleotide of a single guide polynucleotide can be at least 3, 4, 5, 6, 7, 8, 9, 10, 11 , 12, 13, 14, 15, 16, 17, 18, 19, 20, 21, 22, 23, 24, 25, 26, 27, 28, 29, 30, 31 , 32, 33, 34, 35, 36, 37, 38, 39, 40, 41 , 42, 43, 44, 45, 46, 47, 48, 49, 50, 51 , 52, 53, 54, 55, 56, 57, 58, 59, 60, 61 , 62, 63, 64, 65, 66, 67, 68, 69, 70, 71 , 72, 73, 74, 75, 76, 77, 78, 78, 79, 80, 81 , 82, 83, 84, 85, 86, 87, 88, 89, 90, 91, 92, 93, 94, 95, 96, 97, 98, 99 or 100 nucleotides in length. In another aspect, the nucleotide sequence linking the crNucleotide and the tracrNucleotide of a single guide polynucleotide can comprise a tetraloop sequence, such as, but not limiting to a GAAA tetraloop sequence.

Nucleotide sequence modification of the guide polynucleotide, VT domain and/or CER domain can be selected from, but not limited to, the group consisting of a 5' cap, a 3' polyadenylated tail, a riboswitch sequence, a stability control sequence, a sequence that forms a dsRNA duplex, a modification or sequence that targets the guide polynucleotide to a subcellular location, a modification or sequence that provides for tracking , a modification or sequence that provides a binding site for proteins, a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

In aspects, the guide nucleotide and Cas endonuclease are capable of forming a complex that enables the Cas endonuclease to introduce a double strand break at a DNA target site.

In aspects, the variable target domain is 12, 13, 14, 15, 16, 17, 18, 19, 20, 21, 22, 23, 24, 25, 26, 27, 28, 29 or 30 nucleotides in length.

In aspects, the guide nucleotide comprises a cRNA (or cRNA fragment) and a tracrRNA (or tracrRNA fragment) of the type II CRISPR/Cas system that can form a complex with a type II Cas endonuclease, wherein the guide nucleotide Cas endonuclease complex can direct the Cas endonuclease to a plant genomic target site, enabling the Cas endonuclease to introduce a double strand break into the genomic target site. In an aspect the guide nucleotide can be introduced into a plant or plant cell directly using any method known in the art such as, but not limited to, particle bombardment or topical applications.
In aspects, the guide nucleotide can be introduced indirectly by introducing a recombinant DNA molecule comprising the corresponding guide DNA sequence operably linked to a plant specific promoter that is capable of transcribing the guide nucleotide in the plant cell. The term "corresponding guide DNA" includes a DNA molecule that is identical to the RNA molecule but has a "T" substituted for each "U" of the RNA molecule.

In aspects, the guide nucleotide is introduced via particle bombardment or using the disclosed methods and compositions for *Agrobacterium* transformation of a recombinant DNA construct comprising the corresponding guide DNA operably linked to a plant U6 polymerase III promoter.

In aspects, the RNA that guides the RNA Cas9 endonuclease complex is a duplexed RNA comprising a duplex crRNA-tracrRNA. One advantage of using a guide nucleotide versus a duplexed crRNA- tracrRNA is that only one expression cassette needs to be made to express the fused guide nucleotide.

The terms "target site," "target sequence," "target DNA," "target locus," "genomic target site," "genomic target sequence," and "genomic target locus" are used interchangeably herein and refer to a polynucleotide sequence in the genome (including choloroplastic and mitochondrial DNA) of a plant cell at which a double- strand break is induced in the plant cell genome by a Cas endonuclease. The target site can be an endogenous site in the plant genome, or alternatively, the target site can be heterologous to the plant and thereby not be naturally occurring in the genome, or the target site can be found in a heterologous genomic location compared to where it occurs in nature.
As used herein, terms "endogenous target sequence" and "native target sequence" are used interchangeably herein to refer to a target sequence that is endogenous or native to the genome of a plant and is at the endogenous or native position of that target sequence in the genome of the plant. In an aspect, the target site can be similar to a DNA recognition site or target site that is specifically recognized and/or bound by a double-strand break inducing agent such as a LIG3-4 endonuclease (US patent publication 2009- 0133152 A1 (published May 21 , 2009) or a MS26++ meganuclease (U.S. patent application 13/526912 filed June 19, 2012).

An "artificial target site" or "artificial target sequence" are used interchangeably herein and refer to a target sequence that has been introduced into the genome of a plant. Such an artificial target sequence can be identical in sequence to an endogenous or native target sequence in the genome of a plant but be located in a different position (i.e., a non-endogenous or non-native position) in the genome of a plant.
An "altered target site," "altered target sequence," "modified target site," and "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

### VI. METHODS TO INTRODUCE NUCLEOTIDES FOR SITE-SPECIFIC INTEGRATION

In an aspect, the disclosed methods and compositions can be used to introduce into a plant cell with increased efficiency and speed polynucleotides useful for the targeted integration of nucleotide sequences into a plant derived from the plant cell. For example, the disclosed methods and compositions can be used to introduce transfer cassettes comprising nucleotide sequences of interest flanked by non-identical recombination sites to transform a plant comprising a target site. In an aspect, the target site contains at least a set of non-identical recombination sites corresponding to those on the transfer cassette. The exchange of the nucleotide sequences flanked by the recombination sites is effected by a recombinase. Thus, the disclosed methods and compositions can be used for the introduction of transfer cassettes for targeted integration of nucleotide sequences, wherein the transfer cassettes which are flanked by non-identical recombination sites are recognized by a recombinase that recognizes and implements recombination at the nonidentical recombination sites. Accordingly, the disclosed methods and compositions can be used to improve efficiency and speed of development of plants, derived from plant cells, containing non-identical recombination sites.

In an aspect, the present disclosure further provides methods for transformation of a plant, wherein the method comprises introducing a polynucleotide of interest into a target site in the genome of a plant cell, the method comprising the steps of: (a) contacting an explant from the plant with an *Agrobacterium* strain comprising a first vector comprising: (i) an origin of replication for propagation and stable maintenance in *Escherichia coli*; (ii) an origin of replication for propagation and stable maintenance in *Agrobacterium* spp.; (iii) a selectable marker gene; and (iv) *Agrobacterium* virulence genes *virB1-B11*; *virC1-C2*; *virD1-D2*; and *virG* genes, and a second vector comprising a transfer cassette comprising the polynucleotide of interest flanked by nonidentical recombination sites; (b) co-cultivatiing the tissue with the *Agrobacterium;* and (c) regenerating a transformed plant from the tissue that expresses the polynucleotide sequence of interest; wherein the explant is derived from a plant with a genome comprising a target site flanked by non identical recombination sites which correspond to the flanking sites of the transfer cassette. The method can further comprise providing a recombinase that recognizes and implements recombination at the nonidentical recombination sites, the recombinase being provided to the plant explant, a plantlet derived from the somatic embryo, or a plant derived from a plantlet derived from a plant cell.

Thus, the disclosed methods and compositions can further comprise compositions and methods for the directional, targeted integration of exogenous nucleotides into a transformed plant. In an aspect, the disclosed methods use novel recombination sites in a gene targeting system which facilitates directional targeting of desired genes and nucleotide sequences into corresponding recombination sites previously introduced into the target plant genome.

In an aspect, a nucleotide sequence flanked by two non-identical recombination sites is introduced into one or more cells of an explant derived from the target organism's genome establishing a target site for insertion of nucleotide sequences of interest. Once a stable plant or cultured tissue is established, a second construct, or nucleotide sequence of interest, flanked by corresponding recombination sites as those flanking the target site, is introduced into the stably transformed plant or tissues in the presence of a recombinase protein. This process results in exchange of the nucleotide sequences between the non-identical recombination sites of the target site and the transfer cassette.

It is recognized that the transformed plant prepared in this manner may comprise multiple target sites; *i.e.,* sets of non-identical recombination sites. In this manner, multiple manipulations of the target site in the transformed plant are available. By target site in the transformed plant is intended a DNA sequence that has been inserted into the transformed plant's genome and comprises non-identical recombination sites.

Examples of recombination sites for use in the disclosed method are known in the art and include FRT sites (See, for example, Schlake and Bode (1994) Biochemistry 33: 12746- 12751; Huang et al. (1991) Nucleic Acids Research 19: 443-448; Paul D. Sadowski (1995) In Progress in Nucleic Acid Research and Molecular Biology vol. 51, pp. 53-91; Michael M. Cox (1989) In Mobile DNA, Berg and Howe (eds) American Society of Microbiology, Washington D. C., pp. 116-670; Dixon *et al.* (1995) 18: 449-458; Umlauf and Cox (1988) The EMBO Journal 7: 1845-1852; Buchholz et al. (1996) Nucleic Acids Research 24: 3118- 3119; Kilby et al. (1993) Trends Genet. 9: 413-421: Rossant and Geagy (1995) Nat. Med. 1: 592-594; Albert et al. (1995) The Plant J. 7: 649-659: Bayley et al. (1992) Plant Mol. Biol. 18: 353-361; Odell et al. (1990) Mol. Gen. Genet. 223: 369-378; and Dale and Ow (1991) Proc. Natl. Acad. Sci. USA 88: 10558-105620.) ; Lox (Albert et al. (1995) Plant J. 7: 649-659; Qui et al. (1994) Proc. Natl. Acad. Sci. USA 91: 1706-1710; Stuurman et al. (1996) Plant Mol. Biol. 32: 901-913; Odell et al. (1990) Mol. Gen. Gevet. 223: 369-378; Dale et al. (1990) Gene 91: 79-85; and Bayley et al. (1992) Plant Mol. Biol. 18: 353-361.) The two-micron plasmid, found in most naturally occurring strains of Saccharomyces cerevisiae, encodes a site-specific recombinase that promotes an inversion of the DNA between two inverted repeats. This inversion plays a central role in plasmid copy-number amplification.

The protein, designated FLP protein, catalyzes site-specific recombination events. The minimal recombination site (FRT) has been defined and contains two inverted 13-base pair (bp) repeats surrounding an asymmetric 8- bp spacer. The FLP protein cleaves the site at the junctions of the repeats and the spacer and is covalently linked to the DNA via a 3'phosphate. Site specific recombinases like FLP cleave and religate DNA at specific target sequences, resulting in a precisely defined recombination between two identical sites. To function, the system needs the recombination sites and the recombinase. No auxiliary factors are needed. Thus, the entire system can be inserted into and function in plant cells. The yeast FLP\FRT site specific recombination system has been shown to function in plants. To date, the system has been utilized for excision of unwanted DNA. See, Lyznik et at. (1993) Nucleic Acid Res. 21: 969-975. In contrast, the present disclosure utilizes non-identical FRTs for the exchange, targeting, arrangement, insertion and control of expression of nucleotide sequences in the plant genome.

In an aspect, a transformed organism of interest, such as an explant from a plant, containing a target site integrated into its genome is needed. The target site is characterized by being flanked by non-identical recombination sites. A targeting cassette is additionally required containing a nucleotide sequence flanked by corresponding non-identical recombination sites as those sites contained in the target site of the transformed organism. A recombinase which recognizes the non-identical recombination sites and catalyzes site-specific recombination is required.

It is recognized that the recombinase can be provided by any means known in the art. That is, it can be provided in the organism or plant cell by transforming the organism with an expression cassette capable of expressing the recombinase in the organism, by transient expression, or by providing messenger RNA (mRNA) for the recombinase or the recombinase protein.

By "non-identical recombination sites" it is intended that the flanking recombination sites are not identical in sequence and will not recombine or recombination between the sites will be minimal. That is, one flanking recombination site may be a FRT site where the second recombination site may be a mutated FRT site. The non-identical recombination sites used in the methods of the present disclosure prevent or greatly suppress recombination between the two flanking recombination sites and excision of the nucleotide sequence contained therein. Accordingly, it is recognized that any suitable non-identical recombination sites may be utilized in the present disclosure, including FRT and mutant FRT sites, FRT and lox sites, lox and mutant lox sites, as well as other recombination sites known in the art.

By suitable non-identical recombination site implies that in the presence of active recombinase, excision of sequences between two non-identical recombination sites occurs, if at all, with an efficiency considerably lower than the recombinationally-mediated exchange targeting arrangement of nucleotide sequences into the plant genome. Thus, suitable non-identical sites for use in the present disclosure include those sites where the efficiency of recombination between the sites is low; for example, where the efficiency is less than about 30 to about 50%, preferably less than about 10 to about 30%, more preferably less than about 5 to about 10 %.

As noted above, the recombination sites in the targeting cassette correspond to those in the target site of the transformed plant. That is, if the target site of the transformed plant contains flanking non-identical recombination sites of FRT and a mutant FRT, the targeting cassette will contain the same FRT and mutant FRT non-identical recombination sites.

It is furthermore recognized that the recombinase, which is used in the disclosed methods, will depend upon the recombination sites in the target site of the transformed plant and the targeting cassette. That is, if FRT sites are utilized, the FLP recombinase will be needed. In the same manner, where lox sites are utilized, the Cre recombinase is required. If the non-identical recombination sites comprise both a FRT and a lox site, both the FLP and Cre recombinase will be required in the plant cell.

The FLP recombinase is a protein which catalyzes a site-specific rection that is involved in amplifying the copy number of the two micron plasmid of S. cerevisiae during DNA replication. FLP protein has been cloned and expressed. See, for example, Cox (1993) Proc. Natl. Acad. Sci. U. S. A. 80: 4223-4227. The FLP recombinase for use in the disclosed methods for targeted integration can be derived from the genus *Saccharomyces.* It may be preferable to synthesize the recombinase using plant preferred codons for optimum expression in a plant of interest. See, for example, U. S. Application Serial No. 08/972,258 filed November 18, 1997, entitled "Novel Nucleic Acid Sequence Encoding FLP Recombinase".

The bacteriophage recombinase Cre catalyzes site-specific recombination between two lox sites. The Cre recombinase is known in the art. See, for example, Guo et al. (1997) Nature 389: 40-46; Abremski et al. (1984) J. Biol. Chem. 259: 1509-1514; Chen et al. (1996) Somat. Cell Mol. Genet. 22: 477-488; and Shaikh et al. (1977) J. Biol. Chem. 272: 5695-5702. Such Cre sequence(s) may also be synthesized using plant preferred codons.

Where appropriate, the nucleotide sequences to be inserted in the plant genome may be optimized for increased expression in the transformed plant. Where mammalian, yeast, or bacterial genes are used in the present disclosure, they can be synthesized using plant preferred codons for improved expression. It is recognized that for expression in monocots, dicot genes can also be synthesized using monocot preferred codons. Methods are available in the art for synthesizing plant preferred genes. See, for example, U. S. Patent Nos. 5,380,831,5,436,391, and Murray et al. (1989) Nucleic Acids Res. 17 : 477-498. The plant preferred codons may be determined from the codons utilized more frequently in the proteins expressed in the plant of interest. It is recognized that monocot or dicot preferred sequences may be constructed as well as plant preferred sequences for particular plant species. See, for example, EPA 0359472; EPA 0385962; WO 91/16432; Perlak et al. (1991) Proc. Natl. Acad. Sci. USA, 88: 3324-3328; and Murray et al. (1989) Nucleic Acids Research, 17: 477-498. U. S. Patent No. 5,380,831; and U. S. Patent No. 5,436,391. It is further recognized that all or any part of the gene sequence may be optimized or synthetic. That is, fully optimized or partially optimized sequences may also be used.

Additional sequence modifications are known to enhance gene expression in a cellular host and can be used in the present disclosure. These include elimination of sequences encoding spurious polyadenylation signals, exon-intron splice site signals, transposon-like repeats, and other such well-characterized sequences, which may be deleterious to gene expression. The G-C content of the sequence may be adjusted to levels average for a given cellular host, as calculated by reference to known genes expressed in the host cell. When possible, the sequence is modified to avoid predicted hairpin secondary MARNA structures.

The present disclosure also encompasses novel FLP recombination target sites (FRT). The FRT has been identified as a minimal sequence comprising two 13 base pair repeats, separated by an 8 base spacer, as follows: 5'-GAAGTTCCTATTC [TCTAGAAA] GTATAGGAACTTC3' wherein the nucleotides within the brackets indicate the spacer region. The nucleotides in the spacer region can be replaced with a combination of nucleotides, so long as the two 13- base repeats are separated by eight nucleotides. Some substitutions of nucleotides in the spacer region may work better than others and determining which substitutions is within the skill of the art. The eight base pair spacer is involved in DNA-DNA pairing during strand exchange. The asymmetry of the region determines the direction of site alignment in the recombination event, which will subsequently lead to either inversion or excision. As indicated above, most of the spacer can be mutated without a loss of function. See, for example, Schlake and Bode (1994) Biochemistry 33: 12746-12751.

Novel FRT mutant sites can be used in the practice of the disclosed methods. Such mutant sites may be constructed by PCR-based mutagenesis. Although mutant FRT sites are known (*e.g.*, see WO/1999/025821, published May 27, 1999), it is recognized that other mutant FRT sites may be used in the practice of the present disclosure. In aspects, the methods and compositions of the present disclosure can use non-identical recombination sites or FRT sites for targeted insertion and expression of nucleotide sequences in a plant genome.

As discussed above, bringing genomic DNA containing a target site with non- identical recombination sites together with a vector containing a transfer cassette with corresponding non-identical recombination sites, in the presence of the recombinase, results in recombination. The nucleotide sequence of the transfer cassette located between the flanking recombination sites is exchanged with the nucleotide sequence of the target site located between the flanking recombination sites. In this manner, nucleotide sequences of interest may be precisely incorporated into the genome of the host.

It is recognized that many variations of the present disclosure can be practiced. For example, target sites can be constructed having multiple non-identical recombination sites. Thus, multiple genes or nucleotide sequences can be stacked or ordered at precise locations in the plant genome. Likewise, once a target site has been established within the genome, additional recombination sites may be introduced by incorporating such sites within the nucleotide sequence of the transfer cassette and the transfer of the sites to the target sequence. Thus, once a target site has been established, it is possible to subsequently add sites, or alter sites through recombination.

Another variation includes providing a promoter or transcription initiation region operably linked with the target site in an organism. Preferably, the promoter will be 5' to the first recombination site. By transforming the organism with a transfer cassette comprising a coding region, expression of the coding region will occur upon integration of the transfer cassette into the target site. This aspect provides for a method to select transformed cells, particularly plant cells, by providing a selectable marker sequence as the coding sequence.

Other advantages of the present system include the ability to reduce the complexity of integration of transgenes or transferred DNA in an organism by utilizing transfer cassettes as discussed above and selecting organisms with simple integration patterns. In the same manner, preferred sites within the genome can be identified by comparing several transformation events. A preferred site within the genome includes one that does not disrupt expression of essential sequences and provides for adequate expression of the transgene sequence.

The disclosed methods also provide for means to combine multiple cassettes at one location within the genome. Recombination sites may be added or deleted at target sites within the genome.

Any means known in the art for bringing the three components of the system together may be used in the present disclosure. For example, a plant can be stably transformed to harbor the target site in its genome. The recombinase may be transiently expressed or provided. Alternatively, a nucleotide sequence capable of expressing the recombinase may be stably integrated into the genome of the plant. In the presence of the corresponding target site and the recombinase, the transfer cassette, flanked by corresponding non- identical recombination sites, is inserted into the transformed plant's genome.

Alternatively, the components of the system may be brought together by sexually crossing transformed plants. In this aspect, a transformed plant, parent one, containing a target site integrated in its genome can be sexually crossed with a second plant, parent two, that has been genetically transformed with a transfer cassette containing flanking non-identical recombination sites, which correspond to those in plant one. Either plant one or plant two contains within its genome a nucleotide sequence expressing recombinase. The recombinase may be under the control of a constitutive or inducible promoter.

Inducible promoters include those described herein above, as well as, heat-inducible promoters, estradiol-responsive promoters, and chemical inducible promoters. Pathogen inducible promoters include those from pathogenesis-related proteins (PR proteins), which are induced following infection by a pathogen; e. g., PR proteins, SAR proteins, beta-1,3-glucanase, chitinase, etc. See, for example, Redolfi et al. (1983) Neth. J. Plant Pathol. 89: 245-254; Uknes et al. (1992) The Plant Cell 4: 645-656; and Van Loon (1985) Plant Mol. Virol. 4: 111-116. In this manner, expression of recombinase and subsequent activity at the recombination sites can be controlled.

Constitutive promoters for use in expression of genes in plants are known in the art. Such promoters include, but are not limited to 35S promoter of cauliflower mosaic virus (Depicker et al. (1982) Mol. Appl. Genet. 1: 561-573; Odell et al. (1985) Nature 313: 810- 812), ubiquitin promoter (Christensen et al. (1992) Plant Mol. Biol. 18: 675-689), promoters from genes such as ribulose bisphosphate carboxylase (De Almeida et al. (1989) Mol. Gen. Genet. 218: 78-98), actin (McElroy et al. (1990) Plant J. 2: 163-171), histone, and DnaJ (Baszczynski et al. (1997) Maydica 42: 189-201).

The disclosed compositions and methods are useful in targeting the integration of transferred nucleotide sequences to a specific chromosomal site. The nucleotide sequence may encode any nucleotide sequence of interest. Particular genes of interest include those which provide a readily analyzable functional feature to the host cell and/or organism, such as marker genes, as well as other genes that alter the phenotype of the recipient cells. Thus, genes effecting plant growth, height, susceptibility to disease, insects, and nutritional value may be utilized in the present disclosure. The nucleotide sequence also may encode an 'antisense' sequence to turn off or modify gene expression.

It is recognized that the nucleotide sequences may be utilized in a functional expression unit or cassette. By functional expression unit or cassette is intended, the nucleotide sequence of interest with a functional promoter, and in most instances a termination region. There are various ways to achieve the functional expression unit within the practice of the present disclosure. In one aspect of the present disclosure, the nucleic acid of interest is transferred or inserted into the genome as a functional expression unit.

Alternatively, the nucleotide sequence may be inserted into a site within the genome which is 3' to a promoter region. In this latter instance, the insertion of the coding sequence 3' to the promoter region is such that a functional expression unit is achieved upon integration. For convenience, for expression in plants, the nucleic acid encoding target sites and the transfer cassettes, including the nucleotide sequences of interest, can be contained within expression cassettes. The expression cassette will comprise a transcriptional initiation region, or promoter, operably linked to the nucleic acid encoding the peptide of interest. Such an expression cassette is provided with a plurality of restriction sites for insertion of the gene or genes of interest to be under the transcriptional regulation of the regulatory regions.

The transcriptional initiation region, the promoter, may be native or homologous or foreign or heterologous to the host, or could be the natural sequence or a synthetic sequence. By foreign is intended that the transcriptional initiation region is not found in the wild-type host into which the transcriptional initiation region is introduced. Either a native or heterologous promoter may be used with respect to the coding sequence of interest.

The transcriptional cassette may include in the 5'-3' direction of transcription, a transcriptional and translational initiation region, a DNA sequence of interest, and a transcriptional and translational termination region functional in plants. The termination region may be native with the transcriptional initiation region, may be native with the DNA sequence of interest, or may be derived from another source. Convenient termination regions are available from the potato proteinase inhibitor (PinII) gene or sequences from Ti-plasmid of *A. tumefaciens,* such as the nopaline synthase, octopine synthase and opaline synthase termination regions. See also, Guerineau et al., (1991) Mol. Gen. Genet. 262: 141-144; Proudfoot (1991) Cell 64: 671-674; Sanfacon et al. (1991) Genes Dev. 5: 141-149; Mogen et al. (1990) Plant Cell 2: 1261-1272; Munroe et al. (1990) Gene 91: 151-158; Ballas et al. 1989) Nucleic Acids Res. 17 : 7891-7903; Joshi et al. (1987) Nucleic Acid Res. 15: 9627-9639.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5'noncoding region) (Elroy-Stein, O., Fuerst, T. R., and Moss, B. (1989) PNAS USA, 86: 6126-6130); potyvirus leaders, for example, TEV leader (Tobacco Etch Virus) (Allison et al. (1986); MDMV leader (Maize Dwarf Mosaic Virus); Virology, 154: 9-20), and human immunoglobulin heavy-chain binding protein (BiP), (Macejak, D. G., and P. Sarnow (1991) Nature, 353: 90-94; untranslated leader from the coat protein MARNA of alfalfa mosaic virus (AMV RNA 4), (Jobling, S. A., and Gehrke, L., (1987) Nature, 325: 622-625; tobacco mosaic virus leader (TMV), (Gallie et al. (1989) Molecular Biology of RNA, pages 237-256, Gallie et al. (1987) Nucl. Acids Res. 15: 3257-3273; maize chlorotic mottle virus leader (MCMV) (Lornmel, S. A. et al. (1991) Virology, 81: 382-385). See also, Della-Cioppa et al. (1987) Plant Physiology, 84: 965-968; and endogenous maize 5' untranslated sequences. Other methods known to enhance translation can also be utilized, for example, introns.

The expression cassettes may contain one or more than one gene or nucleic acid sequence to be transferred and expressed in the transformed plant. Thus, each nucleic acid sequence will be operably linked to 5' and 3' regulatory sequences. Alternatively, multiple expression cassettes may be provided.

### EXPERIMENTAL

### EXAMPLE 1: VIR PLASMIDS WITH IMPROVED FEATURES.

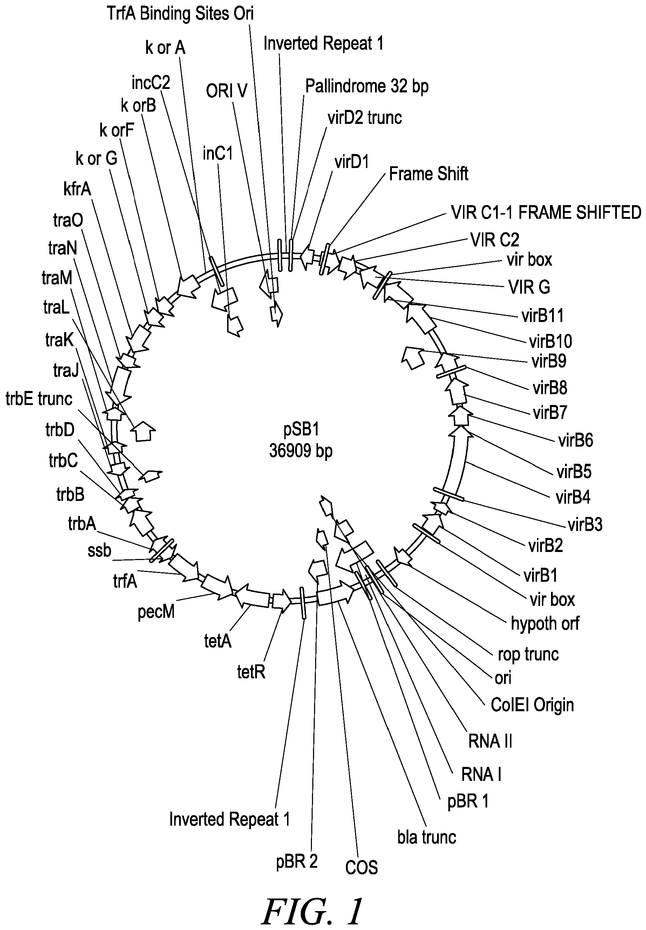

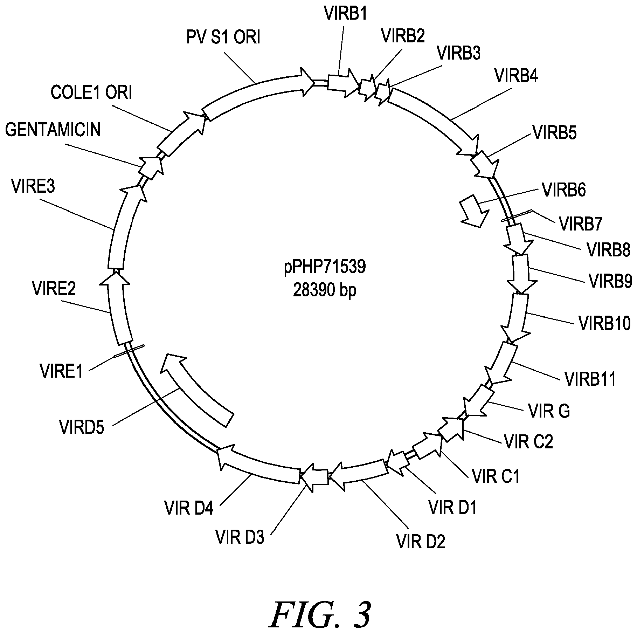

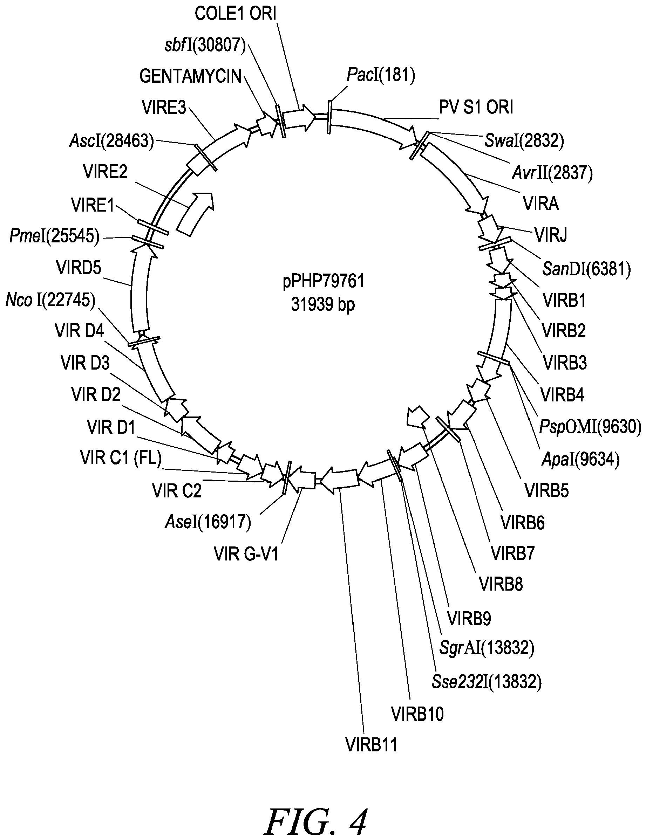

A series of improved superbinary vectors were prepared. Sequence identification numbers (SEQ ID NO:) described herein are listed in Table 23. The vectors, pPHP70298, pPHP71539, and pPHP79761, are shown as plasmid maps in FIGs. 2, 3, and 4, respectively, and have the sequences set forth in SEQ ID NOS: 34, 35, and 36, respectively (see Table 23). For comparison, a plasmid map of the vir gene plasmid pSB1 (Komari, T., et al., Plant Cell Rep. (1990), 9:303-306) is shown in FIG. 1. Among the improvements to pPHP70298 (SEQ ID NO. 34), pPHP71539 (SEQ ID NO. 35), and pPHP79761 SEQ ID NO. 36) is the presence of a smaller, more stable origin of replication, pVS1, instead of a large, unstable origin of replication (RK2). In addition, the frameshift in *virC1-2* operon is repaired, which encodes the overdrive-binding protein for enhanced T-DNA transfer. It is possible that the frame-shift decreases translation of *virC1-2* as the second gene in a dicistronic mRNA. Improved virulence gene functionality was also achieved by introducing either *virD1* and *virD2* (pPHP70298); the complete *virD* operon (*i.e., virD1-D5*) along with the *virE1-3* and *virG* genes (pPHP71539); or the complete *virD* operon (*i.e., virD1-D5*) along with the *virE1-3, virA, virG,* and *virJ* genes (pPHP71539). The arrangement of the vir genes in the pPHP70298 and pPHP71539 vectors (see FIGs. 2 and 3) are a contiguous fragment as present in the parent pTiBo542 plasmid (see FIG. 4). However, in pPHP79761, the arrangement of the *virA* and *virJ* genes are arranged in a continguous fashion (see FIG. 4), whereas these genes are not contiguous in the parent pTiBo542 plasmid (see FIG. 4). In particular, the arrangement of the the *virA* and *virJ* genes in pPHP79761 removes a probable transposable DNA element (IS292) flanked by repeated sequences and four potential coding sequences (see CDS 51-54 in GenBank entry NC 010929). These constructs are smaller than pSB1, despite having an increased number of virulence genes.

Compared to pSB1, nonessential DNA and repetitive elements have been eliminated. For example, the vectors, pPHP70298, pPHP71539, and pPHP79761, do not contain the 7.0 kbp truncated *tra* and *trb* operons or flanking genes included in the 16.2 kb RK2 origin of replication found in pSB1 (see FIG. 1). There were two additional sequences found in pSB1 (see FIG. 1) that were removed in the vectors pPHP70298, pPHP71539, and pPHP79761: (a) a 32 bp palindromic sequence (SEQ ID NO: 61); and (b) a 146 bp inverted repeat sequence (SEQ ID NO: 62). It is believed that these elements in vector backbone decrease overall plasmid stability, and accordingly, their removal from the present vectors should increase overall plasmid stability.

The vectors, pPHP70298, pPHP71539, and pPHP79761, also lack the 2.7 kbp pBR322 fragment found in pSB1 comprising the origin of replication, a beta lactamase coding sequence, and unstable 18 bp poly-G flanked lambda COS sites. The vectors, pPHP70298, pPHP71539, and pPHP79761, instead use a 1.2 kbp ColEl origin of replication which provides for stable maintenance in *Escherichia coli.*

The selectable marker is a gentamycin cassette, which provides for enhanced plasmid stability and faster growth of *Agrobacterium.* The tetracycline resistance gene *tetAR* in pSB1 often leads to slow growth or plasmid-free colonies in *Agrobacterium* strain C58 or mutant *Agrobacterium* colonies resistant to tetracycline (C58 and its derivatives such as AGL0, AGL1, have been described as giving rise to spontaneous *tet* resistant mutants at high frequency; see Luo, Z.K. and Farrand, S.K. (1999) J Bacteriol. 181:618-626). The gentamycin cassette used was the GmR synthetic *aacC1* (based on GenBank Accession No. DQ530421; SEQ ID NO:1), conferring resistance to gentamycin, which does not lead to slow growth or reduced virulence in the recombinant *Agrobacterium* strain harboring the plasmid.

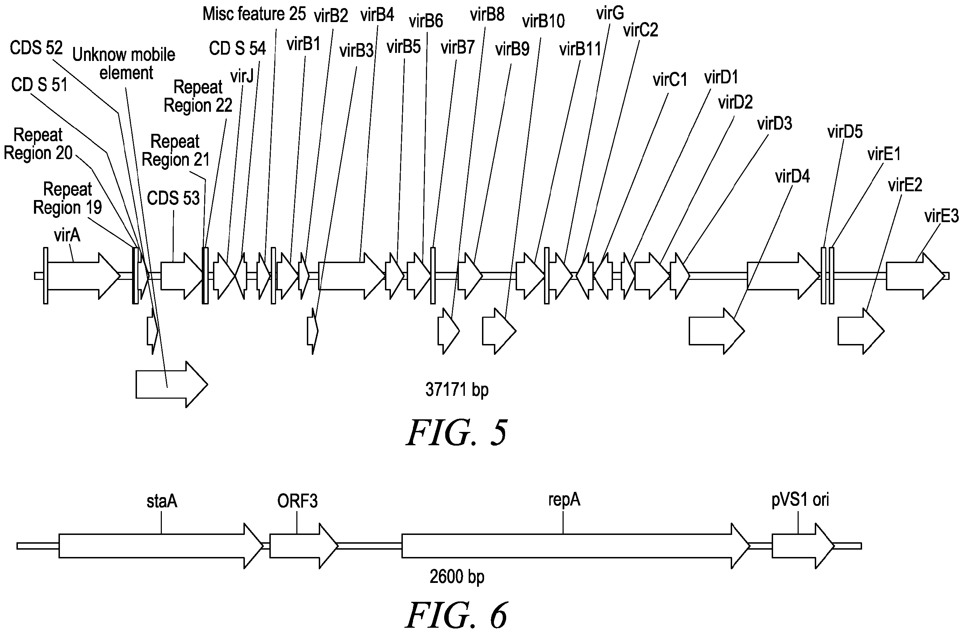

Briefly, the vectors were constructed in a similar manner. The construction of pPHP79761 is described here as an example of the methods used in the construction of pPHP70298 and pPHP71539. The virulence genes were obtained from the *Agrobacterium tumefaciens* Ti plasmid, pTiBo542 (NCBI Reference Sequence: NC_010929.1; see FIG. 5). One skilled in the art would appreciate that vir genes from Agrobacterium Rᵢ plasmid would also be useful in the vectors disclosed herein.

PCR primers were designed to amplify the *virA, virJ,* and *virB1-virE3* coding and regulatory regions from a genomic DNA prep of *Agrobacterium tumefaciens* strain AGL1. The 24 kb virB1-E3 sequence was amplified in 5 pieces to facilitate PCR. Fragments/derivatives were designed with 40 bp overlapping ends to facilitate seamless cloning methods. Unique restriction enzyme sites were included between functional elements to facilitate their exchange with other functionally equivalent elements as shown in FIG. 4. A mobile genetic element labeled as IS292 in the pTiBo542 NCBI Reference Sequence that is present between the virA and virJ genes was not included in pPHP79761 to reduce size and the possibility of its transfer into plants. A gentamicin acetyltransferase gene (based on GenBank Accession No. DQ530421) was synthesized with flanking restriction sites to confer gentamicin resistance to its bacterial hosts. A ColEl origin of replication corresponding to base pairs 2348-3247 of pBR322 (GenBank Accession No. J01749) was included for stable replication in *E. coli.* A 2.6 kb fragment encoding the pVS1 origin of replication (see Stanisich, V. J.Bacteriol. (1984) 129:1227-1233) was included for stable maintenance in *Agrobacterium* (see FIG. 6).

### EXAMPLE 2: TRANSGENIC MAIZE EVENTS VIA AGROBACTERIUM USING THE HELPER PPHP70298.

The vector, pPHP70298, was tested as a helper vector for corn transformation in two different maize cultivars, PHR03 and PH184C, using *Agrobacterium* mediated immature embryo transformation as described herein below. *Agrobacterium tumefaciens* strain LBA4404THY-harboring the ternary vector containing an expression cassette ZmUbi: PMI: PINII and ZmUbi: ZsYellow: PINII (pPHP45981; T-DNA; SEQ ID NO: 28) was tested. Side-by-side experiments were performed with pSB1 plus pPHP45981 and pPHP70298 plus pPHP45981 to compare the effect of these two vectors, pSB1 and pPHP70298, on maize transformation. The transformation was evaluated in terms of callus transformation frequency (Callus Tx%), T0 plant transformation frequency (T0 Tx%), quality event frequency (QE) (defined as the percentage of events with all genes of interest being single copy and not having any vector backbone DNA -QE%) and usable event quality (UE)) (defined as the number of QE events recovered for every 100 embryos infected UE%) for the two vectors. The transformation experiments were performed with a minimum of 600 embryos and the transformation data is summarized in Tables 2 and 3 for genotypes PHR03 and PH184C, respectively. The data in the tables demonstrate that the vector, pPHP70298, yielded a significant improvement in the overall callus and T0 plant transformation frequency in both of the maize cultivars tested.

**Table 2. Transformation (Tx) data obtained in PHR03.**

| Helper Plasmid | Callus Tx (%) | T0 Tx (%) | QE (%) | UE (%) |
|---|---|---|---|---|
| pSB1 | 54 | 14 | 40.7 | 5.7 |
| pPHP70298 | 67 | 29 | 29.5 | 8.5 |

**Table 3. Transformation (Tx) data obtained in PH184C.**

| Helper Plasmid | Callus Tx (%) | T0 Tx (%) | QE (%) | UE |
|---|---|---|---|---|
| pSB1 | 63 | 45 | 41.5 | 18.6 |
| pPHP70298 | 84 | 61 | 20.2 | 12.3 |

*Growing Agrobacterium on solid medium:* Five mL *Agrobacterium* infection medium (700 medium; see Table 21) and 5 µL of 100 mM 3'-5'-Dimethoxy-4'-hydroxyacetophenone (acetosyringone) were added to a 14 mL Falcon tube in a hood. About 3 full loops of *Agrobacterium* were suspended in the tube and the tube was then vortexed to make an even suspension. One mL of the suspension was transferred to a spectrophotometer tube and the OD of the suspension was adjusted to 0.35 at 550 nm. The *Agrobacterium* concentration was approximately 0.5 x 10⁹ cfu/mL. The final *Agrobacterium* suspension was aliquoted into 2 mL microcentrifuge tubes, each containing 1 mL of the suspension. The suspensions were then used as soon as possible.

*Growing Agrobacterium in liquid medium:* One day before infection, a 125 ml flask was set up with 30 mL of 557A (see Table 21) with 30 µL spectinomycin (50mg/mL) and 30 µL acetosyringone (20 mg/mL). A half loopful of *Agrobacterium* was suspended into the flasks and placed on a 200 rpm shaker at 28°C overnight. The *Agrobacterium* culture was centrifuged at 5000 rpm for 10 min. The supernatant was removed and the *Agrobacterium* infection medium (700 medium; see Table 21) with acetosyringone solution was added. The bacteria were resuspended by vortex and the OD of *Agrobacterium* suspension was adjusted to 0.35 at 550 nm.

*Maize Transformation:* Ears of a maize (*Zea mays* L.) cultivar, PHR03 or PH184C, were surface-sterilized for 15-20 min in 20% (v/v) bleach (5.25% sodium hypochlorite) plus 1 drop of Tween 20 followed by 3 washes in sterile water. Immature embryos (IEs) were isolated from ears and were placed in 2 ml of the *Agrobacterium* infection medium (700 medium; see Table 21) with acetosyringone solution. The optimal size of the embryos was 1.5-1.8 mm for PHR03, respectively. The solution was drawn off and 1 ml of *Agrobacterium* suspension was added to the embryos and the tube vortexed for 5-10 sec. The microfuge tube was allowed to stand for 5 min in the hood. The suspension of *Agrobacterium* and embryos were poured onto co-cultivation medium (7101; see Table 21). Any embryos left in the tube were transferred to the plate using a sterile spatula. The *Agrobacterium* suspension was drawn off and the embryos placed axis side down on the media. The plate was sealed with Parafilm™ film (moisture resistant flexible plastic, available at Bemis Company, Inc., 1 Neenah Center 4^{th} floor, PO Box 669, Neenah, WI 54957) and incubated in the dark at 21 °C for 1-3 days of co-cultivation.

Embryos were transferred to resting medium (605W; see Table 21) without selection. Three to 7 days later, they were transferred to selection media 13152T or 13152Z (see Table 21) supplemented with mannose (12.5 g/L) or another selective agent (G418, 150 mg/L). Three weeks after the first round of selection, cultures were transferred to fresh 13152T or 13152Z (see Table 21) containing a selective agent at 3- to 4-week intervals. Once transformed, the tissues were transferred to maturatiom medium (289Q or 289M; see Table 21) supplemented with appropriate selective agent.

### EXAMPLE 3: COMPARISON OF PPHP70298 AND PPHP71539 TO PLASMIDPSB1 FOR MAIZE TRANSFORMATION.

The impact of pPHP70298 and pPHP71539 for maize transformation in the maize genotype PH184C using *Agrobacterium* mediated immature embryo transformation was determined. In this study, ternary production vectors were utilized that harbor at least one or more genes of interest (GOI) operably linked to marker genes, phosphomannose-isomerase (PMI) and phosphinothricin acetyl transferase (moPAT), for plant selection within the T-DNA. The transformation data are presented in Table 4, and these data show that the transformation frequency was at least 1.5- 2-fold higher in the experiments at the callus and T0 plant level for PH184C using pPHP70298 and pPHP71539 compared pSB1. The quality event (QE) frequency (defined as the percentage of events with all genes of interest being single copy and not having any vector backbone DNA) was not significantly different between the three plasmids pSB1, pPHP70298, and pPHP71539, but the usable event frequency (UE) (defined as the number of QE events recovered for every 100 embryos infected) was higher for both pPHP70298 and pPHP71539 for three independent production vectors. The average transformation frequency, QE% and UE for the three production vectors are summarized in Table 4.

**Table 4. Summary of transformation data in the genotype PH184C.**

| Construct | Helper | Callus Tx (%) | T0 Tx (%) | QE (%) | UE |
|---|---|---|---|---|---|
| A | pSB1 | 21.9 | 12.9 | 40.8 | 5.3 |
| | pPHP70298 | 36.9 | 24.1 | 26.3 | 6.3 |
| | pPHP71539 | 48.5 | 33.2 | 28.4 | 9.4 |
| B | pSB1 | 23.7 | 14.5 | 39.1 | 5.7 |
| | pPHP70298 | 32.3 | 18.9 | 32.4 | 6.1 |
| | pPHP71539 | 51 | 29 | 34 | 9.9 |
| C | pSB1 | 23.1 | 13.7 | 30.8 | 4.2 |
| | pPHP70298 | 30.8 | 17 | 29.3 | 5 |
| | pPHP71539 | 56.7 | 31.1 | 27.4 | 8.5 |
| Average | pSB1 | 22.9 | 13.7 | 36.9 | 5 |
| | pPHP70298 | 33.3 | 19.9 | 29.1 | 5.8 |
| | pPHP71539 | 52.3 | 31.1 | 29.9 | 9.3 |

### EXAMPLE 4: IMPROVED TRANSFORMATION WITH PPHP71539.

The impact of pPHP71539 on maize transformation was further tested in another maize cultivar, PHR03, via *Agrobacterium* mediated immature embryo transformation. In this study, production vectors were utilized that harbor at least one or more genes of interest (GOI) operably linked to marker genes, phosphomannose-isomerase (PMI) and phosphinothricin acetyl transferase (moPAT), for plant selection within the T-DNA. As shown in Table 5, pPHP71539 markedly improved transformation frequency without negatively impacting the quality event frequency resulting in significant gains in the usable event recovery in the genotype PHR03 when compared to pSB1. These data further document the improvement achieved with the disclosed plasmids for maize transformation in multiple genotypes.

**Table 5. Transformation (Tx) data using pPHP71539 in the genotype PHR03 with indicated construct.**

| Construct | Helper | Callus Tx (%) | T0 Tx (%) | QE (%) | UE |
|---|---|---|---|---|---|
| D | pSB1 | 23 | 4 | 27 | 1.2 |
| | pPHP71539 | 47 | 22 | 23 | 5.1 |
| E | pSB1 | 8 | 3 | 20 | 0.7 |
| | pPHP71539 | 43 | 16 | 38 | 6 |
| F | pSB1 | 28 | 11 | 33 | 3.5 |
| | pPHP71539 | 45 | 20 | 27 | 5.4 |

One skilled in the art would appreciate that Agrobacteria with helper plasmid pVIR10 (SEQ ID NO: 36; see Table 23) can significantly improve transformation frequency, recovery of quality events and usable quality events in multiple corn inbreds.

### EXAMPLE 5: IMPROVED TRANSIENT DNA DELIVERY AND GENE EXPRESSION IN PLANTS.

The plasmid, pPHP71539, was tested for transient T-DNA delivery in leaf explants using an Agroinfection method. For Agroinfection, young leaf explants were vacuum infiltrated with an *Agrobacterium* strain harboring a production cassette (*i.e.,* a plant protection GOI) and leaves were harvested 2-3 days post infiltration for protein measurements. The protein measurements were made with standard ELISA using replicate samples and repeated twice. The data on transient DNA delivery in maize leaves is presented in Table 6. The protein amount measured in leaves infiltrated with pPHP71539 was significantly higher than the protein amounts seen in the leaves infiltrated with the same gene expression cassette with pSB1. This demonstrates that pPHP71539 facilitated higher T-DNA delivery and improved transient protein expression in maize.

**Table 6. Transient gene expression in maize leaves.**

| Construct | Helper Vector | Protein expression (ppm) | Average expression (ppm) |
|---|---|---|---|
| G | pSB1 | 418 | 429 |
| | | 396 | |
| | | 377 | |
| | | 525 | |
| | pPHP71539 | 537 | 660 |
| | | 604 | |
| | | 810 | |
| | | 691 | |

One skilled in the art would appreciate that Agrobacteria with helper plasmid pVIR7, pVIR9 or pVIR10 can significantly improve transient protein expression in different plant explants in multiple corn inbreds.

### EXAMPLE 6: IMPROVED SORGHUM TRANSFORMATION USING AGROBACTERIUM CARRYING PPHP70298.

To demonstrate the improved functionality of the disclosed vectors for improving plant transformation in other monocots, the transformation frequency was tested for *Agrobacterium-*mediated sorghum transformation using immature embryos from sorghum variety TX430. In the first experiment, *Agrobacterium tumefaciens* strain LBA4404THY- harboring the ternary vector, pPHP45981, containing an expression cassette ZmUbi: PMI: PINII and ZmUbi: ZsYellow: PINII (Seq ID NO. 28) was used. Side-by-side experiments were performed with pPHP45981 plus pSB1 and pPHP45981 plus pPHP70298 to determine the callus transformation frequency (Callus Tx %), T0 plant transformation frequency (T0 Tx %), quality event frequency (QE%) and usable event quality (UE). The transformation data from two independent experiments with a minimum of 300 immature embryos is summarized in Table 7. The strain LBA4404THY- harboring, pPHP45981 plus pPHP70298 plasmid showed improved performance at all stages in sorghum transformation including callus transformation, T0 plant transformation, quality event and overall usable quality event. Additional testing was carried out with constructs containing at least one trait stack and PMI expression cassette plus pPHP71539. The average transformation frequency with pPHP71539 (Table 8) was higher than the normal transformation frequency observed with the ternary vectors containing pSB1 (Table 7).

**Table 7. Transformation data obtained using pPHP70298 in sorghum with construct pPHP45981.**

| Helper | Callus Tx (%) | T0 Tx (%) | QE (%) | UE |
|---|---|---|---|---|
| pSB1 | 4.2 | 2.1 | 64.7 | 1.4 |
| pPHP70298 | 10.5 | 5.7 | 71.8 | 4.1 |

**Table 8. The T0 transformation frequency observed using pPHP71539 in sorghum.**

| Constructs | Callus Tx (%) | T0 Tx (%) |
|---|---|---|
| H | 11 | 5 |
| I | 37 | 19 |
| J | 7 | 4 |

One skilled in the art would appreciate that Agrobacteria with helper plasmid pVIR7 (SEQ ID NO: 34), pVIR9 (SEQ ID NO: 35) or pVIR10 (SEQ ID NO: 36) can significantly improve transformation frequency, recovery of quality events and usable quality events in different sorghum lines.

### EXAMPLE 7: IMPROVED SITE-SPECIFIC INTEGRATION USING PPHP71539.

pPHP71539 for *Agrobacterium*-mediated site-specific integration ("SSI") was assessed on a target line harboring the target locus created in maize cultivar, PHR03, as described in US Patent No. 6,187,994 and U.S. Provisional Appl. No. 62/296639. A target site operably linked to a promoter trap was used to aid in target event identification, and SSI event identification. Lines comprising a promoter trap target site were generated by transformation with a construct comprising pPHP64484 ZmProUbi-FRT1-NptII::PinII + ZmUbiPro::AmCyan::PinII-FRT87 (SEQ ID NO: 29). The binary vectors were generated with a promoter trap selectable marker plus a constitutive promoter (ZmUbiPro; SEQ ID NO: 30) driving the reporter gene (DsRed (SEQ ID NO: 31) flanked by non-identical FRT recombination site pairs (*e.g*., FRT1/FRT87 (SEQ ID NOS: 32 and 33) within the T-DNA, referred to as the donor. The binary vectors were mobilized into the *Agrobacterium* strain AGL1 with and without pPHP71539 for evaluating the effect of pPHP71539 on the recovery of SSI events and usable SSI frequency (precise SSI events). The molecular characterization of the putative events was carried out using qPCR/PCR assays to determine the excision of target gene (NptII), integration of the donor genes (PMI and DsRED), absence of FLP gene (random T-DNA integration), and presence of the FRT pair junction (1/87). A multiplex PCR was performed for vector backbone analysis. A precise SSI event (usable SSI event) was characterized as one which meets the following criteria: (1) single intact copy of the donor genes (PMI and DsRed); (2) absence of the target gene (NptII), FLP, ODP/MoCre; (3) presence of both FRT1 and FRT87 junctions; and, (4) free of any vector backbone insertion. Table 9 summarizes transformation frequency and precise SSI frequency obtained using the construct pPHP71518 in the *Agrobacterium* strain AGL1 with or without the helper plasmid, pPHP71539, in maize cultivar PHR03. Significant improvement in the recovery of putative SSI events and recovery of precise SSI T0 events was observed with the Agro strain containing the pPHP71518 plus pPHP71539 as compared to *Agrobacterium* strain with pPHP71518 alone.

**Table 9. Data representing the transformation frequency and SSI frequency in PHR03 with and without pVIR9 helper plasmid containing the donor construct pPHP71518**

| Construct | Total Embryo | Callus | | T0 | | SSI | |
|---|---|---|---|---|---|---|---|
| | | # Events | Frequency | #Events | Frequency | #Events | Frequency |
| 71518 | 817 | 151 | 18.8% | 78 | 9.5% | 12 | 1.5% |
| 71518+ pPHP71539 | 821 | 190 | 23.3% | 96 | 11.7% | 22 | 2.7% |

One skilled in the art would appreciate that Agrobacteria with helper plasmid pVIR7 or pVIR10 can significantly improve T0 transformation frequency and precise SSI frequency in multiple corn inbreds.

### EXAMPLE 8: IMPROVED WHEAT TRANSFORMATION USING PVIR PLASMID (HIGH EFFICIENCY TRANSFORMATION AND RAPID T0 PLATN PRODUCTION IN WHEAT.

### A. Wheat Transformation Methods

An aliquot of *Agrobacterium* strain LBA4404 THY- containing the vector of interest is removed from storage at -80° and streaked onto solid LB medium (12V) containing a selective agent spectinomycin. The *Agrobacterium* was cultured on the 12V at 21°C in the dark for 2-3 days, at which time a single colony is selected from the plate, streaked onto 810D medium plate containing the selective agent and then incubated at 28°C in the dark overnight. Two to three *Agrobacterium* colonies were picked using a sterile spatula and suspended in ∼ 5 mL wheat infection medium (WI4) (WI4, see Table 10) with 400 uM acetosyringone (AS). The optical density (600 nm) of the suspension was adjusted to about 0.1 to 0.7 using the same medium.

Four to five spikes containing immature seeds (with 1.4-2.3 mm embryos) were collected, and the immature embryos were isolated from the immature seeds. The wheat grains were surface sterilized for 15 min in 20% (v/v) bleach (5.25% sodium hypochlorite) plus 1 drop of Tween 20, followed with 2-3 washes in sterile water. After sterilization, the immature embryos (IEs) were isolated from the wheat grains and placed in 1.5 ml of WI4 medium. The immature embryos were transferred to a 2 mL microcentrifuge tube containing 0.25 mL sterile sand plus 1 mL WI4 medium, and then centrifuged at 10,000 RPM for 30 seconds. Following first centrifugation, the micricentrifuge tube was vortexed at a medium setting for 10 seconds, and again centrifuged a second time at 10,000 RPM for 30 seconds. The embryos were allowed to sit in this suspension for 20 minutes.

In the next step, WI4 medium was decanted and infection of the immature embryos was initiated by adding 1.0 ml of *Agrobacterium* suspension. The infected embryos were allowed to sit for 20 minutes. The suspension of *Agrobacterium* and IEs was poured onto wheat co-cultivation medium #10 (see Table 11). Embryos were poured to the plate using a sterile spatula, with axis side placed down on the media, making sure the embryos are immersed in the solution. The plate was sealed with Parafilm® tape film (moisture resistant flexible plastic, available at Bemis Company, Inc., 1 Neenah Center 4^{th} floor, PO Box 669, Neenah, WI 54957) and incubated in the dark at 21 °C for 3 days of co-cultivation. Tables 10-15 describe liquid wheat infection medium (WI4), wheat co-cultivation medium (WC#10), first round DBC4 medium, second round DBC6 medium, regeneration MSA medium, and regeneration MSB medium.

**Table 10. Composition of wheat liquid infection medium WI4.**

| **WI 4** | |
|---|---|
| DI water | 1000mL |
| MS salt + Vitamins(M519) | 4.43 g |
| Maltose | 30 g |
| Glucose | 10 g |
| MES | 1.95 g |
| 2,4-D (.5mg/L) | 1 ml |
| Picloram ( 10mg/ml) | 200 µl |
| BAP (1mg/L) | .5 ml |
| Adjust PH to 5.8 with KOH | |
| Post sterilization add: | |
| Acetosyringone (400 µM) | 400 µl |

**Table 11. Composition of wheat co-cultivation medium WC#10.**

| **WC # 10** | |
|---|---|
| DI water | 1000mL |
| MS salt + Vitamins(M519) | 4.43 g |
| Maltose | 30 g |
| Glucose | 1 g |
| MES | 1.95 g |
| 2,4-D (.5mg/L) | 1 ml |
| Picloram ( 10mg/ml) | 200 µl |
| BAP (Img/L) | .5 ml |
| 50X CuSO4 (.1M) | 49 µl |
| Adjust PH to 5.8 with KOH and add 2.5 g/L | |
| of Phytagel. | |
| Post sterilization add: | |
| Acetosyringone (400 µM) | 400 µl |

**Table 12. Composition of wheat green tissue culture medium DBC4.**

| **DBC4** | |
|---|---|
| dd H20 | 1000mL |
| MS salt | 4.3 g |
| Maltose | 30 g |
| Myo-inositol | 0.25 g |
| N-Z-Amine-A | 1 g |
| Proline | 0.69 g |
| Thiamine-HCl (0.1mg/mL) | 10 mL |
| 50X CuSO4 (0.1M) | 49 µL |
| 2,4-D (0.5mg/mL) | 2 mL |
| BAP | 1 mL |
| Adjust PH to 5.8 with KOH and then add 3.5 g/L of Phytagel. | |
| Post sterilization add: | |
| Cef(100mg/ml) | 1ml |

**Table 13. Composition of wheat green tissue induction medium DBC6.**

| **DBC6** | |
|---|---|
| dd H20 | 1000mL |
| MS salt | 4.3 g |
| Maltose | 30 g |
| Myo-inositol | 0.25 g |
| N-Z-Amine-A | 1 g |
| Proline | 0.69 g |
| Thiamine-HCl (0.1mg/mL) | 10 mL |
| 50X CuSO4 (0.1M) | 49 µL |
| 2,4-D (0.5mg/mL) | 1 mL |
| BAP | 2 mL |
| Adjust PH to 5.8 with KOH and then add 3.5 g/L of Phytagel. | |
| Post sterilization add: | |
| Cef(100mg/ml) | 1ml |

**Table 14. Composition of wheat regeneration medium MSA.**

| **MSA** | |
|---|---|
| dd H20 | 1000mL |
| MS salt + Vitamins(M519) | 4.43 g |
| Sucrose | 20g |
| Myo- Inositol | 1 g |
| Adjust PH to 5.8 with KOH and then add 3.5 g/L of Phytagel. | |
| Post steriliaztion add: | |
| Cef(100mg/ml) | 1ml |

**Table 15. Composition of wheat regeneration medium MSB.**

| **MSB** | |
|---|---|
| dd H20 | 1000mL |
| MS salt + Vitamins(M519) | 4.43 g |
| Sucrose | 20g |
| Myo- Inositol | 1 g |
| Adjust PH to 5.8 with KOH and then add 3.5 g/L of Phytagel. | |
| Post steriliaztion add: | |
| Cef (100mg/ml) | 1ml |
| IBA | .5 ml |

### B. Wheat Embryo Selection

The immature embryos are transferred to DBC4 green tissue (see Table 12) (GT medium with 100 mg/L cefotaxime (PhytoTechnology Lab., Shawnee Mission, KS) induction medium without selection, in the orientation of the embryo axis being in contact with the medium. The embryos are incubated on this medium at 26-28°C in dim light for two weeks then transfered to DBC6 medium (see Table 13) containing 100 mg/L cefotaxime for another two weeks. At this time, all tissue expressing the fluorescent protein is separated from the non-transformed tissues under a fluorescence microscope and placed on DBC6 GT induction medium (see Table 13) containing 100 mg/L cefotaxime for tissue proliferation.

### C. Regeneration of plantlets and transfer to the greenhouse

The fluorescing tissue is transferred to MSA regeneration medium (see Table 14), and incubated at 26-28°C in bright light for 2-4 weeks. At this point, the tissue is checked for uniform expression of the fluorescent marker genes in transgenic plantlets and for healthy roots. The plantlets are transferred into soil in pots in the greenhouse.

### D. The introduction of the super binary plasmids pPHP71539 and pPHP70298 in Agrobacteria containing plant expression cassettes resulted in improved transient T-DNA delivery and improved recovery of transgenic T0 events in wheat

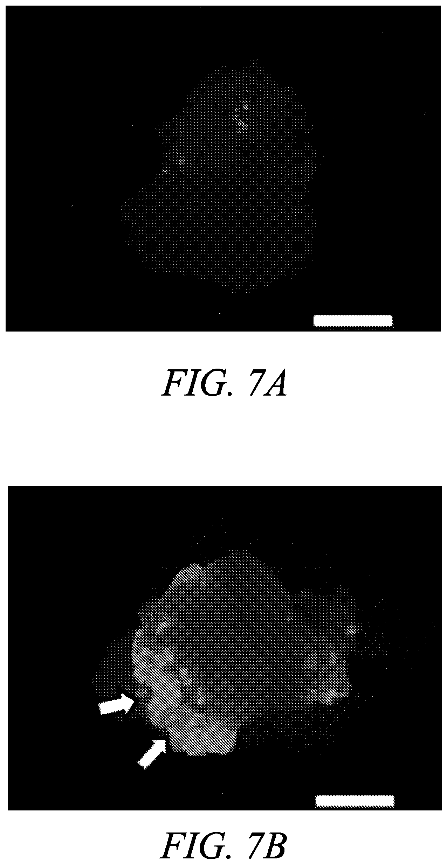

Immature embryos were harvested from wheat cultivar HC0456D and were infected with Agrobacterium strains LBA4404 THY- pPHP71539 and AGL1 containing a T-DNA binary plasmid with the following composition- RB-UBI-ZMPRO: MO-PAT: PROTEIN LINKER:DS-RED:PINII-LB (SEQ ID NO.68. Agrobacterium strain LBA4404 THY- is a weaker strain which previously was shown to result in weak transient T-DNA delivery and has been described elsewhere as a poor strain for wheat transformation, therefore excluded from the study. For transient T-DNA expression 20-30 embryos of the cultivar were infected and DS-RED expression was monitored at 7 days and 15 days post infection. In the experiment, two different Agrobacterium strains AGL1 and LBA4404 Thy-pPHP71539 were tested to characterize the effect of pVIR9 on stable transformation (15 DPI) in wheat (FIG 7). The native AGL1 strain showed several fold lower transient DsRed expression at 7DPI and continued to show fewer DsRed expressing sectors at 15DPI (FIG.7), representing stable T-DNA expression in the infected embryos as compared to strain LBA4404THY-pPHP71539.

In a separate experiment, Agrobacteria containing the pVIR helper plasmid improved stable transformation and increased recovery of T0 events in multiple wheat cultivars. To this end, side-by-side wheat transformation experiments were performed in two different wheat cultivars (Fielder and HC0456D) with Agrobacterium strain AGL1, with and without pVIR helper pPHP70298. Immature embryos were harvested from two wheat cultivars (Fielder and HC0456D) and were infected with Agrobacteria AGL1 and AGL1 plus pPHP70298 containing two different binary plasmids (A and B) containing the following expression cassette; RB-UBI-ZMPRO: MO-PAT: PROTEIN LINKER: DS-RED:PINII-LB (SEQ ID NO.68) to capture T0 plant transformation frequency. The transformation data is summarized in Table 16.

**Table 16. Transformation data using pPHP70298 in multiple wheat cultivars.**

| Cultivar | Construct | AGL1 (T0 TX%) | AGL1 + pPHP70298 (T0 TX%) |
|---|---|---|---|
| Fielder | A | 0.5% (26/5748) | 1.4% (4/278) |
| HC0456D | A | 0% (0/1918) | 1.1% (4/375) |
| Fielder | B | 0.4% (9/2294) | 5.4% (47/863) |
| HC0456D | B | 0% | 5.3% (36/678) |

Wheat immature embryos transformed with AGL1 plus pPHP70298 showed remarkably improved performance at all stages in wheat transformation including callus transformation and T0 plant transformation. The T0 plant transformation frequency with Agrobacteria containing the pVIR7 plasmid was determined to be much higher than the wild-type strain of AGL1 without the pVIR7 helper plasmid. Ochrobactrum containing the pVIR7, the pVIR9 or the pVIR10 plasmids has been used to successfully transform plants (data not shown) as described in U.S. Provisional Appl. No. 62/211267.

One skilled in the art would appreciate that Agrobacteria with helper plasmid pVIR9 (SEQ ID NO: 35, see Table 23) or pVIR10 can significantly improve transformation frequency, recovery of quality events and usable quality events in multiple wheat lines.

### EXAMPLE 9: PVIR9 PLASMID IMPROVED MAIZE TRANSFORMATION

The pVIR9 plasmid was tested for maize transformation using the ternary vector system to transform corn genotype PH184C as described in U.S. Provisional Appl. No. 62/248578. The introduction of the super binary plasmid pPHP71539 in Agrobacteria containing plant expression cassettes resulted in improved transient T-DNA delivery, somatic embryos phenotype and improved recovery of transgenic T0 events in corn.

Briefly, immature embryos (2-2.5mm in length) were harvested from Pioneer maize inbred PH184C) approximately 11 days after pollination, and were infected with Agrobacterium strain LBA4404 THY- containing T-DNA plasmids 1) pPHP80561 or 2) pPHP80559 with the following composition; pPHP80561- RB + LOX P- ZM-AXIG1 1XOP-WUS2::IN2-1 TERM + ZM-PLTP PRO::ZM-ODP2::OS-T28 TERM + PINII+ GZ-W64A TERM + OLE PRO:MO-CRE EXON1:ST-LS1 INTRON2: MO-CRE EXON2-PINII TERM+ SB-UBI PRO: SB-UBI INTRON1:UBI PRO: ZS-GREEN:OS-UBI TERM+ LOXP+ SB-ALS PRO:: HRA::PINII TERM+ LB (SEQ ID NO.69) and, pPHP80559- RB + LOX P- ZM-AXIG1 1XOP-WUS2::IN2-1 TERM + ZM-PLTP PRO::ZM-ODP2::OS-T28 TERM + PINII+ GZ-W64A TERM + LTP2 PRO:MO-CRE EXON1:ST-LS1 INTRON2: MO-CRE EXON2-PINII TERM+ SB-UBI PRO: SB-UBI INTRON1:UBI PRO: ZS-GREEN:OS-UBI TERM+ LOXP+ SB-ALS PRO:: HRA::PINII TERM+ LB (SEQ ID NO. 70). These plasmids were mobilized into two different Agrobacterium strains LBA4404THY-pSB1 and LBA4404THY-pVIR9 (pPHP71539). The Agrobacteria with the plasmid was grown in liquid medium to an optical density of 0.5 (at 520 nm) and the immature embryos (∼600 plus embryos, split ear, three replicates) were incubated in the Agrobacterium suspension for 5 minutes before removal from the liquid to be placed on solid 710I medium. After 24 hours, the embryos were moved to 605T medium (see Table 21) to begin selection against the Agrobacterium. After 6 days, numerous small somatic embryos were observed on the surface of the treated immature embryos. Seven days after Agro-infection, the embryos were transferred to maturation medium (289Q medium with 0.1 mg/l imazapyr), using the imidazolinone herbicide to select for transgenic embryos. After 14 days on the maturation medium, the mature embryos were moved onto rooting medium (13158H medium; 13158 medium plus 25 mg/l cefotaxime) and leaf pieces were sampled for PCR analysis. The data on TX frequency, excised QE frequency and UE frequency is represented in Table 17.

**Table 17. Transformation data in inbred PH184C comparing pVIR9 and pSBlplasmid with two different expression vectors.**

| Treatment | pPHP | Helper | Pro:CRE | # Emb | # T0 plant | TX% | Excised QE events | QE excision % | UQE% |
|---|---|---|---|---|---|---|---|---|---|
| A | pPHP80561 | pVIR9 | Ole | 610 | 120 | 20% | 21 | 18% | 3.4% |
| | pPHP80561 | pSB1 | Ole | 607 | 19 | 3% | 4 | 21% | 0.7% |
| B | pPHP80559 | pVIR9 | LTP2 | 610 | 113 | 19% | 23 | 20% | 3.8% |
| | pPHP80559 | pSB1 | LTP2 | 610 | 42 | 7% | 6 | 14% | 1.0% |

The immature embryos transformed with Agrobacteria containing the pPHP71539 (pVIR9) plasmid produced very high number of T0 plants and resulted in higher T0 transformation frequency as compared to Agrobacteria transformed with the pSB1 plasmid. The Agrobacteria with the helper plasmid pVIR9 also resulted in significantly higher frequency of useable quality events when compared to immature embryos infected with plasmid pSB1.

One skilled in the art would appreciate that Agrobacteria with helper plasmid pVIR7 or pVIR10 instead of pVIR9 can significantly improve maize transformation, quality events and usable quality events.

### EXAMPLE 10. PVIR PLASMID WITH DIFFERENT ORIGINS OF REPLICATION FOR PLANT TRANSFORMATION.

For characterizing the effect of different origins of replication on the helper plasmid for corn transformation, the ternary vector system was used to transform corn genotype HC69. Using the maize transformation approach as described above in Example 9 (U.S. Provisional Appl. No. 62/248578), four pVIR plasmids containing different bacterial origins of replication namely pVS1, pSaparDE and RK2parDE (Table 18) were tested. Immature embryos were harvested from maize inbred (HC69) and were infected with *Agrobacterium* (strain LBA4404 THY-) containing pPHP79066 (SEQ ID NO.71) with the following T-DNA composition and the helper plasmids with different bacterial origins of replication ("ori") namely the following: pVS1 (pVIR10; pPHP79761), pSaparDE (pVIR10; pPHP80399), RK2parDE (pVIR10; pPHP80566) and control (pVIR9; pPHP71539) as detailed in Table 18. For each construct, 150 embryos of the inbred HC69 were transformed using the split ear method to determine the transformation frequency. All the constructs transformed corn at very high frequency demonstrating the differnt ori combinations worked in the pVIR plasmid. The transformation data for inbred HC69 is presented in Table 19.

**Table 18. Testing of different Ori's on pVIR plasmid.**

| Vector# | Binary | pVIR | Ori |
|---|---|---|---|
| 1 | pPHP79066 | pPHP79761 | pVS1 |
| 2 | pPHP79066 | pPHP80399 | pSA parDE |
| 3 | pPHP79066 | pPHP80566 | RK2micro parDE |
| 4 | pPHP79066 | pPHP71539 | pVS1 |

**Table 19. Transformation data in inbred HC69 with pVIR plasmid containing different ori.**

| Binary/pVIR combinations | Total Embryos | Total T0 plants | Transformation frequency |
|---|---|---|---|
| pPHP79066/ pPHP79761 | 183 | 154 | 84% |
| pPHP79066/ pPHP80399 | 160 | 116 | 73% |
| pPHP79066/ pPHP80566 | 176 | 173 | 98% |
| pPHP79066/pPHP71539 | 170 | 175 | 103% |

The pVIR plasmids with different ori transformed corn and regenerated T0 events. The T0 transformation frequency was comparable across all the different ori tested.

### EXAMPLE 11. ENHANCED DELIVERY SYSTEM FOR CRISPR/CAS9 REAGENTS AND GENE MUTATIONS WITH PVIR PLASMID IN CORN.

To characterize the effect of the helper plasmid on CRIPSR/Cas9 delivery and the rate of mutants recovered from the different helper plasmids, a ternary vector system was used to transform corn genotype PH2HT. Using the random transformation protocol, two different constructs were transformed, pPHP78147 (SEQ ID NO.72) and pPHP78148 (SEQ ID NO. 73), each comprising of two guide RNA (gRNA) sequences, specific for generating dropout deletions in target genes- ZM-ARGOS8 and ZM-GCN2. The sequences of the guide RNA sequences and the expression cassettes are described in SEQ ID NO: 72 and SEQ ID NO: 73, respectively. Immature embryos were harvested from maize inbred PH184C and were infected with *Agrobacterium* (strain LBA4404 THY-) containing helper plasmids pPSB1 or pVIR9 containing the T-DNA expression cassettes pPHP78147 or pPHP78148. For each construct approximately 280 embryos of the inbred PH2RT was transformed using the split ear method to determine the transformation frequency, mutation rates, single dropout deletions and biallelic dropout deletion rates. For determining the mutation rates, the T0 plants were subjected to deep sequencing analysis and the molecular event quality was determined by running PCR and qPCR assays on target genes as described previously. The transformation data and the gene edit types resulted from each construct in PH2RT are presented in Table 20.

**Table 20. Types of gene edits resulted from each construct.**

| pPHP | Help er | # Embry o* (no.) | # T0 Even ts | T0 Txn % | Mutati on rate | # 1 allele dropo ut | Singl e allele dropo ut rate | # Biallel ic dropo ut | Biallel ic dropo ut rate |
|---|---|---|---|---|---|---|---|---|---|
| pPHP78147 (ARGOS8) | pSB1 | 285 | 130 | 61.8 % | 42.3% | 3 | 2.3% | 7 | 5.4% |
| pPHP78147 (ARGOS8) | pVIR 9 | 285 | 129 | 80.3 % | 46.5% | 10 | 7.7% | 6 | 4.7% |
| pPHP78148 (GCN2) | pSB1 | 280 | 112 | 47.1 % | 86.6% | 18 | 16.1 % | 5 | 4.5% |
| pPHP78148 (GCN2) | pVIR 9 | 280 | 127 | 59.6 % | 92.1% | 22 | 17.3 % | 8 | 6.3% |

The transformation frequency and mutation rates recovered from the new pVIR superbinary vectors were much higher than that observed with pSB1. The transformation frequency varied depending on the gRNA sequences and the deletion types. For ARGOS8, wherein a larger dropout deletion was targeted (619 bp), observed transformation frequency with pVIR9 was about 80.3% compared to 61.8% with pSB1. The frequency at which single allele dropouts were recovered with the ARGOS8 construct was higher with pVIR9 (7.7%) when compared to pSB1 (2.3%). With the second construct targeting a smaller dropout deletion in ZM-GCN2 (52 bp), embryos transformed with Agro containing the helper plasmid pVIR9 resulted in higher T0 event generation and recovery of mutant events (mutations, single allele and biallelic) as compared to the events recovered from the Agro with the pSB1 helper (Table 20).

One skilled in the art would appreciate that Agrobacteria with helper plasmid pVIR7 or pVIR10 can enhance delivery of CRISPR/Cas9 nucleases or related nucleases to improve genome editing and genome modifications in multiple corn inbreds.

### EXAMPLE 12: IMPROVED RECOVERY OF SITE-SPECIFIC INTEGRATIONS (SSI) EVENTS IN AGROBACTERIUM STRAIN LBA4404THY- HARBORING SUPERBINARY PLASMID.

To determine the effect of the super binary vectors (pVIR9; pPHP71539), on the recovery of precise SSI events, the pVIR9 helper plasmid in *Agrobacterium* strain LBA4404THY- was mobilized. Strain LBA4404THY- has been shown to result in low recovery of precise SSI events (U.S. Provisional Appl. No. 62/296639). To test whether pVIR9 in Agro strain LBA4404THY-improved recovery of SSI events, a binary vector containing the donor cassette with the following expression cassette RB- OS-ACTIN PRO: OS-ACTIN INTRON:: ZM-WUS2::PINII + UBI PRO::UBI1ZM INTRON::ZM-ODP2::PINII TERM:: UBI PRO:UBI1ZM INTRON::MO-FLP::PINII TERM:: CaMV35S TERM + FRT1:PMI::PINII TERM+ TRAIT GENE+ FRT87-LB was mobilized into two different *Agrobacterium* strains AGL1 and LBA4404THY-pPHP71539 (pPHP79366; SEQ ID NO. 74). Donor cassettes were delivered via Agro-mediated transformation into multiple target sites with FRT1-87 landing sites in the genotype PH184C as described below.

Briefly, Agro-mediated transformation into target lines with FRT1-FRT87 landing sites was carried out as follows. Ears of a maize (*Zea mays* L.) cultivar, PH184C, were surface-sterilized for 15-20 min in 20% (v/v) bleach (5.25% sodium hypochlorite) plus 1 drop of Tween 20 followed by 3 washes in sterile water. Immature embryos (IEs), typically 1.5-1.8 mm, were isolated from ears and were placed in 2 ml of the *Agrobacterium* infection medium with acetosyringone solution. The solution was drawn off and 1 ml of *Agrobacterium* suspension was added to the embryos, vortexed for 5-10 seconds, and then incubated 5 min at room temperature. The suspension of *Agrobacterium* and embryos were poured onto 7101 co-cultivation medium (see Table 21). Any embryos left in the tube were transferred to the plate using a sterile spatula. The *Agrobacterium* suspension was drawn off and the embryos placed axis side down on the media. The plate was sealed with Parafilm™ tape (moisture resistant flexible plastic, available at Bemis Company, Inc., 1 Neenah Center 4^{th} floor, PO Box 669, Neenah, WI 54957) and incubated in the dark at 21 °C for 1-3 days of co-cultivation.

Embryos were transferred to resting medium 13265A (see Table 21) without selection. Three to 7 days later, they were transferred to selection media 13152Z (see Table 21) supplemented with mannose or other appropriate selective agent. Three weeks after the first round of selection, cultures were transferred to second round of selection media 13152Z (see Table 21) containing a selective agent at 3- to 4-week intervals. Once transformed, transgenic green tissues are selected and cultured essentially as described in US Patent 7102056, US Patent 8404930, and publication US20130055472.

**Table 21. Media formations for maize transformation, selection and regeneration.**

| **Medium components** | **Uni ts per liter** | **12 V** | **810 I** | **70 0** | **710 I** | **605 J** | **605 T** | **289 Q** | **605 W** | **289 M** |
|---|---|---|---|---|---|---|---|---|---|---|
| MS BASAL SALT MIXTURE | G | | | 4.3 | 4.3 | 4.3 | 4.3 | 4.3 | 4.3 | 4.3 |
| N6 MACRONUTRIENTS 10X | ml | | | | | 60.0 | 60.0 | | 60.0 | |
| POTASSIUM NITRATE | G | | | | | 1.7 | 1.7 | | 1.7 | |
| B5H MINOR SALTS 1000X | ml | | | | | 0.6 | 0.6 | | 0.6 | |
| NaFe EDTA FOR B5H 100X | ml | | | | | 6.0 | 6.0 | | 6.0 | |
| ERIKSSON'S VITAMINS 1000X | ml | | | | | 0.4 | 0.4 | | 0.4 | |
| S&H VITAMIN STOCK 100X | ml | | | | | 6.0 | 6.0 | | 6.0 | |
| THIAMINE. HCL | mg | | | 1.0 | 1.0 | 0.2 | 0.2 | | 0.2 | |
| L-PROLINE | g | | | | 0.7 | 2.0 | 2.0 | 0.7 | 2.0 | 0.7 |
| CASEIN HYDROLYSATE (ACID) | g | | | | | 0.3 | 0.3 | | 0.3 | |
| SUCROSE | g | | | 68. 5 | 20.0 | 20.0 | 20.0 | 60.0 | 20.0 | 60.0 |
| GLUCOSE | g | 5.0 | | 36. 0 | 10.0 | 0.6 | 0.6 | | 10.0 | |
| MALTOSE | g | | | | | | | | | |
| 2,4-D | mg | | | 1.5 | 2.0 | 0.8 | 0.8 | | 0.8 | |
| AGAR | g | 15. 0 | 15.0 | | 8.0 | 6.0 | 6.0 | 8.0 | | |
| PHYTAGEL | g | | | | | | | | 3.5 | |
| DICAMBA | g | | | | | 1.2 | 1.2 | | 1.2 | |
| SILVER NITRATE | mg | | | | | 3.4 | 3.4 | | | |
| AGRIBIO Carbenicillin | mg | | | | | 100. 0 | | | 100. 0 | |
| Timentin | mg | | | | | | 150. 0 | 150 .0 | | |
| Cefotaxime | mg | | | | | | 100. 0 | 100 .0 | | |
| MYO-INOSITOL | g | | | 0.1 | 0.1 | | | 0.1 | | 0.1 |
| NICOTINIC ACID | mg | | | 0.5 | 0.5 | | | | | |
| PYRIDOXINE.HCL | mg | | | 0.5 | 0.5 | | | | | |
| VITAMIN ASSAY CASAMINO ACIDS | g | | | 1.0 | | | | | | |
| MES BUFFER | g | | | | 0.5 | | | | | |
| ACETOSYRINGONE | uM | | | | 100. 0 | | | | | |
| ASCORBIC ACID 10MG/ML (7S) | mg | | | | 10.0 | | | | | |
| MS VITAMIN STOCK SOL. | ml | | | | | | | 5.0 | | 5.0 |
| ZEATIN | mg | | | | | | | 0.5 | | 0.5 |
| CUPRIC SULFATE | mg | | | | | | | 1.3 | 0.05 | 1.3 |
| IAA 0.5MG/ML (28A) | ml | | | | | | | 2.0 | | 2.0 |
| ABA 0.1mm | ml | | | | | | | 1.0 | | 1.0 |
| THIDIAZURON | mg | | | | | | | 0.1 | | 0.1 |
| AGRIBIO Carbenicillin | mg | | | | | | | 100. 0 | | 100. 0 |
| PPT(GLUFOSINATE-NH4) | mg | | | | | | | | | |
| BAP | mg | | | | | | | 1.0 | 0.1 | |
| YEAST EXTRACT (BD Difco) | g | | 5.0 | | | | | | | |
| PEPTONE | g | | 10.0 | | | | | | | |
| SODIUM CHLORIDE | g | | 5.0 | | | | | | | |
| SPECTINOMYCIN | mg | 50. 0 | 100. 0 | | | | | | | |
| FERROUS SULFATE.7H20 | ml | 2.0 | | | | | | | | |
| AB BUFFER 20X (12D) | ml | 50. 0 | | | | | | | | |
| AB SALTS 20X (12E) | ml | 50. 0 | | | | | | | | |
| Benomyl | mg | | | | | | | | | |
| pH | | 5.6 | 5.6 | 5.6 | 5.6 | 5.6 | 5.6 | 5.6 | 5.6 | 5.6 |

**Table 21, continued.**

| **Medium components** | **Un its pe r lite r** | **289 R** | **1315 8H** | **132 24B** | **1326 6K** | **27 2X** | **27 2V** | **131 58** | **1326 5A** | **131 52T** | **131 52Z** |
|---|---|---|---|---|---|---|---|---|---|---|---|
| MS BASAL SALT MIXTURE | G | 4.3 | 4.3 | | 4.3 | 4.3 | 4.3 | 4.3 | 4.3 | 4.3 | 4.3 |
| N6 MACRONUTRIENTS 10X | ml | | | 4.0 | 60.0 | | | | 60.0 | | |
| POTASSIUM NITRATE | G | | | | 1.7 | | | | 1.7 | | |
| B5H MINOR SALTS 1000X | ml | | | | 0.6 | | | | 0.6 | | |
| NaFe EDTA FOR B5H 100X | ml | | | | 6.0 | | | | 6.0 | | |
| ERIKSSON'S VITAMINS 1000X | ml | | | 1.0 | 0.4 | | | | 0.4 | | |
| S&H VITAMIN STOCK 100X | ml | | | | 6.0 | | | | 6.0 | | |
| THIAMINE HCL | mg | | | 0.5 | 0.5 | | | | 0.5 | 1.0 | 1.0 |
| L-PROLINE | G | 0.7 | 0.7 | 2.9 | 2.0 | | | | 2.0 | 0.7 | 0.7 |
| CASEIN HYDROLYSATE (ACID) | G | | | | 0.3 | | | | 0.3 | 1.0 | 1.0 |
| SUCROSE | G | 60. 0 | 60.0 | 190. 0 | 20.0 | 40. 0 | 40. 0 | 40. 0 | 20.0 | | |
| GLUCOSE | G | | | | 0.6 | | | | 10.0 | | |
| MANNOSE | G | | | | | | | | | 12.5 | 12.5 |
| MALTOSE | G | | | | | | | | | 5.0 | 5.0 |
| 2,4-D | mg | | | | 1.6 | | | | 1.6 | 1.0 | 1.0 |
| AGAR | G | | 8.0 | 6.4 | 6.0 | 6.0 | 6.0 | 6.0 | | | |
| PHYTAGEL | G | | | | | | | | 3.5 | | |
| DICAMBA | G | | | | 1.2 | | | | 1.2 | | |
| SILVER NITRATE | mg | | | 8.5 | 1.7 | | | | | | |
| AGRIBIO Carbenicillin | mg | | | | 2.0 | | | | | 100 | |
| Timentin | mg | 15 0.0 | 150. 0 | | | | | | 150. 0 | | 150. 0 |
| Cefotaxime | mg | 10 0.0 | 100. 0 | 25 | 25 | | | | 100. 0 | | 100. 0 |
| MYO-INOSITOL | G | 0.1 | 0.1 | | | 0.1 | 0.1 | 0.1 | | 0.25 | 0.25 |
| NICOTINIC ACID | mg | | | | | | | | | | |
| PYRIDOXINE.HCL | mg | | | | | | | | | | |
| VITAMIN ASSAY CASAMINO ACIDS | G | | | | | | | | | | |
| MES BUFFER | G | | | | | | | | | | |
| ACETOSYRINGONE | uM | | | | | | | | | | |
| ASCORBIC ACID 10MG/ML (7S) | mg | | | | | | | | | | |
| MS VITAMIN STOCK SOL. | ml | 5.0 | 5.0 | | | 5.0 | 5.0 | 5.0 | | | |
| ZEATIN | mg | 0.5 | 0.5 | | | | | | | | |
| CUPRIC SULFATE | mg | 1.3 | 1.3 | | | | | | 0.05 | 1.2 | 1.2 |
| IAA 0.5MG/ML (28A) | ml | 2.0 | 2.0 | | | | | | | | |
| ABA 0.1mm | ml | 1.0 | 1.0 | | | | | | | | |
| THIDIAZURON | mg | 0.1 | 0.1 | | | | | | | | |
| AGRIBIO Carbenicillin | mg | | | | | | | | | | |
| PPT(GLUFOSINATE-NH4) | mg | | | | | | | | | | |
| BAP | mg | | | | | | | | 0.1 | 0.5 | 0.5 |
| YEAST EXTRACT (BD Difco) | G | | | | | | | | | | |
| PEPTONE | G | | | | | | | | | | |
| SODIUM CHLORIDE | G | | | | | | | | | | |
| SPECTINOMYCIN | mg | | | | | | | | | | |
| FERROUS SULFATE.7H20 | ml | | | | | | | | | | |
| AB BUFFER 20X (12D) | ml | | | | | | | | | | |
| AB SALTS 20X (12E) | ml | | | | | | | | | | |
| Benomyl | mg | | | | | | | 100 .0 | | | |
| pH | | 5.6 | 5.6 | 5.6 | 5.6 | 5.6 | 5.6 | 5.6 | 5.6 | 5.6 | 5.6 |

**Table 21, continued.**

| **Medium components** | **Units per liter** | **557A** | **810D** | |
|---|---|---|---|---|
| MS BASAL SALT MIXTURE | G | | | |
| N6 MACRONUTRIENTS 10X | ml | | | |
| POTASSIUM NITRATE | G | | | |
| B5H MINOR SALTS 1000X | ml | | | |
| NaFe EDTA FOR B5H 100X | ml | | | |
| ERIKSSON'S VITAMINS 1000X | ml | | | |
| S&H VITAMIN STOCK 100X | ml | | | |
| THIAMINE. HCL | mg | | | |
| L-PROLINE | g | | | |
| CASEIN HYDROLYSATE (ACID) | g | | | |
| SUCROSE | g | 20.0 | | |
| GLUCOSE | g | | | |
| MALTOSE | g | | | |
| 2,4-D | mg | | | |
| AGAR | g | | | |
| PHYTAGEL | g | | | |
| DICAMBA | g | | | |
| SILVER NITRATE | mg | | | |
| AGRIBIO Carbenicillin | mg | | | |
| Timentin | mg | | | |
| Cefotaxime | mg | | | |
| MYO-INOSITOL | g | | | |
| NICOTINIC ACID | mg | | | |
| PYRIDOXINE.HCL | mg | | | |
| VITAMIN ASSAY CASAMINO ACIDS | g | | | |
| MES BUFFER | g | | | |
| ACETOSYRINGONE | uM | | | |
| ASCORBIC ACID 10MG/ML (7S) | mg | | | |
| MS VITAMIN STOCK SOL. | ml | | | |
| ZEATIN | mg | | | |
| CUPRIC SULFATE | mg | | | |
| IAA 0.5MG/ML (28A) | ml | | | |
| ABA 0.1mm | ml | | | |
| THIDIAZURON | mg | | | |
| AGRIBIO Carbenicillin | mg | | | |
| PPT(GLUFOSINATE-NH4) | mg | | | |
| BAP | mg | | | |
| YEAST EXTRACT (BD Difco) | g | | 5.0 | |
| PEPTONE | g | | 10.0 | |
| SODIUM CHLORIDE | g | | 5.0 | |
| SPECTINOMYCIN | mg | | 50.0 | |
| FERROUS SULFATE.7H20 | ml | | | |
| AB BUFFER 20X (12D) | ml | | | |
| AB SALTS 20X (12E) | ml | | | |
| Benomyl | mg | | | |
| POTASSIUM PHOSPHATE DIBASIC | g | 10.5 | | |
| POTASSIUM PHOSPHATE MONOBASIC ANHYDROUS | g | 4.5 | | |
| AMMONIUM SULFATE | g | 1.0 | | |
| SODIUM CITRATE DIHYDRATE | g | 0.5 | | |
| MAGNESIUM SULFATE 1.0M | ml | 1.0 | | |
| pH | | 5.6 | 6.8 | |

SSI events were identified using a multiplex PCR assay to detect the presence/absence of random *Agrobacterium* T-DNA and vector backbone analysis, and qPCR to determine the excision of the target gene, insertion of FLP (random T-DNA integration), and the FRT pair junction (1/87). The data are summarized in Table 22. A preciseSSI event was identified to have a single copy of the donor gene with intact FRT junctions (1/87), minus the target and FLP having no backbone insertion. The use of the helper plasmid (pVIR9) in the strain LBA4404THY- improved the recovery of SSI events from this strain, compared to the normal SSI recovery using the strain LBA4404THY-without the helper plasmid. In absence of the helper plasmid (pVIR9), LBA4404THY- strain harboring ternary vectors comprising two T-DNA expression cassettes; first T-DNA consisting of ZmUbiPro::FLP::PINII + FRT1-PMI::PINII + ZmUbiPro::DsRED::PINII-FRT87 (pPHP60577; SEQ ID NO.75) and, second T-DNA comprising of; loxP-Rab17Pro::Cre + NosPro::WUS::PINII + ZmUbiPro::BBM::PINII-loxP (pPHP44542; SEQ ID NO.76) resulted in very low SSI frequency (0.1%) in the genotype PHR03 (U.S. Provisional Appl. No. 62/296639) compared to the SSI frequency (0.4-0.5%) observed at multiple RTLs in PH184C, a inbred recalcitrant to Agro SSI, with LBA4404THY-71539 strain.

**Table 22. Transformation frequency and SSI frequency in PH184C with strain AGL1 and LBA4404 with pVIR9**

| Construct/Target | Agro | T0 plants (#) | SSI events (#) | SSI (%) |
|---|---|---|---|---|
| 79366/ZMRTLA | AGL1 | 11 | 5 | 0.6% |
| 793 66/ZMRTLB | AGL1 | 26 | 10 | 1.1% |
| 793 66/ZMRTL0C | AGL1 | 39 | 17 | 2.3% |
| 79366/ZMRTLA | LBA4404 +pVIR9 | 6 | 3 | 0.4% |
| 793 66/ZMRTLB | LBA4404 +pVIR9 | 19 | 4 | 0.5% |
| 793 66/ZMRTLC | LBA4404 +pVIR9 | 32 | 4 | 0.5% |

One skilled in the art would appreciate that incorporation of the helper plasmid pVIR7 (SEQ ID NO: 34) or pVIR10 (SEQ ID NO: 36) in different Agrobacterium strain can enhance the virulence of the Agro strain to improve SSI frequency in multiple corn inbreds.

### EXAMPLE 13: IMPROVING AGRO SSI USING DONOR CASSETTES AND/OR HELPER PLASMIDS WITH DIFFERENT ORIGINS OF REPLICATION

Another way to improve Agro SSI is to build donor cassettes with different bacterial Origins of Replication (Ori) that vary in plasmid copy number (High, medium, low; for eg. RepABC, pRi, pVS1, RK2 etc) allowing to titrate (high to low) the amount of donor DNA molecules delivered into plant cell. Alternatively, the donor cassettes harboring different Ori can be combined with helper plasmids that carry additional virulence genes. These helper plasmids may have different bacterial origins of replication (RepABC, pRi, pVS1, RK2), with varied plasmid copy number. This method may improve the efficiency of SSI event and recovery.

### EXAMPLE 14: SEQUENCE IDENTIFICATION NUMBERS (SEQ ID NO:)

**Table 23.**

| **SEQID** | **DESCRIPTION** |
|---|---|
| **1** | aacC1 gene; Pseudomonas aeruginosa |
| **2** | ColEl ori; Escherichia coli |
| **3** | pVS1 ori; Pseudomonas aeruginosa |
| **4** | VirB1; Agrobacterium tumefaciens |
| **5** | VirB2; Agrobacterium tumefaciens |
| **6** | VirB3; Agrobacterium tumefaciens |
| **7** | VirB4; Agrobacterium tumefaciens |
| **8** | VirB5; Agrobacterium tumefaciens |
| **9** | VirB6; Agrobacterium tumefaciens |
| **10** | VirB7; Agrobacterium tumefaciens |
| **11** | VirB8; Agrobacterium tumefaciens |
| **12** | VirB9; Agrobacterium tumefaciens |
| **13** | VirB10; Agrobacterium tumefaciens |
| **14** | VirB11; Agrobacterium tumefaciens |
| **15** | VirG; Agrobacterium tumefaciens |
| **16** | VirCl; Agrobacterium tumefaciens |
| **17** | VirC2; Agrobacterium tumefaciens |
| **18** | VirD1; Agrobacterium tumefaciens |
| **19** | VirD2; Agrobacterium tumefaciens |
| **20** | VirD3; Agrobacterium tumefaciens |
| **21** | VirD4; Agrobacterium tumefaciens |
| **22** | VirD5; Agrobacterium tumefaciens |
| **23** | VirE1; Agrobacterium tumefaciens |
| **24** | VirE2; Agrobacterium tumefaciens |
| **25** | VirE3; Agrobacterium tumefaciens |
| **26** | VirA; Agrobacterium tumefaciens |
| **27** | VirJ; Agrobacterium tumefaciens |
| **28** | PHP45981; Artificial sequence |
| **29** | PHP64484; Artificial sequence |
| **30** | ZmUbiPro; Artificial sequence |
| **31** | DsRED; Discosoma sp. |
| **32** | FRT1; Saccharomyces cerevisiae |
| **33** | FRT87; Saccharomyces cerevisiae |
| **34** | pVIR7; PHP70298; Artificial sequence |
| **35** | pVIR9; PHP71539; Artificial sequence |
| **36** | pVIR10; PHP79761; Artificial sequence |
| **37** | pRSF1010 ori; Escherichia coli |
| **38** | pRK2 ori; Escherichia coli |
| **39** | aadA selection cassette; Escherichia coli |
| **40** | npt1 selection cassette; Escherichia coli |
| **41** | npt2 selection cassette; Escherichia coli |
| **42** | VirH; Agrobacterium tumefaciens |
| **43** | VirH1; Agrobacterium tumefaciens |
| **44** | VirH2; Agrobacterium tumefaciens |
| **45** | VirK; Agrobacterium tumefaciens |
| **46** | VirL; Agrobacterium tumefaciens |
| **47** | VirM; Agrobacterium tumefaciens |
| **48** | VirP; Agrobacterium tumefaciens |
| **49** | VirQ; Agrobacterium tumefaciens |
| **50** | pSC101 ori; Salmonella tymphimurium |
| **51** | p15A ori, Escherichia coli |
| **52** | R6K ori gamma pir; Escherichia coli |
| **53** | pSa repA ori; Escherichia coli |
| **54** | pRK2 micro; Escherichia coli |
| **55** | PARDE; Escherichia coli |
| **56** | pSaPARDE; Artificial sequence |
| **57** | repABC-pRi1724; Agrobacterium rhizogenes |
| **58** | repABC-pTi-SAKURA; Agrobacterium tumefaciens |
| **59** | repABC; PR1b plasmid, Ruegeria sp. |
| **60** | repABC; pNGR234, Sinorhizobium fredii |
| **61** | pSB1 32 bp palindrome; Artificial sequence |
| **62** | pSB1 142 bp inverted repeat; Artificial sequence |
| **63** | pSB1 tra-trb region; Artificial sequence |
| **64** | trfA; Escherichia coli |
| **65** | oriV; Escherichia coli |
| **66** | pRK2 mini (oriV-nptIII-trfA); Escherichia coli |
| **67** | hpt selection cassette; Escherichia coli |
| **68** | UBI-ZMPRO: MO-PAT: PROTEIN LINKER:DSRED :PINII |
| **69** | pPHP80561 |
| **70** | pPHP80559 |
| **71** | pPHP79066 |
| **72** | pPHP78147 |
| **73** | pPHP78148 |
| **74** | pPHP79366 |
| **75** | pPHP60577 |
| **76** | pPHP44542 |
| **77** | SpcN; Streptomyces spectabilis |
| **78** | aph; Legionella pneumophila |
| **79** | AR-VIRA; Agrobacterium rhizogenes |
| **80** | AR-VIRB 1; Agrobacterium rhizogenes |
| **81** | AR-VIRB2; Agrobacterium rhizogenes |
| **82** | AR-VIRB3; Agrobacterium rhizogenes |
| **83** | AR-VIRB4; Agrobacterium rhizogenes |
| **84** | AR-VIRB5; Agrobacterium rhizogenes |
| **85** | AR-VIRB6; Agrobacterium rhizogenes |
| **86** | AR-VIRB7; Agrobacterium rhizogenes |
| **87** | AR-VIRB8; Agrobacterium rhizogenes |
| **88** | AR-VIRB9; Agrobacterium rhizogenes |
| **89** | AR-VIRB10; Agrobacterium rhizogenes |
| **90** | AR-VIRB11; Agrobacterium rhizogenes |
| **91** | AR-VIRG; Agrobacterium rhizogenes |
| **92** | AR-VIRC1; Agrobacterium rhizogenes |
| **93** | AR-VIRC2; Agrobacterium rhizogenes |
| **94** | AR-VIRD1; Agrobacterium rhizogenes |
| **95** | AR-VIRD2; Agrobacterium rhizogenes |
| **96** | AR-VIRD3; Agrobacterium rhizogenes |
| **97** | AR-VIRBD4; Agrobacterium rhizogenes |
| **98** | AR-VIRD5; Agrobacterium rhizogenes |
| **99** | AR-VIRF; Agrobacterium rhizogenes |
| **100** | AR-VIRE3; Agrobacterium rhizogenes |
| **101** | AR-GALLS; Agrobacterium rhizogenes |
| **102** | BBR1 ori; Bordetella bronchiseptica |

As used herein the singular forms "a", "an", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this disclosure belongs unless clearly indicated otherwise.

All patents, publications and patent applications mentioned in the specification are indicative of the level of those skilled in the art to which this disclosure pertains.

## Claims

1. A vector comprising:
(a) an origin of replication for propagation and stable maintenance in *Escherichia coli*;
(b) an origin of replication for propagation and stable maintenance in *Agrobacterium* spp.;
(c) a selectable marker gene; and
(d) *Agrobacterium* spp. virulence genes:
(i) *virB1-B11* having SEQ ID NOS: 4-14, respectively, or *r-virB1-B11* having SEQ ID NOS: 80-90, respectively,
(ii) *virC1-C2* having SEQ ID NOS: 16-17, respectively, or *r-virC1-C2* having SEQ ID NOS: 92-93, respectively,
(iii) *virD1-D2* having SEQ ID NOS: 18-19, respectively, or *r-virD1-*D2 having SEQ ID NOS: 94-95, respectively, and
(iv) *virG* having SEQ ID NO: 15 or *r-virG* having SEQ ID NO: 91,
wherein the vector comprising *r-virB1-B11* having SEQ ID NOS: 80-90, respectively, *r-virC1-C2* having SEQ ID NOS: 92-93, respectively, r-*virD1-D2* having SEQ ID NOS: 94-95, respectively, and *r-virG* having SEQ ID NO: 91 further comprises *r-galls* having SEQ ID NO: 101.

2. The vector of claim 1, further comprising one or more of *Agrobacterium* spp. virulence genes:
(a) *virA* having SEQ ID NO: 26 or *r-virA* having SEQ ID NO: 79;
(b) *virD3-D5* having SEQ ID NOS: 20-22, respectively, or *r-virD3-D5* having SEQ ID NO: 96-98, respectively;
(c) *virE1-E3* having SEQ ID NOS: 23-25, respectively, or *r-virE3* having SEQ ID NO: 100;
(d) *virH-H2* having SEQ ID NOS: 42-44, respectively;
(e) *virJ* having SEQ ID NO: 27;
(f) *virK* having SEQ ID NO: 45;
(g) *virL* having SEQ ID NO: 46;
(h) *virM* having SEQ ID NO: 47;
(i) *virP* having SEQ ID NO: 48;
(j) *virQ* having SEQ ID NO: 49;
(k) *virD3-D5* having SEQ ID NOS: 20-22, respectively, and *virE1-E3* having SEQ ID NOS: 23-25, respectively, or *r-virD3-D5* having SEQ ID NO: 96-98, respectively, and *r-vir E3* having SEQ ID NO: 100; or
(l) *virA* having SEQ ID NO: 26, *virD3-D5* having SEQ ID NOS: 20-22, respectively, *virE1-E3* having SEQ ID NOS: 23-25, respectively, and *virJ* having SEQ ID NO: 27, or *r-virA* having SEQ ID NO: 79, *r-virD3-D5* having SEQ ID NO: 96-98, respectively, and *r-virE3* having SEQ ID NO: 100.

3. The vector of claim 1 or 2, wherein the origin of replication for propagation and stable maintenance in *Escherichia coli* is derived from a Col El, a pSC101, a p15A, or a R6K origin of replication;
preferably wherein:
(a) the origin of replication derived from the ColEl origin of replication has SEQ ID NO: 2;
(b) the origin of replication derived from the pSC101 origin of replication has SEQ ID NO: 50;
(c) the origin of replication derived from the p15A origin of replication has SEQ ID NO: 51; or
(d) wherein the origin of replication derived from the R6K origin of replication has SEQ ID NO: 52.

4. The vector of any one of claims 1-3, wherein the origin of replication for propagation and stable maintenance in *Agrobacterium* spp. is:
(A) a high copy number origin of replication;
(B) an intermediate copy number origin of replication;
(C) a low copy number origin of replication;
(D) derived from a pRi, a pVS1, a pRSF1010, a pRK2, a pSa, or a pBBR1 origin of replication; preferably wherein the origin of replication for propagation and stable maintenance in *Agrobacterium* spp. is:
(a) a variant of the pRK2 origin of replication;
(b) derived from the pRSF1010 origin of replication;
(c) derived from the pVS1 origin of replication;
(d) derived from the pSa origin of replication; or
(e) an origin of replication having any one of SEQ ID NOS: 3, 37, 38, 53, 57, 58, 59, or 60; or
(E) a repABC compatible origin of replication; preferably wherein the repABC compatible origin of replication has any one of SEQ ID NOS: 57, 58, 59, or 60.

5. The vector of claim 1 or 2, wherein the origin of replication for propagation and stable maintenance in *Escherichia coli* and the origin of replication for propagation and stable maintenance in *Agrobacterium* spp. are the same origin of replication;
preferably wherein the origin of replication is derived from a pRK2 origin of replication, from a pSa origin of replication, or a pRSF1010 origin of replication.

6. The vector of claim 5, wherein the pRK2 origin of replication has SEQ ID NO: 38;
and/or wherein the pRK2 origin of replication is:
(a) a micro pRK2 origin of replication; preferably wherein the micro pRK2 origin of replication has SEQ ID NO: 54; or
(b) a mini pRK2 origin of replication; preferably wherein the mini pRK2 has SEQ ID NO: 66;
and/or wherein the pRK2 origin of replication comprises the *trfA* and *OriV* sequences; preferably wherein the pRK2 origin of replication comprises SEQ ID NOS: 64 and 65.

7. The vector of claim 5, wherein:
(a) the pSa origin of replication has SEQ ID NO: 53; or
(b) the pRSF1010 origin of replication has SEQ ID NO: 37.

8. The vector of any one of claims 5-7, further comprising a sequence derived from the par DE operon;
preferably wherein the par DE operon has SEQ ID NO: 55.

9. The vector of any one of claims 1-8, wherein the selectable marker gene:
(A) provides resistance to gentamicin, neomycin/kanamycin, hygromycin, or spectinomycin; preferably wherein the selectable marker gene is an *aacC1* gene, a *npt1* gene, a *npt2* gene, a *hpt* gene, an aadA gene, a *SpcN* gene, *or an aph* gene;
more preferably wherein:
(a) the *aacC1* selectable marker gene has SEQ ID NO: 1;
(b) the *aadA* selectable marker gene has SEQ ID NO: 39;
(c) the *nptl* selectable marker gene has SEQ ID NO: 40;
(d) the *npt2* selectable marker gene has SEQ ID NO: 41;
(e) the *hpt* selectable marker gene has SEQ ID NO: 67;
(f) the *SpcN* selectable marker gene has SEQ ID NO: 77; or
(g) the *aph* selectable marker gene has SEQ ID NO: 78;
(B) does not provide resistance to tetracycline;
(C) is not a *tetAR* gene; or
(D) is a counter-selectable marker gene; preferably wherein:
(a) the counter-selectable marker gene is a *sacB* gene, a *rpsL* (*strA*) gene, *a pheS* gene, a *dhfr* (*folA*) gene, a *lacY* gene, a *Gata-1* gene, a *ccdB* gene, or a *thyA-* gene;
(b) the vector does not comprise SEQ ID NO: 61;
and/or wherein the vector does not comprise SEQ ID NO: 62;
and/or wherein the vector does not comprise a *tra* operon sequence or a *trb* operon sequence; preferably wherein the vector does not comprise SEQ ID NO: 63.

10. The vector of claim 1, wherein the vector has:
(a) SEQ ID NO: 34;
(b) SEQ ID NO: 35; or
(c) SEQ ID NO: 36.

11. A vector comprising:
(a) an origin of replication for propagation in *Escherichia coli* having SEQ ID NO: 2;
(b) an origin of replication for propagation in *Agrobacterium* spp. having SEQ ID NO: 3;
(c) a selectable marker gene having SEQ ID NO: 1; and:
(I)(d) *Agrobacterium* spp. virulence genes:
(i) *virB1-B11* having SEQ ID NOS: 4-14, respectively, or *r-virB1-B11* having SEQ ID NOS: 80-90, respectively,
(ii) *virC1-C2* having SEQ ID NOS: 16-17, respectively, or *r-virC1-C2* having SEQ ID NOS: 92-93, respectively,
(iii) *virD1-D2* having SEQ ID NOS: 18-19, respectively, or *r-virD1-*D2 having SEQ ID NOS: 94-95, respectively, and
(iv) *virG* having SEQ ID NO: 15 or *r-virG* having SEQ ID NO: 91,
wherein the vector comprising *r-virB1-B11* having SEQ ID NOS: 80-90, respectively, *r-virC1-C2* having SEQ ID NOS: 92-93, respectively, r-*virD1-D2* having SEQ ID NOS: 94-95, respectively, and *r-virG* having SEQ ID NO: 91 further comprises *r-galls* having SEQ ID NO: 101;
(II)(d) *Agrobacterium* spp. virulence genes:
(i) *virB1-B11* having SEQ ID NOS: 4-14, respectively, or *r-virB1-B11* having SEQ ID NOS: 80-90, respectively,
(ii) *virC1-C2* having SEQ ID NOS: 16-17, respectively, or *r-virC1-C2* having SEQ ID NOS: 92-93, respectively,
(iii) *virD1-D5* having SEQ ID NOS: 18-22, respectively, or *r-virD1-D5* having SEQ ID NOS: 94-98, respectively,
(iv) *virE1-E3* having SEQ ID NOS: 23-25, respectively, or *r-virE3* having SEQ ID NO: 100, and
(v) *virG* having SEQ ID NO: 15 or *r-virG* having SEQ ID NO: 91,
wherein the vector comprising *r-virB1-B11* having SEQ ID NOS: 80-90, respectively, *r-virC1-C2* having SEQ ID NOS: 92-93, respectively, *r-virD1-D5* having SEQ ID NOS: 94-98, respectively, *r-virE3* having SEQ ID NO: 100, and *r-virG* having SEQ ID NO: 91 further comprises *r-galls* having SEQ ID NO: 101; or
(III)(d) *Agrobacterium* spp. virulence genes:
(i) *virA* having SEQ ID NO: 26 or *r-virA* having SEQ ID NO: 79,
(ii) *virB1-B11* having SEQ ID NOS: 4-14, respectively, or *r-virB1-B11* having SEQ ID NOS: 80-90, respectively,
(iii) *virC1-C2* having SEQ ID NOS: 16-17, respectively, or *r-virC1-C2* having SEQ ID NOS: 92-93, respectively,
(iv) *virD1-D5* having SEQ ID NOS: 18-22, respectively, or *r-virD1-D5* having SEQ ID NOS: 94-98, respectively,
(v) *virE1-E3* having SEQ ID NOS: 23-25, respectively, or *r-virE3* having SEQ ID NO: 100,
(vi) *virG* having SEQ ID NO: 15 or *r-virG* having SEQ ID NO: 91, and
(vii) *virJ* having SEQ ID NO: 27,
wherein the vector comprising *r-virA* having SEQ ID NO: 79, *r*-vir*B1-B11* having SEQ ID NOS: 80-90, respectively, *r-virC1-C2* having SEQ ID NOS: 92-93, respectively, *r-virD1-D5* having SEQ ID NOS: 94-98, respectively, *r-virE3* having SEQ ID NO: 100, *r-virG* having SEQ ID NO: 91, and *virJ* having SEQ ID NO: 27 further comprises *r-galls* having SEQ ID NO: 101.

12. A method for transformation of a plant comprising the steps of:
(a) contacting a tissue from the plant with an *Agrobacterium* strain or an *Ochrobactrum* strain comprising a first vector of any one of claims 1-11, and a second vector comprising T-DNA borders and a polynucleotide sequence of interest for transfer to the plant;
(b) co-cultivating the tissue with the *Agrobacterium* strain or the *Ochrobactrum* strain; and
(c) regenerating a transformed plant from the tissue that expresses the polynucleotide sequence of interest.

13. A kit comprising:
(a) a vector of any one of claims 1-11; and
(b) instructions for use in transformation of a plant using *Agrobacterium* or *Ochrobactrum.*

## Patentansprüche

1. Vektor, umfassend:
(a) einen Replikationsursprung für Vermehrung und stabile Aufrechterhaltung in *Escherichia coli*;
(b) einen Replikationsursprung für Vermehrung und stabile Aufrechterhaltung in *Agrobacterium* spp.;
(c) ein selektierbares Markergen; und
(d) *Agrobacterium* spp. Virulenzgene:
(i) *virB1-B11* mit entsprechend SEQ ID NO: 4-14 oder *r-virB1-B11* mit entsprechend SEQ ID NO: 80-90,
(ii) *virC1-C2* mit entsprechend SEQ ID NO: 16-17 oder *r-virC1-C2* mit entsprechend SEQ ID NO: 92-93,
(iii) *virD1-D2* mit entsprechend SEQ ID NO: 18-19 oder *r-virD1-D2* mit entsprechend SEQ ID NO: 94-95 und
(iv) *virG* mit SEQ ID NO: 15 oder *r-virG* mit SEQ ID NO: 91,
wobei der Vektor, umfassend *r-virB1-B11* mit entsprechend SEQ ID NO: 80-90, *r-virC1-C2* mit entsprechend SEQ ID NO: 92-93, *r-virD1-D2* mit entsprechend SEQ ID NO: 94-95 und *r-virG* mit SEQ ID NO: 91, ferner *r-galls* mit SEQ ID NO: 101 umfasst.

2. Vektor nach Anspruch 1, ferner umfassend ein oder mehrere *Agrobacterium* spp. Virulenzgene:
(a) *virA* mit SEQ ID NO: 26 oder *r-virA* mit SEQ ID NO: 79;
(b) *virD3-D5* mit entsprechend SEQ ID NO: 20-22 oder *r-virD3-D5* mit entsprechend SEQ ID NO: 96-98;
(c) *virE1-E3* mit entsprechend SEQ ID NO: 23-25 oder *r-virE3* mit SEQ ID NO: 100;
(d) *virH-H2* mit entsprechend SEQ ID NO: 42-44;
(e) *virJ* mit SEQ ID NO: 27;
(f) *virK* mit SEQ ID NO: 45;
(g) *virL* mit SEQ ID NO: 46;
(h) *virM* mit SEQ ID NO: 47;
(i) *virP* mit SEQ ID NO: 48;
(j) *virQ* mit SEQ ID NO: 49;
(k) *virD3-D5* mit entsprechend SEQ ID NO: 20-22 und *virE1-E3* mit entsprechend SEQ ID NO: 23-25 oder *r-virD3-D5* mit entsprechend SEQ ID NO: 96-98 und *r-virE3* mit SEQ ID NO: 100; oder
(l) *virA* mit SEQ ID NO: 26, *virD3-D5* mit entsprechend SEQ ID NO: 20-22, *virE1-E3* mit entsprechend SEQ ID NO: 23-25 und *virJ mit* SEQ ID NO: 27 oder *r-virA* mit SEQ ID NO: 79, *r-virD3-D5* mit entsprechend SEQ ID NO: 96-98 und *r-virE3* mit SEQ ID NO: 100.

3. Vektor nach Anspruch 1 oder 2, wobei der Replikationsursprung für Vermehrung und stabile Aufrechterhaltung in *Escherichia coli* von einem ColE1-, einem pSC101-, einem pl5A- oder einem R6K-Replikationsursprung abgeleitet ist;
bevorzugt wobei:
(a) der von dem ColE1-Replikationsursprung abgeleitete Replikationsursprung SEQ ID NO: 2 hat;
(b) der von dem pSC101-Replikationsursprung abgeleitete Replikationsursprung SEQ ID NO: 50 hat;
(c) der von dem p 15A-Replikationsursprung abgeleitete Replikationsursprung SEQ ID NO: 51 hat; oder
(d) der von dem R6K-Replikationsursprung abgeleitete Replikationsursprung SEQ ID NO: 52 hat.

4. Vektor nach einem der Ansprüche 1-3, wobei der Replikationsursprung für Vermehrung und stabile Aufrechterhaltung in *Agrobacterium* spp. Folgendes ist:
(A) ein Replikationsursprung mit hoher Kopienzahl;
(B) ein Replikationsursprung mit mittlerer Kopienzahl;
(C) ein Replikationsursprung mit niedriger Kopienzahl;
(D) abgeleitet von einem pRi- einem pVS1-, einem pRSF1010-, einem pRK2-, einem pSA- oder einem pBBR1-Replikationsursprung; wobei bevorzugt der Replikationsursprung für Vermehrung und stabile Aufrechterhaltung in *Agrobacterium* spp. Folgendes ist:
(a) eine Variante des pRK2-Replikationsursprungs;
(b) abgeleitet von dem pRSF 1010-Replikationsursprung;
(c) abgeleitet von dem pVS1-Replikationsursprung;
(d) abgeleitet von dem pSa-Replikationsursprung; oder
(e) ein Replikationsursprung mit einem aus SEQ ID NO: 3, 37, 38, 53, 57, 58, 59 oder 60; oder
(E) ein repABC-kompatibler Replikationsursprung; wobei bevorzugt der repABCkompatible Replikationsursprung eine aus SEQ ID NO: 57, 58, 59 oder 60 hat.

5. Vektor nach Anspruch 1 oder 2, wobei der Replikationsursprung für Vermehrung und stabile Aufrechterhaltung in *Escherichia coli* und der Replikationsursprung für Vermehrung und stabile Aufrechterhaltung in *Agrobacterium* spp. der gleiche Replikationsursprung sind;
wobei bevorzugt der Replikationsursprung von einem pRK2-Replikationsursprung, von einem pSa-Replikationsursprung oder einem pRSF1010-Replikationsursprung abgeleitet ist.

6. Vektor nach Anspruch 5, wobei der pRK2-Replikationsursprung SEQ ID NO: 38 hat;
und/oder wobei der pRK2-Replikationsursprung Folgendes ist:
(a) ein Mikro-pRK2-Replikationsursprung; wobei bevorzugt der Mikro-pRK2-Replikationsursprung SEQ ID NO: 54 hat; oder
(b) ein Mini-pRK2-Replikationsursprung; wobei bevorzugt der Mini-pRK2-Replikationsursprung SEQ ID NO: 66 hat;
und/oder wobei der pRK2-Replikationsursprung die *trfA-* und *OriV-Sequenzen* umfasst; wobei bevorzugt der pRK2-Replikationsursprung SEQ ID NO: 64 und 65 umfasst.

7. Vektor nach Anspruch 5, wobei:
(a) der pSa-Replikationsursprung SEQ ID NO: 53 hat; oder
(b) der pRSF1010-Replikationsursprung SEQ ID NO: 37 hat.

8. Vektor nach einem der Ansprüche 5-7, ferner umfassend eine Sequenz, abgeleitet von dem par-DE-Operon;
wobei bevorzugt das par-DE-Operon SEQ ID NO: 55 hat.

9. Vektor nach einem der Ansprüche 1-8, wobei das selektierbare Markergen:
(A) Resistenz gegenüber Gentamicin, Neomycin/Kanamycin, Hygromycin oder Spectinomycin bereitstellt, wobei bevorzugt das selektierbare Markergen ein *aacC1-*Gen, ein *npt1*-Gen, ein *npt2*-Gen, ein *hpt*-Gen, ein aadA-Gen, ein *SpcN*-Gen oder ein *aph*-Gen ist; wobei bevorzugter:
(a) das selektierbare *aacC1*-Markergen SEQ ID NO: 1 hat;
(b) das selektierbare *aadA*-Markergen SEQ ID NO: 39 hat;
(c) das selektierbare *npt1*-Markergen SEQ ID NO: 40 hat;
(d) das selektierbare *npt2*-Markergen SEQ ID NO: 41 hat;
(e) das selektierbare *hpt*-Markergen SEQ ID NO: 67 hat;
(f) das selektierbare *SpcN*-Markergen SEQ ID NO: 77 hat; oder
(g) das selektierbare *aph*-Markergen SEQ ID NO: 78 hat;
(B) keine Resistenz gegenüber Tetracyclin bereitstellt;
(C) kein *tetAR-Gen* ist; oder
(D) ein gegenselektierbares Markergen ist, wobei bevorzugt:
(a) das gegenselektierbare Markergen ein *sacB-Gen,* ein *rpsL* (*strA*) -Gen, ein *pheS*-Gen, ein *dhfr*(*folA*) -Gen, ein *lacY-Gen,* ein *Gata-1*-Gen, ein *ccdB*-Gen oder ein *thyA*-Gen ist;
(b) der Vektor nicht SEQ ID NO: 61 umfasst;
und/oder wobei der Vektor nicht SEQ ID NO: 62 umfasst;
und/oder wobei der Vektor keine *tra*-Operonsequenz oder keine *trb*-Operonsequenz umfasst; wobei bevorzugt der Vektor nicht SEQ ID NO: 63 umfasst.

10. Vektor nach Anspruch 1, wobei der Vektor Folgendes hat:
(a) SEQ ID NO: 34;
(b) SEQ ID NO: 35; oder
(c) SEQ ID NO: 36.

11. Vektor, umfassend:
(a) einen Replikationsursprung für Vermehrung in *Escherichia coli* mit SEQ ID NO: 2;
(b) einen Replikationsursprung für Vermehrung in *Agrobacterium* spp. mit SEQ ID NO: 3;
(c) ein selektierbares Markergen mit SEQ ID NO: 1; und:
(I)(d) *Agrobacterium* spp. Virulenzgene:
(i) *virB1-B11* mit entsprechend SEQ ID NO: 4-14 oder *r-virB1-B11* mit entsprechend SEQ ID NO: 80-90,
(ii) *virC1-C2* mit entsprechend SEQ ID NO: 16-17 oder *r-virC1-C2* mit entsprechend SEQ ID NO: 92-93,
(iii) *virD1-D2* mit entsprechend SEQ ID NO: 18-19 oder *r-virD1-D2* mit entsprechend SEQ ID NO: 94-95 und
(iv) *virG* mit SEQ ID NO: 15 oder *r-virG* mit SEQ ID NO: 91,
wobei der Vektor, umfassend *r-virB1-B11* mit entsprechend SEQ ID NO: 80-90, *r-virC1-C2* mit entsprechend SEQ ID NO: 92-93, *r-virD1-D2* mit entsprechend SEQ ID NO: 94-95 und *r-virG* mit SEQ ID NO: 91, ferner *r-galls* mit SEQ ID NO: 101 umfasst;
(II)(d) *Agrobacterium* spp. Virulenzgene:
(i) *virB1-B11* mit entsprechend SEQ ID NO: 4-14 oder *r-virB1-B11* mit entsprechend SEQ ID NO: 80-90,
(ii) *virC1-C2* mit entsprechend SEQ ID NO: 16-17 oder *r-virC1-C2* mit entsprechend SEQ ID NO: 92-93,
(iii) *virD1-D5* mit entsprechend SEQ ID NO: 18-22 oder *r-virD1-D5* mit entsprechend SEQ ID NO: 94-98,
(iv) *virE1-E3* mit entsprechend SEQ ID NO: 23-25 oder *r-virE3* mit SEQ ID NO: 100 und
(v) *virG* mit SEQ ID NO: 15 oder *r-virG* mit SEQ ID NO: 91,
wobei der Vektor, umfassend *r-virB1-B11* mit entsprechend SEQ ID NO: 80-90, *r-virC1-C2* mit entsprechend SEQ ID NO: 92-93, *r-virD1-D5* mit entsprechend SEQ ID NO: 94-98, *r-virE3* mit SEQ ID NO: 100 und *r-virG* mit SEQ ID NO: 91, ferner *r-galls* mit SEQ ID NO: 101 umfasst; oder
(III)(d) *Agrobacterium* spp. Virulenzgene:
(i) *virA* mit SEQ ID NO: 26 oder *r-virA* mit SEQ ID NO: 79,
(ii) *virB1-B11* mit entsprechend SEQ ID NO: 4-14 oder *r-virB1-B11* mit entsprechend SEQ ID NO: 80-90,
(iii) *virC1-C2* mit entsprechend SEQ ID NO: 16-17 oder *r-virC1-C2* mit entsprechend SEQ ID NO: 92-93,
(iv) *virD1-D5* mit entsprechend SEQ ID NO: 18-22 oder *r-virD1-D5* mit entsprechend SEQ ID NO: 94-98,
(v) *3* mit entsprechend SEQ ID NO: 23-25 oder *r-virE3* mit SEQ ID NO: 100,
(vi) *virG* mit SEQ ID NO: 15 oder *r-virG* mit SEQ ID NO: 91 und
(vii) *virJ* mit SEQ ID NO: 27,
wobei der Vektor, umfassend *r-virA* mit SEQ ID NO: 79, *r-virB1-B11* mit entsprechend SEQ ID NO: 80-90, *r-virC1-C2* mit entsprechend SEQ ID NO: 92-93, *r-virD1-D5* mit entsprechend SEQ ID NO: 94-98, *r-virE3* mit SEQ ID NO: 100 und *r-virG* mit SEQ ID NO: 91 und *virJ* mit SEQ ID NO: 27, ferner *r-galls* mit SEQ ID NO: 101 umfasst.

12. Verfahren für die Transformation einer Pflanze, umfassend die folgenden Schritte:
(a) Kontaktieren eines Gewebes von der Pflanze mit einem *Agrobacterium*-Stamm oder einem *Ochrobactrum*-Stamm, umfassend einen ersten Vektor nach einem der Ansprüche 1-11 und einen zweiten Vektor, umfassend T-DNA-Grenzen und eine Polynukleotidsequenz von Interesse, zur Übertragung zu der Pflanze;
(b) Co-Kultivieren des Gewebes mit dem *Agrobacterium*-Stamm oder einem *Ochrobactrum*-Stamm; und
(c) Regenerieren einer transformierten Pflanze aus dem Gewebe, das die Polynukleotidsequenz von Interesse exprimiert.

13. Kit, umfassend:
(a) einen Vektor nach einem der Ansprüche 1-11; und
(b) Anweisungen für die Verwendung bei der Transformation einer Pflanze unter Verwendung von *Agrobacterium* oder *Ochrobactrum.*

## Revendications

1. Vecteur, comprenant :
(a) une origine de réplication pour propagation et maintien stable dans *Escherichia coli ;*
(b) une origine de réplication pour propagation et maintien stable dans *Agrobacterium* spp. ;
(c) un gène marqueur sélectionnable ; et
(d) des gènes de virulence *Agrobacterium* spp. :
(i) *virB1-B11* ayant SEQ ID n° : 4-14, respectivement, ou *r-virB1-B11* ayant SEQ ID n° : 80-90, respectivement,
(ii) *virC1-C2* ayant SEQ ID n° : 16-17, respectivement, ou *r-virC1-C2* ayant SEQ ID n° : 92-93, respectivement,
(iii) *virD1-D2* ayant SEQ ID n° : 18-19, respectivement, ou *r-virD1-*D2 ayant SEQ ID n° : 94-95, respectivement, et
(iv) *virG* ayant SEQ ID n° : 15 ou *r-virG* ayant SEQ ID n° : 91,
dans lequel le vecteur comprenant *r-virB1-B11* ayant SEQ ID n° : 80-90, respectivement, *r-virC1-C2* ayant SEQ ID n° : 92-93, respectivement, r-*virD1-D2* ayant SEQ ID n° : 94-95, respectivement, et *r-virG* ayant SEQ ID n° : 91 comprend en outre *r-galls* ayant SEQ ID n° : 101.

2. Vecteur selon la revendication 1, comprenant en outre un ou plusieurs de gènes de virulence *Agrobacterium* spp. :
(a) *virA* ayant SEQ ID n° : 26 ou *r-virA* ayant SEQ ID n° : 79 ;
(b) *virD3-D5* ayant SEQ ID n° : 20-22, respectivement, ou *r-virD3-D5* ayant SEQ ID n° : 96-98, respectivement ;
(c) *virE1-E3* ayant SEQ ID n° : 23-25, respectivement, ou *r-virE3* ayant SEQ ID n° : 100 ;
(d) *virH-H2* ayant SEQ ID n° : 42-44, respectivement ;
(e) *virJ* ayant SEQ ID n° : 27 ;
(f) *virK* ayant SEQ ID n° : 45 ;
(g) *virL* ayant SEQ ID n° : 46 ;
(h) *virM* ayant SEQ ID n° : 47 ;
(i) *virP* ayant SEQ ID n° : 48 ;
j) *virQ* ayant SEQ ID n° : 49 ;
(k) *virD3-D5* ayant SEQ ID n° : 20-22, respectivement, et *virE1-E3* ayant SEQ ID n° : 23-25, respectivement, ou *r-virD3-D5* ayant SEQ ID n° : 96-98, respectivement, et *r-virE3* ayant SEQ ID n° : 100 ; ou
(1) *virA* ayant SEQ ID n° : 26, *virD3-D5* ayant SEQ ID n° : 20-22,
respectivement, *virE1-E3* ayant SEQ ID n° : 23-25, respectivement, et *virJ* ayant SEQ ID n° : 27, ou *r-virA* ayant SEQ ID n° : 79, *r-virD3-D5* ayant SEQ ID n° : 96-98, respectivement, et *r-virE3* ayant SEQ ID n° : 100.

3. Vecteur selon la revendication 1 ou 2, dans lequel l'origine de réplication pour propagation et maintien stable dans *Escherichia coli* est dérivée d'une origine de réplication ColE1, pSC101, p15A, ou R6K ;
de préférence dans lequel :
(a) l'origine de réplication dérivée de l'origine de réplication ColE1 a SEQ ID n° : 2 ;
(b) l'origine de réplication dérivée de l'origine de réplication pSC101 a SEQ ID n° : 50 ;
(c) l'origine de réplication dérivée de l'origine de réplication p15A a SEQ ID n° : 51 ; ou
(d) dans lequel l'origine de réplication dérivée de l'origine de réplication R6K a SEQ ID n° : 52.

4. Vecteur selon l'une quelconque des revendications 1 à 3, dans lequel l'origine de réplication pour propagation et maintien stable dans *Agrobacterium* spp. est :
(A) une origine de réplication à nombre élevé de copies ;
(B) une origine de réplication à nombre moyen de copies ;
(C) une origine de réplication à nombre bas de copies ;
(D) dérivée d'une origine de réplication pRi, pVSl, pRSF1010, pRK2, pSa, ou pBBR1 ; de préférence dans lequel l'origine de réplication pour propagation et maintien stable dans *Agrobacterium* spp. est :
(a) un variant de l'origine de réplication pRK2 ;
(b) dérivée de l'origine de réplication pRSF1010 ;
(c) dérivée de l'origine de réplication pVS1 ;
(d) dérivée de l'origine de réplication pSa ; ou
(e) une origine de réplication ayant un quelconque de SEQ ID n° : 3, 37, 38, 53, 57, 58, 59, ou 60 ; ou
(E) une origine de réplication compatible repABC ; de préférence dans lequel l'origine de réplication compatible repABC a un quelconque de SEQ ID n° : 57, 58, 59, ou 60.

5. Vecteur selon la revendication 1 ou 2, dans lequel l'origine de réplication pour propagation et maintien stable dans *Escherichia coli* et l'origine de réplication pour propagation et maintien stable dans *Agrobacterium* spp. sont la même origine de réplication ;
de préférence dans lequel l'origine de réplication est dérivée d'une origine de réplication pRK2, d'une origine de réplication pSa, ou d'une origine de réplication pRSF1010.

6. Vecteur selon la revendication 5, dans lequel l'origine de réplication pRK2 a SEQ ID n° : 38 ; et/ou dans lequel l'origine de réplication pRK2 est :
(a) une origine de réplication micro pRK2 ; de préférence dans lequel l'origine de réplication micro pRK2 a SEQ ID n° : 54 ; ou (b) une origine de réplication mini pRK2 ; de préférence dans lequel mini pRK2 a SEQ ID n° : 66 ;
et/ou dans lequel l'origine de réplication pRK2 comprend les séquences *trfA* et *OriV*; de préférence dans lequel l'origine de réplication pRK2 comprend SEQ ID n° : 64 et 65.

7. Vecteur selon la revendication 5, dans lequel :
(a) l'origine de réplication pSa a SEQ ID n° : 53 ; ou
(b) l'origine de réplication pRSF1010 a SEQ ID n° : 37.

8. Vecteur selon l'une quelconque des revendications 5 à 7, comprenant en outre une séquence dérivée de l'opéron par DE ;
de préférence dans lequel l'opéron par DE a SEQ ID n° : 55.

9. Vecteur selon l'une quelconque des revendications 1 à 8, dans lequel le gène marqueur sélectionnable :
(A) fournit une résistance à la gentamicine, néomycine/kanamycine, hygromycine, ou spectinomycine ; de préférence dans lequel le gène marqueur sélectionnable est un gène *aacC1,* un gène *npt1,* un gène *npt2,* un gène *hpt,* un gène *aadA,* un gène *SpcN,* ou un gène *aph ;*
plus de préférence dans lequel :
(a) le gène marqueur sélectionnable *aacC1* a SEQ ID n° : 1 ;
(b) le gène marqueur sélectionnable *aadA* a SEQ ID n° : 39 ;
(c) le gène marqueur sélectionnable *npt1* a SEQ ID n° : 40 ;
(d) le gène marqueur sélectionnable *npt2* a SEQ ID n° : 41 ;
(e) le gène marqueur sélectionnable *hpt* a SEQ ID n° : 67 ;
(f) le gène marqueur sélectionnable *SpcN* a SEQ ID n° : 77 ; ou
(g) le gène marqueur sélectionnable *aph* a SEQ ID n° : 78 ;
(B) ne fournit pas de résistance à la tétracycline ;
(C) n'est pas un gène *tetAR* ; ou
(D) est un gène marqueur contre-sélectionnable ; de préférence dans lequel :
(a) le gène marqueur contre-sélectionnable est un gène *sacB,* un gène *rpsL (strA),* un gène *pheS,* un gène *dhfr* (*folA*)*,* un gène *lacY,* un gène *Gata-1,* un gène *ccdB,* ou un gène *thyA- ;*
(b) le vecteur ne comprend pas SEQ ID n° : 61 ;
et/ou dans lequel le vecteur ne comprend pas SEQ ID n° : 62 ;
et/ou dans lequel le vecteur ne comprend pas une séquence opéron *tra* ou une séquence opéron *trb* ; de préférence dans lequel le vecteur ne comprend pas SEQ ID n° : 63.

10. Vecteur selon la revendication 1, dans lequel le vecteur a :
(a) SEQ ID n° : 34 ;
(b) SEQ ID n° : 35 ; ou
(c) SEQ ID n° : 36.

11. Vecteur, comprenant :
(a) une origine de réplication pour propagation dans *Escherichia coli* ayant SEQ ID n° :2 ;
(b) une origine de réplication pour propagation dans *Agrobacterium* spp. ayant SEQ ID n° : 3 ;
(c) un gène marqueur sélectionnable ayant SEQ ID n° : 1 ; et : (I)(d) des gènes de virulence *Agrobacterium* spp. :
(i) *virB1-B11* ayant SEQ ID n° : 4-14, respectivement, ou *r-virB1-B11* ayant SEQ ID n° : 80-90, respectivement,
(ii) *virC1-C2* ayant SEQ ID n° : 16-17, respectivement, ou *r-virC1-C2* ayant SEQ ID n° : 92-93, respectivement,
(iii) *virD1-D2* ayant SEQ ID n° : 18-19, respectivement, ou *r-virD1-D2* ayant SEQ ID n° : 94-95, respectivement, et
(iv) *virG* ayant SEQ ID n° : 15 ou *r-virG* ayant SEQ ID n° : 91,
dans lequel le vecteur comprenant *r-virB1-B11* ayant SEQ ID n° : 80- 90, respectivement, *r-virC1-C2* ayant SEQ ID n° : 92-93, respectivement, *rvirD1-D2* ayant SEQ ID n° : 94-95, respectivement, et *r-virG* ayant SEQ ID n° : 91 comprend en outre *r-galls* ayant SEQ ID n° : 101 ;
(II)(d) des gènes de virulence *Agrobacterium* spp. :
(i) *virB1-B11* ayant SEQ ID n° : 4-14, respectivement, ou *r-virB1-B11* ayant SEQ ID n° : 80-90, respectivement,
(ii) *virC1-C2* ayant SEQ ID n° : 16-17, respectivement, ou *r-virC1-C2* ayant SEQ ID n° : 92-93, respectivement,
(iii) *virD1-D5* ayant SEQ ID n° : 18-22, respectivement, ou *r-virD1-D5* ayant SEQ ID n° : 94-98, respectivement,
(iv) *virE1-E3* ayant SEQ ID n° : 23-25, respectivement, ou *r-virE3* ayant SEQ ID n° : 100, et
(v) *virG* ayant SEQ ID n° : 15 ou *r-virG* ayant SEQ ID n° : 91,
dans lequel le vecteur comprenant *r-virB1-B11* ayant SEQ ID n° : 80- 90, respectivement, *r-virC1-C2* ayant SEQ ID n° : 92-93, respectivement, *r-virD1-D5* ayant SEQ ID n° : 94-98, respectivement, *r-virE3* ayant SEQ ID n° : 100, et *r-virG* ayant SEQ ID n° : 91 comprend en outre *r-galls* ayant SEQ ID n° : 101 ; ou
(III)(d) des gènes de virulence *Agrobacterium* spp. :
(i) *virA* ayant SEQ ID n° : 26 ou *r-virA* ayant SEQ ID n° : 79,
(ii) *virB1-B11* ayant SEQ ID n° : 4-14, respectivement, ou *r-virB1-B11* ayant SEQ ID n° : 80-90, respectivement,
(iii) *virC1-C2* ayant SEQ ID n° : 16-17, respectivement, ou *r-virC1-*C2 ayant SEQ ID n° : 92-93, respectivement,
(iv) *virD1-D5* ayant SEQ ID n° : 18-22, respectivement, ou *r-virD1-D5* ayant SEQ ID n° : 94-98, respectivement,
(v) *virE1-E3* ayant SEQ ID n° : 23-25, respectivement, ou *r-virE3* ayant SEQ ID n° : 100,
(vi) *virG* ayant SEQ ID n° : 15 ou *r-virG* ayant SEQ ID n° : 91, et
(vii) *virJ* ayant SEQ ID n° : 27,
dans lequel le vecteur comprenant *r-virA* ayant SEQ ID n° : 79, *r-virB1-B11* ayant SEQ ID n° : 80-90, respectivement, *r-virC1-C2* ayant SEQ ID n° : 92-93, respectivement, *r-virD1-D5* ayant SEQ ID n° : 94-98, respectivement, *r-virE3* ayant SEQ ID n° : 100, *r-virG* ayant SEQ ID n° : 91, et *virJ* ayant SEQ ID n° : 27 comprend en outre *r-galls* ayant SEQ ID n° : 101.

12. Procédé pour transformation d'une plante, comprenant les étapes de :
(a) la mise en contact d'un tissu de la plante avec une souche *Agrobacterium* ou une souche *Ochrobactrum* comprenant un premier vecteur selon l'une quelconque des revendications 1 à 11, et un second vecteur comprenant des bordures T-ADN et une séquence polynucléotidique d'intérêt pour le transfert à la plante ;
(b) la co-cultivation du tissu avec la souche *Agrobacterium* ou la souche *Ochrobactrum* ; et
(c) la régénération d'une plante transformée à partir du tissu qui exprime la séquence polynucléotidique d'intérêt.

13. Kit, comprenant :
(a) un vecteur selon l'une quelconque des revendications 1 à 11 ; et
(b) des instructions pour l'utilisation dans la transformation d'une plante en utilisant *Agrobacterium* ou *Ochrobactrum.*
